(19) Europäisches Patentamt
European Patent Office
Office européen des brevets

(11) Publication number: **0 167 919 B1**

(12) # EUROPEAN PATENT SPECIFICATION

(45) Date of publication of patent specification: **05.05.93**

(51) Int. Cl.$^5$: **C07D 243/18**, C07D 403/06, C07D 403/12, C07D 401/12, C07D 405/12, C07D 409/04, C07D 409/06, C07D 409/12, A61K 31/55

(21) Application number: **85107842.8**

(22) Date of filing: **25.06.85**

(54) **Benzodiazepine derivatives and pharmaceutical compositions containing them.**

(30) Priority: **26.06.84 US 624854**
**25.02.85 US 705272**
**10.06.85 US 741972**

(43) Date of publication of application:
**15.01.86 Bulletin 86/03**

(45) Publication of the grant of the patent:
**05.05.93 Bulletin 93/18**

(84) Designated Contracting States:
**AT BE CH DE FR GB IT LI LU NL SE**

(56) References cited:

| | |
|---|---|
| **CH-A- 581 606** | **DE-A- 1 923 821** |
| **FR-A- 1 437 376** | **FR-A- 2 132 195** |
| **FR-A- 2 220 269** | **FR-A- 2 239 464** |
| **GB-A- 972 968** | **GB-A- 1 034 872** |
| **GB-A- 1 039 947** | **GB-A- 1 063 891** |
| **GB-A- 1 173 320** | **GB-B- 1 409 404** |
| **US-A- 3 344 136** | **US-A- 3 801 568** |
| **US-A- 4 045 569** | **US-B- 3 270 053** |
| **US-B- 3 344 183** | **US-B- 3 402 171** |
| **US-B- 3 558 603** | **US-B- 3 867 529** |

(73) Proprietor: **MERCK & CO. INC.**
**126, East Lincoln Avenue P.O. Box 2000**
**Rahway New Jersey 07065-0900(US)**

(72) Inventor: **Freidinger, Roger M.**
**2185 Rebecca Drive**
**Hatfield PA 19440(US)**
Inventor: **Bock, Mark G.**
**1603 Leon Drive**
**Hatfield PA 19440(US)**
Inventor: **Evans, Ben E.**
**501 Perkiomen Avenue**
**Lansdale PA 19446(US)**

(74) Representative: **Barrett-Major, Julie Diane et al**
**Merck & Co., Inc. European Patent Department Terlings Park Eastwick Road**
**Harlow Essex CM20 2OR (GB)**

EP 0 167 919 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

NATURE, vol. 312, 22nd November 1984, pages 363–364, Basingstoke, US; J. BRADWEJN et al.: "Benzodiazepines antagonize cholecystokinin–induced activation of rat hippocampal neurones"

Japan Journal of Pharmacology 33 Supp. 87 p (1983), Kabota et al

SZABADALMI LEIRAS 178279 and Chemical Abstract 96: 122830Y

Yamanouchi Seiyaku Kenku Hokoku 1974, 2, 196–205 (Japan) and Chemical Abstract 84: 31018a

Synthesis 1979. (9), 718 and 719 and Chemical Abstract 92:76468d

English Translation from Khimiya Geterotsiklicheskikh Soedinenii 1979, No. (4), 545–9, April, 1979 and Chemical Abstract 91:157698k

Journal of Chromotography, 259 (1983) 494–498 and Chemical Abstract 99: 15947g

Fiziol. Aktiv. Veshchestva 1972, 4, 96–100 (Russia) and Chemical Abstract 78: 136245b

European Journal of Drug Metabolism and Pharmacokinetics, 1980, Vol. 5, No. 4, pg. 193–200 and Chemical Abstracts 94:202460b

Journal of Chromotography, 174(1979) 447–450 and Chemical Abstracts 91: 90976d

Boll. Chim. Farm., 107(1968) and Chemical Abstract 69:96684Z

Stanley C.Bell et al. article from May 1968 titled "3 Substituted 1, 4 Benzodiapin–2–Ones" pg. 457–461

Journal Organic Chemistry 1981, 46, pp. 3945–3949

Heterocyclic Chemistry, 1980, 17(5), 865–8 and Chemical Abstract 94:65639a

Farmaco, Ed. Sci. 1982 37 (5), 343–52 (Ital.) and Chemical Abstract 97:23755a

Chem. Abstracts 87:5923j

## Description

Use of benzodiazepine derivatives for the manufacturing of pharmaceutical compositions for use in antagonizing cholecystokinin and gastrin and new benzodiazepine derivatives.

The present invention concerns the use of benzodiazepine derivatives which have a substituent in the 3-position of the seven-membered ring and pharmaceutically acceptable salts or pharmaceutically acceptable quaternary ammonium salts of said benzodiazepine derivatives for the manufacturing of pharmaceutical compositions for use in antagonizing cholecystokinin in gastrointestinal tissue and for antagonizing gastrin by binding to the corresponding cholecystokinin receptor or gastrin receptor.

The present invention furthermore concerns new benzodiazepine derivatives. The benzodiazepine derivatives which are used according to the present invention comprise new chemical substances as well as also the benzodiazepine derivatives which are already disclosed in the prior art and which were already used as active ingredient of pharmaceutical compositions, however for other purposes than antagonizing cholecystokinin in gastrointestinal tissue and for antagonizing gastrin.

## BACKGROUND OF THE INVENTION

Cholecystokinin (CCK) is a neuropeptide composed of thirtythree aminoacids in its originally isolated form. See: Mutt and Jorpes, Biochem. J. 125 678 (1971). Also occurring in circulation are 39, 12, and 8 amino acid forms. The carboxyl terminal octapeptide (CCK-8) is the minimum fully active sequence.

Gastrin occurs in 34, 17 and 14 amino acid forms in cirulation and is related to CCK by indentity of the C-terminal pentapeptides Gly-Trp-Met-Asp-Phe-$NHR_2$.

Gastrin and CCK exist in both gastrointestinal tissue and the central nervous system (see V. Mutt, Gastrointestinal Hormones, G. B. J. Glass, Ed., Raven Press, N.Y., p. 169 and G. Nisson, ibid, p. 127).

CCK is believed to play an important role in appetite regulation and CCK may be a physiological satiety hormone (see G.P. Smith, Eating and Its Disorders, A.J. Stunkard and E. Stellar, Eds, Raven Press, New York, 1984, p. 67).

Among additional effects of CCK are stimulation of colonic motility, stimulation of gall bladder contraction, stimulation of pancreatic enzyme secretion, and inhibition of gastric emptying. CCK reportedly co-exists with dopamine in certain mid-brain neurons and thus may also play a role in the functioning of dopaminergic systems in the brain, as well as serving as a neurotransmitter in its own right. See: A.J. Prange et al., "Peptides in the Central Nervous System", Ann. Repts. Med. Chem. I7 31, 33 (1982) and references cited therein; J.A. Williams, Biomed. Res. 3 107 (1982); and J.E. Morley, Life Sci. 3O, 479, (1982).

The primary role of gastrin appears to be stimulation of secretion of water and electrolytes from the stomach and it is therefore involved in control of gastric acid secretion.

## DESCRIPTION OF THE PRIOR ART

CCK antagonists are useful in the treatment and prevention of CCK-related disorders of the gastrointestinal, central nervous and appetite regulatory systems of animals, specially humans. CCK antagonists are also useful in potentiating and prolonging opiate mediated analgesia and thus have utility in the treatment of pain [see P.L. Faris et al., Science 226, 1215 (1984)].

Three distinct chemical classes of CCK receptor antagonists have been reported.

One class comprises derivatives of cyclic nucleotides; detailed structure-function studies have demonstrated that of the various members of this class, dibutyryl cyclic GMP is the most potent. See: N. Barlos et al., Am. J. Physiol., 242, G 161 (1982) and P. Robberecht et al., Mol., Pharmacol., 17, 268 (1980).

The second class comprises peptide antagonists which are C-terminal fragments and analogs of CCK. Recent structure-function studies have shown that both shorter C-terminal fragments of CCK (Boc-Met-Asp-Phe-$NH_2$,Met-Asp-Phe-$NH_2$) as well as longer CCK fragments Cbz-Tyr($SO_3H$)-Met-Gly-Trp-Met-Asp-$NH_2$) can function as CCK antagonists. See: R.T. Jensen et al., Biochem. Biophys. Acta., 757, 250 (1983) and M. Spanarkel et al., J.Biol. Chem., 258, 6746 (1983). The latter compound was recently reported to be a partial agonist [see J.M. Howanrd et al., Gastroenterology 86(5) Part 2, 1118 (1984)].

All of these compounds are relatively weak antagonists of CCK ($IC_{50}$: $10^{-4}$ - $10^{-6}$ M; generally, $10^{-4}$ M but down to $10^{-6}$ M in the case of peptides). The peptide antagonists have substantial stability and absorption problems.

Gastrin antagonists are useful in the treatment and prevention of gastrin-related disorders of the gastrointestinal system in humans and animals such as ulcers, Zollinger-Ellison syndrome, central G cell hyperplasia and other conditions in which reduced gastrin activity is of therapeutic value. There are no effective receptor antagonists of the in vivo effects of gastrin. J.S. Morley, Gut Pept. Ulcer Proc., Hiroshima Symp. 2nd, 1983, p. I. Very weak in vitro antagonists such as proglumide and certain peptides have been reported, J. Martinez, J. Med. Chem., 27, 1597 (1984).

In the publication of J. Bradwejn et al., Nature, 312, 363 (1984) three benzodiazepine derivatives were investigated as to their activity of antagonizing the CCK-induced activation of rat hippocampal neurones. There is stated in said publication that of the five molecular forms of cholecystokinin which comprise 39, 33, 13, 8 and 4 amino acid residues respectively which were detected in the central nervous system the corresponding sulphated octapeptide, abbreviated as $CCK_8$ is the most abundant form and said form has a powerful excitatory effect on the neurones. Two of the three tested benzodiazepines correspond to the following formula A

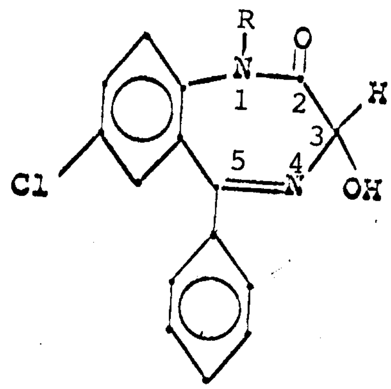

A

and in the corresponding benzodiazepine which is named lorazepam the radical R is hydrogen and in the corresponding compound named flurazepam the radical R is a group of formula

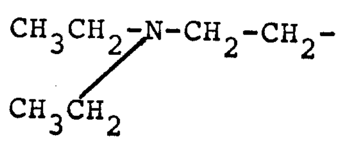

.

In both said compounds the benzene nucleus which is bonded to the carbon atom in the position 5 is substituted in its ortho position with a chlorine atom. The third tested compound which is named diazepam differs from the compounds of formula A in that to the carbon atom in the position 3 of the seven-membered ring there are bonded two hydrogen atoms and no hydroxy group and in said compound the radical R is methyl and the benzene nucleus bonded to the carbon atom in the position 5 is unsubstituted.

Of the three tested compounds lorazepam had the highest antagonizing activity and there is nowhere stated in said publication that the three tested benzodiazepines are also able to antagonize cholecystokinin in the gut.

The benzodiazepine (BZD) structure class has been widely exploited as therapeutic agents, specially as central nervous system (CNS) drugs. These compounds exhibit strong binding to "benzodiazepine receptors" in vitro, but have not been reported to bind to CCK or gastrin receptors. The large majority of reported BZD's do not contain substituents attached to the 3-position of the seven-membered ring. It is well known in the art that 3-substituents result in decreasing CNS activity, specially as these substituents increase in size.

However in the following publications there are described benzodiazepine derivatives which have a substituent in the 3-position.

In the British patent 972 968 there are disclosed benzodiazepine derivatives which correspond to the following formula B

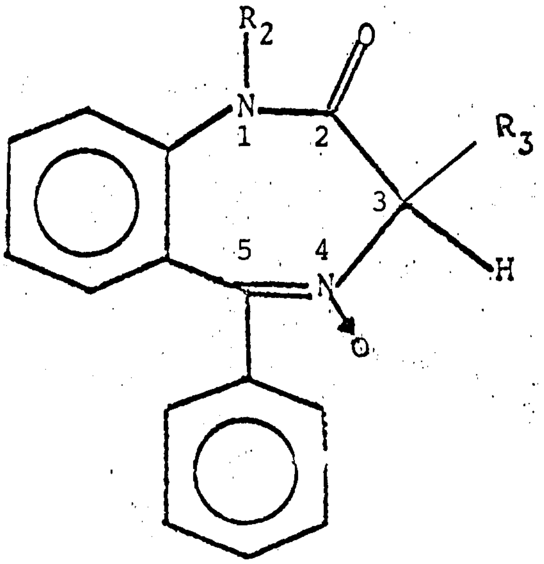

B

and in said compounds of formula B the radical

$R_2$ is hydrogen, alkyl, hydroxyalkyl, aralkyl or alkenyl and the radical

$R_3$ is hydrogen, an alkyl group which is optionally substituted with alkoxy, alkylthio, hydroxy or hydroxyaryl or $R_3$ is an aryl group.

The benzene nucleus which is condensed with the 7 – membered ring of said compounds of formula B can be either monosubstituted with trifluoromethyl or monosubstituted or disubstituted with a substituent selected from a group comprising alkyl, alkoxy, optionally hydroxy substituted alkylthio, nitro, amino, acylated amino, alkylsulphinyl, alkylsulphonyl, hydroxy or halogen and the benzene nucleus which is bonded to the carbon atom in the position 5 is optionally mono – substituted with a substituent selected from the group comprising alkyl, alkoxy, trifluoromethyl or halogen. Specifically disclosed are in examples 50 and 51 of said British patent corresponding compounds in which the radical $R_3$ is a phenyl or a 4 – hydroxybenzyl group, the benzene nucleus which is condensed with the 7 – membered ring is substituted with a chlorine atom in its position 7 and the benzene nucleus which is bonded to the carbon atom in the position 5 is unsubstituted. There is stated in said British patent that the corresponding benzodiazepine derivatives are useful as sedatives, as muscle – relaxant and as anticonvulsants and they may also be used for the relief of tension (see page 1, lines 38 – 41 of said British patent 972 968).

The benzodiazepine compounds disclosed in the British patent 1 173 320 as well correspond to the above stated formula B, except that they are not the corresponding N – oxides, but they have an unsubstituted tertiary nitrogen atom in the position 4 of the 7 – membered ring. In said compounds the radical

$R_2$ is hydrogen or alkyl and the radical

$R_3$ is a phenyl nucleus which is substituted with one or two chloroatoms, one, two or three methoxy groups or with one methylene dioxy group.

In said compounds there has to be furthermore present a chloro substituent in the benzene nucleus which is condensed with the 7 – membered ring and the benzene nucleus which is bonded to the carbon atom in the position 5 of the 7 – memberend ring has to be unsubstituted.

The corresponding compounds are stated to produce a depression of the central nervous system when administered to mice and rats.

In the Swiss patent 581 606 there are described benzodiazepine derivatives which as well correspond to the above stated formula B. In said compounds the radical $R_2$ is a hydrogen atom, an alkyl radical having 1 – 6 carbon atoms or a cyclopropyl group and the radical

$R_3$ is methyl, ethyl, substituted ethyl, propyl, butyl, substituted butyl, benzyl, t – hydroxybenzyl or an indolyl substituted methyl group.

Furthermore the benezene nucleus which is condensed with the 7 – membered ring is optionally substituted with a halo atom, a nitro group or a trifluoromethyl group and the benzene nucleus which is bonded to the carbon atom in the position 5 of the 7 – membered ring is optionally substituted with halogen. Also the compounds disclosed in said Swiss patent are not N – oxides but the nitrogen atom in the position 4 is unsubstituted.

There is explained in column 3 of said Swiss patent that precursors of the corresponding compounds of formula B in which the 7 – membered ring is cleaved between the nitrogen atom in the position 4 and the carbon atom in the position 5 are converted to the corresponding benzodiazepinones after they had been absorbed in the gut and that the tranquilizing and hypnotic activity of the precursors is due to their in vivo cyclization yielding the corresponding benzodiazepinone of the above stated formula B.

In the French patent publication 2 239 464 there are as well described benzodiazepinones which correspond to the above stated formula B except that said compounds are not N – oxides. In said compounds the radical $R_2$ is hydrogen and the radical $R_3$ is an alkyl radical, a indolyl substituted methyl group, a benzyl group or a hydroxy benzyl group. The benzene nucleus which is condensed with the 7 – membered ring is optionally substituted with haline or alkoxy and the benzene nucleus which is bonded to the carbon atom 5 can be optionally substituted with haline. There is explained on page 2, lines 31 – 35 of said publication that the corresponding compounds can be used for the treatment of different psychic illnesses and they for example can be used as tranquilizers or myorelaxants. The corresponding S – forms are far more pharmacologically active than the corresponding R – forms.

In the French patent publication 1 437 376 there are as well described benzodiazepine N – oxides which correspond to the above stated formula B as well as the corresponding compounds in which the nitrogen atom in the position 4 is not an N – oxide but a corresponding tertiary amino nitrogen atom. In the compound disclosed in said French patent publication the radical

$R_2$ is hydrogen or an acyl group derived from a mono – carboxylic acid and the radical

$R_3$ is either a hydrogen atom or an acyloxy group derived from a monocarboxylic acid.

The benzene nucleus which is condensed with the 7 – membered ring can be optionally substituted with 1 or 2 substituents which are selected from the group comprising halo, nitro, haloalkyl and alkylsulphonyl, and instead of the optionally substituted phenyl nucleus which is bonded to the carbon atom in the position 5 of the 7 – membered ring there can be also bonded an aromatic heterocycle like thienyl.

Specifically disclosed are in said French patent publication corresponding compounds in which the acyl and the acyloxy group ($R_2$ and $R_3$) are derived from benzoic acid respectively phenyl acetic acid (see examples 12 and 13).

There is furthermore mentioned in said French patent publication that the corresponding compounds which are acylated have a muscle relaxant activity and can be administered as anticonvulsant respectively some of the compounds furthermore have a tranquilizing effect or a sedative effect.

In the British patent 1 034 872 there are as well disclosed benzodiazepine derivatives which about correspond to the above stated formula B, which however are not N – oxides, but the nitrogen atom in the position 4 of the 7 – membered ring is the nitrogen atom of a corresponding tertiary amino group. In the compounds disclosed in said British patent the radical

$R_2$ is hydrogen or an alkyl group and the radical

$R_3$ is an unsubstituted amino group or a substituted amino group comprising up to 8 carbon atoms, like e.g. an alkyl amino group, a substituted alkyl amino group in which the substituent can be an aryl radical, an amino group, like an alkyl amino group, dialkyl amino group or cyclic amino group or said radical $R_3$ can be a tertiary amino group, like a dialkyl amino group or the nitrogen atom of a heterocyclic structure, like piperidino, piperazino, morpholino or pyrrolidino (see page 1, lines 36 – 5O of said British patent publication).

Furthermore in the compounds described in said British patent the benzene nucleus which is con – densed with the 7 – membered aromatic ring can be optionally substituted with a haline atom and to the carbon atom in the position 5 of the 7 – membered ring there can be bonded either an optionally substituted phenyl radical or a heteroaromatic radical like thienyl.

The compounds disclosed in said British patent are stated to have a muscle relaxant effect, a sedative effect or an anticonvulsant effect.

In the British patent 1 309 947 there are described benzodiazepine derivatives which about correspond to the above stated formula B, which compounds however are not N – oxides, but the nitrogen atom in the position 4 of the 7 – membered ring is a corresponding tertiary amino group. In said compounds furthermore the radical

$R_2$ is hydrogen, halo, alkyl, substituted alkyl wherein the substituents are cyano, halo, cycloalkyl, alkoxy, alkylthio, alkenyloxy, carbamoyl, alkylcarbamoyl, acyloxy, alkylcarbonyl, alkoxycarbonyl, phenyl, substituted phenyl, an amino group or an acid amide group and the radical $R_2$ furthermore can have the meaning of an alkenyl, an alkynyl or a phenyl group and the radical

$R_3$ is hydrogen, alkyl which is optionally substituted with alkoxy or alkylthio, phenyl which is optionally substituted by halo or alkoxy, benzyl or hydroxy benzyl.

Furthermore in said compounds the benzene nucleus which is condensed with the 7-membered ring can be optionally substituted with 1 or 2 substituents and to the carbon atom in the position 5 of the heterocyclic ring there can be bonded instead of an optionally substituted phenyl nucleus also an alkyl group, a phenyl substituted alkyl group, a cycloalkyl group or a heterocyclic group. Furthermore the benzene nucleus which is condensed with the 7-membered ring of the benzodiazepine structure is optionally substituted with 1 or 2 substituents.

The benzodiazepine derivatives described in said British patent are stated to have the activity of tranquilizers, muscle relaxants, antispasmodics, anticonvulsants and hypnotics (see page 2, right column, lines 77 - 83).

In the French patent publication 2 220 269 there are described benzodiazepine derivatives which are closely related to the compounds of formula B stated before, which compounds however are not N-oxides, but the nitrogen atom in the position 4 of the 7-membered ring is the nitrogen atom of a tertiary amino group. In said compound the radical

$R_2$ is an alkyl group which is optionally substituted with halo or dialkyl amino and the radical

$R_3$ is a group of formula

$-CH_2-OR_4$, wherein $R_4$ is hydrogen or an aliphatic or aromatic acyl group.

Furthermore in the compounds described in said French patent publication the benzene nucleus which is condensed with the 7-membered ring has to be substituted with one halo atom or nitro group and the phenyl nucleus which is bonded to the carbon atom in the position 5 of the 7-membered ring is optionally substituted with one halo atom.

The compounds described in said French patent are stated to have a sedatic, a tranquilizing, a muscle relaxant, an anticonvulsant and an anxiolitic activity.

In the British patent 1 063 891 there are described benzodiazepine derivatives which are closely related to the compounds having the above stated formula B, which compounds however are not N-oxides, but corresponding compounds in which the nitrogen atom in the position 4 of the 7-membered ring is the nitrogen atom of a tertiary amino group. The radical

$R_2$ of said compound is a hydrogen atom, a methyl or ethyl group and the radical

$R_3$ is a hydrogen atom, an alkyl, phenyl or a p-hydroxy phenyl group.

The phenyl radical which is bonded to the carbon atom in the position 5 of the 7-membered ring is an unsubstituted phenyl radical and the phenyl nucleus which is condensed with the 7-membered ring has to be substituted with one halo atom.

The compounds described in said patent are stated to be useful as chemotherapeutics, specially for treating nervous disorders.

In the U.S. patent 4 045 569 there are described benzodiazepine derivatives which have a tranquilizing activity and said compounds correspond to formula B stated before, except that they are not N-oxides, i.e. the nitrogen atom in the position 4 of the 7-membered ring is the nitrogen atom of a tertiary amino group. In said compounds furthermore the radical

$R_2$ is an alkyl group and the radical

$R_3$ is an alkyl group which is optionally substituted with a hydroxy group, a benzyl group which is optionally hydroxy substituted, an indolyl substituted methyl group or a phenyl group. The phenyl nucleus which is bonded to the carbon atom in the position 5 of the 7-membered ring is an unsubstituted phenyl group and the phenyl group which is condensed with the 7-membered ring is optionally substituted with one substituent selected from halo, nitro and trifluoro methyl. The corresponding compounds have to have an S-configuration, i.e. a hydrogen atom which is bonded to the carbon atom in the position 3 of the 7-membered ring lies above the plane of said 7-membered ring, while the radical $R_3$ which is bonded to said carbon atom in the position 3 lies below the plane of the 7-membered ring.

To sum up there are accordingly described in the prior art several benzodiazepine derivatives in which the carbon atom in the position 2 of the 7-membered ring is a carbonyl group. Some of said compounds have a substituent in the position 3 of the 7-membered ring and the corresponding compounds usually have a muscle relaxant activity, a sedative activity, a tranquilizing activity and some of them are hypnotics. Nowhere in the prior art, except the citation Nature, 312, pages 363 and 364, there is reported that the benzodiazepine 2-ones described in the prior art had the ability of antagonizing cholecystokinin or of antagonizing gastrin.

Furthermore in the citation Nature there are only described three specific benzodiazepine 2-ones, which are closely related to each other and which compounds are able to antagonize the sulphated form of the cholecystokinin octapeptide in the brain.

It was the aim of the present invention to prepare pharmaceutical preparations which are able to antagonize cholecystokinin not only in the brain but also in the gut and which furthermore are also able to antagonize gastrin, by using as active ingredient of said pharmaceutical composition benzodiazepine derivatives.

It was now surprisingly found out that several benzodiazepine derivatives described in the prior art as well as new benzodiazepine derivatives have the desired activity.

DESCRIPTION OF THE INVENTION

One object of the present invention is the use of compounds of formula I

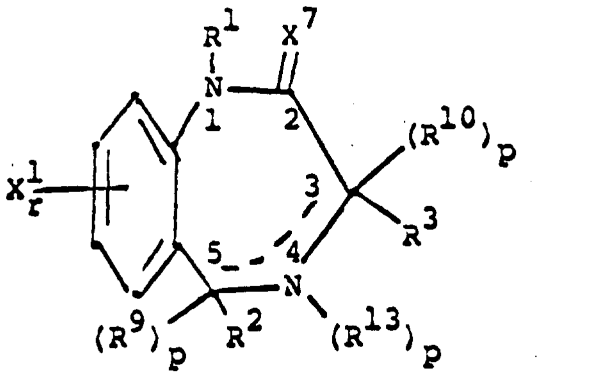

I

or a pharmaceutically acceptable salt or a pharmaceutically acceptable quaternary ammonium salt of said compound of formula I

in which compounds of formula I

$R^1$ is hydrogen, alkyl having 1 − 5 carbon atoms, alkenyl having up to 5 carbon atoms, alkynyl having up to 5 carbon atoms, or a group having the formulae

$-(CH_2)_mCOOR^6$, $-(CH_2)_n-C_3-C_5-$cycloalkyl, $-(CH_2)_mNR^4R^5$, $-(CH_2)_mCONR^4R^5$, $-(CH_2)_mCN$, or $-(CH_2)_nCX^{10}_3$ ;

wherein

| | |
|---|---|
| $R^4$ and $R^5$ | are independently hydrogen, alkyl having 1 − 4 carbon atoms or cycloalkyl having 3 − 5 carbon atoms |
| $R^6$ | is hydrogen, alkyl having 1 − 4 carbon atoms, cycloalkyl having 3 − 5 carbon atoms, unsubstituted phenyl, substituted phenyl, unsubstituted phenyl alkyl or substituted phenyl alkyl in which groups the substituents of the phenyl are 1 or 2 substituents selected from halo, alkyl having 1 − 4 carbon atoms, alkoxy having 1 − 4 carbon atoms, nitro or trifluoro methyl and wherein the alkyl moiety of the corresponding substituted or unsubstituted phenyl alkyl group compises 1 − 4 carbon atoms, |
| $X^{10}$ | is fluoro, chloro or bromo, |
| m | is 1 − 4 and |
| n | is O − 4 ; |
| $R^2$ | is hydrogen, alkyl having 1 − 4 carbon atoms, unsubstituted phenyl, substituted phenyl having 1 or 2 substituents selected from halo, alkyl having 1 − 4 carbon atoms, alkoxy having 1 − 4 carbon atoms, alkylthio having 1 − 4 carbon atoms, carboxysubstituted alkyl having 1 − 4 carbon atoms in the alkyl group, carboxyl, nitro, trifluoromethyl and hydroxy, or the radical $R^2$ is a group having the following formulae |

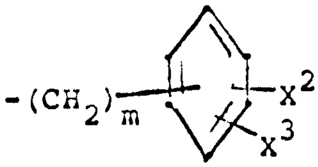

,

$-(CH_2)_mSCH_3$, $-(CH_2)_mSOCH_3$, $-(CH_2)_mSO_2CH_3$, or $-(CH_2)_mCOOR^6$;

wherein

$R^6$ and m are as defined with regard to the radical $R^1$

$X^2$ and $X^3$ are independently hydrogen, hydroxy, nitro, halo, alkyl having 1 − 4 carbon atoms, alkylthio having 1 − 4 carbon atoms or alkoxy having 1 − 4 carbon atoms,

$R^3$ is a group having the formulae

$-(CH_2)_nR^7$,

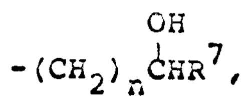

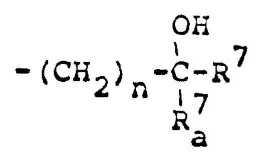

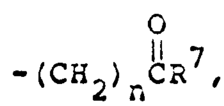

$-(CH_2)_nNR^{18}(CH_2)_qR^7$,

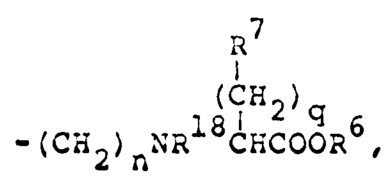

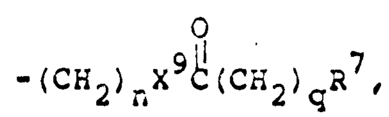

$-NH(CH_2)_{2-3}NHR^7$, $-NH(CH_2)_{2-3}NHCOR^7$,

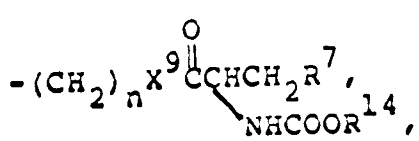

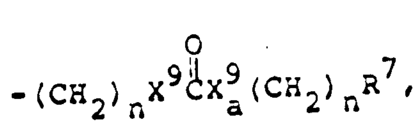

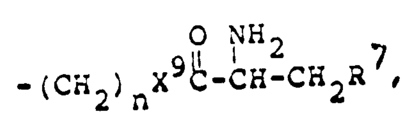

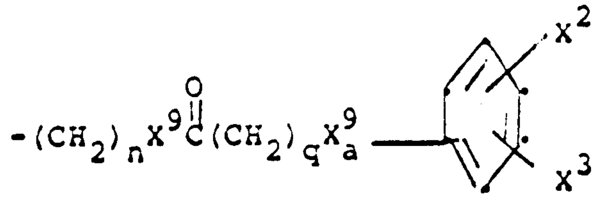

or $-(CH)_2)_nNR^{18}SO_2(CH_2)_qR^7$

wherein

$R^7$ and $R^7_a$ are independently unsubstituted phenyl or substituted phenyl having 1 or 2 substituents which are selected from halo, nitro, hydroxy, alkyl having 1 − 4 carbon atoms, groups having the formulae $CF^3$, CN, $SCF_3$, $C\equiv CH$, $CH_2SCF_3$,

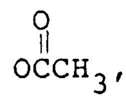

$OCHF_2$, SH, SPh, $PO_3H$, or $-NR^4R^5$,
alkoxy having 1 − 4 carbon atoms or alkylthio having 1 − 4 carbon atoms or the radicals

$R^7$ and $R^7_a$ are alpha naphthyl or beta naphthyl or a group having the formulae

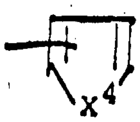 , 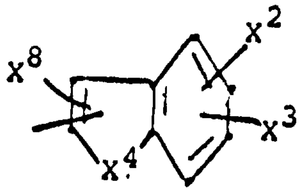 , 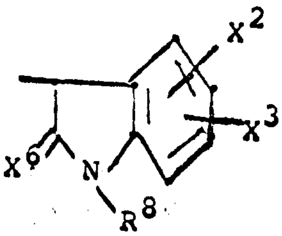

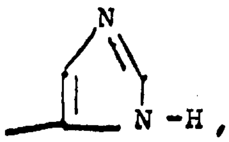 , 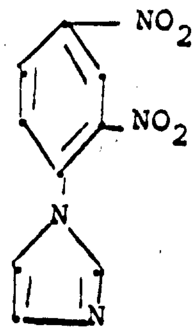 ,

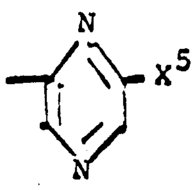 , 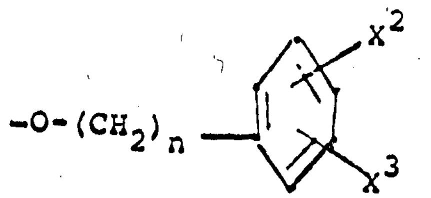 ,

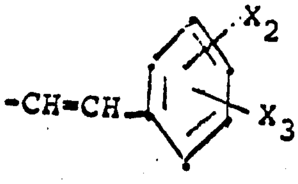 , 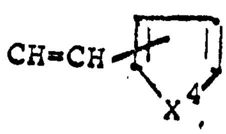

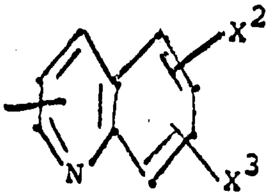 , 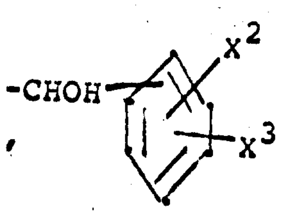 ,

or

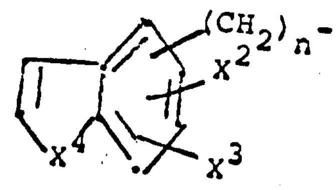

wherein

$R^8$ is hydrogen, alkyl having 1 – 4 carbon atoms, cycloalkyl having 3 – 5 carbon

atoms which is bonded either directly or through an alkylene chain comprising 1 – 4 $-CH_2-$ groups or $R^8$ is a group having the formulae $-(CH_2)_mCONH_2$, $-(CH_2)_mCOOR^6$, $-(CH_2)_mNR^4R^5$,

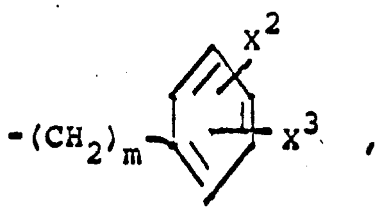

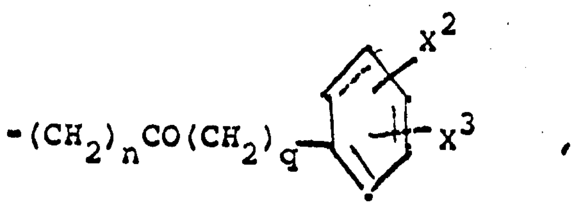

or

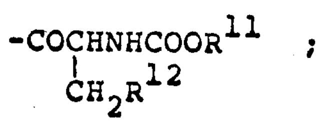

wherein

| | |
|---|---|
| $X^2$ and $X^3$ | are as defined with regard to radical $R^2$, |
| $X^4$ | is S, O, $CH_2$, or $NR^8$, wherein $R^8$ is as defined above, |
| $X^5$ | is hydrogen, halo, nitro or a group of formulae $CF_3$, CN, $-COOR^6$, wherein $R^6$ is as defined above, |
| $X^5$ | is O or HH; |
| $X^5$ | is hydrogen or alkyl having 1 – 4 carbon atoms, |
| $X^5$ and $X^9_a$ | are independently groups of formulae $-O-$ or $-NR^{18}-$ wherein |
| $R^{18}$ | is hydrogen, alkyl having 1 – 4 carbon atoms, formyl, acetyl, propionyl or butyryl, |
| $R^{11}$ and $R^{12}$ | are independently alkyl having 1 – 4 carbon atoms or cycloalkyl having 3 – 5 carbon atoms, |
| $R^{14}$ | is alkyl having 1 – 4 carbon atoms or phenyl alkyl in which the alkyl group has 1 – 4 carbon atoms, |
| $R^9$ and $R^{10}$ | are independently hydrogen, hydroxy or methyl, |
| $R^{13}$ | is hydrogen, alkyl having 1 – 4 carbon atoms, cycloalkyl having 3 – 5 carbon atoms, $-O$, formyl, acetyl, propionyl or butyryl, |
| $X^1$ | is hydrogen, halo, alkyl having 1 – 4 carbon atoms, alkylthio having 1 – 4 carbon atoms, alkoxy having 1 – 4 carbon atoms or a group having the formulae $-NO_2$, $CF_3$, CN, OH, $-(CH_2)_nCOCR^6$, or $-NR^4R^5$; wherein |
| $R^4$, $R^5$, $R^6$ and n | are as defined in connection with the radical $R^1$, |
| r | is 1 or 2, |
| $X^7$ | is O, S, HH, or $NR^{15}$ wherein |
| $R^{15}$ | is hydrogen, alkyl having 1 – 4 carbon atoms or a group of formula |

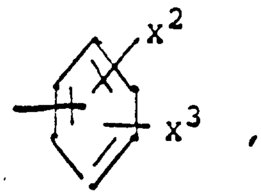

or $-NR^{16}R^{17}$;
wherein

$R^{16}$ and $R^{17}$ are independently hydrogen or a group of formula

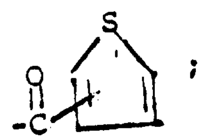

however with the provision that

$X^7$ can be $NR^{15}$ only when $R^1$ is not H;

q is O – 4

--- indicates a single bond or a double bond,

p is O when its adjacent --- is a double bond and is I, when its adjacent --- is a single bond, except that when $R^{13}$ is oxygen and p is I and --- is a double bond and

with the provision that if

$R^3$ is a group of formula $-(CH_2)_nNH(CH_2)_qR^7$,
wherein

q is O or I, or
is a group of formula

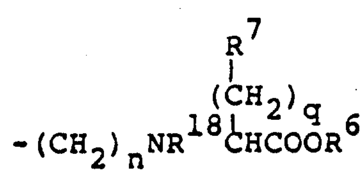

wherein q is 0, then $R^7$ has to have another meaning than that of a group of formula

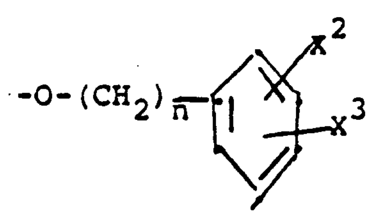

for the manufacturing of pharmaceutical compositions for use in antagonizing cholecystokinin in gastrointestinal tissue and for antagonizing gastrin by binding to the corresponding cholecystokinin receptor or gastrin receptor.

According to a preferred embodiment of the invention there are used compounds of formula I or pharmaceutically acceptable salts or pharmaceutically acceptable quaternary ammonium salts of the compounds of formula I for the manufacturing of the corresponding pharmaceutical compositions the pharmaceutically active ingredient in which compounds of formula I

$R^1$ is hydrogen, alkyl having 1 – 5 carbon atoms

or
a group of formula
$-(CH_2)_mCOOR^6$
wherein

$R^6$ and m are as defined in formula I,

$R^2$ is a group of formula
$-(CH_2)_mCOOR^6$
wherein

$R^6$ and m are as defined above or $R^2$ is unsubstituted phenyl or substituted phenyl having 1 or 2 substituents selected from the group comprising halo, alkyl having 1 – 4 carbon atoms, alkoxy having 1 – 4 carbon atoms, carboxy substituted alkyl having 1 – 4 carbon atoms in the alkyl group, carboxyl, nitro, trifluoro methyl and hydroxy,

$R^3$ is a group having the formulae
$-(CH_2)_nR^7$,

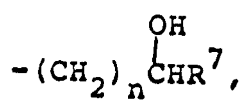

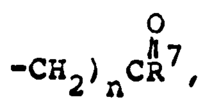

$-(CH_2)_nNH(CH_2)_qR^7$,

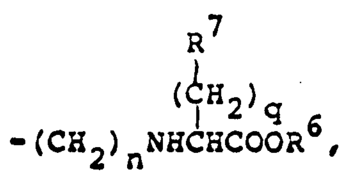

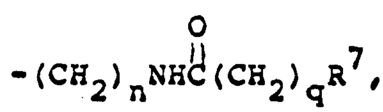

$-NH(CH_2)_{2-3}NHR^7$,

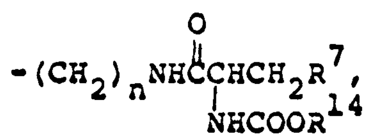

$-NH(CH_2)_{2-3}NHCOR^7$, or

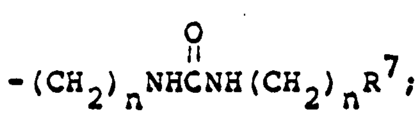

wherein

$R^7$ is unsubstituted phenyl or substituted phenyl having 1 or 2 substituents which are selected from halo, nitro, hydroxy, alkyl having 1 – 4 carbon atoms, alkoxy having 1 – 4 carbon atoms, alkylthio having 1 – 4 carbon atoms, trifluoro methyl groups or groups having the formulae
CN, $-C\equiv CH$, $SCF_3$,

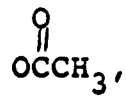

$OCHF_2$, SPh or $-NR^4R^5$

wherein

$R^4$ and $R^5$ are independently hydrogen or alkyl having 1 − 4 carbon atoms, or the radical

$R^7$ is alpha naphthyl or beta naphthyl or a group having the formulae

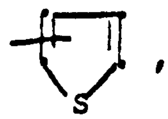 ,

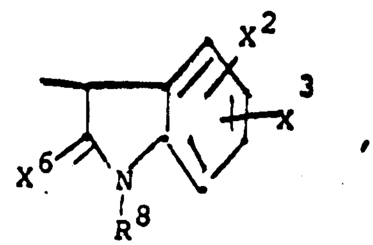 ,

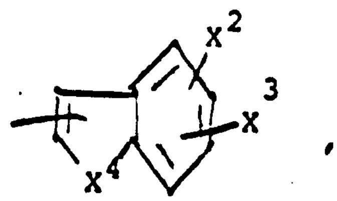 ,

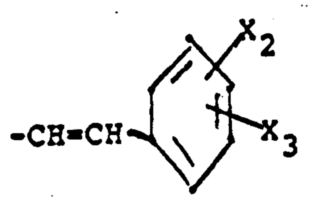 , or 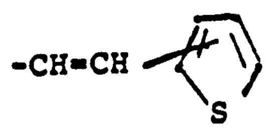

wherein

$R^8$ is hydrogen, alkyl having 1 − 4 carbon atoms or a group of formula

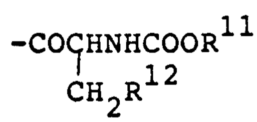

wherein

$R^{11}$ and $R^{12}$ are independently alkyl having 1 − 4 carbon atoms,

$X^2$ and $X^3$ are independently hydrogen, hydroxy, nitro, halo, alkylthio having 1 − 4 carbon atoms, alkyl having 1 − 4 carbon atoms or alkoxy having 1 − 4 carbon atoms,

$X^4$ is S, O, or $NR^8$ wherein $R^8$ is as defined above,

$X^6$ is O or HH;

$R^{14}$ is alkyl having 1 − 4 carbon atoms and

q is O − 4,

$R^9$ and $R^{10}$ are independently hydrogen, hydroxy or methyl,

$R^{13}$ is hydrogen, alkyl having 1 − 4 carbon atoms, −O, formyl, acetyl, propionyl or butyryl,

$X^1$ is hydrogen, halo, alkyl having 1 − 4 carbon atoms, alkylthio having 1 − 4 carbon atoms, alkoxy having 1 − 4 carbon atoms or a group having the formulae $-NO_2$, $CF_3$, CN, OH, $-(CH_2)_nCOOR^6$, or $-NR^4R^5$; wherein

$R^6$ is as defined in connection with radical $R^1$ and $R^4$ and $R^5$ are as defined in connection with the radical $R^3$,

| | |
|---|---|
| $X^7$ | is O |
| m | is 1 − 4 |
| n | is O − 4 |
| − − − | indicates a single bond or a double bond, |
| p | is O when the adjacent − − − is a double bond and is I, when its adjacent − − − is a single bond, except that when $R^{13}$ is oxygen and p is I and − − − − is a double bond and |
| r | is I or 2. |

Still more preferred it is to use as pharmaceutically active ingredient a compound of formula I or a pharmaceutically acceptable salt or a pharmaceutically acceptable quaternary ammonium salt of said compound of formula I in which

| | |
|---|---|
| $R^1$ | is hydrogen, methyl, ethyl, carboxymethyl, or ethylcarboxymethyl; |
| $R^2$ | is a group of formula $-(CH_2)_{1-2}COOR^6$; wherein |
| $R^6$ | is hydrogen or alkyl having 1 − 4 carbon atoms or |
| $R^2$ | is unsubstituted phenyl or substituted phenyl comprising 1 or 2 substituents which are halo or carboxyl, |
| $R^3$ | is a group having the formulae |

$-(CH_2)_nR^7$,

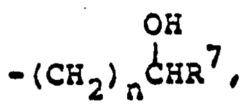

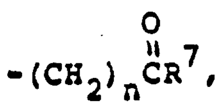

$-(CH_2)_nNH(CH_2)_qR^7$,

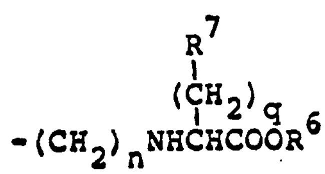,

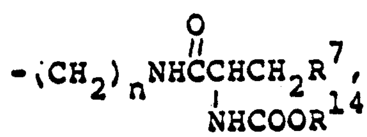

or

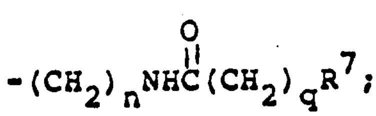

wherein

| | |
|---|---|
| $R^7$ | is alpha naphthyl, beta naphthyl or substituted phenyl having 1 or 2 substituents which are selected from halo, trifluoromethyl and alkyl having 1 − 4 carbon atoms or |
| $R^7$ | is a group having the formulae |

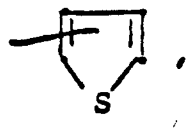

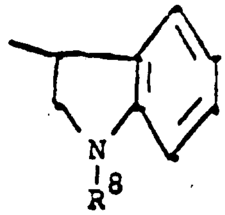
,
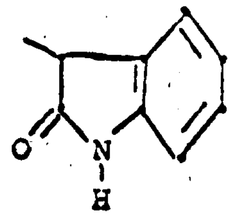
,

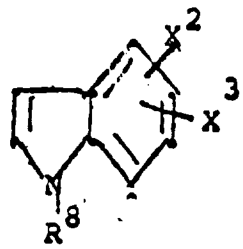

or

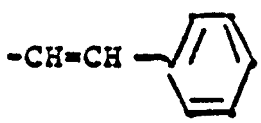

wherein

| | |
|---|---|
| $R^8$ | is hydrogen, methyl or ethyl, |
| $X^2$ and $X^3$ | are independently hydrogen, hydroxy, nitro or halo, |
| $R^{14}$ | is t-butyl; |
| n | is O - 4; |
| q | is O - 4; and |
| $R^6$ | is as defined in connection with the radical $R^2$, |
| $R^9$ and $R^{10}$ | are independently hydrogen, hydroxy or methyl, |
| $R^{13}$ | is hydrogen, methyl or formyl, |
| $X^1$ | is hydrogen, halo, or a group having the formulae $-NO_2$, $CF_3$, CN or OH |
| $X^7$ | is O; |
| - - - | indicates a single bond or a double bond, |
| p | is O when the adjacent - - - is a double bond and is I when its adjacent - - - is a single bond, and |
| r | is I or 2. |

The most preferred class of pharmaceutically active ingredients of the above stated preferred group corresponding to formula I are those compounds of formula I in which

| | |
|---|---|
| $R^1$ | is hydrogen, methyl or carboxymethyl, |
| $R^2$ | is phenyl, o-fluorophenyl, p-fluorophenyl, o-chlorophenyl, p-chlorophenyl, o-carboxyphenyl, 2,4-dichlorophenyl, 2,6-difluorphenyl, $-CH_2COOEt$, $-CH_2COO-t-Bu$, $-CH_2CH_2COOEt$, or $-CH_2CH_2COO-t-Bu$; |
| $R^3$ | is $-(CH_2)_{1-2}R^7$, |

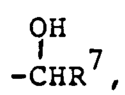

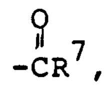

$-(CH_2)_{0-1}NH(CH_2)_{1-2}R^7$,

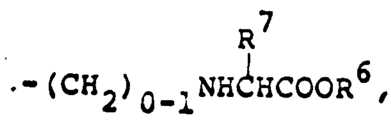 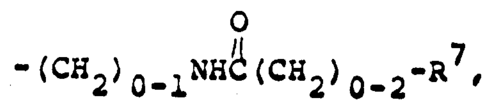

or

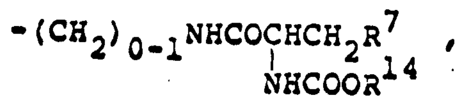

$R^7$ is alpha naphthyl, beta naphthyl, monohalophenyl or dihalophenyl or a group having the formulae

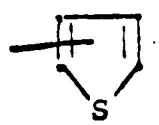

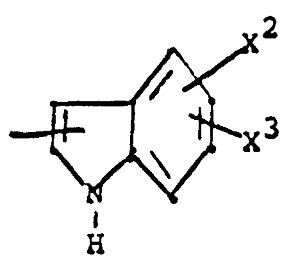

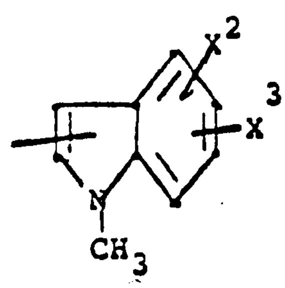

wherein

$X^2$ and $X^3$ are independently hydrogen, hydroxy, fluoro or chloro,
$R^6$ is hydrogen, methyl or ethyl,
$R^{14}$ is t-butyl,
$R^9$ and $R^{10}$ are independently hydrogen or hydroxy,
$R^{13}$ is hydrogen,
$X^1$ is hydrogen, 7-chloro, 7-fluoro, or 7-nitro;
$X^7$ is oxygen,

- - -  is a single bond or a double bond,
p  is O when its adjacent - - - is a double bond and is I, when its adjacent - - - is a single bond and
r  is I.

As already outlined before the active ingredients of formula I for use in the invention comprise compounds which are already described in the prior art, however not as antagonists of cholecystokinin and as antagonists of gastrin, as well as compounds which are new. It furthermore was surprisingly found out that the compounds used in the inventive process have the ability of binding strongly to CCK receptors, however only weakyl to benzodiazepine receptors, specially with increasing size of the substituents of the compounds of formula I.

As it can be seen from the above stated definition of the preferred compounds of formula I the stereo-chemistry of said compounds in the position 3 is R, which accordingly corresponds to a D-amino acid like D-tryptophan. Contrary to this the preferred stereo-chemistry of the prior art benzodiazepines in the position 3 is S, which accordingly would correspond to a L-amino acid, such as L-tryptophan.

In the compounds of formula I corresponding substituents which occur more than once in any structure are intended to be independent of the corresponding definition elsewhere in the structure. This for instance is true for the different substituents as well as the symbols m, n and p. With the ring fragment

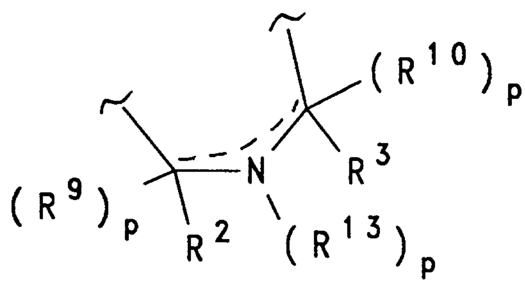

since each p is independently 1 or 0, represents the three structures

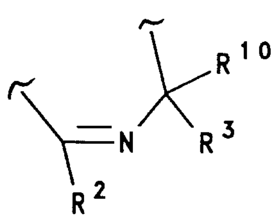

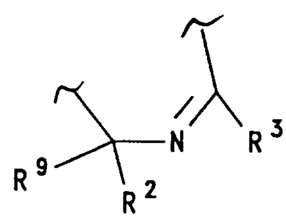

and

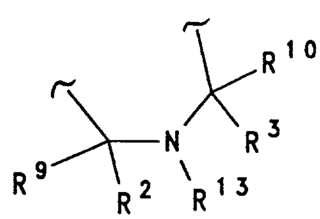

when $R^{13}$ is not O.

The halo substituents of the compounds of formula I are fluoro, chloro, bromo or iodo.

A further object of the present invention are new benzodiazepine derivatives which correspond to the following formula Ia

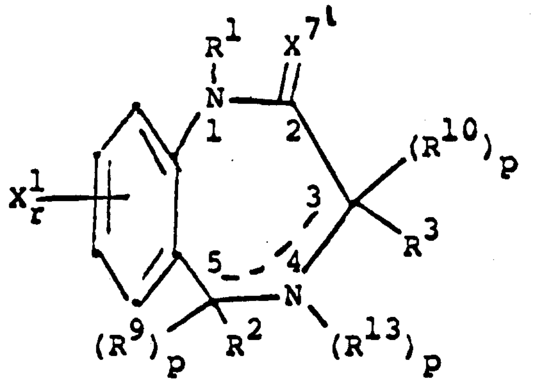

Ia

wherein

$X^{7'}$ is S, HH or $NR^{15}$ and wherein the remaining substituents and symbols have the same meaning as in formula I or salts or quaternary ammonium salts of said new compounds of formula Ia.

The pharmaceutically acceptable salts of the compounds of formula I used for the preparation of the pharmaceutical compositions are e.g. corresponding salts of the compounds of formula I with non-toxic inorganic acids or non-toxic organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic and isethionic.

The pharmaceutically acceptable salts can be synthesized from the compounds of Formula I which contain a basic or acidic moiety by conventional chemical methods. Generally, the salts are prepared by reacting the free base or acid with stoichiometric amounts or with an excess of the desired salt forming inorganic or organic acid or base in a suitable solvent or various combinations of solvents.

The pharmaceutically acceptable salts of the acid of Formula I are also readily prepared by conventional procedures such as treating an acid of Formula I with an appropriate amount of a base, such as an alkali or alkaline earth metal hydroxide e.g. sodium, potassium, lithium, calcium, or magnesium, or an organic base such as an amine, e.g., dibenzylethylenediamine, trimethylamine, piperidine, pyrrolidine and benzylamine, or a quaternary ammonium hydroxide such as tetramethylammonium hydroxide.

The pharmaceutical preparations of compounds of Formula I. can be used for the treatment and the prevention of disorders of the gastrointestinal, and appetite regulatory systems of mammals, especially of man. Specifically, they are useful in treatment and prevention of disorders of gastric acid secretion, gastrointestinal motility, pancreatic secretions, and dopaminergic functions. The compositions which contain compounds of Formula I are especially useful in the prevention and treatment of irritable bowel syndrome, ulcers, excess pancreatic or gastric secretion, acute pancreatitis and motility disorders.

The pharmaceutical compositions contain the compounds of formula I or salts thereof either alone, or preferably, in combination with pharmaceutically acceptable carriers or diluents, according to standard pharmaceutical practice. The compositions can be administered orally or parenterally. Parenteral administration includes intravenous, intramuscular, intraperitoneal, subcutaneous and topical administration.

For oral use of an antagonist of CCK or gastrin the compositions can be administered, for example, in the form of tablets or capsules, or as an aqueous solution or suspension. In the case of tablets for oral use, carriers which are commonly used include lactose and corn starch, and lubricating agents, such as magnesium stearate, are commonly added. For oral administration in capsule form, useful diluents are lactose and dried corn starch. When aqueous suspensions are required for oral use, the active ingredient is combined with emulsifying and suspending agents. If desired, certain sweetening and/or flavoring agents can be added. For intramuscular, intraperitoneal, subcutaneous and intravenous use, sterile solutions of the active ingredient are usually prepared, and the pH of the solutions should be suitably adjusted and buffered. For intravenous use, the total concentration of solutes should be controlled to render the preparation isotonic.

When a composition containing a compound of Formula I or a salt thereof is used as an antagonist of CCK or gastrin in a human subject, the daily dosage will normally be determined by the prescribing physician. Moreover, the dosage will vary according to the age, weight, and response of the individual patient, as well as the severity of the patient's symptoms. However, in most instances, an effective daily

dosage will be in the range from about 0.05 mg to about 50 mg/kg and preferably 0.5 mg to about 20 mg/kg in a single or divided doses. On the other hand, it may be necessary to use dosages outside these limits in some cases.

The compounds of Formula I are prepared according to the following schemes.

REACTION SCHEME I

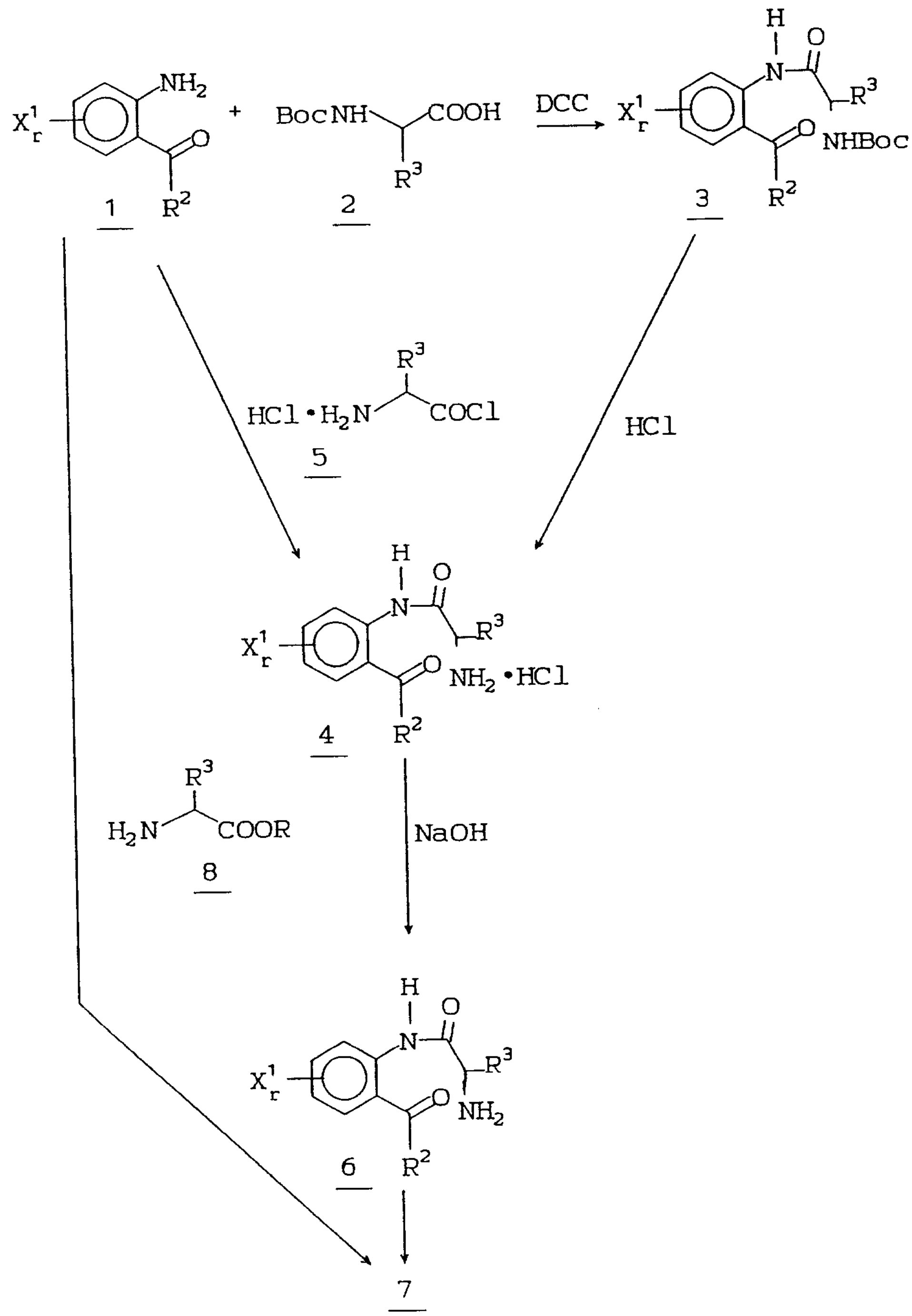

REACTION SCHEME I (Cont'd)

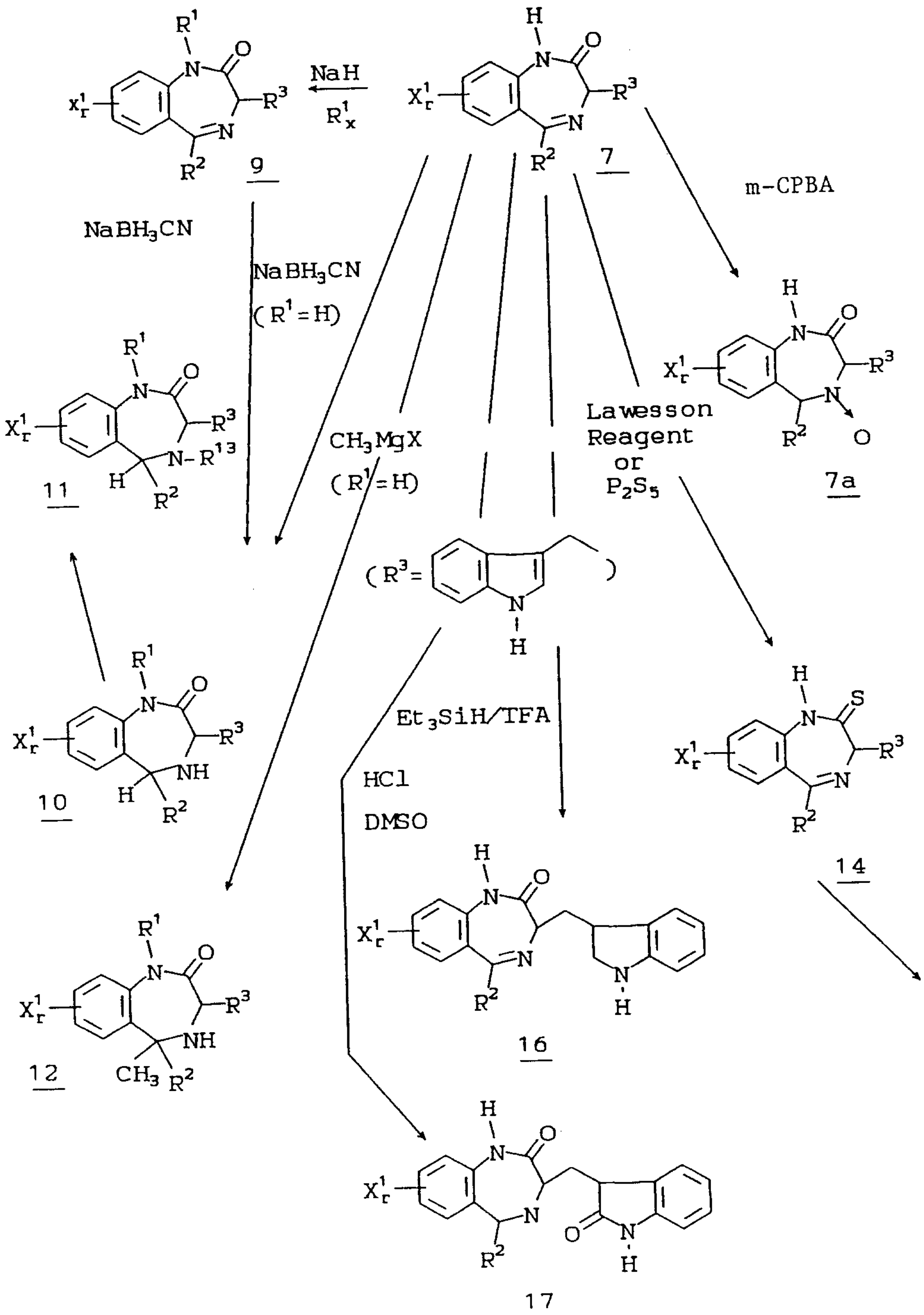

REACTION SCHEME I (Cont'd)

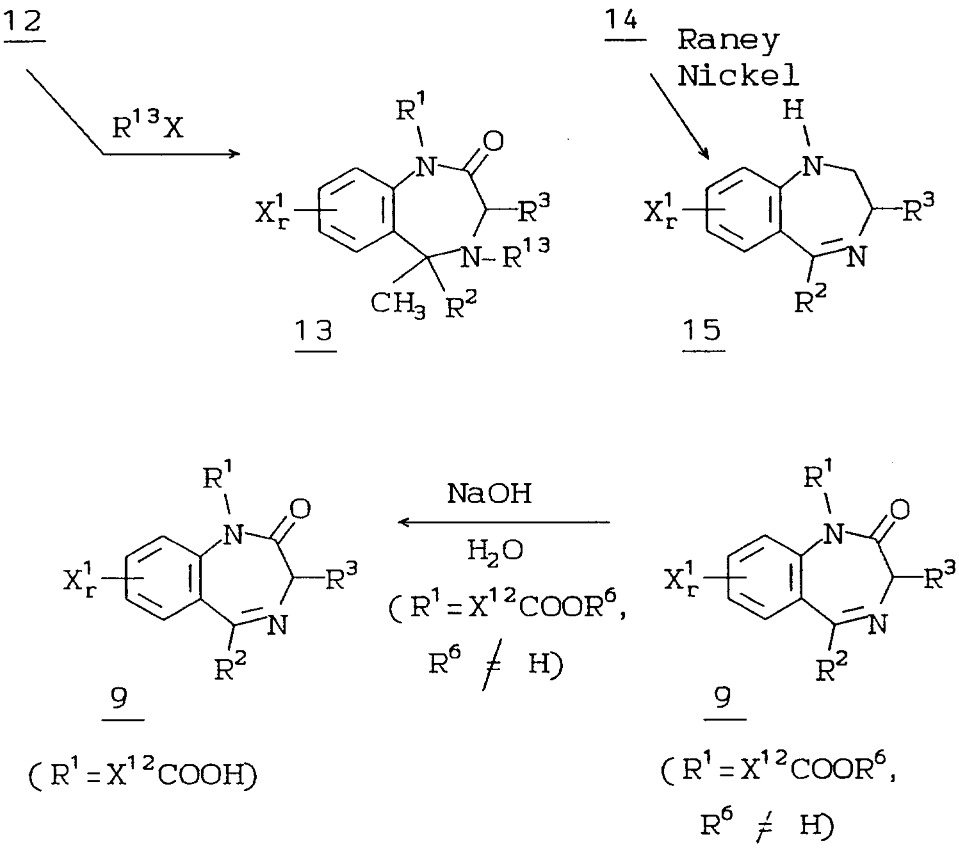

REACTION SCHEME II

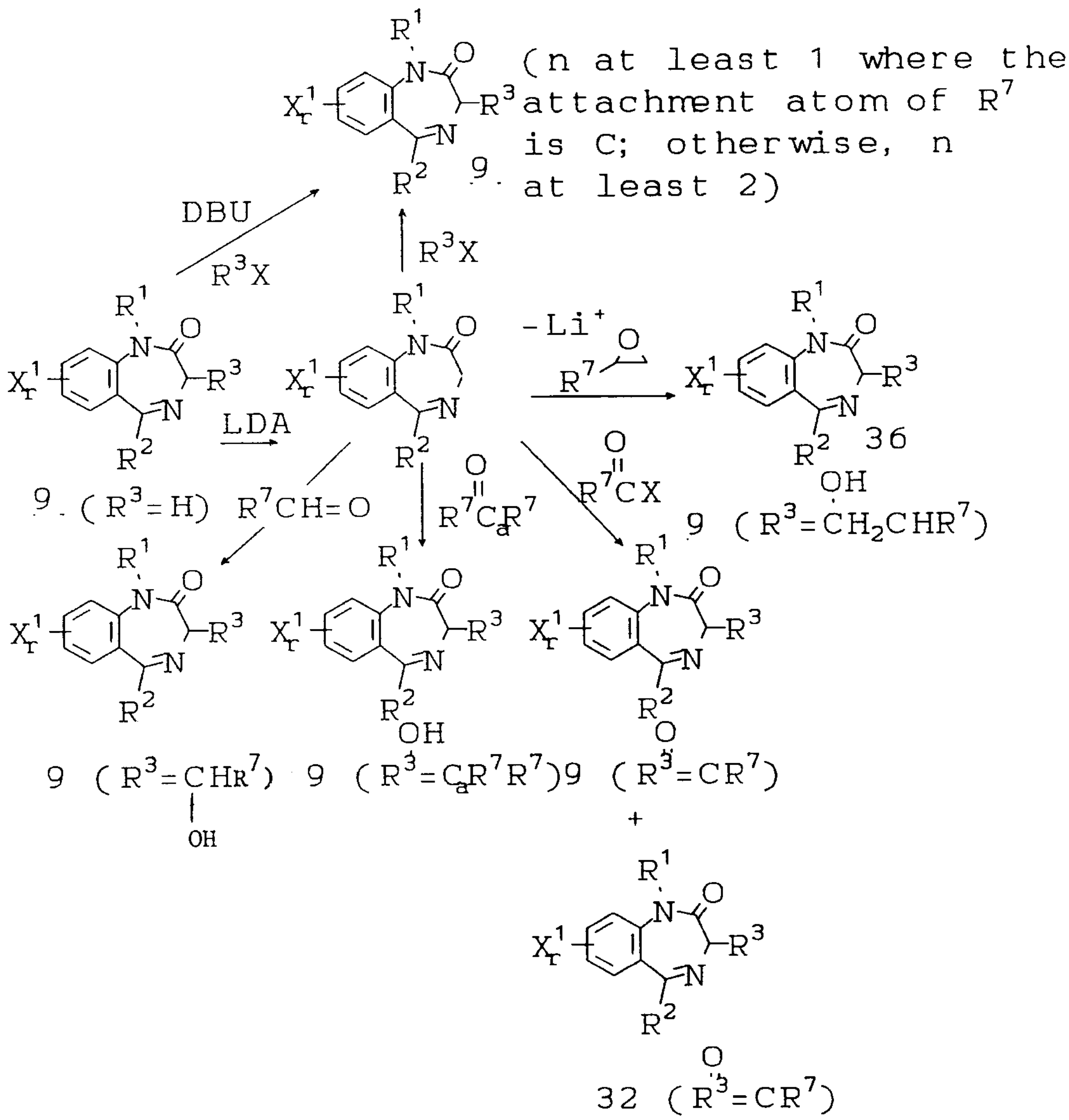

REACTION SCHEME II (Cont'd)

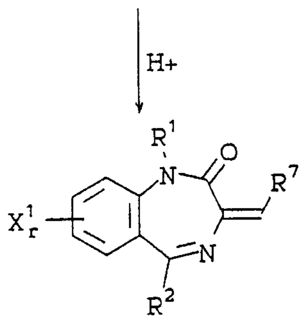


18

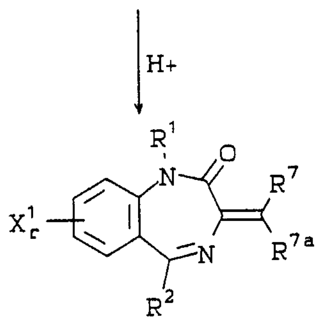


19

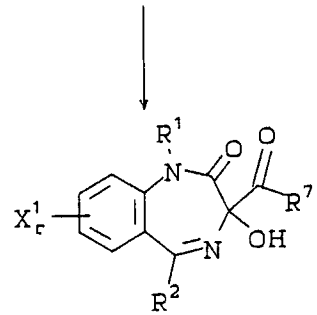

20

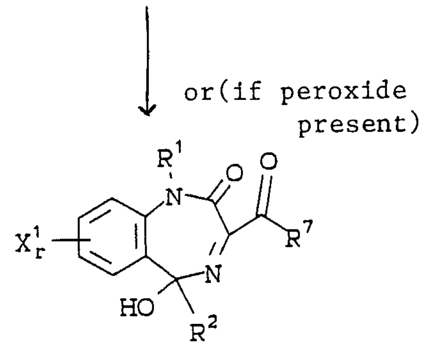


21

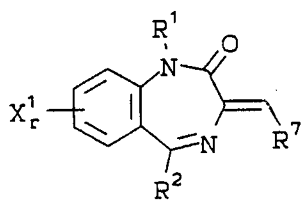

22

REACTION SCHEME III

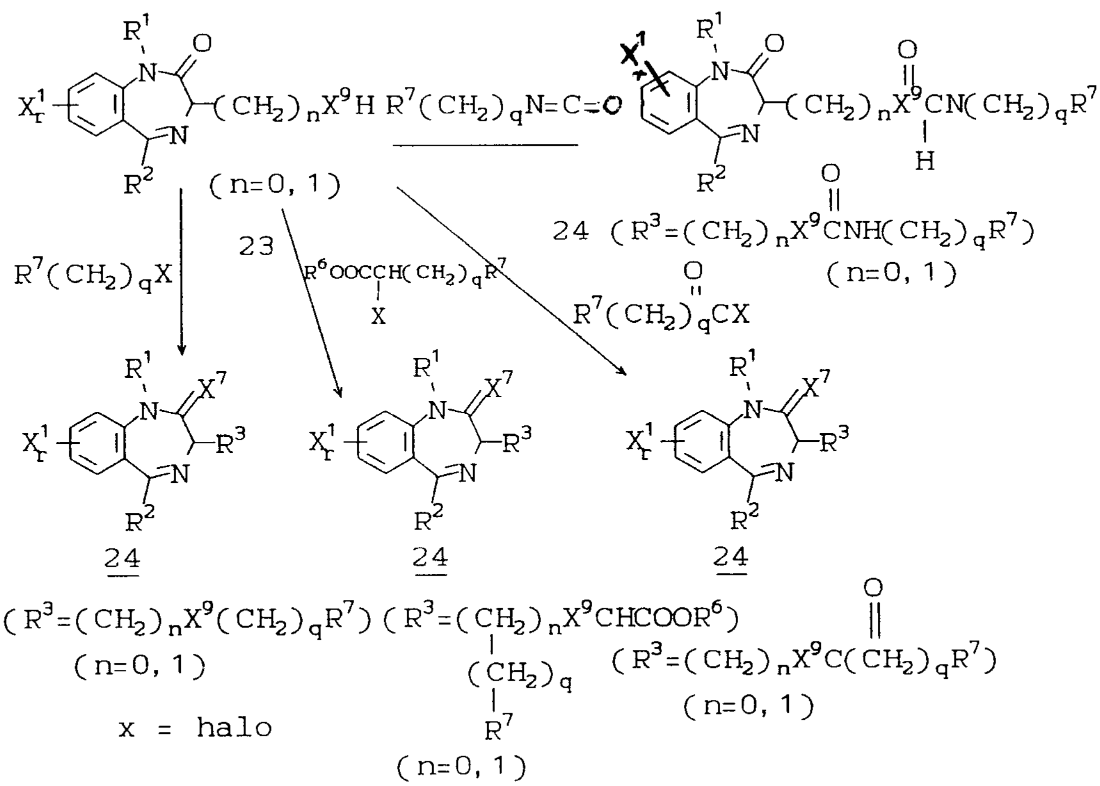


23 (n=0, 1)

24 ($R^3=(CH_2)_nX^9C(O)NH(CH_2)_qR^7$) (n=0, 1)

24 ($R^3=(CH_2)_nX^9(CH_2)_qR^7$) (n=0, 1)

x = halo

24 ($R^3=(CH_2)_nX^9CH(COOR^6)(CH_2)_qR^7$) (n=0, 1)

24 ($R^3=(CH_2)_nX^9C(O)(CH_2)_qR^7$) (n=0, 1)

REACTION SCHEME IIIa

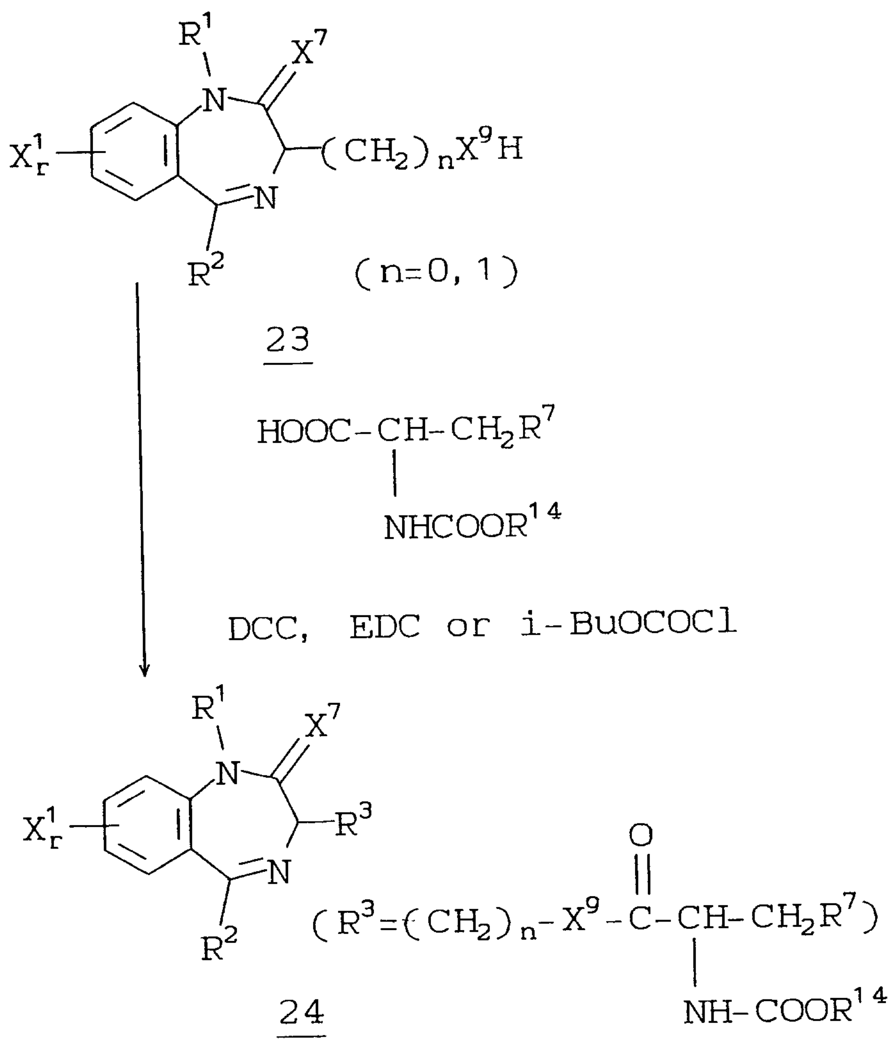


23

24

REACTION SCHEME IIIb

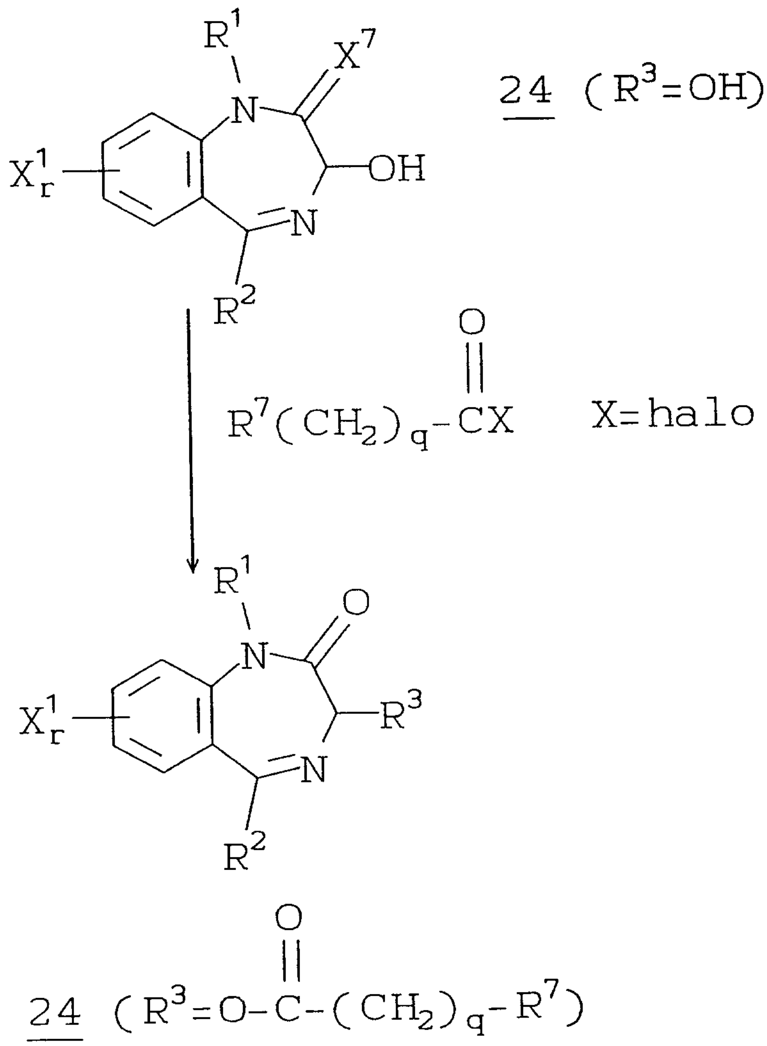


24 ($R^3$=OH)

24

REACTION SCHEME IIIc

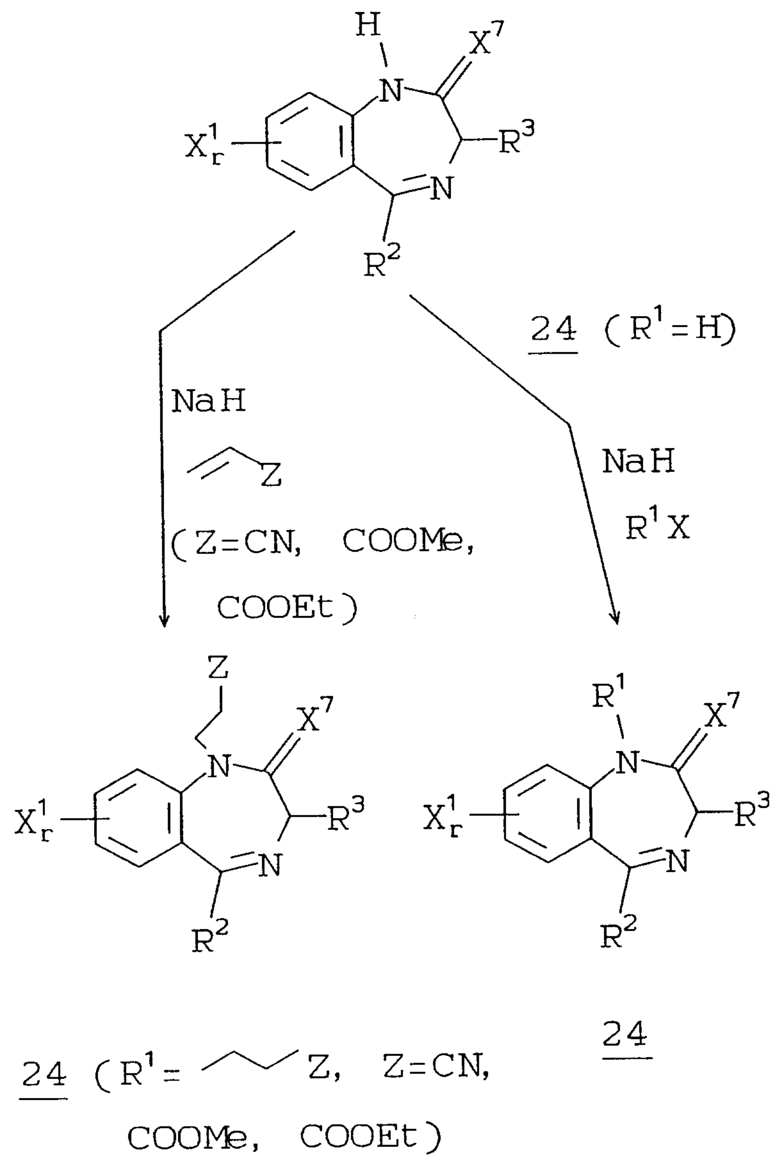

REACTION SCHEME IIId

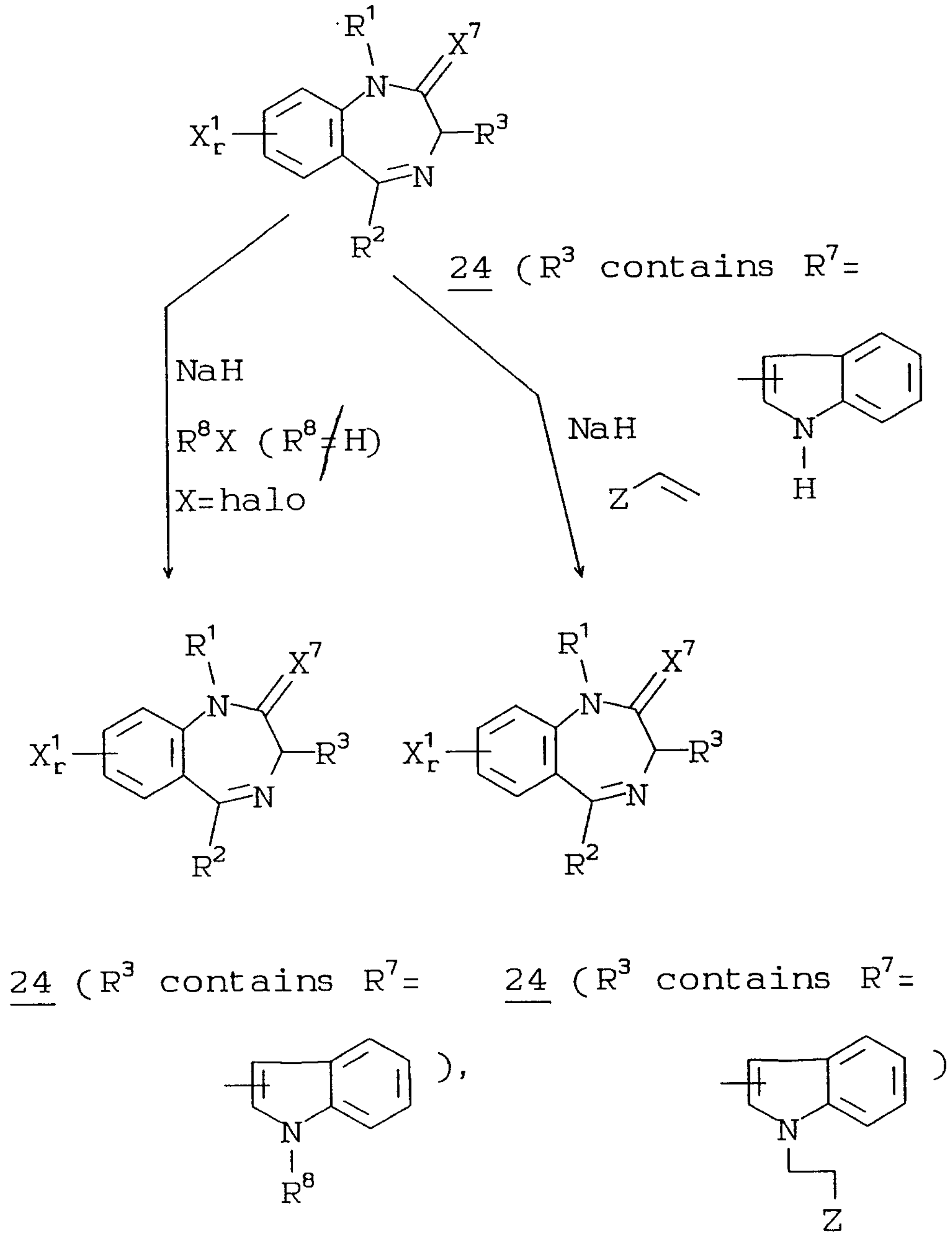


Where, in the 24 compound, $R^1$ and/or $R^8$ is an ester [$(X^{12})COO-C_1-C_3$ alkyl] moiety, this group can be conventionally hydrolyzed to obtain the corresponding acid moiety or treated with $NH_3$ to obtain the corresponding amide moiety.

REACTION SCHEME IV

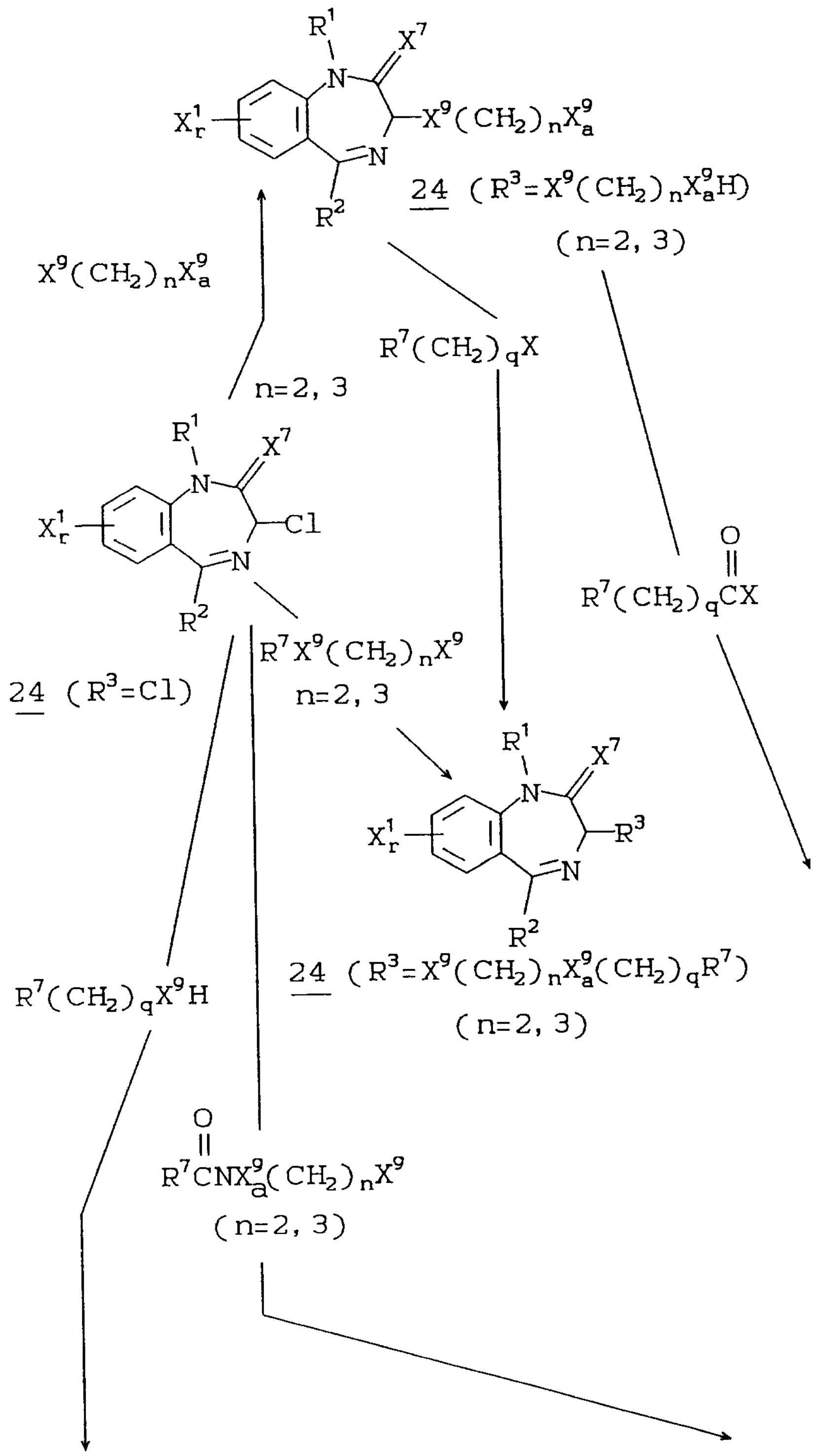

REACTION SCHEME IV (Cont'd)

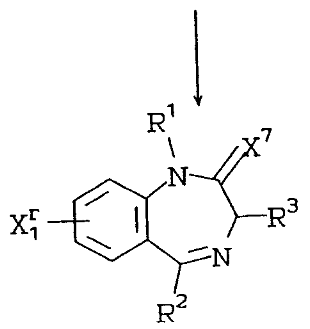

24 ($R^3=X^9(CH_2)_qR^7$)

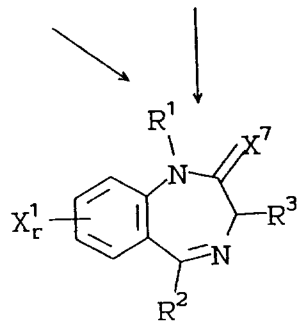

24 ($R^3=X^9(CH_2)_nX^9C(=O)(CH_2)_qR^7$)
(n=2, 3)

SCHEME IVa

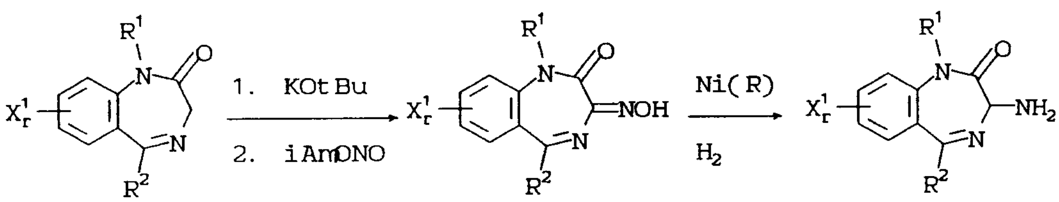


9 ($R^3$=H) 9 ($R^3$=NOH) 9 ($R^3=NH_2$)

REACTION SCHEME V

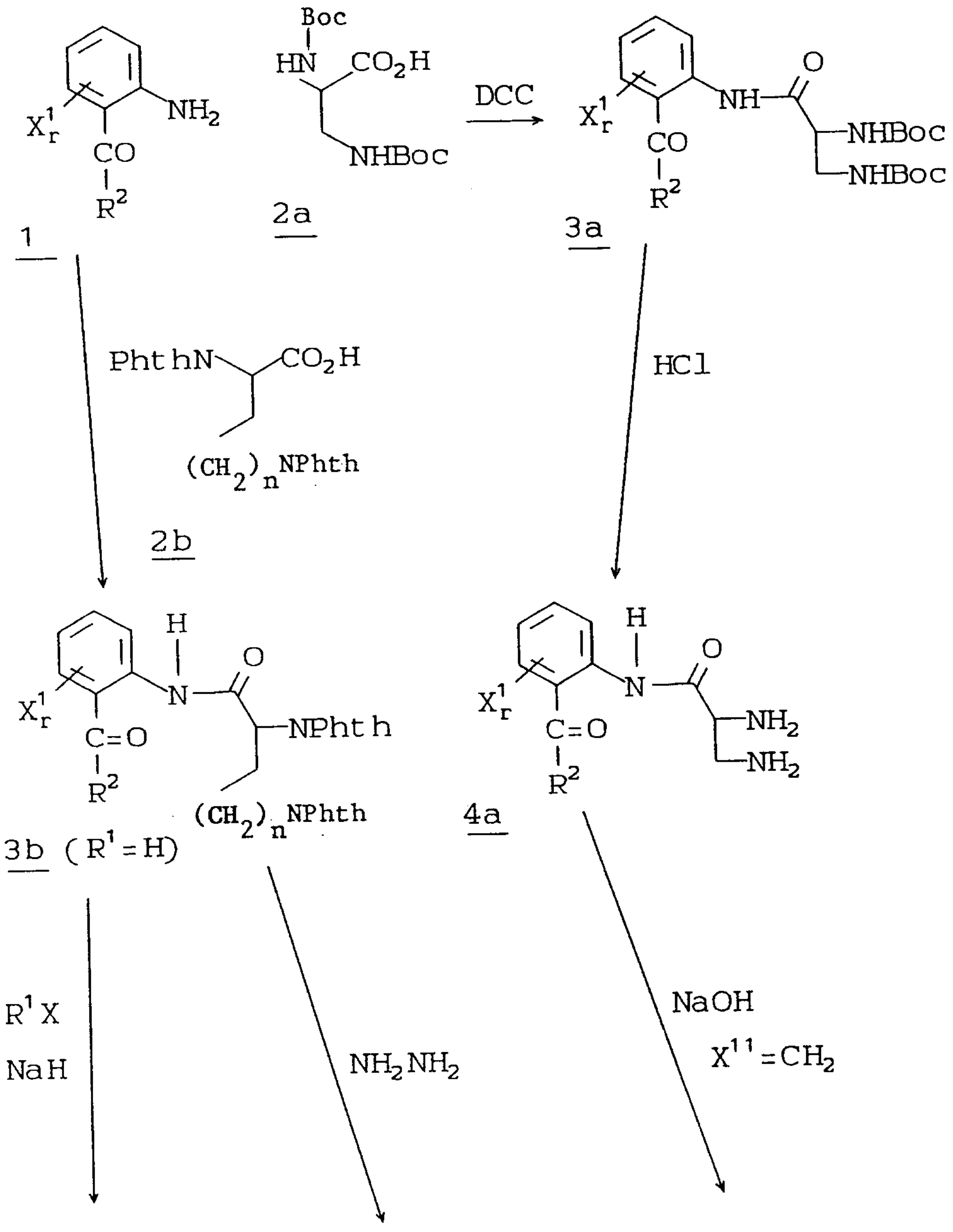


1 2a 3a 2b 3b ($R^1$ = H) 4a

REACTION SCHEME V (Cont'd)

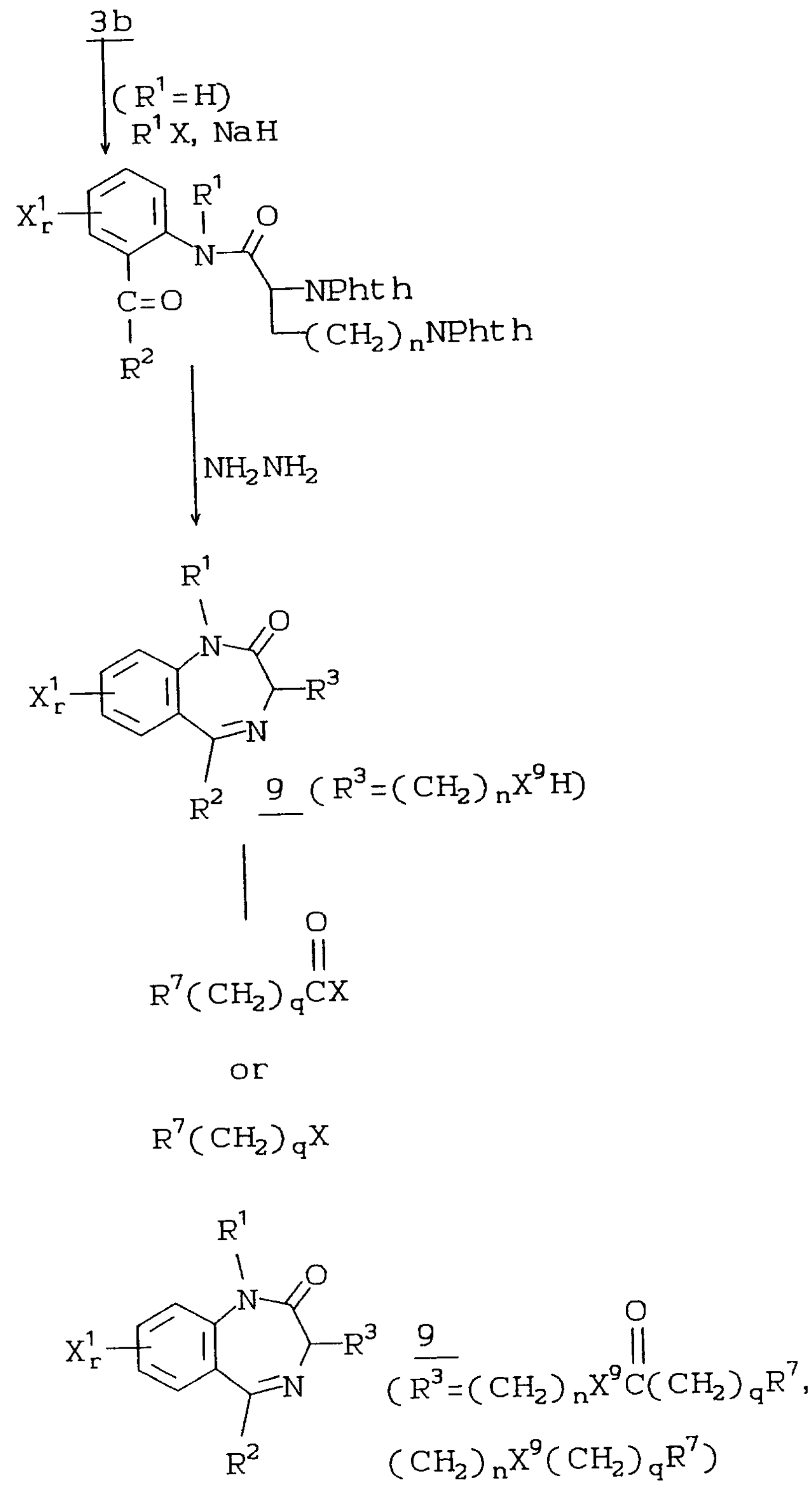

REACTION SCHEME V (Cont'd)

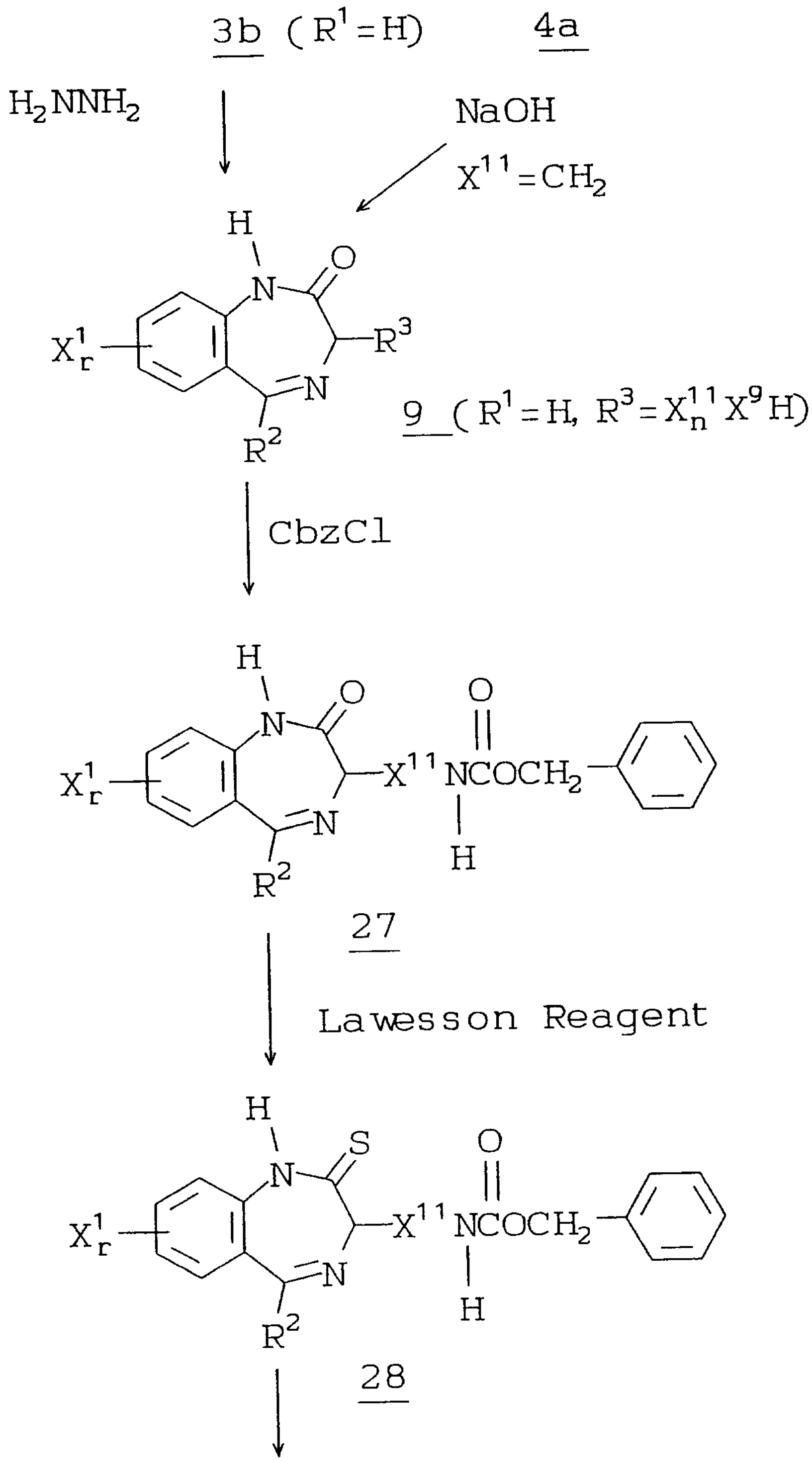

REACTION SCHEME V (Cont'd)

28

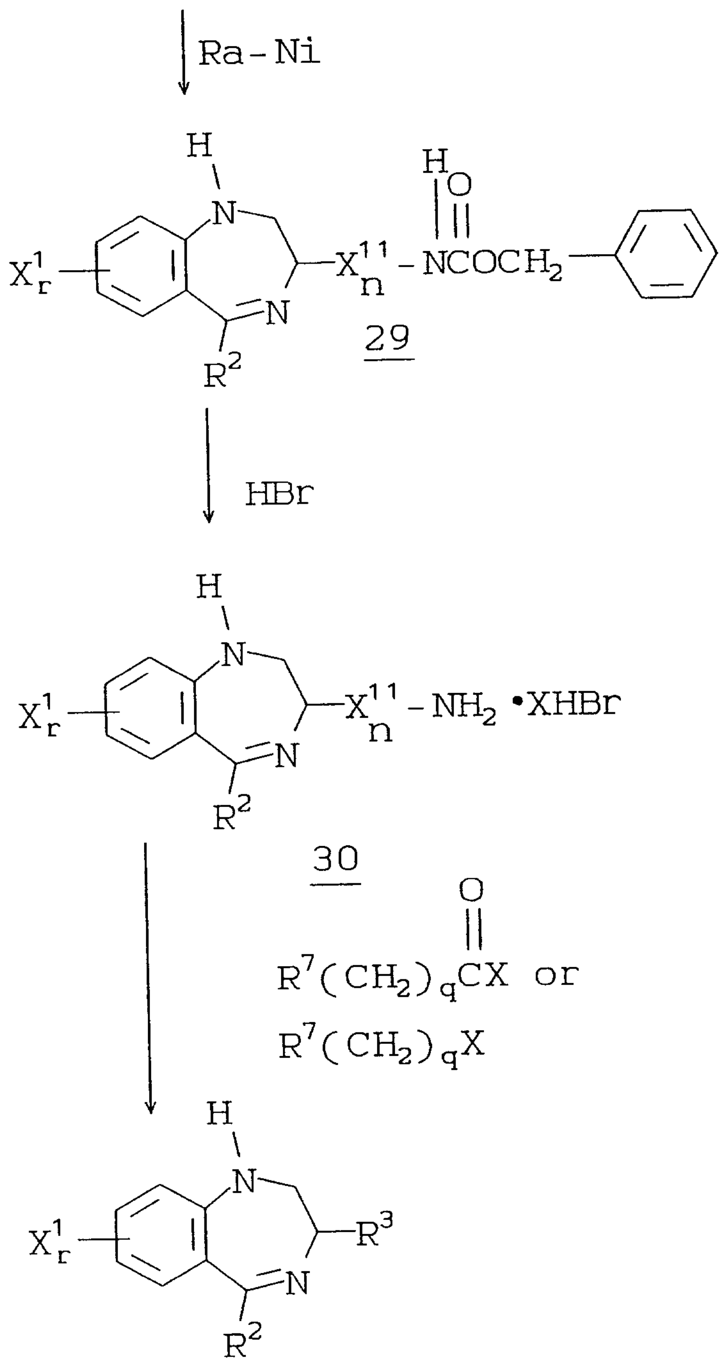


29

30

31

REACTION SCHEME V (Cont'd)

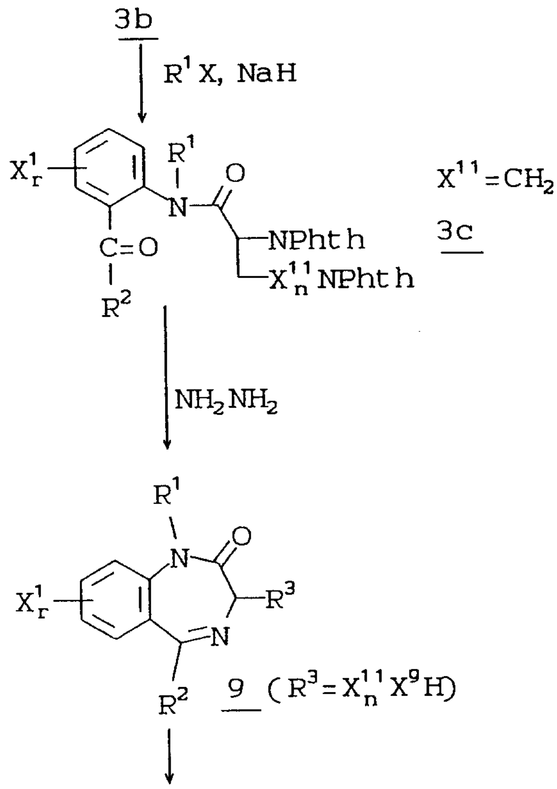

REACTION SCHEME V (Cont'd)

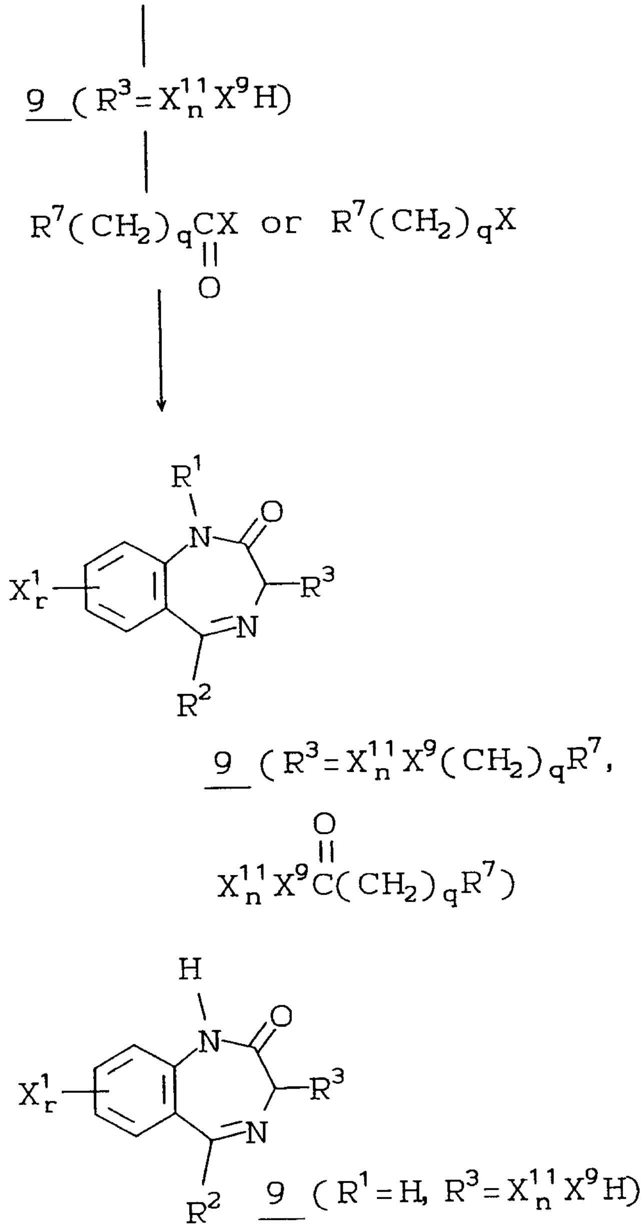


2-Aminoarylketones 1, (Scheme I) preferably 2-amino-benzophenones containing various substituents in the aryl rings, preferably halo substituents, are coupled to N-protected D-amino acids 2 - (preferably, Boc-amino acids) using dicyclohexylcarbodiimide (DCC) or other conventional peptide coupling reagent. The product 3 is N-deprotected by treatment with acid, preferably anhydrous HCl in ethyl acetate, to give the $\alpha$-aminoacyl derivative 4 of the 2-aminoarylketone. Alternatively, this same product is obtained by treatment of the 2-aminoarylketone 1 with the acid chloride hydrochloride 5 of the D-amino acid, which is prepared from the amino acid with $PCl_5$-AcCl.

Treatment of this $\alpha$-aminoacyl derivative 4 with base, preferably aqueous sodium hydroxide in methanol, gives the free base 6 which is cyclized to the 3,5-disubstituted benzodiazepine 7 upon stirring in the methanolic base for 2-120 hours, preferably 48 hours. Alternatively, the 3,5-disubstituted benzodiazepine 7 is obtained by heating the 2-aminoarylketone 1 with the ester 8, preferably methyl or ethyl, of the D-amino acid, preferably in refluxing pyridine, for 2-48 hours, preferably for 18 hours.

Alternatively (Scheme V), the ketones 1 may be coupled with N-phthalylamino acids such as 2b to give the products 3b using DCC or other conventional peptide coupling reagent. 3b may be deprotected and cyclized to 9 ($R^1=H$, $R^3=(CH_2)_nX^9H$) by treating with hydrazine. Alternatively, 3b may be first alkylated

by treatment with sodium hydride followed by an alkyl halide in dimethylformamide (DMF) to give the alkyl derivative 3c. Treating this product with hydrazine gives the $N^1$-alkylbenzodiazepine, 9 ($R^3 = (CH_2)_nX^9H$).

9 ($R^3 = (CH_2)_nX^9H$) are alkylated by treatment with alkyl halide or dialkyl sulfate or acylated by treatment with acid halides or anhydrides, preferably in the presence of base such as triethyl amine. The products are the alkyl and acyl derivatives 9 ($R^3 = (CH_2)_nX^9(CH_2)_qR^7$ and

$$R^3 = (CH_2)_nX^9\overset{\overset{O}{\|}}{C}(CH_2)_qR^7).$$

Alternatively, protection of the 3-amino function in 9 ($R^3 = (CH_2)_nNH_2$), preferably with benzylchloroformate affords the acyl derivative 27. Treatment of this material with $P_2S_5$ or preferably with Lawesson's reagent in toluene gives the thioamide 28 which is converted to the amine 29 with Raney nickel in ethanol. Deprotection of the resulting product 29 via hydrogenolysis, or preferably by the action of hydrobromic acid, yields the corresponding amino compound 30. Alkylation of 30 by treatment with alkyl halide or dialkyl sulfonate or acylation with carboxylic acid halide or carboxylic acid anhydride in the presence of an acid binding agent such as triethylamine or preferably with a carboxylic acid in the presence of a peptide coupling reagent such as dicyclohexyl-carbodiimide gives the alkyl or acyl derivatives 31.

3,5-Disubstituted benzodiazepines 7 (Scheme I) are also treated with sodium hydride in dimethylformamide (DMF), followed by an alkyl halide, to give the 1-alkyl derivatives 9. These or the parent 1-unsubstituted compound 7 are reduced, preferably with sodium cyanoborohydride and acetic acid at 15°, to give the corresponding 4,5-dihydro compounds 10. These are alkylated on $N_4$ by treatment with alkyl halide or dialkyl sulfate. Alternatively, the 4,5-dihydro compounds are acylated on $N_4$ by treatment with acyl halides or anhydrides, preferably in the presence of base such as triethylamine. The products are the alkyl and acyl derivatives 11. Alternatively, where $R^1$ is $-(CH_2)_mCOOR^6$ ($R^6$ not = H), 9 are treated with a base such as sodium hydroxide in methanol to give the acids 9 ($R^1 = (CH_2)_mCOOH$).

The 3,5-disubstituted benzodiazepines 7 are treated with alkyl- or arylmagnesium halides, preferably methylmagnesium iodide, to give the dihydro compounds 12. The products are alkylated and acylated on nitrogen, as described for the 3,5-disubstituted-4,5-dihydro derivatives, to give the derivatives 13.

The 3,5-disubstituted benzodiazepines 7 are treated with $P_2S_5$ or Lawesson's reagent (2,4-bis-(4-methoxyphenyl)-2,4-dithioxo-1,3,2,4-dithiadiphosphetane) to give the 2-thiones 14. These are reduced with Raney nickel to the 2-unsubstituted compounds 15. The latter may be alkylated with alkyl halide or sulfate, acylated with acyl halide or anhydride, reduced with sodium cyanoborohydride, or substituted with alkyl- or aryl magnesium halide as described for 7 above.

Where the 3-position in a 3,5-disubstituted benzodiazepine 7 bears a substituent containing an indole moiety, preferably 3-indolylmethyl, reduction with triethylsilane/TFA provides the corresponding indoline 16. Alternatively, oxidation with HCl-dimethylsulfoxide provides the oxindole 17. 16 and 17 may be subjected to the reactions described for 7 to obtain alkyl, acyl, and dihydro derivatives. Dialkyl, alkylacyl, and trialkyl compounds may also be made using these methods.

The 3,5-disubstituted benzodiazepines 7 may also be oxidized, preferably with m-chloroperoxybenzoic acid, to give the corresponding 4-N-oxides 7a.

Alternatively, (Scheme II) 3-unsubstituted-5-substituted-1-substituted or unsubstituted benzodiazepines 9 ($R^1 = H$) (Scheme II) prepared as described in the prior art may be treated with base, preferably lithium diisopropylamide, in an inert solvent, preferably THF, according to the procedure of J. Org. Chem., 46 4945 (1981). The resulting salt may be alkylated to obtain 9 with, for example, benzyl bromide or gramine methiodide. The resulting racemates may be resolved to obtain the preferred 3(R) enantiomers, or may be used as such.

Alternatively, the salt may be treated with an alkyl or aryl aldehyde, ketone, or acid halide or anhydride to give the 1-hydroxymethylene compounds

$$\underline{9}\ (R^3 = \overset{\overset{OH}{|}}{C}HR^7)$$

or

9 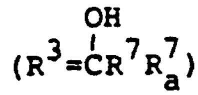

or the 1-ketomethylene derivatives

9 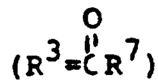

and

32 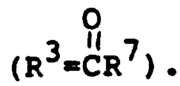.

If the acid halide reaction is carried out in solvent containing peroxide, the 3- and 5-hydroxy analogs 20 and 21 (resp.) may be obtained.

The hydroxymethylene compounds

9 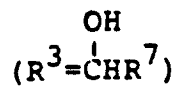

or

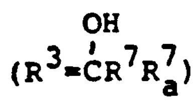

may be treated with acids,
preferably trifluoroacetic acid, to obtain the olefins 18, 19, and/or 22.

Alternatively, 3-substituted benzodiazepines 9 may be obtained by treating the 3-unsubstituted compound 9 ($R^3 = H$) with 1,8-diazabicyclo[5.4.0] undec-7-ene (DBU) and alkylating agent such as alkyl halide or sulfate or, preferably, gramine methiodide. Resolution to obtain the preferred 3(R) enantiomer may be carried out as described above.

3-Amino-5-substituted-1-substituted or unsubstituted benzodiazepines 9 ($R^3 = NH_2$) are prepared as described in the prior art. Alternatively, 9 ($R^3 = NH_2$) are prepared as shown in Scheme IVb. Treatment of the 3-unsubstituted compound 9 ($R^3 = H$) with a suitable base, preferably potassium t-butoxide, followed by a nitrosating agent, preferably isoamyl nitrate, provides the oxime 9 ($R^3 = =NOH$). Reduction, preferably with Raney nickel, gives the 3-amino compounds 9 ($R^3 = NH_2$).

3-Amino and 3-aminomethyl-5-substituted-1-substituted or unsubstituted benzodiazepines 23 - (Scheme III) are alkylated with alkyl halides or with $\alpha$-halo acids and esters to give the alkyl derivatives 24 ($R^3 = (CH_2)_nNH(CH_2)_qR^7$) and

9 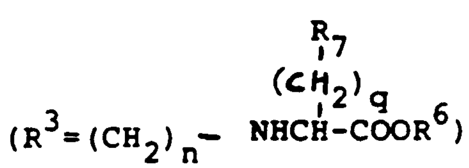.

With acyl halides, the amines 23 give the corresponding amides

$$\underline{24}\ (R^3 = (CH_2)_nNH\overset{O}{\overset{\parallel}{C}}(CH_2)_qR^7).$$

With isocyanates, the amines 23 give the corresponding ureas

$$\underline{24}\ (R^3 = (CH_2)_n\overset{H}{N}\overset{O}{\overset{\parallel}{C}}\overset{H}{N}(CH_2)_qR^7).$$

With N-protected or unprotected $\alpha$-amino acids and a coupling reagent such as DCC, EDC, or isobutyl chloroformate, the amines 23 give the amides

$$\underline{24}\ (R^3 = (CH_2)_nNH\overset{O}{\overset{\parallel}{C}}\underset{NHCOOR^{14}}{CH}CH_2R^7).$$

3-Hydroxy-5-substituted-7-substituted or unsubstituted-1-substituted or unsubstituted benzodiazepines 24 ($R^3 = OH$) (Scheme IIIb) are acylated with acyl halides to give the esters

$$\underline{24}\ (R^3 = O\overset{O}{\overset{\parallel}{C}}(CH_2)_qR^7).$$

3-Chloro-5-substituted-1-substituted or unsubstituted benzodiazepines 24 ($R^3 = Cl$) (Scheme IV) may be used to monoalkylate amines to give the 3-substituted amino compounds 24 ($R^3 = NH_2$). The 3-chloro compounds 29 may also be used to monoalkylate 1,2-ethanediamine and 1,3-propanediamine to give the compounds 24 ($R^3 = NH(CH_2)NH_2$). These may be alkylated to provide 24 ($R^3 = NH(CH_2)_nNH-(CH_2)qR^7$) or acylated to give

$$\underline{24}\ (R^3 = NH(CH_2)_nNH\overset{O}{\overset{\parallel}{C}}(CH_2)_qR^7).$$

Alternatively, the latter two compounds may be obtained from the previously mono-alkylated or acylated diamine and chloro compound 24 ($R^3 = Cl$).

3-Substituted-5-substituted-7-substituted or unsubstituted benzodiazepines 24 ($R^1 = H$) (Scheme IIIc) may be treated with sodium hydride in a suitable solvent, such as DMF, followed by an alkyl halide to provide the 1-alkyl derivatives 24. When an acrylate such as methyl or ethyl acrylate or acylonitrile is substituted for the alkyl halide, the 1-(2-substituted)ethyl compounds

24 ($R^1$ = 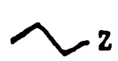)

are obtained.

When $R^3$ contains $R^7$ where $R^7$ is 1-unsubstituted-2- or 3-indolyl (Scheme IIId), the compounds 24 may be further alkylated by treatment with sodium hydride followed by an alkyl halide or an acrylate, such as methyl or ethyl acrylate or acrylonitrile, or an activated amino acid such as Boc-phenylalanine

anhydride to give the corresponding 1-substituted indole compounds 24 (Scheme IIId) in which $R^8$ is as defined herein and $R^8$ is other than hydrogen.

The compounds 24 wherein $R^1$ and/or $R^8$ is $(CH_2)_m-COOMe$ or $(CH_2)_m-COOEt$ may be treated with sodium hydroxide in an aqueous solvent, preferably aqueous solvent, preferably aqueous methanol, and then acidified to give the corresponding acids 24, wherein $R^1$ and/or $R^8$ is $(CH_2)_nCOOH$. Alternatively, these same compounds may be treated with aqueous or anhydrous ammonia to give the amides 24 wherein $R^1$ and/or $R^8$ is $(CH_2)_mCONH_2$.

In cases where the starting materials are optically active, the chirality at $C_3$ is controlled by the synthesis. When racemic starting materials are employed, racemic products are obtained. The enantiomers may be separated by resolution.

## In Vitro Activity of Formula I

The biological activity of the compounds of Formula I have been evaluated using 1.)an $^{125}I-CCK$ receptor binding assay and in vitro isolated tissue preparations and 2.) $^{125}I$-gastrin and $^{3}H$-pentagastrin binding assays.

## Materials and Methods

### 1. CCK Receptor Binding (Pancreas)

CCK-33 was radiolabeled with $^{125}I$-Bolton Hunter reagent (2000 Ci/mmole) as described by Sankara et al. (J. Biol. Chem. 254: 9349-9351, 1979). Receptor binding was performed according to Innis and Snyder (Proc. Natl. Acad. Sci. 77: 6917-6921, 1980) with the minor modification of adding the additional protease inhibitors, phenylmethane sulfonyl fluoride and o-phenanthroline. The latter two compounds have no effect on the $^{125}I$-CCK receptor binding assay.

Male Sprague-Dawley rats (200-350g) were sacrificed by decapitation. The whole pancreas was dissected free of fat tissue and was homogenized in 20 volumes of ice-cold 50 mM, Tris HCl (pH 7.7 at 25°C) with a Brinkmann Polytron PT 10. The homogenates were centrifuged at 48,000 g for 10 min. Pellets were resuspended in Tris Buffer, centrifuged as above and resuspended in 200 volumes of binding assay buffer (50 mM Tris HCl, pH 7.7 at 25°C, 5 mM dithiothrietol, 0.1 mM bacitracin, 1.2 mM phenylmethane sulfonyl fluoride and 0.5 mM o-phenanthroline). For the binding assay, 25 µl of buffer (for total binding) or unlabeled CCK-8 sulfate to give a final concentration of 1 µM (for nonspecific binding) or the compounds of Formula I (for determination of inhibition of $^{125}I$-CCK binding) and 25 µl of $^{125}I$-CCK-33 (30,000-40,000 cpm) were added to 450 µl of the membrane suspensions in microfuge tubes. All assays were run in duplicate or triplicate. The reaction mixtures were incubated at 37°C for 30 minutes and centrifuged in a Beckman Microfuge (4 minutes) immediately after adding 1 ml of ice-cold incubation buffer. The supernatant was aspirated and discarded, pellets were counted with a Beckman gamma 5000. For Scatchard analysis (Ann. N.Y. Acad. Sci. 51: 660, 1949), $^{125}I$-CCK-33 was progressively diluted with increasing concentrations of CCK-33.

### 2. CCK Receptor Binding (Brain)

CCK-33 was radiolabeled and the binding was performed according to the description for the pancreas method with modifications according to Saito et al., J. Neurochem. 37:483-490, 1981.

Male Hartley guinea pigs (300-500g) were sacrificed by decapitation and the brains were removed and placed in ice-cold 50 mM, Tris HCl plus 7.58 g/l Trizma-7.4 (pH 7.4 at 25°C). Cerebral cortex was dissected and used as a receptor source. Each gram of fresh guinea pig brain tissue was homogenized in 10 ml of Tris/Trizma buffer with a Brinkman polytron PT-10. The homogenates were centrifuged at 42,000 g for 15 minutes. Pellets were resuspended in Tris Buffer, centrifuged as above and resuspended in 200 volumes of binding assay buffer (10 mM N-2-hydroxyethyl-piperazine-N'-2-ethane sulfonic acid (HEPES), 5 mM $MgCl_2$, 0.25 mg/ml bacitracin, 1 mM ethylene glycol-bis-(β-aminoethylether-N,N'-tetraacetic acid) (EGTA), and 0.4% bovine serum albumin (BSA)). For the binding assay, 25 µl of buffer (for total binding) or unlabeled CCK-8 sulfate to give a final concentration of 1µm (for nonspecific binding) or the compounds of Formula I (for determination of inhibition of $^{125}I$-CCK binding) and 25 µl of $^{125}I$-CCK-33 (30,000-40,000 cpm) were added to 450 µl of the membrane suspensions in microfuge tubes. All assays were run in duplicate or triplicate. The reaction mixtures were incubated at 25°C for 2 hours and centrifuged in a Beckman Microfuge (4 minutes) immediately after adding 1 ml of ice-cold incubation

buffer. The supernatant was aspirated and discarded, pellets were counted with a Beckman gamma 5000.

The compounds of Formula I can be determined to be competitive antagonists of CCK according to the following essays.

3. Isolated guinea pig gall bladder

Mole Hartley guinea pigs (400 – 600 g) are sacrificed by decapitation. The whole gall bladder is dissected free from adjacent tissues and cut into two equal halves. The gall bladder strips are suspended along the axis of the bile duct in a 5 ml organ bath under 1 g tension. The organ bath contains a Kreb's bicarbonate solution (NaCl 118 mM, KCl 4.75 mM, CaCl 2.54 mM, $KH_2PO_4$ 1.19 mM, Mg $SO_4$ 1.2 mM, $NaHCO_3$ 25 mM and dextrose 11 mM) maintained at 32°C and bubbled with 95% $0_2$ and 5% $CO_2$. Isometric contractions are recorded using Statham (60 g; 0.12 mm) strain gauges and a Hewlett – Packard (77588) recorder. The tissues are washed every 10 minutes for 1 hour to obtain equilibrium prior to the beginning of the study. CCK – 8 is added cumulatively to the baths and $EC_{50}$'s determined using regression analysis. After washout (every 10 minutes for 1 hour), the compound of Formula I is added at least 5 minutes before the addition of CCk – 8 and the $EC_{50}$ of CCK – 8 in the presence of the compound of Formula I similarly determined.

4. Isolated longitudinal muscle of guinea pig ileum

Longitudinal muscle strips with attached nerve plexus are prepared as described in Brit. J. Pharmac. 23: ; 356 – 363, 1964; J. Physiol. 194: 13 – 33, 1969. Male Hartley guinea pigs are decapitated and the ileum removed (10 cm of the terminal ileum is discarded and the adjacent 20 cm piece used). A piece (10 cm) of the ileum is stretched on a glass pipette. Using a cotton applicator to stroke tangentially away from the mesentery attachment at one end, the longitudinal muscle is separated from the underlying circular muscle. The longitudinal muscle is then tied to a thread and by gently pulling, stripped away from the entire muscle. A piece of approximately 2 cm is suspended in 5 ml organ bath containing Krebs solution and bubbled with 95% $O_2$ and 5% $CO_2$ at 37°C under 0.5 g tension. CCK – 8 is added cumulatively to the baths and $EC_{50}$ values in the presence and absence of compounds of Formula I determined as described in the gall bladder protocol (above).

Gastrin Antagonism

Gastrin antagonist activity of compounds of Formula I is determined using the following assay.

Gastrin Receptor Binding in Guinea Pig Gastric Glands Preparation of guinea pig gastric mucosal glands

Guinea pig gastric mucosal glands were prepared by the procedure of Berglingh and Obrink Acta Physiol. Scand. 96: 150 (1976) with a slight modification according to Praissman et al. C. J. Receptor Res. 3: (1983). Gastric mucosa from guinea pigs ( 300 – 500 g body weight, male Hartley) were washed thoroughly and minced with fine scissors in standard buffer consisting of the following: 130 mM NaCl, 12 mM $NaHCO_3$, 3 mM $NaH_2PO_4$, 3 mM $Na_2HPO_4$, 3 mM $K_2HPO_4$, 2 mM $MgSO_4$, 1mM $CaCl_2$, 5 mM glucose and 4 mM L – glutamine, 25 mM HEPES at pH 7.4. The minced tissues were washed and then incubated in a 37°C shaker bath for 40 minutes with the buffer containing 0.1% collagenase and 0.1% BSA and bubbled with 95% $O_2$ and 5% $CO_2$. The tissues were passed twice through a 5 ml glass syringe to liberate the gastric glands, and then filtered through 200 mesh nylon. The filtered glands were centrifuged at 270 g for 5 minutes and washed twice by resuspension and centrifugation.

Binding studies

The washed guinea pig gastric glands prepared as above were resuspended in 25 ml of standard buffer containing 0.25 mg/ml of bacitracin. For binding studies, to 220 $\mu$l of gastric glands in triplicate tubes, 10 $\mu$l of buffer (for total binding) or gastrin (1 $\mu$M final concentration, for nonspecific binding) or test compound and 10 $\mu$l of $^{125}I$ – gastrin (NEN, 2200 Ci/mmole, 25 pM final) or $^{3}H$ – pentagastrin (NEN 22 Ci/mmole, 1 nM final) were added. The tubes were aerated with 95% $O_2$ and 5% $CO_2$ and capped. The reaction mixtures after incubation at 25°C for 30 minutes were filtered under reduced pressure on glass G/F B filters (Whatman) and immediately washed further with 4 x 4 ml of standard buffer containing 0.1% BSA. The radioactivity on the filters was measured using a Beckman gamma 5500 for $^{125}I$ – gastrin or liquid

scintillation counting for $^{3}H$-pentagastrin.

In Vitro Results

1. Effect of The Compounds of Formula I on $^{125}I$-CCK-33 receptor binding

The preferred compounds of Formula I are those which inhibited specific $^{125}I$-CCK-33 binding in a concentration dependent manner.

Scatchard analysis of specific $^{125}I$-CCK-33 receptor binding in the absence and presence of the compounds of Formula I indicated the compound of Formula I competitively inhibited specific $^{125}I$-CCK-33 receptor binding since it increased the $K_D$ (dissociation constant) without affecting the $B_{max}$ (maximum receptor number). A $K_i$ value (dissociation constant of inhibitor) of the compounds of Formula I was estimated.

The data of Table 1 were obtained for compounds of Formula I.

**TABLE I**

**CCK Receptor Binding Results**

| Compound of Example | $IC_{50}$ (μM) $^{125}$I-CCK Pancreas | $^{125}$I-CCK Brain |
|---|---|---|
| 2 & 3 | 0.40 | 81.50 |
| 4a & 44 | 0.36 | 16.00 |
| 4b | 0.27 | 18.00 |
| 5 | 3.40 | 100.00 |
| 6 | 1.20 | 50.00 |
| 12 | 4.00 | ca. 100 |
| 28 | 5.00 | ca. 100 |
| 31 | 1.40 | ca. 100 |
| 34 | 4.50 | ca. 100 |
| 36 | 0.30 | 30.00 |
| 37 | 2.20 | 30.00 |
| 39 | 100.00 | 30.00 |
| 40 | 3.60 | ca. 100 |
| 43 | 0.30 | 23.00 |
| 50 | 15.00 | 2.60 |
| 51 | ca. 100 | 32 |
| 52 | ca. 100 | 33 |
| 53a | 100.00 | 2.60 |
| 57 | 2.90 | 100.00 |
| 58 | 18.00 | 12.00 |
| 59 | 1.40 | ca. 100 |
| 60 | 1.30 | 100.00 |
| 68 | 7.00 | 30.00 |
| 73 | 0.0047 | 8.00 |
| 74 | 3.00 | 100.00 |

TABLE I (cont'd)
CCK Receptor Binding Results

| Compound of Example | $IC_{50}$ (μM) $^{125}I$-CCK Pancreas | $^{125}I$-CCK Brain |
|---|---|---|
| 75 | 4.80 | 100.00 |
| 76 | 1.00 | 11.00 |
| 77 | 6.00 | 20.00 |
| 78 | 0.0014 | 6 |
| 79(A) | 0.0008 | 0.8 |
| 79(B) | 0.0014 | 15 |
| 80 | 0.0023 | 3.4 |
| 81a | 0.0014 | 0.3 |
| 81b | 0.0013 | 1.0 |
| 87 | 0.0011 | 0.27 |
| 88 | 0.0006 | 0.3 |
| 89 | 0.019 | 1.1 |
| 90 | 0.049 | 11 |
| 91 | 0.0025 | 2.9 |
| 92 | 0.0043 | 1.6 |
| 93 | 0.7 | 2.9 |
| 94 | 0.053 | 3.8 |
| 105 | 0.0021 | 3 |
| 111 | 0.006 | 40 |
| 113 | 0.0015 | 5.6 |
| 114 | 0.005 | 12 |
| 121 | 0.011 | 5.5 |
| 128 | 0.009 | 32 |
| 131 | 0.0083 | 40 |

TABLE I (cont'd)

CCK Receptor Binding Results

| Compound of Example | IC50 (μM) $^{125}$I-CCK Pancreas | $^{125}$I-CCK Brain |
|---|---|---|
| 132 | 0.032 | >100 |
| 134 | 0.015 | 40 |
| 154 | 0.0035 | 3.5 |
| 156 | 0.0035 | 4 |
| 159 | 0.0034 | 3 |
| 160 | 0.020 | 12 |
| 167 | 0.00075 | 1.7 |
| 168 | 0.015 | 2.4 |

Preferred compounds of Formula I are those wherein R' is H, methyl, ethyl, carboxymethyl, ethyl-carboxymethyl and carboxyethyl.

Other series of preferred compounds are those wherein $R^2$ is phenyl, p-chlorophenyl, o-chlorophenyl, p-fluorophenyl, o-fluorophenyl, 2-4-dichlorophenyl, 2-6-difluorophenyl, $-CH_2COO$-t-butyl, or $-CH_2COOEt$.

Other series of preferred compounds are those wherein $R^3$ is 2- or 3-indolylmethyl, -CO-thiophene, -NHCO-2-indolyl, NHCO-2-(1-methylindolyl), NHCO-2-(5-fluoroindolyl), NHCO-2-benzofuranyl, NHCO-2-benzothieyl, NHCO-2-(3-methylindenyl), NHCO-(mono- or dihalophenyl), NHCO-phenylethenyl, NHCO-(mono- or dimethyl or trifluoromethylphenyl), NHCONH-(mono- or di-halophenyl), CO-2-(1-methyl)-indolyl, CO-3-(1-methyl)indolyl, or -CHOH-1-methylindol-3-yl.

When p is 1 for any of $R^9$, $R^{10}$, or $R^{13}$, it is preferred that $R^9$ is H or hydroxyl, $R^{10}$ is H or hydroxyl, and $R^{13}$ is H.

It is preferred that $X_r^1$ is H, Cl, F, $CF_3$, OH or $NO_2$.

Examples of Formula I compounds are tabulated below.

TABLE 2

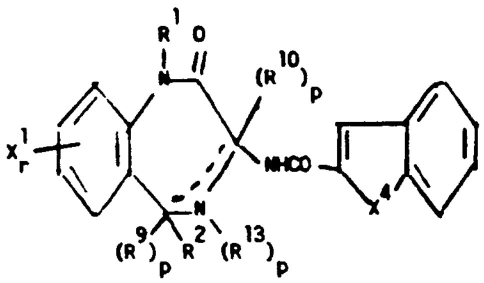

$X^4$=NH, N-$CH_3$, O, or S

| $X^1$ | r | $R^1$ | $(R^9)_p$ | $R^2$ | $(R^{13})_p$ | $(R^{10})_p$ |
|---|---|---|---|---|---|---|
| H | 1 | H | - | Ph | - | H |
| Cl | 1 | H | - | Ph | - | H |
| F | 1 | H | - | Ph | - | H |
| $CF_3$ | 1 | H | - | Ph | - | H |
| OH | 1 | H | - | Ph | - | H |
| $NO_2$ | 1 | H | - | Ph | - | H |
| H | 1 | $CH_3$ | - | Ph | - | H |
| Cl | 1 | $CH_3$ | - | Ph | - | H |
| F | 1 | $CH_3$ | - | Ph | - | H |
| $CF_3$ | 1 | $CH_3$ | - | Ph | - | H |
| OH | 1 | $CH_3$ | - | Ph | - | H |
| $NO_2$ | 1 | $CH_3$ | - | Ph | - | H |
| H | 1 | $CH_2COOH$ | - | Ph | - | H |
| Cl | 1 | $CH_2COOH$ | - | Ph | - | H |
| F | 1 | $CH_2COOH$ | - | Ph | - | H |
| $CF_3$ | 1 | $CH_2COOH$ | - | Ph | - | H |
| OH | 1 | $CH_2COOH$ | - | Ph | - | H |
| $NO_2$ | 1 | $CH_2COOH$ | - | Ph | - | H |
| H | 1 | $CH_2CH_3$ | - | Ph | - | H |
| OH | 1 | $CH_2CH_3$ | - | Ph | - | H |
| H | 1 | $CH_2COOEt$ | - | Ph | - | H |
| OH | 1 | $CH_2COOEt$ | - | Ph | - | H |
| H | 1 | $CH_2CH_2COOH$ | - | Ph | - | H |

TABLE 2 (cont'd)

| $X^1$ | r | $R^1$ | $(R^9)_p$ | $R^2$ | $(R^{13})_p$ | $(R^{10})_p$ |
|---|---|---|---|---|---|---|
| OH | 1 | $CH_2CH_2COOH$ | - | Ph | - | H |
| H | 1 | H | - | o-F-Ph | - | H |
| Cl | 1 | H | - | o-F-Ph | - | H |
| F | 1 | H | - | o-F-Ph | - | H |
| $CF_3$ | 1 | H | - | o-F-Ph | - | H |
| OH | 1 | H | - | o-F-Ph | - | H |
| $NO_2$ | 1 | H | - | o-F-Ph | - | H |
| H | 1 | $CH_3$ | - | o-F-Ph | - | H |
| Cl | 1 | $CH_3$ | - | o-F-Ph | - | H |
| F | 1 | $CH_3$ | - | o-F-Ph | - | H |
| $CF_3$ | 1 | $CH_3$ | - | o-F-Ph | - | H |
| OH | 1 | $CH_3$ | - | o-F-Ph | - | H |
| $NO_2$ | 1 | $CH_3$ | - | o-F-Ph | - | H |
| H | 1 | $CH_2COOH$ | - | o-F-Ph | - | H |
| Cl | 1 | $CH_2COOH$ | - | o-F-Ph | - | H |
| F | 1 | $CH_2COOH$ | - | o-F-Ph | - | H |
| $CF_3$ | 1 | $CH_2COOH$ | - | o-F-Ph | - | H |
| OH | 1 | $CH_2COOH$ | - | o-F-Ph | - | H |
| $NO_2$ | 1 | $CH_2COOH$ | - | o-F-Ph | - | H |
| H | 1 | $CH_2CH_3$ | - | o-F-Ph | - | H |
| OH | 1 | $CH_2CH_3$ | - | o-F-Ph | - | H |
| H | 1 | $CH_2COOEt$ | - | o-F-Ph | - | H |
| OH | 1 | $CH_2COOEt$ | - | o-F-Ph | - | H |
| H | 1 | $CH_2CH_2COOH$ | - | o-F-Ph | - | H |
| OH | 1 | $CH_2CH_2COOH$ | - | o-F-Ph | - | H |
| H | 1 | H | - | p-Cl-Ph | - | H |
| F | 1 | H | - | p-Cl-Ph | - | H |
| $CF_3$ | 1 | H | - | p-Cl-Ph | - | H |
| OH | 1 | H | - | p-Cl-Ph | - | H |
| H | 1 | $CH_3$ | - | p-Cl-Ph | - | H |

TABLE 2 (cont'd)

| $X^1$ | r | $R^1$ | $(R^9)_p$ | $R^2$ | $(R^{13})_p$ | $(R^{10})_p$ |
|---|---|---|---|---|---|---|
| F | 1 | $CH_3$ | - | p-Cl-Ph | - | H |
| $CF_3$ | 1 | $CH_3$ | - | p-Cl-Ph | - | H |
| OH | 1 | $CH_3$ | - | p-Cl-Ph | - | H |
| H | 1 | $CH_2COOH$ | - | p-Cl-Ph | - | H |
| F | 1 | $CH_2COOH$ | - | p-Cl-Ph | - | H |
| $CF_3$ | 1 | $CH_2COOH$ | - | p-Cl-Ph | - | H |
| OH | 1 | $CH_2COOH$ | - | p-Cl-Ph | - | H |
| H | 1 | $CH_2CH_3$ | - | p-Cl-Ph | - | H |
| H | 1 | $CH_2COOEt$ | - | p-Cl-Ph | - | H |
| H | 1 | $CH_2CH_2COOH$ | - | p-Cl-Ph | - | H |
| H | 1 | H | - | $CH_2COOt\text{-}Bu$ | - | H |
| Cl | 1 | H | - | $CH_2COOt\text{-}Bu$ | - | H |
| F | 1 | H | - | $CH_2COOt\text{-}Bu$ | - | H |
| $CF_3$ | 1 | H | - | $CH_2COOt\text{-}Bu$ | - | H |
| OH | 1 | H | - | $CH_2COOt\text{-}Bu$ | - | H |
| $NO_2$ | 1 | H | - | $CH_2COOt\text{-}Bu$ | - | H |
| H | 1 | $CH_3$ | - | $CH_2COOt\text{-}Bu$ | - | H |
| Cl | 1 | $CH_3$ | - | $CH_2COOt\text{-}Bu$ | - | H |
| F | 1 | $CH_3$ | - | $CH_2COOt\text{-}Bu$ | - | H |
| $CF_3$ | 1 | $CH_3$ | - | $CH_2COOt\text{-}Bu$ | - | H |
| OH | 1 | $CH_3$ | - | $CH_2COOt\text{-}Bu$ | - | H |
| $NO_2$ | 1 | $CH_3$ | - | $CH_2COOt\text{-}Bu$ | - | H |
| H | 1 | $CH_2COOH$ | - | $CH_2COOt\text{-}Bu$ | - | H |
| Cl | 1 | $CH_2COOH$ | - | $CH_2COOt\text{-}Bu$ | - | H |
| F | 1 | $CH_2COOH$ | - | $CH_2COOt\text{-}Bu$ | - | H |
| $CF_3$ | 1 | $CH_2COOH$ | - | $CH_2COOt\text{-}Bu$ | - | H |
| OH | 1 | $CH_2COOH$ | - | $CH_2COOt\text{-}Bu$ | - | H |
| $NO_2$ | 1 | $CH_2COOH$ | - | $CH_2COOt\text{-}Bu$ | - | H |
| H | 1 | $CH_2CH_3$ | - | $CH_2COOt\text{-}Bu$ | - | H |
| OH | 1 | $CH_2CH_3$ | - | $CH_2COOt\text{-}Bu$ | - | H |

TABLE 2 (cont'd)

| $X^1$ | r | $R^1$ | $(R^9)_p$ | $R^2$ | $(R^{13})_p$ | $(R^{10})_p$ |
|---|---|---|---|---|---|---|
| H | 1 | $CH_2COOEt$ | - | $CH_2COOt$-Bu | - | H |
| OH | 1 | $CH_2COOEt$ | - | $CH_2COOt$-Bu | - | H |
| H | 1 | $CH_2CH_2COOH$ | - | $CH_2COOt$-Bu | - | H |
| OH | 1 | $CH_2CH_2COOH$ | - | $CH_2COOt$-Bu | - | H |
| H | 1 | H | - | $CH_2COOEt$ | - | H |
| Cl | 1 | H | - | $CH_2COOEt$ | - | H |
| F | 1 | H | - | $CH_2COOEt$ | - | H |
| $CF_3$ | 1 | H | - | $CH_2COOEt$ | - | H |
| OH | 1 | H | - | $CH_2COOEt$ | - | H |
| $NO_2$ | 1 | H | - | $CH_2COOEt$ | - | H |
| H | 1 | $CH_3$ | - | $CH_2COOEt$ | - | H |
| Cl | 1 | $CH_3$ | - | $CH_2COOEt$ | - | H |
| F | 1 | $CH_3$ | - | $CH_2COOEt$ | - | H |
| $CF_3$ | 1 | $CH_3$ | - | $CH_2COOEt$ | - | H |
| OH | 1 | $CH_3$ | - | $CH_2COOEt$ | - | H |
| $NO_2$ | 1 | $CH_3$ | - | $CH_2COOEt$ | - | H |
| H | 1 | $CH_2COOH$ | - | $CH_2COOEt$ | - | H |
| Cl | 1 | $CH_2COOH$ | - | $CH_2COOEt$ | - | H |
| F | 1 | $CH_2COOH$ | - | $CH_2COOEt$ | - | H |
| $CF_3$ | 1 | $CH_2COOH$ | - | $CH_2COOEt$ | - | H |
| OH | 1 | $CH_2COOH$ | - | $CH_2COOEt$ | - | H |
| $NO_2$ | 1 | $CH_2COOH$ | - | $CH_2COOEt$ | - | H |
| H | 1 | $CH_2CH_3$ | - | $CH_2COOEt$ | - | H |
| OH | 1 | $CH_2CH_3$ | - | $CH_2COOEt$ | - | H |
| H | 1 | $CH_2COOEt$ | - | $CH_2COOEt$ | - | H |
| OH | 1 | $CH_2COOEt$ | - | $CH_2COOEt$ | - | H |
| H | 1 | $CH_2CH_2COOH$ | - | $CH_2COOEt$ | - | H |
| OH | 1 | $CH_2CH_2COOH$ | - | $CH_2COOEt$ | - | H |

TABLE 3

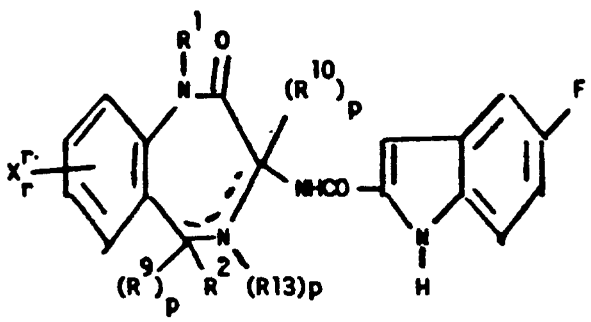

| $X^1$ | r | $R^1$ | $(R^9)_p$ | $R^2$ | $(R^{13})_p$ | $(R^{10})_p$ |
|---|---|---|---|---|---|---|
| H | 1 | H | - | Ph | - | H |
| Cl | 1 | H | - | Ph | - | H |
| F | 1 | H | - | Ph | - | H |
| $CF_3$ | 1 | H | - | Ph | - | H |
| OH | 1 | H | - | Ph | - | H |
| $NO_2$ | 1 | H | - | Ph | - | H |
| H | 1 | $CH_3$ | - | Ph | - | H |
| Cl | 1 | $CH_3$ | - | Ph | - | H |
| F | 1 | $CH_3$ | - | Ph | - | H |
| $CF_3$ | 1 | $CH_3$ | - | Ph | - | H |
| OH | 1 | $CH_3$ | - | Ph | - | H |
| $NO_2$ | 1 | $CH_3$ | - | Ph | - | H |
| H | 1 | $CH_2COOH$ | - | Ph | - | H |
| Cl | 1 | $CH_2COOH$ | - | Ph | - | H |
| F | 1 | $CH_2COOH$ | - | Ph | - | H |
| $CF_3$ | 1 | $CH_2COOH$ | - | Ph | - | H |
| OH | 1 | $CH_2COOH$ | - | Ph | - | H |
| $NO_2$ | 1 | $CH_2COOH$ | - | Ph | - | H |
| H | 1 | $CH_2CH_3$ | - | Ph | - | H |
| OH | 1 | $CH_2CH_3$ | - | Ph | - | H |

TABLE 3 (cont'd)

| $X^1$ | r | $R^1$ | $(R^9)_p$ | $R^2$ | $(R^{13})_p$ | $(R^{10})_p$ |
|---|---|---|---|---|---|---|
| H | 1 | $CH_2COOEt$ | - | Ph | - | H |
| OH | 1 | $CH_2COOEt$ | - | Ph | - | H |
| H | 1 | $CH_2CH_2COOH$ | - | Ph | - | H |
| OH | 1 | $CH_2CH_2COOH$ | - | Ph | - | H |
| H | 1 | H | - | o-F-Ph | - | H |
| Cl | 1 | H | - | o-F-Ph | - | H |
| F | 1 | H | - | o-F-Ph | - | H |
| $CF_3$ | 1 | H | - | o-F-Ph | - | H |
| OH | 1 | H | - | o-F-Ph | - | H |
| $NO_2$ | 1 | H | - | o-F-Ph | - | H |
| H | 1 | $CH_3$ | - | o-F-Ph | - | H |
| Cl | 1 | $CH_3$ | - | o-F-Ph | - | H |
| F | 1 | $CH_3$ | - | o-F-Ph | - | H |
| $CF_3$ | 1 | $CH_3$ | - | o-F-Ph | - | H |
| OH | 1 | $CH_3$ | - | o-F-Ph | - | H |
| $NO_2$ | 1 | $CH_3$ | - | o-F-Ph | - | H |
| H | 1 | $CH_2COOH$ | - | o-F-Ph | - | H |
| Cl | 1 | $CH_2COOH$ | - | o-F-Ph | - | H |
| F | 1 | $CH_2COOH$ | - | o-F-Ph | - | H |
| $CF_3$ | 1 | $CH_2COOH$ | - | o-F-Ph | - | H |
| OH | 1 | $CH_2COOH$ | - | o-F-Ph | - | H |
| $NO_2$ | 1 | $CH_2COOH$ | - | o-F-Ph | - | H |
| H | 1 | $CH_2CH_3$ | - | o-F-Ph | - | H |
| OH | 1 | $CH_2CH_3$ | - | o-F-Ph | - | H |
| H | 1 | $CH_2COOEt$ | - | o-F-Ph | - | H |
| OH | 1 | $CH_2COOEt$ | - | o-F-Ph | - | H |
| H | 1 | $CH_2CH_2COOH$ | - | o-F-Ph | - | H |
| OH | 1 | $CH_2CH_2COOH$ | - | o-F-Ph | - | H |
| H | 1 | H | - | p-Cl-Ph | - | H |
| F | 1 | H | - | p-Cl-Ph | - | H |

TABLE 3 (cont'd)

| $X^1$ | r | $R^1$ | $(R^9)_p$ | $R^2$ | $(R^{13})_p$ | $(R^{10})_p$ |
|---|---|---|---|---|---|---|
| $CF_3$ | 1 | H | - | p-Cl-Ph | - | H |
| OH | 1 | H | - | p-Cl-Ph | - | H |
| H | 1 | $CH_3$ | - | p-Cl-Ph | - | H |
| F | 1 | $CH_3$ | - | p-Cl-Ph | - | H |
| $CF_3$ | 1 | $CH_3$ | - | p-Cl-Ph | - | H |
| OH | 1 | $CH_3$ | - | p-Cl-Ph | - | H |
| H | 1 | $CH_2COOH$ | - | p-Cl-Ph | - | H |
| F | 1 | $CH_2COOH$ | - | p-Cl-Ph | - | H |
| $CF_3$ | 1 | $CH_2COOH$ | - | p-Cl-Ph | - | H |
| OH | 1 | $CH_2COOH$ | - | p-Cl-Ph | - | H |
| H | 1 | $CH_2CH_3$ | - | p-Cl-Ph | - | H |
| H | 1 | $CH_2COOEt$ | - | p-Cl-Ph | - | H |
| H | 1 | $CH_2CH_2COOH$ | - | p-Cl-Ph | - | H |
| H | 1 | H | - | $CH_2COOt$-Bu | - | H |
| Cl | 1 | H | - | $CH_2COOt$-Bu | - | H |
| F | 1 | H | - | $CH_2COOt$-Bu | - | H |
| $CF_3$ | 1 | H | - | $CH_2COOt$-Bu | - | H |
| OH | 1 | H | - | $CH_2COOt$-Bu | - | H |
| $NO_2$ | 1 | H | - | $CH_2COOt$-Bu | - | H |
| H | 1 | $CH_3$ | - | $CH_2COOt$-Bu | - | H |
| Cl | 1 | $CH_3$ | - | $CH_2COOt$-Bu | - | H |
| F | 1 | $CH_3$ | - | $CH_2COOt$-Bu | - | H |
| $CF_3$ | 1 | $CH_3$ | - | $CH_2COOt$-Bu | - | H |
| OH | 1 | $CH_3$ | - | $CH_2COOt$-Bu | - | H |
| $NO_2$ | 1 | $CH_3$ | - | $CH_2COOt$-Bu | - | H |
| H | 1 | $CH_2COOH$ | - | $CH_2COOt$-Bu | - | H |
| Cl | 1 | $CH_2COOH$ | - | $CH_2COOt$-Bu | - | H |
| F | 1 | $CH_2COOH$ | - | $CH_2COOt$-Bu | - | H |
| $CF_3$ | 1 | $CH_2COOH$ | - | $CH_2COOt$-Bu | - | H |
| OH | 1 | $CH_2COOH$ | - | $CH_2COOt$-Bu | - | H |

TABLE 3 (cont'd)

| $X^1$ | r | $R^1$ | $(R^9)_p$ | $R^2$ | $(R^{13})_p$ | $(R^{10})_p$ |
|---|---|---|---|---|---|---|
| $NO_2$ | 1 | $CH_2COOH$ | - | $CH_2COOt$-Bu | - | H |
| H | 1 | $CH_2CH_3$ | - | $CH_2COOt$-Bu | - | H |
| OH | 1 | $CH_2CH_3$ | - | $CH_2COOt$-Bu | - | H |
| H | 1 | $CH_2COOEt$ | - | $CH_2COOt$-Bu | - | H |
| OH | 1 | $CH_2COOEt$ | - | $CH_2COOt$-Bu | - | H |
| H | 1 | $CH_2CH_2COOH$ | - | $CH_2COOt$-Bu | - | H |
| OH | 1 | $CH_2CH_2COOH$ | - | $CH_2COOt$-Bu | - | H |
| H | 1 | H | - | $CH_2COOEt$ | - | H |
| Cl | 1 | H | - | $CH_2COOEt$ | - | H |
| F | 1 | H | - | $CH_2COOEt$ | - | H |
| $CF_3$ | 1 | H | - | $CH_2COOEt$ | - | H |
| OH | 1 | H | - | $CH_2COOEt$ | - | H |
| $NO_2$ | 1 | H | - | $CH_2COOEt$ | - | H |
| H | 1 | $CH_3$ | - | $CH_2COOEt$ | - | H |
| Cl | 1 | $CH_3$ | - | $CH_2COOEt$ | - | H |
| F | 1 | $CH_3$ | - | $CH_2COOEt$ | - | H |
| $CF_3$ | 1 | $CH_3$ | - | $CH_2COOEt$ | - | H |
| OH | 1 | $CH_3$ | - | $CH_2COOEt$ | - | H |
| $NO_2$ | 1 | $CH_3$ | - | $CH_2COOEt$ | - | H |
| H | 1 | $CH_2COOH$ | - | $CH_2COOEt$ | - | H |
| Cl | 1 | $CH_2COOH$ | - | $CH_2COOEt$ | - | H |
| F | 1 | $CH_2COOH$ | - | $CH_2COOEt$ | - | H |
| $CF_3$ | 1 | $CH_2COOH$ | - | $CH_2COOEt$ | - | H |
| OH | 1 | $CH_2COOH$ | - | $CH_2COOEt$ | - | H |
| $NO_2$ | 1 | $CH_2COOH$ | - | $CH_2COOEt$ | - | H |
| H | 1 | $CH_2CH_3$ | - | $CH_2COOEt$ | - | H |
| OH | 1 | $CH_2CH_3$ | - | $CH_2COOEt$ | - | H |
| H | 1 | $CH_2COOEt$ | - | $CH_2COOEt$ | - | H |
| OH | 1 | $CH_2COOEt$ | - | $CH_2COOEt$ | - | H |
| H | 1 | $CH_2CH_2COOH$ | - | $CH_2COOEt$ | - | H |
| OH | 1 | $CH_2CH_2COOH$ | - | $CH_2COOEt$ | - | H |

TABLE 4

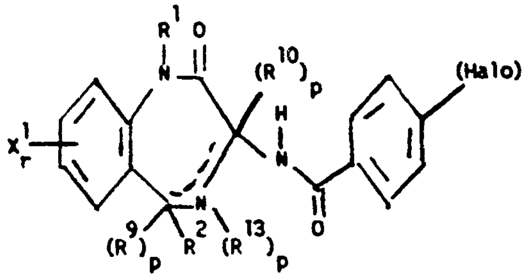

| $X^1$ | r | $R^1$ | $(R^9)_p$ | $R^2$ | $(R^{13})_p$ | $(R^{10})_p$ |
|---|---|---|---|---|---|---|
| H | 1 | H | - | Ph | - | H |
| Cl | 1 | H | - | Ph | - | H |
| F | 1 | H | - | Ph | - | H |
| $CF_3$ | 1 | H | - | Ph | - | H |
| OH | 1 | H | - | Ph | - | H |
| $NO_2$ | 1 | H | - | Ph | - | H |
| H | 1 | $CH_3$ | - | Ph | - | H |
| Cl | 1 | $CH_3$ | - | Ph | - | H |
| F | 1 | $CH_3$ | - | Ph | - | H |
| $CF_3$ | 1 | $CH_3$ | - | Ph | - | H |
| OH | 1 | $CH_3$ | - | Ph | - | H |
| $NO_2$ | 1 | $CH_3$ | - | Ph | - | H |
| H | 1 | $CH_2COOH$ | - | Ph | - | H |
| Cl | 1 | $CH_2COOH$ | - | Ph | - | H |
| F | 1 | $CH_2COOH$ | - | Ph | - | H |
| $CF_3$ | 1 | $CH_2COOH$ | - | Ph | - | H |
| OH | 1 | $CH_2COOH$ | - | Ph | - | H |
| $NO_2$ | 1 | $CH_2COOH$ | - | Ph | - | H |
| H | 1 | $CH_2CH_3$ | - | Ph | - | H |
| OH | 1 | $CH_2CH_3$ | - | Ph | - | H |

TABLE 4 (cont'd)

| $X^1$ | r | $R^1$ | $(R^9)_p$ | $R^2$ | $(R^{13})_p$ | $(R^{10})_p$ |
|---|---|---|---|---|---|---|
| H | 1 | $CH_2COOEt$ | - | Ph | - | H |
| OH | 1 | $CH_2COOEt$ | - | Ph | - | H |
| H | 1 | $CH_2CH_2COOH$ | - | Ph | - | H |
| OH | 1 | $CH_2CH_2COOH$ | - | Ph | - | H |
| H | 1 | H | - | o-F-Ph | - | H |
| Cl | 1 | H | - | o-F-Ph | - | H |
| F | 1 | H | - | o-F-Ph | - | H |
| $CF_3$ | 1 | H | - | o-F-Ph | - | H |
| OH | 1 | H | - | o-F-Ph | - | H |
| $NO_2$ | 1 | H | - | o-F-Ph | - | H |
| H | 1 | $CH_3$ | - | o-F-Ph | - | H |
| Cl | 1 | $CH_3$ | - | o-F-Ph | - | H |
| F | 1 | $CH_3$ | - | o-F-Ph | - | H |
| $CF_3$ | 1 | $CH_3$ | - | o-F-Ph | - | H |
| OH | 1 | $CH_3$ | - | o-F-Ph | - | H |
| $NO_2$ | 1 | $CH_3$ | - | o-F-Ph | - | H |
| H | 1 | $CH_2COOH$ | - | o-F-Ph | - | H |
| Cl | 1 | $CH_2COOH$ | - | o-F-Ph | - | H |
| F | 1 | $CH_2COOH$ | - | o-F-Ph | - | H |
| $CF_3$ | 1 | $CH_2COOH$ | - | o-F-Ph | - | H |
| OH | 1 | $CH_2COOH$ | - | o-F-Ph | - | H |
| $NO_2$ | 1 | $CH_2COOH$ | - | o-F-Ph | - | H |
| H | 1 | $CH_2CH_3$ | - | o-F-Ph | - | H |
| OH | 1 | $CH_2CH_3$ | - | o-F-Ph | - | H |
| H | 1 | $CH_2COOEt$ | - | o-F-Ph | - | H |
| OH | 1 | $CH_2COOEt$ | - | o-F-Ph | - | H |
| H | 1 | $CH_2CH_2COOH$ | - | o-F-Ph | - | H |
| OH | 1 | $CH_2CH_2COOH$ | - | o-F-Ph | - | H |
| H | 1 | H | - | p-Cl-Ph | - | H |
| F | 1 | H | - | p-Cl-Ph | - | H |

TABLE 4 (cont'd)

| $X^1$ | r | $R^1$ | $(R^9)_p$ | $R^2$ | $(R^{13})_p$ | $(R^{10})_p$ |
|---|---|---|---|---|---|---|
| $CF_3$ | 1 | H | - | p-Cl-Ph | - | H |
| OH | 1 | H | - | p-Cl-Ph | - | H |
| H | 1 | $CH_3$ | - | p-Cl-Ph | - | H |
| F | 1 | $CH_3$ | - | p-Cl-Ph | - | H |
| $CF_3$ | 1 | $CH_3$ | - | p-Cl-Ph | - | H |
| OH | 1 | $CH_3$ | - | p-Cl-Ph | - | H |
| H | 1 | $CH_2COOH$ | - | p-Cl-Ph | - | H |
| F | 1 | $CH_2COOH$ | - | p-Cl-Ph | - | H |
| $CF_3$ | 1 | $CH_2COOH$ | - | p-Cl-Ph | - | H |
| OH | 1 | $CH_2COOH$ | - | p-Cl-Ph | - | H |
| H | 1 | $CH_2CH_3$ | - | p-Cl-Ph | - | H |
| H | 1 | $CH_2COOEt$ | - | p-Cl-Ph | - | H |
| H | 1 | $CH_2CH_2COOH$ | - | p-Cl-Ph | - | H |
| H | 1 | H | - | $CH_2COOt$-Bu | - | H |
| Cl | 1 | H | - | $CH_2COOt$-Bu | - | H |
| F | 1 | H | - | $CH_2COOt$-Bu | - | H |
| $CF_3$ | 1 | H | - | $CH_2COOt$-Bu | - | H |
| OH | 1 | H | - | $CH_2COOt$-Bu | - | H |
| $NO_2$ | 1 | H | - | $CH_2COOt$-Bu | - | H |
| H | 1 | $CH_3$ | - | $CH_2COOt$-Bu | - | H |
| Cl | 1 | $CH_3$ | - | $CH_2COOt$-Bu | - | H |
| F | 1 | $CH_3$ | - | $CH_2COOt$-Bu | - | H |
| $CF_3$ | 1 | $CH_3$ | - | $CH_2COOt$-Bu | - | H |
| OH | 1 | $CH_3$ | - | $CH_2COOt$-Bu | - | H |
| $NO_2$ | 1 | $CH_3$ | - | $CH_2COOt$-Bu | - | H |
| H | 1 | $CH_2COOH$ | - | $CH_2COOt$-Bu | - | H |
| Cl | 1 | $CH_2COOH$ | - | $CH_2COOt$-Bu | - | H |
| F | 1 | $CH_2COOH$ | - | $CH_2COOt$-Bu | - | H |
| $CF_3$ | 1 | $CH_2COOH$ | - | $CH_2COOt$-Bu | - | H |
| OH | 1 | $CH_2COOH$ | - | $CH_2COOt$-Bu | - | H |

TABLE 4 (cont'd)

| $X^1$ | r | $R^1$ | $(R^9)_p$ | $R^2$ | $(R^{13})_p$ | $(R^{10})_p$ |
|---|---|---|---|---|---|---|
| $NO_2$ | 1 | $CH_2COOH$ | - | $CH_2COOt$-Bu | - | H |
| H | 1 | $CH_2CH_3$ | - | $CH_2COOt$-Bu | - | H |
| OH | 1 | $CH_2CH_3$ | - | $CH_2COOt$-Bu | - | H |
| H | 1 | $CH_2COOEt$ | - | $CH_2COOt$-Bu | - | H |
| OH | 1 | $CH_2COOEt$ | - | $CH_2COOt$-Bu | - | H |
| H | 1 | $CH_2CH_2COOH$ | - | $CH_2COOt$-Bu | - | H |
| OH | 1 | $CH_2CH_2COOH$ | - | $CH_2COOt$-Bu | - | H |
| H | 1 | H | - | $CH_2COOEt$ | - | H |
| Cl | 1 | H | - | $CH_2COOEt$ | - | H |
| F | 1 | H | - | $CH_2COOEt$ | - | H |
| $CF_3$ | 1 | H | - | $CH_2COOEt$ | - | H |
| OH | 1 | H | - | $CH_2COOEt$ | - | H |
| $NO_2$ | 1 | H | - | $CH_2COOEt$ | - | H |
| H | 1 | $CH_3$ | - | $CH_2COOEt$ | - | H |
| Cl | 1 | $CH_3$ | - | $CH_2COOEt$ | - | H |
| F | 1 | $CH_3$ | - | $CH_2COOEt$ | - | H |
| $CF_3$ | 1 | $CH_3$ | - | $CH_2COOEt$ | - | H |
| OH | 1 | $CH_3$ | - | $CH_2COOEt$ | - | H |
| $NO_2$ | 1 | $CH_3$ | - | $CH_2COOEt$ | - | H |
| H | 1 | $CH_2COOH$ | - | $CH_2COOEt$ | - | H |
| Cl | 1 | $CH_2COOH$ | - | $CH_2COOEt$ | - | H |
| F | 1 | $CH_2COOH$ | - | $CH_2COOEt$ | - | H |
| $CF_3$ | 1 | $CH_2COOH$ | - | $CH_2COOEt$ | - | H |
| OH | 1 | $CH_2COOH$ | - | $CH_2COOEt$ | - | H |
| $NO_2$ | 1 | $CH_2COOH$ | - | $CH_2COOEt$ | - | H |
| H | 1 | $CH_2CH_3$ | - | $CH_2COOEt$ | - | H |
| OH | 1 | $CH_2CH_3$ | - | $CH_2COOEt$ | - | H |
| H | 1 | $CH_2COOEt$ | - | $CH_2COOEt$ | - | H |
| OH | 1 | $CH_2COOEt$ | - | $CH_2COOEt$ | - | H |
| H | 1 | $CH_2CH_2COOH$ | - | $CH_2COOEt$ | - | H |
| OH | 1 | $CH_2CH_2COOH$ | - | $CH_2COOEt$ | - | H |

TABLE 5

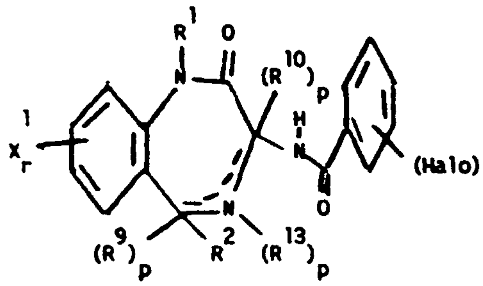

| $X^1$ | r | $R^1$ | $(R^9)_p$ | $R^2$ | $(R^{13})_p$ | $(R^{10})_p$ |
|---|---|---|---|---|---|---|
| H | 1 | H | - | Ph | - | H |
| Cl | 1 | H | - | Ph | - | H |
| F | 1 | H | - | Ph | - | H |
| $CF_3$ | 1 | H | - | Ph | - | H |
| OH | 1 | H | - | Ph | - | H |
| $NO_2$ | 1 | H | - | Ph | - | H |
| H | 1 | $CH_3$ | - | Ph | - | H |
| Cl | 1 | $CH_3$ | - | Ph | - | H |
| F | 1 | $CH_3$ | - | Ph | - | H |
| $CF_3$ | 1 | $CH_3$ | - | Ph | - | H |
| OH | 1 | $CH_3$ | - | Ph | - | H |
| $NO_2$ | 1 | $CH_3$ | - | Ph | - | H |
| H | 1 | $CH_2COOH$ | - | Ph | - | H |
| Cl | 1 | $CH_2COOH$ | - | Ph | - | H |
| F | 1 | $CH_2COOH$ | - | Ph | - | H |
| $CF_3$ | 1 | $CH_2COOH$ | - | Ph | - | H |
| OH | 1 | $CH_2COOH$ | - | Ph | - | H |
| $NO_2$ | 1 | $CH_2COOH$ | - | Ph | - | H |
| H | 1 | $CH_2CH_3$ | - | Ph | - | H |

TABLE 5 (cont'd)

| $X^1$ | r | $R^1$ | $(R^9)_p$ | $R^2$ | $(R^{13})_p$ | $(R^{10})_p$ |
|---|---|---|---|---|---|---|
| OH | 1 | $CH_2CH_3$ | - | Ph | - | H |
| H | 1 | $CH_2COOEt$ | - | Ph | - | H |
| OH | 1 | $CH_2COOEt$ | - | Ph | - | H |
| H | 1 | $CH_2CH_2COOH$ | - | Ph | - | H |
| OH | 1 | $CH_2CH_2COOH$ | - | Ph | - | H |
| H | 1 | H | - | o-F-Ph | - | H |
| Cl | 1 | H | - | o-F-Ph | - | H |
| F | 1 | H | - | o-F-Ph | - | H |
| $CF_3$ | 1 | H | - | o-F-Ph | - | H |
| OH | 1 | H | - | o-F-Ph | - | H |
| $NO_2$ | 1 | H | - | o-F-Ph | - | H |
| H | 1 | $CH_3$ | - | o-F-Ph | - | H |
| Cl | 1 | $CH_3$ | - | o-F-Ph | - | H |
| F | 1 | $CH_3$ | - | o-F-Ph | - | H |
| $CF_3$ | 1 | $CH_3$ | - | o-F-Ph | - | H |
| OH | 1 | $CH_3$ | - | o-F-Ph | - | H |
| $NO_2$ | 1 | $CH_3$ | - | o-F-Ph | - | H |
| H | 1 | $CH_2COOH$ | - | o-F-Ph | - | H |
| Cl | 1 | $CH_2COOH$ | - | o-F-Ph | - | H |
| F | 1 | $CH_2COOH$ | - | o-F-Ph | - | H |
| $CF_3$ | 1 | $CH_2COOH$ | - | o-F-Ph | - | H |
| OH | 1 | $CH_2COOH$ | - | o-F-Ph | - | H |
| $NO_2$ | 1 | $CH_2COOH$ | - | o-F-Ph | - | H |
| H | 1 | $CH_2CH_3$ | - | o-F-Ph | - | H |
| OH | 1 | $CH_2CH_3$ | - | o-F-Ph | - | H |
| H | 1 | $CH_2COOEt$ | - | o-F-Ph | - | H |
| OH | 1 | $CH_2COOEt$ | - | o-F-Ph | - | H |
| H | 1 | $CH_2CH_2COOH$ | - | o-F-Ph | - | H |
| OH | 1 | $CH_2CH_2COOH$ | - | o-F-Ph | - | H |

TABLE 5 (cont'd)

| $X^1$ | r | $R^1$ | $(R^9)_p$ | $R^2$ | $(R^{13})_p$ | $(R^{10})_p$ |
|---|---|---|---|---|---|---|
| H | 1 | H | - | o-Cl-Ph | - | H |
| F | 1 | H | - | p-Cl-Ph | - | H |
| $CF_3$ | 1 | H | - | p-Cl-Ph | - | H |
| OH | 1 | H | - | p-Cl-Ph | - | H |
| H | 1 | $CH_3$ | - | p-Cl-Ph | - | H |
| F | 1 | $CH_3$ | - | p-Cl-Ph | - | H |
| $CF_3$ | 1 | $CH_3$ | - | p-Cl-Ph | - | H |
| OH | 1 | $CH_3$ | - | p-Cl-Ph | - | H |
| H | 1 | $CH_2COOH$ | - | p-Cl-Ph | - | H |
| F | 1 | $CH_2COOH$ | - | p-Cl-Ph | - | H |
| $CF_3$ | 1 | $CH_2COOH$ | - | p-Cl-Ph | - | H |
| OH | 1 | $CH_2COOH$ | - | p-Cl-Ph | - | H |
| H | 1 | $CH_2CH_3$ | - | p-Cl-Ph | - | H |
| H | 1 | $CH_2COOEt$ | - | p-Cl-Ph | - | H |
| H | 1 | $CH_2CH_2COOH$ | - | p-Cl-Ph | - | H |
| H | 1 | H | - | $CH_2COOt$-Bu | - | H |
| Cl | 1 | H | - | $CH_2COOt$-Bu | - | H |
| F | 1 | H | - | $CH_2COOt$-Bu | - | H |
| $CF_3$ | 1 | H | - | $CH_2COOt$-Bu | - | H |
| OH | 1 | H | - | $CH_2COOt$-Bu | - | H |
| $NO_2$ | 1 | H | - | $CH_2COOt$-Bu | - | H |
| H | 1 | $CH_3$ | - | $CH_2COOt$-Bu | - | H |
| Cl | 1 | $CH_3$ | - | $CH_2COOt$-Bu | - | H |
| F | 1 | $CH_3$ | - | $CH_2COOt$-Bu | - | H |
| $CF_3$ | 1 | $CH_3$ | - | $CH_2COOt$-Bu | - | H |
| OH | 1 | $CH_3$ | - | $CH_2COOt$-Bu | - | H |
| $NO_2$ | 1 | $CH_3$ | - | $CH_2COOt$-Bu | - | H |
| H | 1 | $CH_2COOH$ | - | $CH_2COOt$-Bu | - | H |
| Cl | 1 | $CH_2COOH$ | - | $CH_2COOt$-Bu | - | H |
| F | 1 | $CH_2COOH$ | - | $CH_2COOt$-Bu | - | H |

TABLE 5 (cont'd)

| $X^1$ | r | $R^1$ | $(R^9)_p$ | $R^2$ | $(R^{13})_p$ | $(R^{10})_p$ |
|---|---|---|---|---|---|---|
| $CF_3$ | 1 | $CH_2COOH$ | - | $CH_2COOt$-Bu | - | H |
| OH | 1 | $CH_2COOH$ | - | $CH_2COOt$-Bu | - | H |
| $NO_2$ | 1 | $CH_2COOH$ | - | $CH_2COOt$-Bu | - | H |
| H | 1 | $CH_2CH_3$ | - | $CH_2COOt$-Bu | - | H |
| OH | 1 | $CH_2CH_3$ | - | $CH_2COOt$-Bu | - | H |
| H | 1 | $CH_2COOEt$ | - | $CH_2COOt$-Bu | - | H |
| OH | 1 | $CH_2COOEt$ | - | $CH_2COOt$-Bu | - | H |
| H | 1 | $CH_2CH_2COOH$ | - | $CH_2COOt$-Bu | - | H |
| OH | 1 | $CH_2CH_2COOH$ | - | $CH_2COOt$-Bu | - | H |
| H | 1 | H | - | $CH_2COOEt$ | - | H |
| Cl | 1 | H | - | $CH_2COOEt$ | - | H |
| F | 1 | H | - | $CH_2COOEt$ | - | H |
| $CF_3$ | 1 | H | - | $CH_2COOEt$ | - | H |
| OH | 1 | H | - | $CH_2COOEt$ | - | H |
| $NO_2$ | 1 | H | - | $CH_2COOEt$ | - | H |
| H | 1 | $CH_3$ | - | $CH_2COOEt$ | - | H |
| Cl | 1 | $CH_3$ | - | $CH_2COOEt$ | - | H |
| F | 1 | $CH_3$ | - | $CH_2COOEt$ | - | H |
| $CF_3$ | 1 | $CH_3$ | - | $CH_2COOEt$ | - | H |
| OH | 1 | $CH_3$ | - | $CH_2COOEt$ | - | H |
| $NO_2$ | 1 | $CH_3$ | - | $CH_2COOEt$ | - | H |
| H | 1 | $CH_2COOH$ | - | $CH_2COOEt$ | - | H |
| Cl | 1 | $CH_2COOH$ | - | $CH_2COOEt$ | - | H |
| F | 1 | $CH_2COOH$ | - | $CH_2COOEt$ | - | H |
| $CF_3$ | 1 | $CH_2COOH$ | - | $CH_2COOEt$ | - | H |
| OH | 1 | $CH_2COOH$ | - | $CH_2COOEt$ | - | H |
| $NO_2$ | 1 | $CH_2COOH$ | - | $CH_2COOEt$ | - | H |
| H | 1 | $CH_2CH_3$ | - | $CH_2COOEt$ | - | H |
| OH | 1 | $CH_2CH_3$ | - | $CH_2COOEt$ | - | H |
| H | 1 | $CH_2COOEt$ | - | $CH_2COOEt$ | - | H |

TABLE 5 (cont'd)

| $X^1$ | r | $R^1$ | $(R^9)_p$ | $R^2$ | $(R^{13})_p$ | $(R^{10})_p$ |
|---|---|---|---|---|---|---|
| OH | 1 | $CH_2COOEt$ | - | $CH_2COOEt$ | - | H |
| H | 1 | $CH_2CH_2COOH$ | - | $CH_2COOEt$ | - | H |
| OH | 1 | $CH_2CH_2COOH$ | - | $CH_2COOEt$ | - | H |

TABLE 6

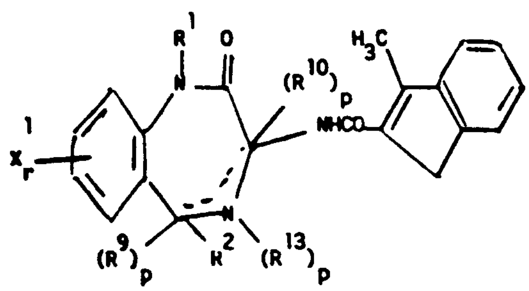

| $X^1$ | r | $R^1$ | $(R^9)_p$ | $R^2$ | $(R^{13})_p$ | $(R^{10})_p$ |
|---|---|---|---|---|---|---|
| H | 1 | H | - | Ph | - | H |
| Cl | 1 | H | - | Ph | - | H |
| F | 1 | H | - | Ph | - | H |
| $CF_3$ | 1 | H | - | Ph | - | H |
| OH | 1 | H | - | Ph | - | H |
| $NO_2$ | 1 | H | - | Ph | - | H |
| H | 1 | $CH_3$ | - | Ph | - | H |
| Cl | 1 | $CH_3$ | - | Ph | - | H |
| F | 1 | $CH_3$ | - | Ph | - | H |
| $CF_3$ | 1 | $CH_3$ | - | Ph | - | H |
| OH | 1 | $CH_3$ | - | Ph | - | H |
| $NO_2$ | 1 | $CH_3$ | - | Ph | - | H |
| H | 1 | $CH_2COOH$ | - | Ph | - | H |
| Cl | 1 | $CH_2COOH$ | - | Ph | - | H |
| F | 1 | $CH_2COOH$ | - | Ph | - | H |
| $CF_3$ | 1 | $CH_2COOH$ | - | Ph | - | H |
| OH | 1 | $CH_2COOH$ | - | Ph | - | H |
| $NO_2$ | 1 | $CH_2COOH$ | - | Ph | - | H |
| H | 1 | $CH_2CH_3$ | - | Ph | - | H |

TABLE 6 (cont'd)

| $X^1$ | r | $R^1$ | $(R^9)_p$ | $R^2$ | $(R^{13})_p$ | $(R^{10})_p$ |
|---|---|---|---|---|---|---|
| OH | 1 | $CH_2CH_3$ | - | Ph | - | H |
| H | 1 | $CH_2COOEt$ | - | Ph | - | H |
| OH | 1 | $CH_2COOEt$ | - | Ph | - | H |
| H | 1 | $CH_2CH_2COOH$ | - | Ph | - | H |
| OH | 1 | $CH_2CH_2COOH$ | - | Ph | - | H |
| H | 1 | H | - | o-F-Ph | - | H |
| Cl | 1 | H | - | o-F-Ph | - | H |
| F | 1 | H | - | o-F-Ph | - | H |
| $CF_3$ | 1 | H | - | o-F-Ph | - | H |
| OH | 1 | H | - | o-F-Ph | - | H |
| $NO_2$ | 1 | H | - | o-F-Ph | - | H |
| H | 1 | $CH_3$ | - | o-F-Ph | - | H |
| Cl | 1 | $CH_3$ | - | o-F-Ph | - | H |
| F | 1 | $CH_3$ | - | o-F-Ph | - | H |
| $CF_3$ | 1 | $CH_3$ | - | o-F-Ph | - | H |
| OH | 1 | $CH_3$ | - | o-F-Ph | - | H |
| $NO_2$ | 1 | $CH_3$ | - | o-F-Ph | - | H |
| H | 1 | $CH_2COOH$ | - | o-F-Ph | - | H |
| Cl | 1 | $CH_2COOH$ | - | o-F-Ph | - | H |
| F | 1 | $CH_2COOH$ | - | o-F-Ph | - | H |
| $CF_3$ | 1 | $CH_2COOH$ | - | o-F-Ph | - | H |
| OH | 1 | $CH_2COOH$ | - | o-F-Ph | - | H |
| $NO_2$ | 1 | $CH_2COOH$ | - | o-F-Ph | - | H |
| H | 1 | $CH_2CH_3$ | - | o-F-Ph | - | H |
| OH | 1 | $CH_2CH_3$ | - | o-F-Ph | - | H |
| H | 1 | $CH_2COOEt$ | - | o-F-Ph | - | H |
| OH | 1 | $CH_2COOEt$ | - | o-F-Ph | - | H |
| H | 1 | $CH_2CH_2COOH$ | - | o-F-Ph | - | H |
| OH | 1 | $CH_2CH_2COOH$ | - | o-F-Ph | - | H |

TABLE 6 (cont'd)

| $X^1$ | r | $R^1$ | $(R^9)_p$ | $R^2$ | $(R^{13})_p$ | $(R^{10})_p$ |
|---|---|---|---|---|---|---|
| H | 1 | H | - | p-Cl-Ph | - | H |
| F | 1 | H | - | p-Cl-Ph | - | H |
| $CF_3$ | 1 | H | - | p-Cl-Ph | - | H |
| OH | 1 | H | - | p-Cl-Ph | - | H |
| H | 1 | $CH_3$ | - | p-Cl-Ph | - | H |
| F | 1 | $CH_3$ | - | p-Cl-Ph | - | H |
| $CF_3$ | 1 | $CH_3$ | - | p-Cl-Ph | - | H |
| OH | 1 | $CH_3$ | - | p-Cl-Ph | - | H |
| H | 1 | $CH_2COOH$ | - | p-Cl-Ph | - | H |
| F | 1 | $CH_2COOH$ | - | p-Cl-Ph | - | H |
| $CF_3$ | 1 | $CH_2COOH$ | - | p-Cl-Ph | - | H |
| OH | 1 | $CH_2COOH$ | - | p-Cl-Ph | - | H |
| H | 1 | $CH_2CH_3$ | - | p-Cl-Ph | - | H |
| H | 1 | $CH_2COOEt$ | - | p-Cl-Ph | - | H |
| H | 1 | $CH_2CH_2COOH$ | - | p-Cl-Ph | - | H |
| H | 1 | H | - | $CH_2COOt$-Bu | - | H |
| Cl | 1 | H | - | $CH_2COOt$-Bu | - | H |
| F | 1 | H | - | $CH_2COOt$-Bu | - | H |
| $CF_3$ | 1 | H | - | $CH_2COOt$-Bu | - | H |
| OH | 1 | H | - | $CH_2COOt$-Bu | - | H |
| $NO_2$ | 1 | H | - | $CH_2COOt$-Bu | - | H |
| H | 1 | $CH_3$ | - | $CH_2COOt$-Bu | - | H |
| Cl | 1 | $CH_3$ | - | $CH_2COOt$-Bu | - | H |
| F | 1 | $CH_3$ | - | $CH_2COOt$-Bu | - | H |
| $CF_3$ | 1 | $CH_3$ | - | $CH_2COOt$-Bu | - | H |
| OH | 1 | $CH_3$ | - | $CH_2COOt$-Bu | - | H |
| $NO_2$ | 1 | $CH_3$ | - | $CH_2COOt$-Bu | - | H |
| H | 1 | $CH_2COOH$ | - | $CH_2COOt$-Bu | - | H |
| Cl | 1 | $CH_2COOH$ | - | $CH_2COOt$-Bu | - | H |
| F | 1 | $CH_2COOH$ | - | $CH_2COOt$-Bu | - | H |

TABLE 6 (cont'd)

| $X^1$ | r | $R^1$ | $(R^9)_p$ | $R^2$ | $(R^{13})_p$ | $(R^{10})_p$ |
|---|---|---|---|---|---|---|
| $CF_3$ | 1 | $CH_2COOH$ | - | $CH_2COOt$-Bu | - | H |
| OH | 1 | $CH_2COOH$ | - | $CH_2COOt$-Bu | - | H |
| $NO_2$ | 1 | $CH_2COOH$ | - | $CH_2COOt$-Bu | - | H |
| H | 1 | $CH_2CH_3$ | - | $CH_2COOt$-Bu | - | H |
| OH | 1 | $CH_2CH_3$ | - | $CH_2COOt$-Bu | - | H |
| H | 1 | $CH_2COOEt$ | - | $CH_2COOt$-Bu | - | H |
| OH | 1 | $CH_2COOEt$ | - | $CH_2COOt$-Bu | - | H |
| H | 1 | $CH_2CH_2COOH$ | - | $CH_2COOt$-Bu | - | H |
| OH | 1 | $CH_2CH_2COOH$ | - | $CH_2COOt$-Bu | - | H |
| H | 1 | H | - | $CH_2COOEt$ | - | H |
| Cl | 1 | H | - | $CH_2COOEt$ | - | H |
| F | 1 | H | - | $CH_2COOEt$ | - | H |
| $CF_3$ | 1 | H | - | $CH_2COOEt$ | - | H |
| OH | 1 | H | - | $CH_2COOEt$ | - | H |
| $NO_2$ | 1 | H | - | $CH_2COOEt$ | - | H |
| H | 1 | $CH_3$ | - | $CH_2COOEt$ | - | H |
| Cl | 1 | $CH_3$ | - | $CH_2COOEt$ | - | H |
| F | 1 | $CH_3$ | - | $CH_2COOEt$ | - | H |
| $CF_3$ | 1 | $CH_3$ | - | $CH_2COOEt$ | - | H |
| OH | 1 | $CH_3$ | - | $CH_2COOEt$ | - | H |
| $NO_2$ | 1 | $CH_3$ | - | $CH_2COOEt$ | - | H |
| H | 1 | $CH_2COOH$ | - | $CH_2COOEt$ | - | H |
| Cl | 1 | $CH_2COOH$ | - | $CH_2COOEt$ | - | H |
| F | 1 | $CH_2COOH$ | - | $CH_2COOEt$ | - | H |
| $CF_3$ | 1 | $CH_2COOH$ | - | $CH_2COOEt$ | - | H |
| OH | 1 | $CH_2COOH$ | - | $CH_2COOEt$ | - | H |
| $NO_2$ | 1 | $CH_2COOH$ | - | $CH_2COOEt$ | - | H |
| H | 1 | $CH_2CH_3$ | - | $CH_2COOEt$ | - | H |
| OH | 1 | $CH_2CH_3$ | - | $CH_2COOEt$ | - | H |
| H | 1 | $CH_2COOEt$ | - | $CH_2COOEt$ | - | H |

TABLE 6 (cont'd)

| $X^1$ | r | $R^1$ | $(R^9)_p$ | $R^2$ | $(R^{13})_p$ | $(R^{10})_p$ |
|---|---|---|---|---|---|---|
| OH | 1 | $CH_2COOEt$ | - | $CH_2COOEt$ | - | H |
| H | 1 | $CH_2CH_2COOH$ | - | $CH_2COOEt$ | - | H |
| OH | 1 | $CH_2CH_2COOH$ | - | $CH_2COOEt$ | - | H |

TABLE 7

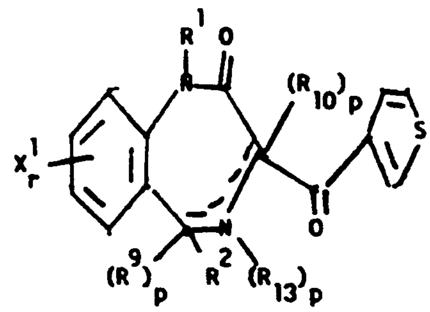

| $X^1$ | r | $R^1$ | $(R^9)_p$ | $R^2$ | $(R^{13})_p$ | $(R^{10})_p$ |
|---|---|---|---|---|---|---|
| H | 1 | H | - | Ph | - | H |
| Cl | 1 | H | - | Ph | - | H |
| F | 1 | H | - | Ph | - | H |
| $CF_3$ | 1 | H | - | Ph | - | H |
| OH | 1 | H | - | Ph | - | H |
| $NO_2$ | 1 | H | - | Ph | - | H |
| H | 1 | $CH_3$ | - | Ph | - | H |
| Cl | 1 | $CH_3$ | - | Ph | - | H |
| F | 1 | $CH_3$ | - | Ph | - | H |
| $CF_3$ | 1 | $CH_3$ | - | Ph | - | H |
| OH | 1 | $CH_3$ | - | Ph | - | H |
| $NO_2$ | 1 | $CH_3$ | - | Ph | - | H |
| H | 1 | $CH_2COOH$ | - | Ph | - | H |
| Cl | 1 | $CH_2COOH$ | - | Ph | - | H |
| F | 1 | $CH_2COOH$ | - | Ph | - | H |
| $CF_3$ | 1 | $CH_2COOH$ | - | Ph | - | H |
| OH | 1 | $CH_2COOH$ | - | Ph | - | H |
| $NO_2$ | 1 | $CH_2COOH$ | - | Ph | - | H |
| H | 1 | $CH_2CH_3$ | - | Ph | - | H |
| OH | 1 | $CH_2CH_3$ | - | Ph | - | H |
| H | 1 | $CH_2COOEt$ | - | Ph | - | H |

TABLE 7 (cont'd)

| $X^1$ | r | $R^1$ | $(R^9)_p$ | $R^2$ | $(R^{13})_p$ | $(R^{10})_p$ |
|---|---|---|---|---|---|---|
| OH | 1 | $CH_2COOEt$ | - | Ph | - | H |
| H | 1 | $CH_2CH_2COOH$ | - | Ph | - | H |
| OH | 1 | $CH_2CH_2COOH$ | - | Ph | - | H |
| H | 1 | H | - | o-F-Ph | - | H |
| Cl | 1 | H | - | o-F-Ph | - | H |
| F | 1 | H | - | o-F-Ph | - | H |
| $CF_3$ | 1 | H | - | o-F-Ph | - | H |
| OH | 1 | H | - | o-F-Ph | - | H |
| $NO_2$ | 1 | H | - | o-F-Ph | - | H |
| H | 1 | $CH_3$ | - | o-F-Ph | - | H |
| Cl | 1 | $CH_3$ | - | o-F-Ph | - | H |
| F | 1 | $CH_3$ | - | o-F-Ph | - | H |
| $CF_3$ | 1 | $CH_3$ | - | o-F-Ph | - | H |
| OH | 1 | $CH_3$ | - | o-F-Ph | - | H |
| $NO_2$ | 1 | $CH_3$ | - | o-F-Ph | - | H |
| H | 1 | $CH_2COOH$ | - | o-F-Ph | - | H |
| Cl | 1 | $CH_2COOH$ | - | o-F-Ph | - | H |
| F | 1 | $CH_2COOH$ | - | o-F-Ph | - | H |
| $CF_3$ | 1 | $CH_2COOH$ | - | o-F-Ph | - | H |
| OH | 1 | $CH_2COOH$ | - | o-F-Ph | - | H |
| $NO_2$ | 1 | $CH_2COOH$ | - | o-F-Ph | - | H |
| H | 1 | $CH_2CH_3$ | - | o-F-Ph | - | H |
| OH | 1 | $CH_2CH_3$ | - | o-F-Ph | - | H |
| H | 1 | $CH_2COOEt$ | - | o-F-Ph | - | H |
| OH | 1 | $CH_2COOEt$ | - | o-F-Ph | - | H |
| H | 1 | $CH_2CH_2COOH$ | - | o-F-Ph | - | H |
| OH | 1 | $CH_2CH_2COOH$ | - | o-F-Ph | - | H |
| H | 1 | H | - | p-Cl-Ph | - | H |
| F | 1 | H | - | p-Cl-Ph | - | H |

TABLE 7 (cont'd)

| $X^1$ | r | $R^1$ | $(R^9)_p$ | $R^2$ | $(R^{13})_p$ | $(R^{10})_p$ |
|---|---|---|---|---|---|---|
| $CF_3$ | 1 | H | - | p-Cl-Ph | - | H |
| OH | 1 | H | - | p-Cl-Ph | - | H |
| H | 1 | $CH_3$ | - | p-Cl-Ph | - | H |
| F | 1 | $CH_3$ | - | p-Cl-Ph | - | H |
| $CF_3$ | 1 | $CH_3$ | - | p-Cl-Ph | - | H |
| OH | 1 | $CH_3$ | - | p-Cl-Ph | - | H |
| H | 1 | $CH_2COOH$ | - | p-Cl-Ph | - | H |
| F | 1 | $CH_2COOH$ | - | p-Cl-Ph | - | H |
| $CF_3$ | 1 | $CH_2COOH$ | - | p-Cl-Ph | - | H |
| OH | 1 | $CH_2COOH$ | - | p-Cl-Ph | - | H |
| H | 1 | $CH_2CH_3$ | - | p-Cl-Ph | - | H |
| H | 1 | $CH_2COOEt$ | - | p-Cl-Ph | - | H |
| H | 1 | $CH_2CH_2COOH$ | - | p-Cl-Ph | - | H |
| H | 1 | H | - | $CH_2COOt$-Bu | - | H |
| Cl | 1 | H | - | $CH_2COOt$-Bu | - | H |
| F | 1 | H | - | $CH_2COOt$-Bu | - | H |
| $CF_3$ | 1 | H | - | $CH_2COOt$-Bu | - | H |
| OH | 1 | H | - | $CH_2COOt$-Bu | - | H |
| $NO_2$ | 1 | H | - | $CH_2COOt$-Bu | - | H |
| H | 1 | $CH_3$ | - | $CH_2COOt$-Bu | - | H |
| Cl | 1 | $CH_3$ | - | $CH_2COOt$-Bu | - | H |
| F | 1 | $CH_3$ | - | $CH_2COOt$-Bu | - | H |
| $CF_3$ | 1 | $CH_3$ | - | $CH_2COOt$-Bu | - | H |
| OH | 1 | $CH_3$ | - | $CH_2COOt$-Bu | - | H |
| $NO_2$ | 1 | $CH_3$ | - | $CH_2COOt$-Bu | - | H |
| H | 1 | $CH_2COOH$ | - | $CH_2COOt$-Bu | - | H |
| Cl | 1 | $CH_2COOH$ | - | $CH_2COOt$-Bu | - | H |
| F | 1 | $CH_2COOH$ | - | $CH_2COOt$-Bu | - | H |
| $CF_3$ | 1 | $CH_2COOH$ | - | $CH_2COOt$-Bu | - | H |
| OH | 1 | $CH_2COOH$ | - | $CH_2COOt$-Bu | - | H |

TABLE 7 (cont'd)

| $X^1$ | r | $R^1$ | $(R^9)_p$ | $R^2$ | $(R^{13})_p$ | $(R^{10})_p$ |
|---|---|---|---|---|---|---|
| $NO_2$ | 1 | $CH_2COOH$ | - | $CH_2COOt$-Bu | - | H |
| H | 1 | $CH_2CH_3$ | - | $CH_2COOt$-Bu | - | H |
| OH | 1 | $CH_2CH_3$ | - | $CH_2COOt$-Bu | - | H |
| H | 1 | $CH_2COOEt$ | - | $CH_2COOt$-Bu | - | H |
| OH | 1 | $CH_2COOEt$ | - | $CH_2COOt$-Bu | - | H |
| H | 1 | $CH_2CH_2COOH$ | - | $CH_2COOt$-Bu | - | H |
| OH | 1 | $CH_2CH_2COOH$ | - | $CH_2COOt$-Bu | - | H |
| H | 1 | H | - | $CH_2COOEt$ | - | H |
| Cl | 1 | H | - | $CH_2COOEt$ | - | H |
| F | 1 | H | - | $CH_2COOEt$ | - | H |
| $CF_3$ | 1 | H | - | $CH_2COOEt$ | - | H |
| OH | 1 | H | - | $CH_2COOEt$ | - | H |
| $NO_2$ | 1 | H | - | $CH_2COOEt$ | - | H |
| H | 1 | $CH_3$ | - | $CH_2COOEt$ | - | H |
| Cl | 1 | $CH_3$ | - | $CH_2COOEt$ | - | H |
| F | 1 | $CH_3$ | - | $CH_2COOEt$ | - | H |
| $CF_3$ | 1 | $CH_3$ | - | $CH_2COOEt$ | - | H |
| OH | 1 | $CH_3$ | - | $CH_2COOEt$ | - | H |
| $NO_2$ | 1 | $CH_3$ | - | $CH_2COOEt$ | - | H |
| H | 1 | $CH_2COOH$ | - | $CH_2COOEt$ | - | H |
| Cl | 1 | $CH_2COOH$ | - | $CH_2COOEt$ | - | H |
| F | 1 | $CH_2COOH$ | - | $CH_2COOEt$ | - | H |
| $CF_3$ | 1 | $CH_2COOH$ | - | $CH_2COOEt$ | - | H |
| OH | 1 | $CH_2COOH$ | - | $CH_2COOEt$ | - | H |
| $NO_2$ | 1 | $CH_2COOH$ | - | $CH_2COOEt$ | - | H |
| H | 1 | $CH_2CH_3$ | - | $CH_2COOEt$ | - | H |
| OH | 1 | $CH_2CH_3$ | - | $CH_2COOEt$ | - | H |
| H | 1 | $CH_2COOEt$ | - | $CH_2COOEt$ | - | H |
| OH | 1 | $CH_2COOEt$ | - | $CH_2COOEt$ | - | H |
| H | 1 | $CH_2CH_2COOH$ | - | $CH_2COOEt$ | - | H |

TABLE 7 (cont'd)

| $X^1$ | r | $R^1$ | $(R^9)_p$ | $R^2$ | $(R^{13})_p$ | $(R^{10})_p$ |
|---|---|---|---|---|---|---|
| OH | 1 | $CH_2CH_2COOH$ | - | $CH_2COOEt$ | - | H |
| H | 1 | $CH_3$ | - | Ph | - | OH |
| H | 1 | $CH_2CH_3$ | - | Ph | - | OH |
| H | 1 | $CH_2COOEt$ | - | Ph | - | OH |
| H | 1 | $CH_3$ | - | o-F-Ph | - | OH |
| H | 1 | $CH_2CH_3$ | - | o-F-Ph | - | OH |
| H | 1 | $CH_2COOEt$ | - | o-F-Ph | - | OH |
| H | 1 | $CH_3$ | - | $CH_2COOt$-Bu | - | OH |
| H | 1 | $CH_2CH_3$ | - | $CH_2COOt$-Bu | - | OH |
| H | 1 | $CH_2COOEt$ | - | $CH_2COOt$-Bu | - | OH |

TABLE 8

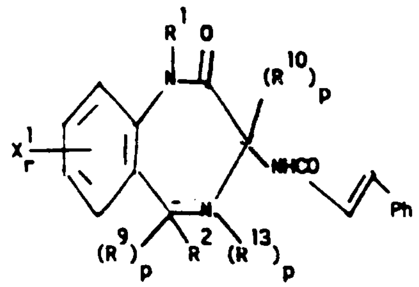

| $X^1$ | r | $R^1$ | $(R^9)_p$ | $R^2$ | $(R^{13})_p$ | $(R^{10})_p$ |
|---|---|---|---|---|---|---|
| H | 1 | H | - | Ph | - | H |
| Cl | 1 | H | - | Ph | - | H |
| F | 1 | H | - | Ph | - | H |
| $CF_3$ | 1 | H | - | Ph | - | H |
| OH | 1 | H | - | Ph | - | H |
| $NO_2$ | 1 | H | - | Ph | - | H |
| H | 1 | $CH_3$ | - | Ph | - | H |
| Cl | 1 | $CH_3$ | - | Ph | - | H |
| F | 1 | $CH_3$ | - | Ph | - | H |
| $CF_3$ | 1 | $CH_3$ | - | Ph | - | H |
| OH | 1 | $CH_3$ | - | Ph | - | H |
| $NO_2$ | 1 | $CH_3$ | - | Ph | - | H |
| H | 1 | $CH_2COOH$ | - | Ph | - | H |
| Cl | 1 | $CH_2COOH$ | - | Ph | - | H |
| F | 1 | $CH_2COOH$ | - | Ph | - | H |
| $CF_3$ | 1 | $CH_2COOH$ | - | Ph | - | H |
| OH | 1 | $CH_2COOH$ | - | Ph | - | H |
| $NO_2$ | 1 | $CH_2COOH$ | - | Ph | - | H |
| H | 1 | $CH_2CH_3$ | - | Ph | - | H |
| OH | 1 | $CH_2CH_3$ | - | Ph | - | H |
| H | 1 | $CH_2COOEt$ | - | Ph | - | H |

TABLE 8 (cont'd)

| $X^1$ | r | $R^1$ | $(R^9)_p$ | $R^2$ | $(R^{13})_p$ | $(R^{10})_p$ |
|---|---|---|---|---|---|---|
| OH | 1 | $CH_2COOEt$ | - | Ph | - | H |
| H | 1 | $CH_2CH_2COOH$ | - | Ph | - | H |
| OH | 1 | $CH_2CH_2COOH$ | - | Ph | - | H |
| H | 1 | H | - | o-F-Ph | - | H |
| Cl | 1 | H | - | o-F-Ph | - | H |
| F | 1 | H | - | o-F-Ph | - | H |
| $CF_3$ | 1 | H | - | o-F-Ph | - | H |
| OH | 1 | H | - | o-F-Ph | - | H |
| $NO_2$ | 1 | H | - | o-F-Ph | - | H |
| H | 1 | $CH_3$ | - | o-F-Ph | - | H |
| Cl | 1 | $CH_3$ | - | o-F-Ph | - | H |
| F | 1 | $CH_3$ | - | o-F-Ph | - | H |
| $CF_3$ | 1 | $CH_3$ | - | o-F-Ph | - | H |
| OH | 1 | $CH_3$ | - | o-F-Ph | - | H |
| $NO_2$ | 1 | $CH_3$ | - | o-F-Ph | - | H |
| H | 1 | $CH_2COOH$ | - | o-F-Ph | - | H |
| Cl | 1 | $CH_2COOH$ | - | o-F-Ph | - | H |
| F | 1 | $CH_2COOH$ | - | o-F-Ph | - | H |
| $CF_3$ | 1 | $CH_2COOH$ | - | o-F-Ph | - | H |
| OH | 1 | $CH_2COOH$ | - | o-F-Ph | - | H |
| $NO_2$ | 1 | $CH_2COOH$ | - | o-F-Ph | - | H |
| H | 1 | $CH_2CH_3$ | - | o-F-Ph | - | H |
| OH | 1 | $CH_2CH_3$ | - | o-F-Ph | - | H |
| H | 1 | $CH_2COOEt$ | - | o-F-Ph | - | H |
| OH | 1 | $CH_2COOEt$ | - | o-F-Ph | - | H |
| H | 1 | $CH_2CH_2COOH$ | - | o-F-Ph | - | H |
| OH | 1 | $CH_2CH_2COOH$ | - | o-F-Ph | - | H |
| H | 1 | H | - | p-Cl-Ph | - | H |
| F | 1 | H | - | p-Cl-Ph | - | H |
| $CF_3$ | 1 | H | - | p-Cl-Ph | - | H |

TABLE 8 (cont'd)

| $X^1$ | r | $R^1$ | $(R^9)_p$ | $R^2$ | $(R^{13})_p$ | $(R^{10})_p$ |
|---|---|---|---|---|---|---|
| OH | 1 | H | - | p-Cl-Ph | - | H |
| H | 1 | $CH_3$ | - | p-Cl-Ph | - | H |
| F | 1 | $CH_3$ | - | p-Cl-Ph | - | H |
| $CF_3$ | 1 | $CH_3$ | - | p-Cl-Ph | - | H |
| OH | 1 | $CH_3$ | - | p-Cl-Ph | - | H |
| H | 1 | $CH_2COOH$ | - | p-Cl-Ph | - | H |
| F | 1 | $CH_2COOH$ | - | p-Cl-Ph | - | H |
| $CF_3$ | 1 | $CH_2COOH$ | - | p-Cl-Ph | - | H |
| OH | 1 | $CH_2COOH$ | - | p-Cl-Ph | - | H |
| H | 1 | $CH_2CH_3$ | - | p-Cl-Ph | - | H |
| H | 1 | $CH_2COOEt$ | - | p-Cl-Ph | - | H |
| H | 1 | $CH_2CH_2COOH$ | - | p-Cl-Ph | - | H |
| H | 1 | H | - | $CH_2COOt$-Bu | - | H |
| Cl | 1 | H | - | $CH_2COOt$-Bu | - | H |
| F | 1 | H | - | $CH_2COOt$-Bu | - | H |
| $CF_3$ | 1 | H | - | $CH_2COOt$-Bu | - | H |
| OH | 1 | H | - | $CH_2COOt$-Bu | - | H |
| $NO_2$ | 1 | H | - | $CH_2COOt$-Bu | - | H |
| H | 1 | $CH_3$ | - | $CH_2COOt$-Bu | - | H |
| Cl | 1 | $CH_3$ | - | $CH_2COOt$-Bu | - | H |
| F | 1 | $CH_3$ | - | $CH_2COOt$-Bu | - | H |
| $CF_3$ | 1 | $CH_3$ | - | $CH_2COOt$-Bu | - | H |
| OH | 1 | $CH_3$ | - | $CH_2COOt$-Bu | - | H |
| $NO_2$ | 1 | $CH_3$ | - | $CH_2COOt$-Bu | - | H |
| H | 1 | $CH_2COOH$ | - | $CH_2COOt$-Bu | - | H |
| Cl | 1 | $CH_2COOH$ | - | $CH_2COOt$-Bu | - | H |
| F | 1 | $CH_2COOH$ | - | $CH_2COOt$-Bu | - | H |
| $CF_3$ | 1 | $CH_2COOH$ | - | $CH_2COOt$-Bu | - | H |
| OH | 1 | $CH_2COOH$ | - | $CH_2COOt$-Bu | - | H |
| $NO_2$ | 1 | $CH_2COOH$ | - | $CH_2COOt$-Bu | - | H |

TABLE 8 (cont'd)

| $X^1$ | r | $R^1$ | $(R^9)_p$ | $R^2$ | $(R^{13})_p$ | $(R^{10})_p$ |
|---|---|---|---|---|---|---|
| H | 1 | $CH_2CH_3$ | - | $CH_2COOt$-Bu | - | H |
| OH | 1 | $CH_2CH_3$ | - | $CH_2COOt$-Bu | - | H |
| H | 1 | $CH_2COOEt$ | - | $CH_2COOt$-Bu | - | H |
| OH | 1 | $CH_2COOEt$ | - | $CH_2COOt$-Bu | - | H |
| H | 1 | $CH_2CH_2COOH$ | - | $CH_2COOt$-Bu | - | H |
| OH | 1 | $CH_2CH_2COOH$ | - | $CH_2COOt$-Bu | - | H |
| H | 1 | H | - | $CH_2COOEt$ | - | H |
| Cl | 1 | H | - | $CH_2COOEt$ | - | H |
| F | 1 | H | - | $CH_2COOEt$ | - | H |
| $CF_3$ | 1 | H | - | $CH_2COOEt$ | - | H |
| OH | 1 | H | - | $CH_2COOEt$ | - | H |
| $NO_2$ | 1 | H | - | $CH_2COOEt$ | - | H |
| H | 1 | $CH_3$ | - | $CH_2COOEt$ | - | H |
| Cl | 1 | $CH_3$ | - | $CH_2COOEt$ | - | H |
| F | 1 | $CH_3$ | - | $CH_2COOEt$ | - | H |
| $CF_3$ | 1 | $CH_3$ | - | $CH_2COOEt$ | - | H |
| OH | 1 | $CH_3$ | - | $CH_2COOEt$ | - | H |
| $NO_2$ | 1 | $CH_3$ | - | $CH_2COOEt$ | - | H |
| H | 1 | $CH_2OOH$ | - | $CH_2COOEt$ | - | H |
| Cl | 1 | $CH_2COOH$ | - | $CH_2COOEt$ | - | H |
| F | 1 | $CH_2COOH$ | - | $CH_2COOEt$ | - | H |
| $CF_3$ | 1 | $CH_2COOH$ | - | $CH_2COOEt$ | - | H |
| OH | 1 | $CH_2COOH$ | - | $CH_2COOEt$ | - | H |
| $NO_2$ | 1 | $CH_2COOH$ | - | $CH_2COOEt$ | - | H |
| H | 1 | $CH_2CH_3$ | - | $CH_2COOEt$ | - | H |
| OH | 1 | $CH_2CH_3$ | - | $CH_2COOEt$ | - | H |
| H | 1 | $CH_2COOEt$ | - | $CH_2COOEt$ | - | H |
| OH | 1 | $CH_2COOEt$ | - | $CH_2COOEt$ | - | H |
| H | 1 | $CH_2CH_2COOH$ | - | $CH_2COOEt$ | - | H |
| OH | 1 | $CH_2CH_2COOH$ | - | $CH_2COOEt$ | - | H |

TABLE 9

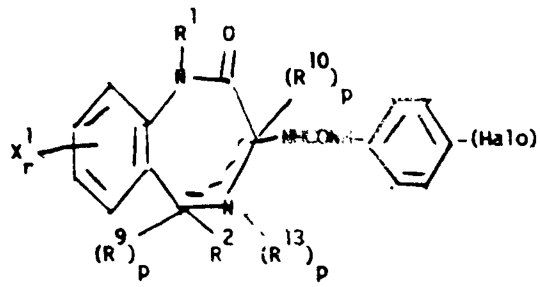

| $X^1$ | r | $R^1$ | $(R^9)_p$ | $R^2$ | $(R^{13})_p$ | $(R^{10})_p$ |
|---|---|---|---|---|---|---|
| H | 1 | H | - | Ph | - | H |
| Cl | 1 | H | - | Ph | - | H |
| F | 1 | H | - | Ph | - | H |
| $CF_3$ | 1 | H | - | Ph | - | H |
| OH | 1 | H | - | Ph | - | H |
| $NO_2$ | 1 | H | - | Ph | - | H |
| H | 1 | $CH_3$ | - | Ph | - | H |
| Cl | 1 | $CH_3$ | - | Ph | - | H |
| F | 1 | $CH_3$ | - | Ph | - | H |
| $CF_3$ | 1 | $CH_3$ | - | Ph | - | H |
| OH | 1 | $CH_3$ | - | Ph | - | H |
| $NO_2$ | 1 | $CH_3$ | - | Ph | - | H |
| H | 1 | $CH_2COOH$ | - | Ph | - | H |
| Cl | 1 | $CH_2COOH$ | - | Ph | - | H |
| F | 1 | $CH_2COOH$ | - | Ph | - | H |
| CF | 1 | $CH_2COOH$ | - | Ph | - | H |
| OH | 1 | $CH_2COOH$ | - | Ph | - | H |
| $NO_2$ | 1 | $CH_2COOH$ | - | Ph | - | H |
| H | 1 | $CH_2CH_3$ | - | Ph | - | H |
| OH | 1 | $CH_2CH_3$ | - | Ph | - | H |
| H | 1 | $CH_2COOEt$ | - | Ph | - | H |

TABLE 9 (cont'd)

| $X^1$ | r | $R^1$ | $(R^9)_p$ | $R^2$ | $(R^{13})_p$ | $(R^{10})_p$ |
|---|---|---|---|---|---|---|
| OH | 1 | $CH_2COOEt$ | - | Ph | - | H |
| H | 1 | $CH_2CH_2COOH$ | - | Ph | - | H |
| OH | 1 | $CH_2CH_2COOH$ | - | Ph | - | H |
| H | 1 | H | - | o-F-Ph | - | H |
| Cl | 1 | H | - | o-F-Ph | - | H |
| F | 1 | H | - | o-F-Ph | - | H |
| $CF_3$ | 1 | H | - | o-F-Ph | - | H |
| OH | 1 | H | - | o-F-Ph | - | H |
| $NO_2$ | 1 | H | - | o-F-Ph | - | H |
| H | 1 | $CH_3$ | - | o-F-Ph | - | H |
| Cl | 1 | $CH_3$ | - | o-F-Ph | - | H |
| F | 1 | $CH_3$ | - | o-F-Ph | - | H |
| $CF_3$ | 1 | $CH_3$ | - | o-F-Ph | - | H |
| OH | 1 | $CH_3$ | - | o-F-Ph | - | H |
| $NO_2$ | 1 | $CH_3$ | - | o-F-Ph | - | H |
| H | 1 | $CH_2COOH$ | - | o-F-Ph | - | H |
| Cl | 1 | $CH_2COOH$ | - | o-F-Ph | - | H |
| F | 1 | $CH_2COOH$ | - | o-F-Ph | - | H |
| $CF_3$ | 1 | $CH_2COOH$ | - | o-F-Ph | - | H |
| OH | 1 | $CH_2COOH$ | - | o-F-Ph | - | H |
| $NO_2$ | 1 | $CH_2COOH$ | - | o-F-Ph | - | H |
| H | 1 | $CH_2CH_3$ | - | o-F-Ph | - | H |
| OH | 1 | $CH_2CH_3$ | - | o-F-Ph | - | H |
| H | 1 | $CH_2COOEt$ | - | o-F-Ph | - | H |
| OH | 1 | $CH_2COOEt$ | - | o-F-Ph | - | H |
| H | 1 | $CH_2CH_2COOH$ | - | o-F-Ph | - | H |
| OH | 1 | $CH_2CH_2COOH$ | - | o-F-Ph | - | H |
| H | 1 | H | - | p-Cl-Ph | - | H |
| F | 1 | H | - | p-Cl-Ph | - | H |
| $CF_3$ | 1 | H | - | p-Cl-Ph | - | H |

TABLE 9 (cont'd)

| $X^1$ | r | $R^1$ | $(R^9)_p$ | $R^2$ | $(R^{13})_p$ | $(R^{10})_p$ |
|---|---|---|---|---|---|---|
| OH | 1 | H | - | p-Cl-Ph | - | H |
| H | 1 | $CH_3$ | - | p-Cl-Ph | - | H |
| F | 1 | $CH_3$ | - | p-Cl-Ph | - | H |
| $CF_3$ | 1 | $CH_3$ | - | p-Cl-Ph | - | H |
| OH | 1 | $CH_3$ | - | p-Cl-Ph | - | H |
| H | 1 | $CH_2COOH$ | - | p-Cl-Ph | - | H |
| F | 1 | $CH_2COOH$ | - | p-Cl-Ph | - | H |
| $CF_3$ | 1 | $CH_2COOH$ | - | p-Cl-Ph | - | H |
| OH | 1 | $CH_2COOH$ | - | p-Cl-Ph | - | H |
| H | 1 | $CH_2CH_3$ | - | p-Cl-Ph | - | H |
| H | 1 | $CH_2COOEt$ | - | p-Cl-Ph | - | H |
| H | 1 | $CH_2CH_2COOH$ | - | p-Cl-Ph | - | H |
| H | 1 | H | - | $CH_2COOt$-Bu | - | H |
| Cl | 1 | H | - | $CH_2COOt$-Bu | - | H |
| F | 1 | H | - | $CH_2COOt$-Bu | - | H |
| $CF_3$ | 1 | H | - | $CH_2COOt$-Bu | - | H |
| OH | 1 | H | - | $CH_2COOt$-Bu | - | H |
| $NO_2$ | 1 | H | - | $CH_2COOt$-Bu | - | H |
| H | 1 | $CH_3$ | - | $CH_2COOt$-Bu | - | H |
| Cl | 1 | $CH_3$ | - | $CH_2COOt$-Bu | - | H |
| F | 1 | $CH_3$ | - | $CH_2COOt$-Bu | - | H |
| $CF_3$ | 1 | $CH_3$ | - | $CH_2COOt$-Bu | - | H |
| OH | 1 | $CH_3$ | - | $CH_2COOt$-Bu | - | H |
| $NO_2$ | 1 | $CH_3$ | - | $CH_2COOt$-Bu | - | H |
| H | 1 | $CH_2COOH$ | - | $CH_2COOt$-Bu | - | H |
| Cl | 1 | $CH_2COOH$ | - | $CH_2COOt$-Bu | - | H |
| F | 1 | $CH_2COOH$ | - | $CH_2COOt$-Bu | - | H |
| $CF_3$ | 1 | $CH_2COOH$ | - | $CH_2COOt$-Bu | - | H |
| OH | 1 | $CH_2COOH$ | - | $CH_2COOt$-Bu | - | H |
| $NO_2$ | 1 | $CH_2COOH$ | - | $CH_2COOt$-Bu | - | H |

TABLE 9 (cont'd)

| $X^1$ | r | $R^1$ | $(R^9)_p$ | $R^2$ | $(R^{13})_p$ | $(R^{10})_p$ |
|---|---|---|---|---|---|---|
| H | 1 | $CH_2CH_3$ | - | $CH_2COOt\text{-}Bu$ | - | H |
| OH | 1 | $CH_2CH_3$ | - | $CH_2COOt\text{-}Bu$ | - | H |
| H | 1 | $CH_2COOEt$ | - | $CH_2COOt\text{-}Bu$ | - | H |
| OH | 1 | $CH_2COOEt$ | - | $CH_2COOt\text{-}Bu$ | - | H |
| H | 1 | $CH_2CH_2COOH$ | - | $CH_2COOt\text{-}Bu$ | - | H |
| OH | 1 | $CH_2CH_2COOH$ | - | $CH_2COOt\text{-}Bu$ | - | H |
| H | 1 | H | - | $CH_2COOEt$ | - | H |
| Cl | 1 | H | - | $CH_2COOEt$ | - | H |
| F | 1 | H | - | $CH_2COOEt$ | - | H |
| $CF_3$ | 1 | H | - | $CH_2COOEt$ | - | H |
| OH | 1 | H | - | $CH_2COOEt$ | - | H |
| $NO_2$ | 1 | H | - | $CH_2COOEt$ | - | H |
| H | 1 | $CH_3$ | - | $CH_2COOEt$ | - | H |
| Cl | 1 | $CH_3$ | - | $CH_2COOEt$ | - | H |
| F | 1 | $CH_3$ | - | $CH_2COOEt$ | - | H |
| $CF_3$ | 1 | $CH_3$ | - | $CH_2COOEt$ | - | H |
| OH | 1 | $CH_3$ | - | $CH_2COOEt$ | - | H |
| $NO_2$ | 1 | $CH_3$ | - | $CH_2COOEt$ | - | H |
| H | 1 | $CH_2COOH$ | - | $CH_2COOEt$ | - | H |
| Cl | 1 | $CH_2COOH$ | - | $CH_2COOEt$ | - | H |
| F | 1 | $CH_2COOH$ | - | $CH_2COOEt$ | - | H |
| $CF_3$ | 1 | $CH_2COOH$ | - | $CH_2COOEt$ | - | H |
| OH | 1 | $CH_2COOH$ | - | $CH_2COOEt$ | - | H |
| $NO_2$ | 1 | $CH_2COOH$ | - | $CH_2COOEt$ | - | H |
| H | 1 | $CH_2CH_3$ | - | $CH_2COOEt$ | - | H |
| OH | 1 | $CH_2CH_3$ | - | $CH_2COOEt$ | - | H |
| H | 1 | $CH_2COOEt$ | - | $CH_2COOEt$ | - | H |
| OH | 1 | $CH_2COOEt$ | - | $CH_2COOEt$ | - | H |
| H | 1 | $CH_2CH_2COOH$ | - | $CH_2COOEt$ | - | H |
| OH | 1 | $CH_2CH_2COOH$ | - | $CH_2COOEt$ | - | H |

TABLE 10

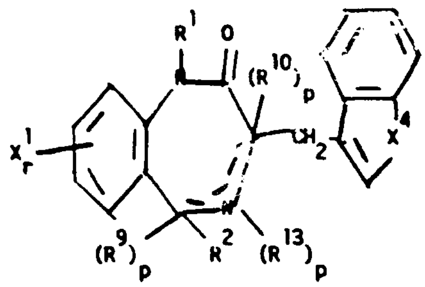

$X_4$=NH, $NCH_3$, O, or S

| $X^1$ | r | $R^1$ | $(R^9)_p$ | $R^2$ | $(R^{13})_p$ | $(R^{10})_p$ |
|---|---|---|---|---|---|---|
| H | 1 | H | - | Ph | - | H |
| Cl | 1 | H | - | Ph | - | H |
| F | 1 | H | - | Ph | - | H |
| $CF_3$ | 1 | H | - | Ph | - | H |
| OH | 1 | H | - | Ph | - | H |
| $NO_2$ | 1 | H | - | Ph | - | H |
| H | 1 | $CH_3$ | - | Ph | - | H |
| Cl | 1 | $CH_3$ | - | Ph | - | H |
| F | 1 | $CH_3$ | - | Ph | - | H |
| $CF_3$ | 1 | $CH_3$ | - | Ph | - | H |
| OH | 1 | $CH_3$ | - | Ph | - | H |
| $NO_2$ | 1 | $CH_3$ | - | Ph | - | H |
| H | 1 | $CH_2COOH$ | - | Ph | - | H |
| Cl | 1 | $CH_2COOH$ | - | Ph | - | H |
| F | 1 | $CH_2COOH$ | - | Ph | - | H |
| $CF_3$ | 1 | $CH_2COOH$ | - | Ph | - | H |
| OH | 1 | $CH_2COOH$ | - | Ph | - | H |
| $NO_2$ | 1 | $CH_2COOH$ | - | Ph | - | H |
| H | 1 | $CH_2CH_3$ | - | Ph | - | H |
| OH | 1 | $CH_2CH_3$ | - | Ph | - | H |

TABLE 10 (cont'd)

| $X^1$ | r | $R^1$ | $(R^9)_p$ | $R^2$ | $(R^{13})_p$ | $(R^{10})_p$ |
|---|---|---|---|---|---|---|
| H | 1 | $CH_2COOEt$ | - | Ph | - | H |
| OH | 1 | $CH_2COOEt$ | - | Ph | - | H |
| H | 1 | $CH_2CH_2COOH$ | - | Ph | - | H |
| OH | 1 | $CH_2CH_2COOH$ | - | Ph | - | H |
| H | 1 | H | - | o-F-Ph | - | H |
| Cl | 1 | H | - | o-F-Ph | - | H |
| F | 1 | H | - | o-F-Ph | - | H |
| $CF_3$ | 1 | H | - | o-F-Ph | - | H |
| OH | 1 | H | - | o-F-Ph | - | H |
| $NO_2$ | 1 | H | - | o-F-Ph | - | H |
| H | 1 | $CH_3$ | - | o-F-Ph | - | H |
| Cl | 1 | $CH_3$ | - | o-F-Ph | - | H |
| F | 1 | $CH_3$ | - | o-F-Ph | - | H |
| $CF_3$ | 1 | $CH_3$ | - | o-F-Ph | - | H |
| OH | 1 | $CH_3$ | - | o-F-Ph | - | H |
| $NO_2$ | 1 | $CH_3$ | - | o-F-Ph | - | H |
| H | 1 | $CH_2COOH$ | - | o-F-Ph | - | H |
| Cl | 1 | $CH_2COOH$ | - | o-F-Ph | - | H |
| F | 1 | $CH_2COOH$ | - | o-F-Ph | - | H |
| $CF_3$ | 1 | $CH_2COOH$ | - | o-F-Ph | - | H |
| OH | 1 | $CH_2COOH$ | - | o-F-Ph | - | H |
| $NO_2$ | 1 | $CH_2COOH$ | - | o-F-Ph | - | H |
| H | 1 | $CH_2CH_3$ | - | o-F-Ph | - | H |
| OH | 1 | $CH_2CH_3$ | - | o-F-Ph | - | H |
| H | 1 | $CH_2COOEt$ | - | o-F-Ph | - | H |
| OH | 1 | $CH_2COOEt$ | - | o-F-Ph | - | H |
| H | 1 | $CH_2CH_2COOH$ | - | o-F-Ph | - | H |
| OH | 1 | $CH_2CH_2COOH$ | - | o-F-Ph | - | H |
| H | 1 | H | - | p-Cl-Ph | - | H |

TABLE 10 (cont'd)

| $X^1$ | r | $R^1$ | $(R^9)_p$ | $R^2$ | $(R^{13})_p$ | $(R^{10})_p$ |
|---|---|---|---|---|---|---|
| F | 1 | H | - | p-Cl-Ph | - | H |
| $CF_3$ | 1 | H | - | p-Cl-Ph | - | H |
| OH | 1 | H | - | p-Cl-Ph | - | H |
| H | 1 | $CH_3$ | - | p-Cl-Ph | - | H |
| F | 1 | $CH_3$ | - | p-Cl-Ph | - | H |
| $CF_3$ | 1 | $CH_3$ | - | p-Cl-Ph | - | H |
| OH | 1 | $CH_3$ | - | p-Cl-Ph | - | H |
| H | 1 | $CH_2COOH$ | - | p-Cl-Ph | - | H |
| F | 1 | $CH_2COOH$ | - | p-Cl-Ph | - | H |
| $CF_3$ | 1 | $CH_2COOH$ | - | p-Cl-Ph | - | H |
| OH | 1 | $CH_2COOH$ | - | p-Cl-Ph | - | H |
| H | 1 | $CH_2CH_3$ | - | p-Cl-Ph | - | H |
| H | 1 | $CH_2COOEt$ | - | p-Cl-Ph | - | H |
| H | 1 | $CH_2CH_2COOH$ | - | p-Cl-Ph | - | H |
| H | 1 | H | - | $CH_2COOt$-Bu | - | H |
| Cl | 1 | H | - | $CH_2COOt$-Bu | - | H |
| F | 1 | H | - | $CH_2COOt$-Bu | - | H |
| $CF_3$ | 1 | H | - | $CH_2COOt$-Bu | - | H |
| OH | 1 | H | - | $CH_2COOt$-Bu | - | H |
| $NO_2$ | 1 | H | - | $CH_2COOt$-Bu | - | H |
| H | 1 | $CH_3$ | - | $CH_2COOt$-Bu | - | H |
| Cl | 1 | $CH_3$ | - | $CH_2COOt$-Bu | - | H |
| F | 1 | $CH_3$ | - | $CH_2COOt$-Bu | - | H |
| $CF_3$ | 1 | $CH_3$ | - | $CH_2COOt$-Bu | - | H |
| OH | 1 | $CH_3$ | - | $CH_2COOt$-Bu | - | H |
| $NO_2$ | 1 | $CH_3$ | - | $CH_2COOt$-Bu | - | H |
| H | 1 | $CH_2COOH$ | - | $CH_2COOt$-Bu | - | H |
| Cl | 1 | $CH_2COOH$ | - | $CH_2COOt$-Bu | - | H |
| F | 1 | $CH_2COOH$ | - | $CH_2COOt$-Bu | - | H |
| $CF_3$ | 1 | $CH_2COOH$ | - | $CH_2COOt$-Bu | - | H |

TABLE 10 (cont'd)

| $X^1$ | r | $R^1$ | $(R^9)_p$ | $R^2$ | $(R^{13})_p$ | $(R^{10})_p$ |
|---|---|---|---|---|---|---|
| OH | 1 | $CH_2COOH$ | - | $CH_2COOt$-Bu | - | H |
| $NO_2$ | 1 | $CH_2COOH$ | - | $CH_2COOt$-Bu | - | H |
| H | 1 | $CH_2CH_3$ | - | $CH_2COOt$-Bu | - | H |
| OH | 1 | $CH_2CH_3$ | - | $CH_2COOt$-Bu | - | H |
| H | 1 | $CH_2COOEt$ | - | $CH_2COOt$-Bu | - | H |
| OH | 1 | $CH_2COOEt$ | - | $CH_2COOt$-Bu | - | H |
| H | 1 | $CH_2CH_2COOH$ | - | $CH_2COOt$-Bu | - | H |
| OH | 1 | $CH_2CH_2COOH$ | - | $CH_2COOt$-Bu | - | H |
| H | 1 | H | - | $CH_2COOEt$ | - | H |
| Cl | 1 | H | - | $CH_2COOEt$ | - | H |
| F | 1 | H | - | $CH_2COOEt$ | - | H |
| $CF_3$ | 1 | H | - | $CH_2COOEt$ | - | H |
| OH | 1 | H | - | $CH_2COOEt$ | - | H |
| $NO_2$ | 1 | H | - | $CH_2COOEt$ | - | H |
| H | 1 | $CH_3$ | - | $CH_2COOEt$ | - | H |
| Cl | 1 | $CH_3$ | - | $CH_2COOEt$ | - | H |
| F | 1 | $CH_3$ | - | $CH_2COOEt$ | - | H |
| $CF_3$ | 1 | $CH_3$ | - | $CH_2COOEt$ | - | H |
| OH | 1 | $CH_3$ | - | $CH_2COOEt$ | - | H |
| $NO_2$ | 1 | $CH_3$ | - | $CH_2COOEt$ | - | H |
| H | 1 | $CH_2COOH$ | - | $CH_2COOEt$ | - | H |
| Cl | 1 | $CH_2COOH$ | - | $CH_2COOEt$ | - | H |
| F | 1 | $CH_2COOH$ | - | $CH_2COOEt$ | - | H |
| $CF_3$ | 1 | $CH_2COOH$ | - | $CH_2COOEt$ | - | H |
| OH | 1 | $CH_2COOH$ | - | $CH_2COOEt$ | - | H |
| $NO_2$ | 1 | $CH_2COOH$ | - | $CH_2COOEt$ | - | H |
| H | 1 | $CH_2CH_3$ | - | $CH_2COOEt$ | - | H |
| OH | 1 | $CH_2CH_3$ | - | $CH_2COOEt$ | - | H |
| H | 1 | $CH_2COOEt$ | - | $CH_2COOEt$ | - | H |

TABLE 10 (cont'd)

| $X^1$ | r | $R^1$ | $(R^9)_p$ | $R^2$ | $(R^{13})_p$ | $(R^{10})_p$ |
|---|---|---|---|---|---|---|
| OH | 1 | $CH_2COOEt$ | - | $CH_2COOEt$ | - | H |
| H | 1 | $CH_2CH_2COOH$ | - | $CH_2COOEt$ | - | H |
| OH | 1 | $CH_2CH_2COOH$ | - | $CH_2COOEt$ | - | H |
| H | 1 | $CH_3$ | - | Ph | - | OH |
| H | 1 | $CH_2CH_3$ | - | Ph | - | OH |
| H | 1 | $CH_2COOEt$ | - | Ph | - | OH |
| H | 1 | $CH_3$ | - | o-F-Ph | - | OH |
| H | 1 | $CH_2CH_3$ | - | o-F-Ph | - | OH |
| H | 1 | $CH_2COOEt$ | - | o-F-Ph | - | OH |
| H | 1 | $CH_3$ | - | $CH_2COOt$-Bu | - | OH |
| H | 1 | $CH_2CH_3$ | - | $CH_2COOt$-Bu | - | OH |
| H | 1 | $CH_2COOEt$ | - | $CH_2COOt$-Bu | - | OH |

TABLE 11

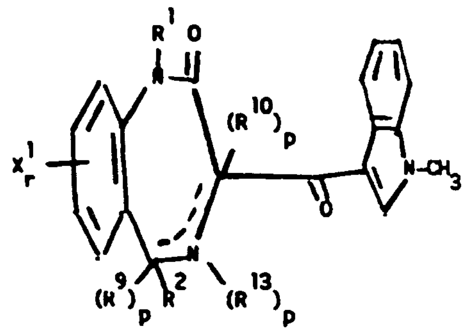

| $X^1$ | r | $R^1$ | $(R^9)_p$ | $R^2$ | $(R^{13})_p$ | $(R^{10})_p$ |
|---|---|---|---|---|---|---|
| H | 1 | H | - | Ph | - | H |
| Cl | 1 | H | - | Ph | - | H |
| F | 1 | H | - | Ph | - | H |
| $CF_3$ | 1 | H | - | Ph | - | H |
| OH | 1 | H | - | Ph | - | H |
| $NO_2$ | 1 | H | - | Ph | - | H |
| H | 1 | $CH_3$ | - | Ph | - | H |
| Cl | 1 | $CH_3$ | - | Ph | - | H |
| F | 1 | $CH_3$ | - | Ph | - | H |
| $CF_3$ | 1 | $CH_3$ | - | Ph | - | H |
| OH | 1 | $CH_3$ | - | Ph | - | H |
| $NO_2$ | 1 | $CH_3$ | - | Ph | - | H |
| H | 1 | $CH_2COOH$ | - | Ph | - | H |
| Cl | 1 | $CH_2COOH$ | - | Ph | - | H |
| F | 1 | $CH_2COOH$ | - | Ph | - | H |
| $CF_3$ | 1 | $CH_2COOH$ | - | Ph | - | H |
| OH | 1 | $CH_2COOH$ | - | Ph | - | H |
| $NO_2$ | 1 | $CH_2COOH$ | - | Ph | - | H |
| H | 1 | $CH_2CH_3$ | - | Ph | - | H |
| OH | 1 | $CH_2CH_3$ | - | Ph | - | H |
| H | 1 | $CH_2COOEt$ | - | Ph | - | H |

TABLE 11 (cont'd)

| $X^1$ | r | $R^1$ | $(R^9)_p$ | $R^2$ | $(R^{13})_p$ | $(R^{10})_p$ |
|---|---|---|---|---|---|---|
| OH | 1 | $CH_2COOEt$ | - | Ph | - | H |
| H | 1 | $CH_2CH_2COOH$ | - | Ph | - | H |
| OH | 1 | $CH_2CH_2COOH$ | - | Ph | - | H |
| H | 1 | H | - | o-F-Ph | - | H |
| Cl | 1 | H | - | o-F-Ph | - | H |
| F | 1 | H | - | o-F-Ph | - | H |
| $CF_3$ | 1 | H | - | o-F-Ph | - | H |
| OH | 1 | H | - | o-F-Ph | - | H |
| $NO_2$ | 1 | H | - | o-F-Ph | - | H |
| H | 1 | $CH_3$ | - | o-F-Ph | - | H |
| Cl | 1 | $CH_3$ | - | o-F-Ph | - | H |
| F | 1 | $CH_3$ | - | o-F-Ph | - | H |
| $CF_3$ | 1 | $CH_3$ | - | o-F-Ph | - | H |
| OH | 1 | $CH_3$ | - | o-F-Ph | - | H |
| $NO_2$ | 1 | $CH_3$ | - | o-F-Ph | - | H |
| H | 1 | $CH_2COOH$ | - | o-F-Ph | - | H |
| Cl | 1 | $CH_2COOH$ | - | o-F-Ph | - | H |
| F | 1 | $CH_2COOH$ | - | o-F-Ph | - | H |
| $CF_3$ | 1 | $CH_2COOH$ | - | o-F-Ph | - | H |
| OH | 1 | $CH_2COOH$ | - | o-F-Ph | - | H |
| $NO_2$ | 1 | $CH_2COOH$ | - | o-F-Ph | - | H |
| H | 1 | $CH_2CH_3$ | - | o-F-Ph | - | H |
| OH | 1 | $CH_2CH_3$ | - | o-F-Ph | - | H |
| H | 1 | $CH_2COOEt$ | - | o-F-Ph | - | H |
| OH | 1 | $CH_2COOEt$ | - | o-F-Ph | - | H |
| H | 1 | $CH_2CH_2COOH$ | - | o-F-Ph | - | H |
| OH | 1 | $CH_2CH_2COOH$ | - | o-F-Ph | - | H |
| H | 1 | H | - | p-Cl-Ph | - | H |
| F | 1 | H | - | p-Cl-Ph | - | H |

TABLE 11 (cont'd)

| $X^1$ | r | $R^1$ | $(R^9)_p$ | $R^2$ | $(R^{13})_p$ | $(R^{10})_p$ |
|---|---|---|---|---|---|---|
| $CF_3$ | 1 | H | - | p-Cl-Ph | - | H |
| OH | 1 | H | - | p-Cl-Ph | - | H |
| H | 1 | $CH_3$ | - | p-Cl-Ph | - | H |
| F | 1 | $CH_3$ | - | p-Cl-Ph | - | H |
| $CF_3$ | 1 | $CH_3$ | - | p-Cl-Ph | - | H |
| OH | 1 | $CH_3$ | - | p-Cl-Ph | - | H |
| H | 1 | $CH_2COOH$ | - | p-Cl-Ph | - | H |
| F | 1 | $CH_2COOH$ | - | p-Cl-Ph | - | H |
| $CF_3$ | 1 | $CH_2COOH$ | - | p-Cl-Ph | - | H |
| OH | 1 | $CH_2COOH$ | - | p-Cl-Ph | - | H |
| H | 1 | $CH_2CH_3$ | - | p-Cl-Ph | - | H |
| H | 1 | $CH_2COOEt$ | - | p-Cl-Ph | - | H |
| H | 1 | $CH_2CH_2COOH$ | - | p-Cl-Ph | - | H |
| H | 1 | H | - | $CH_2COOt$-Bu | - | H |
| Cl | 1 | H | - | $CH_2COOt$-Bu | - | H |
| F | 1 | H | - | $CH_2COOt$-Bu | - | H |
| $CF_3$ | 1 | H | - | $CH_2COOt$-Bu | - | H |
| OH | 1 | H | - | $CH_2COOt$-Bu | - | H |
| $NO_2$ | 1 | H | - | $CH_2COOt$-Bu | - | H |
| H | 1 | $CH_3$ | - | $CH_2COOt$-Bu | - | H |
| Cl | 1 | $CH_3$ | - | $CH_2COOt$-Bu | - | H |
| F | 1 | $CH_3$ | - | $CH_2COOt$-Bu | - | H |
| $CF_3$ | 1 | $CH_3$ | - | $CH_2COOt$-Bu | - | H |
| OH | 1 | $CH_3$ | - | $CH_2COOt$-Bu | - | H |
| $NO_2$ | 1 | $CH_3$ | - | $CH_2COOt$-Bu | - | H |
| H | 1 | $CH_2COOH$ | - | $CH_2COOt$-Bu | - | H |
| Cl | 1 | $CH_2COOH$ | - | $CH_2COOt$-Bu | - | H |
| F | 1 | $CH_2COOH$ | - | $CH_2COOt$-Bu | - | H |
| $CF_3$ | 1 | $CH_2COOH$ | - | $CH_2COOt$-Bu | - | H |
| OH | 1 | $CH_2COOH$ | - | $CH_2COOt$-Bu | - | H |

TABLE 11 (cont'd)

| $X^1$ | r | $R^1$ | $(R^9)_p$ | $R^2$ | $(R^{13})_p$ | $(R^{10})_p$ |
|---|---|---|---|---|---|---|
| $NO_2$ | 1 | $CH_2COOH$ | - | $CH_2COOt$-Bu | - | H |
| H | 1 | $CH_2CH_3$ | - | $CH_2COOt$-Bu | - | H |
| OH | 1 | $CH_2CH_3$ | - | $CH_2COOt$-Bu | - | H |
| H | 1 | $CH_2COOEt$ | - | $CH_2COOt$-Bu | - | H |
| OH | 1 | $CH_2COOEt$ | - | $CH_2COOt$-Bu | - | H |
| H | 1 | $CH_2CH_2COOH$ | - | $CH_2COOt$-Bu | - | H |
| OH | 1 | $CH_2CH_2COOH$ | - | $CH_2COOt$-Bu | - | H |
| H | 1 | H | - | $CH_2COOEt$ | - | H |
| Cl | 1 | H | - | $CH_2COOEt$ | - | H |
| F | 1 | H | - | $CH_2COOEt$ | - | H |
| $CF_3$ | 1 | H | - | $CH_2COOEt$ | - | H |
| OH | 1 | H | - | $CH_2COOEt$ | - | H |
| $NO_2$ | 1 | H | - | $CH_2COOEt$ | - | H |
| H | 1 | $CH_3$ | - | $CH_2COOEt$ | - | H |
| Cl | 1 | $CH_3$ | - | $CH_2COOEt$ | - | H |
| F | 1 | $CH_3$ | - | $CH_2COOEt$ | - | H |
| $CF_3$ | 1 | $CH_3$ | - | $CH_2COOEt$ | - | H |
| OH | 1 | $CH_3$ | - | $CH_2COOEt$ | - | H |
| $NO_2$ | 1 | $CH_3$ | - | $CH_2COOEt$ | - | H |
| H | 1 | $CH_2COOH$ | - | $CH_2COOEt$ | - | H |
| Cl | 1 | $CH_2COOH$ | - | $CH_2COOEt$ | - | H |
| F | 1 | $CH_2COOH$ | - | $CH_2COOEt$ | - | H |
| $CF_3$ | 1 | $CH_2COOH$ | - | $CH_2COOEt$ | - | H |
| OH | 1 | $CH_2COOH$ | - | $CH_2COOEt$ | - | H |
| $NO_2$ | 1 | $CH_2COOH$ | - | $CH_2COOEt$ | - | H |
| H | 1 | $CH_2CH_3$ | - | $CH_2COOEt$ | - | H |
| OH | 1 | $CH_2CH_3$ | - | $CH_2COOEt$ | - | H |
| H | 1 | $CH_2COOEt$ | - | $CH_2COOEt$ | - | H |
| OH | 1 | $CH_2COOEt$ | - | $CH_2COOEt$ | - | H |
| H | 1 | $CH_2CH_2COOH$ | - | $CH_2COOEt$ | - | H |

TABLE 11 (cont'd)

| $X^1$ | r | $R^1$ | $(R^9)_p$ | $R^2$ | $(R^{13})_p$ | $(R^{10})_p$ |
|---|---|---|---|---|---|---|
| OH | 1 | $CH_2CH_2COOH$ | - | $CH_2COOEt$ | - | H |
| H | 1 | $CH_3$ | - | Ph | - | OH |
| H | 1 | $CH_2CH_3$ | - | Ph | - | OH |
| H | 1 | $CH_2COOEt$ | - | Ph | - | OH |
| H | 1 | $CH_3$ | - | o-F-Ph | - | OH |
| H | 1 | $CH_2CH_3$ | - | o-F-Ph | - | OH |
| H | 1 | $CH_2COOEt$ | - | o-F-Ph | - | OH |
| H | 1 | $CH_3$ | - | $CH_2COOt$-Bu | - | OH |
| H | 1 | $CH_2CH_3$ | - | $CH_2COOt$-Bu | - | OH |
| H | 1 | $CH_2COOEt$ | - | $CH_2COOt$-Bu | - | OH |

TABLE 12

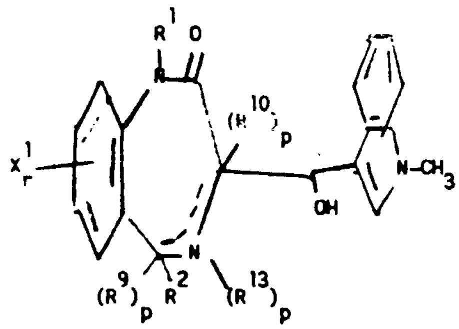

| $X^1$ | r | $R^1$ | $(R^9)_p$ | $R^2$ | $(R^{13})_p$ | $(R^{10})_p$ |
|---|---|---|---|---|---|---|
| H | 1 | H | - | Ph | - | H |
| Cl | 1 | H | - | Ph | - | H |
| F | 1 | H | - | Ph | - | H |
| $CF_3$ | 1 | H | - | Ph | - | H |
| OH | 1 | H | - | Ph | - | H |
| $NO_2$ | 1 | H | - | Ph | - | H |
| H | 1 | $CH_3$ | - | Ph | - | H |
| Cl | 1 | $CH_3$ | - | Ph | - | H |
| F | 1 | $CH_3$ | - | Ph | - | H |
| $CF_3$ | 1 | $CH_3$ | - | Ph | - | H |
| OH | 1 | $CH_3$ | - | Ph | - | H |
| $NO_2$ | 1 | $CH_3$ | - | Ph | - | H |
| H | 1 | $CH_2COOH$ | - | Ph | - | H |
| Cl | 1 | $CH_2COOH$ | - | Ph | - | H |
| F | 1 | $CH_2COOH$ | - | Ph | - | H |
| $CF_3$ | 1 | $CH_2COOH$ | - | Ph | - | H |
| OH | 1 | $CH_2COOH$ | - | Ph | - | H |
| $NO_2$ | 1 | $CH_2COOH$ | - | Ph | - | H |
| H | 1 | $CH_2CH_3$ | - | Ph | - | H |
| OH | 1 | $CH_2CH_3$ | - | Ph | - | H |

TABLE 12 (cont'd)

| $X^1$ | r | $R^1$ | $(R^9)_p$ | $R^2$ | $(R^{13})_p$ | $(R^{10})_p$ |
|---|---|---|---|---|---|---|
| H | 1 | $CH_2COOEt$ | - | Ph | - | H |
| OH | 1 | $CH_2COOEt$ | - | Ph | - | H |
| H | 1 | $CH_2CH_2COOH$ | - | Ph | - | H |
| OH | 1 | $CH_2CH_2COOH$ | - | Ph | - | H |
| H | 1 | H | - | o-F-Ph | - | H |
| Cl | 1 | H | - | o-F-Ph | - | H |
| F | 1 | H | - | o-F-Ph | - | H |
| $CF_3$ | 1 | H | - | o-F-Ph | - | H |
| OH | 1 | H | - | o-F-Ph | - | H |
| $NO_2$ | 1 | H | - | o-F-Ph | - | H |
| H | 1 | $CH_3$ | - | o-F-Ph | - | H |
| Cl | 1 | $CH_3$ | - | o-F-Ph | - | H |
| F | 1 | $CH_3$ | - | o-F-Ph | - | H |
| $CF_3$ | 1 | $CH_3$ | - | o-F-Ph | - | H |
| OH | 1 | $CH_3$ | - | o-F-Ph | - | H |
| $NO_2$ | 1 | $CH_3$ | - | o-F-Ph | - | H |
| H | 1 | $CH_2COOH$ | - | o-F-Ph | - | H |
| Cl | 1 | $CH_2COOH$ | - | o-F-Ph | - | H |
| F | 1 | $CH_2COOH$ | - | o-F-Ph | - | H |
| $CF_3$ | 1 | $CH_2COOH$ | - | o-F-Ph | - | H |
| OH | 1 | $CH_2COOH$ | - | o-F-Ph | - | H |
| $NO_2$ | 1 | $CH_2COOH$ | - | o-F-Ph | - | H |
| H | 1 | $CH_2CH_3$ | - | o-F-Ph | - | H |
| OH | 1 | $CH_2CH_3$ | - | o-F-Ph | - | H |
| H | 1 | $CH_2COOEt$ | - | o-F-Ph | - | H |
| OH | 1 | $CH_2COOEt$ | - | o-F-Ph | - | H |
| H | 1 | $CH_2CH_2COOH$ | - | o-F-Ph | - | H |
| OH | 1 | $CH_2CH_2COOH$ | - | o-F-Ph | - | H |
| H | 1 | H | - | p-Cl-Ph | - | H |
| F | 1 | H | - | p-Cl-Ph | - | H |

TABLE 12 (cont'd)

| $X^1$ | r | $R^1$ | $(R^9)_p$ | $R^2$ | $(R^{13})_p$ | $(R^{10})_p$ |
|---|---|---|---|---|---|---|
| $CF_3$ | 1 | H | - | p-Cl-Ph | - | H |
| OH | 1 | H | - | p-Cl-Ph | - | H |
| H | 1 | $CH_3$ | - | p-Cl-Ph | - | H |
| F | 1 | $CH_3$ | - | p-Cl-Ph | - | H |
| $CF_3$ | 1 | $CH_3$ | - | p-Cl-Ph | - | H |
| OH | 1 | $CH_3$ | - | p-Cl-Ph | - | H |
| H | 1 | $CH_2COOH$ | - | p-Cl-Ph | - | H |
| F | 1 | $CH_2COOH$ | - | p-Cl-Ph | - | H |
| $CF_3$ | 1 | $CH_2COOH$ | - | p-Cl-Ph | - | H |
| OH | 1 | $CH_2COOH$ | - | p-Cl-Ph | - | H |
| H | 1 | $CH_2CH_3$ | - | p-Cl-Ph | - | H |
| H | 1 | $CH_2COOEt$ | - | p-Cl-Ph | - | H |
| H | 1 | $CH_2CH_2COOH$ | - | p-Cl-Ph | - | H |
| H | 1 | H | - | $CH_2COOt$-Bu | - | H |
| Cl | 1 | H | - | $CH_2COOt$-Bu | - | H |
| F | 1 | H | - | $CH_2COOt$-Bu | - | H |
| $CF_3$ | 1 | H | | $CH_2COOt$-Bu | - | H |
| OH | 1 | H | - | $CH_2COOt$-Bu | - | H |
| $NO_2$ | 1 | H | - | $CH_2COOt$-Bu | - | H |
| H | 1 | $CH_3$ | - | $CH_2COOt$-Bu | - | H |
| Cl | 1 | $CH_3$ | - | $CH_2COOt$-Bu | - | H |
| F | 1 | $CH_3$ | - | $CH_2COOt$-Bu | - | H |
| $CF_3$ | 1 | $CH_3$ | - | $CH_2COOt$-Bu | - | H |
| OH | 1 | $CH_3$ | - | $CH_2COOt$-Bu | - | H |
| $NO_2$ | 1 | $CH_3$ | - | $CH_2COOt$-Bu | - | H |
| H | 1 | $CH_2COOH$ | - | $CH_2COOt$-Bu | - | H |
| Cl | 1 | $CH_2COOH$ | - | $CH_2COOt$-Bu | - | H |
| F | 1 | $CH_2COOH$ | - | $CH_2COOt$-Bu | - | H |
| $CF_3$ | 1 | $CH_2COOH$ | - | $CH_2COOt$-Bu | - | H |
| OH | 1 | $CH_2COOH$ | - | $CH_2COOt$-Bu | - | H |

TABLE 12 (cont'd)

| $X^1$ | r | $R^1$ | $(R^9)_p$ | $R^2$ | $(R^{13})_p$ | $(R^{10})_p$ |
|---|---|---|---|---|---|---|
| $NO_2$ | 1 | $CH_2COOH$ | - | $CH_2COOt$-Bu | - | H |
| H | 1 | $CH_2CH_3$ | - | $CH_2COOt$-Bu | - | H |
| OH | 1 | $CH_2CH_3$ | - | $CH_2COOt$-Bu | - | H |
| H | 1 | $CH_2COOEt$ | - | $CH_2COOt$-Bu | - | H |
| OH | 1 | $CH_2COOEt$ | - | $CH_2COOt$-Bu | - | H |
| H | 1 | $CH_2CH_2COOH$ | - | $CH_2COOt$-Bu | - | H |
| OH | 1 | $CH_2CH_2COOH$ | - | $CH_2COOt$-Bu | - | H |
| H | 1 | H | - | $CH_2COOEt$ | - | H |
| Cl | 1 | H | - | $CH_2COOEt$ | - | H |
| F | 1 | H | - | $CH_2COOEt$ | - | H |
| $CF_3$ | 1 | H | - | $CH_2COOEt$ | - | H |
| OH | 1 | H | - | $CH_2COOEt$ | - | H |
| $NO_2$ | 1 | H | - | $CH_2COOEt$ | - | H |
| H | 1 | $CH_3$ | - | $CH_2COOEt$ | - | H |
| Cl | 1 | $CH_3$ | - | $CH_2COOEt$ | - | H |
| F | 1 | $CH_3$ | - | $CH_2COOEt$ | - | H |
| $CF_3$ | 1 | $CH_3$ | - | $CH_2COOEt$ | - | H |
| OH | 1 | $CH_3$ | - | $CH_2COOEt$ | - | H |
| $NO_2$ | 1 | $CH_3$ | - | $CH_2COOEt$ | - | H |
| H | 1 | $CH_2COOH$ | - | $CH_2COOEt$ | - | H |
| Cl | 1 | $CH_2COOH$ | - | $CH_2COOEt$ | - | H |
| F | 1 | $CH_2COOH$ | - | $CH_2COOEt$ | - | H |
| $CF_3$ | 1 | $CH_2COOH$ | - | $CH_2COOEt$ | - | H |
| OH | 1 | $CH_2COOH$ | - | $CH_2COOEt$ | - | H |
| $NO_2$ | 1 | $CH_2COOH$ | - | $CH_2COOEt$ | - | H |
| H | 1 | $CH_2CH_3$ | - | $CH_2COOEt$ | - | H |
| OH | 1 | $CH_2CH_3$ | - | $CH_2COOEt$ | - | H |
| H | 1 | $CH_2COOEt$ | - | $CH_2COOEt$ | - | H |
| OH | 1 | $CH_2COOEt$ | - | $CH_2COOEt$ | - | H |
| H | 1 | $CH_2CH_2COOH$ | - | $CH_2COOEt$ | - | H |
| OH | 1 | $CH_2CH_2COOH$ | - | $CH_2COOEt$ | - | H |

TABLE 13

Compounds of the Formula

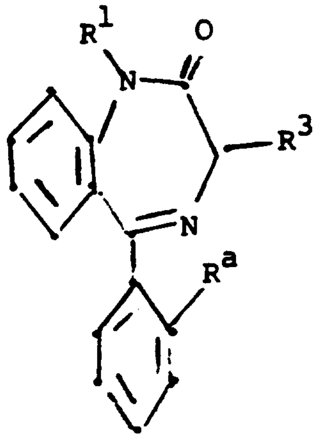

| No. | $R^a$ | $R^1$ | $R^3$ |
|---|---|---|---|
| 577 | F | $-CH_2-CF_3$ | $-CH_2-$ 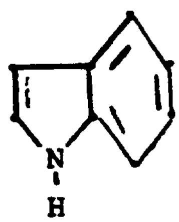 |
| 586 | F | H | 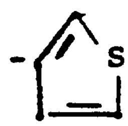 |
| 625 | H | H | $-NH-CO-CH_2-$ 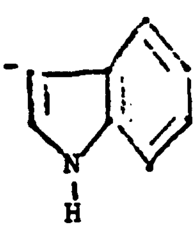 |

TABLE 13 (Cont'd)

| No. | $R^a$ | $R^1$ | $R^3$ |
| --- | --- | --- | --- |
| 643 | F | $-(CH_2)_2-CN$ | 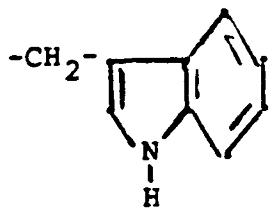 |
| 648 | F | H | 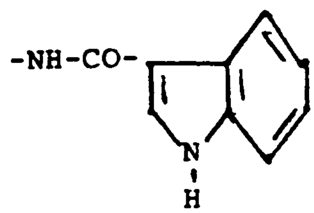 |
| 651 | F | H | 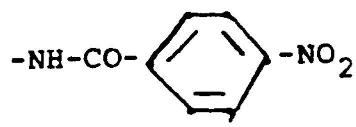 |
| 652 | H | H | 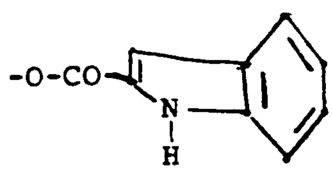 |
| 659 | F | H | 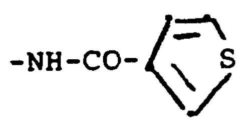 |
| 665 | H | H | 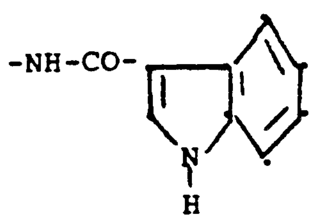 |

TABLE 13 (Cont'd)

| No. | $R^a$ | $R^1$ | $R^3$ |
| --- | --- | --- | --- |
| 666 | F | H | $-NH-CO-CH_2-$ 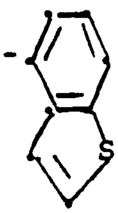 |
| 668 | F | H | $-NH-SO_2-$ 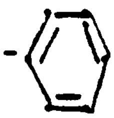 $-CH_3$ |
| 676 | F | H | $-NH-CO-$ 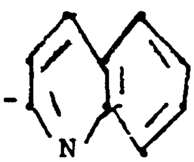 |
| 677 | F | H | $-NH-CO-CHOH-$ 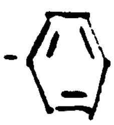 |
| 678 | F | H | $-NH-CO-$ 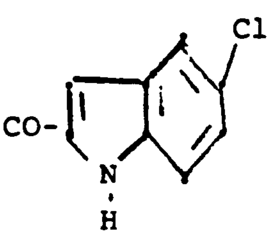 |
| 679 | H | H | $-N(CH_3)-CO-$ 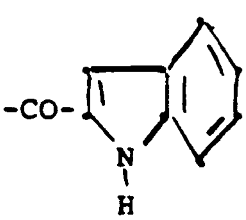 |

## TABLE 13 (Cont'd)

| No. | $R^a$ | $R^1$ | $R^3$ |
|---|---|---|---|
| 686 | F | H | -NH-CO- 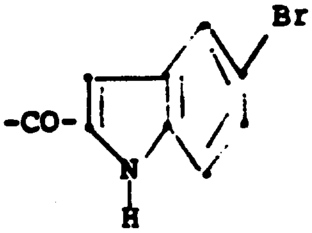 |
| 688 | F | H | -NH-CO- 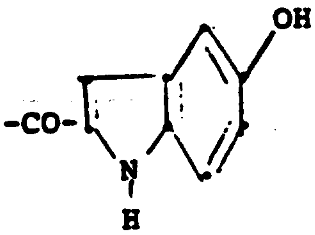 |
| 690 | F | $-CH_2-CO-NH_2$ | $-CH_2-$ 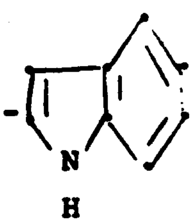 |
| 691 | F | H | $-NH-CH_2-$ 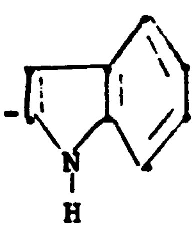 |
| 692 | F | H | $-NH-CO-CH_2-NH-$ 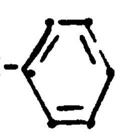 |

TABLE 13 (Cont'd)

| No. | $R^a$ | $R^1$ | $R^3$ |
|---|---|---|---|
| 694 | F | H | -NH-CO- 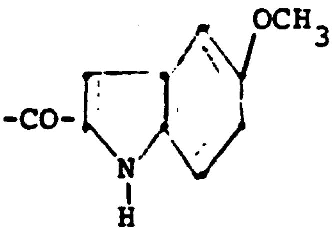 |
| 695 | F | H | -NH-CO- 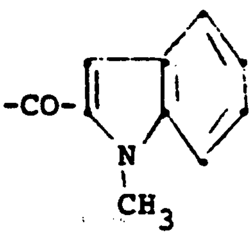 |
| 716 | H | H | -NH-CO- 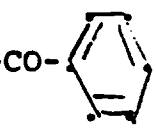 |
| 720 | F | H | -NH-CO- 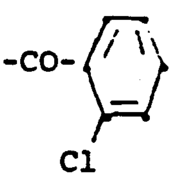 |
| 722 | H | H | -NH-CO- 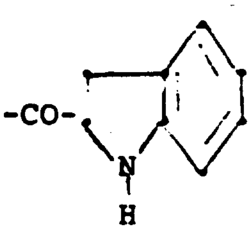 |

**TABLE 13 (Cont'd)**

| No. | $R^a$ | $R^1$ | $R^3$ | |
|---|---|---|---|---|
| 724 | H | H | -NH-CO- 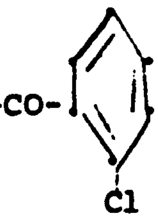 | |
| 725 | H | $CH_3$ | -NH-CO- 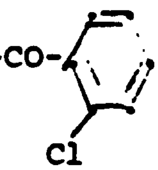 | [(-)-enantiomer] |
| 726 | H | $CH_3$ | -NH-CO- 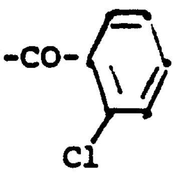 | [(+)-enantiomer] |
| 736 | F | H | -NH-CO- 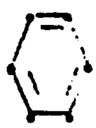 $-CF_3$ | |
| 737 | F | H | -NH-CO- 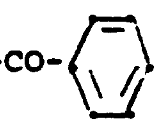 $-CH_3$ | |
| 727 | H | $CH_3$ | $-N(CH_3)-CO-$ 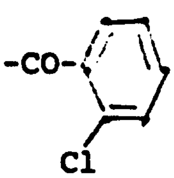 | |

TABLE 13 (Cont'd)

| No. | $R^a$ | $R^1$ | $R^3$ |
|---|---|---|---|
| 728 | H | $CH_3$ | 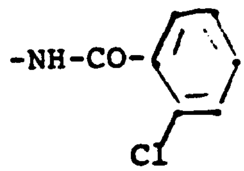 |
| 740 | H | H | 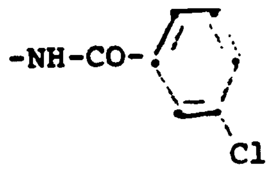 |
| 745 | F | H | 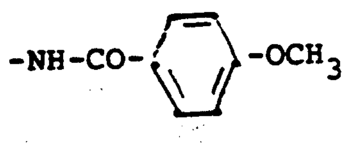 |
| 752 | F | H | 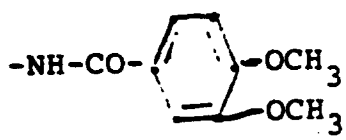 |
| 753 | F | H | 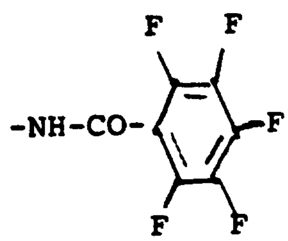 |
| 755 | H | H | 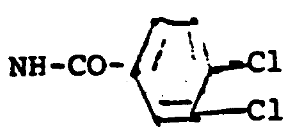 |

## TABLE 13 (Cont'd)

| No. | $R^a$ | $R^1$ | $R^3$ |
|---|---|---|---|
| 761 | F | $CH_3$ | 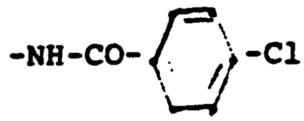 ($N^4$-oxide) |
| 763 | F | H | 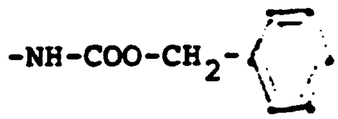 |
| 772 | H | H | 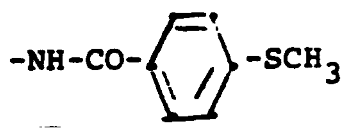 |
| 779 | F | 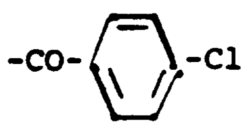 | 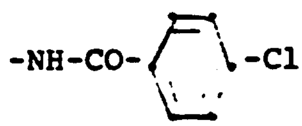 |
| 781 | H | H | 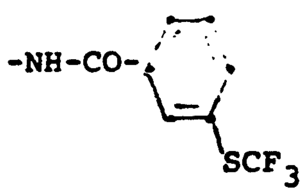 |
| 782 | H | H | 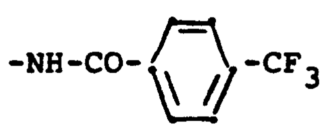 |
| 786 | F | 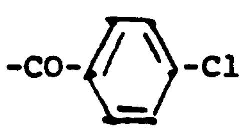 | 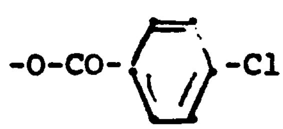 |

TABLE 13 (Cont'd)

| No. | R^a | R^1 | R^3 |
|---|---|---|---|
| 787 | F | H | 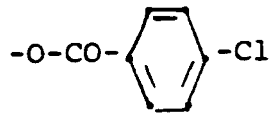 |
| 790 | F | $CH_3$ | 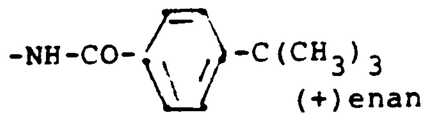 (+)enantiomer |
| 791 | F | H | 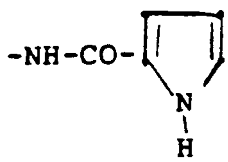 |
| 793 | H | H | 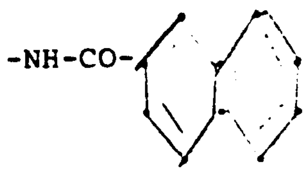 |
| 794 | F | $CH_3$ | 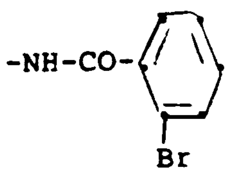 (-)enantiomer |
| 795 | F | $CH_3$ | 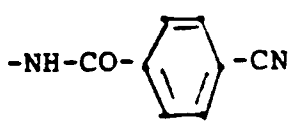 (+)enantiomer |

TABLE 13 (Cont'd)

| No. | $R^a$ | $R^1$ | $R^3$ |
|---|---|---|---|
| 796 | H | H | -NH-CO- 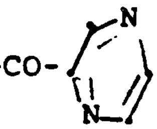 |
| 799 | H | H | -NH-CO- 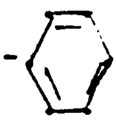 $-(CH_2)_2-CH_3$ |
| 800 | H | H | -NH-CO- 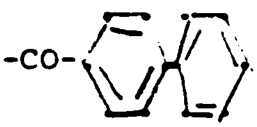 |
| 801 | H | H | -NH-CO- 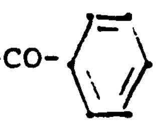 $-(CH_2)_4-CH_3$ |
| 802 | H | H | -NH-CO- 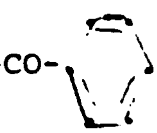 $-C(CH_3)_3$ |
| 803 | H | H | -NH-CO- 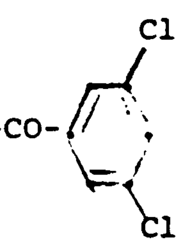 |
| 804 | H | H | -NH-CO- 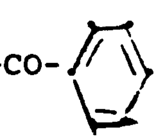 -OH |

## TABLE 13 (Cont'd)

| No. | $R^a$ | $R^1$ | $R^3$ | |
|---|---|---|---|---|
| 805 | H | H | -NH-CO- 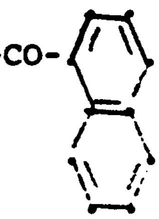 | |
| 816 | H | H | -NH-CO- 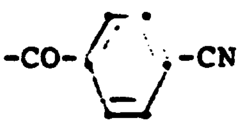 | |
| 825 | F | $CH_3$ | -NH-CO- 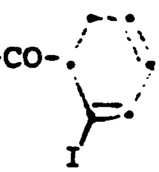 | (+)enantiomer |
| 827 | F | $CH_3$ | -NH-CO- 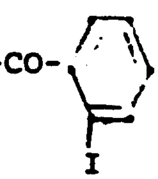 | (-)enantiomer |
| 829 | F | $CH_3$ | -NH-CO- 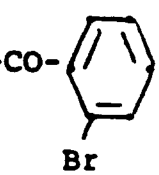 | (+)enantiomer |
| 830 | F | $CH_3$ | -NH-CO- 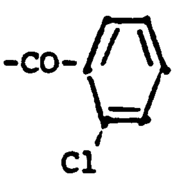 | (+)enantiomer |

Other compounds of Formula I are listed on the following table.

**TABLE 14**

| No. | Compound |
|---|---|
| 632 | 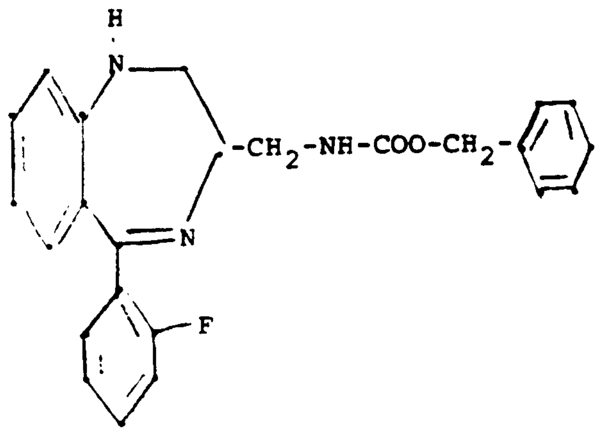 |
| 633 | 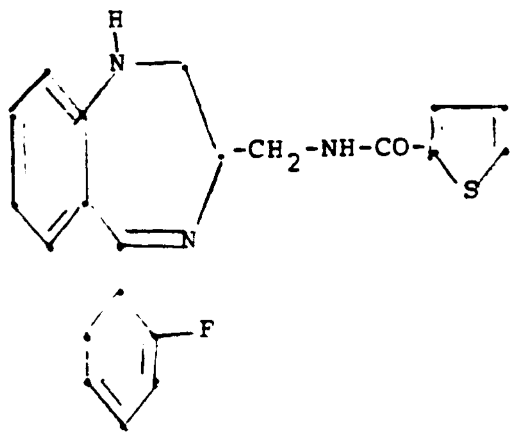 |

TABLE 14 (Cont'd)

| No. | Compound |
|---|---|
| 636 | 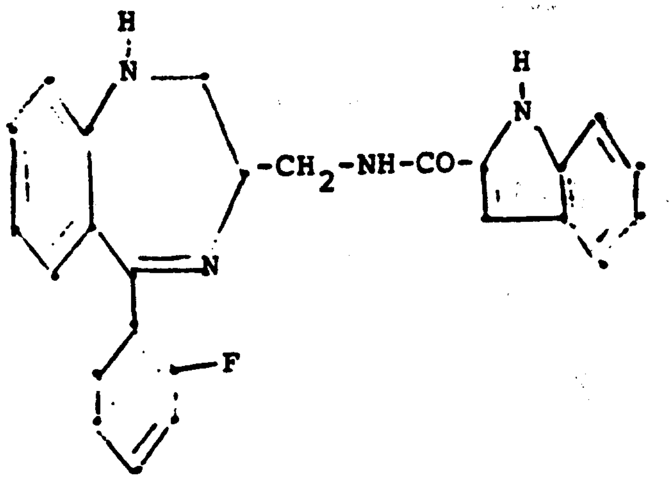 |
| 638 | 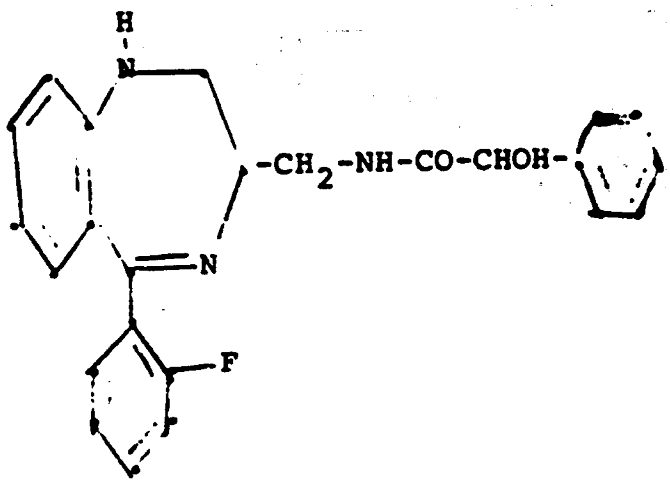 |

TABLE 14 (Cont'd)

| No. | Compound |
|---|---|
| 646 | 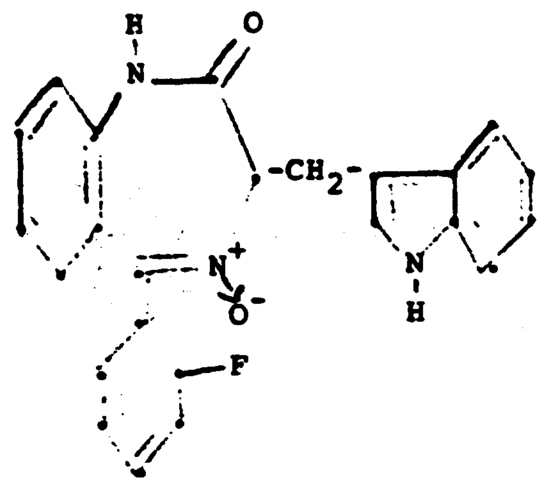 |
| 732 | 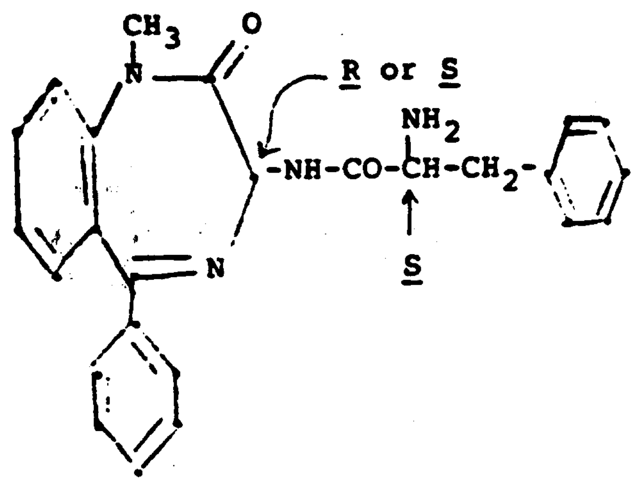 |

TABLE 14 (Cont'd)

| No. | Compound |
|---|---|
| 733 | 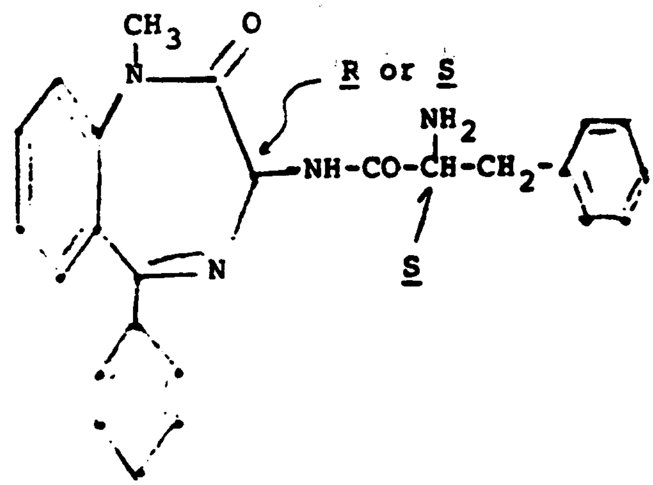 |
| 777 | 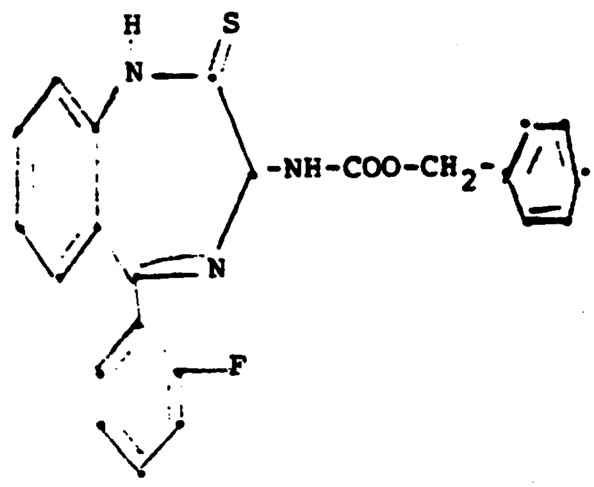 |

TABLE 14 (Cont'd)

| No. | Compound |
|---|---|
| 808 | 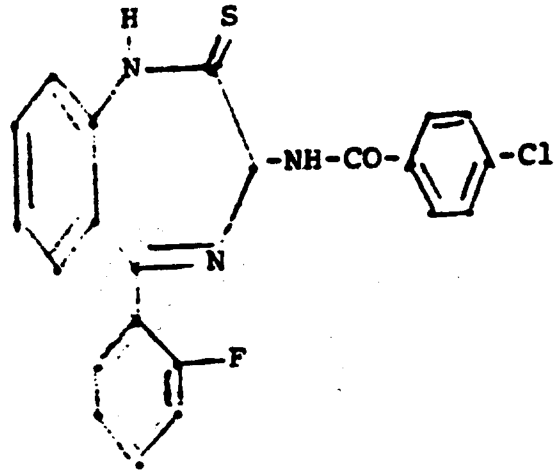 |
| 809 | 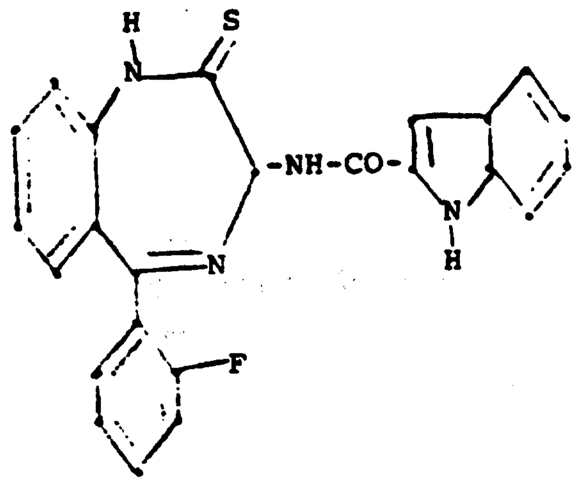 |

TABLE 14 (Cont'd)

| No. | Compound |
|---|---|
| 826 | 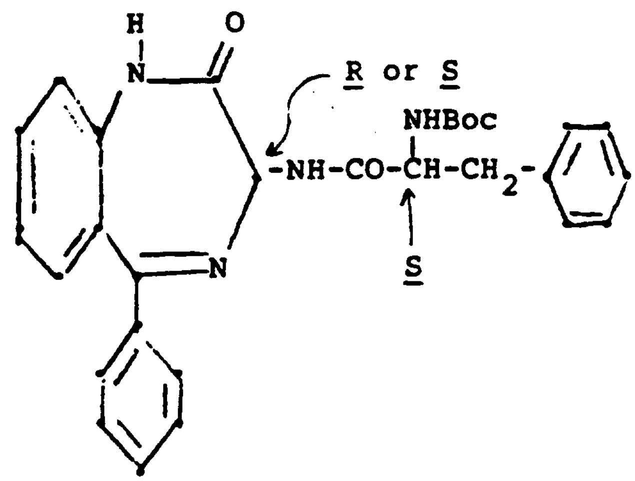 |
| 828 | 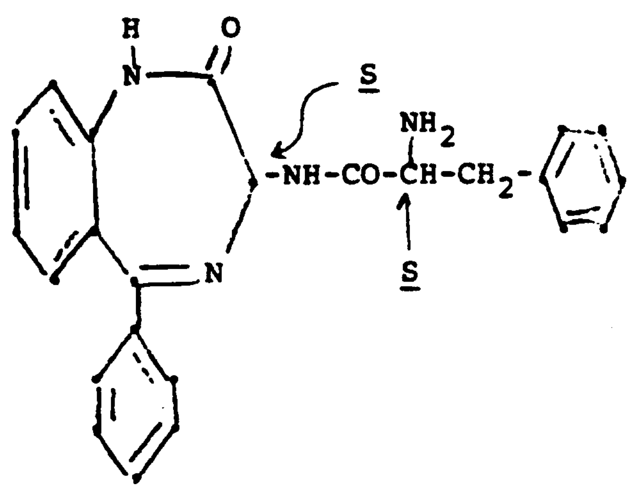 |

The invention is further defined by reference to the following preparations and examples, which are intended to be illustrative and not limiting.

All temperatures are in degrees Celsius.

EXAMPLE 1

2 – N – ($N^{\alpha}$ – Boc – D – tryptophanyl)amino – 2' – fluorobenzophenone

2 – Amino – 2' – fluorobenzophenone (4 g, 18.6 mmole), Boc – D – tryptophan (5.65 g, 18.6 mmole) and dicyclohexylcarbodiimide (DCC) (18.6 ml of a 1M solution in methylene chloride, 18.6 mmole) were combined in 28 ml of dry tetrahydrofuran stirred in an ice bath. The mixture was allowed to warm to room temperature and stirred overnight. The solids were removed by filtration and the filtrate evaporated in vacuo. The residue was chromatographed on 9" (23 cm) of silica gel (230 – 400 mesh) in a 55 mm diameter column using 1L of each of methylene chloride and 2% and 3% (v/v) diethyl ether in methylene chloride.

The product fractions were combined and evaporated in vacuo. The residue was crystallized from diethyl ether and the resulting solid dried in vacuo at 40° for 20 hours: (m.p. 64 – 67°).

The compound showed a single component by thin layer chromatography (TLC) ($R_f = 0.36$, silica gel plate eluted with 6% (v/v) diethyl ether in methylene chloride). The NMR spectrum was consistent with the title structure and verified the presence of $Et_2O$.

| Anal. Calc'd for $C_{29}H_{28}FN_3O_4.Et_2O$: | | | |
|---|---|---|---|
| | C, 68.85; | H, 6.65; | N, 7.30. |
| Found: | C, 69.25; | H, 6.75; | N, 7.30. |

EXAMPLE 2

1,3 – Dihydro – 5 – (2 – fluorophenyl) – 3(R) – (3' – indolyl)methyl – 2H – 1,4 – benzodiazepin – 2 – one

2 – N – ($N^{\alpha}$ – Boc – D – tryptophanyl)amino – 2' – fluorobenzophenone (4.0 g = 8.0 mmole) in 37 ml of ethyl acetate was stirred in an ice bath and saturated with hydrogen chloride gas for 20 minutes. The mixture was evaporated to dryness in vacuo to give 2 – N – (D – tryptophanyl)amino – 2' – fluorobenzophenone hydrochloride. The residue in 125 ml of methanol was treated with 30 ml of water and the pH of the mixture adjusted to 8.5 – 9.0 with 10% sodium hydroxide solution. The mixture was stirred at room temperature for three days.

The suspension was filtered and the resulting white solid dried in vacuo at 40° overnight: (m.p. 251 – 254°).

The compound showed a single component by thin layer chromatography (TLC) ($R_f = 0.59$, silica gel plate eluted with 1:1 (v/v) diethyl ether/methylene chloride) and by HPLC (greater than 99%). The NMR spectrum was consistent with the title structure. The mass spectrum showed a molecular ion at m/e = 383.

| Anal. Calcd. for $C_{24}H_{18}FN_3O$: | | | |
|---|---|---|---|
| | C, 75.18; | H, 4.73; | N, 10.96. |
| Found: | C, 74.88; | H, 4.70, | N, 10.65. |

EXAMPLE 3

1,3 – Dihydro – 5 – (2 – fluorophenyl) – 3(R) – (3' – indolyl)methyl – 2H – 1,4 – benzodiazepin – 2 – one

2 – Amino – 2' – fluorobenzophenone (12.5 g = 58 mmole) was stirred in 100 ml of dry tetrahydrofuran in an ice bath. D – Tryptophan acid chloride hydrochloride (16 g = 62 mmole), slurried in 50 ml of tetrahydrofuran, was added over 10 minutes, and the mixture stirred 2 hours in the ice bath. The resulting solid was filtered, then added to 200 ml of methanol containing 200 ml of water. The pH was adjusted to 8.5 – 9.0 with 10% sodium hydroxide, the mixture was stirred for three days, then filtered. The solid was dried in vacuo at 40°.

EXAMPLE 4

1,3 – Dihydro – 5 – (2 – fluorophenyl) – 3(R) – [3' – (1' – methylindolyl) – methyl] – 1 – methyl – 2H – 1,4 – benzodiazepin – 2 – one(A) and 1,3 – dihydro – 5 – (2 – fluorophenyl) – 3(R) – (3' – indolyl) methyl – 1 – methyl – 2H – 1,4 – benzodiazepin – 2 – one (B)

A 1,3 – Dihydro – 5 – (2 – fluorophenyl) – 3(R) – (3' – indolyl)methyl – 2H – 1,4 – benzodiazepin – 2 – one (0.85 g, 2.2 mmole) and sodium hydride (0.11 g of a 50% suspension in mineral oil, 2.3 mmole) were stirred in 10 ml of dry, degassed dimethylformamide under nitrogen in an ice bath. After 40 minutes, methyl iodide (0.14 mL = 2.25 mmole) was added in one portion. The mixture was stirred for 1.5 hours at room temperature, then poured into 100 ml of water and extracted with methylene chloride ($CH_2Cl_2$) (3 x 30 mL). The $CH_2Cl_2$ layers were washed with water, dried over potassium carbonate, filtered and evaporated in vacuo. The residue was chromatographed on 9" (23 cm) of silica gel (250 – 400 mesh) in a 55 mm diameter column eluted with 4% (v/v) diethyl ether in $CH_2Cl_2$. The first product eluted was A

which was obtained as a glass upon evaporation. The solid was dried in vacuo at room temperature: (m.p. 97 – 100 ° ( )).

The compound showed a single component by thin layer chromatography ($R_f = 0.57$, silica gel plate eluted with 10% (v/v) diethyl ether in $CH_2Cl_2$) and by HPLC (98%). The NMR spectrum was consistent with the title structure and verified the presence of $CH_2Cl_2$. The mass spectrum showed a molecular ion at m/e = 411.

| Anal. Calc'd. for $C_{26}H_{22}FN_3O \cdot 0.1\ CH_2Cl_2$ | | | |
|---|---|---|---|
| | C, 74.64; | H, 5.33, | N, 10.01. |
| Found: | C, 74.69; | H, 5.32; | N, 9.63. |

B The second component eluted was the monomethyl compound B which was obtained as a foam (0.66 g) upon evaporation. Crystallization from hexane/$CH_2Cl_2$ gave analytical material; (m.p. 80 – 85 ° ( )).

The compound showed a single component by thin layer chromatography (silica gel plates eluted with 4% (v/v) diethyl ether in $CH_2Cl_2$) and by HPLC (99%). The NMR spectrum was consistent with the title structure and verified the presence of $CH_2Cl_2$.

| Anal. Calc'd for $C_{25}H_{20}FN_3O \cdot 0.75\ CH_2Cl_2$: | | | |
|---|---|---|---|
| | C, 67.06, | H, 4.70; | N, 9.11; |
| Found: | C, 67.04; | H, 4.81; | N, 9.14. |

EXAMPLE 5

7 – Chloro – 1,3 – dihydro – 3(R) – (3' – indolyl)methyl – 5 – phenyl – 2H – 1,4 – benzodiazepin – 2 – one

2 – Amino – 5 – chlorobenzophenone (1.2 g, 5.2 mmole) and D – tryptophan methyl ester hydrochloride (1.3 g, 5.1 mmole) were combined in dry pyridine (25 mL) and heated at reflux under nitrogen for 5 hrs. The mixture was evaporated in vacuo and the residue washed twice with pH 6 buffer and dissolved in ethyl acetate (50 mL). The ethyl acetate solution was dried over sodium sulfate, filtered, and evaporated in vacuo to give an oil which was chromatographed on a 13 inch (33 cm) column of silica gel (230 – 400 mesh) in a 25 mm diameter column eluted with 20% (v/v) ether methylene choride. The product fractions were evaporated in vacuo to give the title compound as a white solid which was dried in vacuo at 100 ° : (m.p. 130 – 155 ° ( )).

The compound showed a single spot by thin layer chromatography ($R_f = 0.36$, silica gel plate eluted with 4:1 $CH_2Cl_2$/ether). The NMR spectrum was consistent with the title structure and verified the presence of ether. The compound was 99.8% pure by HPLC. The mass spectrum showed a molecular ion at m/e = 399.

| Anal. Calc'd for $C_{24}H_{18}ClN_3O \cdot 0.5C_4H_{10}O$: | | | | |
|---|---|---|---|---|
| | C, 71.47; | H, 5.31; | N, 9.62; | Cl, 8.12. |
| Found | C, 71.62; | H, 5.83; | N, 9.47; | Cl, 8.24. |

EXAMPLE 6

1,3 – Dihydro – 3(R) – (3' – indolyl)methyl – 5 – phenyl – 2H – 1,4 – benzodiazepin – 2 – one

The procedure of Example 1 was carried out using 2 – aminobenzophenone (1.97 g, 0.01 mole), Boc – D – tryptophan (3.04 g, 0.01 mole) and DCC (10 mL of 1M solution in methylene chloride ($CH_2Cl_2$) in THF (15 mL). The crude product obtained after filtration and evaporation of the mixture was deprotected and cyclized by the procedure of Example 2. The mixture was evaporated in vacuo, combined with water (50 mL) and extracted with chloroform (250 mL). The chloroform solution was hired over potassium carbonate, filtered, and evaporated to dryness in vacuo. Recrystallization from a mixture of acetone (50 mL) and ether (50 mL) gave a white solid which was dried in vacuo at 100 ° : (m.p. 260 – 263 ° (d)).

The compound showed a single spot by TLC ($R_f = 0.53$, silica gel plate eluted with 1:1 $CH_2Cl_2$/ether). The NMR spectrum was consistent with the title structure and verified the presence of acetone. The compound was 99.6% pure by HPLC. The mass spectrum showed a molecular ion at m/e = 365.

| Anal. Calc'd for $C_{24}H_{19}N_3O \cdot 0.5C_3H_6O$: | | | |
|---|---|---|---|
| | C, 77.64, | H, 5.62, | N, 10.65. |
| Found: | C, 77.34, | H, 5.44, | N, 10.87. |

EXAMPLE 7

1,3 – Dihydro – 3(S) – [3' – (1' – methylindolyl)methyl] – 1 – methyl – 5 – methylthio – 2H – 1,4 – benzodiazepin – 2 – one

1,3 – Dihydro – 3(S) – (3' – indolyl)methyl – 2H – 1,4 – benzodiazepin – 2 – one – 5 – thione (450 mg, 1.4 mmole) was suspended in 30 ml of toluene, 8 ml of tetrahydrofuran, and 15 ml of 40% sodium hydroxide solution. This mixture was treated with 203 mg (0.6 mmole) of tetra – n – butylammonium sulfate and 0.25 ml (4.0 mmole) of iodomethane and stirred rapidly at room temperature. After four hours the phases were separated and the aqueous layer extracted once with ethyl acetate. The combined organic extracts were washed with water (2 X 50 ml) and brine, then dried ($MgSO_4$) and concentrated in vacuo to afford a yellow oil. Preparative thick layer chromatography (hexane – ethyl acetate 2:1 v/v) afforded the title compound as a white solid. $R_f$ = 0.45 (2:1 hexane – ethyl acetate). The analytical sample was recrystallized from ethyl acetate – ether, m.p. 170 ° C; TLC, HPLC: 99% pure. Pmr ($CDCl_3$): according to theory (methyl proton resonate 2.46 ppm, 3.39 ppm, and 3.72 ppm respectively). MS (20 ev.): 363 ($M^+$), 184, 144.

| Elemental Analysis: $C_{21}H_{21}N_3OS$ | | | |
|---|---|---|---|
| Calc'd. : | N, 11.56; | C, 69.39; | H, 5.82. |
| Found: | N, 11.47; | C, 69.22; | H, 6.04. |

EXAMPLE 8

1,3 – Dihydro – 3(S) – (3' – indolyl)methyl – 1 – methyl – 5 – methylthio – 2H – 1,4 – benzodiazepin – 2 – one

1,3 – Dihydro – 3(S) – (3' – indolyl)methyl – 2H – 1,4 – benzodiazepin – 2 – one – 5 – thione (450 mg, 1.4 mmole) was suspended in 30 ml of toluene, 8 ml of tetrahydrofuran, and 15 ml of 40% sodium hydroxide solution. The mixture was treated with 203 mg (0.6 mmole) of tetra – n – butylammonium sulfate and 0.25 ml (4.0 mmole) of iodomethane and stirred rapidly at room temperature. After four hours the phases were separated and the aqueous layer extracted once with ethyl acetate. The combined organic extracts were washed with water (2 X 50 ml) and brine, then dried ($MgSO_4$) and concentrated in vacuo to afford a yellow oil. Preparative thick layer chromatography (hexane – ethyl acetate 2:1 v/v) afforded the title compound as a white solid. $R_f$ = 0.40 (2:1 hexane – ethyl acetate). The analytical sample was recrystallized from ethyl acetate – ether, m.p. 90 – 91 ° C. TLC, HPLC: 99% pure. Pmr ($CDCl_3$): according to theory (methyl protons resonate at 2.45 ppm and 3.40 ppm, respectively). MS (20 ev): 349 ($M^+$), 302, 220, 130.

| Elemental Analysis: $C_{20}H_{19}N_3OS$. | | | |
|---|---|---|---|
| Calc'd. : | N, 12.02; | C, 68.74; | H, 5.48. |
| Found: | N, 12.10; | C, 68.58; | H, 5.71. |

EXAMPLE 9

1,3-Dihydro-5-(2-fluorophenyl)-3(R)-(3'-α-indolenyl) methyl-2H-1,4-benzodiazepin-2-one

1,3-Dihydro-5-(2-fluorophenyl)-3(R)-(3'-indolyl)methyl-2H-1,4-benzodiazepin-2-one (120 mg, 0.31 mmole) was dissolved in 2 ml of trifluoroacetic acid. The resulting orange solution was treated with 0.5 ml (3.1 mmole) of triethylsilane and stirred rapidly at room temperature. After two hours, the reaction mixture was rotoevaporated to dryness and the residue was partitioned between water/ethyl acetate. The organic phase was washed with sodium bicarbonate solution (sat.), and brine, then dried ($MgSO_4$) and concentrated. The analytical sample was obtain via preparative thick layer chromatography on silica gel (1:1 hexane-ethyl acetate v/v, multiple elutions).
$R_f$ = 0.38 (2:1 ethyl acetate-hexane).
Pmr ($CDCl_3$): in accord with theory.
MS (FAB): 386 (M+H).

| Elemental Analysis: $C_{24}H_{20}FN_3O \cdot 0.4H_2O$ | | | |
|---|---|---|---|
| Calc'd. : | N, 10.70; | C, 73.41; | H, 5.34. |
| Found: | N, 10.50; | C, 73.62; | H, 5.45. |

EXAMPLE 10

1,3-Dihydro-5-(2-fluorophenyl)-3(R)-(3'-β-indolenyl) methyl-2H-1,4-benzodiazepin-2-one

1,3-Dihydro-5-(2-fluorophenyl)-3(R)-(3'-indolyl)methyl-2H-1,4-benzodiazepin-2-one (120 mg, 0.31 mmole) was dissolved in 2 ml of trifluoroacetic acid. The resulting orange solution was treated with 0.5 ml (3.1 mmole) of triethylsilane and stirred rapidly at room temperature. After two hours, the reaction mixture was rotoevaporated to dryness and the residue was partitioned between water/ethyl acetate. The organic phase was washed with sodium bicarbonate solution (sat.), and brine, then dried ($MgSO_4$) and concentrated. The analytical sample was obtained via preparative thick layer chromatography on silica gel (1:1 hexane-ethyl acetate v/v, multiple elutions). $R_f$ = 0.30 (2:1 ethyl acetate-hexane). Pmr ($CDCl_3$): in accord with theory. MS (FAB): 386 (M+H).

| Elemental Analysis: $C_{24}H_{20}FN_3O \cdot 0.3H_2O$ | | | |
|---|---|---|---|
| Calc'd. : | N, 10.75; | C, 73.75; | H, 5.31. |
| Found: | N, 10.57; | C, 73.86; | H, 5.38. |

EXAMPLE 11

1,3-Dihydro-5-(2-fluorophenyl)-3(R)-(3'-indolyl)methyl-2H-1,4-benzodiazepin-2-thione

1,3-Dihydro-5-(2-fluorophenyl)-3(R)-(3'-indolyl)methyl-2H-1,4-benzodiazepin-2-one (6.98 g, 18.20 mmole) was refluxed with 4.41 g (10.92 mmole) of 2,4-bis-(4-methoxyphenyl)-2,4-dithioxo-1,3,2,4-dithiadiphosphetane in 100 ml of toluene for 1.5 hours. The solvent was removed in vacuo and the residue partitioned between ethylacetate and 10% sodium hydroxide solution. The organic phase was washed with 10% sodium hydroxide (3 X 50 ml) and brine, then dried ($MgSO_4$) and rotoevaporated to give an orange oil (10 g). Plug filtration of the crude product through silica gel (100 g) afforded a solid which was recrystallized from ether to afford the analytical sample.
m.p. 147-148°C.
Pmr: according to theory.

EXAMPLE 12

1,3-Dihydro-5-(2-fluorophenyl)-3(R)-(3'-indolyl)methyl-2H-1,4-benzodiazepine

To a solution of 1,3-dihydro-5-(2-fluorophenyl)-3(R)-(3'-indolyl)methyl-2H-1,4-benzodiazepin-2-thione (178 mg, 0.44 mmole) in 20 ml of absolute ethanol was added at room temperature one spatula of moist (ethanol) Raney-nickel catalyst (freshly prepared according to Fieser and Fieser, "Reagents for Organic Synthesis", Vol. I, p. 729, John Wiley & Sons., Inc. N.Y., 1967). The resulting suspension was protected from moisture and stirred rapidly for one hour. The reaction mixture was filtered and the filtrate concentrated to give 150 mg of a yellow oil. Purification via silica gel chromatography (chloroform-methanol-ammonia 95:5:0.5 v/v) afforded the analytical sample.

TLC, HPLC: confirmed purity.

MS (20 ev): 369 ($M^+$), 239, 212, 130, 83.

Pmr ($CDCl_3$): according to theory.

| Elemental Analysis: $C_{24}H_{20}FN_3 \cdot 0.07\ CHCl_3$. | | | |
|---|---|---|---|
| Calc'd. : | N, 11.12; | C, 76.52; | H, 5.35. |
| Found: | N, 10.90; | C, 76.66; | H, 5.59. |

EXAMPLE 13

7-Chloro-1,3-dihydro-3(R)-benzyl-5-phenyl-2H-1,4-benzodiazepin-2-one

The procedure of Example 1 was carried out using 2-amino-5-chlorobenzophenone (2.32 gm, 0.01 mol), Boc-D-Phenylalanine (2.65 gm, 0.01 mol), and DCC (10 ml of 1.0 M solution in $CH_2Cl_2$) in $CH_2Cl_2$ (10 ml). After filtration and evaporation, the crude solid was deprotected and cyclized by the procedure of Example 2. After stirring 5 days, the mixture was evaporated in vacuo, treated with $H_2O$ (50 ml), and extracted with EtoAc (2 x 100 ml). The combined organic extracts were washed with brine (50 ml), dried over $MgSO_4$, filtered and evaporated to dryness in vacuo. Chromatography on silica gel eluted with 7.5% (v/v) $Et_2O$ in $CH_2Cl_2$ gave a white foam which was crystallized from $Et_2O$. The solid was dried in vacuo at 65 °C: (m.p. 154-7 °C).

The compound showed a single spot by TLC ($R_f$ = 0.32, silica gel plate eluted with 10% (v/v) $Et_2O$ in $CH_2Cl_2$). The NMR spectrum was consistent with the title structure. The compound was 100% pure by HPLC.

| Anal. Calc'd for $C_{22}H_{17}ClN_2O$: | | | | |
|---|---|---|---|---|
| | C, 73.23; | H, 4.75; | N, 7.76; | Cl, 9.83. |
| Found: | C, 73.59; | H, 4.78; | N, 7.95; | Cl, 10.03. |

EXAMPLE 14

7-Chloro-1,3-dihydro-3(R)-(2-methyl-1-propyl)-5-phenyl-2H-1,4-benzodiazepin-2-one

The procedure of Example 1 was carried out using 2-amino-5-chlorobenzophenone (2.32 gm, 0.01 ml), Boc-D-Leucine monohydrate (2.49 gm, 0.01 mol), and DCC (10 ml of 1.0 M solution in $CH_2Cl_2$) in $CH_2Cl_2$ (25 ml). Filtration, concentration in vacuo and chromatography (silica gel, 5% (v/v) $Et_2O$ in $CH_2Cl_2$) gave a yellow oil which was deprotected and cyclized by the procedure of Example 2. After stirring 48 h, the mixture was evaporated in vacuo, treated with $H_2O$ (50 ml), and extracted with EtOAc (2 x 200 ml). The combined organic extracts were washed with brine (50 ml), dried over $MgSO_4$, filtered, and evaporated to dryness in vacuo. Chromatography (silica gel, 7.5% (v/v) $Et_2O$ in $CH_2Cl_2$) of the crude product gave a white foam which was crystallized from $Et_2O$. The solid was dried in vacuo at 65 °C: (m.p. 156-60 °C).

The compound showed a single spot by TLC ($R_f$ = 0.38, silica gel plate, 10% (v/v) $Et_2O$ in $CH_2Cl_2$). The NMR spectrum was consistent with the title structure. The compound was 100% pure by HPLC.

| Anal. Calc'd for $C_{19}H_{19}ClN_2O$: | | | | |
|---|---|---|---|---|
| | C, 69.82; | H, 5.86; | N, 8.57; | Cl, 10.85. |
| Found: | C, 69.81; | H, 5.84; | N, 8.71; | Cl, 11.20. |

EXAMPLE 15

3(R)-Benzyloxymethyl-7-chloro-1,3-dihydro-5-phenyl-2H-1,4-benzodiazepin-2-one

The procedure of Example 1 was carried out using 2-amino-5-chlorobenzophenone (2.32 gm, 0.01 mol), N-Boc-O-Benzyl-D-serine (2.95 gm, 0.01 mol), and DCC (10 ml of 1.0 M solution in $CH_2Cl_2$) in $CH_2Cl_2$ (10 ml). Filtration, concentration in vacuo and chromatography (silica gel, $CH_2Cl_2$) gave a colorless oil which was deprotected and cyclized by the procedure of Example 2. After stirring 5 days, the mixture was evaporated in vacuo, treated with $H_2O$ (50 ml), and extracted with EtOAc (2 x 100 ml). The combined organic extracts were washed with brine (50 ml), dried over $MgSO_4$, filtered, and evaporated to dryness in vacuo. Chromatography (silica gel, 75% (v/v) $Et_2O$ in $CH_2Cl_2$) of the crude product gave a white foam which was crystallized from $Et_2O$. The solid was dried in vacuo at 65°C: (m.p. 113-5°C).

The compound showed a single spot by TLC ($R_f$=0.27, silica gel plate, 10% (v/v) $Et_2O$ in $CH_2Cl_2$). The NMR spectrum was consistent with the title structure and verified the presence of $Et_2O$ and $H_2O$. The compound was 100% pure by HPLC.

| Anal. Calc'd for $C_{23}H_{19}ClN_2O_2 \cdot 0.1\ C_4H_{10}O \cdot 0.25\ H_2O$: | | | | |
|---|---|---|---|---|
| | C, 69.78; | H, 5.13; | N, 6.96; | Cl, 8.80. |
| Found: | C, 69.53; | H, 5.17; | N, 6.99; | Cl, 8.98. |

EXAMPLE 16

7-Chloro-1,3-dihydro-3(R)-(4-benzyloxybenzyl)-5-phenyl-2H-1,4-benzodiazepin-2-one

The procedure of Example 1 was carried out using 2-amino-5-chlorobenzophenone (2.32 gm, 0.01 mol), N-Boc-O-Benzyl-D-Tyrosine (3.71 gm, 0.01 mol), and DCC (10 ml of 1.0 M solution in $CH_2Cl_2$) in $CH_2Cl_2$ (10 ml). After filtration and evaporation, the crude solid was deprotected and cyclized by the procedure of Example 2. After stirring 5 days, the mixture was evaporated in vacuo, treated with $H_2O$ (75 ml), and extracted with EtOAc (2 x 125 ml). The combined organic extracts were washed with brine (50 ml), dried over $MgSO_4$, filtered, and evaporated to dryness in vacuo. Chromatography (silica gel, 7.5% (v/v) $Et_2O$ in $CH_2Cl_2$) of the crude product gave a white foam which was dried at 69°C in vacuo: (m.p. 97-101°C).

The compound showed a single spot by TLC ($R_f$=0.37, silica gel plate, 10% (v/v) $Et_2O$ in $CH_2Cl_2$). The NMR spectrum was consistent with the title structure. The compound was greater than 99.5% pure by HPLC.

| Anal. Calc'd for $C_{29}H_{23}ClN_2O_2$: | | | |
|---|---|---|---|
| | C, 74.59; | H, 4.97; | N, 6.00. |
| Found: | C, 74.52; | H, 4.78; | N, 6.01. |

EXAMPLE 17

7-Chloro-1,3-dihydro-3(RS)-(1-naphthyl)methyl-5-phenyl-2H-1,4-benzodiazepin-2-one

The procedure of Example 1 was carried out using 2-amino-5-chlorobenzophenone (845 mg, 3.65 mmol), N-Boc-α-DL-naphthylalanine (1.15 gm, 3.65 mmol), and DCC (3.65 ml of 1.0 M solution in $CH_2Cl_2$) in THF (5 ml). Filtration, concentration in vacuo and chromatography (silica gel, 1% (v/v) $Et_2O$ in

$CH_2Cl_2$) gave a light yellow foam which was deprotected and cyclized by the procedure of Example 2. After stirring 14 days, the mixture was evaporated in vacuo, treated with $H_2O$ (25 ml), and extracted with $CH_2Cl_2$ (2 x 50 ml). The combined organic extracts were washed with brine (25 ml), dried over $MgSO_4$, filtered, and evaporated to dryness in vacuo. Chromatography (silica gel, 3% (v/v) $Et_2O$ in $CH_2Cl_2$) of the crude product gave a white foam which was crystallized from hexane. The solid was dried in vacuo at 100°C: (m.p. 180–2°C).

The compound showed a single spot by TLC ($R_f$ = 0.36. silica gel plate, 10% (v/v) $Et_2O$ in $CH_2Cl_2$). The NMR spectrum was consistent with the title structure. The compound was greater than 99.9% pure by HPLC.

| Anal. Calc'd for $C_{26}H_{19}ClN_2O$: | | | | |
|---|---|---|---|---|
| | C, 76.00; | H, 4.66; | H, 6.82; | Cl, 8.63. |
| Found: | C, 75.99; | H, 4.68; | N, 6.65; | Cl, 8.76. |

EXAMPLE 18

7-Chloro-1,3-dihydro-3(RS)-(2-naphthyl)methyl-5-phenyl-2H-1,4-benzodiazepin-2-one

The procedure of Example 1 was carried out using 2-amino-5-chlorobenzophenone (845 mg, 3.65 mmol), N-Boc-$\beta$-DL-naphthylalanine (1.15 gm, 3.65 mmol), and DCC (3.65 ml of 1.0 M solution in $CH_2Cl_2$) in THF (5 ml). Filtration, concentration in vacuo and chromatography (silica gel, 1% (v/v) $Et_2O$ in $CH_2Cl_2$) gave a foam which was deprotected and cyclized by the procedure of Example 2. After stirring 24 hours, the mixture was evaporated in vacuo, treated with $H_2O$ (25 ml), and extracted with EtOAc (2 x 50 ml). The combined organic extracts were washed with brine (25 ml), dried over $MgSO_4$, filtered, and evaporated to dryness in vacuo. Chromatography (silica gel, 5% (v/v) $Et_2O$ in $CH_2Cl_2$) of the crude product gave a foam which was crystallized from $Et_2O$/hexane. The solid was dried in vacuo at 100°C: (m.p. 140–2°C).

The compound showed a single spot by TLC ($R_f$ = 0.38, silica gel plate, 10% (v/v) $Et_2O$ in $CH_2Cl_2$). The NMR spectrum was consistent with the title structure. The compound was greater than 99.7% pure by HPLC.

| Anal. Calc'd for $C_{26}H_{19}ClN_2O$: | | | | |
|---|---|---|---|---|
| | C, 76.00; | H, 4.66; | N, 6.82; | Cl, 8.63. |
| Found: | C, 75.77; | H, 4.68; | N, 6.77; | Cl, 8.87. |

EXAMPLE 19

1,3-Dihydro-5-(2-fluorophenyl)-3(RS)-(2-thienyl)methyl-2H-1,4-benzodiazepin-2-one

The procedure of Example 1 was carried out using 2-amino-2'-fluorobenzophenone (1.26 gm, 5.86 mmol), N-Boc-$\beta$-(2-thienyl)-DL-alanine (1.75 gm, 6.45 mmol), and DCC (6.45 ml of 1.0M solution in $CH_2Cl_2$) in $CH_2Cl_2$ (25 ml). Filtration, concentration in vacuo and flash chromatography (silica gel, 1% (v/v) $Et_2O$ in $CH_2Cl_2$) gave a white foam which was deprotected and cyclized by the procedure of Example 2. After stirring 3 days, the mixture was evaporated in vacuo, treated with $H_2O$ (50 ml) and extracted with EtOAc (2 x 100 ml). The combined organic extracts were washed with brine (50 ml), dried over $MgSO_4$, filtered, and evaporated to dryness in vacuo. The resulting foam was crystallized from $Et_2O$ to give the title compound as a white solid. The solid was dried in vacuo at 65°C: (m.p. 189–91°C).

The compound showed a single spot by TLC ($R_f$ = 0.54, silica gel plate, 20% (v/v) $Et_2O$ in $CH_2Cl_2$).

The NMR spectrum was consistent with the title structure. The compound was greater than 97.9% pure by HPLC.

| Anal. Calc'd for $C_{20}H_{15}FN_2OS$: | | | |
|---|---|---|---|
| | C, 68.55; | H, 4.32; | N, 8.00. |
| Found: | C, 68.74; | H, 4.47; | N, 8.02. |

EXAMPLE 20

1,3-Dihydro-5-(2-fluorophenyl)-3(RS)-(3-thienyl)-2H-1,4-benzodiazepin-2-one

The procedure of Example 1 was carried out using 2-amino-2'-fluorobenzophenone (1.59 g, 7.40 mmol), DL-α-Boc-amino-3-thiopheneacetic acid (2.0 gm, 7.77 mmol), and DCC (7.77 ml of 1.0M solution in $CH_2Cl_2$) in $CH_2Cl_2$ (15 ml). Filtration, concentration in vacuo and chromatography (silica gel, 3% (v/v) $Et_2O$ in $CH_2Cl_2$) gave a white foam which was deprotected (HCl/EtoAc, 0°) and cyclized by heating (70°C oil bath) in MeOH for 48 hours. The solvent was removed in vacuo and the residue crystallized from $Et_2O$. The compound was dried in vacuo at 65°C: (m.p. 219-23°C).

The compound showed a single spot by TLC ($R_f$=0.24, silica gel plate, 30% (v/v) EtOAc in hexane). The NMR spectrum was consistent with the title structure. The compound was greater than 98.5% pure by HPLC.

| Anal. Calc'd for $C_{19}H_{13}FN_2OS$: | | | |
|---|---|---|---|
| | C, 67.84; | H, 3.90; | N, 8.33. |
| Found: | C, 67.75; | H, 4.13; | N, 7.98. |

EXAMPLE 21

1,3-Dihydro-5-(2-fluorophenyl)-3(R)-[3'-ß-(1'-t-Boc-L-leucyl)-indolenyl]methyl-2H-1,4-benzodiazepin-2-one

1,3-Dihydro-5-(2-fluorophenyl)-3(R)-(3'-β-indolenyl)methyl-2H-1,4-benzodiazepin-2-one (100 mg, 0.259 mmol), N-Boc-L-Leucine monohydrate (64.7 mg, 0.259 mmol), 1-ethyl-3-(3-dimethylaminopropyl)carbodiimide hydrochloride (EDC, 49.8 mg, 0.259 mmol), and 1-hydroxybenzotriazole hydrate (HBT, 35.0 mg, 0.259 mmol) were combined in freshly degassed dimethylformamide (DMF, 2 ml) and stirred at room temperature. The pH of the solution was adjusted to 9.0-9.5 with triethylamine (0.108 ml, 0.777 mmol) and stirring was continued for 24 hours. The mixture was evaporated in vacuo, treated with 10% $Na_2CO_3$ (aq) (20 ml) and extracted with EtOAc (2 x 30 ml). The combined extracts were washed with $H_2O$ (20 ml) and brine (20 ml), dried over $MgSO_4$, filtered, and evaporated to dryness in vacuo. The residue was chromatographed (silica gel, 30% (v/v) EtOAc in hexane) to give the title compound as a foam. The foam was dried in vacuo at 65°C: (m.p. 118-30°C).

The compound showed a single spot by TLC ($R_f$=0.38, silica gel plate, 40% (v/v) EtOAc in hexane). The NMR spectrum was consistent with the title structure and verified the presence of hexane. The compound was greater than 97% pure by HPLC. The mass spectrum showed a molecular ion at m/e = 598.

| Anal. Calc'd for $C_{35}H_{39}FN_4O_4.1/3C_6H_{14}$: | | | |
|---|---|---|---|
| | C, 70.83; | H, 7.02; | N, 8.93. |
| Found: | C, 70.93; | H, 6.88; | N, 8.94. |

EXAMPLE 22

**1,3-Dihydro-5-(2-fluorophenyl)-3(R)-[3'-ß-(1'-t-Boc-D-leucyl)-indolenyl]methyl-2H-1,4-benzodiazepin-2-one**

The procedure of Example 21 was carried out using the same reagents and amounts except N-Boc-D-leucine monohydrate was substituted for N-Boc-L-leucine monohydrate. After 24 hours a second portion of Boc-D-Leucine monohydrate (32 mg, 0.129 mmol), EDC (25 mg, 0.130 mmol), and HBT (17.5 mg; 0.130 mmol) was added and the pH readjusted to 9.0-9.5 with $Et_3N$. The reaction was worked up as in Example 21, and the title compound was obtained as a foam. This was dried in vacuo at 65°C: (m.p. 135-48°C).

The compound showed a single spot by TLC ($R_f$=0.37, silica gel plate, 40% (v/v) EtOAc in hexane). The NMR spectrum was consistent with the title structure. The compound was 87.5% pure by HPLC.

| Anal. Calc'd for $C_{35}H_{39}FN_4O_4$: | | | |
|---|---|---|---|
| | C, 70.21; | H, 6.57; | N, 9.36. |
| Found: | C, 70.25; | H, 6.89; | N, 9.53. |

EXAMPLE 23

1,3-Dihydro-5-(2-fluorophenyl)-3(R)-[3'-α-(1'-t-Boc-L-leucyl)-indolenyl]methyl-2H-1,4-benzodiazepin-2-one

The procedure of Example 21 was carried out using the same reagents and quantities except 1,3-dihydro-5-(2-fluorophenyl)-3(R)-(3'-α-indolenyl) methyl-2H-1,4-benzodiazepin-2-one was substituted for the 3'-β isomer. After 24 hours the reaction was worked up in the same manner and the title compound was obtained as a foam. This was dried in vacuo at 65°C: (m.p. 130-48°C).

The compound showed a single spot by TLC ($R_f$=0.39, silica gel plate, 40% (v/v) EtOAc in hexane). The NMR spectrum was consistent with the title compound. The compound was 91% pure by HPLC,

| Anal. Calc'd for $C_{35}H_{39}FN_4O_4$: | | | |
|---|---|---|---|
| | C, 70.21; | H, 6.57; | N, 9.36. |
| Found: | C, 70.54; | H, 6.98; | N, 9.39. |

EXAMPLE 24

**1,3-Dihydro-5-(2-fluorophenyl)-3(R)-[3'-α-(1'-t-Boc-D-leucyl)-indolenyl]methyl-2H-1,4-benzodiazepin-2-one**

The procedure of Example 23 was carried out using the same reagents and quantities except Boc-D-Leucine was substituted for Boc-L-Leucine. After 24 hours the reaction was worked up in the same manner and the title compound was obtained as a white foam. This was dried in vacuo at 65°C: (m.p. 130-145°C).

The compound showed a single spot by TLC ($R_f$=0.39, silica gel plate, 40% (v/v) EtOAc in hexane). The NMR spectrum was consistent with the title structure. The compound was 95.1% pure by HPLC.

| Anal. Calc'd for $C_{35}H_{39}FN_4O_4$: | | | |
|---|---|---|---|
| | C, 70.21; | H, 6.57; | N, 9.36. |
| Found: | C, 70.31; | H, 6.81; | N, 9.67. |

EXAMPLE 25

7-Chloro-1,3,4,5-tetrahydro-3(R)-(3'-indolyl)methyl-5-phenyl-2H-1,4-benzodiazepin-2-one

7-Chloro-1,3,dihydro-3(R)-(3'-indolyl)methyl-5-phenyl-2H-1,4-benzodiazepin-2-one etherate (240 mg, 0.506 mmol) was dissolved in acetic acid (10 ml) and cooled to 10°C. To the yellow solution was added sodium cyanoborohydride (63.6 mg, 1.01 mmol) all at once. After stirring 15 minutes at 10°C, the reaction was diluted with $H_2O$ (10 ml), basified with sat'd $Na_2CO_3$ (aq.), and extracted with EtOAc (2 x 25 ml). The combined organic extracts were washed with brine, dried over $MgSO_4$, filtered, and evaporated to dryness in vacuo. The residue was chromatographed (silica gel, 900/10/1/1 (v/v/v/v) of $CH_2Cl_2$/MeOH/$H_2O$/HoAc) and the product fractions evaporated to dryness in vacuo. The residue was dissolved in absolute ethanol, filtered, and treated with 5.37 M HCl in ethanol until the solution was acidic. The product crystallized as fine white needles which were dried in vacuo at 82°C: (m.p. 198-204°C).

The compound showed a single spot by TLC ($R_f$=0.35, silica gel plate, 300/10/1/1 (v/v/v/v) $CH_2Cl_2$/MeOH/$H_2O$/HoAc). The NMR spectrum was consistent with the title structure and verified the presence of $H_2O$. The mass spectrum showed a molecular ion at m/e = 401.

| Anal. Calc'd for $C_{24}H_{20}ClN_3O \cdot HCl \cdot 0.75H_2O$: | | | | |
|---|---|---|---|---|
| | C, 63.79; | H, 5.02; | N, 9.30; | Cl, 15.69. |
| Found: | C, 63.59; | H, 4.94; | N, 9.39; | Cl, 15.32. |

EXAMPLE 26

7-Chloro-1,3,4,5-tetrahydro-3(S)-(3'-indolyl)methyl-5-phenyl-2H-1,4-benzodiazepin-2-one

7-Chloro-1,3-dihydro-3(S)-(3'-indolyl)methyl-5-phenyl-2H-1,4-benzodiazepin-2-one (300 mg, 0.750 mmol) was dissolved in acetic acid (10 ml) and cooled to 10°C. To the yellow solution was added sodium cyanoborohydride (63.6 mg, 1.01 mmol) all at once. After stirring 15 minutes at 10°C, the reaction was diluted with $H_2O$ (10 ml), basified with sat'd $Na_2CO_3$(aq.), and extracted with EtOAc (2 x 25 ml). The combined organic extracts were washed with brine (10 ml), dried over $MgSO_4$, filtered, and evaporated to dryness in vacuo. The crude residue was dissolved in absolute ethanol (3 ml), filtered, and treated with 5.37M ethanolic HCl until the solution was acidic. The product crystallized as fine white needles which were dried in vacuo at 82°C: (m.p. 198-204°C).

The compound showed a single spot by TLC ($R_f$=0.30, silica gel plate, 300/10/1/1 (v/v/v/v) of $CH_2Cl_2$/MeOH/$H_2O$/HoAc). The NMR spectrum was consistent with the title structure and verified the presence of $H_2O$ and ethanol.

| Anal. Calc'd for $C_{24}H_{20}ClN_3O \cdot HCl \cdot 0.5\ H_2O.0.25\ C_2H_5OH$: | | | | |
|---|---|---|---|---|
| | C, 64.12; | H, 5.16; | N, 9.16; | Cl, 15.45. |
| Found: | C, 63.91; | H, 5.02; | N, 9.01; | Cl, 15.36. |

EXAMPLE 27

**4-(p-Chlorobenzoyl)-5-(2-fluorophenyl)-3(R)-[3'-(1'-methylindolyl)-methyl]-1-methyl-1,3,4,5-tetrahydro-2H-1,4-benzodiazepin-2-one (A) and 4-acetyl-5-(2-fluorophenyl)-3(R)-[3'-(1'-methylindolyl)-methyl]-1-methyl-1,3,4,5-tetrahydro-2H-1,4-benzodiazepin-2-one (B)**

The procedure of Example 25 was carried out using 1,3-dihydro-5-(2-fluorophenyl)-3(R)-[3'-(1'-methylindolyl)-methyl]-1-methyl-2H-1,4-benzodiazepin-2-one (1.0 gm, 2.43 mmol) and sodium cyanoborohydride (305 mg, 4.86 mmol) in glacial acetic acid (4 ml). The crude reduction product obtained upon evaporation of the EtOAc extracts was used without further purification.

A The crude reduction product (200 mg, 0.484 mmol) was partitioned between $CH_2Cl_2$ (6 ml) and $H_2O$ (5 ml) and cooled to 0°C. 1N NaOH (0.73 ml) was added, followed by p-chlorobenzoyl chloride (.092 ml, 0.726 mmol). After 24 hours at ambient temperature, a second portion of 1N NaOH (0.50 ml) and p-chlorobenzoyl chloride (.045 ml, 0.354 mmol) was added, and after 24 hours a third portion of 1N NaOH ( 50 ml) and p-chlorobenzoylchloride (.045 ml, 0.354 mmol) was added. After another 24 hours, the mixture was extracted with $CH_2Cl_2$ (3 x 10 ml). The combined organic layers were washed with 10% $NaHCO_3$ (10 ml), $H_2O$ (10 ml), and brine (10 ml), dried over $MgSO_4$, filtered, and evaporated in vacuo. Chromatography (silica gel, 5% (v/v) $Et_2O$ in $CH_2Cl_2$) of the crude residue gave a foam which was crystallized from $Et_2O$. The compound was dried in vacuo at 78°C: (m.p. 237-43°C).

| Anal. Calc'd for $C_{33}H_{27}FClN_3O_2$.0.05 $Et_2O$: | | | | |
|---|---|---|---|---|
| | C, 71.75; | H, 4.99; | N, 7.56; | Cl, 6.38. |
| Found: | C, 71.84; | H, 5.28; | N, 7.92; | Cl, 6.63. |

The compound showed a single spot by TLC ($R_f$=0.50, silica gel plate, 4% (v/v) $Et_2O$ in $CH_2Cl_2$). The NMR spectrum was consistent with the title structure and verified the presence of $Et_2O$. The compound was greater than 99% pure by HPLC.

B: The crude reduction product (200 mg, 0.484 mmol) was dissolved in $CH_2Cl_2$ (10 ml) and 3 portions of acetyl chloride (each 0.026 ml, 0.363 mmol) and triethylamine (0.35 ml, 0.363 mmol) were added at 3 hour intervals. Water (2 ml) was then added and the mixture was extracted with $CH_2Cl_2$ (3 x 10 ml). The combined organic layers, were washed with 10% $Na_2CO_3$ (aq.) (10 ml), $H_2O$ (10 ml) and brine (10 ml), dried over $MgSO_4$, filtered, and evaporated in vacuo. Chromatography (silica gel, 5% (v/v) $Et_2O$ in $CH_2Cl_2$) of the crude residue gave a white foam which was crystallized from $Et_2O$. The compound was dried in vacuo at 78°C: (m.p. 214-216.5°C).

The compound showed a single spot by TLC ($R_f$=0.41, silica gel plate, 15% (v/v) $Et_2O$ in $CH_2Cl_2$). The NMR spectrum was consistent with the title structure. The compound was greater than 99.5% pure by HPLC. The mass spectrum showed a molecular ion at m/e = 455.

| Anal. Calc'd for $C_{28}H_{26}FN_3O_2$: | | | |
|---|---|---|---|
| | C, 73.82; | H, 5.75; | N, 9.23. |
| Found: | C, 73.62; | H, 5.93; | N, 9.22. |

EXAMPLE 28

7-Chloro-5-(2-chlorophenyl)-1,3-dihydro-3(R)-(3'-indolyl)methyl-2H-1,4-benzodiazepin-2-one

The procedure of Example 1 was carried out using 2-amino-2',5-dichlorobenzophenone (2.66 g, 0.01 mole), Boc-D-tryptophan (3.04 g, 0.01 mole), and DCC (10 ml of 1 M solution in methylene chloride) in THF (15 ml). The crude product obtained after filtration and evaporation of the mixture was chromatographed on silica gel (230-400 mesh, 9 inch (23 cm) column 55 mm diameter), using methylene chloride followed by 5% (v/v) ether/methylene chloride. The product fractions were evaporated in vacuo to give the product as a foam. This material was deprotected and cyclized using the procedure of Example 2. The cyclization in this case required 15 days. At the end of this time the mixture was evaporated in vacuo, treated with water (10 ml), and extracted with methylene chloride (3 x 50 ml). The methylene chloride layers were dried over potassium carbonate, filtered, and evaporated in vacuo to give the crude product as a foam. This material was chromatographed on silica gel (230-400 mesh, 8 inch (20 cm) column, 25 mm diameter, elution with methylene chloride followed by 10% (v/v) ether/methylene chloride). The product fractions were evaporated in vacuo and the residue crystallized from ether by addition of cyclohexane. The title compound was obtained as a white solid which was dried in vacuo at 80°: (mp 140-170° (d)).

The compound showed a single spot by TLC ($R_f$ = 0.61, silica gel plate eluted with 1:1 (v/v) ether/methylene chloride). The NMR spectrum was consistent with the title structure. The mass spectrum showed a molecular ion at m/e = 433. The compound was 98% pure by HPLC.

| Analysis Calc'd for $C_{24}H_{17}Cl_2N_3O$: | | | |
|---|---|---|---|
| | C, 66.37; | H, 3.94; | N, 9.68: |
| Found: | C, 66.70; | H, 4.05; | N, 9.61. |

EXAMPLE 29

1,3-Dihydro-3(R)-(3'-indolyl)methyl-5-methyl-2H-1,4-benzodiazepin-2-one

The procedure of Example 1 was carried out using 2-aminobenzophenone (1.35 g, 0.01 mole), Boc-D-tryptophan (3.04 g, 0.01 mole), and DCC (10 ml of 1M solution in methylene chloride) in THF (15 ml). The mixture was filtered, evaporated in vacuo and the residue chromatographed on silica gel (230-400 mesh, 9 inch (23 cm) column, 55 mm diameter) eluted with methylene chloride followed by 5%, 7-1/2% and 8% (v/v) ether/methylene chloride. The product fractions were evaporated in vacuo and the residue was deprotected and cyclized by the procedure of Example 2. The cyclization required seven days. The mixture was evaporated in vacuo and partitioned between water and methylene chloride. The methylene chloride layers were washed twice with water, dried over magnesium sulfate, filtered and evaporated in vacuo. The residue was chromatographed on silica gel (230-400 mesh, 11 inch (28 cm) column, 25 mm diameter, 1:1 and 2:1 (v/v) ether/methylene chloride elution). The product fractions were evaporated in vacuo to provide the title compound: (mp 185-190°). The compound was dried in vacuum at 100° overnight.

The compound showed a single spot by TLC ($R_f$=0.29, silica gel plate eluted with 1:1 (v/v) ether/methylene chloride). The NMR spectrum was consistent with the title structure. The mass spectrum showed a molecular ion at m/e=303. The compound was 95.6% pure by HPLC.

| Analysis Calc'd for: $C_{19}H_{17}N_3O \cdot 0.1H_2O$: | | | |
|---|---|---|---|
| | C, 74.78; | H, 5.68; | N, 13.78; |
| Found: | C, 74.60; | H, 6.06; | N, 13.74. |

EXAMPLE 30

1 – Benzyl – 7 – chloro – 1,3 – dihydro – 3(R) – (3' – indolyl) methyl – 5 – phenyl – 2H – 1,4 – benzodiazepin – 2 – one

The procedure of Example 4 was carried out using 7 – chloro – 1,3 – dihydro – 3(R) – (3' – indolyl) – methyl – 5 – phenyl – 2H – 1,4 – benzodiazepin – 2 – one etherate (0.1 g, 0.22 mmole) in place of 1,3 – dihydro – 5 – (2 – fluorophenyl) – 3(R) – (3' – indolyl)methyl – 2H – 1,4 – benzodiazepin – 2 – one, and 50% so – dium hydride in mineral oil (0.015 g, 0.31 mmole) in dry DMF (2 ml). In place of methyl iodide, benzyl bromide (0.058 g, 0.34 mmole) was added to the mixture. Chromatography on a 6 inch (15 cm), 15 mm diameter silica gel column with 5% (v/v) ether/methylene chloride elution and evaporation of the product fractions gave a residue which was recrystallized from cyclohexane to provide the title compound which was dried in vacuo at 60°: (mp ca. 80° (indistinct)).

The compound showed a single spot by TLC ($R_f$ = 0.66, silica gel plate eluted with 10% (v/v) ether/methylene chloride). The NMR spectrum was consistent with the title structure and verified the presence of approximately 1/2 mole of cyclohexane. The compound was 100% pure by HPLC. The mass spectrum showed a molecular ion at m/e = 489.

| Analysis Calc'd for: $C_{31}H_{24}ClN_3O \cdot 0.5C_6H_{12}$: | | | | |
|---|---|---|---|---|
| | C, 76.74; | H, 5.68; | N, 7.90; | Cl, 6.66; |
| Found: | C, 76.83; | H, 5.71; | N, 7.79; | Cl, 6.72. |

EXAMPLE 31

7 – Chloro – 1,3 – Dihydro – 3(R) – (3' – indolyl)methyl – 1 – methyl – 5 – phenyl – 2H – 1,4 – benzodiazepin – 2 – one

The procedure of Example 4 was carried out using 7 – chloro – 1,3 – dihydro – 3(R) – (3' – indolyl) – methyl – 5 – phenyl – 2H – 1,4 – benzodiazepin – 2 – one etherate (0.1 g, 0.22 mmole) in place of 1,3 – dihydro – 5 – (2 – fluorophenyl) – 3(R) – (3' – indolyl)methyl – 2H – 1,4 – benzodiazepin – 2 – one, 50% sodium hydride in mineral oil (0.014 g, 0.29 mmole), and methyl iodide (0.045 g, 0.32 mmole) in DMF (2 ml). Chromatography on a six inch (15 cm), 15 mm diameter silica gel column provide the title compound which, after evaporation and in vacuo, was dissolved in acetone, precipitated with water and filtered. The resulting solid was dried in vacuo at 70°:(mp 134 – 152 (indistinct)).

The compound showed a single spot by TLC ($R_f$ = 0.22, silica gel plate eluted with 5% (v/v) ether/methylene chloride. The NMR spectrum was consistent with the title structure. The compound was 98.9% pure by HPLC. The mass spectrum showed a molecular ion at m/e = 413.

| Analysis Calc'd for: $C_{25}H_{20}ClN_3O$: | | | | |
|---|---|---|---|---|
| | C, 72.54; | H, 4.87; | N, 10.15; | Cl, 8.57; |
| Found: | C, 72.38; | H, 4.88, | N, 10.20; | Cl, 8.32. |

EXAMPLE 32

1,3 – Dihydro – 5 – (2 – fluorophenyl) – 3(S) – (3' – indolyl) methyl – 2H – 1,4 – benzodiazepin – 2 – one

The procedure of Example 1 was carried out using 0.7 g (3.25 mmole) of 2 – amino – 2' – fluoroben – zophenone, 0.99 g, (3.25 mmole) of Boc – L – tryptophan, and 3.25 ml (3.25 mmole) of 1M DCC/$CH_2Cl_2$ in 5 ml of THF. The product obtained by silica gel chromatography (10 inch (25 cm) column, 25 mm diameter, methylene chloride and 2% and 3% (v/v) ether/methylene chloride elution) was deprotected and cyclized according to the procedure of Example 2. The cyclization required three days. The resulting mixture was evaporated in vacuo, partitioned between water and methylene chloride, and separated. The aqueous layer was extracted twice with methylene chloride, and the combined methylene chloride layers were washed

with water, dried over sodium sulfate, filtered, and evaporated in vacuo. The residue was recrystallized from acetone/ether, and the resulting solid dried in vacuo at 100°: (mp 255–257°).

The compound showed a single component by TLC ($R_f$ = 0.59, silica gel plate eluted with 1:1 (v/v) methylene chloride/ether. The NMR spectrum was consistent with the title structure. The mass spectrum showed a molecular ion at m/e = 383. The compound was 99.3% pure by HPLC.

| Analysis Calc'd for $C_{24}H_{18}FN_3O$: | | | |
|---|---|---|---|
| | C, 75.18; | H, 4.73; | N, 10.96; |
| Found: | C, 75.45; | H, 4.71; | N, 11.11. |

## EXAMPLE 33

### 1-Benzyl-7-chloro-1,3-dihydro-3(S)-(3'-indolyl) methyl-5-phenyl-2H-1,4-benzodiazepin-2-one

The procedure of Example 4 was carried out using 7-chloro-1,3-dihydro-3(S)-(3'-indolyl)-methyl-5-phenyl-2H-1,4-benzodiazepin-2-one etherate (0.1 g, 0.22 mmole) in place of 1,3-dihydro-5-(2-fluorophenyl)-3(R)-(3'-indolyl)methyl-2H-1,4-benzodiazepin-2-one, 50% sodium hydride in mineral oil (0.014 g, 0.29 mmole), and benzyl bromide (0.058 g, 0.34 mmole) in place of methyl iodide. The reaction was run in 1.5 ml of dry DMF. Silica gel chromatography (8 inch (20 cm) column, 15 mm diameter, methylene chloride and 5% (v/v) ether/methylene chloride elution)) and evaporation of the product fractions in vacuo gave the title compound which was dried in vacuo at 60°: (mp 80–120° (indistinct)).

The compound showed a single component by TLC ($R_f$ = 0.40, silica gel plate eluted with 5% (v/v) ether/methylene chloride). The NMR spectrum was consistent with the title structure and showed 1/2 mole of cyclohexane. The compound was 99.3% pure by HPLC. The mass spectrum showed a molecular ion at m/e = 489.

| Analysis Calc'd for $C_{31}H_{24}ClN_3O \cdot 1/2\ C_6H_{12}$: | | | | |
|---|---|---|---|---|
| | C, 76.74; | H, 5.68; | N, 7.90; | Cl, 6.66; |
| Found: | C, 76.56; | H, 5.67; | N, 7.86; | Cl, 7.00. |

## EXAMPLE 34

### 7-Chloro-1,3-dihydro-3(R)-(3'-indolyl)methyl-5-phenyl-2H-1,4-benzodiazepin-2-thione

7-Chloro-1,3-dihydro-3(R)-(3'-indolyl)methyl-5-phenyl-2H-1,4-benzodiazepin-2-one etherate (1.0 g, 2.1 mmole) and $P_2S_5$ (0.51 g, 2.3 mmole) were combined in dry pyridine (16 ml) and heated at reflux for 40 minutes. Pyridine was removed by evaporation in vacuo and the residue treated with ice water and extracted with methylene chloride. The methylene chloride layers were combined, dried over potassium carbonate, filtered, and evaporated in vacuo to give a foam. This material was chromatographed on silica gel (9 inch (23 cm) column, 25 mm diameter, 15% (v/v) ether/methylene chloride elution), and the product fractions evaporated. The residue was recrystallized from acetone/ethyl acetate and the solid dried in vacuo at 90°: (mp 279–280°).

The compound showed a single spot by thin layer chromatography ($R_f$ = 0.32, silica gel plate eluted with 10% (v/v) ether/methylene chloride). The NMR spectrum was consistent with the title structure. The compound was 98.6% pure by HPLC. The mass spectrum showed a molecular ion at m/e = 415.

| Analysis Calc'd for $C_{24}H_{18}ClN_3S$: | | | | |
|---|---|---|---|---|
| | C, 69.30; | H, 4.36; | N, 10.10; | S, 7.71; |
| Found: | C, 69.39; | H, 4.39; | N, 10.14; | S, 7.46. |

EXAMPLE 35

1,3 – Dihydro – 5 – (2 – fluorophenyl) – 3(R) – (3' – indolyl) methyl – 2H – 1,4 – benzodiazepin – 2 – [N' – (3 – thienoyl)] hydrazide

1,3 – Dihydro – 5 – (2 – fluorophenyl) – 3(R) – (3' – indolyl)methyl – 2H – 1,4 – benzodiazepin – 2 – thione (0.28 g, 0.7 mmole) and 3 – thienoyl chloride (0.1 g, 0.7 mmole) were combined in ether (5 ml) and THF (1 ml) and stirred at room temperature. After one hour the mixture was filtered and evaporated in vacuo, and the residue chromatographed on silica gel (8 inch (20 cm) column, 25 mm diameter, 1 – 1/2% followed by 3% (v/v) methanol/methylene chloride elution). The product fractions were evaporated in vacuo and the resulting solid dried in vacuo at 70°: (mp 207 – 209° ( )).

The compound showed a single spot by TLC ($R_f$ = 0.4, silica gel plate eluted with 5% (v/v) methanol/methylene chloride). The NMR spectrum was consistent with the title structure. The compound was 92% pure by HPLC.

| Analysis Calc'd for $C_{29}H_{22}FN_5OS \cdot 0.2H_2O$: | | | |
|---|---|---|---|
| | C, 68.13; | H, 4.42; | N, 13.70; |
| Found: | C, 68.19; | H, 4.30; | N, 13.91. |

EXAMPLE 36

1,3 – Dihydro – 1 – ethyl – 5 – (2 – fluorophenyl) – 3(R) – (3' – indolyl)methyl – 2H – 1,4 – benzodiazepin – 2 – one

The procedure of Example 4 was carried out using ethyl iodide (0.35 g, 2.25 mmole) in place of methyl iodide. Silica gel chromatography followed by evaporation in vacuo gave the product which was dried at room temperature in vacuo (mp 95 – 113°).

The compound showed a single spot by thin layer chromatography ($R_f$ = 0.44, silica gel plate eluted with 10% (v/v) ether/methylene chloride). The NMR spectrum was consistent with the title structure and showed the presence of approximately 0.15 mole of methylene chloride. The compound was 95.3% pure by HPLC. The mass spectrum showed a molecular ion at me = 411.

| Analysis Calc'd for: $C_{26}H_{22}FN_3O \cdot 0.15CH_2Cl_2$: | | | |
|---|---|---|---|
| | C, 74.04; | H, 5.30; | N, 9.91; |
| Found: | C, 74.17; | H, 5.22; | N, 10.02. |

EXAMPLE 37

1 – Cyclopropylmethyl – 1,3 – dihydro – 5 – (2 – fluorophenyl) – 3(R) – (3' – indolyl)methyl – 2H – 1,4 – benzodiazepin – 2 – one

The procedure of Example 4 was carried out using cyclopropylmethylbromide (0.30 g, 2.25 mmole) in place of methyl iodide. The product obtained by chromatography and evaporation was recrystallized from a mixture of methylene, chloride, ether, and hexane, and the resulting solid dried in vacuo at 80°: (mp 207.5 – 208.5°).

The compound showed a single component by TLC ($R_f$ = 0.26, silica gel plate eluted with 4% (v/v) ether/methylene chloride). The NMR spectrum was consistent with the title structure. The compound was 99.6% pure by HPLC. The mass spectrum showed a molecular ion at m/e = 437.

| Analysis Calc'd for $C_{28}H_{24}FN_3O \cdot 0.07CH_2Cl_2$: | | | |
|---|---|---|---|
| | C, 76.02; | H, 5.49; | N, 9.48; |
| Found: | C, 75.96; | H, 5.42; | N, 9.30. |

EXAMPLE 38

1,3-Dihydro-5-(2-fluorophenyl)-3(R)-(3'-indolyl) methyl-1-pentyl-2H-1,4-benzodiazepin-2-one

The procedure of Example 4 was carried out using 1-bromopentane (0.34 g, 2.25 mmole) in place of methyl iodide. The product obtained after silica gel chromatography and evaporation was crystallized from ether and dried in vacuo at 80°: (mp 150-151°).

The compound showed a single component by thin layer chromatography ($R_f$ = 0.37, silica gel plate eluted with 4% (v/v) ether/methylene chloride). The NMR spectrum was consistent with the title structure. The compound was 99.9% pure by HPLC. The mass spectrum showed a molecular ion at me =453.

| Analysis Calc'd for: $C_{29}H_{28}FN_3O$: | | | |
|---|---|---|---|
| | C, 76.79; | H, 6.22; | N, 9.26; |
| Found: | C, 76.64; | H, 6.39; | N, 8.83. |

EXAMPLE 39

1,3-Dihydro-5-(2-fluorophenyl)-3(R)-(3'-indolyl) methyl-1-(3-methylbutyl)-2H-1,4-benzodiazepine-2-one

The procedure of Example 4 was carried out using 1-bromo-3-methylbutane (0.34 g, 2.25 mmole) in place of methyl iodide. The product obtained after silica gel chromatography and evaporation was crystallized from ether and dried in vacuo at 80°: (mp = 198-199.5°).

The compound showed a single component by thin layer chromatography ($R_f$ = 0.30, silica gel plate eluted with 4% (v/v) ether/methylene chloride). The NMR spectrum was consistent with the title structure and showed the presence of 0.2 mole of ether. The compound was 99.9% pure by HPLC. The mass spectrum showed a molecular ion at m/e = 453.

| Analysis Calc'd for: $C_{29}H_{28}FN_3O \cdot 0.2C_4H_{10}O$: | | | |
|---|---|---|---|
| | C, 76.42; | H, 6.46; | N, 8.97; |
| Found: | C, 76.52; | H, 6.38; | N, 9.01. |

EXAMPLE 40

1,3-Dihydro-5-(2-fluorophenyl)-3(R)-(3'-indolyl)methyl-1-(2,2,2-trifluoroethyl)-2H-1,4-benzodiazepin-2-one

The procedure of Example 4 was carried out using 2,2,2-trifluoroethyl iodide (0.47 g, 2.25 mmole) in place of methyl iodide. Following addition of the trifluoroethyl iodide, the reaction was heated for 18 hours in an oil bath thermostatted at 65°. Workup and chromatography as described in Example 4 gave a product which was recrystallized from ether and dried in vacuo at 80°: (mp 189-192°).

The compound showed a single component by thin layer chromatography ($R_f$ = 0.50, silica gel plate eluted with 5% (v/v) ether/methylene chloride). The NMR spectrum was consistent with the title structure. The compound was 99.2% pure by HPLC. The mass spectrum showed a molecular ion at m/e = 465.

| Analysis Calc'd for: $C_{26}H_{19}F_4N_3O$: | | | |
|---|---|---|---|
| | C, 67.09; | H, 4.11; | N, 9.03; |
| Found: | C, 67.32; | H, 4.31; | N, 8.98. |

EXAMPLE 41

1,3 – Dihydro – 1 – (2 – dimethylaminoethyl) – 5 – (2 – fluorophenyl) 3(R) – (3' – indolyl)methyl – 2H – 1,4 – benzodiazepin – 2 – one

The procedure of Example 4 was carried out using 1 – chloro – 2 – (dimethylamino)propane (0.24 g, 2.25 mmole) in place of methyl iodide. Following addition of the chloride, the reaction was stirred at room temperature for 5 days and then worked up as described in Example 4. The chromatographed product was crystallized from methylene chloride/hexane and the resulting solid dried in vacuo at 80°: (mp 200 – 201°).

The compound showed a single component by TLC ($R_f$ = 0.30, silica gel plate eluted with 5% (v/v) methanol/methylene chloride). The NMR spectrum was consistent with the title structure. The compound was 99.6% pure by HPLC. The mass spectrum showed a molecular ion at m/e = 454.

| Analysis Calc'd for: $C_{28}H_{27}FN_4O$: | | | |
|---|---|---|---|
| | C, 73.98; | H, 5.99; | N, 12.33; |
| Found: | C, 73.92; | H, 6.00; | N, 11.28. |

EXAMPLE 42

1,3 – Dihydro – 1 – (ethoxycarbonylmethyl) – 5 – (2 – fluorophenyl) – 3(R) – (3' – indolyl)methyl – 2H – 1,4 – benzodiazepin – 2 – one

The procedure of Example 4 was carried out using ethyl bromoacetate (0.38 g, 2.25 mmole) in place of methyl iodide. The chromatographed product was evaporated and dried in vacuo at room temperature: (mp 88 – 100°).

The compound showed a single component by TLC ($R_f$ = 0.42, silica gel plate eluted with 10% (v/v) ether/methylene chloride). The NMR spectrum was consistent with the title structure and showed the presence of 0.24 mole of methylene chloride. The compound was 92.6% pure by HPLC. The mass spectrum showed a molecular ion at m/e = 469.

| Analysis Calc'd for $C_{28}H_{24}FN_3O_3 \cdot 0.24CH_2Cl_2$: | | | |
|---|---|---|---|
| | C, 69.23; | H, 5.04; | N, 8.58; |
| Found: | C, 69.14; | H, 5.09; | N, 8.87. |

EXAMPLE 43

1 – Carboxymethyl – 1,3 – dihydro – 5 – (2 – fluorophenyl) – 3(R) – 3' – indolyl)methyl – 2H – 1,4 – benzodiazepin – 2 – one

1,3 – Dihydro – 1 – (ethoxycarbonylmethylene) – 5 – (2 – fluorophenyl) – 3(R) – (3' – indolyl)methyl – 2H – 1,4 – benzodiazepin – 2 – one (83.2 mg, 0.177 mmole), and 1 molar sodium hydroxide (0.18 ml, 0.18 mmole) were combined in 1 ml of methanol and stirred at room temperature for 24 hours. The solution was acidified with 1 molar hydrochloric acid, and the mixture evaporated in vacuo. The residue was taken up in methylene chloride, washed with water, dried over sodium sulfate, filtered, and evaporated in vacuo to dryness. The residue was triturated with ether followed by petroleum ether, and filtered to give the product which was dried in vacuo at 80°; (mp 175 – 180° ( )).

The compound showed a single component by TLC ($R_f$ = 0.52, silica gel plate eluted with 90:10:1:1 (v/v/v/v) methylene chloride/methanol/acetic acid/water). The NMR spectrum was consistent with the title structure and showed the presence of both ether and hexane. The compound was 97.2% pure by HPLC. The mass spectrum showed a molecular ion at m/e = 441.

| Analysis Calc'd for $C_{26}H_{20}FN_3O_3 \cdot 0.1C_4H_{10}O \cdot 0.04C_6H_{14}.H_2O$: | | | |
|---|---|---|---|
| | C, 68.02; | H, 5.05; | N, 8.94; |
| Found: | C, 67.91; | H, 5.04; | N, 8.92. |

EXAMPLE 44

1,3-Dihydro-5-(2-fluorophenyl)-3(R)-[3'-(1'-methylindolyl)methyl]-1-methyl-2H-1,4-benzodiazepin-2-one

The method of Example 4 was employed except that the starting material was 1,3-dihydro-5-(2-fluorophenyl)-3(R)-(3'-indolyl)methyl-1-methyl-2H-1,4-benzodiazepin-2-one (1.3 g, 3.3 mmole). Fifty percent sodium hydride in mineral oil (0.16 g, 3.3 mmole) and methyl iodide (0.47 g, 3.3 mmole) were employed in 10 ml of dry DMF. Following workup and chromatography as in Example 4, the product was obtained having physical properties identical to those reported in Example 4.

EXAMPLE 45

1,3-Dihydro-5-(2-fluorophenyl)-3(R)-[3'-(1'-p-chlorobenzyloylindolyl)methyl]-1-methyl-2H-1,4-benzodiazepin-2-one

The procedure of Example 4 was carried out using 1,3-dihydro-5-(2-fluorophenyl)-3(R)-(3'-indolyl)methyl-1-methyl-2H-1,4-benzodiazepin-2-one (0.345 g, 0.87 mmole) in place of 1,3-dihydro-5-(2-fluorophenyl)-3(R)-(3'-indolyl)methyl-2H-1,4-benzodiazepin-2-one, and p-chlorobenzoyl chloride (0.26 g, 1.5 mmole) in place of methyl iodide. The reaction, employing 0.047 g (0.97 mmole) of 50% sodium hydride in mineral oil, was carried out in 10 ml of dry DMF. Silica gel chromatography as described in Example 4, followed by evaporation in vacuo and trituration with hexane, gave a solid which was dried in vacuo at 50°: (mp 75° ( )).

The compound showed a single component by TLC ($R_f$ = 0.57, silica gel plate eluted with 4% (v/v) ether/methylene chloride). The NMR spectrum was consistent with the title structure and verified the presence of approximately 0.3 mole of hexane. The compound was 99.3% pure by HPLC.

| Analysis Calc'd for $C_{32}H_{23}FClN_3O.0.3C_6H_{14}$: | | | | |
|---|---|---|---|---|
| | C, 72.25; | H, 4.88; | N, 7.48; | Cl, 6.31; |
| Found: | C, 72.42; | H, 5.02; | N, 7.50; | Cl, 6.55. |

EXAMPLE 46

7-Chloro-1,3-dihydro-3(R)-[3'(1'-benzylindolyl)methyl] 1-methyl-5-phenyl-2H-1,4-benzodiazepin-2-one

The procedure of Example 45 was carried out using 0.042 g (0.88 mmole) of 50% sodium hydride, and benzylbromide (0.16 g, 0.92 mmole) in place of p-chlorobenzoyl chloride. Reaction was conducted in 4 ml of dry DMF. Following silica gel chromatography and evaporation, the product was recrystallized from cyclohexane and dried in vacuo at 60°: (mp 77-80° (indistinct)).

The compound showed a single component by TLC ($R_f$ = 0.59, silica gel plate eluted with 5% (v/v) ether/methylene chloride). The NMR spectrum was consistent with the title structure and showed the presence of 1/3 mole of cyclohexane. The compound was 98.7% pure by HPLC. The mass spectrum showed a molecular ion at m/e = 503.

| Analysis Calc'd for $C_{32}H_{26}ClN_3O \cdot 1/3C_6H_{12}$: | | | | |
|---|---|---|---|---|
| | C, 76.75; | H, 5.68; | N, 7.90; | Cl, 6.66; |
| Found: | C, 76.50; | H, 5.74; | N, 7.59; | Cl, 6.90. |

EXAMPLE 47

1,3-Dihydro-3(RS)-[1-hydroxy-1-(3'-indolyl)]methyl-1-methyl-5-phenyl-2H-1,4-benzodiazepin-2-one

The lithium salt of 1,3-dihydro-1-methyl-5-phenyl-2H-1,4-benzodiazepin-2-one (1.25 g, 5 mmole) was made according to the procedure of J. Org. Chem. 46, 3945 (1981) using 1.01 g (10 mmole) of diisopropylamine, and 6.7 ml of a 1.5 molar solution (10 mmole) of n-butylithium in hexane. This anion solution was added by syringe to a solution of 0.725 g (5 mmole) of indole-3-carboxaldehyde in 15 ml of dry THF stirred under nitrogen in a dry ice-acetone bath. The mixture was warmed to room temperature, stirred for 1 1/2 hours and then quenched by the addition of saturated sodium chloride solution. The mixture was separated and the aqueous layer extracted twice with methylene chloride (2 x 10 ml). The organic layers were dried over sodium sulfate, filtered and evaporated to dryness in vacuo. The residue was chromatographed on silica gel (230-400 mesh, 8 inch (20 cm) column, 25 mm diameter, 1:1 ether/methylene chloride elution). The evaporated product fractions were crystallized from ether and dried in vacuo at 70°: (mp 218-221°).

The compound showed a single component by TLC ($R_f$ = 0.30, silica gel plate eluted with 1:1 (v/v) ether/methylene chloride). The NMR spectrum was consistent with the title structure. The compound was 90% pure by HPLC. The mass spectrum showed a molecular ion at me = 395.

| Analysis Calc'd for $C_{25}H_{21}N_3O_2 \cdot 0.25H_2O$: | | | |
|---|---|---|---|
| | C, 75.07; | H, 5.42; | N, 10.51; |
| Found: | C, 75.04; | H, 5.50; | N, 10.59. |

EXAMPLE 48

1,3-Dihydro-1-methyl-5-phenyl-3-(RS)-(3-thienoyl)-2H-1,4-benzodiazepin-2-one

The procedure of Example 47 was carried out using thiophene-3-carbonyl chloride (730 mg, 5.0 mmol) in place of indole-3-carboxaldehyde. Following chromatography (silica gel, 5% (v/v) $Et_2O$ in $CH_2Cl_2$), the product was evaporated to dryness and crystallized from $Et_2O$. The solid was dried in vacuo at 65°C: (m.p. 205-8°C).

The compound showed a single spot by TLC ($R_f$=0.54, silica gel plate, 10% (v/v) $Et_2O$ in $CH_2Cl_2$). The NMR spectrum was consistent with the title structure. The compound was greater than 92.4% pure by HPLC. The mass spectrum showed a molecular ion at m/e = 360.

| Anal. Calc'd for $C_{21}H_{16}N_2O_2S$: | | | |
|---|---|---|---|
| | C, 69.98; | H, 4.47; | N, 7.77. |
| Found: | C, 70.27; | H, 4.64; | N, 7.69. |

EXAMPLE 49

1,3 – Dihydro – 3 – (RS) – [1 – hydroxy – 1 – (3 – thienyl)]methyl – 1 – methyl – 5 – phenyl – 2H – 1,4 – benzodiazepin – 2 – one

The procedure of Example 47 was carried out using thiophene – 3 – carboxaldehyde (560 mg, 5.0 mmol) in place of indole – 3 – carboxaldehyde. Following chromatography (silica gel, 15% (v/v) $Et_2O$ in $CH_2Cl_2$), the product was evaporated to dryness and crystallized from $Et_2O$. The solid was dried in vacuo at 65 ° C: (m.p. 189 – 91 ° C).

The compound showed a single spot by TLC ($R_f$ = 0.36, silica gel plate, 15% (v/v) $Et_2O$ in $CH_2Cl_2$). The NMR spectrum was consistent with the title structure. The compound was greater than 99.0% pure by HPLC. The mass spectrum showed a molecular ion at m/e = 362.

| Anal. Calc'd for $C_{21}H_{18}N_2O_2S$: | | | |
|---|---|---|---|
| | C, 69.59; | H, 5.01; | N, 7.73. |
| Found: | C, 69.62; | H, 5.01; | N, 7.57. |

EXAMPLE 50

1,3 – Dihydro – 3(RS) – [1 – hydroxy – 1 – [3 – (1 – methylindolyl)]] – methyl – 1 – methyl – 5 – phenyl – 2H – 1,4 – benzodiazepin – 2 – one (two stereoisomers, A and B)

The procedure of Example 47 was carried out using 1 – methylindole – 3 – carboxaldehyde (797 mg, 5.0 mmol) in place of indole – 3 – carboxaldehyde. The product diastereomers were separated by chromatog – raphy (silica gel, 10% (v/v) $Et_2O$ in $CH_2Cl_2$) and evaporated to dryness.

A: The faster running component (TLC – $R_f$ = 0.41, silica gel plate, 60% (v/v) EtOAc in hexane) was crystallized from $Et_2O$. The solid was dried in vacuo at 65 ° C: (m.p. 218 – 21 ° C).

The compound showed a single spot by TLC. The NMR spectrum was consistent with the title structure. The compound was greater than 96.7% pure by HPLC. The mass spectrum showed a molecular ion at m/e = 409.

| Anal. Calc'd for $C_{26}H_{23}N_3O_2$: | | | |
|---|---|---|---|
| | C, 76.26; | H, 5.66; | N, 10.26. |
| Found: | C, 76.26; | H, 5.84; | H, 10.34. |

B: The slower running component (TLC – $R_f$ = 0.30, silica gel plate, 60% (v/v) EtOAc in hexane) was crystallized from $Et_2O$. The solid was dried in vacuo at 65 ° C: (m.p. 125 – 30 ° C).

The compound was a single spot by TLC. The NMR spectrum was consistent with the title structure and cnfirmed the presence of $Et_2O$. The compound was greater than 95.7% pure by HPLC.The mass spectrum showed a molecular ion at m/e = 409).

| Anal. Calc'd for $C_{26}H_{23}N_3O_2.0.9C_4H_{10}O$: | | | |
|---|---|---|---|
| | C, 74.66; | H, 6.77; | N, 8.83. |
| Found: | C, 74.61; | H, 6.80; | N, 9.10. |

EXAMPLE 51

1,3 – Dihydro – 3(RS) – (1 – hydroxy – 1 – phenyl)methyl – 1 – methyl – 5 – phenyl – 2H – 1,4 – benzodiazepin – 2 – one

The procedure of Example 47 was carried out using benzyldehyde (0.53 g, 5 mmole) in place of indole – 3 – carboxaldehyde. The chromatographed product was crystallized from ether and dried in vacuo at

70°: (mp 192 – 193°).

The compound showed a single component by TLC ($R_f$ = 0.53, silica gel plate eluted with 1:1 (v/v) ether/methylene chloride). The NMR spectrum was consistent with the title structure and showed the presence of 0.1 mole of ether. The compound was 99.9% pure by HPLC. The mass spectrum showed a molecular ion at m/e = 338.

| Analysis Calc'd for $C_{23}H_{20}N_2O_2 \cdot 0.1C_4H_{10}O$: | | | |
|---|---|---|---|
| | C, 77.24; | H, 5.82; | N, 7.70: |
| Found: | C, 77.11; | H, 5.83; | N, 7.93. |

EXAMPLE 52

1,3 – Dihydro – 3(RS) – [1 – hydroxy – 1 – (2 – thienyl)]methyl – 1 – methyl – 5 – phenyl – 2H – 1,4 – benzodiazepin – 2 – one

The procedure of Example 47 was carried out using 2 – thiophene – carboxaldehyde (0.56 g, 5 mmole) in place of indole – 3 – carboxaldehyde. The chromatographed and evaporated product was crystallized from ether and dried in vacuo at 70°: (mp 184 – 185°).

The compound showed a single component by TLC ($R_f$ = 0.54, silica gel plate eluted with 1:1 (v/v) ether/methylene chloride). The NMR spectrum was consistent with the title structure. The compound was 99.8% pure by HPLC.

| Analysis Calc'd for $C_{21}H_{18}N_2O_2S$: | | | |
|---|---|---|---|
| | C, 69.59; | H, 5.01; | N, 7.73; |
| Found: | C, 69.59; | H, 5.10; | N, 8.06. |

EXAMPLE 53

1,3 – Dihydro – 3 – (RS) – hydroxy – 1 – methyl – 5 – phenyl – 3 – (3' – thienoyl) – 2H – 1,4 – benzodiazepin – 2 – one (A) and 1,5 – Dihydro – 5 – (RS) – hydroxy – 1 – methyl – 5 – phenyl – 3 – (3' – thienoyl) – 2H – 1,4 – benzodiazepin – 2 – one (B)

The procedure of Example 47 was carried out using 0.75 g (5 mmole) of 3 – thienoyl chloride in place of indole – 3 – carboxaldehyde. In this reaction, the THF employed was subsequently shown to contain significant quantities of organic peroxides. Workup and chromatography as in Example 47 provided two products each of which was evaporated in vacuo and crystallized from ether.

A: The first product obtained was A, which was dried in vacuo at 70°: (mp 193 – 194°).

The compound showed a single component by TLC ($R_f$ = 0.57, silica gel plate eluted with 1:1 (v/v) methylene/chloride ether). The NMR spectrum was consistent with the title structure. The compound was 99.4% pure by HPLC. The mass spectrum showed a molecular ion at m/e = 376. The infrared spectrum showed a strong absorption at 1675 $cm^{-1}$.

| Analysis Calc'd for $C_{21}H_{16}N_2O_3S$: | | | |
|---|---|---|---|
| | C, 67.00; | H, 4.28; | N, 7.44; |
| Found: | C, 67.04; | H, 4.37; | N, 7.49. |

B: The second compound obtained was B, which was dried in vacuo at 70°: (mp 173 – 175°).

The compound showed a single component by TLC ($R_f$ = 0.64, silica gel plate eluted with 1:1 methylene chloride/ether). The NMR spectrum was consistent with the title structure. The mass spectrum showed a molecular ion at m/e = 376. The compound was 99.6% pure by HPLC. The infrared spectrum showed strong absorption at 1695 and 1720 $cm^{-1}$.

| Analysis Calc'd for $C_{21}H_{16}N_2O_3S$: | | | |
|---|---|---|---|
| | C, 67.00; | H, 4.28; | N, 7.44; |
| Found: | C, 66.91; | H, 4.46; | N, 7.32. |

EXAMPLE 54

7-Chloro-1,3-dihydro-3(R)-[(2',3'-dihydro-2'-oxo-1'H-indol-3'-yl)methyl]-5-phenyl-2H-1,4-benzodiazepin-2-one

7-Chloro-1,3-dihydro-3(R)-indolylmethyl-5-phenyl-2H-1,4-benzodiazepin-2-one (200 mg, 0.5 mmol) was dissolved in DMSO (4.8 g, 10 mmol) followed by the addition of concentrated HCl (5 mmol). The molar ratio of DMSO to HCl was 2:1. Additional reagents were added to drive the reaction to completion. The additions were:

| | |
|---|---|
| 0.71 ml DMSO<br>0.4 ml HCl | 1.54 ml DMSO<br>0.75 ml HCl |

When little starting material remained, the reaction was poured into an Erlenmeyer flask with water (20 ml), and 5 g of $NaHCO_3$ was added. Water (100 ml) was added and the mixture was extracted with 4x50 ml of n-butanol. The n-butanol solution was washed with water (3x100 ml). The n-butanol solution was evaporated and the residue was dissolved in ether and purified by preparative TLC.

The product was a pair of diasteriomers; the NMR spectrum was consistent with the title compound.

HPLC indicated two components: 54% and 43%.

TLC in 95/5/0.5 $CHCl_3$-MeOH-$H_2O$ $R_f$=0.3 (silica gel GF)

Mass Spec. gave a (M+1) at 416.

EXAMPLE 55

7-Chloro-1,3-dihydro-3(R)-[(3'-(2,4-dinitrophenyl)-imidazol-5'-yl)-methyl]-5-phenyl-2H-1,4-benzodiazepin-2-one

Boc-DNP-D-Histidine (1.7 g, 4 mmol) and 2-amino-5-chlorobenzophenone (0.9 g, 4 mmol) were combined in 10 ml of THF and stirred until a clear orange solution was obtained. 4.3 mL of DCC (1M) in THF was added and the reaction was stirred overnight. The reaction was filtered and evaporated. The residue was purified by flash chromatography on a silica gel 60 column with a 90:10 chloroform ether solvent system.

The resultant t-BOC protected compound was dissolved in 30 ml of ethyl acetate. The solution was cooled to -25°C. HCl gas was added until the solution was saturated. The temperature was allowed to rise to 0°C. When the reaction was complete by TLC, the ethyl acetate was evaporated and the residue was dissolved in methanol. The pH of the solution was adjusted with 10% aqueous sodium hydroxide to pH 9. After the reaction stirred overnight, the solvent was evaporated and the residue was chromatographed on a silica gel 60 column with chloroform, to give the title compound.

HPLC: 91%.

TLC: $R_f$=0.6 in 90/10/1 $CHCl_3$-MeOH-aqueous ammonia (silica gel GF)

Mass Spec. molecular ion at 516.

NMR agreed with the title compound.

| Elemental analysis for $C_{25}H_{17}ClN_6O_5$ .1.8$H_2O$ | | | |
|---|---|---|---|
| Calcd: | C, 54.65; | H, 3.82; | N, 15.30. |
| Observed: | C, 54.38; | H, 3.89; | N, 15.31. |

EXAMPLE 56

7 – Chloro – 1,3 – dihydro – 3(R) – (3' – imidazol – 5' – yl)methyl – 5 – phenyl – 2H – 1,4 – benzodiazepin – 2 – one

This compound was obtained as a second product from the reaction sequence of Example 55. This material, which had a positive Sanger test for histidine, eluted from the silica column after the compound of Example 55, HPLC: 87%.

TLC: $R_f$ – 0.3 in 90/10/1 $CHCl_3$ – MeOH – aqueous ammonia (silica gel GF).

Mass Spec. molecular ion at 350.

NMR was consistent with the title compound.

| Elemental Analysis for: $C_{19}H_{15}ClN_4O$ 0.93 $H_2O$ 0.28$NH_3$ | | | |
|---|---|---|---|
| Calcd: | C, 61.29; | H, 4.79; | N, 16.33. |
| Found: | C, 61.68; | H, 5.12; | N, 16.61. |

EXAMPLE 57

3(RS) – [3' – (5' – Bromoindolyl)methyl] – 1,3 – dihydro – 5 – phenyl – 2H – 1,4 – benzodiazepin – 2 – one

The synthesis was carried out as described for Example 55 starting with Boc – 5 – bromo – DL – tryptophan and 2 – aminobenzophenone. The crude product was purified by column chromatography (silica gel) using 90/10 chloroform – ether as the elution solvent.

HPLC: 99%.

| Elemental analysis calcd: | | | |
|---|---|---|---|
| | N, 8.91; | C, 61.15; | H, 4.41 |
| Found: | N, 8.43; | C, 61.43; | H, 4.20. |

Mass Spec. molecular ion at 443.

NMR: The NMR was in agreement with the title compound.

EXAMPLE 58

**5-o-Carboxyphenyl-1,3-dihydro-3(R)-(3'-indolyl)methyl-2H-1,4-benzodiazepin-2-one**

2 – Amino – 2' – carboxybenzophenone (2.41 g, 10 mmol) was suspended in THF, $CH_2Cl_2$, EtOAc and tryptophanyl chloride hydrochloride (2.59 g, 10 mmol) was added. The mixture was stirred at room temperature until reaction was complete by TLC. A solid was collected by filtration, dried, and dissolved in 40 ml of methanol. The pH of the solution was adjusted to a pH of 8 – 10 with 10% aqueous sodium hyroxide. After standing at room temperature for about 3 days, the solution was acidified to a pH of about 3. The solvent was evaporated and the residue was dissolved in 95/5 $CHCl_3/CH_3OH$ and flash chromatog – raphed on a silica gel 60 column with a 95:5 and 90:10 chloroform – methanol solvent system to give the title compound.

HPLC: 96%.

| Elemental analysis calcd: | | | |
|---|---|---|---|
| | C, 61.73; | H, 3.97; | N, 8.38 |
| Found: | C, 61.70; | H, 4.09; | N, 8.48. |

Mass Spec. molecular ion observed at 409.
NMR: The spectrum agreed with the title compound.

EXAMPLE 59

**1,3-Dihydro-3(RS)-[3'-(5'-fluoroindolyl)methyl]-5-o-fluorophenyl-2H-1,4-benzodiazepin-2-one**

5-Fluorotryptophyl chloride hydrochloride (1.38 g, 5 mmole), prepared from 5-fluoro-DL-tryptophan and $PCl_5$ in acetylchloride, was suspended in 15 ml of THF. 2-Amino-2'-fluorobenzophenone 1.07 g (5.0 mmol) was added to the stirred mixture. After stirring overnight the solvent was evaporated and the solid was dissolved in 50 ml of methanol. The pH of the solution was adjusted to 8-9 with 10% aqueous sodium hydroxide. The solution stood for 24 hours at room temperature. The solvent was evaporated and the crude reaction product was purified by flash chromatography with 98:2 chloroform/methanol to give the title compound.
TLC: $R_f = 0.3$ in 97:3 $CHCl_3/CH_3OH$ (silica gel GF).

| Elemental analysis calcd for $C_{24}H_{17}F_2N_3O$ .0.18$CHCl_3$ | | | |
|---|---|---|---|
| | C, 68.75; | H, 4.10; | N, 9.94 |
| Found: | C, 68.78; | H, 4.04; | N, 9.85. |

NMR was in agreement with the title compound.

EXAMPLE 60

**1,3-Dihydro-3(RS)-[3'-(6'-fluoroindolyl)methyl]-5-o-fluorophenyl-2H-1,4-benzodiazepin-2-one**

The compound was prepared according to the procedure of Example 59, using 6-fluorotryptophyl chloride hydrochloride in place of the 5-fluoro compound.
The final product was obtained as a solid which crystallized in pure form from chloroform.
TLC: $R_f = 0.4$ in 97:3 $CHCl_3/CH_3OH$ (silica gel GF)

| Elemental analysis calcd: | | | |
|---|---|---|---|
| | C, 70.62; | H, 4.20; | N, 10.26 |
| Found: | C, 70.62; | H, 4.10; | N, 10.25. |

NMR was in agreement with the title compound.

EXAMPLE 61

2-N-[2(RS)3-bis-(Boc-amino)propanoyl]amino-2'-fluorobenzophenone

The procedure of Example 1 was carried out using 2-amino-2'-fluorobenzophenone (430 mg, 2.0 mmole), 2(R,S),3-bis-(Boc-amino)propionic acid (617 mg, 2.03 mmole), and dicyclohexylcarbodiimide (2.03 ml of a 1.0 M solution in methylene chloride) in 10 ml of methylene chloride. Filtration, concentration in vacuo and flash chromatography (silica gel, 10% ethyl ether in methylene chloride) gave a foam, the PMR spectrum of which was consistent with the title compound.

EXAMPLE 62

**2-N-[2(RS),3-diphthalylaminopropanoyl]amino-2'-fluoro-benzophenone**

2-Amino-2'-fluorobenzophenone (2.10 g, 9.8 mmole) was reacted with 2,3-diphthalylaminopropionyl chloride (5 g, 9.8 mmole) in 100 ml of tetrahydrofuran. After 2.5 hours the reaction mixture was rotoevaporaed to give 7 g of a yellow foam. The foam was heated for 30 minutes in 6N hydrochloric acid (100ml) and the resulting off-white solid collected and dried. Recrystallization from ethyl acetate afforded the analytical sample, m.p. 210.5-211.5°.
NMR ($CD_3OD$): in agreement with title compound.

| Analysis Calc'd for $C_{32}H_{20}FN_3O_6$ | | | |
|---|---|---|---|
| | N, 7.48; | C, 68.45; | H, 3.59. |
| Found: | N, 7.46; | C, 68.59; | H, 3.63. |

EXAMPLE 63

1,3-Dihydro-5-(2'-fluorophenyl)-3(RS)-aminomethyl-2H-1,4-benzodiazepin-2-one

The procedure of Example 2 was carried out in which 2-N-[2(RS)-((1,1-dimethylethoxy)carbonyl)-amino-3-((1,1-dimethylethoxy)carbonyl)aminopropanoyl]amino-2'-fluorobenzophenone (600 mg, 1.2 mmole) was reacted in succession with excess HCl gas in ethyl acetate (15 ml) at 0° and then sodium hydroxide (0.1M solution) in aqueous methanol (10 ml). The pH of the reaction mixture was approximately 9.0. Work-up afforded the title compound as a solid, mp 168-169°; in 90% yield.

NMR ($CDCl_3$): Spectrum in agreement with title compound.
MS (14 ev.): 283 ($M^+$) 253.

| Analysis Calc'd for $C_{16}H_{14}FN_3O \cdot 0.05C_6H_{14}$ | | | |
|---|---|---|---|
| | N, 14.61; | C, 68.07; | H, 5.15. |
| Found: | N, 14.87; | C, 68.21; | H, 5.33. |

EXAMPLE 64

1,3-Dihydro-5-(2'-fluorophenyl)-3(RS)-aminomethyl-2H-1,4-benzodiazepin-2-one

2-N-[2(RS),3-diphthalylaminopropanoyl]amino-2'-fluorobenzophenone (1.07 g, 1.90 mmole) was suspended in 55 ml of methanol and treated with 1 ml of 95% hydrazine. The reaction mixture was protected from moisture and stirred at room temperature. Within one hour, the reaction mixture became homogeneous. On further reaction, phthalhydrazide precipitated from solution. After 14 hours, the reaction

was filtered and the filtrate concentrated. The residue was partitioned between methylene chloride and water; the organic phase was washed wth water until it was free of hydrazine (Tollen's reagent negative), then dried and concentrated to give 480 mg of an oil which crystallized on standing. Trituration of the resulting solid with ether gave the analytical sample, m.p. 168 – 169°, identical spectroscopically with the material prepared in Example 63.

EXAMPLE 65

1,3 – Dihydro – 5 – (2' – fluorophenyl) – 3(R) – (4 – amino)butyl – 2H – 1,4 – benzodiazepin – 2 – one

The procedure of Example 64 was followed whereby 2 – N – [2(R),6 – diphthalylaminohexanoyl]amino – 2' – fluorobenzophenone (5.4 g) was deprotected and cyclized with 10 ml of 95% hydrazine in 150 ml of methanol. Workup afforded 1.35 g of product which was purified via silica gel chromatography (chloroform – methanol – ammonia, 80:30:4 v/v).
NMR ($CDCl_3$): in agreement with title compound.

| Analysis Calc'd for $C_{19}H_{20}FN_3O \cdot 0.17CHCl_3$ | | | |
|---|---|---|---|
| | N, 12.15; | C, 66.60; | H, 5.88. |
| Found: | N, 12.32; | C, 66.66; | H, 6.05. |

EXAMPLE 66

1,3 – Dihydro – 5 – (2' – fluorophenyl) – 3(RS) – (benzyloxycarbonyl)aminomethyl – 2H – 1,4 – benzodiazepin – 2 – one

To a solution of 50 ml of methylene chloride containing 260 mg (0.91 mmol) of 1,3 – dihydro – 5 – (2 – fluorophenyl) – 3(RS) – aminomethyl – 2H – 1,4 – benzodiazepin – 2 – one and 224 mg (1.83 mmol) of 4 – dimethylaminopyridine was added 0.51 ml (3.57 mmol) of benzylchloroformate. The resulting reaction mixture was allowed to stand at room temperature overnight and then was diluted with methylene chloride (200 ml). The reaction was then washed in succession with saturated sodium bicarbonate solution and brine, then dried ($MgSO_4$) and concentrated. The residual oil was chromatographed on silica gel (chloroform – methanol – ammonia, 95:5:0.5 v/v elution) to afford 370 mg of the analytical product, m.p. 88° (soften), 90 – 92°C.
TLC: Single component, $R_f$ = 0.35 (95:5:0.5, chloroform – methanol – ammonia).
NMR: Consistent with title structure.

| Anal. calc'd for $C_{24}H_{20}FN_3O_3 \cdot 1/4\ H_2O$ | | | |
|---|---|---|---|
| | N, 9.96; | C, 68.32; | H, 4.89; |
| Found: | N, 9.86; | C, 68.45; | H, 5.15. |

EXAMPLE 67

1,3 – Dihydro – 5 – (2' – fluorophenyl) – 3(RS) – (3 – thiophenecarbonyl)aminomethyl – 2H – 1,4 – benzodiazepin – 2 – one

1,3 – Dihydro – 5 – (2' – fluorophenyl) – 3(RS) – aminomethyl – 2H – 1,4 – benzodiazepin – 2 – one (140 mg, 0.49 mmole) and 3 – thiophenecarbonyl chloride (88 mg, 0.60 mmole) were dissolved in 10 ml of dry tetrahydrofuran at room temperature. To this solution was added 69 µl of triethylamine. After addition was complete, stirring was continued for 15 minutes more and the reaction mixture was partitioned between ethylacetate (60 ml) and sodium bicarbonate solution (sat.). The organic phase was washed with 10% sodium hydroxide solution (1 x 20 ml) and then with 10% hydrochloric acid solution. From this acidic solution were deposited off – white crystals, after overnight standing. The solid was washed with water and dried to give 140 mg of the analytical product, mp 237 – 240° (An additional 70 mg of product was obtained

as the free base after concentration of the organic extracts.) The analytical product was greater than 98% pure by HPLC.
MS (14 ev.): 393 (M − HCl), 266.
NMR (DMSO − $d_6$): in agreement with title compound.

| Analysis Calc'd for $C_{21}H_{17}ClFN_3O_2S$: | | | |
|---|---|---|---|
| | N, 9.77; | C, 58.67; | H, 3.98. |
| Found: | N, 9.89; | C, 58.75; | H, 4.17. |

EXAMPLE 68

1,3 − dihydro − 5 − (2' − fluorophenyl) − 3(RS) − (2 − indole carbonylaminomethyl) − 2H − 1,4 − benzodiazepin − 2 − one

1,3 − Dihydro − 5 − (2 − fluorophenyl) − 3(RS) − aminomethyl − 2H − 1,4 − benzodiazepin − 2 − one (80 mg, 0.282 mmole) and indole − 2 − carbonyl chloride (53 mg, 0.30 mmol) were mixed in 5 ml of methylene chloride at room temperature. The homogeneous reaction mixture was protected from moisture and treated with 42 µl (0.30 mmole) of triethylamine. Within five min., triethylamine hydrochloride precipitated. The reaction mixture was stirred at room temperatre overnight and then partitioned between methylene chloride and saturated sodium bicarbonate solution. The resulting solid was collected, washed with water and dried over $P_2O_5$ at 70 ° C. In this way, 39 mg of the analytical product was obtained, m.p.: 315 − 317 ° (d).
NMR(DMSO − $d_6$): Consistent with the title structure.
MS: Molecular ion at m/e = 426.

| Anal. calc'd for $C_{25}H_{19}FN_4O_2 \cdot 1.25\ H_2O$ | | | |
|---|---|---|---|
| | C, 66.88; | H, 4.82; | N, 12.48; |
| Found: | C, 66.76; | H, 4.52. | N, 12.25; |

EXAMPLE 69

1,3 − Dihydro − 3(RS) − [3' − (RS) − (1,3 − dihydro − 5 − (2' − fluorophenyl) − 2H − 1,4 − benzodiazepin − 2 − one)]methylaminomethyl − 5 − (2' − fluorophenyl) − 2H − 1,4 − benzodiazepin − 2 − one

1,3 − Dihydro − 5 − (2' − fluorophenyl) − 3(RS) − aminomethyl − 2H − 1,4 − benzodiazepin − 2 − one (60 mg, 0.21 mmole) was dissolved in 3 ml of isopropanol and treated with triethylamine (30 µl, 0.22 mmole). The resulting solution was heated to reflux for 18 hours, cooled and concentrated. The residual oil was chromatographed on silica gel (chloroform − methanol − ammonia, 90:10:1 v/v) to give 25 mg of the desired product as an off − white solid, mp 155 − 158 ° (with gas evolution).
MS (FAB): 550 (M + H), 549 ($M^+$), 282 (base peak).
NMR ($CDCl_3$): in agreement with title compound.

| Analysis Calc'd for $C_{32}H_{25}F_2N_5O_2 \cdot 0.35\ CHCl_3$: | | | |
|---|---|---|---|
| | N, 11.84; | C, 65.70; | H, 4.32. |
| Found: | N, 11.68; | C, 65.53; | H, 4.46. |

EXAMPLE 70

1,3-Dihydro-5-(2'-fluorophenyl)-3-(RS)-(6'-chloropyrazinyl)aminomethyl-2H-1,4-benzodiazepin-2-one

1,3-Dihydro-5-(2-fluorophenyl)-3-(RS)-aminomethyl-2H-1,4-benzodiazepin-2-one (72 mg, 0.25 mmol), 2,6-dichloropyrazine (45 mg, 0.30 mmol)and anhydrous potassium carbonate (83 mg, 0.60 mmol) were combined at room temperature with 2 ml of dry dimethylformamide. The resulting suspension was stirred rapidly for 24 hours and 37 mg more of 2,6-dichlorpyrazine was added. After 72 hours total reaction time, the reaction mixture was poured into water (10 ml) and extracted with ethyl acetate (3 x 20 ml). The combined organic extracts were washed with water and brine, dried ($MgSO_4$) and concentrated to give 70 mg of crude product. The analytical sample was obtained by preparative thick layer chromatography (chloroform - methanol - ammonia, 95:5:0.5 v/v one elution).
$R_f$ = 0.25, m.p. 140° (soften), 148-152°.
NMR ($CDCl_3$): Consistent with the title structure.
MS (14 ev): 395 ($M^+$), 266, 254, 211.

| Anal. calc'd for $C_{20}H_{15}ClFN_5O.1/4\ H_2O$: | | | |
|---|---|---|---|
| | N, 17.49; | C, 60.00; | H, 3.90; |
| Found: | N, 16.59; | C, 59.87; | H, 3.90. |

EXAMPLE 71

2-N-Methyl-N-[2(RS),3-diphthalylaminopropanoyl]amino-2'-fluorobenzophenone

Following the procedure of Example 4, 2-N-[2(RS),3-diphthalylaminopropanoyl]amino-2'-fluorobenzophenone (677 mg, 1.20 mmole) was converted to the title compound with sodium hydride (63 mg, 1.31 mmole) and methyliodide (81.5 µl, 1.31 mmole) in 5 ml of N,N-dimethylformamide. Work-up afforded the crude product which was purified by silica gel chromatography (ethyl acetate-hexane elution, 3:2 v/v); the analytical sample was obtained as white prisms by recrystallizing the chromatographed material from ethyl acetate, mp 252°.
MS (14 ev.): 575 ($M^+$), 453, 429, 309.
NMR ($CDCl_3$): in agreement with title compound.

| Analysis Calc'd for $C_{33}H_{22}FN_3O_6 \cdot 0.15\ C_4H_8O_2$: | | | |
|---|---|---|---|
| | N, 7.13; | C, 68.54; | H, 3.94. |
| Found: | N, 7.12; | C, 68.43; | H, 4.26. |

EXAMPLE 72

1,3-Dihydro-5-(2'-fluorophenyl)-3(RS)-aminomethyl-1-methyl-2H-1,4-benzodiazepin-2-one

Following the procedure of Example 64, 2-N-methyl-N-[2(RS),3-diphthalylaminopropanoyl]-amino-2'-fluorobenzophenone (220 mg, 0.38 mmole) was converted to the title compound with 95% hydrazine (1 ml) in 40 ml of methanol. The analytical material was obtained via chromatography on silica gel (chloroform-methanol-ammonia, 90:10:1 v/v). The PMR spectrum ($CDCl_3$) confirmed the structure of the product; N-methyl proton at 3.46 ppm.

EXAMPLE 73

3(RS)-(2-indolecarbonylamino)-1,3-dihydro-5-phenyl-2H-1,4-benzodiazepin-2-one

3-(RS)-Amino-1,3-dihydro-5-phenyl-2H-1,4-benzodiazepin-2-one (75 mg, 0.298 mmol), and indole-2-carbonyl chloride (58.8 mg, 0.327 mmol) were combined in $CH_2Cl_2$ (2 ml) and the pH adjusted to 9.0 with triethylamine (41 μl, 0.298 mmol). After stirring 10 min., the reaction was chromatographed on silica gel (180/10/1/1 of $CH_2Cl_2$/MeOH/$H_2O$/HOAc). The combined product factions were washed with dilute $NaHCO_3$ (aq) (1X), $H_2O$ (1X) and brine (1X), dried over $MgSO_4$, filtered and stripped to give the title compound as a white solid from ether: (m.p. 265-268°).
TLC: Silica GF (10% MeOH in $CH_2Cl_2$), $R_f$ = 0.63, single homogeneous component.
NMR: Consistent with title structure and verifies the presence of 0.2 $(C_2H_5)_2O$.
HPLC: Greater than 99.2% pure.
M.S.: Mol. Ion = 394 m/e (free base).

| Anal. Calc'd for $C_{24}H_{18}N_4O_2.0.2\ (C_2H_5)_2O$: | | | |
|---|---|---|---|
| | C, 72.78; | H, 4.93; | N, 13.69; |
| Found: | C, 72.45; | H, 4.60; | N, 13.65. |

EXAMPLE 74

1,3-Dihydro-3(RS)-[2-(3-indolyl)ethyl]amino-5-phenyl-2H-1,4-benzodiazepin-2-one

3-(RS)-Chloro-1,3,-dihydro-5-phenyl-2H-1,4-benzodiazepine-2-one(68 mg, 0.25 mmol), 3-(2-amionoethyl)indole (40 mg, 0.25 mmol) and sodium hydroxide (0.1 ml of 2.5N solution) were combined in methanol (4 ml) and stirred at room temperature for 18 hours. The mixture was evaporated in vacuo, and the residue was dissolved in methylene chloride and chromatographed on silica gel (5% v/v MeOH in $CH_2Cl_2$). The product fractions were evaporated in vacuo and the resulting solid crystallized from ether and dried in vacuo at 60°: (m.p. 196-197.5 (d)). TLC: Single spot ($R_f$ = 0.46, silica gel plate, 10% (v/v) MeOH in $CH_2Cl_2$).
NMR: The spectrum was consistent with the title structure and verified the presence of $CH_2Cl_2$.
HPLC: Greater than 94% pure.
MS: A molecular ion at m/e = 394.

| Anal. calc'd. for $C_{25}H_{22}N_4O.0.13\ CH_2Cl_2$: | | | |
|---|---|---|---|
| | C, 74.43; | H, 5.53; | N, 13.82; |
| Found: | C, 74.62; | H, 5.47; | N, 13.62. |

EXAMPLE 75

3(RS)-[3-(3-indole)propionylamino]-1,3-dihydro-5-phenyl-2H-1,4-benzodiazepin-2-one

The procedure of Example 77 was carried out using 3-(3-indolyl)propionic acid (0.076 g, 0.4 mmol) in place of BOC-L-tryptophan. The product was chromatographed on silica gel using a gradient of 1:1 $Et_2O/CH_2Cl_2$ containing 0 to 2% $CH_2OH$. The product was crystallized from acetone and dried in vacuo at 60°: (m.p. 176-182°).
TLC: Single spot ($R_f$ = 0.66, silica gel plate, 10% (v/v) MeOH in $CH_2Cl_2$).
NMR: The spectrum was consistent with the title structure.
HPLC: 99.7% pure.
MS: A molecular ion at m/e = 422.

| Anal. calc'd for $C_{26}H_{22}N_4O_2.0.5\ H_2O$: | | | |
|---|---|---|---|
| | C, 72.37; | H, 5.37; | N, 12.99; |
| Found: | C, 72.31; | H, 5.57; | N, 12.98. |

EXAMPLE 76

3(RS)-(3-indoleacetylamino)-1,3-dihydro-5-phenyl-2H-1,4-benzodiazepin-2-one

3-(RS)-Amino-1,3-dihydro-5-phenyl-2H-1,4-benzodiazepin-2-one (75 mg, 0.298 mmol) and indole-3-acetyl chloride (57.8 mg, 0.298 mmol) were combined in $CH_2Cl_2$ (2 ml) and the pH adjusted to 9.0 with triethylamine (TEA) 41 ml, 0.298 mmol). After stirring 15 min., a second portion of indole-3-acetyl chloride (44 mg, 0.175 mmol) and TEA (30 µl, 0.215 mmol) were added and the reaction stirred an additional 15 min. The completed reaction was diluted with $CH_2Cl_2$, washed with $H_2O$ (1X) and brine (1X), dried over $MgSO_4$, filtered and stripped to dryness in vacuo. The residue was chromatographed on silica gel (5% MeOH in $CH_2Cl_2$) to give the title compound as a pinkish solid from $Et_2O$: (m.p. 264-265°).
TLC: Silca GF (10% MeOH in $CH_2Cl_2$), $R_f$ = 0.44, single homogeneous component.
NMR: Consistent with title structure.
HPLC: Greater than 93.1% pure.
M.S.: molecular ion at m/e = 408.

| Anal. calc'd for $C_{25}H_{20}N_4O_2$: | | | |
|---|---|---|---|
| | C, 73.51; | H, 4.94; | N, 13.72; |
| Found: | C, 73.54; | H, 4.94; | N, 13.32. |

EXAMPLE 77

3(RS)-(Boc-L-tryptophyl)amino-1,3-dihydro-5-phenyl-2H-1,4-benzodiazepin-2-one

3-(RS)-Amino-1,3-dihydro-5-phenyl-2H-1,4-benzodiazepin-2-one (0.1 g, 0.4 mmol), BOC-L-tryptophan (0.12 g, 0.4 mmol), and DCC (0.4 ml of a 1 M solution in $CH_2Cl_2$, 0.4 mmol) were combined in 2 ml of THF to which were added 2 ml of DMF and 2 ml of $CH_2Cl_2$. The mixture was treated with triethylamine (0.11 ml), stoppered, and stirred at room temperature for four days. The mixture was treated with citric acid solution (10%, 3 ml) end $CH_2Cl_2$ (5 ml), shaken and separated. The aqueous phase was extracted with $CH_2Cl_2$ (2 x 5 ml). The combined organic layers were washed with citric acid (10%, 2 x 5 ml), sodium bicarbonate (10%, 2 x 5 ml), and $H_2O$ (10 ml), dried over sodium sulfate, filtered, and evaporated to dryness in vacuo. The residue was chromatographed on silica gel (1:1 (v/v) $Et_2O/CH_2Cl_2$) and the combined product fractions evaporated to dryness in vacuo. The residue was triturated with petroleum ether and the solid dried in vacuo at 70°: (m.p. 173-177° (↑)).
TLC: Single spot ($R_f$ = 0.56, silica gel plate, 10% (v/v) $CH_3OH$ in $CH_2Cl_2$).
NMR: The spectrum was consistent with the title structure and verified the presence of two diastereomers.
HPLC: Greater than 99.7% pure (36% and 63.7%).
MS (FAB): a molecular ion at m/e = 537.

| Anal. calc'd for $C_{31}H_{31}N_5O_4$: | | | |
|---|---|---|---|
| | C, 69.25; | H, 5.81; | N, 13.03; |
| Found: | C, 69.48; | H, 6.18; | N, 12.96. |

EXAMPLE 78

1 – Carboxymethyl – 1,3 – dihydro – 3(RS) – (2 – indolecarbonylamino) – 5 – phenyl – 2H – 1,4 – benzodiazepin – 2 – one

The procedure of Example 4 was carried out using 1,3 – dihydro – 3(RS) – (2 – indolecarbonylamino) – 5 – phenyl – 2H – 1,4 – benzodiazepin – 2 – one (0.87 g, 2.2 mmol) in place of 1,3 – dihydro – 5 – (2 – fluorophenyl) – 3(R) – (3' – indolyl)methyl – 2H – 1,4 – benzodiazepin – 2 – one and ethyl bromoacetate (0.38 g, 2.25 mmole) in place of methyl iodide. The chromatographed product (7% ether in $CH_2Cl_2$) (0.073 g, 0.15 mmol) and sodium hydroxide (0.2 ml, 1N, 0.2 mmol) were stirred together in $CH_3OH$ (1 ml) at room temperature for 18 hours. The mixture was concentrated in vacuo, diluted to 3 ml with $H_2O$, made acidic with 1N HCl, and extracted with $CH_2Cl_2$ (3 x 5 ml). The combined organic layers were treated with methanol (1 ml) to dissolve precipitated solid, dried over $Na_2SO_4$, filtered, and evaporated to dryness in vacuo. The residue was crystallized from ether (4 ml) and the solid dried in vacuo at 80°: (m.p. 275 – 278° (d) (↑)).
TLC: A single spot ($R_f$ = 0.21, silica gel plate, 180:10:1:1 (v/v/v/v) $CH_2Cl_2$:MeOH:HOAc: $H_2O$).
NMR: Spectrum was consistent with the title structure and verified with presence of $Et_2O$ and $CH_2Cl_2$.
HPLC: Greater than 98.5% pure.
MS: A molecular ion at m/e = 452.

| Anal. calc'd for $C_{26}H_{20}N_4O_4$.0.3 $CH_2Cl_2$.0.3 $C_4H_{10}O$ | | | |
|---|---|---|---|
| | C, 66.03; | H, 4.76; | N, 11.20; |
| Found: | C, 65.93; | H, 4.56; | N, 11.22. |

EXAMPLE 79

1,3 – Dihydro – 3(RS) – (2 – indolecarbonylamino) – 1 – methyl – 5 – phenyl – 2H – 1,4 – benzodiazepin – 2 – one (A) and 1,3 – dihydro – 1 – methyl – 3(RS) – [2 – (1 – methylindole)carbonylamino] – 5 – phenyl – 2H – 1,4 – benzodiazepin – 2 – one (B)

The procedure of Example 4 was carried out using 1,3 – dihydro – 3(RS) – (2 – indolecarbonylamino) – 5 – phenyl – 2H – 1,4 – benzodiazepin – 2 – one (0.87 g, 2.2 mmol) in place of 1,3 – dihydro – 5 – (2 – fluorophenyl) – 3(R) – (3' – indolyl)methyl – 2H – 1,4 – benzodiazepin – 2 – one. Chromatography using 7% (v/v) diethyl ether in $CH_2Cl_2$ and evaporation of the product fractions in vacuo gave A and B which were each crystallized from ether and dried in vacuo at 80°.

Compound A: (m.p. 268 – 270° (d))

TLC: A single spot ($R_f$ = 0.43, silica gel plate, 10% (v/v) $Et_2O$ in $CH_2Cl_2$).
NMR: Spectrum was consistent with the title structure and verified the presence of $Et_2O$ and $CH_2Cl_2$.
HPLC: 99% pure.
MS: A molecular ion at m/e = 408.

| Anal. calc'd for $C_{25}H_{20}N_4O_2$.0.15 $CH_2Cl_2$.0.1 $C_4H_{10}O$: | | | |
|---|---|---|---|
| | C, 71.60; | H, 5.01; | N, 13.07; |
| Found: | C, 71.79; | H, 5.01; | N, 13.01. |

Compound B: (m.p. 202.5° – 203°).

TLC: A single spot ($R_f$ = 0.67, silica gel plate, 10% (v/v) $Et_2O$ in $CH_2Cl_2$).
NMR: Spectrum was consistent with the title structure.
HPLC: Greater than 98.2% pure.
MS: A molecular ion at m/e = 422.

| Anal. calc'd for $C_{26}H_{22}N_4O_2$: | | | |
| --- | --- | --- | --- |
| Found: | C, 73.91; C, 74.05; | H, 5.25; H, 5.20; | N, 13.26; N, 13.51. |

EXAMPLE 80

1,3-Dihydro-1-methyl-3(RS)-(4-chlorophenylcarbonyl)-amino-5-(2-fluorophenyl)-2H-1,4-benzodiazepin-2-one

To a suspension of sodium hydride (50%) (84 mg, 1.82 mmole) in 4 ml of dry dimethylformamide at 0°C was added, under nitrogen, 1,3-dihydro-3(RS)-(4-chlorophenylcarbonyl)amino-5-(2-fluorophenyl)-2H-1,4-benzodiazepin-2-one (648 mg, 1.59 mmole). The resulting reaction mixture became homogeneous over a one-hour period, was stirred one hour more at 0°C and then treated with iodomethane (108 µl, 1.74 mmole). The reaction mixture was warmed to room temperature and after one hour was quenched with brine. The aqueous mixture was extracted with ethyl acetate and the combined organic extracts were washed with brine. Rotoevaporation of the dried extracts ($MgSO_4$) gave a semi-solid which was chromatographed on silica gel (chloroform-methanol-ammonia 95:5:0.5 v/v elution) to give 130 mg of recovered starting material and 360 mg of the analytical sample $R_f$ = 0.78, m.p. 171.5-172°C.
NMR ($CDCl_3$): consistent with the title structure
MS (14 ev): 421 ($M^+$) 282, 266, 255,241.

| Analysis calc'd for $C_{23}H_{17}ClFN_3O_2$ | | | |
| --- | --- | --- | --- |
| Calc'd: Found: | N, 9.96; N, 10.08; | C, 65.48; C, 65.79; | H, 4.06 H, 4.08. |

EXAMPLE 81

1,3-Dihydro-5-(2-fluorophenyl)-3(RS)-(2-indolecarbonylamino)-1-methyl-2H-1,4-benzodiazepin-2-one (A) and 1,3-Dihydro-5-(2-fluorophenyl)-1-methyl-3(RS)-[2'-(1-methylindole)carbonylamino]-2H-1,4-benzodiazepin-2-one (B)

The procedure of Example 4 was carried out using 1,3-dihydro-5-(2-fluorophenyl)-3(RS)-(2-indolecarbonylamino)-2H-1,4-benzodiazepin-2-one (0.91 g, 2.2 mmole) in place of 1,3-dihydro-5-(2-fluorophenyl)-3(R)(3'-indolyl)methyl-2H-1,4-benzodiazepin-2-one. Chromatography using 10% (v/v) diethyl ether in $CH_2Cl_2$ and evaporation of the product fractions in vacuo gave A and B which were each crystallized from $Et_2O/CH_2Cl_2$ (2/1, v/v) and dried in vacuo at 40°C.

Compound A: (m.p. 282-283.5°).

TLC: A single spot ($R_f$ = 0.53, silica gel plate, 10% (v/v) $Et_2O$ in $CH_2Cl_2$).
NMR: The spectrum was consistent with the title structure and verified the presence of ether (1/2 mole) and $CH_2Cl_2$ (3/4 mole).
HPLC: Greater than 97% pure.
MS: A molecular ion at m/e = 426.

| Anal. calc'd for $C_{25}H_{19}FN_4O_2.0.5\ C_4H_{10}O.0.75\ CH_2Cl_2$: | | | |
| --- | --- | --- | --- |
| Found: | C, 63.22; C, 63.41; | H, 4.88; H, 4.66; | N, 10.63; N, 10.59. |

Compound B: (m.p. 178 – 181 ° )

TLC: A single spot ($R_f$ = 0.76, silica gel plate, 10% (v/v) $Et_2O$ in $CH_2Cl_2$).
NMR: The spectrum was consistent with the title structure.
HPLC: Greater than 89% pure.
M.S.: A molecular ion at m/e = 440.

| Anal. calc'd for $C_{26}H_{21}FN_4O_2.0.75\ H_2O$: | | | |
|---|---|---|---|
| | C, 68.78; | H, 4.99; | N, 12.34; |
| Found: | C, 68.76; | H, 4.73; | N, 12.38. |

EXAMPLE 82

3(RS) – (2(S) – tert – Butoxycarbonylamino – 3 – phenylpropanoylamino) – 1,3 – dihydro – 5 – phenyl – 2H – 1,4 – benzodiazepin – 2 – one

3 – (RS) – Amino – 1,3 – dihydro – 5 – phenyl – 2H – 1,4 – benzodiazepin – 2 – one (1.3 g, 5.17 mmole), Boc – L – phenylalanine (1.37 g, 5.17 mmole), HBT (0.70 g, 5.17 mmole), and EDC (0.99 g, 5.17 mmole) were combined in DMF (30 ml) and stirred at room temperature. The pH of the mixture was adjusted to 9.5 with triethylamine. After 1/2 hour, the DMF was removed in vacuo and the residue treated with 10% citric acid (10 ml), neutralized with $Na_2CO_3$ and extracted with $CH_2Cl_2$ (3 x 15 ml). The combined organic layers were washed with water, dried over $Na_2SO_4$, filtered, and evaporated to dryness in vacuo. The residue was chromatographed on silica gel (90/3/0.3/0.3 $CH_2Cl_2$/MeOH/$H_2O$/HOAc) and the combined product fractions evaporated to dryness in vacuo. The residue was dissolved in $CH_2Cl_2$ (10 ml), washed with saturated $Na_2CO_3$ solution (2 ml), dried over $Na_2SO_4$, filtered and evaporated to dryness. The residue was treated with $Et_2O$ and evaporated five times to give the title compound as a mixture of diastereomers (m.p. 143 – 153 ° C).
TLC: silica gel (90/10/1/1 $CH_2Cl_2$/MeOH/MoAc/$H_20$), $R_f$ = 0.58
NMR: consistent with structure
HPLC: 97.5% pure (two diastereomers, 1:1)
M.S.: A molecular ion at m/e = 498.

| Anal. Calc'd for $C_{29}H_{30}N_4O_4$: | | | |
|---|---|---|---|
| | C, 69.86; | H, 6.07; | N, 11.24. |
| Found: | C, 69.58; | H, 6.12; | N, 11.22. |

EXAMPLE 83

3(RS) – (2(S) – tert – Butoxycarbonylamino – 3 – phenylpropanoylamino) – 1,3 – dihydro – 1 – methyl – 5 – phenyl – 2H – 1,4 – benzodiazepin – 2 – one

3(RS) – (2(S) – tert – Butoxycarbonylamino – 3 – phenylpropanoylamino) – 1,3 – dihydro – 5 – phenyl – 2H – 1,4 – benzodiazepin – 2 – one (2.5 gm, 5.01 mmol) was dissolved in DMF (20 ml) cooled to 0 ° C, treated with a 50% oil dispersion of sodium hydride (241 mg, 5.01 mmol) and stirred 30 minutes. The resulting orange solution was treated with methyl iodide (711 mg, 5.01 mmol) and stirred 1 hour at 25 ° C. The DMF was removed in vacuo, and the resulting residue treated with dilute $Na_2CO_3$ (aqueous) and extracted with EtOAc (3x). The organic extracts were combined, washed with $H_2O$ (1x), dried over $MgSO_4$, filtered and evaporated to dryness in vacuo to give a yellow oil (3.57 gm). Flash chromatography on silica gel (15% EtOAc in $CH_2Cl_2$) gave the title compound as a white foam (1.8 gm) from ether: (m.p. 117 – 20 ° C) (soften)).
TLC: Silica GF (180/10/1/1 of $CH_2Cl_2$/MeOH/$H_2O$/HoAc $R_f$ = 0.48, clean, homogeneous component
NMR: Consistent with structure
HPLC: 98.5% pure (as a 1/1 mixture of diastereomers)

M.S.: Molecular ion at m/e = 512.

| Anal. calc'd for $C_{30}H_{32}N_4O_4$: | | | |
|---|---|---|---|
| | C, 70.29; | H, 6.29; | N, 10.93; |
| Found: | C, 69.99; | H, 6.32; | N, 10.81. |

EXAMPLE 84

3(R and S)−(2(S)−Amino−3−phenylpropanoylamino)−1,3−dihydro−1−methyl−5−phenyl−2H−1,4−benzodiazepin−2−one

3(RS)−(2(S)−tert−Butoxycarbonylamino−3−phenylpropanoylamino)−1,3−dihydro−1−methyl−5−phenyl−2H−1,4−benzodiazepin−2−one (1.8 gm, 3.51 mmol) was dissolved in EtOAc (25 ml), cooled to 0 °C, and the solution saturated with HCl (g) over a 10 minute period. After stirring an additional 10 minutes the solvent was removed in vacuo. The solid residue was dissolved in $H_2O$, basified with saturated $Na_2CO_3$ (aq.) and extracted with EtOAc (3x). The organic layers were combined, washed with brine, dried over $Na_2SO_4$, filtered and stripped to dryness in vacuo to give a grey foam (1.46 gm). Flash chromatography on silica gel (90/10/1/1 of $CH_2Cl_2$/MeOH/$H_2O$/HOAc) separated the 1/1 pair of diastereomers into a clean upper ($R_f$=0.36) and clean lower ($R_f$=0.24) component. Each component was evaporated to dryness in vacuo, dissolved in $CH_2Cl_2$, washed with saturated $Na_2CO_2$ (aq.) (1x), brine (1x), dried over $Na_2SO_4$ and filtered. The individual filtrates were concentrated to dryness to give the separated diastereomers as white foams (upper component, 605 mg; lower component, 570 mg).

Upper Component(3(S)isomer): (m.p. 92−108 °C (shrink and soften))

TLC: Silica gel (90/10/1/1 of $CH_2Cl_2$/MeOH/$H_2O$/HoAc) $R_f$=0.36, single, homogeneous component
NMR: Consistent with structure.
HPLC: Greater than 98.8% single component (100% diastereomerically pure).
M.S.: Molecular ion at m/e = 412

| Anal. calc'd for $C_{35}H_{24}N_4O_2$: | | | |
|---|---|---|---|
| | C, 72.79; | H, 5.87; | N, 13.58; |
| Found: | C, 72.79; | H, 5.96; | N, 13.31. |

Lower Component(3(R)isomer): (m.p. 97−108 °C (shrink and soften))

TLC: silica gel (90/10/1/1 of $CH_2Cl_2$/MeOH/$H_2O$/HoAc) $R_f$=0.24, single, homogeneous component
NMR: Consistent with structure.
HPLC: Greater than 99.2% single component (containing less than 0.8% of upper component)
M.S.: Molecular ion at m/e = 412

| Anal. calc'd for $C_{25}H_{24}N_4O_2$: | | | |
|---|---|---|---|
| | C, 72.79; | H, 5.87; | N, 13.58; |
| Found: | C, 72.44; | H, 5.85; | N, 13.48. |

EXAMPLE 85

3(R)− and 3(S)−Amino−1,3−dihydro−1−methyl−5−phenyl−2H−1,4−benzodiazepin−2−one

3(S)−(2(S)−amino−3−phenylpropanoylamino)−1,3−dihydro−1−methyl−5−phenyl−2H−1,4−benzodiazepin−2−one, (Example 84, upper component), (1.15 g, 2.79 mmole) was combined with

phenylisothiocyanate (395 mg, 2.93 mmole) in $CH_2Cl_2$ (20 ml) and the mixture concentrated on a steam bath. The resulting oil was twice diluted with $CH_2Cl_2$ (20 ml) and both times re-concentrated on the steam bath. The oil was evaporated in vacuo to a foam which was treated with TFA (15 ml) and warmed for 18 minutes in an oil bath thermostatted at 52°. The TFA was removed in vacuo. The residue was treated twice with $CH_2Cl_2$ and with $Et_2O$, evaporated in vacuo after each treatment, and the resulting oil chromatographed on silica gel (90/10/1/1 of $CH_2Cl_2/MeOH/H_2O/HoAc$). The product fractions were evaporated in vacuo, and the residue was dissolved in $CH_2Cl_2$, washed with a small volume of 5% NaOH, dried over $Na_2SO_4$, filtered, and evaporated to give the levorotatory (3(S)) isomer of the title structure.

TLC: Silica gel (90/10/1/1 $CH_2Cl_2/MeOH/H_2O/HoAc$) $R_f = 0.31$
NMR: Consistent with structure, verifies presence of 0.15 mole of EtOAc
HPLC: Greater than 97.6% pure
M.S.: Molecular ion at m/e = 265
$[\alpha]_D^{25}$ = −236° (0.0033 g/ml, $CH_2Cl_2$)

| Anal. calc'd for $C_{16}H_{15}N_3O.0.15\ H_2O.0.15\ C_4H_{10}O$: | | | |
|---|---|---|---|
| | C, 71.43; | H, 6.07; | N, 15.06; |
| Found: | C, 71.44; | H, 5.95; | N, 15.11. |

3(R)-(2(S)-amino-3-phenylpropanoylamino)-1,3-dihydro-1-methyl-5-phenyl-2H-1,4-benzodiazepin-2-one (Example 84, lower component) was converted by the same procedure to the dextrorotatory (3(R) enantiomer of the title compound.

TLC: Silica gel (90/10/1/1 $CH_2Cl_2/MeOH/H_2O/HoAc$) $R_f = 0.31$
NMR: Consistent with structure, verifies presence of 0.15 mole of EtOAc
HPLC: Greater than 96.7% pure
M.S.: Molecular ion at m/e = 265
$[\alpha]_D^{25}$ = +227° (0.0033 g/ml, $CH_2Cl_2$)

| Anal. calc'd for $C_{16}H_{15}N_3O.0.15\ H_2O.0.15\ C_4H_{10}O$: | | | |
|---|---|---|---|
| | C, 71.43; | H, 6.07; | N, 15.06; |
| Found: | C, 71.14; | H, 5.99; | N, 14.90. |

## EXAMPLE 86

### 3(R) and 3(S)-Amino-1,3-dihydro-5-(2-fluorophenyl)-1-methyl-2H-1,4-benzodiazepin-2-one

The procedure of Example 82 was carried out using 3-(RS)-amino-1,3-dihydro-5-(2-fluorophenyl)-2H-1,4-benzodiazepin-2-one in place of 3-(RS)-amino-1,3-dihydro-5-phenyl-2H-1,4-benzodiazepin-2-one. The product was methylated using the procedure of Example 83 and the resulting methyl derivative was deprotected and separated using the procedure of Example 84. The separated isomers were each treated with phenyl isothiocyanate followed by TEA according to the method of Example 85 giving the 3(R) and 3(S) isomers of the title compound.

3(S) isomer:

TLC: Silica gel (90/10/1/1 $CH_2Cl_2/MeOH/H_2O/HoAc$), $R_f = 0.37$
NMR: Consistent with structure
HPLC: 95% pure
M.S.: Molecular ion at m/e = 283
$[\alpha]_D^{25}$ = −86.3° (0.0025 g/ml, $CH_2Cl_2$)

3(R) isomer:

TLC: Silica gel (90/10/1/1 $CH_2Cl_2$/MeOH/$H_2O$/HoAc), $R_f = 0.37$
NMR: Consistent with structure
M.S.: Molecular ion at m/e = 283
$[\alpha]_D^{25}$ = +71.4° (0.0028 g/ml, $CH_2Cl_2$)

EXAMPLE 87

3(S)-(-)-1,3-Dihydro-3-(2-indolecarbonylamino)-1-methyl-5-phenyl-2H-1,4-benzodiazepin-2-one

3(S)-(-)-3-Amino-1,3-dihydro-1-methyl-5-phenyl-2H-1,4-benzodiazepin-2-one (595 mg, 2.24 mmole) was dissolved in $CH_2Cl_2$ (15 ml) and treated with 2-indolecarbonyl chloride (403 mg, 2.24 mmole) followed by triethylamine (227 mg, 2.24 mmole). The mixture was stirred at room temperature for 30 minutes and concentrated in vacuo. The residue was chromatographed on silica gel (5% $Et_2O$/$CH_2Cl_2$) and the combined product fractions evaporated to dryness in vacuo. Three times, $Et_2O$ (15 ml) was added and evaporated in vacuo to give the title compound: (m.p. 168-185°).
TLC: Silica gel (6% $Et_2O$/$CH_2Cl_2$), $R_f = 0.23$
NMR: Consistent with structure
HPLC: Greater than 99% pure
M.S.: Molecular ion at m/e = 408
$[\alpha]_D^{25}$ = -103° (0.0078 g/ml, $CH_2Cl_2$)

| Anal. calc'd for $C_{25}H_{20}N_4O_2$ | C, 73.51; | H, 4.94; | N, 13.72; |
|---|---|---|---|
| Found: | C, 73.38; | H, 4.80; | N, 13.66. |

EXAMPLE 88

3(S)-(+)-1,3-Dihydro-5-(2-fluorophenyl)-3-(2-indolecarbonylamino)-1-methyl-2H-1,4-benzodiazepin-2-one

The procedure of Example 87 was carried out using 3(S)-(-)-3-amino-1,3-dihydro-5-(2-fluorophenyl)-1-methyl-2H-1,4-benzodiazepin-2-one in place of 3(S)-(-)-3-amino-1,3-dihydro-1-methyl-5-phenyl-2H-1,4-benzodiazepin-2-one. The title compound was obtained as a foam: (m.p. 162-187°).
TLC: Silica gel (10% $Et_2O$/$CH_2Cl_2$) $R_f = 0.30$
NMR: Consistent with structure, verifies presence of 0.2 $Et_2O$
HPLC: Greater than 99.6% pure
M.S.: Molecular ion at m/e = 426
$[\alpha]_D^{25}$ = +5.57° (0.0031 g/ml, $CH_2Cl_2$)

| Anal. calc'd for $C_{25}H_{19}FN_4O_2.0.2C_4H_{10}O$ | C, 70.22; | H, 4.80; | N, 12.70; |
|---|---|---|---|
| Found: | C, 70.13; | H, 4.75; | N, 12.61. |

EXAMPLE 89

3(R)-(-)-1,3-Dihydro-5-(2-fluorophenyl)-3-(2-indolecarbonylamino)-1-methyl-2H-1,4-benzodiazepin-2-one

The procedure of Example 88 was carried out using 3(R)-(+)-3-amino-1,3-dihydro-5-(2-fluorophenyl)-1-methyl-2H-1,4-benzodiazepin-2-one in place of its 3(S)-(-) isomer. The title compound was obtained as a foam; (m.p. 162-187°)
TLC: Silica gel (10% $Et_2O/CH_2Cl_2$) $R_f = 0.30$
NMR: Consistent with structure, verifies presence of 0.1 $Et_2O$
HPLC: Greater than 99.6% pure
M.S.: Molecular ion at m/e = 426
$[\alpha]_D^{25}$ = -5.65° (0.0023 g/ml, $CH_2Cl_2$)

| Anal. calc'd for $C_{25}H_{19}FN_4O_2.0.1C_4H_{10}O$ | C, 70.31; | H, 4.65; | N, 12.92; |
|---|---|---|---|
| Found: | C, 70.16; | H, 4.64; | N, 12.86. |

EXAMPLE 90

3(R)-(-)-1,3-Dihydro-3-(4-chlorobenzoylamino)-5-(2-fluorophenyl)-1-methyl-2H-1,4-benzodiazepin-2-one

3(R)-(+)-3-Amino-1,3-dihydro-5-(2-fluorophenyl)-1-methyl-2H-1,4-benzodiazepin-2-one (350 mg, 1.24 mmole) was dissolved in $CH_2Cl_2$ (4 ml) and treated with 4-chlorobenzoyl chloride (217 mg, 1.24 mmole) followed by triethylamine (125 mg, 1.24 mmole). The mixture was stirred at room temperature for 30 minutes and concentrated in vacuo. The residue was chromatographed on silica gel (4% $Et_2O/CH_2Cl_2$) and the combined product fractions evaporated to dryness in vacuo. Ether was added and removed in vacuo three times, giving the title compound as a foam; (m.p. 113-128°).
TLC: Silica gel (10% $Et_2O/CH_2Cl_2$) $R_f = 0.43$
NMR: Consistent with structure
HPLC: Greater than 99.6% pure
M.S.: Molecular ion at m/e = 421
$[\alpha]_D^{25}$ = -12.8° (0.0031 g/ml, $CH_2Cl_2$)

| Anal. calc'd for $C_{23}H_{17}ClFN_3O_2$ | C, 65.48; | H, 4.06; | N, 9.96; |
|---|---|---|---|
| Found: | C, 65.48; | H, 4.17; | N, 9.93. |

EXAMPLE 91

3(S)-(+)-1,3-Dihydro-3-(4-chlorobenzoylamino)-5-(2-fluorophenyl)-1-methyl-2H-1,4-benzodiazepin-2-one

The procedure of Example 90 was carried out using 3(S)-(-)-3-amino-1,3-dihydro-5-(2-fluorophenyl)-1-methyl-2H-1,4-benzodiazepin-2-one in place of its 3(R)-(+)-isomer. The title compound was obtained as a foam; (m.p. 113-128°).
TLC: Silica gel (10% $Et_2O/CH_2Cl_2$) $R_f = 0.43$
NMR: Consistent with structure.
HPLC: Greater than 99.6% pure
M.S.: Molecular ion at m/e = 421
$[\alpha]_D^{25}$ = +13.2° (0.0032 g/ml, $CH_2Cl_2$).

| Anal. calc'd for $C_{23}H_{17}ClFN_3O_2$ | | | |
|---|---|---|---|
| | C, 65.48; | H, 4.06; | N, 9.96; |
| Found: | C, 65.43; | H, 4.09; | N, 9.81. |

EXAMPLE 92

3(S)-(-)-1,3-Dihydro-3-(4-bromobenzoylamino)-1-methyl-5-phenyl-2H-1,4-benzodiazepin-2-one

3(S)-(-)-3-Amino-1,3-dihydro-1-methyl-5-phenyl-2H-1,4-benzodiazepin-2-one (35 mg, 0.132 mmole) was dissolved in $CH_2Cl_2$ (1 ml) and treated with 4-bromobenzoylchloride (29 mg, 0.132 mmole) followed by triethylamine (13.3 mg, 10.132 mmole). The mixture was stirred at room temperature for 30 minutes and concentrated in vacuo. The residue was chromatographed on silica gel (3% $Et_2O/CH_2Cl_2$) and the combined product fractions evaporated to dryness in vacuo. Ether was added and removed in vacuo three times, giving the title compound as a foam; (m.p. 120-133°).
TLC: Silica gel (7% $Et_2O/CH_2Cl_2$), $R_f$ = 0.36
NMR: Consistent with structure
HPLC: Greater than 99.1% pure
M.S.: Molecular ion at m/e 447
$[\alpha]_D^{25}$ = -72.4° (0.0027 g/ml, $CH_2Cl_2$).

| Anal. calc'd for $C_{23}H_{18}BrN_3O_2$ | | | |
|---|---|---|---|
| | C, 61.62; | H, 4.05; | N, 9.37; |
| Found: | C, 61.94; | H, 4.07; | N, 9.20. |

EXAMPLE 93

3(R)-(+)-1,3-Dihydro-3-(4-bromobenzoylamino)-1-methyl-5-phenyl-2H-1,4-benzodiazepin-2-one

The procedure of Example 92 was carried out using 3(R)-(+)-3-amino-1,3-dihydro-1-methyl-5-phenyl-2H-1,4-benzodiazepin-2-one in place of its 3(S)-(-) isomer. The title compound was obtained as a foam; (m.p. 120-133°)
TLC: Silica gel (7% $Et_2O/CH_2Cl_2$); $R_f$ = 0.36
NMR: Consistent with structure
HPLC: Greater than 99.2% pure
M.S.: Molecular ion at m/e = 447
$[\alpha]_D^{25}$ = +75.1° (0.0022 g/ml, $CH_2Cl_2$).

| Anal. calc'd for $C_{23}H_{18}BrN_3O_2$ | | | |
|---|---|---|---|
| | C, 61.62; | H, 4.05; | N, 9.37; |
| Found: | C, 62.00; | H, 4.12; | N, 9.27. |

EXAMPLE 94

3(R)-(+)-1,3-Dihydro-3-(2-indolecarbonylamino)-1-methyl-5-phenyl-2H-1,4-benzodiazepin-2-one

The procedure of Example 87 was carried out using 3(R)-(+)-3-amino-1,3-dihydro-1-methyl-5-phenyl-2H-1,4-benzodiazepin-2-one in place of its 3(S)-(-) isomer. The title compound was obtained as a foam; (m.p. 168-185°).

TLC: Silica gel (6% EtO/$CH_2Cl_2$); $R_f = 0.23$
NMR: Consistent with structure
HPLC: Greater than 99.2% pure
M.S.: Molecular ion at m/e = 408
$[\alpha]_D^{25}$ = +100° (0.0052 g/ml, $CH_2Cl_2$).

| Anal. calc'd for $C_{25}H_{20}N_4O_2$ | | | |
|---|---|---|---|
| | C, 73.51; | H, 4.94; | N, 13.72; |
| Found: | C, 73.16; | H, 4.88; | N, 13.53. |

## EXAMPLE 95

Z-1,3-Dihydro-1-methyl-5-phenyl-3-(3-thienylmethylene)-2H-1,4-benzodiazepin-2-one and E-1,3-Dihydro-1-methyl-5-phenyl-3-(3-thienylmethylene)-2H-1,4-benzodiazepin-2-one

To a cooled (-60°C) solution of diisopropylamine (0.84 ml, 6.0 mmol) in THF (10.2 ml) was added 1.5M butyllithium in hexane (4.0 ml, 6.0 mmol). The solution was stirred 10 min. at -60°C and then warmed to 25°C. The light yellow solution was recooled to -60°C and treated with solid 1,3-dihydro-1-methyl-5-phenyl-2H-1,4-benzodiazepin-2-one (75 mg, 3.0 mmol) portionwise (5 x 15 mg). The reaction was permitted to warm to 0°C and then recooled to -60°C. A solution of thiophene-3-carboxaldehyde (336 mg, 3.0 mmol) in THF (6 ml) was added to the deep red anion solution, the cooling bath was removed, and the reaction allowed to warm to 25°C. The reaction was quenched with brine and extracted with ether (3X). The combined extracts were washed with $H_2O$ (1X), dried over $MgSO_4$, filtered, and stripped to dryness in vacuo. The crude red oil was chromatographed on silica gel (10% $Et_2O$ in $CH_2Cl_2$) to give the intermediate alcohol as a buff-colored solid: 210 mg, m.p. 188-9°C.
TLC: silica GF (10% $Et_2O$ in $CH_2Cl_2$) single homogeneous component. A portion of this product (171 mg, 0.472 mmol) was refluxed in a mixture of trifluoroacetic acid (3 ml) and trifluoroacetic anhydride (1 ml) for 12 hrs. The solvent was removed in vacuo and the residue was treated with $H_2O$, basified with 10% NaOH (aq) and extracted with ether (3X). The combined extracts were washed with $H_2O$ (1X), dried over $MgSO_4$, filtered and stripped to dryness in vacuo to give a crude oil. Chromatography on silica gel (2% $Et_2O$ in $CH_2Cl_2$) provided the title compounds which were obtained as light yellow solids from ether.

Z-isomer: (m.p. 196-197°C).

TLC: Silica GF (4% $Et_2O$ in $CH_2Cl_2$), $R_f$ = 0.37, single homogeneous component.
PMR: Consistent with the title structure.
HPLC: Greater than 99.8% pure.
M.S.: Mol. ion = 344 m/e.

| Anal. calc'd for $C_{21}H_{16}N_2OS$: | | | |
|---|---|---|---|
| | C, 73.23; | H, 4.68; | N, 8.13; |
| Found: | C, 73.37; | H, 4.78; | N, 7.79. |

F-isomer: (m.p. 194-196°C).

TLC: Silica GF (4% $Et_2O$ in $CH_2Cl_2$), $R_f$ = 0.28 single homogeneous component.
PMR: Consistent with the title structure.
HPLC: Greater than 99.9% pure.
M.S.: Mol. ion = 344 m/e.

| Anal. calc'd for $C_{21}H_{16}N_2OS$: | | | |
|---|---|---|---|
| | C, 73.23; | H, 4.68; | N, 8.13; |
| Found: | C, 73.12; | H, 4.83; | N, 7.73. |

EXAMPLE 96

3(RS) - (BOC - D - tryptophyl)amino - 1,3 - dihydro - 5 - phenyl - 2H - 1,4 - benzodiazepin - 2 - one

The procedure of Example 77 was carried out using BOC - D - tryptophan in place of BOC - L - tryptophan. The chromatographed product was crystallizd from $Et_2O$ and dried in vacuo at 80°: (m.p. 171 - 174° (↑)).
TLC: A single spot ($R_f$ = 0.56, silica gel plate, 10% (v/v) $CH_3OH$ in $CH_2Cl_2$).
NMR: The spectrum was consistent with the title structure and verified the presence of two diastereomers.
HPLC: Greater than 98.4% pure (68.9% and 29.5%).

| Anal. calc'd for $C_{31}H_{31}N_5O_4$: | | | |
|---|---|---|---|
| | C, 69.25; | H, 5.81; | N, 13.03; |
| Found: | C, 69.24; | H, 6.03; | N, 13.04. |

EXAMPLE 97

3(RS) - [4 - (3 - Indole)butyrylamino] - 1,3 - dihydro - 5 - phenyl - 2H - 1,4 - benzodiazepin - 2 - one

The procedure of Example 77 was carried out using 4 - (3 - indolyl)butyric acid (0.082 g, 0.4 mmol) in place of BOC - L - tyrptophan. The product was chromatographed as in Example 75, crystallized from a mixture of acetone (1 ml) and ether (3 ml), and dried in vacuo at 80°: (m.p., 258 - 259°).
NMR: The spectrum was consistent with the title structure.
HPLC: 98.9% pure.
MS: A molecular ion at m/e = 436.

| Anal. calc'd for $C_{27}H_{24}N_4O_2$: | | | |
|---|---|---|---|
| | C, 74.29; | H, 5.54; | N, 12.84; |
| Found: | C, 74.39; | H, 5.65; | N, 12.93. |

EXAMPLE 98

1,3 - Dihydro - 3(RS) - (benzyloxycarbonyl)aminomethyl - 5 - (2 - fluorophenyl) - 2H - 1,4 - benzodiazepine

To a magnetically stirred solution of 1,3 - dihydro - 3(RS) - benzyloxycarbonyl)aminomethyl - 5 - (2 - fluorophenyl) - 2H - 1,4 - benzodiazepin - 2 - thione (1.85 g, 4.3 mmol) in 150 ml of ethanol were added, at room temperature, three portions of freshly prepared Raney nickel (slurried in ethanol, approximately 4 - 5 g). The resulting reaction mixture was stirred vigorously overnight and treated with an additional equal portion of Raney nickel. After 50 hours of total reaction time, the suspension was filtered carefully; the residual Raney nickel was washed copiously with ethanol. Concentration of the filtrate under reduced pressure gave 880 mg of product essentially homogeneous by TLC (ethyl acetate - hexane 1:1 v/v). The analytical sample was obtained via silica gel chromatography (chloroform - methanol 96:4) as a foam. TLC, HPLC greater than 97% pure.
NMR ($CDCl_3$): Consistent with the title structure.
MS (14 ev): 403 ($M^+$), 295, 253, 239, 219.

| Anal. calc'd for $C_{24}H_{22}FN_3O_2.0.03\ CHCl_3$: | | | |
|---|---|---|---|
| | N, 10.32; | C, 70.90, | H, 5.45; |
| Found: | N, 10.16; | C, 70.89; | H, 5.60. |

EXAMPLE 99

1,3 – Dihydro – 3(RS) – [3' – (thiophene)carbonyl]aminomethyl – 5 – (2 – fluorophenyl) – 2H – 1,4 – benzodiazepine

1,3 – Dihydro – 3(RS) – aminomethyl – 5 – (2 – fluorophenyl) – 2H – 1,4 – benzodiazepine hydrobromide (300 mg, 0.59 mmol) and 3 – thiophenecarboxylic acid chloride (150 mg, 1.02 mmol) were combined in 50 ml of methylene chloride. The reaction mixture was immersed in an ice bath and treated with triethylamine (330 µl, 2.36 mmol). After addition was complete, stirring was continued at 0 ° C for 10 min. more and then at room temperature for 15 min. The reaction mixture was partitioned between methylene chloride and saturated sodium bicarbonate solution. The phases were separated and the organic layer was washed with brine, then dried ($MgSO_4$) and concentrated under reduced pressure. The crude product (300 mg) was purified via silica gel chromatography (chloroform – methanol – ammonia, 95:5:0.5 v/v, elution) to give the analytical sample.
NMR ($CDCl_3$): Consistent with the title structure.
MS (14 ev): 379 ($M^+$)

| Anal. calc'd for $C_{21}H_{18}FN_3OS.0.1\ CHCl_3$: | | | |
|---|---|---|---|
| | N, 10.74; | C, 64.75; | H, 4.66; |
| Found: | N, 10.45; | C, 64.51; | H, 4.82. |

EXAMPLE 100

1,3 – Dihydro – 3(RS) – (2' – indolecarbonyl)aminomethyl – 5 – (2 – fluorophenyl) – 2H – 1,4 – benzodiazepine Solvate

1,3 – Dihydro – 3(RS) – aminomethyl – 5 – (2 – fluorophenyl) – 2H – 1,4 – benzodiazepine hydrobromide (300 mg, 0.59 mmol) and 2 – indole carboxylic acid chloride (127 mg, 0.70 mmol) were combined in 30 ml of methylene chloride. The reaction mixture was immersed in an ice bath and treated with triethylamine (330 µl, 2.36 mmol). After addition was complete, stirring was continued at 0 ° C for 10 min. more and then at room temperature for 15 minutes. The reaction mixture was partitioned between methylene chloride and saturated sodium bicarbonate solution. The phases were separated and the organic layer was washed with brine, then dried ($MgSO_4$) and concentrated under reduced pressure. The crude product (220 mg) was purified via silica gel chromatography (chloroform – methanol elution, 95:5 v/v) to give the analytical sample.
NMR ($CDCl_3/CD_3OD$): Consistent with the title structure.
MS (14 ev): 412 ($M^+$), 252, 239.

| Anal. calc'd for $C_{25}H_{21}FN_4O.0.15\ CHCl_3$: | | | |
|---|---|---|---|
| | N, 13.01; | C, 70.19; | H, 4.95; |
| Found: | N, 12.70; | C, 70.19; | H, 5.18. |

EXAMPLE 101

**1,3-Dihydro-3(RS)-(2-L-hydroxy-2-phenylacetyl)aminomethyl-5-(2-fluorophenyl)-2H-1,4-benzodiazepine**

1,3-Dihydro-3(RS)-aminomethyl-5-(2-fluorophenyl)-2H-1,4-benzodiazepine hydrobromide (300 mg, 0.59 mmol) and L-mandelic acid (134 mg, 0.88 mmol) were combined in 5 ml of dimethylformamide and treated with 1-ethyl-3-(3-dimethylaminopropyl)carbodiimide hydrochloride (169 mg, 0.88 mmol). The pH of the resulting reaction mixture was adjusted to 8.5 with triethylamine and the reaction was stirred at room temperature overnight. The solvent was removed under reduced pressure and the residue was dissolved in ethyl acetate (60ml). The organic phase was then washed in succession with sodium bicarbonate solution (3 x 50 ml) and brine. The dried ($MgSO_4$) extracts were concentrated to give 200 mg of crude product as a mixture of diastereomers. Preparative thick layer chromatography (chloroform - ethanol - ammonia elution, 90:10:1 v/v) afforded the less polar, faster moving component as a homogeneous analytical simple.
HPLC: Greater than 98% pure.
NMR ($CDCl_3$): Consistent with the title structure.
MS (14 ev): 403 ($M^+$), 252, 239,212.

| Anal. calc'd for $C_{24}H_{22}FN_3O_2.0.5\ H_2O$ | | | |
|---|---|---|---|
| | N, 10.18; | C, 69.82; | H, 5.62; |
| Found: | N, 9.67; | C, 69.81; | H, 5.55. |

EXAMPLE 102

1-(2-Cyanoethyl)-1,3-dihydro-5-(2-fluorophenyl)-3(R)-(3'-indolyl)methyl-2H-1,4-benzodiazepin-2-one (A, 85%) and 1-(2-cyanoethyl)-1,3-dihydro-5-(2-fluorophenyl)-3(R)-[1'-(2-cyanoethyl)-3'-indolyl]-methyl-2H-1,4-benzodiazpin-2-one (B, 15%)

The procedure of Example 4 was carried out using acrylonitrile (0.12 g, 2,3 mmol) in place of methyl iodide. The chromatographed product, a mixture of A (85%) and B (15%) was dried in vacuo at 90°: (m.p. 97-105° (↑)).
NMR: The spectrum was consistent with the 85:15 mixture of the title structure and showed the presence of 0.9 mol of DMF.
HPLC: 96.4% (82.4% + 14.0%).
TLC: A single spot ($R_f$ = 0.22, silica gel plate, 5% (v/v) $Et_2O$ in $CH_2Cl_2$).
MS: Molecular ions at m/e=436 and 489.

| Anal. calc'd for 0.85 $C_{27}H_{21}FN_4O$ + 0.15 $C_{30}H_{24}FN_5O.0.9\ C_3H_7NO$: | | | |
|---|---|---|---|
| | C, 71.07; | H, 5.35; | N, 13.88; |
| Found: | C, 70.95; | H, 5.18; | N, 13.63. |

EXAMPLE 103

1-(2-Carboxyethyl)-1,3-dihydro-5-(2-fluorophenyl)-3(R)-(3'-indolyl)methyl-2H-1,4-benzodiazepin-2-one

The procedure of Example 4 was carried out using ethyl acrylate (0.22 g, 2.2 mmole) in place of methyl iodide. The chromatographed product was evaporated in vacuo, dissolved in methanol (5 ml), treated with sodium hydroxide (0.91 ml of 1 M solution), and stirred at room temperaure for 24 hours. The mixture was

evaporated in vacuo, and the residue was dissolved in water (10 ml), washed with ether (10 ml), acidified with 1 N HCl, and extracted with $CH_2Cl_2$ (3 x 10 ml). The $CH_2Cl_2$ layers were washed with water (1 x 10 ml), dried over sodium sulfate, filtered, and evaporated to dryness in vacuo. The residue was chromatographed on silica gel (180:5:1:1 followed by 180:10:1:1 (v/v/v/v) $CH_2Cl_2$:$CH_3OH$:HoAc:$H_2O$) and the product evaporated to dryness in vacuo. The residue was dried in vacuo at 40°: (m.p.~75-90° foam, ~130-160° melt).

TLC: A single spot ($R_f$ = 0.32, silica gel plate, 180:10:1:1 (v/v/v/v) $CH_2Cl_2$:$CH_3OH$:HOAc:$H_2O$).

NMR: The spectrum was consistent with the title structure and verified the presence of ether.

HPLC: 99.6% pure.

MS: A molecular ion at m/e = 455.

| Anal. calc'd for $C_{27}H_{22}FN_3O_3$.0.55 $C_4H_{10}O$.0.35 $H_2O$): | | | |
|---|---|---|---|
| | C, 69.78; | H, 5.66; | N, 8.36; |
| Found: | C, 69.72; | H, 5.29; | N, 8.07. |

EXAMPLE 104

1,3-Dihydro-3(R)-(3'-indolyl)methyl-5-(2-fluorophenyl)-2H-1,4-benzodiazepin-2-one-4-oxide

1,3-Dihydro-3(R)-(3'-indolyl)methyl-5-(2-fluorophenyl)-2H-1,4-benzodiazepin-2-one (300 mg, 0.78 mmol) and m-chloroperoxybenzoic acid (85%) (156 mg, 0.90 mmol) were combined at room templerature in 20 ml of chloroform. The reaction mixture was allowed to stand at room temperarure overnight, then was diluted with 30 ml of chloroform and washed with cold, saturated sodium bicarbonate solution. The combined organic extracts were washed with brine, dried ($MgSO_4$) and concentrated to afford 310 mg of crude product. Silica gel chromatography (hexane-ethyl acetate, 1:2 v/v) provided the analytical sample.

HPLC: 99% pure.

NMR ($CDCl_3$): Consistent with the title structure.

MS (14 ev): 415, 397, 369, 267.

| Anal. calc'd. for $C_{24}H_{18}FN_3O_2$.1.0 $CHCl_3$ | | | |
|---|---|---|---|
| | N, 8.10; | C, 57.87; | H, 3.69; |
| Found: | N, 8.09; | C, 58.14; | H, 3.82. |

EXAMPLE 105

1,3-Dihydro-5-(2-fluorophenyl)-3-(RS)-(2-indolecarbonyl amino)-2H-1,4-benzodiazepin-2-one

3-(RS)-Amino-1,3-dihydro-5-(2-fluorophenyl)-2H-1,4-benzodiazepin-2-one (1.5 gm, 5.57 mmol), indole-2-carbonyl chloride (1.05 gm, 5.85 mmol) and triethylamine (0.814 ml, 5.85 mmol) were combined in $CH_2Cl_2$ (15 ml) and stirred 10 min. The reaction was concentrated and chromatographed on silica gel (5% MeOH in $CH_2Cl_2$) to give the title compound as a white solid from $CH_2Cl_2$: (m.p. 290-291°).

TLC: Silica GF (5% MeOH in $CH_2Cl_2$), single homogeneous component.

NMR: Consistent with title structure and verifies the presence of 0.16 $CH_2Cl_2$.

HPLC: Greater than 99% pure.

M.S.: Mol. ion = 412 m/e (free base).

| Anal. calc'd for $C_{24}H_{17}FN_4O_2.0.16\ CH_2Cl_2$: | | | |
|---|---|---|---|
| | C, 68.11; | H, 4.10; | N, 13.15; |
| Found: | C, 68.06, | H, 4.12; | N, 12.91. |

EXAMPLE 106

1,3-Dihydro-3-(RS)-(4-nitrophenylcarbonyl)amino-5-(2-fluorophenyl)-2H-1,4-benzodiazepin-2-one

1,3-Dihydro-3-(RS)-amino-5-(2-fluorophenyl)-2H-1,4-benzodiazepin-2-one (100 mg, 0.37 mmol) and p-nitrobenzoic acid (70 mg, 0.41 mmol) were combined at room temperature in 5 ml of methylene chloride. To this reaction mixture was added 1-ethyl-3-(3-dimethylaminopropyl)carbodiimide hydrochloride (79 mg, 0.41 mmol). The pH of the reaction mixture was then adjusted to 8.5 with triethylamine and stirring was continued at room temperature overnight. The reaction mixture was partitioned between methylene chloride and 10% citric acid solution. The phases were separated and the organic layer was washed in succession with 10% citric acid solution (1 x 30 ml), saturated sodium bicarbonate solution (2 x 30 ml) and brine. The dried ($MgSO_4$) extracts were concentrated to yield 83 mg of crude product. Preparative thick layer chromatography (chloroform - methanol - ammonia, 96:4:0.4 v/v) afforded the analytical sample (70 mg).
HPLC: Greater than 96.5% pure.
NMR ($CDCl_3$): Consistent with the title structure.
MS (14 ev): 418 ($M^+$), 268, 252.

| Anal. calc'd for $C_{22}H_{15}FN_4O_4.0.1\ CHCl_3$ | | | |
|---|---|---|---|
| | N, 13.02; | C, 61.68; | H, 3.54; |
| Found: | N, 12.66; | C, 61.94; | H, 3.74. |

EXAMPLE 107

1,3-Dihydro-3-(RS)-(2-indolecarbonyloxy)-5-phenyl-2H-1,4-benzodiazepin-2-one

1,3-Dihydro-3-(RS)-hydroxy-5-phenyl-2H-1,4-benzodiazepin-2-one (100 mg, 0.398 mmol) was dissolved in $CH_2Cl_2$ (10 ml), treated with indole-2-carbonyl chloride (78.6 mg, 0.438 mmol) and 4-dimethylaminopyridine (DMAP, 53.5 mg, 0.438 mmol) and stirred 16 hrs. at 25° C. A second portion of indole-2-carbonylchloride (78.6 mg, 0.438 mmol and DMAP (53.5 mg, 0.438 mmol) was added and the reaction stirred an additional 24 hrs. Chromatography of the reaction mixture on silica gel (1% MeOH in $CH_2Cl_2$) gave the title compound (100 mg) as a white solid from MeCN: (m.p. 271-273°).
TLC: Silca GF (4% MeOH in $CH_2Cl_2$), $R_f$ = 0.41, single homogeneous component.
NMR: Consistent with title structure.
HPLC: Greater than 98.6% pure.
MS: Molecular ion at m/e = 395.

| Anal. calc'd for $C_{24}H_{17}N_3O_3$: | | | |
|---|---|---|---|
| | C, 72.90; | H, 4.33; | N, 10.63; |
| Found: | C, 72.70; | H, 4.31; | N, 10.64. |

EXAMPLE 108

1,3-Dihydro-5-(2-fluorophenyl)-3-(RS)-(3-thiophene carbonylamino)-2H-1,4-benzodiazepin-2-one

3-(RS)-Amino-1,3-dihydro-5-(2-fluorophenyl)-2H-1,4-benzodiazepin-2-one (75 mg, 0.229 mmol), thiophene-3-carbonyl chloride (44.9 mg, 0.306 mmol) and triethylamine (42.5 µl, 0.306 mmol) were combined in $CH_2Cl_2$ (4 ml) and stirred 10 min. at 25°C. The reaction was concentrated and chromatographed on silica gel (2% MeOH in $CH_2Cl_2$) to give the title compound as a white solid from $Et_2O$: (m.p. 238-239°).
TLC: Silica GF (5% MeOH in $CH_2Cl_2$), $R_f$ = 0.36, single homogeneous component.
NMR: Consistent with title structure and verifies the presence of .05 $(C_2H_5)_2O$ and 0.70 $H_2O$).
HPLC: Greater than 98.8% pure.
MS: Mol. ion = 379 m/e (free base).

| Anal. calc'd for $C_{20}H_{14}FN_3O_2S$. .05 $(C_2H_5)_2O$.0.70 $H_2O$: | | | |
|---|---|---|---|
| | C, 61.30; | H, 4.05; | N, 10.62; |
| Found: | C, 61.24; | H, 3.68; | N, 10.57. |

EXAMPLE 109

1,3-Dihydro-3-(RS)-(3-indolecarbonylamino)-5-phenyl-2H-1,4-benzodiazepin-2-one

3-(RS)-Amino-1,3-dihydro-5-phenyl-2H-1,4-benzodiazepin-2-one (49.2 mg, 0.196 mmol), indole-3-carboxylic acid (37.9 mg, 0.235 mmol) and 1M DCC in $CH_2Cl_2$ solution (0.235 ml, 0.235 mmol) were mixed in DMF (2 ml) and the pH adjusted to 9.0 with triethylamine (32.7 µl, 0.235 mmol). The reaction was stirred 18 hrs. at 25°C, the DMF removed in vacuo, and the residue chromatographed on a Waters Semi-Prep C-18 30 x 0.9 cm column (gradient elution of 5 to 95% $CH_3CN$ in $H_2O$) to give the title compound as a white solid from MeOH/ether: (m.p. 265-268°).
TLC: Silica GF (90/10/1/1 of $CH_2Cl_2$/MeOH/$H_2O$/HOAc), $R_f$ = 0.57, single homogeneous component.
NMR: Consistent with title structure and verifies the presence of 2.0 $CH_3OH$.
HPLC: 100% pure.
MS: Mol. ion = 394 m/e (free base).

| Anal. calc'd for $C_{24}H_{18}N_4O_2$.2$CH_3OH$: | | | |
|---|---|---|---|
| | C, 68.10; | H, 5.72; | N, 12.22; |
| Found: | C, 68.19; | H, 4.62; | N, 12.50. |

EXAMPLE 110

1,3-Dihydro-3-(RS)-(4-thianaphtheneacetyl)amino-5-(2-fluorophenyl)-2H-1,4-benzodiazepin-2-one

1,3-Dihydro-3-(RS)-amino-5-(2-fluorophenyl)-2H-1,4-benzodiazepin-2-one (100 mg, 0.37 mmol) and 4-thianaptheneacetic acid (79 mg, 0.41 mmol) were combined at room temperature in 5 ml of methylene chloride. To this reaction mixture was added 1-ethyl-3-(3-dimethylaminopropyl)carbodiimide hydrochloride (79 mg, 0.41 mmole). The pH of the reaction mixture was then adjusted to 8.5 with triethylamine and stirring was continued at room temperature overnight. The reaction mixture was partitioned between methylene chloride and 10% citric acid solution. The phases were separated and the organic layer was washed in succession with 10% citric acid solution (1 x 30 ml), saturated sodium bicarbonate solution (2 x 30 ml) and brine. The dried ($MgSO_4$) extracts were concentrated to yield 130mg of crude product. Preparative thick layer chromatography (chloroform - methanol - ammonia, 95:5:0.5 v/v) afforded the analytical sample, m.p. 259-260°C.

NMR ($CDCl_3$): consistent with the title structure.
MS (14 ev): 443 ($M^+$), 268, 174.

| Anal. calc'd for $C_{25}H_{18}FN_3O_2S.0.075\ CHCl_3$ | | | |
|---|---|---|---|
| | N, 9.28; | C, 66.56; | H, 4.02; |
| Found: | N, 9.10; | C, 66.53; | h, 4.11. |

EXAMPLE 111

1,3-Dihydro-3-(RS)-(4-chlorophenylcarbonyl)amino-5-(2-fluorophenyl)-2H-1,4-benzodiazepin-2-one

1,3-Dihydro-3-(RS)-amino-5-(2-fluorophenyl)-2H-1,4-benzodiazepin-2-one (100 mg, 0.37 mmol) and p-chlorobenzoyl chloride (52 µl, 0.41 mmole) were combined at room temperature in 5 ml of methylene chloride. The resulting solution was protected from moisture and stirred at room temperature overnight. The reaction mixture was diluted with 70 ml of methylene chloride and washed with sodium bicarbonate solution (sat.) and brine. The organic extracts were dried ($MgSO_4$) and concentrated to give 150 mg of crude product. Chromatography on silica gel (chloroform - methanol - ammonia, 95:5:0.5 v/v) and trituration with hexane yielded the analytical product as a white powder, m.p. 258-259°C.
HPLC: Greater than 98% pure.
NMR: ($CDCl_3$): Consistent with the title structure.
MS (14 ev): 407 ($M^+$), 268, 252, 241.

| Anal. calc'd for $C_{22}H_{15}ClFN_3O_2.0.2\ CHCl_3$ | | | |
|---|---|---|---|
| | N, 9.73; | C, 61.76; | H, 3.55; |
| Calc'd: | N, 9.34; | C, 61.65; | H. 3.68. |

EXAMPLE 112

1,3-Dihydro-3-(RS)-(4-methylphenylsulfonyl)amino-5-(2-fluorophenyl)-2H-1,4-benzodiazepin-2-one

1,3-Dihydro-3-(RS)-amino-5-(2-fluorophenyl)-2H-1,4-benzodiazepin-2-one (116 mg, 0.43 mmole) and p-toluenesulfonyl chloride (82 mg, 0.43 mmole) were combined at room temperature in 5 ml of methylene chloride. The pH of the reaction mixture was then adjusted to 8.5 with triethylamine and stirring was continued at room temperature overnight. The reaction mixture was partitioned between methylene chloride and 10% citric acid solution. The phases were separated and the organic layer was washed in succession with 10% citric acid solution (1 x 30 ml), saturated sodium bicarbonate solution (2 x 30 ml) and brine. The dried ($MgSO_4$) extracts were concentrated to yield 200 mg of crude product. Recrystallization from ethyl acetate afforded the analytical sample as white needles, m.p. 215-216°C.
HPLC: Greater than 99% pure.
NMR ($CDCl_3$): Consistent with the title structure.
MS (14 ev): 359, 316, 268, 241, 225, 212, 92.

| Anal. calc'd for $C_{22}H_{18}FN_3O_3S.0.1C_4H_8O_2$ | | | |
|---|---|---|---|
| | N, 9.72; | C, 62.23; | H, 4.38; |
| Found: | N, 9.64; | C, 61.92 | H, 4.31. |

EXAMPLE 113

1-Carboxymethyl-1,3-dihydro-5-(2-fluorophenyl)-3(RS)-(2-indolecarbonylamino)-2H-1,4-benzodiazepin-2-one

The procedure of Example 4 was carried out using 1,3-dihydro-5-(2-fluorophenyl)-3(RS)-(2-indolecarbonylamino)-2H-1,4-benzodiazepin-2-one (0.92 g, 2.2 mmole) in place of 1,3-dihydro-5-(2-fluorophenyl)-3-(R)-(3'-indolyl)-methyl-2H-1,4-benzodiazepin-2-one, and ethyl bromoacetate (0.38 g, 2.25 mmol) in place of methyl iodide. The chromatographed product (10% ether in $CH_2Cl_2$) (0.05 g, 0.098 mmol) and sodium hydroxide (0.14 ml, 1N, 0.14 mmol) were stirred together in $CH_3OH$ (3 ml) at room temperature for 36 hours. The mixture was concentrated in vacuo, diluted to 5 ml with $H_2O$, made acidic with 1 N HCl, and extracted with $CH_2Cl_2$ (3 x 5 ml). The organic layers were combined, washed with water (1 x 5 ml), dried over $Na_2SO_4$, filtered, and evaporated to dryness in vacuo. The residue was crystallized from acetone (0.1 ml) and $Et_2O$ (2 ml) and the solid dried in vacuo at 60°; (m.p. 278-278.5° (d)).
TLC: A single spot ($R_f$ = 0.27, silica gel plate, 180:10:1:1 (v/v/v/v) $CH_2Cl_2$:$CH_3OH$:HOAc:$H_2O$).
NMR: The spectrum was consistent with the title structure and verified the presence of ether and acetone.
HPLC: 99.4% pure.
MS: A molecular ion at m/e = 470.

| Anal. calc'd for $C_{26}H_{19}FN_4O_4$.0.6$C_3H_6O$ .0.2$C_4H_{10}O$ .0.8 $H_2O$: | | | |
|---|---|---|---|
| | C, 64.25; | H, 4.94; | N, 10.48; |
| Found: | C, 64.29; | H, 4.56; | N, 10.23. |

EXAMPLE 114

1,3-Dihydro-3-(RS)-(5-fluoroindole-2-carbonylamino)-5-(2-fluorophenyl)-2H-1,4-benzodiazepin-2-one

3-(RS)-Amino-1,3-dihydro-5-(2-fluorophenyl)-2H-1,4-benzodiazepin-2-one (100 mg, 0.398 mmol) was suspended in 2 ml of methylene chloride. 5-fluoroindole-2-carboxylic acid chloride (87 mg, 0.438 mmol) was added to the methylene chloride suspension. The pH of the stirred mixture was adjusted to 9 with 100 $\mu$l of triethylamine. The reaction mixture was stirred for 24 hours. The mixture was then diluted with 1 ml of methanol and filtered. The filtrate was pipeted onto a 2000 $\mu$ Analtech preparative TLC plate which was developed in a 95:5:0.5 chloroform, methanol, water (CMW) solvent system. The product band was collected. The silica was washed with 90:10:1 CMW. The filtrate was evaporated and the residue was dissolved in methanol and placed in a small vial. The solvent was evaporated to yield 15.2 mg of product.
HPLC: 90% pure.
MS: $M^+$ (14 ev), m/e 430.
NMR: Consistent with title product.

| Anal. calc'd for $C_{24}H_{16}F_2N_4O_2$ 1.6$CH_3OH$ | | | |
|---|---|---|---|
| | N, 11.63; | C, 63.83; | H, 4.65; |
| Found: | N, 11.66; | C, 63.84; | H, 3.72. |

EXAMPLE 115

1,3-Dihydro-3-(RS)-(3'-methylindenyl-2-carbonyl)amino-5-(2-fluorophenyl)-2H-1,4-benzodiazepin-2-one

1,3-Dihydro-3-(RS)-amino-5-(2-fluorophenyl)-2H-1,4-benzodiazepin-2-one (100 mg, 0.37 mmol) and 3-methylindene-2-carboxylic acid (70 mg, 0.40 mmol) were combined at room temperature

in 5 ml of methylene chloride. To this reaction mixture was added 1-ethyl-3-(3-dimethylaminopropyl)-carbodiimide hydrochloride (80 mg, 0.41 mmol). The pH of the reaction mixture was then adjusted to 8.0 with triethylamine and stirring was continued at room temperature overnight (19 hours). The reaction mixture was partitioned between methylene chloride and 10% citric acid solution. The phases were separated and the organic layer was washed in succession with 10% citric acid solution (1 x 30 ml), saturated sodium bicarbonate solution (2 x 30 ml), and brine. The dried ($MgSO_4$) extrcts were concentrated to yield 130 mg of crude product. Preparative thick layer chromatography (hexane - ethyl acetate, 1:1 v/v) afforded the analytical sample.
HPLC: Greater than 98% pure.
NMR ($CDCl_3$): Consistent with the title structure.
MS (14 ev): 425 ($M^+$), 268, 199, 156.

| Anal. calc'd for $C_{26}H_{20}FN_3O_2.1.25\ H_2O$ | | | |
|---|---|---|---|
| | N, 9.38; | C, 69.70; | H, 5.06; |
| Found: | N, 8.86; | C, 69.75; | H, 4.85. |

EXAMPLE 116

1,3-Dihydro-3-(RS)-(2-quinaldyl)amino-5-(2-fluorophenyl)-2H-1,4-benzodiazepin-2-one

1,3-Dihydro-3(RS)-amino-5-(2-fluorophenyl)-2H-1,4-benzodiazepin-2-one (100 mg, 0.37 mmol) and 2-quinoline carboxylic acid (quinaldic acid) (70 mg, 0.40 mmol) were combined at room temperature in 5 ml of methylene chloride. To this reaction mixture was added 1-ethyl-3-(3-dimethylaminopropyl)carbodiimide hydrochloride (76 mg, 0.40 mmole). The pH of the reaction mixture was then adjusted to 8.5 with triethylamine and stirring was continued at room temperature for 48 hours. The reaction mixture was partioned between methylene chloride and 10% citric acid solution. The phases were separated and the organic layer was washed in succession with 10% citric acid solution (1 x 30 ml), saturated sodium bicarbonate solution (2 x 30 ml) and brine. The dried ($MgSO_4$) extracts were concentrated to yield 150 mg of crude product. Preparative thick layer chromatography (chloroform - methanol - ammonia, 97:3:0.3 v/v) afforded the analytical sample (60 mg).
NMR ($CDCl_3$): Consistent with the title structure.
MS (14 ev): 424 ($M^+$), 268, 241, 198, 184.

| Anal. calc'd for $C_{25}H_{17}FN_4O_2.0.75\ H_2O$ | | | |
|---|---|---|---|
| | N, 12.79; | C, 68.56; | H, 4.25; |
| Found: | N, 13.35; | C, 68.53; | H, 4.23. |

EXAMPLE 117

**1,3-Dihydro-3-(RS)-(2-L-hydroxy-2-phenylacetyl)amino-5-(2-fluorophenyl)-2H-1,4-benzodiazepin-2-one**

1,3-Dihydro-3-(RS)-amino-5-(2-fluorophenyl)-2H-1,4-benzodiazepin-2-one (100 mg, 0.37 mmol) and L-mandelic acid (63 mg, 0.41 mmol) were combined at room temperature in 10 ml of methylene chloride. To this reaction mixture was added 1-ethyl-3-(3-dimethylaminopropyl)carbodiimide hydrochloride (79 mg, 0.41 mmol). The pH of the reaction mixture was then adjusted to 8.5 with triethylamine and stirring was continued at room temperature for 96 hours. The reaction mixture was partitioned between methylene chloride and 10% citric acid solution. The phases were separated and the organic layer was washed in succession with 10% citric acid solution (1 x 30 ml), saturated sodium bicarbonate solution (2 x 30 ml) and brine. The dried ($MgSO_4$) extracts were concentrated to yield 130 mg

of crude product as a mixture of diastereomers. Preparative thick layer chromatography (chloroform − methanol − ammonia, 95:5:0.5, v/v) afforded the analytical sample.
NMR ($CDCl_3$): consistent with the title structure.

EXAMPLE 118

1,3−Dihydro−3−(RS)−(5−Chloroindole−2−carbonylamino)−5−(2−fluorophenyl)−2H−1,4−benzodiazepin−2−one

3−(RS)−Amino−1,3−dihydro−5−(2−fluorophenyl)−2H−1,4−benzodiazepin−2−one (100 mg, 0.391 mmol) was suspended in 2 ml of methylene chloride. 5−Chloroindole−2−carboxylic acid chloride (86.7 mg, 0.438 mmol) was added. The pH of the stirred mixture was adjusted to 9 with triethylamine (95 µl). The reaction mixture was stirred for 24 hours. The mixture was then diluted with 1 ml of methanol and filtered. The filtrate was pipeted onto a 2000 µ Analtech preparative TLC plate which was developed in a 95:5:0.5 chloroform, methanol, water (CMW) solvent system. The product band was collected. The silica was washed with 90:10:1 CMW. The filtrate was evaporated and the residue was dissolved in methanol and placed in a small vial. The solvent was evaporated to yield 16.4 mg of purified product.
HPLC: 90% pure.
MS (14 ev): ($M^+$) m/e 446.
NMR: Consistent with title product.

| Anal. calc'd for $C_{24}H_{16}Cl_1FN_4O_2$ .0.8$CH_3OH$ | | | |
|---|---|---|---|
| | C, 63.04; | H, 4.09; | N, 11.86; |
| Found: | C, 63.03; | H, 3.66; | N, 11.58. |

EXAMPLE 119

3−(RS)−[N−(2−indolecarbonyl)−N−methylamino]−1,3−dihydro−5−phenyl−2H−1,4−benzodiazepin−2−one

1,3−Dihydro−3−(RS)−methylamino−5−phenyl−2H−1,4−benzodiazepin−2−one (130 mg, 0.49 mmol) and indole−2−carbonyl chloride (88 mg, 0.49 mmol) were combined in $CH_2Cl_2$ (5 ml) and stirred 2 hours at 25°C. The reaction was concentrated and chromatographed on silica gel (3% MeOH in $CH_2Cl_2$) to give the title compound as a white solid from $CH_2Cl_2$: (m.p. 287−288.5°).
TLC: Silca GF (5% MeOH in $CH_2Cl_2$), $R_f$ = 0.41, single homogeneous component.
NMR: Consistent with title structure and verified the presence of 0.25 $H_2O$.
HPLC: Greater than 97.2% pure.
MS: Mol. ion = 408 m/e (free base).

| Anal. calc'd for $C_{25}H_{20}N_4O_2$.0.25$H_2O$ | | | |
|---|---|---|---|
| | C, 72.70; | H, 5.00; | N, 13.57; |
| Found: | C, 72.64; | H, 4.87; | N, 13.30. |

EXAMPLE 120

1,3−Dihydro−3−(RS)−(5−Bromoindole−2−carbonylamino)−5−(2−fluorophenyl)−2H−1,4−benzodiazepin−2−one

The procedure of Example 114 was carried out using 5−bromoindole−2−carboxylic acid chloride (113 mg, 0.438 mmole) in place of 5−fluoroindole−2−carboxylic acid chloride.
HPLC: 82% pure.
MS: $M^+$ (14 ev) , m/e 490.
NMR: Consistent with title product.

| Anal. calc'd for $C_{24}H_{16}BrFN_4O_2$ .0.28$CHCl_3$ | | | |
|---|---|---|---|
| | N, 10.68; | C, 55.57; | H, 3.13; |
| Found: | N, 10.31; | C, 55.98; | H, 3.36. |

EXAMPLE 121

3-(RS)-Cinnamoylamino-1,3-dihydro-5-(2-fluorophenyl)-2H-1,4-benzodiazepin-2-one

3-(RS)-Amino-1,3-dihydro-5-(2'-fluorophenyl)-2H-1,4-benzodiazepin-2-one (50 mg, 0.186 mmol) was suspended in methylene chloride (1 ml). Cinnamoyl chloride (34.5 mg, 0.207mmol) was added to the methylene chloride mixture. The pH of the stirred mixture was adjusted to 9 with 50 µl of triethylamine. After stirring for 16 hours the mixture was filtered. The product in the filtrate was purified by prep TLC. The product band was collected by washing the silica containing the product, with 80:20:2 CMW. The solvent was evaporated and the residue was dissolved in methanol, placed in a small vial and evaporated. Yield 16.6 mg.
HPLC: 97% pure.
MS: $M^+$ (14 ev) m/e 399
NMR: Consistent with title structure.

| Anal. calc'd for $C_{24}H_{18}FN_3O_2$ .0.126$CHCl_3$ | | | |
|---|---|---|---|
| | N, 10.18; | C, 70.24; | H, 4.42; |
| Found: | N, 10.08; | C, 70.07; | H, 4.46. |

EXAMPLE 122

1,3-Dihydro-3-(RS)-(5-hydroxy-2-indolylcarbonyl)amino-5-(2-fluorophenyl)-2H-1,4-benzodiazepin-2-one

1,3-Dihydro-3-(RS)-amino-5-(2-fluorophenyl)-2H-1,4-benzodiazepin-2-one (100 mg, 0.37 mmole) and 5-hydroxyindole-2-carboxylic acid (75 mg, 0.44 mmole) were combined at room temperature in a mixture of 1 ml of dimethylformamide and 5 ml of methylene chloride. To this reaction mixture was added 1-ethyl-3-(3-dimethylaminopropyl)carbodiimide hydrochloride (76 mg, 0.40 mmol). The pH of the reaction mixture was then adjusted to 8.5 with triethylamine and stirring was continued at room temperature for 48 hours. The solvent was removed under reduced pressure and the residue was partitioned between ethyl acetate and 10% citric acid solution. The phases were separated and the organic layer was washed in succession with 20% citric acid solution (1 x 30 ml), saturated sodium bicarbonate solution (2 x 30 ml) and brine. The dried ($MgSO_4$) extracts were concentrated to yield 200 mg of the product. Preparative thick layer chromatography (chloroform - ethanol - ammonia, 90:10:1, v/v) afforded the analytical sample (80 mg).
NMR ($CD_3OD$): Consistent with the title structure.
MS (14 ev): 428 ($M^+$), 227, 176, 159.

| Anal. calc'd. for $C_{24}H_{17}FN_4O_3$.0.25 $CHCl_3$ | | | |
|---|---|---|---|
| | N, 12.23; | C, 63.56; | H, 3.79; |
| Found: | N, 12.09; | C, 63.99; | H, 4.09. |

EXAMPLE 123

1-Carboxamidomethyl-1,3-dihydro-3R-(3-indolylmethyl)-5-(2-fluorophenyl)-2H-1,4-benzodiazepin-2-one

1,3-Dihydro-3R-(3-indolylmethyl)-(2-fluorophenyl)-2H-1,4-benzodiazepin-2-one (10 g, 26 mmol) was stirred in 120 ml of degassed DMF at 0°C under nitrogen with sodium hydride (1.25 g, 26 mmol) until homogeneous ( 1 hour). Ethylbromoacetate (2.88 ml, 26 mmol) was added and the reaction mixture was stirred at room temperature for 1 hour. The reaction was quenched in 1 l of water. The aqueous solution was extracted with 3 x 250 ml of methylene chloride. The methylene chloride solution was washed with 250 ml water. The organic phase was separated, dried over sodium sulfate and concentrated in vacuo.

A portion of the crude ester (530 mg) was dissolved in 50 ml of methanol. The solution was stirred in a pressure bottle and saturated with ammonia at 0°C. The bottle was sealed and the solution was stirred at room temperature for 48 hours. The solution was concentrated in vacuo. This gave a solid which was purified by flash chromatogrphy in a 97:3 chloroform/methanol solvent system to 245 mg of purified product.

HPLC: 99% pure.
MS: $M^+$ (14 ev) m/e 440
NMR: Consistent with title structure.

| Anal. calc'd for $C_{26}H_{21}FN_4O_2$ .0.53$H_2O$ | | | |
|---|---|---|---|
| | N, 12.45; | C, 69.39; | H, 4.82; |
| Found: | N, 12.27; | C, 69.32; | H, 4.80. |

EXAMPLE 124

1,3-Dihydro-5-(2-fluorophenyl)-3-(RS)-(2-indolylmethylamino)-2H-1,4-benzodiazepin-2-one

3-(RS)-Chloro-1,3-dihydro-5-(2-fluorophenyl)-2H-1,4-benzodiazepin-2-one (150 mg, 0.520 mmol) and 2-aminomethylindole (75.9 mg, 0.520 mmol) were combined in 1,2-dimethoxyethane (3 ml) and the mixture stirred 20 min. at 25°C. The mixture was evaporated to dryness in vacuo and the residue treated with $H_2O$ and extracted with EtOAc (3x). The combined extracts were washed with $H_2O$ (1X), dried over $MgSO_4$, filtered and stripped to dryness in vacuo to give an orange oil which, after chromatography on silica gel (4% MeOH in $CH_2Cl_2$) provided the title compound as a white solid from ether: (m.p. 200-202°).

TLC: Silica GF (5% MeOH in $CH_2Cl_2$), $R_f$ = 0.37, single homogeneous component.
NMR: Consistent with title structure.
HPLC: Greater than 97.7% pure.
MS: Molecular ion at m/e=398.

| Anal. calc'd for $C_{24}H_{19}FN_4O$: | | | |
|---|---|---|---|
| | C, 72.35; | H, 4.81; | N, 14.06; |
| Found: | C, 72.48; | H, 4.81; | N, 13.69. |

EXAMPLE 125

1,3-Dihydro-3-(RS)-(phenylaminomethylcarbonyl)amino-5-(2-fluorophenyl)-2H-1,4-benzodiazepin-2-one

1,3-Dihydro-3-(RS)-amino-5-(2-fluorophenyl)-2H-1,4-benzodiazepin-2-one (100 mg, 0.37 mmol) and N-phenyl glycine (64 mg, 0.42 mmol) were combined at room temperature in 5 ml of methlylene chloride. To this reaction mixture was added 1-ethyl-3-(3-dimethylaminopropyl)-carbodiimide hydrochloride (81 mg, 0.42 mmole). The pH of the reaction mixture was then adjusted to 8.5

with triethylamine and stirring was continued at room temperature overnight. More N-phenylglycine and carbodiimide reagent were added (0.2 equivalents) and stirring was continued. The reaction mixture was partitioned between methylene chloride and 10% citric acid solution after 48 hours reaction time. The phases were separated and the organic layer was washed in succession with 20% citric acid solution (1 x 30 ml), saturated sodium bicarbonate solution (2 x 30 ml) and brine. The dried ($MgSO_4$) extracts were concentrated to yield 200 mg of crude product. Preparative thick layer chromatography (chloroform - ethanol - ammonia 92:8:0.8 v/v) afforded the analytical sample (100 mg), m.p. 145-146°.
NMR ($CDCl_3$): Consistent with the title structure.
MS (14 ev): 402 ($M^+$), 265.

| Anal. calc'd for $C_{23}H_{19}FN_4O_2.0.55\ CHCl_3$ | | | |
|---|---|---|---|
| | N, 11.97; | C, 60.43; | H, 4.21; |
| Found: | N, 11.80; | C, 60.37; | H, 4.06. |

EXAMPLE 126

1,3-Dihydro-3-(RS)-(5-methoxyindole-2-carbonylamino)-5-(2-fluorophenyl)-2H-1,4-benzodiazepin-2-one

3-(RS)-Amino-1,3-dihydro-5-(2-fluorophenyl)-2H-1,4-benzodiazepin-2-one (50 mg, 0.186 mmol) was suspended in 1 ml of methylene chloride. 5-Methoxyindole-2-carboxylic acid (36.9 mg, 0.207 mmol) was added to the suspension followed by the addition of 38.5 mg (0.2 mmol) of EDC. The mixture was brought to pH ~ 8 with ~ 60 µl of triethylamine. The solid which formed after 3 min. was filtered after 5 hours and washed with chloroform. The filtrate was applied to a 2000 µ preparative TLC plate and eluted with 90:10:1 chloroform:methanol:water (CMW). The product was extracted from silica with methanol and evaporated.
HPLC: 98% pure.
MS: $M^+$ (14 ev) m/e 442
NMR: Consistent with title structure.

| Anal. calc'd for $C_{25}H_{19}FN_4O_3\ .0.1CHCl_3$ | | | |
|---|---|---|---|
| | N, 12.33; | C, 66.34; | H, 4.24; |
| Found: | N, 10.59; | C, 66.19; | H, 4.23. |

EXAMPLE 127

1,3-Dihydro-3-(RS)-(1-methylindole-2-carbonylamino)-5-(2-fluorophenyl)-2H-1,4-benzodiazepine-2-one

3-(RS)-Amino-1,3-dihydro-5-(2-fluorophenyl)-2H-1,4-benzodiazepin-2-one (50 mg, 0.186 mmol) was suspended in 1 ml of methylene chloride. 1-Methylindole-2-carboxylic acid (36.2 mg, 0.2 mmol) was added to the solution followed by the addition of 38.5 mg (0.2 mmol) of EDC. The pH of the solution was brought to ~8 with ~60 µl of triethylamine. After stirring for 4 hours the product was purified by preparative TLC on a 2000 µ silica gel plate with a 95:5:0.5 chloroform/methanol/water solvent system. The product band was collected and isolated by washing the silica with 90:10:1 CMW. yield 16.5 mg.
HPLC: 99% pure
MS: $M^+$ (14 ev) m/e 426
NMR: Consistent with title structure.

| Analysis calc'd for $C_{25}H_{19}FN_4O_2$ 0.8$CH_3OH$ | | | |
|---|---|---|---|
| | N, 12.39; | C, 68.54; | H, 4.95; |
| Found: | N, 12.34; | C, 68.29; | H, 4.18. |

EXAMPLE 128

1,3-Dihydro-5-(2-fluorophenyl)-3-(RS)-(2-benzofurancarbonylamino)-2H-1,4-benzodiazepin-2-one

3-(RS)-Amino-1,3-dihydro-5-(2-fluorophenyl)-2H-1,4-benzodiazepin-2-one (80 mg, 0.297 mmol), benzofuran-2-carboxylic acid (48 mg, 0.297 mmol), and EDC (56.9 mg, 0.297 mmol) were combined in $CH_2Cl_2$ (3 ml) and the pH adjusted to 9.5 with triethylamine (41 µl, 0.297 mmol). After stirring 30 minutes at 25°C, the reaction was concentrated and chromatographed on silica gel (3% MeOH in $CH_2Cl_2$) to give the title compound as a white solid from $CH_2Cl_2/Et_2O$:
(m.p. 289-291°).
TLC: Silica GF (5% MeOH in $CH_2Cl_2$), $R_f$ = 0.48, single homogeneous component.
NMR: Consistent with title structure and verified the presence of 0.15 $CH_2Cl_2$ and 0.1 $(C_2H_5)_2O$.
HPLC: Greater than 99.7% pure.
M.S.: Mol. ion = 413 m/e (free base).

| Anal. Calc'd for $C_{25}H_{16}FN_3O_3$.0.15 $CH_2Cl_2$.0.10 $(C_2H_5)_2O$: | | | |
|---|---|---|---|
| Calc'd: | C, 68.01; | H, 4.02; | N, 9.69; |
| Found: | C, 68.22; | H, 3.86; | N, 9.36. |

EXAMPLE 129

1-Ethoxycarbonylmethyl-1,3-dihydro-3(RS)-(4-chlorophenylcarbonyl)amino-5-(2-fluorophenyl)-2H-1,4-benzodiazepin-2-one

To a suspension of sodium hydride (50%) (24.4 mg, 0.51 mmole) in 2 ml of dry dimethylformamide at 0°C was added, under nitrogen, 1,3-dihydro-3(RS)-(4-chlorophenylcarbonyl)amino-5-(2-fluorophenyl)-2H-1,4-benzodiazepin-2-one (197.3 mg, 0.48 mmole). The resulting reaction mixture became homogeneous over a one-hour period, was stirred one hour more at 0°C and then treated with ethylbromoacetate (55 µl, 0.50 mmole). The reaction mixture was warmed to room temperature and after one hour was quenched with brine. The aqueous mixture was extracted with ethyl acetate and the combined organic extracts were washed with brine. Rotoevaporation of the dried extracts ($MgSO_4$) gave a semi-solid which was chromatographed on silica gel (chloroform-methanol-ammonia 95:5:0.5 v/v elution) to afford 64 mg of the analytical sample. mp 172° (soften), 177-178°C.
NMR ($CDCl_3$): Consistent with the title structure.
MS (14 ev): 493 ($M^+$), 364, 354, 338, 327, 313

| Analysis calc'd for $C_{26}H_{21}ClFN_3O_4$.0.1 $C_4H_8O_2$ | | | |
|---|---|---|---|
| | N, 8.35; | C, 63.05; | H, 4.32; |
| Found: | N, 8.16; | C, 62.89; | H, 4.44. |

EXAMPLE 130

1,3-Dihydro-3-(RS)-(4-chlorophenylcarbonyl)amino-5-phenyl-2H-1,4-benzodiazepin-2-one

1,3-Dihydro-3-(RS)-amino-5-phenyl-2H-1,4-benzodiazepin-2-one (500 mg, 1.98 mmole) and p-chlorobenzoyl chloride (255 µl, 2.00 mmole) were combined at room temperature in 30 ml of

methylene chloride. The resulting solution was protected from moisture and stirred at room temperature overnight. The reaction mixture was diluted with 70 ml of methylene chloride and washed with sodium bicarbonate solution (sat.) and brine. The organic extracts were dried ($MgSO_4$) and concentrated to give the crude product. Trituration with ether afforded the analytical sample as a white solid.
NMR ($CDCl_3$): Consistent with the title structure.
MS (14 ev): 389 ($M^+$), 250, 234.

| Analysis calc'd for: $C_{22}H_{16}ClN_3O_2$ | | | |
|---|---|---|---|
| | N, 10.78; | C, 67.78; | H, 4.13; |
| Found: | N, 10.71; | C, 67.79; | H, 3.97. |

EXAMPLE 131

1,3-Dihydro-1-methyl-3-(RS)-(4-chlorophenylcarbonyl)amino-5-phenyl-2H-1,4-benzodiazepin-2-one

To a suspension of sodium hydride (50%) (10 mg, 0.21 mmole) in 1 ml of dry dimethylformamide at 0°C was added, under a nitrogen, 1,3-dihydro-3-(RS)-(4-chlorophenylcarbonyl)amino-5-phenyl-2H-1,4-benzodiazepin-2-one (65.5 mg, 0.166 mmole). The resulting reaction mixture became homogeneous over a one-hour period, was stirred one hour more at 0°C and then treated with iodomethane (10.8 µl, 0.17 mmole). The reaction mixture was warmed to room temperature and after one hour was quenched with brine. The aqueous mixture was extracted with ethyl acetate and the combined organic extracts were washed with brine. Rotoevaporation of the dried extracts ($MgSO_4$) gave a semi-solid which was chromatographed on silica gel (chloroform-methanol-ammonia 95:5:0.5 v/v elution) to give the analytical sample.
NMR ($CDCl_3$): Consistent with the title structure;
MS (14 ev): 403 ($M^+$)

| Analysis calc'd for: $C_{23}H_{18}ClN_3O_2$: | | | |
|---|---|---|---|
| | N, 10.40; | C, 68.40: | H, 4.49: |
| Found: | N, 10.11; | C, 68.50; | H, 4.57. |

EXAMPLE 132

1-Carboxymethyl-1,3-dihydro-3-(RS)-(4-chlorophenylcarbonyl)amino-5-(2-fluorophenyl)-2H-1,4-benzodiazepin-2-one

To a suspension of sodium hydride (50%) (14.0 mg, 0.30 mmole) in 2 ml of dry dimethylformamide at 0°C was added, under nitrogen, 1,3-dihydro-3-(RS)-(4-chlorophenylcarbonyl)amino-5-(2-fluoropheny1)-2H-1,4-benzodiazepin-2-one (103.0 mg, 0.25 mmole). The resulting reaction mixture became homogeneous over a one-hour period, was stirred one hour more at 0°C and then treated with 1 ml of dimethylformamide containing sodium iodoacetate (56 mg) (0.27 mmole). The reaction mixture was warmed to room temperature and after 12 hours was quenched with brine. The aqueous mixture was extracted with ethyl acetate and the combined organic extracts were washed with brine. Rotoevaporation of the dried extracts ($MgSO_4$) gave a semi-solid which was chromatographed on silica gel (chloroform-methanol-acetic acid, 93:6:1 v/v) to provide the analytical sample: (m.p. 225-228°C, from methanol).
FABMS: m/e = 466 (M + H), 245, 177.
NMR (DMSO-$d_6$): consistent with title structure.

| Anal. Calc'd for $C_{24}H_{17}ClFN_3O_4$ 0.45NaI 0.75 $H_2O$ | | | |
|---|---|---|---|
| | C, 52.71; | H, 3.41; | N, 7.68. |
| Found: | C, 52.87: | H, 3.64; | N, 7.43. |

EXAMPLE 133

1,3-Dihydro-3-(RS)-(2-indolinecarbonylamino)-5-phenyl-2 H-1,4-benzodiazepin-2-one

3-(RS)-Amino-1,3-dihydro-5-phenyl-2H-1,4-benzodiazepin-2-one (100 mg, 0.398 mmol), 1-indoline-2-carboxylic acid (64.9 mg, 0.398 mmol), 1-hydroxybenzotriazole hydrate (HBT, 53.8 mg, 0.398 mmol), and 1-ethyl-3-(3-dimethylaminopropyl)carbodiimide hydrochloride (EDC, 76.3 mg, 0.398 mmol) were combined in DMF (2 ml) and the pH of the solution was adjusted to 9.0-9.5 with triethylamine (TEA, 95 µl, 0.683 mmol). After stirring 15 minutes at 25°C, the DMF was removed in vacuo, the residue treated with $H_2O$ and extracted with EtOAc (3x). The combined organic extracts were washed with brine, dried over $MgSO_4$, filtered and stripped to dryness in vacuo to give a white solid (180 mg). Flash chromatography on silica gel (267/10/1 of $CH_2Cl_2$/MeOH/concentrated $NH_4OH$) gave a white solid (38 mg) from EtOAc/hexane. The product is a single stereoisomer whose absolute configuration is unknown; m.p. 252-272°C (slowly shrinkes to a cloudy melt).
TLC: Silica GF (190/10/1 of $CH_2Cl_2$/MeOH/concentrated $NH_4OH$), $R_f$ = 0.40, single, clean component.
NMR: Consistent with title structure and verifies the presence of EtOAc.
HPLC: Greater than >96% pure.
MS: Molecular ion at m/e = 396.

| Anal. calc'd for $C_{24}H_{20}N_4O_2$ .0.45$C_4H_8O_2$ | | | |
|---|---|---|---|
| | C, 71.06; | H, 5.46; | N, 12.85; |
| Found: | C, 70.71; | H, 5.11; | N, 13.20. |

EXAMPLE 134

1,3-Dihydro-5-(2-fluorophenyl)-3-(RS)-(p-trifluoromethylbenzoylamino)-2H-1,4-benzodiazepin-2-one

1,3-Dihydro-3-(RS)-amino-5-(2-fluorophenyl)-2H-1,4-benzodiazepin-2-one (42 mg, 0.156 mmole) and p-trifluoromethylbenzoyl chloride (32.5 mg, 0.156 mmole) were combined in 3 ml of methylene chloride ($CH_2Cl_2$), treated with triethylamine (0.0157 g, 0.156 mmole) and stirred at room temperature 15 minutes. The mixture was diluted with $CH_2Cl_2$ (20 ml), washed with 10% citric acid (2 x 5 ml), dilute sodium bicarbonate (2 x 5 ml), and water (2 x 5 ml), dried over sodium sulfate, filtered, and evaporated to dryness in vacuo. The residue was crystallized from ethyl acetate (0.4 ml)/ether (1 ml) to give the title compound which was dried in vacuo at 90°: (m.p. 209-211°).
TLC: Single spot, $R_f$ = 0.62, silica gel plate, 90:10:1:1 (v:v:v:v) $CH_2Cl_2$:MeOH:HOAc:$H_2O$.
NMR: The spectrum was consistent with the title structure and verified the presence of EtOAc.
HPLC: Greater than 98% pure.
MS: Molecular ion at m/e = 441.

| Anal. calc'd for $C_{23}H_{15}F_4N_3O_2$. 0.2EtOAc: | | | |
|---|---|---|---|
| | C, 62.27; | H, 3.64; | N, 9.16; |
| Found: | C, 62.25; | H, 3.61; | N, 9.11. |

EXAMPLE 135

**1,3-Dihydro-5-(2-fluorophenyl)-3-(RS)-(p-methylbenzoylamino)-2H-1,4-benzodiazepin-2-one**

The procedure of Example 134 was carried out using p-methylbenzoyl chloride (24 mg, 0.156 mmole) in place of p-trifluoromethylbenzoyl chloride. The title compound was crystallized from $CH_2Cl_2$ (3 ml)/$Et_2O$ (1 ml) and dried in vacuo at 90°: (m.p. 275-276° (d)).
TLC: Single spot, $R_f$ = 0.62, silica gel plate, 90:10:1:1 (v:v:v:v) $CH_2Cl_2$:MeOH:HOAc:$H_2O$.
NMR: The spectrum was consistent with the title structure.
HPLC: Greater than 98% pure.
MS: Molecular ion at m/e = 387.

| Anal. calc'd for $C_{23}H_{18}FN_3O_2$. 0.4$H_2O$: | | | |
|---|---|---|---|
| | C, 70.00; | H, 4.80; | N, 10.65; |
| Found: | C, 70.04; | H, 4.68; | N, 10.56. |

EXAMPLE 136

**1,3-Dihydro-5-(2-fluorophenyl)-3-(RS)-(p-methoxybenzoylamino)-2H-1,4-benzodiazepin-2-one**

The procedure of Example 134 was carried out using p-methoxybenzoyl chloride (26.6 mg, 0.156 mmole) in place of p-trifluoromethylbenzoyl chloride. The title compound was crystallized from $CH_2Cl_2$ (2 ml)/$Et_2O$ (1 ml) and dried in vacuo at 90°: (m.p. 231-233°).
TLC: Single spot, $R_f$ = 0.47, silica gel plate, 5% (v/v) MeOH/$CH_2Cl_2$.
NMR: The spectrum was consistent with the title structure.
HPLC: Greater than 97% pure.
MS: Molecular ion at m/e = 403.

| Anal. calc'd for $C_{23}H_{18}FN_3O_3$: | | | |
|---|---|---|---|
| | C, 68.48; | H, 4.50; | N, 10.42; |
| Found: | C, 68.62; | H, 4.60; | N, 10.36. |

EXAMPLE 137

**3-(RS)-(o-Chlorobenzoylamino)-1,3-dihydro-5-phenyl-2H-1,4-benzodiazepin-2-one**

3-(RS)-Amino-1,3-dihydro-5-phenyl-2H-1,4-benzodiazepine-2-one (250 mg, 0.93 mmol) was suspended in methylene chloride (10 ml) and treated with o-chlorobenzoylchloride (0.124 ml, 0.97 mmol) followed by triethylamine (0.143 ml, 0.97 mmol). The solution was stirred at room temperature overnight. The reaction solution was chromatographed on silica gel (chloroform followed by 97/3 chloroform/methanol) and the combined product fractions were evaporated to dryness in vacuo. TLC: Silica

gel (90:10:1, $CHCl_3$:$CH_3OH$:$H_2O$), $R_f$ = 0.85.
NMR: Consistent with structure.
HPLC: 99% pure.
MS: Molecular ion at m/e = 389.

| Anal. calc'd for $C_{22}H_{16}ClN_3O_2$: | | | |
|---|---|---|---|
| | C, 67.78; | H, 4.14; | N, 10.77; |
| Found: | C, 67.34; | H, 4.00; | N, 10.72. |

EXAMPLE 138

## 3-(RS)-(o-Chlorobenzoylmethylamino-1,3-dihydro-1-methyl-5-phenyl-2H-1,4-benzodiazepin-2-one

3-(RS)-1,3-Dihydro-(o-Chlorobenzoylamino)-5-phenyl-2H-1,4-benzodiazepin-2-one (200 mg, 0.51 mmol) and sodium hydride (52 mg of a 50% suspension in mineral oil, 1.094 mmol) were stirred in 2 ml of dry, degassed dimethylformamide under nitrogen in an ice bath. The mixture was stirred until homogeneous. After 2 hours, methyl iodide (38 µl, 1.094 mmol) was added in one portion. The reaction was stirred for 1 hour at 0°C and 1 hour at room temperature. The reaction was quenched with 3 ml of saturated sodium chloride solution. The mixture was extracted with ethyl acetate. The clear solution obtained when chloroform was added was evaporated to dryness then chromatographed on silica gel with chloroform as the elution solvent. The 7:1 mixture of the di and mono substituted compounds was further purified by preparative TLC. (Analtech silica gel 2000 µ prep TLC plates developed twice in a 98:2 chloroform/methanol solvent system).
TLC: Silica gel 97:2 $CHCl_3$:MeOH, $R_f$ = 0.35.
NMR: Consistent with structure.
MS: Molecular ion m/e = 417
HPLC: 98%.

| Anal. calc'd for $C_{24}H_{20}ClN_3O_2$ 0.35$CHCl_3$: | | | |
|---|---|---|---|
| | C, 63.62; | H, 4.46; | N, 9.14; |
| Found: | C, 63.40; | H, 4.55; | N, 8.97. |

EXAMPLE 139

## 3-(RS)-(o-Chlorobenzoylamino)-1,3-dihydro-1-methyl-5-phenyl-2H-1,4-benzodiazepin-2-one

3-(RS)-1,3-Dihydro-(o-Chlorobenzoylamino)-5-phenyl-2H-1,4-benzodiazepin-2-one (207 mg, 0.53 mmol) and sodium hydride (26 mg of a 50% suspension in mineral oil, 0.54 mmol) were stirred in 2 ml of dry, degassed dimethylformamide under nitrogen in an ice bath. The mixture was stirred until homogenous. After 2 hours, methyl iodide (34 µl, 0.547 mmol) was added in one portion. (The remainder of the experiment proceeds as described in Example 139).
NMR: Consistent with structure.
HPLC: 98%.
MS: Molecular ion m/e 403.

| Anal. calc'd for $C_{23}H_{18}ClN_3O_2$ 0.62$H_2O$ | | | |
|---|---|---|---|
| | C, 66.56; | H, 4.67; | N, 10.12; |
| Found: | C, 66.71; | H, 4.53; | N, 9.90. |

EXAMPLE 140

**3-(RS)-(m-Chlorobenzoylamino)-1,3-dihydro-5-phenyl-2H-1,4-benzodiazepin-2-one**

The procedure of Example 137 was carried out using m-chlorobenzoyl chloride in place of o-chlorobenzoylchloride. The reaction was chromatographed using chloroform as the elution solvent.
TLC: Silica gel 90:10:1 CMA; $R_f$ = 0.8.
NMR: Consistent with structure.
HPLC: 96%.
MS: Molecular ion at m/e 389.

| Anal. calc'd for $C_{22}H_{16}N_3O_2$ 0.62$CHCl_3$: | | | |
|---|---|---|---|
| | C, 59.86; | H, 3.69; | N, 9.30; |
| Found: | C, 59.99; | H, 3.75; | N, 9.18. |

EXAMPLE 141

3-(RS)-(3,4-Dichlorobenzoylamino)-1,3-dihydro-5-phenyl-2H-1,4-benzodiazepin-2-one

The EDC procedure in Example 126 was carried out using 3,4-dichlorobenzoic acid in place of 5-methoxyindole-2-carboxylic acid. The reaction product was dissolved in chloroform and chromatographed with chloroform followed by 99:1 $CHCl_3$:MeOH(CM).
TLC: Silica gel 97:3 CM, $R_f$ = 0.45.
HPLC: 100%.
NMR: Consistent with structure.
MS: Molecular ion at m/e 423.

| Anal. calc'd for $C_{22}H_{15}Cl_2N_3O_2$ 0.08$CHCl_3$ | | | |
|---|---|---|---|
| | C, 61.12; | H, 3.50; | N, 9.69; |
| Found: | C, 61.05; | H, 3.50; | N, 9.30. |

EXAMPLE 142

**3-(RS)-(p-Chlorobenzoylamino)1,3-dihydro-5-(2'-fluorophenyl)-1-methyl-4-oxo-2H-1,4-benzodiazepin-2-one**

3-(RS)-(p-Chlorobenzoylamino)1,3-Dihydro-5-(2'-fluorophenyl)-1-methyl-2H-1,4-benzodiazepin-2-one (50 mg, 0.118 mmol) was stirred in 3 ml of chloroform. m-Chloroperoxybenzoic acid (23.6 mg, 0.137 mmol) was added. After stirring overnight another 23.6 mg of MCPBA was added. The solution was stirred for 48 hours then diluted with chloroform and washed with cold saturated sodium

bicarbonate. The chloroform solution was dried over sodium sulfate and evaporated. The residue obtained after evaporation was purified by preparative TLC with 98:2 $CHCl_3$:MeOH (CM) as the developing solvent.
TLC: Silica gel 98:2 CM, $R_f = 0.4$ CM.
NMR: Consistent with structure.
HPLC: 95%.
MS: Molecular ion at m/e = 437.

| Anal. calc'd for $C_{23}H_{17}ClFN_3O_3$ 0.05$CHCl_3$: | | | |
|---|---|---|---|
| | C, 62.37; | H, 3.87; | N, 9.46; |
| Found: | C, 62.41; | H, 3.80; | H, 9.43. |

EXAMPLE 143

1,3 – Dihydro – 5 – Phenyl – 3 – (RS) – (4' – methylthiobenzoylamino) – 2H – 1,4 – benzodiazepin – 2 – one

The EDC procedure in Example 126 was carried out using 4 – methyl thiobenzoic acid in place of 5 – methoxyindole – 2 – carboxylic acid. The reaction solution was chromatographed on a silica gel column with chloroform followed by 99:1 $CHCl_3$:MeOH (CM).
TLC: Silica gel 97:3 CM, $R_f = 0.3$
NMR: Consistent with structure.
HPLC: 97%.
MS: Molecular ion at m/e 401.

| Anal. calc'd for $C_{23}H_{19}N_3O_2S$ 0.65$CHCl_3$: | | | |
|---|---|---|---|
| | C, 59.28; | H, 4.13; | N, 8.77; |
| Found: | C, 59.33; | H, 4.21; | N, 8.57. |

EXAMPLE 144

1 – 3 – Dihydro – 3 – (RS) – (4' – Fluorobenzoylamino) – 5 – phenyl – 2H – 1,4 – benzodiazepin – 2 – one

The procedure of Example 137 was carried out using 4 – fluorobenzoyl chloride in place of o – chlorobenzoyl chloride. The reaction was chromatographed on silica gel using chloroform as the elution solvent.
TLC: Silica gel 97:3 $CHCl_3$:MeOH (CM), $R_f = 0.33$.
NMR: Consistent with structure.
HPLC: 95%.
MS: Molecular ion at m/e 373.

| Anal. calc'd for $C_{22}H_{16}FN_3O_2$ 0.2$H_2O$: | | | |
|---|---|---|---|
| | C, 70.09; | H, 4.39; | N, 11.15; |
| Found: | C, 70.14; | H, 4.36; | N, 10.93. |

EXAMPLE 145

1,3 – Dihydro – 5 – Phenyl – 3 – (RS) – (4' – trifluoromethylbenzoylamino) – 2H – 1,4 – benzodiazepin – 2 – one

The procedure of Example 137 was carried out using 4 – trifluoromethylbenzoyl chloride in place of o – chlorobenzoyl chloride. The reaction was chromatographed on silica gel using chloroform as the elution solvent.
TLC: Silica gel 97:3 $CHCl_3$:MeOH (CM), $R_f = 0.3$.

NMR: Consistent with structure.
HPLC: 99%.
MS: Molecular ion at m/e 423.

| Anal. calc'd for $C_{23}H_{16}F_3N_3O_2$: | | | |
|---|---|---|---|
| | C, 65.24; | H, 3.81; | N, 9.92; |
| Found: | C, 65.14; | H, 3.94; | N, 9.69. |

EXAMPLE 146

1,3-Dihydro-3-(RS)-(4'-tert-Butylbenzoylamino)-5-phenyl-2H-1,4-benzodiazepin-2-one

The procedure of Example 137 was carried out using 4-tert-butylbenzoyl chloride in place of o-chlorobenzoyl chloride. The reaction was chromatographed on silica gel using chloroform as the elution solvent.
TLC: Silica 97:3, $CHCl_3$:MeOH, $R_f = 0.35$.
NMR: Consistent with structure.
HPLC: 98%.
MS: Molecular ion at m/e 411.

| Anal. calc'd for $C_{26}H_{25}N_3O_2$ 0.14$CHCl_3$: | | | |
|---|---|---|---|
| | C, 73.31; | H, 5.92; | N, 9.81; |
| Found: | C, 73.69; | H, 6.07; | N, 9.78. |

EXAMPLE 147

3-(RS)-(3,5-Dichlorobenzoylamino)1,3-dihydro-5-phenyl-2H-1,4-benzodiazepin-2-one

The EDC procedure in Example 126 was carried out using 3,5-dichlorobenzoic acid in place of 5-methoxyindole-2-carboxylic acid. The reaction was diluted with chloroform and chromatographed on a silica gel column with chloroform as the elution solvent.
TLC: Silica gel 97:3 $CHCl_3$:MeOH (CM), $R_f = 0.5$
NMR: Consistent with structure.
HPLC: 96%.
MS: Molecular ion at m/e 423.

| Anal. calc'd for $C_{22}H_{15}Cl_2N_3O_2$: | | | |
|---|---|---|---|
| | C, 62.27; | H, 3.56; | N, 9.90; |
| Found: | C, 62.65; | H, 3.67; | N, 9.80. |

EXAMPLE 148

1-3-Dihydro-3-(RS)-(p-Hydroxybenzoylamino)-5-phenyl-2H-1,4-benzodiazepin-2-one

The EDC procedure in Example 126 was carried out using p-hydroxybenzoic acid in place of 5-methoxyindole-2-carboxylic acid. The reaction was chromatographed on silica gel with chloroform as the elution solvent.

TLC: Silica gel 97:3 $CHCl_3$:MeOH, $R_f = 0.50$.
NMR: Consistent with structure.
HPLC: 99%.
MS: Molecular ion at 371.

| Anal. calc'd for $C_{22}H_{17}N_3O_3$: | | | |
|---|---|---|---|
| | C, 71.15; | H, 4.61; | N, 11.31; |
| Found: | C, 70.05; | H, 4.63; | H, 11.21. |

EXAMPLE 149

3-(RS)-(4'-Cyanobenzoylamino)1,3-dihydro-5-phenyl-2H-1,4-benzodiazepin-2-one

The procedure in Example 137 was carried out using 4-cyanobenzoyl chloride in place of o-chlorobenzoyl chloride. The reaction was chromatographed on silica gel using chloroform followed by 98:2 $CHCl_3$:MeOH (CM) as the elution solvents.
TLC: Silica gel 97:3 CM, $R_f = 0.3$.
NMR: Consistent with structure.
HPLC: 99.6%.
MS: Molecular ion at m/e = 380.

| Anal. calc'd for $C_{23}H_{16}N_4O_2$ $0.41H_2O$ | | | |
|---|---|---|---|
| | C, 71.24; | H, 4.37; | N, 14.45; |
| Found: | C, 71.53; | H, 4.37; | N, 14.73. |

EXAMPLE 150

3(S)-(-)-3-(2-Chlorobenzoylamino)-1,3-dihydro-1-methyl-5-phenyl-2H-1,4-benzodiazepin-2-one

The procedure of Example 134 was carried out using 3(S)-(-)-3-amino-1,3-dihydro-5-phenyl-1-methyl-2H-1,4-benzodiazepin-2-one (41.4 mg, 0.156 mmole) in place of 1,3-dihydro-3(RS)-amino-5-(2-fluorophenyl)-2H-1,4-benzodiazepin-2-one and 2-chlorobenzoylchloride (27.3 mg, 0.156 mmole) in place of p-trifluoromethylbenzoyl chloride. The product was chromatographed on silica gel (5% (v/v) $Et_2O$ in $CH_2Cl_2$ elution). The combined product fractions were evaporated to dryness in vacuo to give the title compound which was dried in vacuo at 78°C: (m.p. 100-118°C).
TLC: Single spot, $R_f = 0.24$, silica gel plate, 5% (v/v) $Et_2O$ in $CH_2Cl_2$.
NMR: Consistent with structure.
HPLC: Greater than 99% pure.
MS: Molecular ion at m/e = 403.
$[\alpha]_D^{25} = -90.4°$ (1.15 mg/ml, $CH_2Cl_2$).

| Anal. calc'd for $C_{23}H_{18}ClN_3O$: | | | |
|---|---|---|---|
| | C, 68.40; | H, 4.49; | N, 10.41; |
| Found: | C, 68.20; | H, 4.73; | N, 10.07. |

EXAMPLE 151

3(R)-(+)-3-(2-Chlorobenzoylamino)-1,3-dihydro-1-methyl-5-phenyl-2H-1,4-benzodiazepin-2-one

The procedure of Example 134 was carried out using 3(R)-(+)-3-amino-1,3-dihydro-5-phenyl-1-methyl-2H-1,4-benzodiazepin-2-one (41.4 mg, 0.156 mmole) in place of 1,3-dihydro-3(RS)-amino-5-(2-fluorophenyl)-2H-1,4-benzodiazepin-2-one, and 2-chlorobenzoyl chloride (27.3 mg, 0.156 mmole) in place of p-trifluoromethylbenzoyl chloride. The product was chromatographed on silica gel (5% (v/v) $Et_2O$ in $CH_2Cl_2$ elution). The combined product fractions were evaporated to dryness in vacuo to give the title compound which was dried in vacuo at 78°C: (m.p. 102-120°C).
TLC: Single spot, $R_f = 0.24$, silica gel plate, 5% (v/v) $Et_2O$ in $CH_2Cl_2$.
NMR: Consistent with structure.
HPLC: Greater than 98% pure.
MS: Molecular ion at m/e = 403.
$[\alpha]_D^{25}$ = +95.4° (1.75 mg/ml, $CH_2Cl_2$).

| Anal. calc'd for $C_{23}H_{18}ClN_3O$: | C, 68.40; | H, 4.49; | N, 10.41; |
|---|---|---|---|
| Found: | C, 68.74; | H, 4.68; | N, 10.16. |

EXAMPLE 152

**1,3-Dihydro-3(RS)-(p-dimethylaminobenzoylamino)-5-(2-fluorophenyl)-2H-1,4-benzodiazepin-2-one**

The procedure of Example 134 was carried out using p-dimethylaminobenzoyl chloride (28.6 mg, 0.156 mmole) in place of p-trifluoromethylbenzoyl chloride. The citric acid and sodium bicarbonate washes were omitted. The title compound was crystallized from $CH_2Cl_2$ (6 ml)/$Et_2O$ (5 ml) and dried in vacuo at 90°: (m.p. 256-258°C).
TLC: Single spot, $R_f = 0.60$, silica gel plate, 90:10:1:1 (v/v/v/v) $CH_2Cl_2$:MeOH:HOAc:$H_2O$.
NMR: The spectrum was consistent with the title structure and verified the presence of $H_2O$.
HPLC: Greater than 98% pure.
MS: Molecular ion at m/e = 416.

| Anal. calc'd for $C_{24}H_{21}FN_4O_2$. 0.15$H_2O$: | C, 68.77; | H, 5.12; | N, 13.37; |
|---|---|---|---|
| Found: | C, 68.73; | H, 5.16; | N, 13.27. |

EXAMPLE 153

1,3-Dihydro-3(RS)-(3,4-dimethoxybenzoylamino)-5-(2-fluorophenyl)-2H-1,4-benzodiazepin-2-one

The procedure of Example 134 was carried out using 3,4-dimethoxybenzoyl chloride (31.3 mg, 0.156 mmole) in place of p-trifluoromethylbenzoyl chloride. The title compound was crystallized from $CH_2Cl_2$ - (1.5 ml)/$Et_2O$ (3 ml) and dried in vacuo at 90°: (m.p. 206-207.5°C).
TLC: Single spot, $R_f = 0.64$, silica gel plate, 90:10:1:1 (v:v:v:v) $CH_2Cl_2$:MeOH:HOAc:$H_2O$.
NMR: The spectrum was consistent with the title structure and verified the presence of $Et_2O$ and $CH_2Cl_2$.
HPLC: Greater than 99% pure.

MS: Molecular ion at m/e = 433.

| Anal. calc'd for $C_{24}H_{20}FN_3O_4 . 0.13C_4H_{10}O. 0.13CH_2Cl_2$: | | | |
|---|---|---|---|
| | C, 65.24; | H, 4.79; | N, 9.26; |
| Found: | C, 65.22; | H, 4.55; | N, 9.14. |

EXAMPLE 154

3(S)-(+)-3-(3-Bromobenzoylamino)-1,3-dihydro-5-(2-fluorophenyl)-1-methyl-2H-1,4-benzodiazepin-2-one

The procedure of Example 134 was carried out using 3(S)-(-)-3-amino-1,3-dihydro-5-(2-fluorophenyl)-1-methyl-2H-1,4-benzodiazepin-2-one (44.2 mg, 0.156 mmole) in place of 1,3-dihydro-3(RS)-amino-5-(2-fluorophenyl)-2H-1,4-benzodiazepin-2-one and 3-bromobenzoyl chloride (34.2 mg, 0.156 mmole) in place of p-trifluoromethylbenzoyl chloride. The title compound was crystallized from $Et_2O$ and dried in vacuo at 100°C: (m.p. 172-178°C).
TLC: Single spot, $R_f$ = 0.66, silica gel plate, 15% (v/v) $Et_2O$ in $CH_2Cl_2$.
NMR: Consistent with structure.
HPLC: Greater than 99% pure.
MS: Molecular ion at m/e = 465.
$[\alpha]_D^{25}$ = +16.7° (0.0025 g/ml, $CH_2Cl_2$).

| Anal. calc'd for $C_{23}H_{17}BrFN_3O_2$: | | | |
|---|---|---|---|
| | C, 59.24; | H, 3.67; | N, 9.01; |
| Found: | C, 59.45; | H, 3.80; | N, 8.97. |

EXAMPLE 155

1,3-Dihydro-5-phenyl-3(RS)-(3-trifluoromethylthiobenzoylamino)-2H-1,4-benzodiazepin-2-one

3(RS)-Amino-1,3-dihydro-5-phenyl-2H-1,4-benzodiazepin-2-one (80.0 mg, 0.318 mmole), 3-trifluoromethylthiobenzoic acid (70.7 mg, 0.318 mmole), HBT (43.0 mg, 0.318 mmole) and EDC (61.0 mg, 0.318 mmole) were combined in dry DMF (2 ml) and stirred at room temperature. The pH of the mixture was adjusted to 9.0-9.5 with triethylamine (64.4 mg, 0.636 mmole) and the mixture stirred for 10 minutes. The DMF was removed in vacuo, and the residue was treated with 10% citric acid and extracted with EtOAc. The combined organic fractions were washed with sodium carbonate solution, dried over $Na_2SO_4$, filtered, and evaporated to dryness in vacuo. The residue was crystallized from EtOAc to give the title compound which was dried in vacuo at 100°C: (m.p. 230-232°C).
TLC: Single spot, $R_f$ = 0.32, silica gel plate, 15% (v/v) $Et_2O$ in $CH_2Cl_2$.
NMR: Consistent with structure.
HPLC: Greater than 98% pure.
MS: Molecular ion at m/e = 455.

| Anal. calc'd for $C_{23}H_{16}F_3N_3O_2S$: | | | |
|---|---|---|---|
| | C, 60.65; | H, 3.54; | N, 9.23; |
| Found: | C, 60.82; | H, 3.51; | N, 9.35. |

EXAMPLE 156

3(S)-(+)-3-(4-Bromobenzoylamino)-1,3-dihydro-5-(2-fluorophenyl)-1-methyl-2H-1,4-benzodiazepin-2-one

The procedure of Example 134 was carried out using 3(S)-(-)-3-amino-1,3-dihydro-5-(2-fluorophenyl)-1-methyl-2H-1,4-benzodiazepin-2-one (44.2 mg, 0.156 mmole) in place of 1,3-dihydro-3(RS)-amino-5-(2-fluorophenyl)-2H-1,4-benzodiazepin-2-one and 4-bromobenzoyl chloride (34.2 mg, 0.156 mmole) in place of p-trifluoromethylbenzoyl chloride. The title compound was chromatographed on silica gel (5% $Et_2O$ in $CH_2Cl_2$ elution) and the product fractions evaporated to dryness in vacuo. The title compound was dried in vacuo at 82°C: (m.p. 123-135°C).
TLC: Single spot, $R_f = 0.46$, silica gel plate, 10% (v/v) $Et_2O$ in $CH_2Cl_2$.
NMR: Consistent with structure.
HPLC: Greater than 99% pure.
MS: Molecular ion at m/e = 465.
$[\alpha]_D^{25}$ = +9.6° (0.0023 g/ml, $CH_2Cl_2$).

| Anal. calc'd for $C_{23}H_{17}BrFN_3O_2$: | | | |
|---|---|---|---|
| | C, 59.24; | H, 3.67; | N, 9.01; |
| Found: | C, 59.12; | H, 3.75; | N, 8.77. |

EXAMPLE 157

3(S)-(+)-3-(4-t-Butylbenzoylamino)-1,3-dihydro-5-(2-fluorophenyl)-1-methyl-2H-1,4-benzodiazepin-2-one

The procedure of Example 134 was carried out using 3(S)-(-)-3-amino-1,3-dihydro-5-(2-fluorophenyl)-1-methyl-2H-1,4-benzodiazepin-2-one (44.2 mg, 0.156 mmole) in place of 1,3-dihydro-3(RS)-amino-5-(2-fluorophenyl)-2H-1,4-benzodiazepin-2-one and 4-t-butylbenzoyl chloride (30.7 mg, 0.156 mmole) in place of p-trifluoromethylbenzoyl chloride. The product was chromatographed on silica gel (4% $Et_2O$ in $CH_2Cl_2$ elution), and the product fractions evaporated to dryness in vacuo. The title compound was dried in vacuo at 82°C: (m.p. 184-190°C).
TLC: Single spot, $R_f = 0.37$, silica gel plate, 5% (v/v $Et_2O$ in $CH_2Cl_2$).
NMR: Consistent with structure.
HPLC: Greater than 99% pure.
MS: Molecular ion at m/e = 443.
$[\alpha]_D^{25}$ = +6.7° (0.0021 g/ml, $CH_2Cl_2$).

| Anal. calc'd for $C_{27}H_{26}FN_3O_2$: | | | |
|---|---|---|---|
| | C, 73.12; | H, 5.91; | N, 9.48; |
| Found: | C, 73.03; | H, 6.11; | N, 9.44. |

EXAMPLE 158

1,3-Dihydro-5-(2-fluorophenyl)-3(RS)-(pyrrole-2-carbonylamino)-2H-1,4-benzodiazepin-2-one

The procedure of Example 134 was carried out using pyrrole-2-carbonyl chloride (20.2 mg, 0.156 mmole) in place of p-trifluoromethylbenzoyl chloride. Without washing, the reaction mixture was chromatographed on silica gel (225:10:1:1 (v:v:v:v) $CH_2Cl_2$:MeOH:HOAc:$H_2O$ elution). The combined product fractions were evaporated to dryness in vacuo and crystallized from EtOAc to give the title compound which was dried in vacuo at 82°C: (m.p. 271-274°C).
TLC: Single spot, $R_f = 0.35$, silica gel plate, 180:10:1:1 (v/v/v/v) $CH_2Cl_2$:MeOH:HOAc:$H_2O$.

NMR: Consistent with structure, verifies presence of 0.25 EtOAc.
HPLC: Greater than 95% pure.
MS: Molecular ion at m/e = 362.

| Anal. calc'd for $C_{20}H_{15}FN_4O_2 . 0.25C_4H_{10}O$: | | | |
|---|---|---|---|
| | C, 65.62; | H, 4.46; | N, 14.58; |
| Found: | C, 65.60; | H, 4.55; | N, 14.53. |

EXAMPLE 159

3(S)-(+)-1,3-Dihydro-5-(2-fluorophenyl)-3-(4-iodobenzoylamino)-1-methyl-2H-1,4-benzodiazepin-2-one

The procedure of Example 134 was carried out using 3(S)-(-)-3-amino-1,3-dihydro-5-(2-fluorophenyl)-1-methyl-2H-1,4-benzodiazepin-2-one (44.2 mg, 0.156 mmole) in place of 1,3-(dihydro-3(RS)-amino-5-(2-fluorophenyl)-2H-1,4-benzodiazepin-2-one and 4-iodobenzoyl chloride (41.6 mg, 0.156 mmole in place of p-trifluoromethylbenzoyl chloride. The product was chromatographed on silica gel (5% (v/v) $Et_2O$ in $CH_2Cl_2$ elution) and the product fractions evaporated to dryness in vacuo. The title compound was dried in vacuo at 82°C: (m.p. 128-140°C).
TLC: Single spot, $R_f$ = 0.51, silica gel plate, 10% (v/v) $Et_2O$ in $CH_2Cl_2$.
NMR: Consistent with structure.
HPLC: Greater than 99% pure.
MS: Molecular ion at m/e = 513.
$[\alpha]_D^{25}$ = +8.4° (0.0028 g/ml, $CH_2Cl_2$).

| Anal. calc'd for $C_{23}H_{17}FIN_3O_2$: | | | |
|---|---|---|---|
| | C, 53.82; | H, 3.34; | N, 8.19; |
| Found: | C, 53.72; | H, 3.44; | N, 8.00. |

EXAMPLE 160

1,3-Dihydro-3(RS)-(2-naphthoylamino)-5-phenyl-2H-1,4-benzodiazepin-2-one

The procedure of Example 134 was carried out using 3(RS)-amino-1,3-dihydro-5-phenyl-2H-1,4-benzodiazepin-2-one (39.2 mg, 0.156 mmole) in place of 1,3-dihydro-3(RS)-amino-5-(2-fluorophenyl)-2H-1,4-benzodiazepin-2-one and 2-naphthoyl chloride (29.7 mg, 0.156 mmole) in place of p-trifluoromethylbenzoyl chloride. The product was chromatographed on silica gel (15% (v/v) $Et_2O$ in $CH_2Cl_2$ elution). The combined product fractions were evaporated to dryness in vacuo and crystallized from $CH_2Cl_2$/EtOAc to give the title compound which was dried in vacuo at 82°C: (m.p. 293-294°C).
TLC: Single spot, $R_f$ = 0.28, silica gel plate, 15% (v/v) $Et_2O$ in $CH_2Cl_2$.
NMR: Consistent with structure.
HPLC: Greater than 99% pure.
MS: Molecular ion at m/e = 405.

| Anal. calc'd for $C_{26}H_{19}N_3O_2$: | | | |
|---|---|---|---|
| | C, 77.02; | H, 4.72; | N, 10.37; |
| Found: | C, 76.88; | H, 4.85; | N, 10.50. |

EXAMPLE 161

3(S)-(-)-3-(2-Bromobenzoylamino)-1,3-dihydro-5-(2-fluorophenyl)-1-methyl-2H-1,4-benzodiazepin-2-one

The procedure of Example 134 was carried out using 3(S)-(-)-3-amino-1,3-dihydro-5-(2-fluorophenyl)-1-methyl-2H-1,4-benzodiazepin-2-one (44.2 mg, 0.156 mmole) in place of 1,3-dihydro-3(RS)-amino-5-(2-fluorophenyl)-2H-1,4-benzodiazepin-2-one and 2-bromobenzoyl chloride (34.2 mg, 0.156 mmole) in place of p-trifluoromethylbenzoyl chloride. The product was chromatographed on silica gel (5% (v/v) $Et_2O$ in $CH_2Cl_2$ elution). The combined product fractions were evaporated to dryness. The residue was crystallized from $Et_2O$ to give the title compound which was dried in vacuo at 82°C: (m.p. 165-185°C).
TLC: Single spot, $R_f$ = 0.38, silica gel plate, 10% (v/v) $Et_2O$ in $CH_2Cl_2$.
NMR: Consistent with structure.
HPLC: Greater than 99% pure.
MS: Molecular ion at m/e = 465.
$[\alpha]_D^{25}$ = -24.1° (0.0037 g/ml, $CH_2Cl_2$).

| Anal. calc'd for $C_{23}H_{17}BrFN_3O_2$: | | | |
|---|---|---|---|
| | C, 59.24; | H, 3.67; | N, 9.01; |
| Found: | C, 59.14; | H, 3.61; | N, 9.06. |

EXAMPLE 162

3(S)-(+)-3-(4-Cyanobenzoylamino)-1,3-dihydro-5-(2-fluorophenyl)-1-methyl-2H-1,4-benzodiazepin-2-one

The procedure of Example 134 was carried out using 3(S)-(-)-3-amino-1,3-dihydro-5-(2-fluorophenyl)-1-methyl-2H-1,4-benzodiazepin-2-one (44.2 mg, 0.156 mmole) in place of 1,3-dihydro-3(RS)-amino-5-(2-fluorophenyl)-2H-1,4-benzodiazepin-2-one and 4-cyanobenzoyl chloride (25.8 mg, 0.156 mmole) in place of p-trifluoromethylbenzoyl chloride. The product was chromatographed on silica gel (8% (v/v) $Et_2O$ in $CH_2Cl_2$ elution). The combined product fractions were evaporated to dryness in vacuo to give the title compound which was dried in vacuo at 82°C: (m.p. 130-147°C).
TLC: Single spot, $R_f$ = 0.29, silica gel plate, 10% (v/v) $Et_2O$ in $CH_2Cl_2$.
NMR: Consistent with structure, verifies presence of 0.1 $Et_2O$.
HPLC: Greater than 98% pure.
MS: Molecular ion at m/e = 412.
$[\alpha]_D^{25}$ = +13.0° (0.0027 g/ml, $CH_2Cl_2$).

| Anal. calc'd for $C_{24}H_{17}FN_4O_2 . 0.1C_4H_{10}O$: | | | |
|---|---|---|---|
| | C, 69.80; | H, 4.32; | N, 13.34; |
| Found: | C, 69.50; | H, 4.43; | N, 13.44. |

EXAMPLE 163

**1,3-Dihydro-5-phenyl-3(RS)-(4-n-propylbenzoylamino)-2H-1,4-benzodiazepin-2-one**

The procedure of Example 134 was carried out using 3(RS)-amino-1,3-dihydro-5-phenyl-2H-1,4-benzodiazepin-2-one (39.2 mg, 0.156 mmole) in place of 1,3-dihydro-3(RS)-amino-5-(2-fluorophenyl)-2H-1,4-benzodiazepin-2-one and 4-n-propylbenzoyl chloride (28.5 mg, 0.156 mmole) in place of p-trifluoromethylbenzoyl chloride. The product was chromatographed on silica gel (15% (v/v) $Et_2O$ in $CH_2Cl_2$ elution).The combined product fractions were evaporated to dryness in vacuo and crystallized from $Et_2O$ to give the title compound which was dried in vacuo at 82°C: (m.p.. 158-162°C).
TLC: Single spot, $R_f$ = 0.24, silica gel plate, 15% (v/v) $Et_2O$ in $CH_2Cl_2$.
NMR: Consistent with structure.
HPLC: Greater than 99% pure.
MS: Molecular ion at m/e = 397.

| Anal. calc'd for $C_{25}H_{23}N_3O_2$: | | | |
|---|---|---|---|
| | C, 75.54; | H, 5.83; | N, 10.57; |
| Found: | C, 75.16; | H, 5.98; | N, 10.74. |

EXAMPLE 164

1,3-Dihydro-5-phenyl-3(RS)-(4-phenylbenzoylamino)-2H-1,4-benzodiazepin-2-one

The procedure of Example 134 was carried out using 3(RS)-amino-1,3-dihydro-5-phenyl-2H-1,4-benzodiazepin-2-one (39.2 mg, 0.156 mmole) in place of 1,3-dihydro-3(RS)-amino-5-(2-fluorophenyl)-2H-1,4-benzodiazepin-2-one and 4-phenylbenzoyl chloride (33.8 mg, 0.156 mmole) in place of p-trifluoromethylbenzoyl chloride. The product was chromatographed on silica gel (15% (v/v) $Et_2O$ in $CH_2Cl_2$ elution).The combined product fractions were evaporated to dryness in vacuo and crystallized from $Et_2O$ to give the title compound which was dried in vacuo at 82°C: (m.p. 274-276°C).
TLC: Single spot, $R_f$ = 0.24, silica gel plate, 15% (v/v) $Et_2O$ in $CH_2Cl_2$.
NMR: Consistent with structure.
HPLC: Greater than 98% pure.
MS: Molecular ion at m/e = 431.

| Anal. calc'd for $C_{28}H_{21}N_3O_2$: | | | |
|---|---|---|---|
| | C, 77.94; | H, 4.91; | N, 9.74; |
| Found: | C, 77.69; | H, 5.17; | N, 9.84. |

EXAMPLE 165

**1,3-Dihydro-3(RS)-(4-n-pentylbenzoylamino)-5-phenyl-2H-1,4-benzodiazepin-2-one**

The procedure of Example 134 was carried out using 3(RS)-amino-1,3-dihydro-5-phenyl-2H-1,4-benzodiazepin-2-one (39.2 mg, 0.156 mmole) in place of 1,3-dihydro-3(RS)-amino-5-(2-fluorophenyl)-2H-1,4-benzodiazepin-2-one and 4-n-pentylbenzoyl chloride (32.9 mg, 0.156 mmole)

in place of p−trifluorobenzoyl chloride. The product was chromatographed on silica gel (15%, (v/v) $Et_2O$ in $CH_2Cl_2$ elution). The combined product fractions were evaporated to dryness in vacuo and crystallized from $Et_2O$ to give the title compound which was dried in vacuo at 82° C: (m.p. 203−205° C).
TLC: Single spot, $R_f$ = 0.28, silica gel plate, 15% (v/v) $Et_2O$ in $CH_2Cl_2$.
NMR: Consistent with structure.
HPLC: Greater than 99% pure.
MS: Molecular ion at m/e = 425.

| Anal. calc'd for $C_{27}H_{27}N_3O_2$: | | | |
|---|---|---|---|
| | C, 76.21; | H, 6.40; | N, 9.88; |
| Found: | C, 76.07; | H, 6.53; | N, 10.00. |

EXAMPLE 166

1,3−Dihydro−3(RS)−(1−naphthoylamino)−5−phenyl−2H−1,4−benzodiazepin−2−one

The procedure of Example 134 was carried out using 3(RS)−amino−1,3−dihydro−5−phenyl−2H−1,4−benzodiazepin−2−one (39.2 mg, 0.156 mmole) in place of 1,3−dihydro−3(RS)−amino−5−(2−fluorophenyl)−2H−1,4−benzodiazepin−2−one and 1−naphthoyl chloride (29.7 mg, 0.156 mmole) in place of p−trifluoromethylbenzoyl chloride. The product was chromatographed on silica gel (15% (v/v) $Et_2O$ in $CH_2Cl_2$ elution). The combined product fractions were evaporated to dryness in vacuo and crystallized from $Et_2O$ to give the title compound which was dried in vacuo at 65° C: (m.p. 162−167° C).
TLC: Single spot, $R_f$ = 0.22, silica gel plate, 15% (v/v) $Et_2O$ in $CH_2Cl_2$.
NMR: Consistent with structure.
HPLC: Greater than 96% pure.
MS: Molecular ion at m/e = 405.

| Anal. calc'd for $C_{26}H_{19}N_3O_2$: | | | |
|---|---|---|---|
| | C, 77.02; | H, 4.72; | N, 10.37; |
| Found: | C, 77.20; | H, 4.91; | N, 10.25. |

EXAMPLE 167

3(S)−(+)−1,3−Dihydro−5−(2−fluorophenyl)−3−(3−iodobenzoylamino)−1−methyl−2H−1,4−benzodiazepin−2−one

The procedure of Example 134 was carried out using 3(S)−(−)−3−amino−1,3−dihydro−5−(2−fluorophenyl)−1−methyl−2H−1,4−benzodiazepin−2−one (44.2 mg, 0.156 mmole) in place of 1,3−dihydro−3(RS)−amino−5−(2−fluorophenyl)−2H−1,4−benzodiazepin−2−one and 3−iodobenzoyl chloride (41.6 mg, 0.156 mmole) in place of p−trifluoromethylbenzoyl chloride. The product was chromatographed on silica gel (5% (v/v) $Et_2O$ in $CH_2Cl_2$ elution). The combined product fractions were evaporated to dryness in vacuo to give the title compound which was dried in vacuo at 65° C: (m.p. 105−120° C).
TLC: Single spot, $R_f$ = 0.34, silica gel plate, 5% (v/v) $Et_2O$ in $CH_2Cl_2$.
NMR: Consistent with structure.
HPLC: Greater than 96% pure.
MS: Molecular ion at m/e = 513.
$[\alpha]_D^{25}$ = +13.0° (0.0024 g/ml, $CH_2Cl_2$).

| Anal. calc'd for $C_{23}H_{17}FIN_3O_2$: | | | |
|---|---|---|---|
| | C, 53.82; | H, 3.34; | N, 8.19; |
| Found: | C, 54.10; | H, 3.46; | N, 8.18. |

EXAMPLE 168

3(R)-(-)-1,3-Dihydro-5-(2-fluorophenyl)-3-(3-iodobenzoylamino)-1-methyl-2H-1,4-benzodiazepin-2-one

The procedure of Example 134 was carried out using 3(R)-(+)-3-amino-1,3-dihydro-5-(2-fluorophenyl)-1-methyl-2H-1,4-benzodiazepin-2-one (44.2 mg, 0.156 mmole) in place of 1,3-dihydro-3(RS)-amino-5-(2-fluorophenyl)-2H-1,4-benzodiazepin-2-one and 3-iodobenzoyl chloride (41.6 mg, 0.156 mmole) in place of p-trifluoromethylbenzoyl chloride. The product was chromatographed on silica gel (5% (v/v) $Et_2O$ in $CH_2Cl_2$ elution). The combined product fractions were evaporated to dryness in vacuo to give the title compound which was dried in vacuo at 65°C: (m.p. 169-172°C).

TLC: Single spot, $R_f = 0.38$, silica gel plate, 5% (v/v) $Et_2O$ in $CH_2Cl_2$.
NMR: Consistent with structure.
HPLC: Greater than 97% pure.
MS: Molecular ion at m/e = 513.
$[\alpha]_D^{25} = -10.2°$ (0.0026 g/ml, $CH_2Cl_2$).

| Anal. calc'd for $C_{23}H_{17}FIN_3O_2$: | | | |
|---|---|---|---|
| | C, 53.82; | H, 3.34; | N, 8.19; |
| Found: | C, 54.07; | H, 3.42; | N, 8.50. |

EXAMPLE 169

3(R)-(+)-1,3-Dihydro-5-(2-fluorophenyl)-3-(2-iodobenzoylamino)-1-methyl-2H-1,4-benzodiazepin-2-one

The procedure of Example 134 was carried out using 3(R)-(+)-3-amino-1,3-dihydro-5-(2-fluorophenyl)-1-methyl-2H-1,4-benzodiazepin-2-one (44.2 mg, 0.156 mmole) in place of 1,3-dihydro-3(RS)-amino-5-(2-fluorophenyl)-2H-1,4-benzodiazepin-2-one, and 2-iodobenzoyl chloride (41.6 mg, 0.156 mmole) in place of p-trifluoromethylbenzoyl chloride. The product was chromatographed on silica gel (5% (v/v) $Et_2O$ in $CH_2Cl_2$ elution). The combined product fractions were evaporated to dryness in vacuo and crystallized from ether to give the title compound which was dried in vacuo at 65°C: (m.p. 231-235°C).

TLC: Single spot, $R_f = 0.24$, silica gel plate, 5% (v/v) $Et_2O$ in $CH_2Cl_2$.
NMR: Consistent with structure.
HPLC: Greater than 99% pure.
MS: Molecular ion at m/e = 513.
$[\alpha]_D^{25} = +26.1°$ (0.0028 g/ml, $CH_2Cl_2$).

| Anal. calc'd for $C_{23}H_{17}FIN_3O_2$: | | | |
|---|---|---|---|
| | C, 53.82; | H, 3.34; | N, 8.19; |
| Found: | C, 53.71; | H, 3.38; | N, 8.14. |

EXAMPLE 170

3(S)-(-)-1,3-Dihydro-5-(2-fluorophenyl)-3-(2-iodobenzoylamino)-1-methyl-2H-1,4-benzodiazepin-2-one

The procedure of Example 134 was carried out using 3(S)-(-)-3-amino-1,3-dihydro-5-(2-fluorophenyl)-1-methyl-2H-1,4-benzodiazepin-2-one (44.2 mg, 0.156 mmole) in place of 1,3-dihydro-3(RS)-amino-5-(2-fluorophenyl)-2H-1,4-benzodiazepin-2-one and 2-iodobenzoyl chloride (41.6 mg, 0.156 mmole) in place of p-trifluoromethylbenzoyl chloride. The product was chromatographed on silica gel (5% (v/v) $Et_2O$ in $CH_2Cl_2$ elution). The combined product fractions were evaporated to dryness in vacuo and crystallized from $Et_2O$ to give the title compound which was dried in vacuo at 65° C: (m.p. 230-232° C).
TLC: Single spot, $R_f = 0.24$, silica gel plate, 5% (v/v) $Et_2O$ in $CH_2Cl_2$.
NMR: Consistent with structure.
HPLC: Greater than 98% pure.
MS: Molecular ion at m/e = 513.
$[\alpha]_D^{25}$ = -25.6° (0.0029 g/ml, $CH_2Cl_2$).

| Anal. calc'd for $C_{23}H_{17}FIN_3O_2$: | | | |
|---|---|---|---|
| | C, 53.82; | H, 3.34; | N, 8.19; |
| Found: | C, 53,62; | H, 3.25; | N, 8.30. |

EXAMPLE 171

3(R)-(+)-3-(2-Bromobenzoylamino)-1,3-dihydro-5-(2-fluorophenyl)-1-methyl-2H-1,4-benzodiazepin-2-one

The procedure of Example 134 was carried out using 3(R)-(+)-3-amino-1,3-dihydro-5-(2-fluorophenyl)-1-methyl-2H-1,4-benzodiazepin-2-one (44.2 mg, 0.156 mmole) in place of 1,3-dihydro-3(RS)-amino-5-(2-fluorophenyl)-2H-1,4-benzodiazepin-2-one and 2-bromobenzoyl chloride (34.2 mg, 0.156 mmole) in place of p-trifluoromethylbenzoyl chloride. The product was chromatographed on silica gel (5% (v/v) $Et_2O$ in $CH_2Cl_2$ elution). The combined product fractions were evaporated to dryness in vacuo and crystallized from $Et_2O$ to give the title compound which was dried in vacuo at 65° C: (m.p. 155-160° C).
TLC: Single spot, $R_f = 0.28$, silica gel plate, 5% (v/v) $Et_2O$ in $CH_2Cl_2$.
NMR: Consistent with structure.
HPLC: Greater than 99% pure.
MS: Molecular ion at m/e = 465.
$[\alpha]_D^{25}$ = +26.3° (0.0034 g/ml, $CH_2Cl_2$).

| Anal. calc'd for $C_{23}H_{17}BrFN_3O_2$: | | | |
|---|---|---|---|
| | C, 59,24; | H, 3.67; | N, 9.01; |
| Found: | C, 59.15; | H, 3.70; | N, 9.12. |

EXAMPLE 172

3(R)-(+)-3-(2-Chlorobenzoylamino)-1,3-dihydro-5-(2-fluorophenyl)-1-methyl-2H-1,4-benzodiazepin-2-one

The procedure of Example 134 was carried out using 3(R)-(+)-3-amino-1,3-dihydro-5-(2-fluorophenyl)-1-methyl-2H-1,4-benzodiazepin-2-one (44.2 mg, 0.156 mmole) in place of 1,3-dihydro-3(RS)-amino-5-(2-fluorophenyl)-2H-1,4-benzodiazepin-2-one and 2-chlorobenzoyl chloride (27.3 mg, 0.156 mmole) in place of p-trifluoromethylbenzoyl chloride. The product was

chromatographed on silica gel (5% (v/v) $Et_2O$ in $CH_2Cl_2$ elution). The combined product fractions were evaporated to dryness in vacuo and crystallized from $CH_2Cl_2$ to give the title compound which was dried in vacuo at 65° C: (m.p. 157 - 165° C).
TLC: Single spot, $R_f = 0.25$, silica gel plate, 5% (v/v) $Et_2O$ in $CH_2Cl_2$.
NMR: Consistent with structure.
HPLC: Greater than 99% pure.
MS: Molecular ion at m/e = 421.
$[\alpha]_D^{25}$ = +16.7° (0.0032 g/ml, $CH_2Cl_2$).

| Anal. calc'd for $C_{23}H_{17}ClFN_3O_2$: | | | |
|---|---|---|---|
| | C, 65.48; | H, 4.06; | N, 9.96; |
| Found: | C, 65.63; | H, 4.10; | N, 10.03. |

## EXAMPLE 173

### 1,3-Dihydro-5-(2-fluorophenyl)-3(RS)-phenylcarbonylamino-2H-1,4-benzodiazepin-2-one

The procedure of Example 134 was carried out using benzoyl chloride (21.9 mg, 0.156 mmole) in place of p-trifluoromethylbenzoyl chloride. The title compound was crystallized from ethyl acetate and dried in vacuo at 75° C: (m.p. 243 - 244° C).
TLC: Single spot, $R_f = 0.18$, silica gel plate, (chloroform - methanol, 1:1 v/v).
NMR: The spectrum was consistent with the title structure.
HPLC: Greater than 98% pure.
MS: Molecular ion at m/e = 373.

| Anal. calc'd for | | | |
|---|---|---|---|
| | C, 70.76; | H, 4.32; | N, 11.25; |
| Found: | C, 70.63; | H, 4.35; | N, 11.07. |

## EXAMPLE 174

### 1,3-Dihydro-5-(2-fluorophenyl)-3(RS)-(2-chlorophenyl)carbonylamino-2H-1,4-benzodiazepin-2-one

The procedure of Example 134 was carried out using 2-chlorobenzoyl chloride (27.3 mg, 0.156 mmole) in place of p-trifluoromethylbenzoyl chloride. The title compound was crystallized from ethyl acetate and dried in vacuo at 75° C: (m.p. 224 - 224.5° C).
TLC: Single spot, $R_f = 0.27$, silica gel plate, (chloroform - methanol, 97:3 v/v).
NMR: The spectrum was consistent with the title structure.
HPLC: Greater than 98% pure.
MS: Molecular ion at m/e = 407.

| Anal. calc'd for $C_{22}H_{15}ClFN_3O_2 . 0.1C_4H_8O_2$: | | | |
|---|---|---|---|
| | C, 64.57; | H, 3.82; | N, 10.08; |
| Found: | C, 64.30; | H, 3.76; | N, 9.99. |

EXAMPLE 175

1,3-Dihydro-5-(2-fluorophenyl)-3(RS)-benzyloxycarbonylamino-2H-1,4-benzodiazepin-2-one

The procedure of Example 134 was carried out using benzyl chloroformate (26.6 mg, 0.156 mmole) in place of p-trifluoromethylbenzoyl chloride. The title compound was crystallized from ethyl acetate and dried in vacuo at 75 °C: (m.p. 208 °C).
TLC: Single spot, $R_f$ = 0.37, silica gel plate, (hexane-ethyl acetate, 1:1 v/v).
NMR: The spectrum was consistent with the title structure.
HPLC: Greater than 98% pure.
MS: Molecular ion at m/e = 403.

| Anal. calc'd for $C_{23}H_{18}FN_3O_3$: | C, 68.48; | H, 4.50; | N, 10.42; |
|---|---|---|---|
| Found: | C, 68.84; | H, 4.62; | N, 10.49. |

EXAMPLE 176

1,3-Dihydro-5-(2-fluorophenyl)-3-(RS)-benzyloxycarbonylamino-2H-1,4-benzodiazepin-2-thione

1,3-Dihydro-5-(2-fluorophenyl)-3-(RS)-benzyloxycarbonylamino-2H-1,4-benzodiazepin-2-one (6.5 g, 16.1 mmole) and 2,4-bis-(4-methoxyphenyl)-2,4-dithioxo-1,3,2,4-dithiaphosphetane (4.9 g, 12.1 mmole) were combined in 500 ml of toluene and heated at reflux for 1.5 hours. The reaction mixture was cooled, diluted to 700 ml with ethyl acetate and washed with 10% sodium hydroxide solution (4 x 50 ml) and brine. The organic phase was dried ($Na_2SO_4$) and concentrated under reduced pressure to yield 12 g of crude product. Trituration with ethyl acetate gave 4.0 g of the analytical product as a yellow powder. Chromatography of the mother liquors on silica gel (hexane-ethyl acetate elution, 1:1 v/v) afforded an additional 2.2 g of pure product: m.p. 190-191 °C.
NMR ($CDCl_3$): Confirmed structure of the title compound.
MS (14 ev): 419 ($M^+$), 311, 284, 256, 243, 224.

| Anal. calc'd for $C_{23}H_{18}FN_3O_2S$: | N, 10.02; | C, 65.86; | H, 4.33; |
|---|---|---|---|
| Found: | N, 9 79; | C, 65.59; | H, 4.44. |

EXAMPLE 177

1-(4-Chlorophenyl)carbonyl-1,3-dihydro-5-(2-fluorophenyl)-3(RS)-(4-chlorophenyl)-carbonylamino-2H-1,4-benzodiazepin-2-one

To a solution of 1,3-dihydro-5-(2-fluorophenyl)-3-amino-2H-1,4-benzodiazepin-2-one (400 mg, 1.49 mmole) in 25 ml of methylene chloride was added p-chlorobenzoyl chloride (380 µl, 3.0 mmole). Triethylamine was added to bring the pH of the reaction mixture to approximately 6 (moist pH paper) followed by 4-dimethylamino pyridine (183 mg, 1.5 mmole). After stirring at room temperature overnight the reaction mixture was diluted with methylene chloride to 200 ml and washed in succession with 10% citric acid solution (3 x 50 ml), saturated sodium bicarbonate solution, and brine. The organic extracts were dried ($MgSO_4$) and concentrated to give 890 mg of crude product. Silica gel chromatography (hexane-ethyl acetate, 1:1 v/v) afforded the analytical product: m.p. 190-191 °C.
TLC: Single spot, $R_f$ = 0.70, silica gel (hexane-ethyl acetate, 1:1 v/v).
NMR: The spectrum is consistent with the title structure.
HPLC: Greater than 97% pure.
MS: Molecular ion m/e = 546.

| Anal. calc'd for $C_{29}H_{18}Cl_2FN_3O_3$: | | | |
|---|---|---|---|
| | N, 7.69; | C, 63.74; | H, 3.32; |
| Found: | N, 7.58; | C, 63.88; | H, 3.46. |

EXAMPLE 178

1-(4-Chlorophenyl)carbonyl-1,3-dihydro-5-(2-fluorophenyl)-3(RS)-(4-chlorophenyl)-carbonyloxy-2H-1,4-benzodiazepin-2-one

A suspension of 1,3-dihydro-5-(2-fluorophenyl)-3-hydroxy-2H-1,4-benzodiazepin-2-one (610 mg, 2.25 mmole) in 25 ml of methylene chloride was treated with 4-chlorobenzoyl chloride (0.314 ml, 2.48 mmole) at room temperature. 4-Dimethylaminopyridine (303 mg, 2.48 mmole) was added and within minutes the reaction mixture became homogeneous. The reaction mixture was protected from moisture and stirred at room temperature overnight. An additional equivalent each of 4-chlorobenzoyl chloride and 4-dimethylaminopyridine were added and stirring was continued for 8 hours at 40-45°C. The reaction mixture was diluted to 150 ml with methylene chloride and washed in succession with 10% citric acid solution (3 x 50 ml), saturated sodium bicarbonate solution (3 x 50 ml) and brine (50 ml). Rotoevaporation of the dried ($MgSO_4$) organic phase gave a foam which on trituration with ether afforded a beige solid. Recrystallization from ethyl acetate afforded 612 mg of the title compound as a white powder in analytical purity: m.p. 198-199°C.
NMR (DMSO-$d_6$): The spectrum is consistent with the title structure.
MS (14 ev): 547 ($M^+$), 407, 379, 374, 363, 224, 156.

| Anal. calc'd for $C_{29}H_{17}Cl_2FN_2O_4$: | | | |
|---|---|---|---|
| | N, 5.11; | C, 63.63; | H, 3.13; |
| Found: | N, 5.03; | C, 63.68; | H, 3.08. |

EXAMPLE 179

1,3-Dihydro-5-(2-fluorophenyl)-3(RS)-(4-chlorophenyl)oxy-2H-1,4-benzodiazepin-2-one

A suspension of 1,3-dihydro-5-(2-fluorophenyl)-3-hydroxy-2H-1,4-benzodiazepin-2-one (610 mg, 2.25 mmole) in 25 ml of methylene chloride was treated with 4-chlorobenzoyl chloride (0.314 ml, 2.48 mmole) at room temperature. 4-Dimethylaminopyridine (303 mg, 2.48 mmole) was added and within minutes the reaction mixture became homogeneous. The reaction mixture was protected from moisture and stirred at room temperature overnight. An additional equivalent, each of 4-chlorobenzoyl chloride and 4-dimethylaminopyridine were added and stirring was continued for 8 hours at 40-45°C. The reaction mixture was diluted to 150 ml with methylene chloride and washed in succession with 10% citric acid solution (3 x 50 ml), saturated sodium bicarbonate solution (3 x 50 ml) and brine (50 ml). Rotoevaporation of the dried ($MgSO_4$) organic phase gave a foam which on trituration with ether afforded a beige solid. The mother liquors were concentrated and the residue chromatographed on silica gel (hexane-ethyl acetate, 1:1 v/v) to give the title compound.
NMR ($CDCl_3$): The spectrum is consistent with the title structure.

| Anal. calc'd for $C_{22}H_{14}ClFN_2O_3$: | | | |
|---|---|---|---|
| | N, 6.85; | C, 64.63; | H, 3.45; |
| Found: | N, 6.68; | C, 64.64; | H, 3.60. |

EXAMPLE 180

1,3-Dihydro-5-(2-fluorophenyl)-3-(RS)-(4-chlorophenyl)carbonylamino-2H-1,4-benzodiazepin-2-thione

A mixture of 1,3-dihydro-5-(2-fluorophenyl)-3-(RS)-amino-2H-1,4-benzodiazepin-2-thione (200 mg, 0.70 mmole), 4-chlorobenzoic acid (120 mg, 0.77 mmole) and 1-ethyl-3-(3-dimethylaminopropyl) carbodiimide hydrochloride (150 mg, 0.77 mmole) were combined in 2 ml of dry N,N-dimethylformamide at room temperature. The pH of the homogeneous reaction mixture was then adjusted to 8 with triethylamine. The reaction mixture was protected from moisture and stirred at room temperature overnight (about 90% complete after 1 hour). The solvent was removed under reduced pressure and the residue dissolved in 100 ml of ethyl acetate. The organic phase was then washed in succession with 10% citric acid solution (2 x 20 ml), saturated sodium bicarbonate solution (20 ml), and brine. The dried ($MgSO_4$) organic phase was rotoevaporated to dryness to yield 300 mg of crude product. Preparative thick layer chromatography on $SiO_2$ (hexane-ethyl acetate, 2:1) gave the analytical sample as a solvate: m.p. 156-158°C.
NMR (DMSO-$d_6$): Confirmed structure of the title compound.
MS (14 ev): 423 ($M^+$), 391, 284, 268, 236, 139.

| Anal. calc'd for $C_{22}H_{15}ClFN_3OS$. $0.10C_4H_8O_2$: | | | |
|---|---|---|---|
| | N, 9.71; | C, 62.17; | H, 3.68; |
| Found: | N, 9.39; | C, 62.45; | H, 4.01. |

EXAMPLE 181

1,3-Dihydro-5-(2-fluorophenyl)-3-(RS)-(2-indole) carbonylamino-2H-1,4-benzodiazepin-2-thione

A mixture of 1,3-dihydro-5-(2-fluorophenyl)-3-(RS)-amino-2H-1,4-benzodiazepin-2-thione (400 mg, 1.40 mmole), indole-2-carboxylic acid (248 mg, 1.54 mmole) and 1-ethyl)-3-(3-dimethylaminopropyl) carbodiimide hydrochloride (295 mg, 1.54 mmole) were combined in 10 ml of dry N,N-dimethylformamide at room temperature. The pH of the homogeneous reaction mixture was then adjusted to 8 with triethylamine. The reaction mixture was protected from moisture and stirred at room temperature overnight (about 50% complete after 1 hour). The solvent was removed under reduced pressure and the residue dissolved in 200 ml of ethyl acetate. The organic phase was then washed in succession with 10% citric acid solution (2 x 25 ml), saturated sodium bicarbonate solution (25 ml), and brine. The dried ($MgSO_4$) organic phase was rotoevaporated to dryness to yield 1.4 g of crude product. Preparative thick layer chromatography on $SiO_2$ (hexane-ethyl acetate, 1:1) gave the analytical sample as a beige powder: m.p. 209-211°C.
NMR ($CDCl_3$): Confirmed structure of the title compound.
MS (14 ev): 428 ($M^+$), 396, 394, 296, 293, 252, 249.

| Anal. calc'd for $C_{24}H_{17}FN_4OS$. $0.15C_4H_8O_2$: | | | |
|---|---|---|---|
| | N, 12.69; | C, 66.89; | H, 4.15; |
| Found: | N, 12.92; | C, 66.69; | H, 3.90. |

EXAMPLE 182

1,3-Dihydro-3(RS)-(4-chlorophenyl)aminocarbonylamino-2H-1,4-benzodiazepin-2-one

To a solution of 85 mg (0.315 mmole) of 1,3-dihydro-3(RS)-amino-5-(2-fluorophenyl)-2H-1,4-benzodiazepin-2-one in 8 ml of dry tetrahydrofuran was added 4-chlorophenylisocyanate (40 µl, 0.315 mmole) at room temperature. Within 15 minutes a flocculant, white precipitate formed. Stirring was

continued for 8 hours more and the reaction mixture was filtered. The collected solids were washed with hot methanol and dried in vacuo to give the analytical product: m.p. 278 °C.
NMR (DMSO − $d_6$): Confirms structure assignment of product.

| Anal. calc'd for $C_{22}H_{16}ClFN_4O_2$: | | | |
|---|---|---|---|
| | N, 13.25; | C, 62.48; | H, 3.81; |
| Found: | N, 13.09; | C, 62.33; | H, 3.86. |

## EXAMPLE 183

### 1,3 − Dihydro − 1 − methyl − 3 − oximino − 5 − phenyl − 2H − 1,4 − benzodiazepin − 2 − one

To a suspension of potassium tert − butoxide (24.9 g, 222 mmole) in 600 ml of dry tetrahydrofuran was added 200 ml of dry tert − butylalcohol at − 20 °C under nitrogen. To this solution was then added via addition funnel 1,3 − dihydro − 1 − methyl − 5 − phenyl − 2H − 1,4 − benzodiazepin − 2 − one (25 g, 99.9 mmole) in 260 ml of tetrahydrofuran. The resulting wine colored solution was stirred for 2 hours at − 20 °C and treated with 17.4 ml (130 mmole) of isoamyl nitrite. The reaction mixture was warmed to 0 °C over 15 minutes and quenched with the addition of 60 ml of cold water and 20 ml of glacial acetic acid. All solvents were removed under reduced pressure and the residue was partitioned between ethyl acetate (600 ml) and brine (100 ml). The phases were separated and the organic extracts were dried ($Na_2SO_4$) and concentrated. The resulting semi − solid was triturated with ether to give 21 g of off − white solid. m.p. 234 − 235 °C;
$R_f$ = 0.15 (ethylacetate − hexane, 1:1); $R_f$ = 0.28 chloroform − ethanol, 95:5);
ir(KBr, partial): 3300, 1650, 1595, 1320, 1205, 1030, 975 $cm^{-1}$.
MS (14 ev.): 279 ($M^+$), 262, 249, 236, 222.
NMR ($CDCl_3$): 3.5 (3H, $CH_3$ − N), confirms structure assignment.

| Elemental Analysis: $C_{16}H_{13}N_3O_2$. | | | |
|---|---|---|---|
| Calcd: | C, 4.69; | H, 68.81; | N, 15.04. |
| Found: | C, 4.62; | H, 68.67; | N, 15.08. |

## EXAMPLE 184

### 3(R S) − Amino − 1,3 − dihydro − 1 − methyl − 5 − phenyl − 2H − 1,4 − benzodiazepin − 2 − one

A solution of 150 ml of methanol containing 5 g (17.9 mmole) of 1,3 − dihydro − 1 − methyl − 3 − oximino − 5 − phenyl − 1,4 − benzodiazepin − 2 − one was treated with a slurry of active Raney − nickel catalyst[1] in ethanol (10 g). The resulting suspension was hydrogenated on a Parr apparatus at 60 psi and 23 °C for 30 hours. The catalyst was removed by filtration and the filtrate was concentrated to afford the title compound in 95% yield.
$R_f$ = 0.23 (chloroform − ethanol, 95:5), $R_f$ = 0.23 (chloroform − methanol − acetic acid − water, 90:10:1:1)
NMR ($CDCl_3$): spectrum confirms structure assignment.

[1] Raney-Nickel catalyst was prepared according to Fieser & Fieser, Reagents for Organic Synthesis, Vol. I, John Wiley & Sons, Inc., New York 1967, p. 729.

## Claims

**Claims for the following Contracting States : BE, CH, DE, FR, GB, IT, LI, LU, NL, SE**

**1.** Use of compounds of formula I

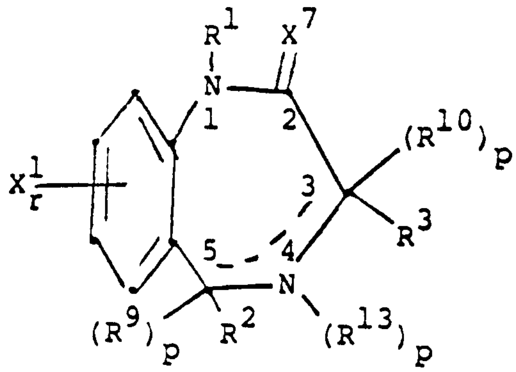

I

or a pharmaceutically acceptable salt or a pharmaceutically acceptable quaternary ammonium salt of said compound of formula I

in which compounds of formula I

$R^1$ is hydrogen, alkyl having 1 – 5 carbon atoms, alkenyl having up to 5 carbon atoms, alkynyl having up to 5 carbon atoms, or a group having the formulae $-(CH_2)_mCOOR^6$, $-(CH_2)_n-C_3-C_5-$cycloalkyl, $-(CH_2)_mNR^4R^5$, $-(CH_2)_mCONR^4R^5$, $-(CH_2)_mCN$, or $-(CH_2)_nCX^{10}_3$ ;
wherein

$R^4$ and $R^5$ are independently hydrogen, alkyl having 1 – 4 carbon atoms or cycloalkyl having 3 – 5 carbon atoms

$R^6$ is hydrogen, alkyl having 1 – 4 carbon atoms, cycloalkyl having 3 – 5 carbon atoms, unsubstituted phenyl, substituted phenyl, unsubstituted phenyl alkyl or substituted phenyl alkyl in which groups the substituents of the phenyl are 1 or 2 substituents selected from halo, alkyl having 1 – 4 carbon atoms, alkoxy having 1 – 4 carbon atoms, nitro or trifluoro methyl and wherein the alkyl moiety of the corresponding substituted or unsubstituted phenyl alkyl group comprises 1 – 4 carbon atoms,

$X^{10}$ is fluoro, chloro or bromo,

m is 1 – 4 and

n is O – 4;

$R^2$ is hydrogen, alkyl having 1 – 4 carbon atoms, unsubstituted phenyl, substituted phenyl having 1 or 2 substituents selected from halo, alkyl having 1 – 4 carbon atoms, alkoxy having 1 – 4 carbon atoms, alkylthio having 1 – 4 carbon atoms, carboxy substituted alkyl having 1 – 4 carbon atoms in the alkyl group, carboxyl, nitro, trifluoromethyl and hydroxy,
or the radical $R^2$ is a group having the following formulae

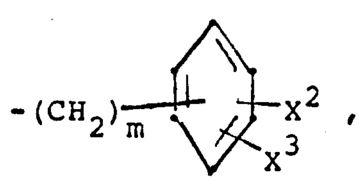

$-(CH_2)_mSCH_3$, $-(CH_2)_mSOCH_3$, $-(CH_2)_mSO_2CH_3$, or $-(CH_2)_mCOOR^6$;
wherein

$R^6$ and m are as defined with regard to the radical $R^1$

$X^2$ and $X^3$ are independently hydrogen, hydroxy, nitro, halo, alkyl having 1 – 4 carbon atoms, alkylthio having 1 – 4 carbon atoms or alkoxy having 1 – 4 carbon atoms,

$R^3$ is a group having the formulae $-(CH_2)_nR^7$,

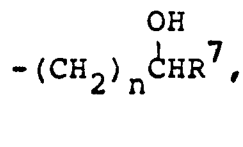 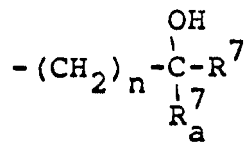

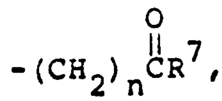

$-(CH_2)_nNR^{18}(CH_2)_qR^7$,

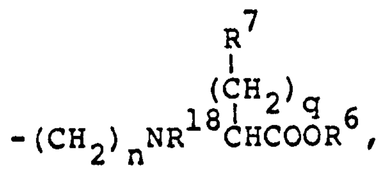

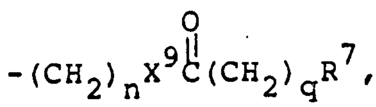

$-NH(CH_2)_{2-3}NHR^7$, $-NH(CH_2)_{2-3}NHCOR^7$,

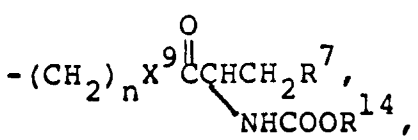

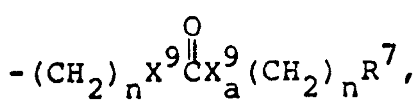 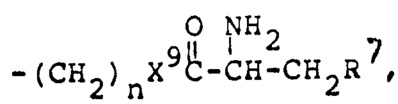

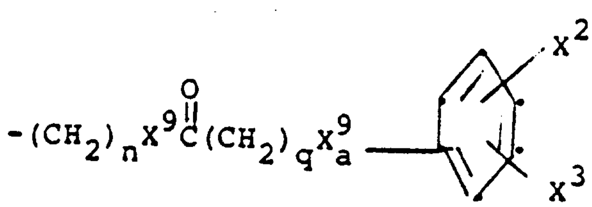 ,

or

$-(CH)_2)_nNR^{18}SO_2(CH_2)_qR^7$

wherein

$R^7$ and $R^7{}_a$ are independently unsubstituted phenyl or substituted phenyl having 1 or 2 substituents which are selected from halo, nitro, hydroxy, alkyl having 1 – 4

carbon atoms, groups having the formulae $CF^3$, CN, $SCF_3$, C≡CH, $CH_2SCF_3$,

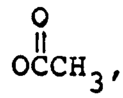

$OCHF_2$, SH, SPh, $PO_3H$, or $-NR^4R^5$,

alkoxy having 1 − 4 carbon atoms or alkylthio having 1 − 4 carbon atoms or the radicals

$R^7$ and $R^7{}_a$ are alpha naphthyl or beta naphthyl or a group having the formulae

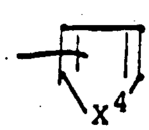 , 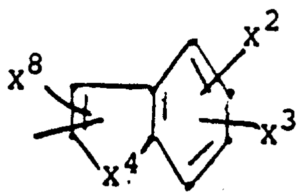 , 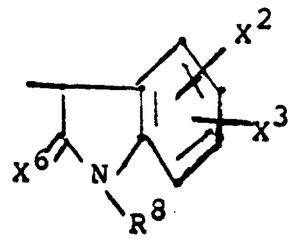

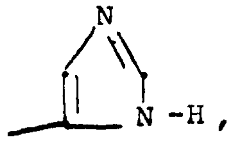, 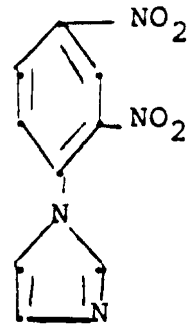,

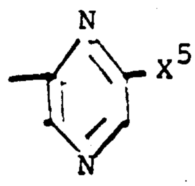, 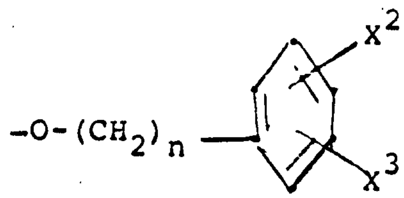,

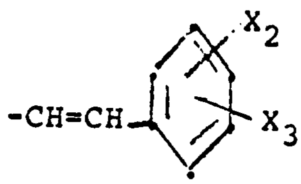, 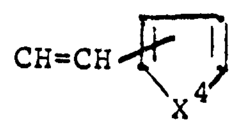

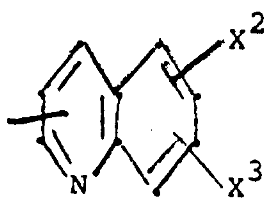, 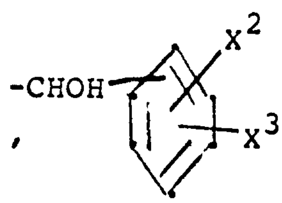, or

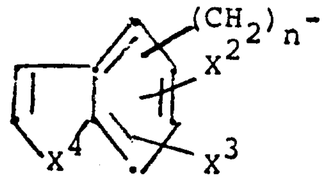

wherein

$R^8$ is hydrogen, alkyl having 1 − 4 carbon atoms, cycloalkyl having 3 − 5 carbon atoms which is bonded either directly or through an alkylene chain comprising 1 − 4 $-CH_2-$ groups or $R^8$ is a group having the formulae $-(CH_2)_mCONH_2$, $-(CH_2)_mCOOR^6$, $-(CH_2)_mNR^4R^5$,

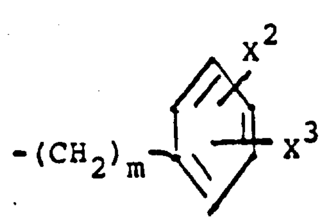,

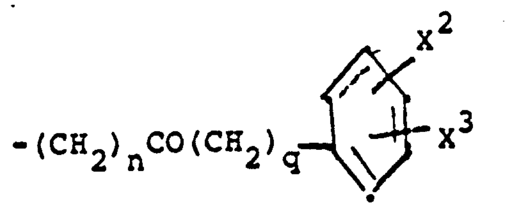,

or

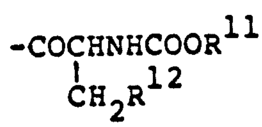;

wherein

X² and X³ — are as defined with regard to radical $R^2$,

$X^4$ is S, O, $CH_2$, or $NR^8$, wherein $R^8$ is as defined above,

$X^5$ is hydrogen, halo, nitro or a group of formulae $CF_3$, CN, $-COOR^6$, wherein $R^6$ is as defined above,

$X^5$ is O or HH;

$X^5$ is hydrogen or alkyl having 1 − 4 carbon atoms,

$X^5$ and $X^5{}_a$ are independently groups of formulae $-O-$ or $-NR^{18}-$ wherein

$R^{18}$ is hydrogen, alkyl having 1 − 4 carbon atoms, formyl, acetyl, propionyl or butyryl,

$R^{11}$ and $R^{12}$ are independently alkyl having 1 − 4 carbon atoms or cycloalkyl having 3 − 5 carbon atoms,

$R^{14}$ is alkyl having 1 − 4 carbon atoms or phenyl alkyl in which the alkyl group has 1 − 4 carbon atoms,

$R^9$ and $R^{10}$ are independently hydrogen, hydroxy or methyl,

$R^{13}$ is hydrogen, alkyl having 1 − 4 carbon atoms, cycloalkyl having 3 − 5 carbon atoms, $-O$, formyl, acetyl, propionyl or butyryl,

$X^1$ is hydrogen, halo, alkyl having 1 − 4 carbon atoms, alkylthio having 1 − 4 carbon atoms, alkoxy having 1 − 4 carbon atoms or a group having the formulae $-NO_2$, $CF_3$ CN, OH, $-(CH_2)_nCOCR^6$, or $-NR^4R^5$; wherein

$R^4$, $R^5$, $R^6$ and n are as defined in connection with the radical $R^1$,

r is 1 or 2,

$X^7$ is O, S, HH, or $NR^{15}$ wherein

$R^{15}$ is hydrogen, alkyl having 1 − 4 carbon atoms or a group of formula

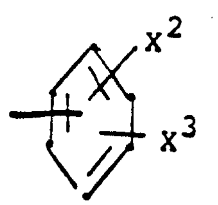,

or $-NR^{16}R^{17}$; wherein

$R^{16}$ and $R^{17}$ are independently hydrogen or a group of formula

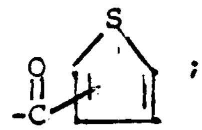

however with the provision that

$X^7$ can be $NR^{15}$ only when $R^1$ is not H;

q is O – 4

- - - indicates a single bond or a double bond,

p is O when its adjacent - - - is a double bond and is 1, when its adjacent - - - is a single bond, except that when $R^{13}$ is oxygen and p is 1 and - - - is a double bond and

with the provision that if

$R^3$ is a group of formula $-(CH_2)_nNH(CH_2)_qR^7$, wherein

q is O or 1, or is a group of formula

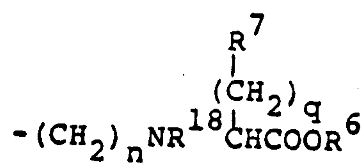

wherein q is O, then $R^7$ has to have another meaning than that of a group of formula

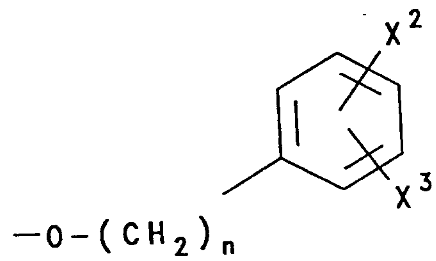

**for the manufacture of a medicament for**
use in antagonizing cholecystokinin in gastrointestinal tissue and for antagonizing gastrin by binding to the corresponding cholecystokinin receptor or gastrin receptor.

2. Use according to claim 1, characterized **in that for the manufacture of a medicament** there is used as pharmaceutically active ingredient a compound of formula I or a pharmaceutically acceptable salt or a pharmaceutically acceptable quaternary ammonium salt of said compound of formula I in which compounds of formula I

$R^1$ is hydrogen, alkyl having 1 – 5 carbon atoms or a group of formula $-(CH_2)_mCOOR^6$ wherein

$R^6$ and m are as defined in claim 1,

$R^2$ is a group of formula $-(CH_2)_mCOOR^6$ wherein

$R^6$ and m are as defined above or $R^2$ is unsubstituted phenyl or substituted phenyl having 1 or 2 substituents selected from halo, alkyl having 1 – 4 carbon atoms, alkoxy having 1

– 4 carbon atoms, carboxy substituted alkyl having 1 – 4 carbon atoms in the alkyl group, carboxyl, nitro, trifluoro methyl and hydroxy,

$R^3$ is a group having the formulae

$-(CH_2)_nR^7$,

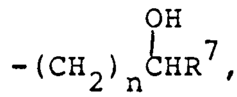

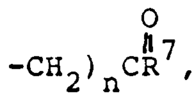

$-(CH_2)_nNH(CH_2)_qR^7$,

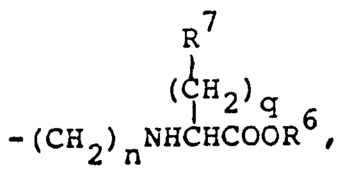

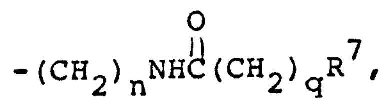

$-NH(CH_2)_{2-3}NHR^7$,

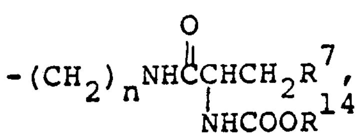

$-NH(CH_2)_{2-3}NHCOR^7$, or

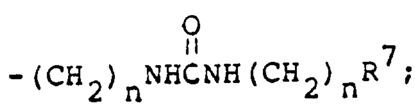

wherein

$R^7$ is unsubstituted phenyl or substituted phenyl having 1 or 2 substituents which are selected from halo, nitro, hydroxy, alkyl having 1 – 4 carbon atoms, alkoxy having 1 – 4 carbon atoms, alkylthio having 1 – 4 carbon atoms, trifluoro – methyl groups or groups having the formulae

CN, $-C\equiv CH$, $SCF_3$,

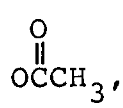

$OCHF_2$, SPh or $-NR^4R^5$

wherein

$R^4$ and $R^5$ are independently hydrogen or alkyl having 1 – 4 carbon atoms,

or the radical

$R^7$ is alpha naphthyl or beta naphthyl or a group having the formulae

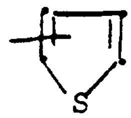 ,

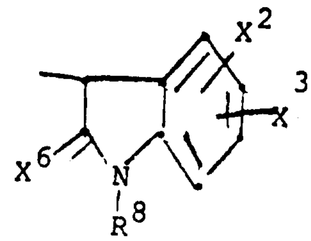 ,

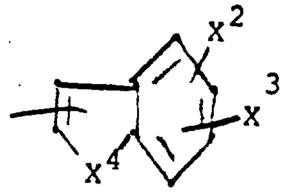 ,

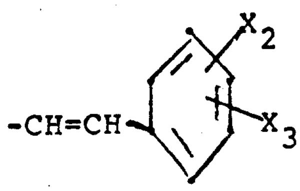 , or 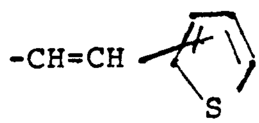

wherein

$R^8$ is hydrogen, alkyl having 1 − 4 carbon atoms or a group of formula

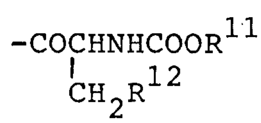

wherein

$R^{11}$ and $R^{12}$ are independently alkyl having 1 − 4 carbon atoms,

$X^2$ and $X^3$ are independently hydrogen, hydroxy, nitro, halo, alkylthio having 1 − 4 carbon atoms, alkyl having 1 − 4 carbon atoms or alkoxy having 1 − 4 carbon atoms,

$X^4$ is S, O, or $NR^8$ wherein $R^8$ is as defined above,

$X^6$ is O or HH;

$R^{14}$ is alkyl having 1 − 4 carbon atoms and

q is 0 − 4,

$R^9$ and $R^{10}$ are independently hydrogen, hydroxy or methyl,

$R^{13}$ is hydrogen, alkyl having l − 4 carbon atoms, −O, formyl, acetyl,propionyl or butyryl,

$X^1$ is hydrogen, halo, alkyl having 1 − 4 carbon atoms, alkylthio having 1 − 4 carbon atoms, alkoxy having 1 − 4 carbon atoms or a group having the formulae $-NO_2$, $CF_3$, CN, OH, $-(CH_2)_nCOOR^6$, or $-NR^4R^5$; wherein

$R^6$ is as defined in connection with radical $R^1$ and $R^4$ and $R^5$ are as defined in connection with the radical $R^3$,

$X^7$ is O

m is l − 4

n is O − 4

$\overline{---}$ indicates a single bond or a double bond,

p is O when the adjacent $\overline{---}$ is a double bond and is l, when its adjacent $\overline{---}$ is a single bond, except that when $R^{13}$ is oxygen and p is l and $\overline{---}$ is a double bond and

r is l or 2.

3. Use according to claim 2, characterized **in that for the manufacture of a medicament** there is used as pharmaceutically active ingredient a compound of formula I or a pharmaceutically acceptable salt or a pharmaceutically acceptable quaternary ammonium salt of said compound of formula I in which

$R^1$ is hydrogen, methyl, ethyl, carboxymethyl, or ethylcarboxymethyl;

$R^2$ is a group of formula

$-(CH_2)_{1-2}COOR^6$;

wherein

$R^6$ is hydrogen or alkyl having 1 − 4 carbon atoms or

$R^2$ is unsubstituted phenyl or substituted phenyl comprising 1 or 2 substituents which are halo or carboxyl,

$R^3$ is a group having the formulae

$-(CH_2)_nR^7$,

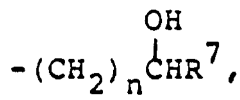

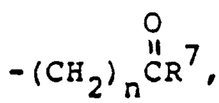

$-(CH_2)_nNH(CH_2)_qR^7$,

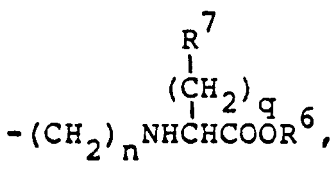

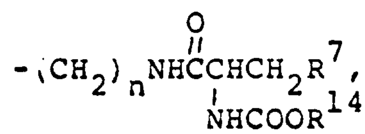

or

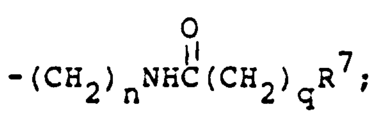

wherein

$R^7$ is alphanaphthyl, betanaphthyl or substituted phenyl having 1 or 2 substituents which are selected from halo, trifluoromethyl and alkyl having 1 − 4 carbon atoms or

$R^7$ is a group having the formulae

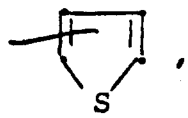

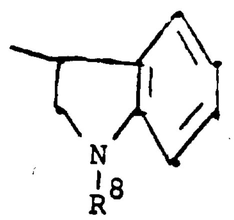,
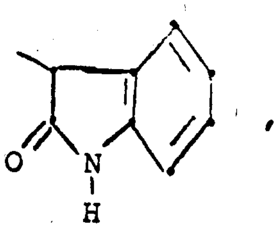,

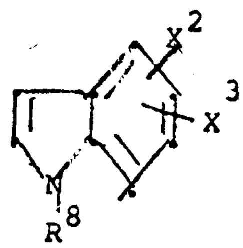,

or

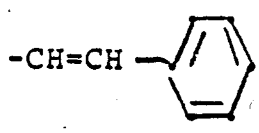

wherein

$R^8$ is hydrogen, methyl or ethyl,
$X^2$ and $X^3$ are independently hydrogen, hydroxy, nitro or halo,
$R^{14}$ is t−butyl;
n is O − 4;
q is O − 4; and
$R^6$ is as defined in connection with the radical $R^2$,
$R^9$ and $R^{10}$ are independently hydrogen, hydroxy or methyl,
$R^{13}$ is hydrogen,methyl or formyl,
$X^1$ is hydrogen, halo, or a group having the formulae $-NO_2$, $CF_3$, CN or OH
$X^7$ is O;
- - - indicates a single bond or a double bond,
p is 0 when the adjacent - - - is a double bond and is I when its adjacent - - - is a single bond, and
r is I or 2.

**4.** Use according to claim 3, characterized **in that for the manufacture of a medicament** there is used as pharmaceutically active ingredient a compound of formula I or a pharmaceutically acceptable salt or a pharmaceutically acceptable quaternary ammonium salt of said compound of formula I, in which compounds of formula I

$R^1$ is hydrogen, methyl or carboxymethyl,
$R^2$ is phenyl, o−fluorophenyl, p−fluorophenyl, o−chlorophenyl, p−chlorophenyl, o−carboxyphenyl, 2,4−dichlorophenyl, 2,6−difluorophenyl, $-CH_2COOEt$, $-CH_2COO-$t−Bu, $-CH_2CH_2COOEt$, or $-CH_2CH_2COO-$t−Bu;
$R^3$ is $-(CH_2)_{1-2}R^7$,

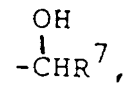

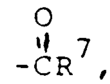

$-(CH_2)_{0-1}NH(CH_2)_{1-2}R^7$,

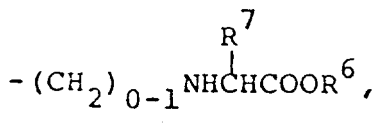 , 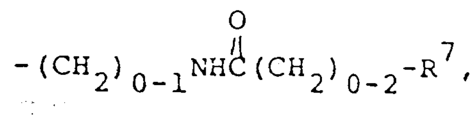

or

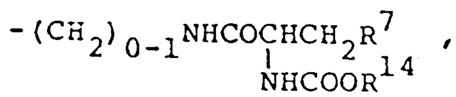

and the stereochemistry relates to D-tryptophan;

$R^7$ is alphanaphthyl, betanaphthyl, monohalophenyl or dihalophenyl or a group having the formulae

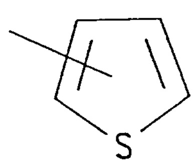 ,

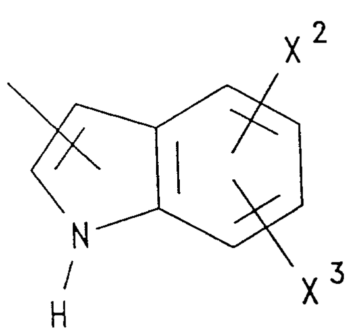 , 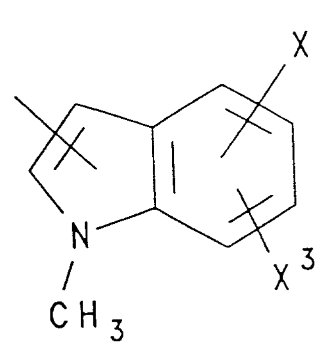

wherein

$X^2$ and $X^3$ are independently hydrogen, hydroxy, fluoro or chloro,

$R^6$ is hydrogen, methyl or ethyl,

$R^{14}$ is t-butyl,

$R^9$ and $R^{10}$ are independently hydrogen or hydroxy,

$R^{13}$ is hydrogen,

$X^1$ is hydrogen, 7-chloro, 7-fluoro, or 7-nitro;

| | |
|---|---|
| $X^7$ | is oxygen, |
| $\underline{- - -}$ | is a single bond or a double bond, |
| p | is O when its adjacent $\underline{- - -}$ is a double bond and is l, when its adjacent $\underline{- - -}$ is a single bond and |
| r | is l. |

**5.** Use according to claim 1, characterized in that the compound of formula I is:

1,3 – dihydro – 5 – (2 – fluorophenyl) – 3(R) – (3' – indolyl)methyl – 2H – 1,4 – benzodiazepin – 2 – one;

1,3 – dihydro – 5 – (2 – fluorophenyl) – 3(R) – [3' – (1' – methylindolyl) – methyl] – 1 – methyl – 2H – 1,4 – benzodiazepin – 2 – one;

1,3 – dihydro – 5 – (2 – fluorophenyl) – 3(R) – (3' – indolyl)methyl – 1 – methyl – 2H – 1,4 – benzodiazepin – 2 – one

7 – chloro – 1,3 – dihydro – 3(R) – (3' – indolyl)methyl – 5 – phenyl – 2H – 1,4 – benzodiazepin – 2 – one;

1,3 – dihydro – 3(R) – (3' – indolyl)methyl – 5 – phenyl – 2H – 1,4 – benzodiazepin – 2 – one;

1,3 – dihydro – 5 – (2 – fluorophenyl) – 3 – (R) – (3' – $\alpha$ – indolenyl) methyl – 2H – 1,4 – benzodiazepin – 2 – one;

1,3 – dihydro – 5 – (2 – fluorophenyl) – 3(R) – (3' – $\beta$ – indolenyl) methyl – 2H – 1,4 – benzodiazepin – 2 – one;

1,3 – dihydro – 5 – (2 – fluorophenyl) – 3(R) – (3' – indolyl)methyl – 2H – 1,4 – benzodiazepin – 2 – thione;

1,3 – dihydro – 5 – (2 – fluorophenyl) – 3(R) – (3' – indolyl)methyl – 2H – 1,4 – benzodiazepine;

7 – chloro – 1,3 – dihydro – 3(R) – benzyl – 5 – phenyl – 2H – 1,4 – benzodiazepin – 2 – one;

3(R) – benzyloxymethyl – 7 – chloro – 1,3 – dihydro – 5 – phenyl – 2H – 1,4 – benzodiazepin – 2 – one;

7 – chloro – 1,3 – dihydro – 3(RS) – (1 – naphthyl)methyl – 5 – phenyl – 2H – 1,4 – benzodiazepin – 2 – one;

7 – chloro – 1,3 – dihydro – 3(RS) – (2 – naphthyl)methyl – 5 – phenyl – 2H – 1,4 – benzodiazepin – 2 – one;

1,3 – dihydro – 5 – (2 – fluorophenyl) – 3(RS) – (2 – thienyl)methyl – 2H – 1,4 – benzodiazepin – 2 – one;

1,3 – dihydro – 5 – (2 – fluorophenyl) – 3(RS) – (3 – thienyl) – 2H – 1,4 – benzodiazepin – 2 – one;

1,3 – dihydro – 5 – (2 – fluorophenyl) – 3(R) – [3' – $\beta$ – (1' – t – Boc – L – leucyl) – indolenyl]methyl – 2H – 1,4 – benzodiazepin – 2 – one;

1,3 – dihydro – 5 – (2 – fluorophenyl) – 3(R) – [3' – $\beta$ – (1' – t – Boc – D – leucyl) – indolenyl]methyl – 2H – 1,4 – benzodiazepin – 2 – one;

1,3 – dihydro – 5 – (2 – fluorophenyl) – 3(R) – [3' – $\alpha$ – (1' – t – Boc – L – leucyl) – indolenyl]methyl – 2H – 1,4 – benzodiazepin – 2 – one;

1,3 – dihydro – 5 – (2 – fluorophenyl) – 3(R) – [3' – $\alpha$ – (1' – t – Boc – D – leucyl) – indolenyl]methyl – 2H – 1,4 – benzodiazepin – 2 – one;

7 – chloro – 1,3,4,5 – tetrahydro – 3(R) – (3' – indolyl)methyl – 5 – phenyl – 2H – 1,4 – benzodiazepin – 2 – one;

7 – chloro – 1,3,4,5 – tetrahydro – 3(S) – (3' – indolyl)methyl – 5 – phenyl – 2H – 1,4 – benzodiazepin – 2 – one;

4 – (p – chlorobenzoyl) – 5 – (2 – fluorophenyl) – 3(R) – [3' – (1' – methylindolyl) – methyl] – 1 – methyl – 1,3,4,5 – tetrahydro – 2H – 1,4 – benzodiazepin – 2 – one;

4 – acetyl – 5 – (2 – fluorophenyl) – 3(R) – [3' – (1' – methylindolyl)methyl] – 1 – methyl – 1,3,4,5 – tetrahydro – 2H – 1,4 – benzodiazepin – 2 – one;

7 – chloro – 5 – (2 – chlorophenyl) – 1,3 – dihydro – 3(R) – (3' – indolyl)methyl – 2H – 1,4 – benzodiazepin – 2 – one;

1,3 – dihydro – 3(R) – (3' – indolyl)methyl – 5 – methyl – 2H – 1,4 – benzodiazepin – 2 – one;

1 – benzyl – 7 – chloro – 1,3 – dihydro – 3(R) – (3' – indolyl)methyl – 5 – phenyl – 2H – 1,4 – benzodiazepin – 2 – one;

7 – chloro – 1,3 – Dihydro – 3(R) – (3' – indolyl)methyl – 1 – methyl – 5 – phenyl – 2H – 1,4 – benzodiazepin – 2 – one;

1,3 – dihydro – 5 – (2 – fluorophenyl) – 3(S) – (3' – indolyl) methyl – 2H – 1,4 – benzodiazepin – 2 – one;

1 – benzyl – 7 – chloro – 1,3 – dihydro – 3(S) – (3' – indolyl) methyl – 5 – phenyl – 2H – 1,4 – benzodiazepin – 2 – one;

7 – chloro – 1,3 – dihydro – 3(R) – (3' – indolyl)methyl – 5 – phenyl – 2H – 1,4 – benzodiazepin – 2 – thione;

1,3 – dihydro – 5 – (2 – fluorophenyl) – 3(R) – (3' – indolyl) methyl – 2H – 1,4 – benzodiazepin – 2 –

[N' – (3 – thienoyl)] hydrazide;

1,3 – dihydro – 1 – ethyl – 5 – (2 – fluorophenyl) – 3(R) – (3' – indolyl)methyl – 2H – 1,4 – benzodiazepin – 2 – one;

1 – cyclopropylmethyl – 1,3 – dihydro – 5 – (2 – fluorophenyl) – 3(R) – (3' – indolyl)methyl – 2H – 1,4 – benzodiazepin – 2 – one;

1,3 – dihydro – 5 – (2 – fluorophenyl) – 3(R) – (3' – indolyl) methyl – 1 – pentyl – 2H – 1,4 – benzodiazepin – 2 – one;

1,3 – dihydro – 5 – (2 – fluorophenyl) – 3(R) – (3' – indolyl) methyl – 1 – (3 – methylbutyl) – 2H – 1,4 – benzodiazepine – 2 – one;

1,3 – dihydro – 5 – (2 – fluorophenyl) – 3(R) – (3' – indolyl)methyl – 1 – (2,2,2 – trifluoroethyl) – 2H – 1,4 – benzodiazepin – 2 – one;

1,3 – dihydro – 1 – (2 – dimethylaminoethyl) – 5 – (2 – fluorophenyl) – 3(R) – (3' – indolyl)methyl – 2H – 1,4 – benzodiazepin – 2 – one;

1,3 – dihydro – 1 – (ethoxycarbonylmethyl) – 5 – (2 – fluorophenyl) – 3(R) – (3' – indolyl)methyl – 2H – 1,4 – benzodiazepin – 2 – one;

1 – carboxymethyl – 1,3 – dihydro – 5 – (2 – fluorophenyl) – 3(R) – 3' – indolyl)methyl – 2H – 1,4 – benzodiazepin – 2 – one; 1,3 – dihydro – 5 – (2 – fluorophenyl) – 3(R) – [3' – (1' – p – chloro benzyloylindolyl)methyl] – 1 – methyl – 2H – 1,4 – benzodiazepin – 2 – one;

7 – chloro – 1,3 – dihydro – 3(R) – [3'(1' – benzylindolyl)methyl] – 1 – methyl – 5 – phenyl – 2H – 1,4 – benzodiazepin – 2 – one;

1,3 – dihydro – 3(RS) – [1 – hydroxy – 1 – (3' – indolyl)]methyl – 1 – methyl – 5 – phenyl – 2H – 1,4 – benzodiazepin – 2 – one;

1,3 – dihydro – 1 – methyl – 5 – phenyl – 3 – (RS) – (3 – thienoyl) – 2H – 1,4 – benzodiazepin – 2 – one;

1,3 – dihydro – 3 – (RS) – [1 – hydroxy – 1 – (3 – thienyl)]methyl – 1 – methyl – 5 – phenyl – 2H – 1,4 – benzodiazepin – 2 – one;

1,3 – dihydro – 3(RS) – [1 – hydroxy – 1 – [3 – (1 – methylindolyl)]] – methyl – 1 – methyl – 5 – phenyl – 2H – 1,4 – benzodiazepin – 2 – one, two stereoisomers;

1,3 – dihydro – 3(RS) – (1 – hydroxy – 1 – phenyl)methyl – 1 – methyl – 5 – phenyl – 2H – 1,4 – benzodiazepin – 2 – one;

1,3 – dihydro – 3(RS) – [1 – hydroxy – 1 – (2 – thienyl)]methyl – 1 – methyl – 5 – phenyl – 2H – 1,4 – benzodiazepin – 2 – one;

1,3 – dihydro – 3 – (RS) – hydroxy – 1 – methyl – 5 – phenyl – 3 – (3' – thienoyl) – 2H – 1,4 – benzodiazepin – 2 – one;

1,5 – dihydro – 5 – (RS) – hydroxy – 1 – methyl – 5 – phenyl – 3 – (3' – thienoyl) – 2H – 1,4 – benzodiazepin – 2 – one;

7 – chloro – 1,3 – dihydro – 3(R) – [(2',3' – dihydro – 2' – oxo – 1'H – indol – 3' – yl)methyl] – 5 – phenyl – 2H – 1,4 – benzodiazepin – 2 – one;

7 – chloro – 1,3 – dihydro – 3(R) – [(3' – (2,4 – dinitrophenyl)imidazol – 5' – yl) – methyl] – 5 – phenyl – 2H – 1,4 – benzodiazepin – 2 – one;

7 – chloro – 1,3 – dihydro – 3(R) – (3' – imidazol – 5' – yl)methyl – 5 – phenyl – 2H – 1,4 – benzodiazepin – 2 – one;

3(RS) – [3' – (5' – Bromoindolyl)methyl] – 1,3 – dihydro – 5 – phenyl – 2H – 1,4 – benzodiazepin – 2 – one;

5 – o – carboxyphenyl – 1,3 – dihydro – 3(R) – (3' – indolyl)methyl – 2H – 1,4 – benzodiazepin – 2 – one;

1,3 – dihydro – 3(RS) – [3' – (5' – fluoroindolyl)methyl] – 5 – o – fluorophenyl – 2H – 1,4 – benzodiazepin – 2 – one;

1,3 – dihydro – 3(RS) – [3' – (6' – fluoroindolyl)methyl] – 5 – o – fluorophenyl – 2H – 1,4 – benzodiazepin – 2 – one;

2 – N – [2(RS),3 – bis – (Bocamino)propanoyl]amino – 2' – fluorobenzophenone;

2 – N – [2(RS),3 – diphthalylaminopropanoyl]amino – 2' – fluorobenzophenone;

1,3 – dihydro – 5 – (2' – fluorophenyl) – 3(RS) – aminomethyl – 2H – 1,4 – benzodiazepin – 2 – one;

1,3 – dihydro – 5 – (2' – fluorophenyl) – 3(R) – (4 – amino)butyl – 2H – 1,4 – benzodiazepin – 2 – one;

1,3 – dihydro – 5 – (2' – fluorophenyl) – 3(RS) – benzyloxycarbonylaminomethyl – 2H – 1,4 – benzodiazepin – 2 – one;

1,3 – dihydro – 5 – (2' – fluorophenyl) – 3(RS) – (3 – thiophenecarbonyl)aminomethyl – 2H – 1,4 – benzodiazepin – 2 – one;

1,3 – dihydro – 5 – (2' – fluorophenyl) – 3(RS) – (2 – indole)carbonylaminomethyl) – 2H – 1,4 – benzodiazepin – 2 – one;

1,3 – dihydro – 5 – (2' – fluorophenyl) – 3(RS) – (6' – chloropyrazinyl)aminomethyl – 2H – 1,4 – benzodiazepin – 2 – one;

1,3 – dihydro – 5 – (2' – fluorophenyl) – 3(RS) – aminomethyl – 1 – methyl – 2H – 1,4 – benzodiazepin – 2 – one;

3(RS) – (2 – indolecarbonylamino) – 1,3 – dihydro – 5 – phenyl – 2H – 1,4 – benzodiazepin – 2 – one;

1,3 – Dihydro – 3(RS) – [2 – (3 – indolyl)ethyl]amino – 5 – phenyl – 2H – 1,4 – benzodiazepin – 2 – one;

3(RS) – [3 – (3 – indole)propionylamino] – 1,3 – dihydro – 5 – phenyl – 2H – 1,4 – benzodiazepin – 2 – one;

3(RS) – (3 – indoleacetylamino) – 1,3 – dihydro – 5 – phenyl – 2H – 1,4 – benzodiazepin – 2 – one;

3(RS) – (Boc – L – tryptophanyl)amino – 1,3 – dihydro – 5 – phenyl – 2H – 1,4 – benzodiazepin – 2 – one;

Z – 1,3 – dihydro – 1 – methyl – 5 – phenyl – 3 – (3 – thienylmethylene) – 2H – 1,4 – benzodiazepin – 2 – one;

E – 1,3 – dihydro – 1 – methyl – 5 – phenyl – 3 – (3 – thienylmethylene) – 2H – 1,4 – benzodiazepin – 2 – one;

3(RS) – (BOC – D – tryptophyl)amino – 1,3 – dihydro – 5 – phenyl – 2H – 1,4 – benzodiazepin – 2 – one;

3(RS) – [4 – (3 – indole)butyrylamino] – 1,3 – dihydro – 5 – phenyl – 2H – 1,4 – benzodiazepin – 2 – one;

1,3 – dihydro – 3(RS) – (benzyloxycarbonyl)aminomethyl – 5 – (2 – fluorophenyl) – 2H – 1,4 – benzodiazepine;

1,3 – dihydro – 3(RS) – [3' – (thiophene)carbonyl]aminomethyl – 5 – (2 – fluorophenyl) – 2H – 1,4 – benzodiazepine;

1,3 – dihydro – 3(RS) – (2 – indolecarbonyl)aminomethyl – 5 – (2 – fluorophenyl) – 2H – 1,4 – benzodiazepine;

1,3 – dihydro – 3(RS) – (2 – L – hydroxy – 2 – phenylacetyl)aminomethyl – 5 – (2 – fluorophenyl) – 2H – 1,4 – benzodiazepine;

1 – (2 – cyanoethyl) – 1,3 – dihydro – 5 – (2 – fluorophenyl) – 3(R) – (3' – indolyl)methyl – 2H – 1,4 – benzodiazepin – 2 – one;

1 – (2 – cyanoethyl) – 1,3 – dihydro – 5 – (2 – fluorophenyl) – 3(R) – [1' – (2 – cyanoethyl) – 3' – indolyl] – methyl – 2H – 1,4 – benzodiazpin – 2 – one;

1 – (2 – carboxyethyl) – 1,3 – dihydro – 5 – (2 – fluorophenyl) – 3(R) – (3' – indolyl)methyl – 2H – 1,4 – benzodiazepin – 2 – one;

1,3 – dihydro – 3(R) – (3' – indolyl)methyl – 5 – (2 – fluorophenyl) – 2H – 1,4 – benzodiazepin – 2 – one – 4 – oxide;

1,3 – dihydro – 5 – (2 – fluorophenyl) – 3 – (RS) – (2 – indolecarbonylamino) – 2H – 1,4 – benzodiazepin – 2 – one;

1,3 – dihydro – 5 – (2 – fluorophenyl) – 3(RS) – (2 – indolecarbonylamino) – 1 – methyl – 2H – 1,4 – benzodiazepin – 2 – one;

1,3 – dihydro – 5 – (2 – fluorophenyl) – 1 – methyl – 3(RS) – [2' – (1 – methylindole)carbonylamino] – 2H – 1,4 – benzodiazepin – 2 – one;

1,3 – dihydro – 3(RS) – (4 – nitrophenylcarbonyl)amino – 5 – (2 – fluorophenyl) – 2H – 1,4 – benzodiazepin – 2 – one;

1,3 – dihydro – 3(RS) – (2 – indolecarbonyloxy) – 5 – phenyl – 2H – 1,4 – benzodiazepin – 2 – one;

1,3 – dihydro – 5 – (2 – fluorophenyl) – 3(RS) – (3 – thiophene carbonylamino) – 2H – 1,4 – benzodiazepin – 2 – one;

1,3 – dihydro – 3(RS) – (3 – indolecarbonylamino) – 5 – phenyl – 2H – 1,4 – benzodiazepin – 2 – one;

1,3 – dihydro – 3(RS) – (4 – thianaphtheneacetyl)amino – 5 – (2 – fluorophenyl) – 2H – 1,4 – benzodiazepin – 2 – one;

1,3 – dihydro – 3(RS) – (4 – chlorophenylcarbonyl)amino – 5 – (2 – fluorophenyl) – 2H – 1,4 – benzodiazepin – 2 – one;

1,3 – dihydro – 3(RS) – (4 – methylphenylsulfonyl)amino – 5 – (2 – fluorophenyl) – 2H – 1,4 – benzodiazepin – 2 – one;

1 – carboxymethyl – 1,3 – dihydro – 5 – (2 – fluorophenyl) – 3(RS) – (2 – indolecarbonylamino) – 2H – 1,4 – benzodiazepin – 2 – one;

1,3 – Dihydro – 3(RS) – (5 – fluoroindole – 2 – carbonylamino) – 5 – (2 – fluorophenyl) – 2H – 1,4 – benzodiazepin – 2 – one;

1,3 – dihydro – 3(RS) – (3' – methylindenyl – 2 – carbonyl)amino – 5 – (2 – fluorophenyl) – 2H – 1,4 – benzodiazepin – 2 – one;

1,3 – dihydro – 3(RS) – (2 – quinaldyl)amino – 5 – (2 – fluorophenyl) – 2H – 1,4 – benzodiazepin – 2 –

one;

1,3 – dihydro – 3(RS) – (2 – L – hydroxy – 2 – phenylacetyl)amino – 5 – (2 – fluorophenyl) – 2H – 1,4 – benzodiazepin – 2 – one;

1,3 – Dihydro – 3(RS) – (5 – chloroindole – 2 – carbonylamino) – 5 – (2 – fluorophenyl) – 2H – 1,4 – benzodiazepin – 2 – one;

3(RS) – [N – (2 – indolecarbonyl) – N – methylamino] – 1,3 – dihydro – 5 – phenyl – 2H – 1,4 – benzodiazepin – 2 – one;

1,3 – dihydro – 3(RS) – (2 – indolecarbonylamino) – 1 – methyl – 5 – phenyl – 2H – 1,4 – benzodiazepin – 2 – one;

1,3 – dihydro – 1 – methyl – 3(RS) – [2 – (1 – methylindole)carbonylamino] – 5 – phenyl – 2H – 1,4 – benzodiazepin – 2 – one;

1 – carboxymethyl – 1,3 – dihydro – 3(RS) – (2 – indolecarbonylamino) – 5 – phenyl – 2H – 1,4 – benzodiazepin – 2 – one;

1,3 – Dihydro – 3(RS) – (5 – bromoindole – 2 – carbonylamino) – 5 – (2 – fluorophenyl) – 2H – 1,4 – benzodiazepin – 2 – one;

3(RS) – cinnamoylamino – 1,3 – dihydro – 5 – (2 – fluorophenyl) – 2H – 1,4 – benzodiazepin – 2 – one;

1,3 – dihydro – 3(RS) – (5 – hydroxy – 2 – indolylcarbonyl)amino – 5 – (2 – fluorophenyl) – 2H – 1,4 – benzodiazepin – 2 – one;

1 – carboxamidomethyl – 1,3 – dihydro – 3R – (3 – indolylmethyl) – 5 – (2 – fluorophenyl) – 2H – 1,4 – benzodiazepin – 2 – one;

1,3 – dihydro – 5 – (2 – fluorophenyl) – 3 – (RS) – (2 – indolylmethylamino) – 2H – 1,4 – benzodiazepin – 2 – one;

1,3 – dihydro – 3(RS) – (phenylaminomethylcarbonyl)amino – 5 – (2 – fluorophenyl) – 2H – 1,4 – benzodiazepin – 2 – one;

1,3 – Dihydro – 3(RS) – (5 – methoxyindole – 2 – carbonylamino) – 5 – (2 – fluorophenyl) – 2H – 1,4 – benzodiazepin – 2 – one;

1,3 – Dihydro – 3(RS) – (1 – methylindole – 2 – carbonylamino) – 5 – (2 – fluorophenyl) – 2H – 1,4 – benzodiazepine – 2 – one;

1,3 – dihydro – 1 – methyl – 3(RS) – (4 – chlorophenylcarbonyl)amino – 5 – (2 – fluorophenyl) – 2H – 1,4 – benzodiazepin – 2 – one;

1,3 – dihydro – 5 – (2 – fluorophenyl) – 3(RS) – (2 – benzofurancarbonylamino) – 2H – 1,4 – benzodiazepin – 2 – one;

1 – ethoxycarbonylmethyl – 1,3 – dihydro – 3(RS) – (4 – chlorophenylcarbonyl)amino – 5 – (2 – fluorophenyl) – 2H – 1,4 – benzodiazepin – 2 – one;

1,3 – dihydro – 3(RS) – (4 – chlorophenylcarbonyl)amino – 5 – phenyl – 2H – 1,4 – benzodiazepine – 2 – one;

1,3 – dihydro – 1 – methyl – 3(RS) – (4 – chlorophenylcarbonyl)amino – 5 – phenyl – 2H – 1,4 – benzodiazepin – 2 – one;

1 – carboxymethyl – 1,3 – dihydro – 3(RS) – (4 – chlorophenylcarbonyl)amino – 5 – (2 – fluorophenyl) – 2H – 1,4 – benzodiazepin – 2 – one;

3(RS) – (2(S) – tert – Butoxycarbonylamino – 3 – phenylpropanoylamino) – 1,3 – dihydro – 5 – phenyl – 2H – 1,4 – benzodiazepin – 2 – one;

3(RS) – (2(S) – tert – Butoxycarbonylamino – 3 – phenylpropanoylamino) – 1,3 – dihydro – 1 – methyl – 5 – phenyl – 2H – 1,4 – benzodiazepin – 2 – one;

3(R) – and 3(S) – (2(S) – Amino – 3 – phenylpropanoylamino) – 1,3 – dihydro – 1 – methyl – 5 – phenyl – 2H – 1,4 – benzodiazepin – 2 – one;

3(R) – and 3(S) – Amino – 1,3 – dihydro – 1 – methyl – 5 – phenyl – 2H – 1,4 – benzodiazepin – 2 – one;

3(R) – and 3(S) – Amino – 1,3 – dihydro – 5 – (2 – fluorophenyl) – 1 – methyl – 2H – 1,4 – benzodiazepin – 2 – one;

3(S) – ( – ) – 1,3 – Dihydro – 3 – (2 – indolecarbonylamino) – 1 – methyl – 5 – phenyl – 2H – 1,4 – benzodiazepin – 2 – one;

3(S) – ( + ) – 1,3 – Dihydro – 5 – (2 – fluorophenyl) – 3 – (2 – indolecarbonylamino) – 1 – methyl – 2H – 1,4 – benzodiazepin – 2 – one;

3(R) – ( – ) – 1,3 – Dihydro – 5 – (2 – fluorophenyl) – 3 – (2 – indolecarbonylamino) – 1 – methyl – 2H – 1,4 – benzodiazepin – 2 – one;

3(R) – ( – ) – 1,3 – Dihydro – 3 – (4 – chlorobenzoylamino) – 5 – (2 – fluorophenyl) – 1 – methyl – 2H – 1,4 – benzodiazepin – 2 – one;

3(S) – ( + ) – 1,3 – Dihydro – 3 – (4 – chlorobenzoylamino) – 5 – (2 – fluorophenyl) – 1 – methyl – 2H –

1,4 – benzodiazepin – 2 – one;

3(S) – ( – ) – 1,3 – Dihydro – 3 – (4 – bromobenzoylamino) – 1 – methyl – 5 – phenyl – 2H – 1,4 – benzodiazepin – 2 – one;

3(R) – ( + ) – 1,3 – Dihydro – 3 – (4 – bromobenzoylamino) – 1 – methyl – 5 – phenyl – 2H – 1,4 – benzodiazepin – 2 – one;

3(R) – ( + ) – 1,3 – Dihydro – 3 – (2 – indolecarbonylamino) – 1 – methyl – 5 – phenyl – 2H – 1,4 – benzodiazepin – 2 – one;

1,3 – Dihydro – 3 – (RS) – (2 – indolinecarbonylamino) – 5 – phenyl – 2H – 1,4 – benzodiazepin – 2 – one;

1,3 – Dihydro – 5 – (2 – fluorophenyl) – 3 – (RS) – (p – trifluoromethylbenzoylamino) – 2H – 1,4 – benzodiazepin – 2 – one;

1,3 – Dihydro – 5 – (2 – fluorophenyl) – 3 – (RS) – (p – methylbenzoylamino) – 2H – 1,4 – benzodiazepin – 2 – one;

1,3 – Dihydro – 5 – (2 – fluorophenyl) – 3 – (RS) – (p – methoxybenzoylamino) – 2H – 1,4 – benzodiazepin – 2 – one;

3 – (RS) – (o – Chlorobenzoylamino) – 1,3 – dihydro – 5 – phenyl – 2H – 1,4 – benzodiazepin – 2 – one;

3 – (RS) – (o – Chlorobenzoylmethylamino – 1,3 – dihydro – 1 – methyl – 5 – phenyl – 2H – 1,4 – benzodiazepin – 2 – one;

3 – (RS) – (o – Chlorobenzoylamino) – 1,3 – dihydro – 1 – methyl – 5 – phenyl – 2H – 1,4 – benzodiazepin – 2 – one;

3 – (RS) – (m – Chlorobenzoylamino) – 1,3 – dihydro – 5 – phenyl – 2H – 1,4 – benzodiazepin – 2 – one;

3 – (RS) – (3,4 – Dichlorobenzoylamino) – 1,3 – dihydro – 5 – phenyl – 2H – 1,4 – benzodiazepin – 2 – one;

3 – (RS) – (p – Chlorobenzoylamino)1,3 – dihydro – 5 – (2' – fluorophenyl) – 1 – methyl – 4 – oxo – 2H – 1,4 – benzodiazepin – 2 – one;

1,3 – Dihydro – 5 – Phenyl – 3 – (RS) – (4' – methylthiobenzoylamino) – 2H – 1,4 – benzodiazepin – 2 – one;

1 – 3 – Dihydro – 3 – (RS) – (4' – Fluorobenzoylamino) – 5 – phenyl – 2H – 1,4 – benzodiazepin – 2 – one;

1,3 – Dihydro – 5 – Phenyl – 3 – (RS) – (4' – trifluoromethylbenzoylamino) – 2H – 1,4 – benzodiazepin – 2 – one;

1,3 – Dihydro – 3 – (RS) – (4' – tert – Butylbenzoylamino) – 5 – phenyl – 2H – 1,4 – benzodiazepin – 2 – one;

3 – (RS) – (3,5 – Dichlorobenzoylamino)1,3 – dihydro – 5 – phenyl – 2H – 1,4 – benzodiazepin – 2 – one;

1 – 3 – Dihydro – 3 – (RS) – (p – Hydroxybenzoylamino) – 5 – phenyl – 2H – 1,4 – benzodiazepin – 2 – one;

3 – (RS) – (4' – Cyanobenzoylamino)1,3 – dihydro – 5 – phenyl – 2H – 1,4 – benzodiazepin – 2 – one;

3(S) – ( – ) – 3 – (2 – Chlorobenzoylamino) – 1,3 – dihydro – 1 – methyl – 5 – phenyl – 2H – 1,4 – benzodiazepin – 2 – one;

3(R) – ( + ) – 3 – (2 – Chlorobenzoylamino) – 1,3 – dihydro – 1 – methyl – 5 – phenyl – 2H – 1,4 – benzodiazepin – 2 – one;

1,3 – Dihydro – 3(RS) – (p – dimethylaminobenzoylamino) – 5 – (2 – fluorophenyl) – 2H – 1,4 – benzodiazepin – 2 – one;

1,3 – Dihydro – 3(RS) – (3,4 – dimethoxybenzoylamino) – 5 – (2 – fluorophenyl) – 2H – 1,4 – benzodiazepin – 2 – one;

3(S) – ( + ) – 3 – (3 – Bromobenzoylamino) – 1,3 – dihydro – 5 – (2 – fluorophenyl) – 1 – methyl – 2H – 1,4 – benzodiazepin – 2 – one;

1,3 – Dihydro – 5 – phenyl – 3(RS) – (3 – trifluoromethylthiobenzoylamino) – 2H – 1,4 – benzodiazepin – 2 – one;

3(S) – ( + ) – 3 – (4 – Bromobenzoylamino) – 1,3 – dihydro – 5 – (2 – fluorophenyl) – 1 – methyl – 2H – 1,4 – benzodiazepin – 2 – one;

3(S) – ( + ) – 3 – (4 – t – Butylbenzoylamino) – 1,3 – dihydro – 5 – (2 – fluorophenyl) – 1 – methyl – 2H – 1,4 – benzodiazepin – 2 – one;

1,3 – Dihydro – 5 – (2 – fluorophenyl) – 3(RS) – (pyrrole – 2 – carbonylamino) – 2H – 1,4 – benzodiazepin – 2 – one;

3(S) – ( + ) – 1,3 – Dihydro – 5 – (2 – fluorophenyl) – 3 – (4 – iodobenzoylamino) – 1 – methyl – 2H – 1,4 – benzodiazepin – 2 – one;

1,3 – Dihydro – 3(RS) – (2 – naphthoylamino) – 5 – phenyl – 2H – 1,4 – benzodiazepin – 2 – one;

3(S)-(-)-3-(2-Bromobenzoylamino)-1,3-dihydro-5-(2-fluorophenyl)-1-methyl-2H-1,4-benzodiazepin-2-one;
3(S)-(+)-3-(4-Cyanobenzoylamino)-1,3-dihydro-5-(2-fluorophenyl)-1-methyl-2H-1,4-benzodiazepin-2-one;
1,3-Dihydro-5-phenyl-3(RS)-(4-n-propylbenzoylamino)-2H-1,4-benzodiazepin-2-one;
1,3-Dihydro-5-phenyl-3(RS)-(4-phenylbenzoylamino)-2H-1,4-benzodiazepin-2-one;
1,3-Dihydco-3(RS)-(4-n-pentylbenzoylamino)-5-phenyl-2H-1,4-benzodiazepin-2-one;
1,3-Dihydro-3(RS)-(1-naphthoylamino)-5-phenyl-2H-1,4-benzodiazepin-2-one;
3(S)-(+)-1,3-Dihydro-5-(2-fluorophenyl)-3-(3-iodobenzoylamino)-1-methyl-2H-1,4-benzodiazepin-2-one;
3(R)-(-)-1,3-Dihydro-5-(2-fluorophenyl)-3-(3-iodobenzoylamino)-1-methyl-2H-1,4-benzodiazepin-2-one;
3(R)-(+)-1,3-Dihydro-5-(2-fluorophenyl)-3-(2-iodobenzoylamino)-1-methyl-2H-1,4-benzodiazepin-2-one;
3(S)-(-)-1,3-Dihydro-5-(2-fluorophenyl)-3-(2-iodobenzoylamino)-1-methyl-2H-1,4-benzodiazepin-2-one;
3(R)-(+)-3-(2-Bromobenzoylamino)-1,3-dihydro-5-(2-fluorophenyl)-1-methyl-2H-1,4-benzodiazepin-2-one;
3(R)-(+)-3-(2-Chlorobenzoylamino)-1,3-dihydro-5-(2-fluorophenyl)-1-methyl-2H-1,4-benzodiazepin-2-one;
1,3-Dihydro-5-(2-fluorophenyl)-3(RS)-phenylcarbonylamino-2H-1,4-benzodiazepin-2-one;
1,3-Dihydro-5-(2-fluorophenyl)-3(RS)-(2-chlorophenyl)carbonylamino-2H-1,4-benzodiazepin-2-one;
1,3-Dihydro-5-(2-fluorophenyl)-3(RS)-benzyloxycarbonylamino-2H-1,4-benzodiazepin-2-one;
1,3-Dihydro-5-(2-fluorophenyl)-3-(RS)-benzyloxycarbonylamino-2H-1,4-benzodiazepin-2-thione;
1-(4-Chlorophenyl)carbonyl-1,3-dihydro-5-(2-fluorophenyl)-3(RS)-(4-chlorophenyl)-carbonylamino-2H-1,4-benzodiazepin-2-one;
1-(4-Chlorophenyl)carbonyl-1,3-dihydro-5-(2-fluorophenyl)-3(RS)-(4-chlorophenyl)-carbonyloxy-2H-1,4-benzodiazepin-2-one;
1,3-Dihydro-5-(2-fluorophenyl)-3(RS)-(4-chlorophenyl)oxy-2H-1,4-benzodiazepin-2-one;
1,3-Dihydro-5-(2-fluorophenyl)-3-(RS)-(4-chlorophenyl)carbonylamino-2H-1,4-benzodiazepin-2-thione;
1,3-Dihydro-5-(2-fluorophenyl)-3-(RS)-(2-indole) carbonylamino-2H-1,4-benzodiazepin-2-thione or
1,3-Dihydro-3(RS)-(4-chlorophenyl)aminocarbonylamino-2H-1,4-benzodiazepin-2-one.

**6.** Use according to claim 1, characterized in that the compound of formula I is:
(S)-(-)-1,3-dihydro-3-(4-bromobenzoylamino)-1-methyl-5-phenyl-2H-1,4-benzodiazepin-2-one;
3(RS)-(2-indolecarbonylamino)-1,3-dihydro-5-phenyl-2H-1,4-benzodiazepin-2-one;
1,3-Dihydro-3(RS)-(2-indolecarbonylamino)-1-methyl-5-phenyl-2H-1,4-benzodiazepin-2-one;
1,3-dihydro-1-methyl-3(RS)-[2-(1-methylindole) carbonylamino]-5-phenyl-2H-1,4-benzodiazepin-2-one;
1,3-Dihydro-1-methyl-3(RS)-(4-chlorophenylcarbonyl)amino-5-(2-fluorophenyl)-2H-1,4-benzodiazepin-2-one;
1,3-Dihydro-5-(2-fluorophenyl)-3(RS)-(2-indolecarbonylamino)-1-methyl-2H-1,4-benzodiazepin-2-one;
1,3-Dihydro-5-(2-fluorophenyl)-1-methyl-3(RS)-[2'-(1-methylindole)carbonylamino]-2H-1,4-benzodiazepin-2-one;
3(R)-(-)-1,3-Dihydro-3-(4-chlorobenzoylamino)-5-(2-fluorophenyl)-1-methyl-2H-1,4-benzodiazepin-2-one;
3(S)-(+)-1,3-Dihydro-3-(4-chlorobenzoylamino)-5-(2-fluorophenyl)-1-methyl-2H-1,4-benzodiazepin-2-one;

3(R) – ( + ) – 1,3 – Dihydro – 3 – (4 – bromobenzoylamino) – 1 – methyl – 5 – phenyl – 2H – 1,4 – benzodiazepin – 2 – one;

3(R) – ( + ) – 1,3 – Dihydro – 3 – (2 – indolecarbonylamino) – 1 – methyl – 5 – phenyl – 2H – 1,4 – benzodiazepin – 2 – one;

1,3 – Dihydro – 5 – (2 – fluorophenyl) – 3 – (RS) – (2 – indolecarbonylamino) – 2H – 1,4 – benzodiazepin – 2 – one;

1,3 – Dihydro – 3 – (RS) – (2 – indolecarbonyloxy) – 5 – phenyl – 2H – 1,4 – benzodiazepin – 2 – one;

1,3 – Dihydro – 3 – (RS) – (4 – chlorophenylcarbonyl)amino – 5 – (2 – fluorophenyl) – 2H – 1,4 – benzodiazepin – 2 – one;

1 – Carboxymethyl – 1,3 – dihydro – 5 – (2 – fluorophenyl) – 3(RS) – (2 – indolecarbonylamino) – 2H – 1,4 – benzodiazepin – 2 – one;

1,3 – Dihydro – 3 – (RS) – (5 – fluoroindole – 2 – carbonylamino) – 5 – (2 – fluorophenyl) – 2H – 1,4 – benzodiazepin – 2 – one;

3 – (RS) – Cinnamoylamino – 1,3 – dihydro – 5 – (2 – fluorophenyl) – 2H – 1,4 – benzodiazepin – 2 – one;

1,3 – Dihydro – 5 – (2 – fluorophenyl) – 3 – (RS) – (2 – benzofurancarbonylamino) – 2H – 1,4 – benzodiazepin – 2 – one;

1,3 – Dihydro – 1 – methyl – 3 – (RS) – (4 – chlorophenylcarbonyl)amino – 5 – phenyl – 2H – 1,4 – benzodiazepin – 2 – one;

1 – Carboxymethyl – 1,3 – dihydro – 3 – (RS) – (4 – chlorophenylcarbonyl)amino – 5 – (2 – fluorophenyl) – 2H – 1,4 – benzodiazepin – 2 – one;

1,3 – Dihydro – 5 – (2 – fluorophenyl) – 3 – (RS) – (p – trifluoromethylbenzoylamino) – 2H – 1,4 – benzodiazepin – 2 – one;

3(S) – ( + ) – 3 – (3 – Bromobenzoylamino) – 1,3 – dihydro – 5 – (2 – fluorophenyl) – 1 – methyl – 2H – 1,4 – benzodiazepin – 2 – one;

3(S) – ( + ) – 3 – (4 – Bromobenzoylamino) – 1,3 – dihydro – 5 – (2 – fluorophenyl) – 1 – methyl – 2H – 1,4 – benzodiazepin – 2 – one;

3(S) – ( + ) – 1,3 – Dihydro – 5 – (2 – fluorophenyl) – 3 – (4 – iodobenzoylamino) – 1 – methyl – 2H – 1,4 – benzodiazepin – 2 – one;

1,3 – Dihydro – 3(RS) – (2 – naphthoylamino) – 5 – phenyl – 2H – 1,4 – benzodiazepin – 2 – one;

3(S) – ( + ) – 1,3 – Dihydro – 5 – (2 – fluorophenyl) – 3 – (3 – iodobenzoylamino) – 1 – methyl – 2H – 1,4 – benzodiazepin – 2 – one; or

3(R) – ( – ) – 1,3 – Dihydro – 5 – (2 – fluorophenyl) – 3 – (3 – iodobenzoylamino) – 1 – methyl – 2H – 1,4 – benzodiazepin – 2 – one or

3(R) – ( – ) – 1,3 – Dihydro – 5 – (2 – fluorophenyl) – 3 – (2 – indolecarbonylamino) – 1 – methyl – 2H – 1,4 – benzodiazepin – 2 – one.

**7.** Use according to claim 1, characterized in that the compound of formula I is:

1 – carboxymethyl – 1,3 – dihydro – 3(RS) – (2 – indolecarbonylamino) – 5 – phenyl – 2H – 1,4 – benzodiazepin – 2 – one,

(S) – ( – ) – 1,3 – dihydro – 3 – (2 – indolecarbonylamino) – 1 – methyl – 5 – phenyl – 2H – 1,4 – benzodiazepin – 2 – one

(S) – ( + ) – 1,3 – dihydro – 5 – (2 – fluorophenyl) – 3 – (2 – indolecarbonylamino) – 1 – methyl – 2H – 1,4 – benzodiazepin – 2 – one, or

(S) – ( + ) – 1,3 – dihydro – 3 – (4 – chlorobenzoylamino) – 5 – (2 – fluorophenyl) – 1 – methyl – 2H – 1,4 – benzodiazepin – 2 – one.

**8.** Benzodiazepine derivatives which correspond to the following formula Ib

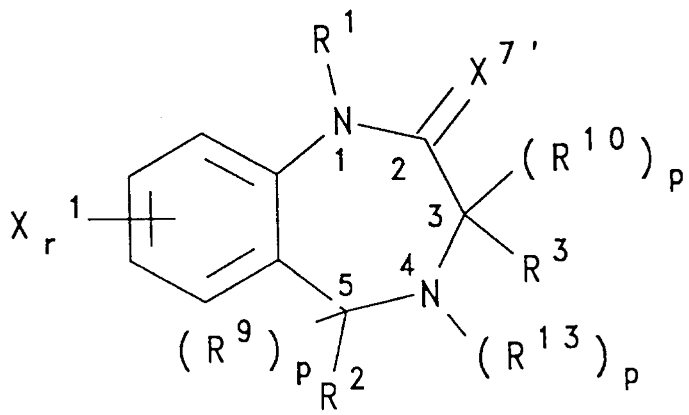

I b

wherein

$X^{7'}$ is S, HH or $NR^{15}$ and

the remaining substituents and symbols have the same meaning as in the compounds of formula I defined in claim 1, with the proviso that $X^{7'}$ can only be $NR^{15}$ when $R^1$ is not H;
or salts or quaternary ammonium salts thereof.

**9.** Benzodiazepine derivative which correspond to the following formula Ic

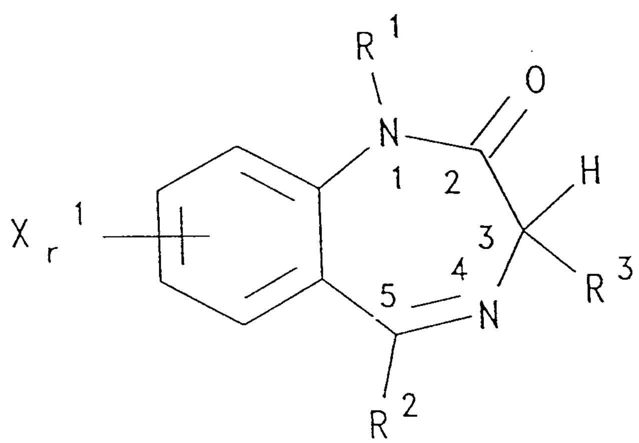

I c

or salts or quaternary ammonium salts of the compounds of formula Ic, in which compounds of formula Ic

$R^1$ is alkyl having 1 − 5 carbon atoms or hydrogen

$R^2$ is unsubstituted phenyl or substituted phenyl having 1 or 2 substituents selected from halo, alkyl having 1 − 4 carbon atoms, alkoxy having 1 − 4 carbon atoms, carboxy substituted alkyl having 1 − 4 carbon atoms in the alkyl group, carboxyl, nitro, trifluoro methyl and hydroxy,

$R^3$ is a group having the formulae

$$-(CH_2)_nNH\overset{O}{\overset{\|}{C}}(CH_2)_qR^7$$

or

$$-(CH_2)_nNH\overset{O}{\overset{\|}{C}}NH(CH_2)_nR^7;$$

wherein

$R^7$ is unsubstituted phenyl or substituted phenyl having 1 or 2 substituents which are selected from halo, nitro, hydroxy, alkyl having 1 – 4 carbon atoms, alkoxy having 1 – 4 carbon atoms, alkylthio having 1 – 4 carbon atoms, trifluoro methyl groups or groups having the formulae

CN, $-C\equiv CH$, $SCF_3$,

$$O\overset{O}{\overset{\|}{C}}CH_3,$$

$OCHF_2$, SPh or $-NR^4R^5$

wherein

$R^4$ and $R^5$ are independently hydrogen or alkyl having 1 – 4 carbon atoms,

or the radical

$R^7$ is a group having the formula

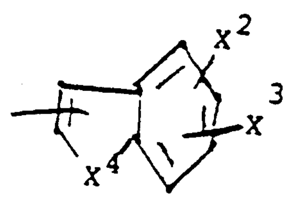

,

wherein

$X^2$ and $X^3$ are independently hydrogen, hydroxy, nitro, halo, alkylthio having 1 – 4 carbon atoms, alkyl having 1 – 4 carbon atoms or alkoxy having 1 – 4 carbon atoms,

$X^4$ is NH

q is O – 4, preferably O and

n is O – 4, preferably O.

**10.** Benzodiazepine derivative according to claim 9, wherein

$R^1$ is methyl,

$R^2$ is unsubstituted phenyl,

$R^3$ is as defined in claim 9 wherein

q and n is 0 and

$R^7$ is as defined in claim 9, wherein, however, in the indolyl group $X^2$ and $X^3$ are hydrogen.

**11.** The compound 3(S)-(-)-1,3-dihydro-3-(2-indolecarbonylamino)-1-methyl-5-phenyl-2H-1,4-benzodiazepin-2-one.

**Claims for the following Contracting State : AT**

**1.** Use of compounds of formula I

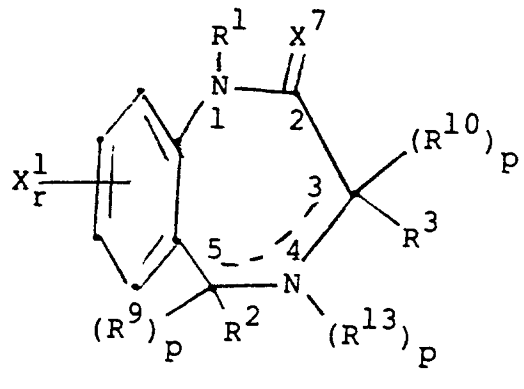

I

or a pharmaceutically acceptable salt or a pharmaceutically acceptable quaternary ammonium salt of said compound of formula I

in which compounds of formula I

$R^1$ is hydrogen, alkyl having 1 − 5 carbon atoms, alkenyl having up to 5 carbon atoms, alkynyl having up to 5 carbon atoms, or a group having the formulae $-(CH_2)_mCOOR^6$, $-(CH_2)_n-C_3-C_5$ − cycloalkyl, $-(CH_2)_mNR^4R^5$, $-(CH_2)_mCONR^4R^5$, $-(CH_2)_mCN$, or $-(CH_2)_nCX^{10}_3$ ;

wherein

$R^4$ and $R^5$ are independently hydrogen, alkyl having 1 − 4 carbon atoms or cycloalkyl having 3 − 5 carbon atoms

$R^6$ is hydrogen, alkyl having 1 − 4 carbon atoms, cycloalkyl having 3 − 5 carbon atoms, unsubstituted phenyl, substituted phenyl, unsubstituted phenyl alkyl or substituted phenyl alkyl in which groups the substituents of the phenyl are 1 or 2 substituents selected from halo, alkyl having 1 − 4 carbon atoms, alkoxy having 1 − 4 carbon atoms, nitro or trifluoro methyl and wherein the alkyl moiety of the corresponding substituted or unsubstituted phenyl alkyl group comprises 1 − 4 carbon atoms,

$X^{10}$ is fluoro, chloro or bromo,

m is 1 − 4 and

n is O − 4;

$R^2$ is hydrogen, alkyl having 1 − 4 carbon atoms, unsubstituted phenyl, substituted phenyl having 1 or 2 substituents selected from halo, alkyl having 1 − 4 carbon atoms, alkoxy having 1 − 4 carbon atoms, alkylthio having 1 − 4 carbon atoms, carboxy substituted alkyl having 1 − 4 carbon atoms in the alkyl group, carboxyl, nitro, trifluoromethyl and hydroxy,

or the radical $R^2$ is a group having the following formulae

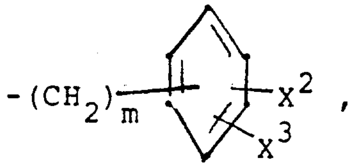

$-(CH_2)_mSCH_3$, $-(CH_2)_mSOCH_3$, $-(CH_2)_mSO_2CH_3$, or $-(CH_2)_mCOOR^6$;

wherein

$R^6$ and m are as defined with regard to the radical $R^1$

$X^2$ and $X^3$ are independently hydrogen, hydroxy, nitro, halo, alkyl having 1 − 4 carbon atoms, alkylthio having 1 − 4 carbon atoms or alkoxy having 1 − 4 carbon atoms,

$R^3$ is a group having the formulae

$-(CH_2)_nR^7$,

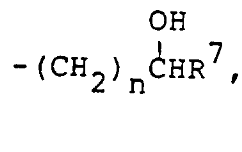

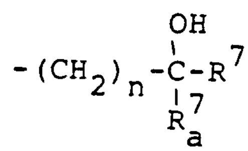

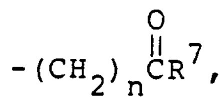

$-(CH_2)_nNR^{18}\ (CH_2)_qR^7$,

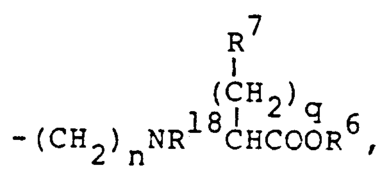

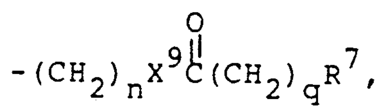

$-NH(CH_2)_{2-3}NHR^7$, $-NH(CH_2)_{2-3}NHCOR^7$,

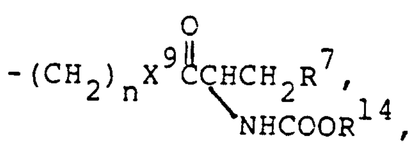

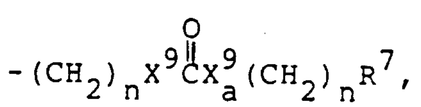

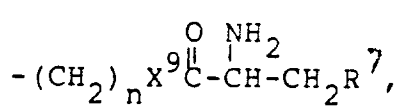

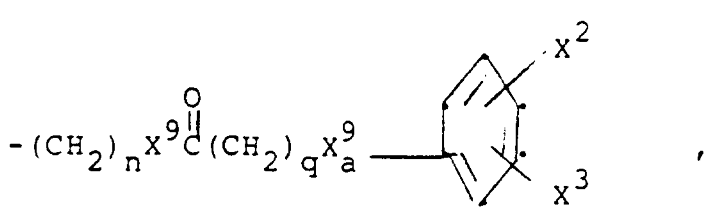

or

$-(CH)_2)_nNR^{18}SO_2(CH_2)_qR^7$

wherein

$R^7$ and $R^7{}_a$ are independently unsubstituted phenyl or substituted phenyl having 1 or 2

substituents which are selected from halo, nitro, hydroxy, alkyl having 1 – 4 carbon atoms, groups having the formulae $CF^3$, CN, $SCF_3$, C≡CH, $CH_2SCF_3$,

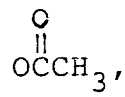

$OCHF_2$, SH, SPh, $PO_3H$, or $-NR^4R^5$,
alkoxy having 1 – 4 carbon atoms or alkylthio having 1 – 4 carbon atoms or the radicals

$R^7$ and $R^7{}_a$ are alpha naphthyl or beta naphthyl or a group having the formulae

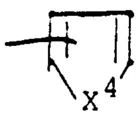

,

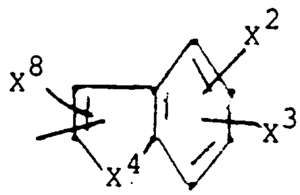

,

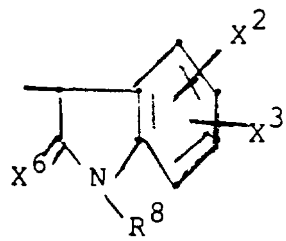

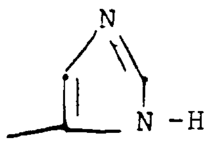,
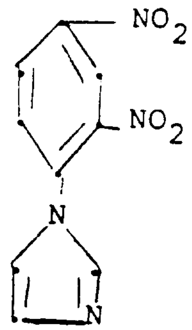,

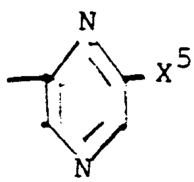,
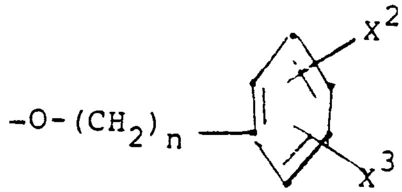,

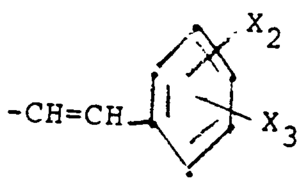,
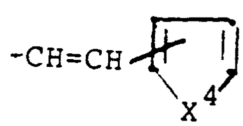

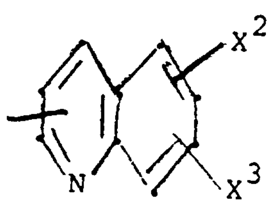
,
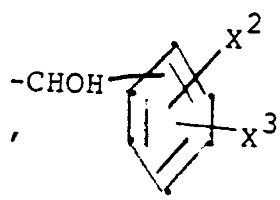, or

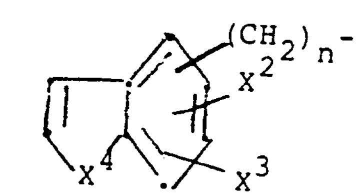

wherein

$R^8$ is hydrogen, alkyl having 1 − 4 carbon atoms, cycloalkyl having 3 − 5 carbon atoms which is bonded either directly or through an alkylene chain comprising 1 − 4 $-CH_2-$ groups or $R^8$ is a group having the formulae $-(CH_2)_mCONH_2$, $-(CH_2)_mCOOR^6$, $-(CH_2)_mNR^4R^5$,

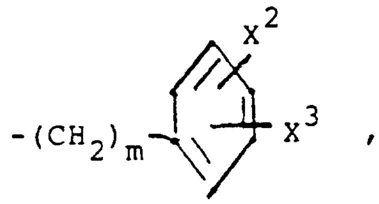

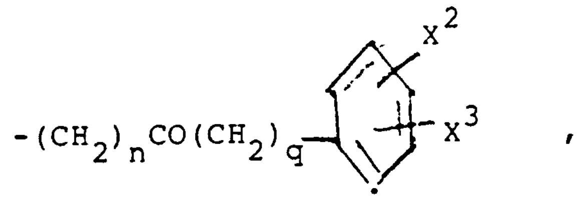

or

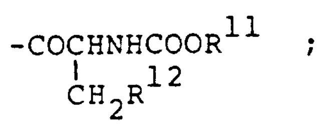

wherein

| | |
|---|---|
| $X^1$ and $X^3$ | are as defined with regard to radical $R^2$, |
| $X^4$ | is S, O, $CH_2$, or $NR^8$, wherein $R^8$ is as defined above, |
| $X^5$ | is hydrogen, halo, nitro or a group of formulae $CF_3$, CN, $-COOR^6$, wherein $R^6$ is as defined above, |
| $X^5$ | is O or HH; |
| $X^5$ | is hydrogen or alkyl having 1 − 4 carbon atoms, |
| $X^5$ and $X^5{}_a$ | are independently groups of formulae −O− or $-NR^{18}-$ wherein |
| $R^{18}$ | is hydrogen, alkyl having 1 − 4 carbon atoms, formyl, acetyl, propionyl or butyryl, |
| $R^{11}$ and $R^{12}$ | are independently alkyl having l − 4 carbon atoms or cycloalkyl having 3 − 5 carbon atoms, |
| $R^{14}$ | is alkyl having 1 − 4 carbon atoms or phenyl alkyl in which the alkyl group has 1 − 4 carbon atoms, |
| $R^9$ and $R^{10}$ | are independently hydrogen, hydroxy or methyl, |
| $R^{13}$ | is hydrogen, alkyl having 1 − 4 carbon atoms, cycloalkyl having 3 − 5 carbon atoms, −O, formyl, acetyl, propionyl or butyryl, |
| $X^1$ | is hydrogen, halo, alkyl having 1 − 4 carbon atoms, alkylthio having 1 − 4 carbon atoms, alkoxy having 1 − 4 carbon atoms or a group having the formulae $-NO_2$, $CF_3$ CN, OH, $-(CH_2)_nCOCR^6$, or $-NR^4R^5$; wherein |
| $R^4$, $R^5$,$R^6$ and n | are as defined in connection with the radical $R^1$, |
| r | is 1 or 2 |
| $X^7$ | is O, S, HH, or $NR^{15}$ wherein |
| $R^{15}$ | is hydrogen, alkyl having 1 − 4 carbon atoms or a group of formula |

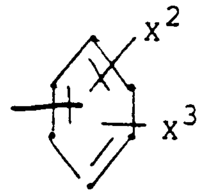

,

or $-NR^{16}R^{17}$;
wherein

$R^{16}$ and $R^{17}$ are independently hydrogen or a group of formula

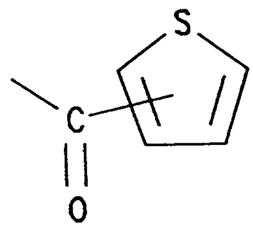

however with the provision that

$X^7$ can be $NR^{15}$ only when $R^1$ is not H;

g is O – 4

- - - indicates a single bond or a double bond,

p is O when its adjacent - - - is a double bond and is 1, when its adjacent - - - is a single bond, except that when $R^{13}$ is oxygen and p is I and - - - is a double bond and

with the provision that if

$R^3$ is a group of formula $-(CH_2)_nNH(CH_2)_qR^7$, wherein

q is O or I, or

is a group of formula

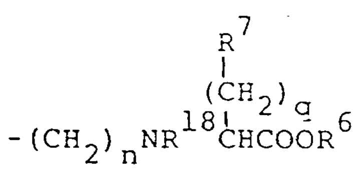

wherein q is O, then $R^7$ has to have another meaning than that of a group of formula

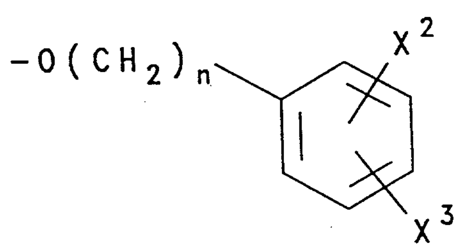

**for the manufacture of a medicament**

for use in antagonizing cholecystokinin in gastrointestinal tissue and for antagonizing gastrin by binding to the corresponding cholecystokinin receptor or gastrin receptor.

**2.** Use according to claim 1, characterized **in that for the manufacture of a medicament** there is used as pharmaceutically active ingredient a compound of formula I or a pharmaceutically acceptable salt or a pharmaceutically acceptable quaternary ammonium salt of said compound of formula I in which compounds of formula I

is hydrogen, alkyl having 1 − 5 carbon atoms
or
a group of formula
$-(CH_2)_mCOOR^6$
wherein

$R^6$ and m — are as defined in claim 1,
is a group of formula
$-(CH_2)_mCOOR^6$
wherein

$R^6$ and m — are as defined above or $R^2$ is unsubstituted phenyl or substituted phenyl having 1 or 2 substituents selected from halo, alkyl having 1 − 4 carbon atoms, alkoxy having 1 − 4 carbon atoms, carboxy substituted alkyl having 1 − 4 carbon atoms in the alkyl group, carboxyl, nitro, trifluoro methyl and hydroxy, is a group having the formulae
$-(CH_2)_nR^7$,

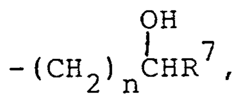

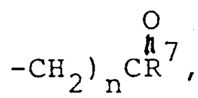

$-(CH_2)_nNH(CH_2)_qR^7$,

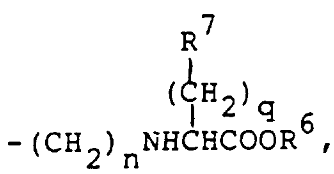 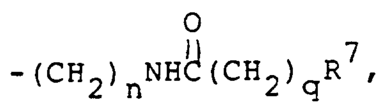

$-NH(CH_2)_{2-3}NHR^7$,

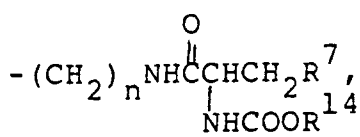

$-NH(CH_2)_{2-3}NHCOR^7$, or

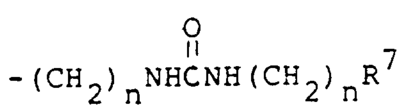

wherein

$R^7$ — is unsubstituted phenyl or substituted phenyl having 1 or 2 substituents which are selected from the group comprising halo, nitro, hydroxy, alkyl having 1 − 4 carbon

atoms, alkoxy having 1 – 4 carbon atoms, alkylthio having 1 – 4 carbon atoms, trifluoro – methyl groups or groups having the formulae
CN, – C$\equiv$CH, $SCF_3$,

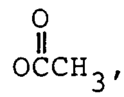

$OCHF_2$, SPh or – $NR^4R^5$ wherein

$R^4$ and $R^5$ are independently hydrogen or alkyl having 1 – 4 carbon atoms, or the radical

$R^7$ is alpha naphthyl or beta naphthyl or a group having the formulae

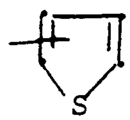
,
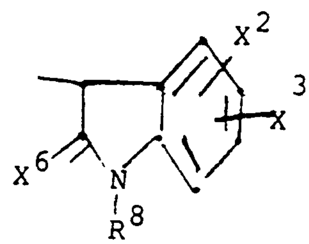
,
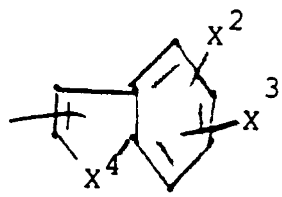
,
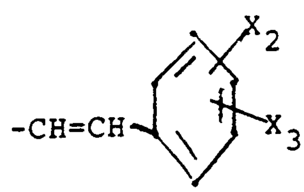
, or
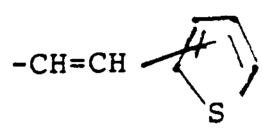

wherein

$R^8$ is hydrogen, alkyl having 1 – 4 carbon atoms or a group of formula

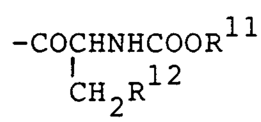

wherein

$R^{11}$ and $R^{12}$ are independently alkyl having 1 – 4 carbon atoms,

$X^2$ and $X^3$ are independently hydrogen, hydroxy, nitro, halo, alkylthio having 1 – 4 carbon atoms, alkyl having 1 – 4 carbon atoms or alkoxy having 1 – 4 carbon atoms,

$X^4$ is S, O, or $NR^8$ wherein $R^8$ is as defined above,

$X^6$ is O or HH;

$R^{14}$ is alkyl having 1 – 4 carbon atoms and

q is 0 – 4,

$R^9$ and $R^{10}$ are independently hydrogen, hydroxy or methyl,

$R^{13}$ is hydrogen, alkyl having l – 4 carbon atoms, – O, formyl, acetyl,propionyl or butyryl,

$X^1$ is hydrogen, halo, alkyl having 1 – 4 carbon atoms, alkylthio having 1 – 4 carbon

atoms, alkoxy having 1 − 4 carbon atoms or a group having the formulae $-NO_2$, $CF_3$, CN, OH, $-(CH_2)_nCOOR^6$, or $-NR^4R^5$; wherein

$R^6$ is as defined in connection with radical $R^1$ and $R^4$ and $R^5$ are as defined in connection with the radical $R^3$,

$X^7$ is O

m is l − 4

n is O − 4

--- indicates a single bond or a double bond,

p is O when the adjacent --- is a double bond and is l, when its adjacent --- is a single bond, except that when $R^{13}$ is oxygen and p is l and --- is a double bond and

r is l or 2.

**3.** Use according to claim 2, characterized **in that for the manufacture of a medicament** there is used as pharmaceutically active ingredient a compound of formula I or a pharmaceutically acceptable salt or a pharmaceutically acceptable quaternary ammonium salt of said compound of formula I in which

$R^1$ is hydrogen, methyl, ethyl, carboxymethyl, or ethylcarboxymethyl;

$R^2$ is a group of formula

$-(CH_2)_{1-2}COOR^6$;

wherein

$R^6$ is hydrogen or alkyl having l − 4 carbon atoms or

$R^2$ is unsubstituted phenyl or substituted phenyl comprising 1 or 2 substituents which are halo or carboxyl,

$R^3$ is a group having the formulae

$-(CH_2)_nR^7$,

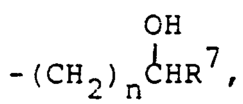

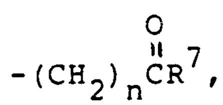

$-(CH_2)_nNH(CH_2)_qR^7$,

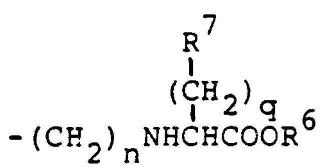

,

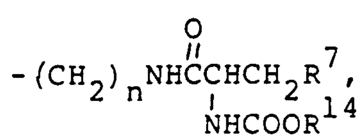

or

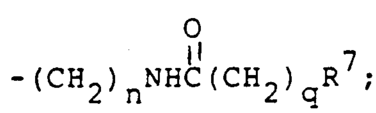

wherein

$R^7$ is alphanaphthyl, betanaphthyl or substituted phenyl having 1 or 2 substituents which

are selected from halo, trifluoromethyl and alkyl having 1 – 4 carbon atoms or

$R^7$ is a group having the formulae

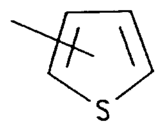

,

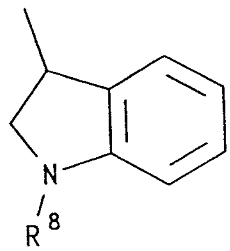

,

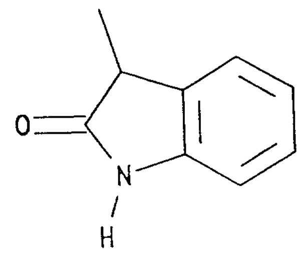

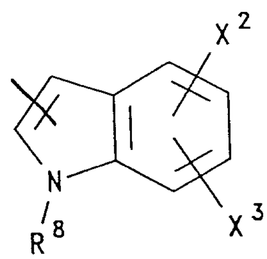

,

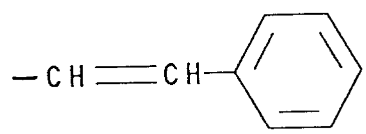

wherein

$R^8$ is hydrogen, methyl or ethyl,

$X^2$ and $X^3$ are independently hydrogen, hydroxy, nitro or halo,

$R^{14}$ is t – butyl;

n is O – 4;

q is O – 4; and

$R^6$ is as defined in connection with the radical $R^2$

$R^9$ and $R^{10}$ are independently hydrogen, hydroxy or methyl,

$R^{13}$ is hydrogen,methyl or formyl,

$X^1$ is hydrogen, halo, or a group having the formulae $-NO_2$, $CF_3$, CN or OH

$X^7$ is O;

– – – indicates a single bond or a double bond,

p is 0 when the adjacent – – – is a double bond and is I when its adjacent – – – is a single bond, and

r is I or 2.

**4.** Use according to claim 3, characterized **in that for the manufacture of a medicament** there is used as pharmaceutically active ingredient a compound of formula I or a pharmaceutically acceptable salt or a pharmaceutically acceptable quaternary ammonium salt of said compound of formula I, in which compounds of formula I

$R^1$ is hydrogen, methyl or carboxymethyl,

$R^2$ is phenyl, o – fluorophenyl, p – fluorophenyl, o – chlorophenyl, p – chlorophenyl, o – carboxyphenyl, 2,4 – dichlorophenyl, 2,6 – difluorophenyl, $-CH_2COOEt$, $-CH_2COO$ – t – Bu, $-CH_2CH_2COOEt$, or $-CH_2CH_2COO$ – t – Bu;

$R^3$ is $-(CH_2)_{1-2}R^7$,

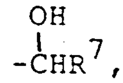

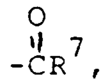

$-(CH_2)_{0-1}NH(CH_2)_{1-2}R^7$,

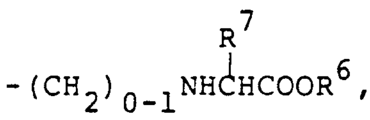 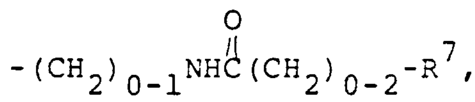

or

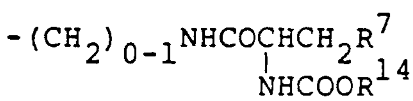 ,

and the stereochemistry relates to D-tryptophan;

R[7] is alphanaphthyl, betanaphthyl, monohalophenyl or dihalophenyl or a group having the formulae

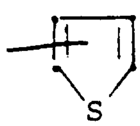 ,

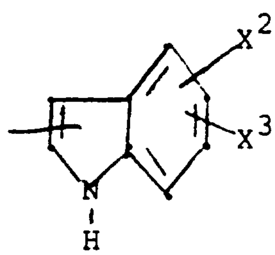 ,

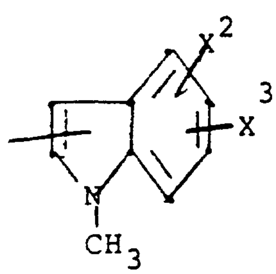 ,

wherein

$X^2$ and $X^3$ are independently hydrogen, hydroxy, fluoro or chloro,

$R^6$ is hydrogen, methyl or ethyl,

| | |
|---|---|
| $R^{14}$ | is t-butyl, |
| $R^9$ and $R^{10}$ | are independently hydrogen or hydroxy, |
| $R^{13}$ | is hydrogen, |
| $X^1$ | is hydrogen, 7-chloro, 7-fluoro, or 7-nitro; |
| $X^7$ | is oxygen, |
| --- | is a single bond or a double bond, |
| p | is O when its adjacent --- is a double bond and is l, when its adjacent --- is a single bond and |
| r | is l. |

**5.** Use according to claim 1, characterized in that the compound of formula I is:

1,3-dihydro-5-(2-fluorophenyl)-3(R)-(3'-indolyl)methyl-2H-1,4-benzodiazepin-2-one;

1,3-dihydro-5-(2-fluorophenyl)-3(R)-[3'-(1'-methylindolyl)-methyl]-1-methyl-2H-1,4-benzodiazepin-2-one;

1,3-dihydro-5-(2-fluorophenyl)-3(R)-(3'-indolyl)methyl-1-methyl-2H-1,4-benzodiazepin-2-one

7-chloro-1,3-dihydro-3(R)-(3'-indolyl)methyl-5-phenyl-2H-1,4-benzodiazepin-2-one;

1,3-dihydro-3(R)-(3'-indolyl)methyl-5-phenyl-2H-1,4-benzodiazepin-2-one;

1,3-dihydro-5-(2-fluorophenyl)-3(R)-(3'-α-indolenyl) methyl-2H-1,4-benzodiazepin-2-one;

1,3-dihydro-5-(2-fluorophenyl)-3(R)-(3'-β-indolenyl) methyl-2H-1,4-benzodiazepin-2-one;

1,3-dihydro-5-(2-fluorophenyl)-3(R)-(3'-indolyl)methyl-2H-1,4-benzodiazepin-2-thione;

1,3-dihydro-5-(2-fluorophenyl)-3(R)-(3'-indolyl)methyl-2H-1,4-benzodiazepine;

7-chloro-1,3-dihydro-3(R)-benzyl-5-phenyl-2H-1,4-benzodiazepin-2-one;

3(R)-benzyloxymethyl-7-chloro-1,3-dihydro-5-phenyl-2H-1,4-benzodiazepin-2-one;

7-chloro-1,3-dihydro-3(RS)-(1-naphthyl)methyl-5-phenyl-2H-1,4-benzodiazepin-2-one;

7-chloro-1,3-dihydro-3(RS)-(2-naphthyl)methyl-5-phenyl-2H-1,4-benzodiazepin-2-one;

1,3-dihydro-5-(2-fluorophenyl)-3(RS)-(2-thienyl)methyl-2H-1,4-benzodiazepin-2-one;

1,3-dihydro-5-(2-fluorophenyl)-3(RS)-(3-thienyl)-2H-1,4-benzodiazepin-2-one;

1,3-dihydro-5-(2-fluorophenyl)-3(R)-[3'-β-(1'-t-Boc-L-leucyl)-indolenyl]methyl-2H-1,4-benzodiazepin-2-one;

1,3-dihydro-5-(2-fluorophenyl)-3(R)-[3'-β-(1'-t-Boc-D-leucyl)-indolenyl]methyl-2H-1,4-benzodiazepin-2-one;

1,3-dihydro-5-(2-fluorophenyl)-3(R)-[3'-α-(1'-t-Boc-L-leucyl)-indolenyl]methyl-2H-1,4-benzodiazepin-2-one;

1,3-dihydro-5-(2-fluorophenyl)-3(R)-[3'-α-(1'-t-Boc-D-leucyl)-indolenyl]methyl-2H-1,4-benzodiazepin-2-one;

7-chloro-1,3,4,5-tetrahydro-3(R)-(3'-indolyl)methyl-5-phenyl-2H-1,4-benzodiazepin-2-one;

7-chloro-1,3,4,5-tetrahydro-3(S)-(3'-indolyl)methyl-5-phenyl-2H-1,4-benzodiazepin-2-one;

4-(p-chlorobenzoyl)-5-(2-fluorophenyl)-3(R)-[3'-(1'-methylindolyl)-methyl]-1-methyl-1,3,4,5-tetrahydro-2H-1,4-benzodiazepin-2-one;

4-acetyl-5-(2-fluorophenyl)-3(R)-[3'-(1'-methylindolyl)methyl]-1-methyl-1,3,4,5-tetrahydro-2H-1,4-benzodiazepin-2-one;

7-chloro-5-(2-chlorophenyl)-1,3-dihydro-3(R)-(3'-indolyl)methyl-2H-1,4-benzodiazepin-2-one;

1,3-dihydro-3(R)-(3'-indolyl)methyl-5-methyl-2H-1,4-benzodiazepin-2-one;

1-benzyl-7-chloro-1,3-dihydro-3(R)-(3'-indolyl)methyl-5-phenyl-2H-1,4-benzodiazepin-2-one;

7-chloro-1,3-Dihydro-3(R)-(3'-indolyl)methyl-1-methyl-5-phenyl-2H-1,4-benzodiazepin-2-one;

1,3-dihydro-5-(2-fluorophenyl)-3(S)-(3'-indolyl) methyl-2H-1,4-benzodiazepin-2-one;

1-benzyl-7-chloro-1,3-dihydro-3(S)-(3'-indolyl) methyl-5-phenyl-2H-1,4-

benzodiazepin – 2 – one;

7 – chloro – 1,3 – dihydro – 3(R) – (3' – indolyl)methyl – 5 – phenyl – 2H – 1,4 – benzodiazepin – 2 – thione;

1,3 – dihydro – 5 – (2 – fluorophenyl) – 3(R) – (3' – indolyl) methyl – 2H – 1,4 – benzodiazepin – 2 – [N' – (3 – thienoyl)] hydrazide;

1,3 – dihydro – 1 – ethyl – 5 – (2 – fluorophenyl) – 3(R) – (3' – indolyl)methyl – 2H – 1,4 – benzodiazepin – 2 – one;

1 – cyclopropylmethyl – 1,3 – dihydro – 5 – (2 – fluorophenyl) – 3(R) – (3' – indolyl)methyl – 2H – 1,4 – benzodiazepin – 2 – one;

1,3 – dihydro – 5 – (2 – fluorophenyl) – 3(R) – (3' – indolyl) methyl – 1 – pentyl – 2H – 1,4 – benzodiazepin – 2 – one;

1,3 – dihydro – 5 – (2 – fluorophenyl) – 3(R) – (3' – indolyl) methyl – 1 – (3 – methylbutyl) – 2H – 1,4 – benzodiazepine – 2 – one;

1,3 – dihydro – 5 – (2 – fluorophenyl) – 3(R) – (3' – indolyl)methyl – 1 – (2,2,2 – trifluoroethyl) – 2H – 1,4 – benzodiazepin – 2 – one;

1,3 – dihydro – 1 – (2 – dimethylaminoethyl) – 5 – (2 – fluorophenyl) – 3(R) – (3' – indolyl)methyl – 2H – 1,4 – benzodiazepin – 2 – one;

1,3 – dihydro – 1 – (ethoxycarbonylmethyl) – 5 – (2 – fluorophenyl) – 3(R) – (3' – indolyl)methyl – 2H – 1,4 – benzodiazepin – 2 – one;

1 – carboxymethyl – 1,3 – dihydro – 5 – (2 – fluorophenyl) – 3(R) – 3' – indolyl)methyl – 2H – 1,4 – benzodiazepin – 2 – one;

1,3 – dihydro – 5 – (2 – fluorophenyl) – 3(R) – [3' – (1' – p – chlorobenzyloylindolyl)methyl] – 1 – methyl – 2H – 1,4 – benzodiazepin – 2 – one;

7 – chloro – 1,3 – dihydro – 3(R) – [3'(1' – benzylindolyl)methyl] – 1 – methyl – 5 – phenyl – 2H – 1,4 – benzodiazepin – 2 – one;

1,3 – dihydro – 3(RS) – [1 – hydroxy – 1 – (3' – indolyl)]methyl – 1 – methyl – 5 – phenyl – 2H – 1,4 – benzodiazepin – 2 – one;

1,3 – dihydro – 1 – methyl – 5 – phenyl – 3 – (RS) – (3 – thienoyl) – 2H – 1,4 – benzodiazepin – 2 – one;

1,3 – dihydro – 3 – (RS) – [1 – hydroxy – 1 – (3 – thienyl)]methyl – 1 – methyl – 5 – phenyl – 2H – 1,4 – benzodiazepin – 2 – one;

1,3 – dihydro – 3(RS) – [1 – hydroxy – 1 – [3 – (1 – methylindolyl)]] – methyl – 1 – methyl – 5 – phenyl – 2H – 1,4 – benzodiazebin – 2 – one, two stereoisomers;

1,3 – dihydro – 3(RS) – (1 – hydroxy – 1 – phenyl)methyl – 1 – methyl – 5 – phenyl – 2H – 1,4 – benzodiazepin – 2 – one;

1,3 – dihydro – 3(RS) – [1 – hydroxy – 1 – (2 – thienyl)]methyl – 1 – methyl – 5 – phenyl – 2H – 1,4 – benzodiazepin – 2 – one;

1,3 – dihydro – 3 – (RS) – hydroxy – 1 – methyl – 5 – phenyl – 3 – (3' – thienoyl) – 2H – 1,4 – benzodiazepin – 2 – one;

1,5 – dihydro – 5 – (RS) – hydroxy – 1 – methyl – 5 – phenyl – 3 – (3' – thienoyl) – 2H – 1,4 – benzodiazepin – 2 – one;

7 – chloro – 1,3 – dihydro – 3(R) – [(2',3' – dihydro – 2' – oxo – 1'H – indol – 3' – yl)methyl] – 5 – phenyl – 2H – 1,4 – benzodiazepin – 2 – one;

7 – chloro – 1,3 – dihydro – 3(R) – [(3' – (2,4 – dinitrophenyl)imidazol – 5' – yl) – methyl] – 5 – phenyl – 2H – 1,4 – benzodiazepin – 2 – one;

7 – chloro – 1,3 – dihydro – 3(R) – (3' – imidazol – 5' – yl)methyl – 5 – phenyl – 2H – 1,4 – benzodiazepin – 2 – one;

3(RS) – [3' – (5' – Bromoindolyl)methyl] – 1,3 – dihydro – 5 – phenyl – 2H – 1,4 – benzodiazepin – 2 – one;

5 – o – carboxyphenyl – 1,3 – dihydro – 3(R) – (3' – indolyl)methyl – 2H – 1,4 – benzodiazepin – 2 – one;

1,3 – dihydro – 3(RS) – [3' – (5' – fluoroindolyl)methyl] – 5 – o – fluorophenyl – 2H – 1,4 – benzodiazepin – 2 – one;

1,3 – dihydro – 3(RS) – [3' – (6' – fluoroindolyl)methyl] – 5 – o – fluorophenyl – 2H – 1,4 – benzodiazepin – 2 – one;

2 – N – [2(RS),3 – bis – (Bocamino)propanoyl]amino – 2' – fluorobenzophenone;

2 – N – [2(RS),3 – diphthalylaminopropanoyl]amino – 2' – fluorobenzophenone;

1,3 – dihydro – 5 – (2' – fluorophenyl) – 3(RS) – aminomethyl – 2H – 1,4 – benzodiazepin – 2 – one;

1,3 – dihydro – 5 – (2' – fluorophenyl) – 3(R) – (4 – amino)butyl – 2H – 1,4 – benzodiazepin – 2 – one;

1,3 – dihydro – 5 – (2' – fluorophenyl) – 3(RS) – benzyloxycarbonylaminomethyl – 2H – 1,4 –

benzodiazepin – 2 – one;

1,3 – dihydro – 5 – (2' – fluorophenyl) – 3(RS) – (3 – thiophenecarbonyl)aminomethyl – 2H – 1,4 – benzodiazepin – 2 – one;

1,3 – dihydro – 5 – (2' – fluorophenyl) – 3(RS) – (2 – indole)carbonylaminomethyl) – 2H – 1,4 – benzodiazepin – 2 – one;

1,3 – dihydro – 5 – (2' – fluorophenyl) – 3(RS) – (6' – chloropyrazinyl)aminomethyl – 2H – 1,4 – benzodiazepin – 2 – one;

1,3 – dihydro – 5 – (2' – fluorophenyl) – 3(RS) – aminomethyl – 1 – methyl – 2H – 1,4 – benzodiazepin – 2 – one;

3(RS) – (2 – indolecarbonylamino) – 1,3 – dihydro – 5 – phenyl – 2H – 1,4 – benzodiazepin – 2 – one;

1,3 – Dihydro – 3(RS) – [2 – (3 – indolyl)ethyl]amino – 5 – phenyl – 2H – 1,4 – benzodiazepin – 2 – one;

3(RS) – [3 – (3 – indole)propionylamino] – 1,3 – dihydro – 5 – phenyl – 2H – 1,4 – benzodiazepin – 2 – one;

3(RS) – (3 – indoleacetylamino) – 1,3 – dihydro – 5 – phenyl – 2H – 1,4 – benzodiazepin – 2 – one;

3(RS) – (Boc – L – tryptophanyl)amino – 1,3 – dihydro – 5 – phenyl – 2H – 1,4 – benzodiazepin – 2 – one;

Z – 1,3 – dihydro – 1 – methyl – 5 – phenyl – 3 – (3 – thienylmethylene) – 2H – 1,4 – benzodiazepin – 2 – one;

E – 1,3 – dihydro – 1 – methyl – 5 – phenyl – 3 – (3 – thienylmethylene) – 2H – 1,4 – benzodiazepin – 2 – one;

3(RS) – (BOC – D – tryptophyl)amino – 1,3 – dihydro – 5 – phenyl – 2H – 1,4 – benzodiazepin – 2 – one;

3(RS) – [4 – (3 – indole)butyrylamino] – 1,3 – dihydro – 5 – phenyl – 2H – 1,4 – benzodiazepin – 2 – one;

1,3 – dihydro – 3(RS) – (benzyloxycarbonyl)aminomethyl – 5 – (2 – fluorophenyl) – 2H – 1,4 – benzodiazepine;

1,3 – dihydro – 3(RS) – [3' – (thiophene)carbonyl]aminomethyl – 5 – (2 – fluorophenyl) – 2H – 1,4 – benzodiazepine;

1,3 – dihydro – 3(RS) – (2 – indolecarbonyl)aminomethyl – 5 – (2 – fluorophenyl) – 2H – 1,4 – benzodiazepine;

1,3 – dihydro – 3(RS) – (2 – L – hydroxy – 2 – phenylacetyl)aminomethyl – 5 – (2 – fluorophenyl) – 2H – 1,4 – benzodiazepine;

1 – (2 – cyanoethyl) – 1,3 – dihydro – 5 – (2 – fluorophenyl) – 3(R) – (3' – indolyl)methyl – 2H – 1,4 – benzodiazepin – 2 – one;

1 – (2 – cyanoethyl) – 1,3 – dihydro – 5 – (2 – fluorophenyl) – 3(R) – [1' – (2 – cyanoethyl) – 3' – indolyl] – methyl – 2H – 1,4 – benzodiazpin – 2 – one;

1 – (2 – carboxyethyl) – 1,3 – dihydro – 5 – (2 – fluorophenyl) – 3(R) – (3' – indolyl)methyl – 2H – 1,4 – benzodiazepin – 2 – one;

1,3 – dihydro – 3(R) – (3' – indolyl)methyl – 5 – (2 – fluorophenyl) – 2H – 1,4 – benzodiazepin – 2 – one – 4 – oxide;

1,3 – dihydro – 5 – (2 – fluorophenyl) – 3 – (RS) – (2 – indolecarbonylamino) – 2H – 1,4 – benzodiazepin – 2 – one;

1,3 – dihydro – 5 – (2 – fluorophenyl) – 3(RS) – (2 – indolecarbonylamino) – 1 – methyl – 2H – 1,4 – benzodiazepin – 2 – one;

1,3 – dihydro – 5 – (2 – fluorophenyl) – 1 – methyl – 3(RS) – [2' – (1 – methylindole)carbonylamino] – 2H – 1,4 – benzodiazepin – 2 – one;

1,3 – dihydro – 3(RS) – (4 – nitrophenylcarbonyl)amino – 5 – (2 – fluorophenyl) – 2H – 1,4 – benzodiazepin – 2 – one;

1,3 – dihydro – 3(RS) – (2 – indolecarbonyloxy) – 5 – phenyl – 2H – 1,4 – benzodiazepin – 2 – one;

1,3 – dihydro – 5 – (2 – fluorophenyl) – 3(RS) – (3 – thiophene carbonylamino) – 2H – 1,4 – benzodiazepin – 2 – one;

1,3 – dihydro – 3(RS) – (3 – indolecarbonylamino) – 5 – phenyl – 2H – 1,4 – benzodiazepin – 2 – one;

1,3 – dihydro – 3(RS) – (4 – thianaphtheneacetyl)amino – 5 – (2 – fluorophenyl) – 2H – 1,4 – benzodiazepin – 2 – one;

1,3 – dihydro – 3(RS) – (4 – chlorophenylcarbonyl)amino – 5 – (2 – fluorophenyl) – 2H – 1,4 – benzodiazepin – 2 – one;

1,3 – dihydro – 3(RS) – (4 – methylphenylsulfonyl)amino – 5 – (2 – fluorophenyl) – 2H – 1,4 – benzodiazepin – 2 – one;

1 – carboxymethyl – 1,3 – dihydro – 5 – (2 – fluorophenyl) – 3(RS) – (2 – indolecarbonylamino) – 2H – 1,4 – benzodiazepin – 2 – one;

1,3 – Dihydro – 3(RS) – (5 – fluoroindole – 2 – carbonylamino) – 5 – (2 – fluorophenyl) – 2H – 1,4 – benzodiazepin – 2 – one;

1,3 – dihydro – 3(RS) – (3' – methylindenyl – 2 – carbonyl)amino – 5 – (2 – fluorophenyl) – 2H – 1,4 – benzodiazepin – 2 – one;

1,3 – dihydro – 3(RS) – (2 – quinaldyl)amino – 5 – (2 – fluorophenyl) – 2H – 1,4 – benzodiazepin – 2 – one;

1,3 – dihydro – 3(RS) – (2 – L – hydroxy – 2 – phenylacetyl)amino – 5 – (2 – fluorophenyl) – 2H – 1,4 – benzodiazepin – 2 – one;

1,3 – Dihydro – 3(RS) – (5 – chloroindole – 2 – carbonylamino) – 5 – (2 – fluorophenyl) – 2H – 1,4 – benzodiazepin – 2 – one;

3(RS) – [N – (2 – indolecarbonyl) – N – methylamino] – 1,3 – dihydro – 5 – phenyl – 2H – 1,4 – benzodiazepin – 2 – one;

1,3 – dihydro – 3(RS) – (2 – indolecarbonylamino) – 1 – methyl – 5 – phenyl – 2H – 1,4 – benzodiazepin – 2 – one;

1,3 – dihydro – 1 – methyl – 3(RS) – [2 – (1 – methylindole)carbonylamino] – 5 – phenyl – 2H – 1,4 – benzodiazepin – 2 – one;

1 – carboxymethyl – 1,3 – dihydro – 3(RS) – (2 – indolecarbonylamino) – 5 – phenyl – 2H – 1,4 – benzodiazepin – 2 – one;

1,3 – Dihydro – 3(RS) – (5 – bromoindole – 2 – carbonylamino) – 5 – (2 – fluorophenyl) – 2H – 1,4 – benzodiazepin – 2 – one;

3(RS) – cinnamoylamino – 1,3 – dihydro – 5 – (2 – fluorophenyl) – 2H – 1,4 – benzodiazepin – 2 – one;

1,3 – dihydro – 3(RS) – (5 – hydroxy – 2 – indolylcarbonyl)amino – 5 – (2 – fluorophenyl) – 2H – 1,4 – benzodiazepin – 2 – one;

1 – carboxamidomethyl – 1,3 – dihydro – 3R – (3 – indolylmethyl) – 5 – (2 – fluorophenyl) – 2H – 1,4 – benzodiazepin – 2 – one;

1,3 – dihydro – 5 – (2 – fluorophenyl) – 3 – (RS) – (2 – indolylmethylamino) – 2H – 1,4 – benzodiazepin – 2 – one;

1,3 – dihydro – 3(RS) – (phenylaminomethylcarbonyl)amino – 5 – (2 – fluorophenyl) – 2H – 1,4 – benzodiazepin – 2 – one;

1,3 – Dihydro – 3(RS) – (5 – methoxyindole – 2 – carbonylamino) – 5 – (2 – fluorophenyl) – 2H – 1,4 – benzodiazepin – 2 – one;

1,3 – Dihydro – 3(RS) – (1 – methylindole – 2 – carbonylamino) – 5 – (2 – fluorophenyl) – 2H – 1,4 – benzodiazepine – 2 – one;

1,3 – dihydro – 1 – methyl – 3(RS) – (4 – chlorophenylcarbonyl)amino – 5 – (2 – fluorophenyl) – 2H – 1,4 – benzodiazepin – 2 – one;

1,3 – dihydro – 5 – (2 – fluorophenyl) – 3(RS) – (2 – benzofurancarbonylamino) – 2H – 1,4 – benzodiazepin – 2 – one;

1 – ethoxycarbonylmethyl – 1,3 – dihydro – 3(RS) – (4 – chlorophenylcarbonyl)amino – 5 – (2 – fluorophenyl) – 2H – 1,4 – benzodiazepin – 2 – one;

1,3 – dihydro – 3(RS) – (4 – chlorophenylcarbonyl)amino – 5 – phenyl – 2H – 1,4 – benzodiazepine – 2 – one;

1,3 – dihydro – 1 – methyl – 3(RS) – (4 – chlorophenylcarbonyl)amino – 5 – phenyl – 2H – 1,4 – benzodiazepin – 2 – one;

1 – carboxymethyl – 1,3 – dihydro – 3(RS) – (4 – chlorophenylcarbonyl)amino – 5 – (2 – fluorophenyl) – 2H – 1,4 – benzodiazepin – 2 – one;

3(RS) – (2(S) – tert – Butoxycarbonylamino – 3 – phenylpropanoylamino) – 1,3 – dihydro – 5 – phenyl – 2H – 1,4 – benzodiazepin – 2 – one;

3(RS) – (2(S) – tert – Butoxycarbonylamino – 3 – phenylpropanoylamino) – 1,3 – dihydro – 1 – methyl – 5 – phenyl – 2H – 1,4 – benzodiazepin – 2 – one;

3(R) – and 3(S) – (2(S) – Amino – 3 – phenylpropanoylamino) – 1,3 – dihydro – 1 – methyl – 5 – phenyl – 2H – 1,4 – benzodiazepin – 2 – one;

3(R) – and 3(S) – Amino – 1,3 – dihydro – 1 – methyl – 5 – phenyl – 2H – 1,4 – benzodiazepin – 2 – one;

3(R) – and 3(S) – Amino – 1,3 – dihydro – 5 – (2 – fluorophenyl) – 1 – methyl – 2H – 1,4 – benzodiazepin – 2 – one;

3(S) – ( – ) – 1,3 – Dihydro – 3 – (2 – indolecarbonylamino) – 1 – methyl – 5 – phenyl – 2H – 1,4 – benzodiazepin – 2 – one;

3(S) – ( + ) – 1,3 – Dihydro – 5 – (2 – fluoropheny1) – 3 – (2 – indolecarbonylamino) – 1 – methyl – 2H – 1,4 – benzodiazepin – 2 – one;

3(R) − ( − ) − 1,3 − Dihydro − 5 − (2 − fluorophenyl) − 3 − (2 − indolecarbonylamino) − 1 − methyl − 2H − 1,4 − benzodiazepin − 2 − one;

3(R) − ( − ) − 1,3 − Dihydro − 3 − (4 − chlorobenzoylamino) − 5 − (2 − fluorophenyl) − 1 − methyl − 2H − 1,4 − benzodiazepin − 2 − one;

3(S) − ( + ) − 1,3 − Dihydro − 3 − (4 − chlorobenzoylamino) − 5 − (2 − fluorophenyl) − 1 − methyl − 2H − 1,4 − benzodiazepin − 2 − one;

3(S) − ( − ) − 1,3 − Dihydro − 3 − (4 − bromobenzoylamino) − 1 − methyl − 5 − phenyl − 2H − 1,4 − benzodiazepin − 2 − one;

3(R) − ( + ) − 1,3 − Dihydro − 3 − (4 − bromobenzoylamino) − 1 − methyl − 5 − phenyl − 2H − 1,4 − benzodiazepin − 2 − one;

3(R) − ( + ) − 1,3 − Dihydro − 3 − (2 − indolecarbonylamino) − 1 − methyl − 5 − phenyl − 2H − 1,4 − benzodiazepin − 2 − one;

1,3 − Dihydro − 3 − (RS) − (2 − indolinecarbonylamino) − 5 − phenyl − 2H − 1,4 − benzodiazepin − 2 − one;

1,3 − Dihydro − 5 − (2 − fluorophenyl) − 3 − (RS) − (p − trifluoromethylbenzoylamino) − 2H − 1,4 − benzodiazepin − 2 − one;

1,3 − Dihydro − 5 − (2 − fluorophenyl) − 3 − (RS) − (p − methylbenzoylamino) − 2H − 1,4 − benzodiazepin − 2 − one;

1,3 − Dihydro − 5 − (2 − fluoropheny]) − 3 − (RS) − (p − methoxybenzoylamino) − 2H − 1,4 − benzodiazepin − 2 − one;

3 − (RS) − (o − Chlorobenzoylamino) − 1,3 − dihydro − 5 − phenyl − 2H − 1,4 − benzodiazepin − 2 − one;

3 − (RS) − (o − Chlorobenzoylmethylamino − 1,3 − dihydro − 1 − methyl − 5 − phenyl − 2H − 1,4 − benzodiazepin − 2 − one;

3 − (RS) − (o − Chlorobenzoylamino) − 1,3 − dihydro − 1 − methyl − 5 − phenyl − 2H − 1,4 − benzodiazepin − 2 − one;

3 − (RS) − (m − Chlorobenzoylamino) − 1,3 − dihydro − 5 − phenyl − 2H − 1,4 − benzodiazepin − 2 − one;

3 − (RS) − (3,4 − Dichlorobenzoylamino) − 1,3 − dihydro − 5 − phenyl − 2H − 1,4 − benzodiazepin − 2 − one;

3 − (RS) − (p − Chlorobenzoylamino)1,3 − dihydro − 5 − (2' − fluorophenyl) − 1 − methyl − 4 − oxo − 2H − 1,4 − benzodiazepin − 2 − one;

1,3 − Dihydro − 5 − Phenyl − 3 − (RS) − (4' − methylthiobenzoylamino) − 2H − 1,4 − benzodiazepin − 2 − one;

1 − 3 − Dihydro − 3 − (RS) − (4' − Fluorobenzoylamino) − 5 − phenyl − 2H − 1,4 − benzodiazepin − 2 − one;

1,3 − Dihydro − 5 − Phenyl − 3 − (RS) − (4' − trifluoromethylbenzoylamino) − 2H − 1,4 − benzodiazepin − 2 − one;

1,3 − Dihydro − 3 − (RS) − (4' − tert − Butylbenzoylamino) − 5 − phenyl − 2H − 1,4 − benzodiazepin − 2 − one;

3 − (RS) − (3,5 − Dichlorobenzoylamino)1,3 − dihydro − 5 − phenyl − 2H − 1,4 − benzodiazepin − 2 − one;

1 − 3 − Dihydro − 3 − (RS) − (p − Hydroxybenzoylamino) − 5 − phenyl − 2H − 1,4 − benzodiazepin − 2 − one;

3 − (RS) − (4' − Cyanobenzoylamino)1,3 − dihydro − 5 − phenyl − 2H − 1,4 − benzodiazepin − 2 − one;

3(S) − ( − ) − 3 − (2 − Chlorobenzoylamino) − 1,3 − dihydro − 1 − methyl − 5 − phenyl − 2H − 1,4 − benzodiazepin − 2 − one;

3(R) − ( + ) − 3 − (2 − Chlorobenzoylamino) − 1,3 − dihydro − 1 − methyl − 5 − phenyl − 2H − 1,4 − benzodiazepin − 2 − one;

1,3 − Dihydro − 3(RS) − (p − dimethylaminobenzoylamino) − 5 − (2 − fluorophenyl) − 2H − 1,4 − benzodiazepin − 2 − one;

1,3 − Dihydro − 3(RS) − (3,4 − dimethoxybenzoylamino) − 5 − (2 − fluorophenyl) − 2H − 1,4 − benzodiazepin − 2 − one;

3(S) − ( + ) − 3 − (3 − Bromobenzoylamino) − 1,3 − dihydro − 5 − (2 − fluorophenyl) − 1 − methyl − 2H − 1,4 − benzodiazepin − 2 − one;

1,3 − Dihydro − 5 − phenyl − 3(RS) − (3 − trifluoromethylthiobenzoylamino) − 2H − 1,4 − benzodiazepin − 2 − one;

3(S) − ( + ) − 3 − (4 − Bromobenzoylamino) − 1,3 − dihydro − 5 − (2 − fluorophenyl) − 1 − methyl − 2H − 1,4 − benzodiazepin − 2 − one;

3(S) − ( + ) − 3 − (4 − t − Butylbenzoylamino) − 1,3 − dihydro − 5 − (2 − fluorophenyl) − 1 − methyl − 2H − 1,4 − benzodiazepin − 2 − one;

1,3 – Dihydro – 5 – (2 – fluorophenyl) – 3(RS) – (pyrrole – 2 – carbonylamino) – 2H – 1,4 – benzodiazepin – 2 – one;

3(S) – ( + ) – 1,3 – Dihydro – 5 – (2 – fluorophenyl) – 3 – (4 – iodobenzoylamino) – 1 – methyl – 2H – 1,4 – benzodiazepin – 2 – one;

1,3 – Dihydro – 3(RS) – (2 – naphthoylamino) – 5 – phenyl – 2H – 1,4 – benzodiazepin – 2 – one;

3(S) – ( – ) – 3 – (2 – Bromobenzoylamino) – 1,3 – dihydro – 5 – (2 – fluorophenyl) – 1 – methyl – 2H – 1,4 – benzodiazepin – 2 – one;

3(S) – ( + ) – 3 – (4 – Cyanobenzoylamino) – 1,3 – dihydro – 5 – (2 – fluorophenyl) – 1 – methyl – 2H – 1,4 – benzodiazepin – 2 – one;

1,3 – Dihydro – 5 – phenyl – 3(RS) – (4 – n – propylbenzoylamino) – 2H – 1,4 – benzodiazepin – 2 – one;

1,3 – Dihydro – 5 – phenyl – 3(RS) – (4 – phenylbenzoylamino) – 2H – 1,4 – benzodiazepin – 2 – one;

1,3 – Dihydro – 3(RS) – (4 – n – pentylbenzoylamino) – 5 – phenyl – 2H – 1,4 – benzodiazepin – 2 – one;

1,3 – Dihydro – 3(RS) – (1 – naphthoylamino) – 5 – phenyl – 2H – 1,4 – benzodiazepin – 2 – one;

3(S) – ( + ) – 1,3 – Dihydro – 5 – (2 – fluorophenyl) – 3 – (3 – iodobenzoylamino) – 1 – methyl – 2H – 1,4 – benzodiazepin – 2 – one;

3(R) – ( – ) – 1,3 – Dihydro – 5 – (2 – fluorophenyl) – 3 – (3 – iodobenzoylamino) – 1 – methyl – 2H – 1,4 – benzodiazepin – 2 – one;

3(R) – ( + ) – 1,3 – Dihydro – 5 – (2 – fluorophenyl) – 3 – (2 – iodobenzoylamino) – 1 – methyl – 2H – 1,4 – benzodiazepin – 2 – one;

3(S) – ( – ) – 1,3 – Dihydro – 5 – (2 – fluorophenyl) – 3 – (2 – iodobenzoylamino) – 1 – methyl – 2H – 1,4 – benzodiazepin – 2 – one;

3(R) – ( + ) – 3 – (2 – Bromobenzoylamino) – 1,3 – dihydro – 5 – (2 – fluorophenyl) – 1 – methyl – 2H – 1,4 – benzodiazepin – 2 – one;

3(R) – ( + ) – 3 – (2 – Chlorobenzoylamino) – 1,3 – dihydro – 5 – (2 – fluorophenyl) – 1 – methyl – 2H – 1,4 – benzodiazepin – 2 – one;

1,3 – Dihydro – 5 – (2 – fluorophenyl) – 3(RS) – phenylcarbonylamino – 2H – 1,4 – benzodiazepin – 2 – one;

1,3 – Dihydro – 5 – (2 – fluorophenyl) – 3(RS) – (2 – chlorophenyl)carbonylamino – 2H – 1,4 – benzodiazepin – 2 – one;

1,3 – Dihydro – 5 – (2 – fluorophenyl) – 3(RS) – benzyloxycarbonylamino – 2H – 1,4 – benzodiazepin – 2 – one;

1,3 – Dihydro – 5 – (2 – fluorophenyl) – 3 – (RS) – benzyloxycarbonylamino – 2H – 1,4 – benzodiazepin – 2 – thione;

1 – (4 – Chlorophenyl)carbonyl – 1,3 – dihydro – 5 – (2 – fluorophenyl) – 3(RS) – (4 – chlorophenyl) – carbonylamino – 2H – 1,4 – benzodiazepin – 2 – one;

1 – (4 – Chlorophenyl)carbonyl – 1,3 – dihydro – 5 – (2 – fluorophenyl) – 3(RS) – (4 – chlorophenyl) – carbonyloxy – 2H – 1,4 – benzodiazepin – 2 – one;

1,3 – Dihydro – 5 – (2 – fluorophenyl) – 3(RS) – (4 – chlorophenyl) – oxy – 2H – 1,4 – benzodiazepin – 2 – one;

1,3 – Dihydro – 5 – (2 – fluorophenyl) – 3 – (RS) – (4 – chlorophenyl)carbonylamino – 2H – 1,4 – benzodiazepin – 2 – thione;

1,3 – Dihydro – 5 – (2 – fluorophenyl) – 3 – (RS) – (2 – indole) carbonylamino – 2H – 1,4 – benzodiazepin – 2 – thione or

1,3 – Dihydro – 3(RS) – (4 – chlorophenyl)aminocarbonylamino – 2H – 1,4 – benzodiazepin – 2 – one.

**6.** Use according to claim 1, characterized in that the compound of formula I is:

(S) – ( – ) – 1,3 – dihydro – 3 – (4 – bromobenzoylamino) – 1 – methyl – 5 – phenyl – 2H – 1,4 – benzodiazepin – 2 – one;

3(RS) – (2 – indolecarbonylamino) – 1,3 – dihydro – 5 – phenyl – 2H – 1,4 – benzodiazepin – 2 – one;

1,3 – Dihydro – 3(RS) – (2 – indolecarbonylamino) – 1 – methyl – 5 – phenyl – 2H – 1,4 – benzodiazepin – 2 – one;

1,3 – dihydro – 1 – methyl – 3(RS) – [2 – (1 – methylindole) carbonylamino] – 5 – phenyl – 2H – 1,4 – benzodiazepin – 2 – one;

1,3 – Dihydro – 1 – methyl – 3 – (RS) – (4 – chlorophenylcarbonyl)amino – 5 – (2 – fluorophenyl) – 2H – 1,4 – benzodiazepin – 2 – one;

1,3 – Dihydro – 5 – (2 – fluorophenyl) – 3(RS) – (2 – indolecarbonylamino) – 1 – methyl – 2H – 1,4 – benzodiazepin – 2 – one;

1,3 – Dihydro – 5 – (2 – fluorophenyl) – 1 – methyl – 3(RS) – [2' – (1 – methylindole)carbonylamino] –

2H – 1,4 – benzodiazepin – 2 – one;

3(R) – ( – ) – 1,3 – Dihydro – 3 – (4 – chlorobenzoylamino) – 5 – (2 – fluorophenyl) – 1 – methyl – 2H – 1,4 – benzodiazepin – 2 – one;

3(S) – ( + ) – 1,3 – Dihydro – 3 – (4 – chlorobenzoylamino) – 5 – (2 – fluorophenyl) – 1 – methyl – 2H – 1,4 – benzodiazepin – 2 – one;

3(R) – ( + ) – 1,3 – Dihydro – 3 – (4 – bromobenzoylamino) – 1 – methyl – 5 – phenyl – 2H – 1,4 – benzodiazepin – 2 – one;

3(R) – ( + ) – 1,3 – Dihydro – 3 – (2 – indolecarbonylamino) – 1 – methyl – 5 – phenyl – 2H – 1,4 – benzodiazepin – 2 – one;

1,3 – Dihydro – 5 – (2 – fluorophenyl) – 3 – (RS) – (2 – indolecarbonylamino) – 2H – 1,4 – benzodiazepin – 2 – one;

1,3 – Dihydro – 3 – (RS) – (2 – indolecarbonyloxy) – 5 – phenyl – 2H – 1,4 – benzodiazepin – 2 – one;

1,3 – Dihydro – 3 – (RS) – (4 – chlorophenylcarbonyl)amino – 5 – (2 – fluorophenyl) – 2H – 1,4 – benzodiazepin – 2 – one;

1 – Carboxymethyl – 1,3 – dihydro – 5 – (2 – fluorophenyl) – 3(RS) – (2 – indolecarbonylamino) – 2H – 1,4 – benzodiazepin – 2 – one;

1,3 – Dihydro – 3 – (RS) – (5 – fluoroindole – 2 – carbonylamino) – 5 – (2 – fluorophenyl) – 2H – 1,4 – benzodiazepin – 2 – one;

3 – (RS) – Cinnamoylamino – 1,3 – dihydro – 5 – (2 – fluorophenyl) – 2H – 1,4 – benzodiazepin – 2 – one;

1,3 – Dihydro – 5 – (2 – fluorophenyl) – 3 – (RS) – (2 – benzofurancarbonylamino) – 2H – 1,4 – benzodiazepin – 2 – one;

1,3 – Dihydro – 1 – methyl – 3 – (RS) – (4 – chlorophenylcarbonyl)amino – 5 – phenyl – 2H – 1,4 – benzodiazepin – 2 – one;

1 – Carboxymethyl – 1,3 – dihydro – 3 – (RS) – (4 – chlorophenylcarbonyl)amino – 5 – (2 – fluorophenyl) – 2H – 1,4 – benzodiazepin – 2 – one;

1,3 – Dihydro – 5 – (2 – fluorophenyl) – 3 – (RS) – (p – trifluoromethylbenzoylamino) – 2H – 1,4 – benzodiazepin – 2 – one;

3(S) – ( + ) – 3 – (3 – Bromobenzoylamino) – 1,3 – dihydro – 5 – (2 – fluorophenyl) – 1 – methyl – 2H – 1,4 – benzodiazepin – 2 – one;

3(S) – ( + ) – 3 – (4 – Bromobenzoylamino) – 1,3 – dihydro – 5 – (2 – fluorophenyl) – 1 – methyl – 2H – 1,4 – benzodiazepin – 2 – one;

3(S) – ( + ) – 1,3 – Dihydro – 5 – (2 – fluorophenyl) – 3 – (4 – iodobenzoylamino) – 1 – methyl – 2H – 1,4 – benzodiazepin – 2 – one;

1,3 – Dihydro – 3(RS) – (2 – naphthoylamino) – 5 – phenyl – 2H – 1,4 – benzodiazepin – 2 – one;

3(S) – ( + ) – 1,3 – Dihydro – 5 – (2 – fluorophenyl) – 3 – (3 – iodobenzoylamino) – 1 – methyl – 2H – 1,4 – benzodiazepin – 2 – one; or

3(R) – ( – ) – 1,3 – Dihydro – 5 – (2 – fluorophenyl) – 3 – (3 – iodobenzoylamino) – 1 – methyl – 2H – 1,4 – benzodiazepin – 2 – one or

3(R) – ( – ) – 1,3 – Dihydro – 5 – (2 – fluorophenyl) – 3 – (2 – indolecarbonylamino) – 1 – methyl – 2H – 1,4 – benzodiazepin – 2 – one.

**7.** Use according to claim 1, characterized in that the compound of formula I is:

1 – carboxymethyl – 1,3 – dihydro – 3(RS) – (2 – indolecarbonylamino) – 5 – phenyl – 2H – 1,4 – benzodiazepin – 2 – one,

(S) – ( – ) – 1,3 – dihydro – 3 – (2 – indolecarbonylamino) – 1 – methyl – 5 – phenyl – 2H – 1,4 – benzodiazepin – 2 – one

(S) – ( + ) – 1,3 – dihydro – 5 – (2 – fluorophenyl) – 3 – (2 – indolecarbonylamino) – 1 – methyl – 2H – 1,4 – benzodiazepin – 2 – one,

or

(S) – ( + ) – 1,3 – dihydro – 3 – (4 – chlorobenzoylamino) – 5 – (2 – fluorophenyl) – 1 – methyl – 2H – 1,4 – benzodiazepin – 2 – one.

**8.** Process for preparing novel benzodiazepine derivatives which correspond to the following formula Ia

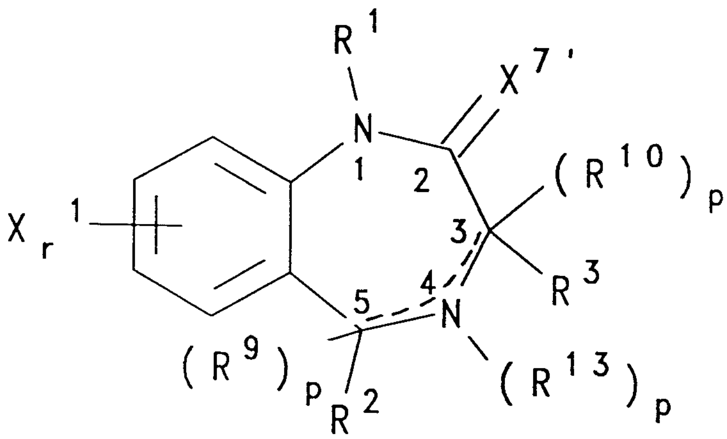

Ia

wherein

$X^{7'}$ is S or HH and

the remaining substituents and symbols have the same meaning as in the compounds of formula I defined in claim 1,

or salts or quaternary ammonium salts of the compounds of formula Ia, comprising:

a) coupling 2-aminoarylketones

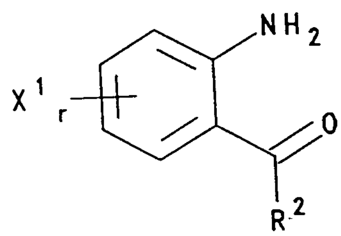

1

to N-protected D-amino acids

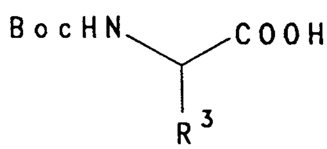

2,

in the presence of a peptide coupling reagent N-deprotecting the product

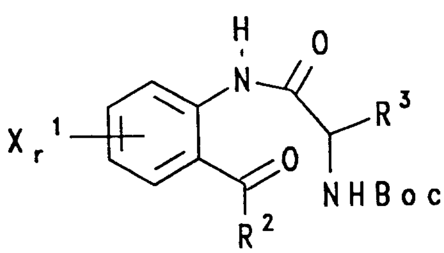

3

with an acid, preferably anhydrous hydrochloric acid to give the α-aminoacyl derivative

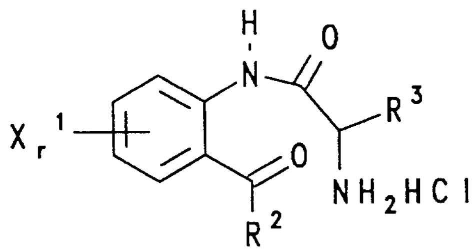 4

this derivative alternatively being obtained by treatment of the 2 – arylaminoketone 1 with an acid chloride hydrochloride

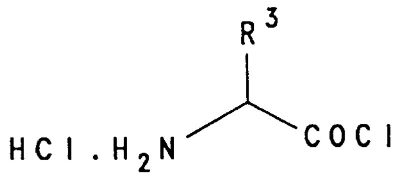 8

treating the α – aminoacyl derivative 4 with base and cyclizising the free base

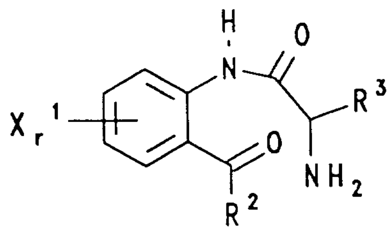 6

to the 3,5 – disubstituted benzodiazepine by stirring in the methanolic base for 2 – 120 hours.

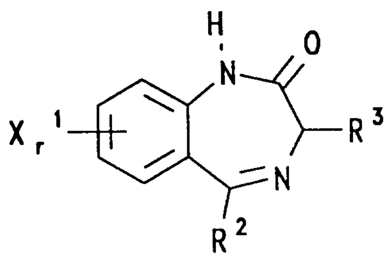 7

this benzodiazepine alternatively being obtained by heating the ketone 1 with an ester

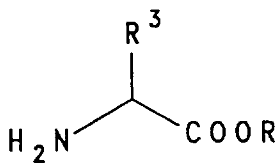 8

in which ester R is preferably methyl or ethyl, treating the 3,5 – disubstituted benzodiazepines 7 with $P_2S_5$ or Lawesson's reagent to give 2 – thiones

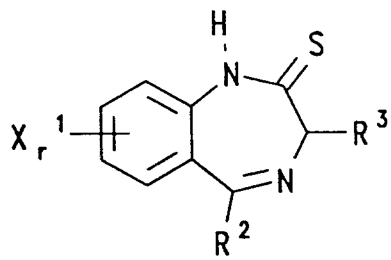

1 4

optionally reducing the 2 – thiones 14 with Raney nickel to the 2 – unsubstituted benzodiazepines

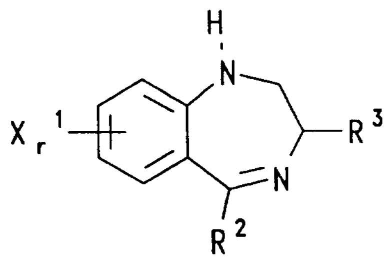

1 5

and optionally alkylating with alkyl halide or sulpate, acylating with acyl halide or anhydride, reducing with sodium cyanoborhydride or substituting with alkyl or aryl magnesium halide to give the corresponding alkyl derivatives, acyl derivatives, 4,5 – dihydro derivatives and substituted derivatives, or

b) coupling the ketones 1 with N – phthalyamino acids

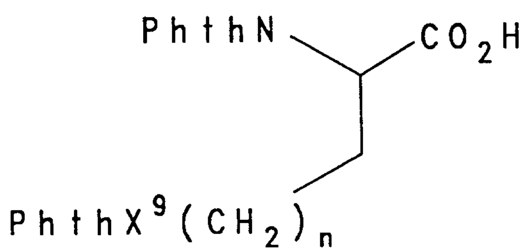

2 b

in the presence of a peptide coupling reagent and cyclizing the intermediate product by treating with hydrazine

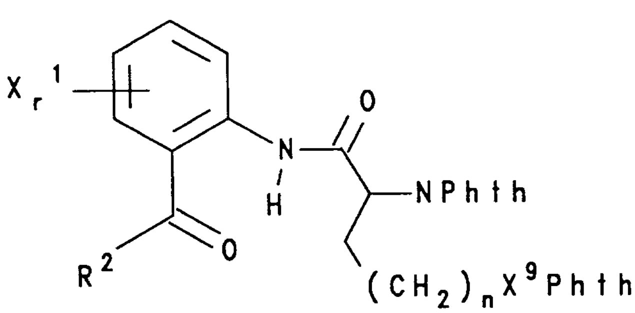

3 b

possibly after N – alkylation by treatment with sodium hydride followed by $R^1X$ in dimethylformamide to give the benzodiazepine derivative 9

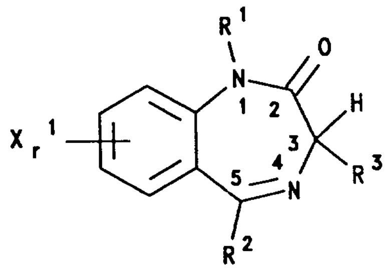

9

wherein

$R^3$ is $-(CH_2)_{n+1}-X^9H$; such compounds of formula 9 may be converted to further compounds of formula 9 by:

a) alkylation by treatment with an alkyl halide or dialkylsulfate having the formula
$R^7-(CH_2)_q-X$
yielding compounds of formula 9a, wherein $R^3$ is
$-(CH_2)_{n+1}X^9(CH_2)_qR^7$

b) acylation by treatment with acid halides or acid anhydrides of formula

$$R^7-(CH_2)_q-\overset{O}{C}-X$$

to yield corresponding acylated derivatives of formula 9b wherein $R^3$ is

$$-(CH_2)_{n+1}X^9\overset{O}{C}(CH_2)_qR^7$$

or

c) converting the compounds of formula 9 wherein $R^3$ is $-(CH_2)_{n+1}-NH_2$ to a corresponding derivative in which the $NH_2$ group is protected with the benzyloxy-carbonyl group and converting this compound to a corresponding 2-thione through reaction with $P_2S_5$ or Lawesson's reagent and optionally reducing the 2-thione with Raney nickel in methanol to the 2-unsubstituted benzodiazepines, followed by a deprotection via hydrogenolysis and optionally alkylating or acylating the resulting products, by treatment with alkyl halide or dialkyl sulphate, in the presence of a peptide coupling agent,

and preparing salts or quaternary ammonium salts thereof, if desired.

**9.** A process for preparing novel benzodiazepine derivatives which correspond to the following formula Ib

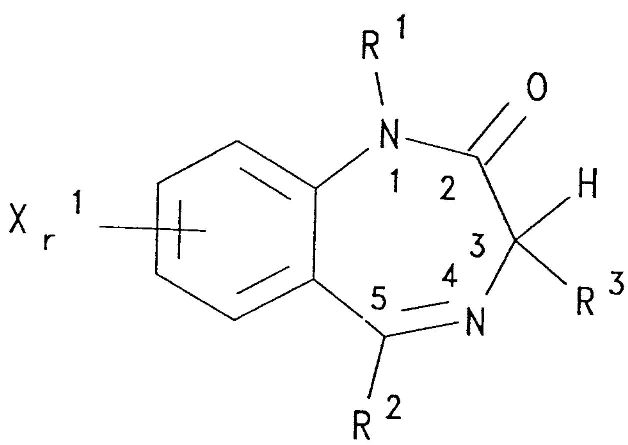

I b

or salts or quaternary ammonium salts of the compounds of formula Ib, in which compounds of formula Ib

$R^1$ is alkyl having 1 − 5 carbon atoms or hydrogen

$R^2$ is unsubstituted phenyl or substituted phenyl having 1 or 2 substituents selected from halo, alkyl having 1 − 4 carbon atoms, alkoxy having 1 − 4 carbon atoms, carboxy substituted alkyl having 1 − 4 carbon atoms in the alkyl group, carboxyl, nitro, trifluoro methyl and hydroxy,

$R^3$ is a group having the formulae

$$-(CH_2)_nNH\overset{O}{\overset{\|}{C}}(CH_2)_qR^7$$

or

$$-(CH_2)_nNH\overset{O}{\overset{\|}{C}}NH(CH_2)_nR^7;$$

wherein

$R^7$ is unsubstituted phenyl or substituted phenyl having 1 or 2 substituents which are selected from halo, nitro, hydroxy, alkyl having 1 − 4 carbon atoms, alkoxy having 1 − 4 carbon atoms, alkylthio having 1 − 4 carbon atoms, trifluoro methyl groups or groups having the formulae

CN, $-C\equiv CH$, $SCF_3$,

$$O\overset{O}{\overset{\|}{C}}CH_3,$$

$OCHF_2$, SPh or $-NR^4R^5$

wherein

$R^4$ and $R^5$ are independently hydrogen or alkyl having 1 − 4 carbon atoms,

or the radical

$R^7$ is a group having the formula

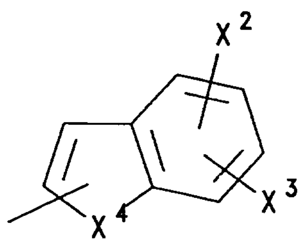

,

wherein

$X^2$ and $X^3$ are independently hydrogen, hydroxy, nitro, halo, alkylthio having 1 − 4 carbon atoms, alkyl having 1 − 4 carbon atoms or alkoxy having 1 − 4 carbon atoms,

$X^4$ is NH

q is O − 4, preferably O and

n is O − 4, preferably O ,

comprising following the routes a) or b) as defined in claim 8 to the extent necessary, or

c) introducing substituent $R^3$, having the formula

$$-NH-\overset{O}{\overset{\|}{C}}-(CH_2)_q-R^7$$

into a 3-chlorosubstituted-5-substituted-1-substituted or unsubstituted benzodiazepine 9d

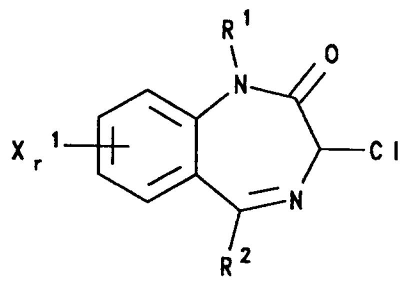

9d

or

d) reacting a 3-amino or 3-aminoalkyl-5-substituted-1-substituted or unsubstituted benzodiazepine

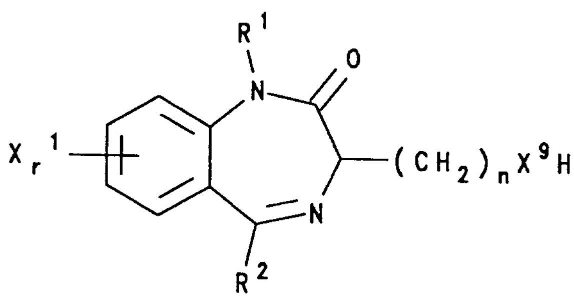

23

with an acyl halide or isocyanate to give the corresponding amide or urea

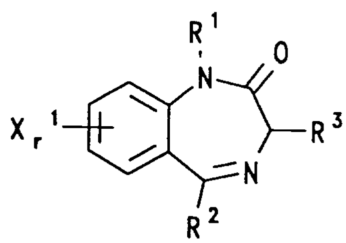

24

$$(R^3 = (CH_2)_nNH\overset{O}{\overset{\|}{C}}(CH_2)_qR^7 \text{ or } (CH_2)_n\overset{H}{N}-\overset{O}{\overset{\|}{C}}-\overset{H}{N}(CH_2)_nR^7$$

and optionally alklating obtained benzodiazepines of formula Ib wherein $R^1$ = H by treating with sodium hydride in a suitable solvent, such as DMF, followed by an alkyl halide and preparing salts or quaternary ammonium salts thereof, if desired.

**10.** A process according to claim 9, wherein a benzodiazepine derivative of formula Ib is prepared, wherein

| | |
|---|---|
| $R^1$ | is methyl, |
| $R^2$ | is unsubstituted phenyl, |
| $R^3$ | is as defined in claim 9 wherein |
| q and n | is 0 and |
| $R^7$ | is as defined in claim 9, wherein, however, in the indolyl group $X^2$ and $X^3$ are hydrogen. |

**11.** A process according to claim 9 for the preparation of the compound 3(S)−(−)−1,3−dihydro−3−(2−indolecarbonylamino)−1−methyl−5−phenyl−2H−1,4−benzodiazepin−2−one.

## Patentansprüche

## Patentansprüche für folgende Vertragsstaaten : BE, CH, DE, FR, GB, IT, LI, LU, NL, SE

**1.** Verwendung von Verbindungen der Formel I

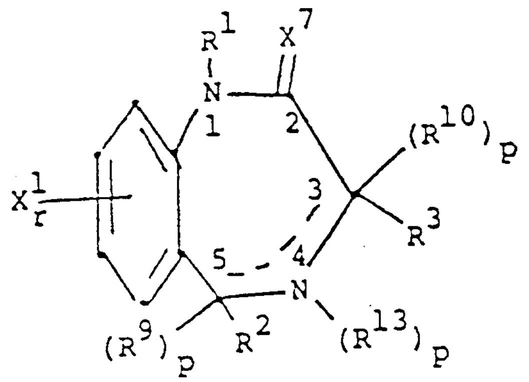

I

oder eines pharmazeutisch verträglichen Salzes oder eines pharmazeutisch verträglichen quaternären Ammoniumsalzes der Verbindung der Formel I,
wobei bei den Verbindungen der Formel I

$R^1$ Wasserstoff, Alkyl mit 1 bis 5 Kohlenstoffatomen, Alkenyl mit bis zu 5 Kohlenstoffatomen, Alkinyl mit bis zu 5 Kohlenstoffatomen, oder eine Gruppe der Formeln $-(CH_2)_mCOOR^6$, $-(CH_2)_n-C_3-C_5-$Cycloalkyl, $-(CH_2)_mNR^4R^5$, $-(CH_2)_mCONR^4R^5$, $-(CH_2)_mCN$ oder $-(CH_2)_nCX^{10}_3$ ist,
wobei $R^4$ und $R^5$ unabhängig voneinander Wasserstoff, Alkyl mit 1 bis 4 Kohlenstoffatomen oder Cycloalkyl mit 3 bis 5 Kohlenstoffatomen ist,
$R^6$ Wasserstoff, Alkyl mit 1 bis 4 Kohlenstoffatomen, Cycloalkyl mit 3 bis 5 Kohlenstoffatomen, unsubstituiertes Phenyl, substituiertes Phenyl, unsubstituiertes Phenyl−alkyl oder substituiertes Phenyl−alkyl ist, wobei bei den Gruppen die Substituenten des Phenylrestes 1 oder 2 Substituenten sind, ausgewählt aus Halogen, Alkyl mit 1 bis 4 Kohlenstoffatomen, Alkoxy mit 1 bis 4 Kohlenstoffatomen, Nitro oder Trifluormethyl sind und wobei die Alkylgruppe der entsprechenden substituierten oder unsubstituierten Phenyl−alkyl−Gruppe 1 bis 4 Kohlenstoffatome enthält,
$X^{10}$ Fluor, Chlor oder Brom ist,
m 1 bis 4 ist und
n 0 bis 4 ist;

$R^2$ Wasserstoff, Alkyl mit 1 bis 4 Kohlenstoffatomen, unsubstituiertes Phenyl, substituiertes Phenyl mit 1 oder 2 Substituenten, ausgewählt aus Halogen, Alkyl mit 1 bis 4 Kohlenstoffatomen, Alkoxy mit 1 bis 4 Kohlenstoffatomen, Alkylthio mit 1 bis 4 Kohlenstoffatomen, Carboxy−substituiertes Alkyl mit 1 bis 4 Kohlenstoffatomen in der Alkylgruppe, Carboxyl, Nitro, Trifluormethyl und Hydroxy, ist,
oder der Rest $R^2$ eine Gruppe ist der folgenden Formeln

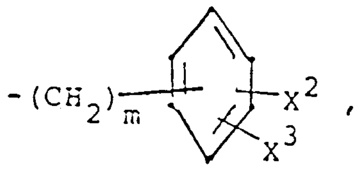

$-(CH_2)_mSCH_3$, $-(CH_2)_mSOCH_3$, $-(CH_2)_mSO_2CH_3$, oder $-(CH_2)_mCOOR^6$;
wobei
$R^6$ und m wie im Zusammenhang mit dem Rest $R^1$ definiert sind,
$X^2$ und $X^3$ unabhängig voneinander Wasserstoff, Hydroxy, Nitro, Halogen, Alkyl mit 1 bis 4 Kohlenstoffatomen, Alkylthio mit 1 bis 4 Kohlenstoffatomen oder Alkoxy mit 1 bis 4 Kohlenstoffatomen ist,

$R^3$ eine Gruppe ist der Formeln
$-(CH_2)_nR^7$,

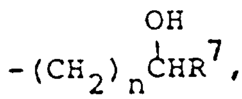
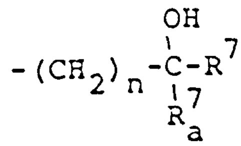

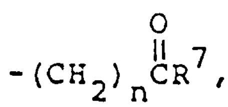

$-(CH_2)_nNR^{18}(CH_2)_qR^7$,

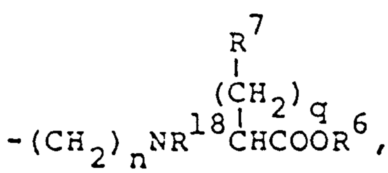

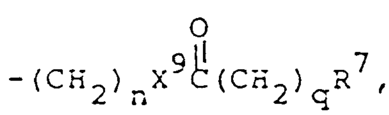

$-NH(CH_2)_{2-3}NHR^7$, $-NH(CH_2)_{2-3}$ $NHCOR^7$,

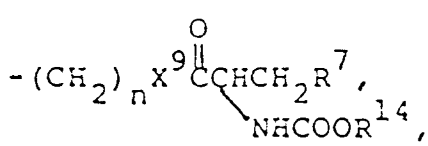

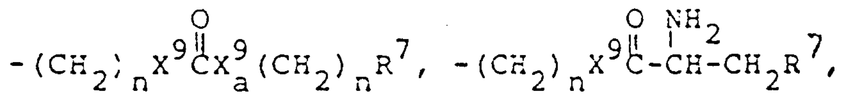

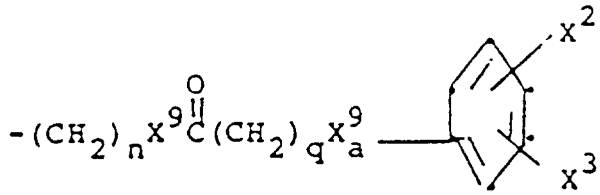

oder $-(CH_2)_nNR^{18}SO_2(CH_2)_qR^7$,
wobei
$R^7$ und $R^7{}_a$ unabhängig voneinander unsubstituiertes Phenyl oder substituiertes Phenyl mit 1 oder 2 Substituenten, ausgewählt aus Halogen, Nitro, Hydroxy, Alkyl mit 1 bis 4 Kohlenstoffatomen, oder Gruppen der Formeln $CF_3$, CN, $SCF_3$, C≡CH, $CH_2SCF_3$, $OCOCH_3$, $OCHF_2$, SH; SPh, $PO_3H$ oder $-NR^4R^5$, Alkoxy mit 1 bis 4 Kohlenstoffatomen oder Alkylthio mit 1 bis 4 Kohlenstoffatomen bedeuten, oder die Reste
$R^7$ und $R^7{}_a$ $\alpha$-Naphthyl oder $\beta$-Naphthyl oder eine Gruppe der folgenden Formeln bedeuten

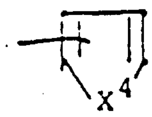 , 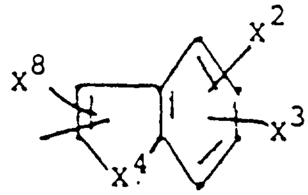 , 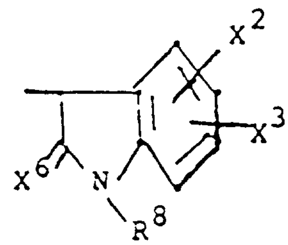

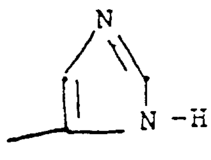 , 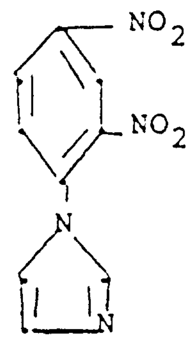 ,

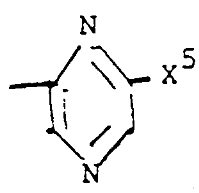 , 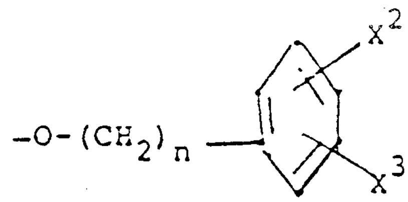 ,

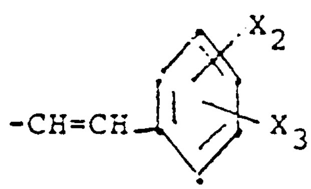 , 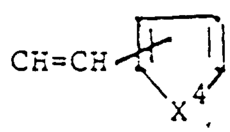

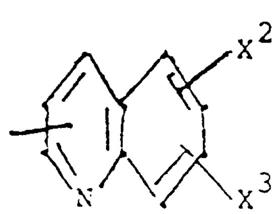 , 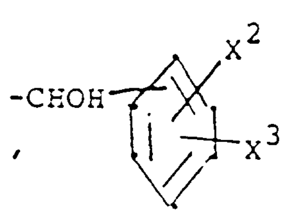 ,

oder

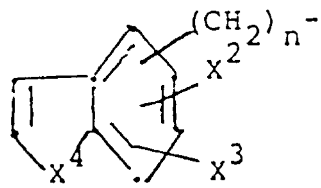

wobei

$R^8$ Wasserstoff, Alkyl mit 1 bis 4 Kohlenstoffatomen, Cycloalkyl mit 3 bis 5 Kohlenstoffatomen bedeutet, das entweder direkt oder über eine Alkylenkette, umfassend 1 bis 4 $-CH_2-$Gruppen, gebunden ist, oder $R^8$ eine Gruppe ist der Formeln $-(CH_2)_mCONH_2$, $-(CH_2)_mCOOR^6$, $-(CH_2)_mNR^4R^5$,

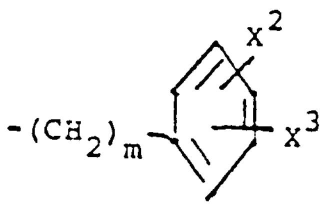 ,

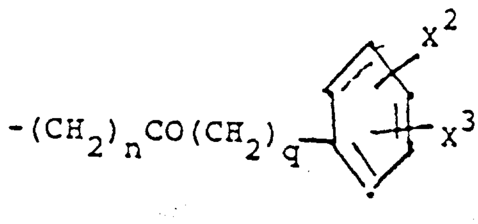

oder

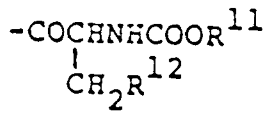 ;

wobei

$X^2$ und $X^3$ wie im Zusammenhang mit dem Rest $R^2$ definiert sind,

$X^4$ S, O, $CH_2$ oder $NR^8$ ist, wobei $R^8$ wie oben definiert ist,

$X^5$ Wasserstoff, Halogen, Nitro oder eine Gruppe der Formeln $CF_3$, CN, $-COOR^6$ ist, wobei $R^6$ wie oben definiert ist,

$X^5$ O oder HH ist,

$X^5$ Wasserstoff oder Alkyl mit 1 bis 4 Kohlenstoffatomen ist,

$X^5$ und $X^5{}_a$ unabhängig voneinander Gruppen sind der Formeln $-O-$ oder $-NR^{18}-$

wobei

$R^{18}$ Wasserstoff, Alkyl mit 1 bis 4 Kohlenstoffatomen, Formyl, Acety, Propionyl oder Butyryl ist

$R^{11}$ und $R^{12}$ unabhängig voneinander Alkyl mit 1 bis 4 Kohlenstoffatomen oder Cycloalkyl mit 3 bis 5 Kohlenstoffatomen sind,

$R^{14}$ Alkyl mit 1 bis 4 Kohlenstoffatomen oder Phenylalkyl ist, wobei die Alkylgruppe 1 bis 4 Kohlenstoffatomen enthält,

$R^9$ und $R^{10}$ unabhängig voneinander Wasserstoff, Hydroxy oder Methyl bedeuten,

$R^{13}$ Wasserstoff, Alkyl mit 1 bis 4 Kohlenstoffatomen, Cycloalkyl mit 3 bis 5 Kohlenstoffatomen, $-O$, Formyl, Acetyl, Propionyl oder Butyryl bedeutet,

$X^1$ Wasserstoff, Halogen, Alkyl mit 1 bis 4 Kohlenstoffatomen, Alkylthio mit 1 bis 4 Kohlenstoffatomen, Alkoxy mit 1 bis 4 Kohlenstoffatomen oder eine Gruppe bedeutet der Formeln $-NO_2$, $CF_3$, CN, OH, $-(CH_2)_nCOCR^6$ oder $-NR^4R^5$, wobei $R^4$, $R^5$, $R^6$ und n wie im Zusammenhang mit $R^1$ definiert sind,

r 1 oder 2 ist,

$X^7$ O, S, HH oder $NR^{15}$ ist, wobei

$R^{15}$ Wasserstoff, Alkyl mit 1 bis 4 Kohlenstoffatomen oder eine Gruppe ist der Formeln

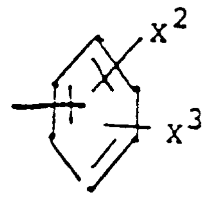

oder $-NR^{16}R^{17}$
wobei $R^{16}$ und $R^{17}$ Wasserstoff oder eine Gruppe bedeuten der Formel

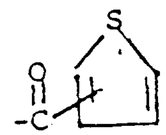

jedoch mit der Maßgabe, daß
$X^7$ nur dann $NR^{15}$ sein kann, wenn $R^1$ nicht H ist;
q 0 bis 4 ist,
– – – eine Einfach– oder Doppelbindung angibt,
p 0 ist, wenn die angrenzende – – – Bindung eine Doppelbindung ist, und 1, wenn die angrenzende – – – Bindung eine Einfachbindung ist, außer wenn $R^{13}$ Sauerstoff ist und p 1 ist und – – – eine Doppelbindung ist, und mit der Maßgabe, daß, wenn $R^3$ eine Gruppe ist der Formel $-(CH_2)_nNH(CH_2)_qR^7$, in der q 0 oder 1 ist, oder eine Gruppe der Formel

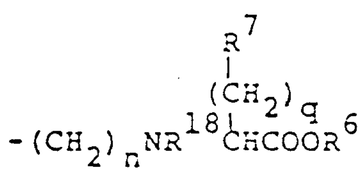

in der q 0 ist, dann $R^7$ eine andere Bedeutung haben muß als eine Gruppe der Formel

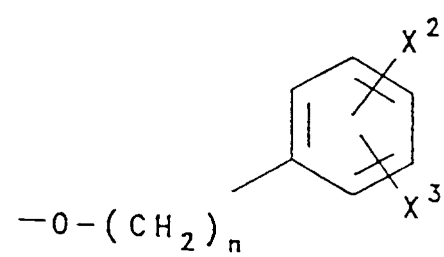

zur Herstellung eines Arzneimittels zur Verwendung als Antagonist gegenüber Cholecystokinin in gastrointestinalem (Magen – Darm – ) Gewebe und als Antagonist gegenüber Gastrin durch Bindung an den entsprechenden Cholecystokinin – Rezeptor oder Gastrin – Rezeptor.

**2.** Verwendung nach Anspruch 1, dadurch gekennzeichnet, daß zur Herstellung eines Arzneimittels als pharmazeutisch wirksamer Bestandteil eine Verbindung der Formel I oder ein pharmazeutisch annehmbares Salz oder ein pharmazeutisch annehmbares quaternäres Ammoniumsalz der Verbindung der Formel I verwendet wird, wobei bei der Verbindung der Formel I

$R^1$ Wasserstoff, Alkyl mit 1 bis 5 Kohlenstoffatomen oder eine Gruppe der Formel $-(CH_2)_mCOOR^6$ ist, wobei
$R^6$ und m wie in Anspruch 1 definiert sind,

$R^2$ eine Gruppe der Formel $-(CH_2)_mCOOR^6$ ist, wobei $R^6$ und m wie oben definiert sind, oder $R^2$ unsubstituiertes Phenyl, substituiertes Phenyl mit 1 oder 2 Substituenten, ausgewählt aus Halogen, Alkyl mit 1 bis 4 Kohlenstoffatomen, Alkoxy mit 1 bis 4 Kohlenstoffatomen, Carboxy-substituiertes Alkyl mit 1 bis 4 Kohlenstoffatomen in der Alkylgruppe, Carboxyl, Nitro, Trifluormethyl und Hydroxy ist,

$R^3$ eine Gruppe ist der Formeln

$-(CH_2)_nR^7$,

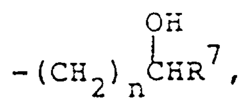

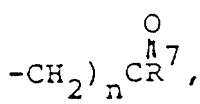

$-(CH_2)_nNH(CH_2)_qR^7$,

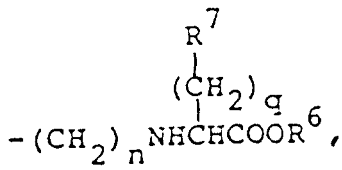

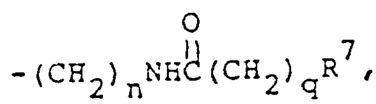

$-NH(CH_2)_{2-3}NHR^7$,

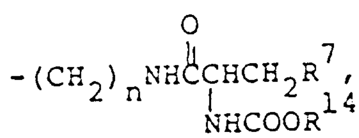

$-NH(CH_2)_{2-3}NHCOR^7$ oder

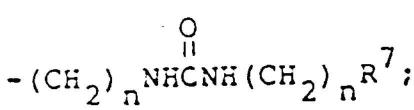

wobei

$R^7$ unsubstituiertes Phenyl oder substituiertes Phenyl mit 1 oder 2 Substituenten, ausgewählt aus Halogen, Nitro, Hydroxy, Alkyl mit 1 bis 4 Kohlenstoffatomen, Alkoxy mit 1 bis 4 Kohlenstoffatomen, Alkylthio mit 1 bis 4 Kohlenstoffatomen, Trifluormethyl oder Gruppen der Formeln CN, $-C{\equiv}CH$, $SCF_3$, $OCCH_3$, $OCHF_2$, SPh, oder $-NR^4R^5$ ist,

wobei $R^4$ und $R^5$ unabhängig voneinander Wasserstoff oder Alkyl mit 1 bis 4 Kohlenstoffatomen sind, oder der Rest

$R^7$ α-Naphthyl oder β-Naphthyl ist oder eine Gruppe ist der Formeln

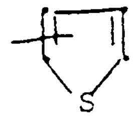 ,

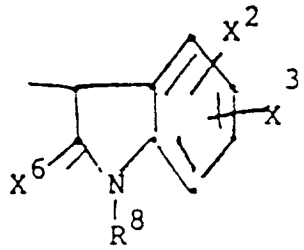 ,

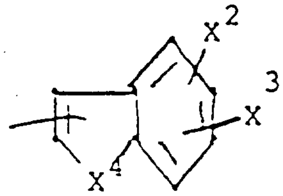 ,

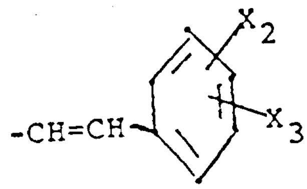 oder 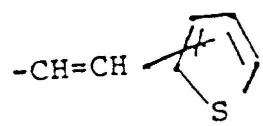

wobei $R^8$ Wasserstoff, Alkyl mit 1 bis 4 Kohlenstoffatomen ist oder eine Gruppe der Formel

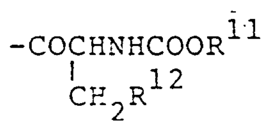

wobei
$R^{11}$ und $R^{12}$ unabhängig voneinander Alkyl mit 1 bis 4 Kohlenstoffatomen sind,
$X^2$ und $X^3$ unabhängig voneinander Wasserstoff, Hydroxy, Nitro, Halogen, Alkylthio mit 1 bis 4 Kohlenstoffatomen, Alkyl mit 1 bis 4 Kohlenstoffatomen oder Alkoxy mit 1 bis 4 Kohlenstoffatomen sind,
$X^4$ S, O, oder $NR^8$ ist, wobei $R^8$ wie oben definiert ist,
$X^6$ O oder HH ist,
$R^{14}$ Alkyl mit 1 bis 4 Kohlenstoffatomen ist und
q 0 bis 4 ist,

| | |
|---|---|
| $R^9$ und $R^{10}$ | unabhängig voneinander Wasserstoff, Hydroxy oder Methyl sind, |
| $R^{13}$ | Wasserstoff, Alkyl mit 1 bis 4 Kohlenstoffatomen −O, Formyl, Acetyl, Propionyl oder Butyryl ist, |
| $X^1$ | Wasserstoff, Halogen, Alkyl mit 1 bis 4 Kohlenstoffatomen, Alkylthio mit 1 bis 4 Kohlenstoffatomen, Alkoxy mit 1 bi 4 Kohlenstoffatomen oder eine Gruppe ist der Formeln $-NO_2$, $CF_3$, CN, OH, $-(CH_2)_nCOOR^6$ oder $-NR^4R^5$, wobei |
| $R^6$ | wie im Zusammenhang mit dem Rest $R^1$ definiert ist und $R^4$ und $R^5$ wie im Zusammenhang mit dem Rest $R^3$ definiert sind, |
| $X^7$ | O ist, |
| m | 1 bis 4 ist, |
| n | 0 bis 4 ist, |
| − − − | eine Einfachbindung oder eine Doppelbindung angibt, |
| p | 0 ist, wenn die angrenzende − − − Bindung eine Doppelbindung ist, und 1, wenn die angrenzende − − − Bindung eine Einfachbindung ist, außer wenn $R^{13}$ Sauerstoff ist und p 1 ist und − − − eine Doppelbindung ist und |
| r | 1 oder 2 ist. |

3. Verwendung nach Anspruch 2, dadurch gekennzeichnet, daß zur Herstellung eines Arzneimittels als pharmazeutisch wirksamer Bestandteil eine Verbindung der Formel I oder ein pharmazeutisch annehmbares Salz oder ein pharmazeutisch annehmbares quaternäres Ammoniumsalz der Verbindung der Formel I verwendet wird, wobei

$R^1$ Wasserstoff, Methyl, Ethyl, Carboxymethyl oder Ethylcarboxymethyl ist,

$R^2$ eine Gruppe ist der Formel $-(CH_2)_{1-2}COOR^6$, wobei $R^6$ Wasserstoff oder Alkyl mit 1 bis 4 Kohlenstoffatomen ist, oder

$R^2$ unsubstituiertes Phenyl, substituiertes Phenyl mit 1 oder 2 Substituenten, die Halogen oder Carboxyl sind, bedeutet,

$R^3$ eine Gruppe ist der Formeln

$-(CH_2)_nR^7$,

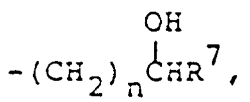

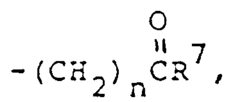

$-(CH_2)_nNH(CH_2)_qR^7$,

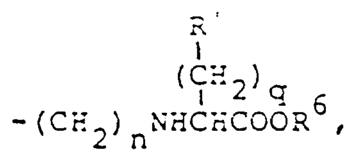,
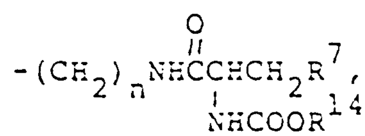

oder

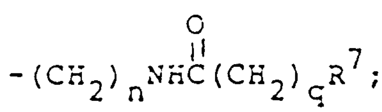

wobei

$R^7$ α-Naphthyl, β-Naphthyl oder substituiertes Phenyl mit 1 oder 2 Substituenten ist, ausgewählt aus Halogen, Trifluormethyl und Alkyl mit 1 bi 4 Kohlenstoffatomen ist oder

$R^7$ eine Gruppe ist der Formeln

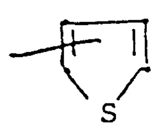,

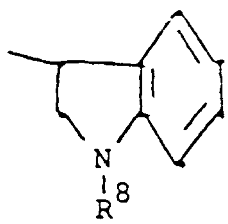 , 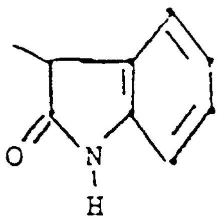 ,

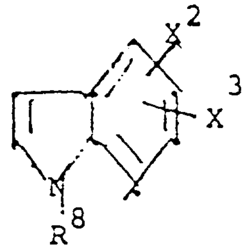 ,

oder

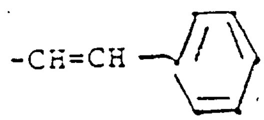

wobei
$R^8$ Wasserstoff, Methyl oder Ethyl ist
$X^2$ und $X^3$ unabhängig voneinander Wasserstoff, Hydroxy, Nitro oder Halogen ist,
$R^{14}$ tert.-Butyl ist,
n 0 bis 4 ist,
q 0 bis 4 ist, und
$R^6$ wie im Zusammenhang mit dem Rest $R^2$ definiert ist,

| | |
|---|---|
| $R^9$ und $R^{10}$ | unabhängig voneinander Wasserstoff, Hydroxy oder Methyl sind, |
| $R^{13}$ | Wasserstoff, Methyl oder Formyl ist, |
| $X^1$ | Wasserstoff, Halogen oder eine Gruppe ist der Formeln $-NO_2$, $CF_3$, CN oder OH ist, |
| $X^7$ | O ist, |
| - - - | eine Einfach- oder Doppelbindung angibt, |
| p | 0 ist, wenn die angrenzende - - - Bindung eine Doppelbindung ist und 1, wenn die angrenzende - - - Bindung eine Einfachbindung ist, und |
| r | 1 oder 2 ist. |

**4.** Verwendung nach Anspruch 3, dadurch gekennzeichnet, daß zur Herstellung eines Arzneimittels als pharmazeutisch wirksamer Bestandteil eine Verbindung der Formel I oder ein pharmazeutisch annehmbares Salz oder ein pharmazeutisch annehmbares quaternäres Ammoniumsalz der Verbindung der Formel I verwendet wird, wobei bei der Formel I

| | |
|---|---|
| $R^1$ | Wasserstoff, Methyl, oder Carboxmethyl ist |
| $R^2$ | Phenyl, o-Fluorphenyl, p-Fluorphenyl, o-Chlorphenyl, p-Chlorphenyl, o-Carboxyphenyl, 2,4-Dichlorphenyl, 2,6-Difluorphenyl, $-CH_2COOEt$, $-CH_2COO-t-Bu$, $-CH_2CH_2COOEt$ oder $CH_2CH_2COO-t-Bu$ ist |
| $R^3$ | $-(CH_2)_{1-2}R^7$, |

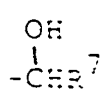 , 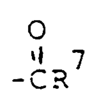 ,

$-(CH_2)_{0-1}NH(CH_2)_{1-2}R^7$,

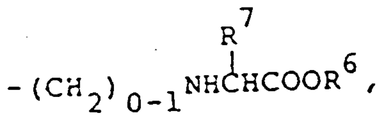 , 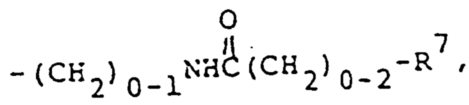

oder

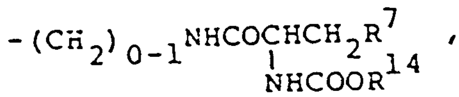

ist,
und die Stöchiometrie sich auf D-Tryptophan bezieht,

$R^7$ α-Naphthyl, β-Naphthyl, Monohalogen-phenyl oder Dihalogen-phenyl oder eine Gruppe ist der Formeln

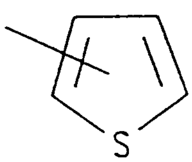 ,

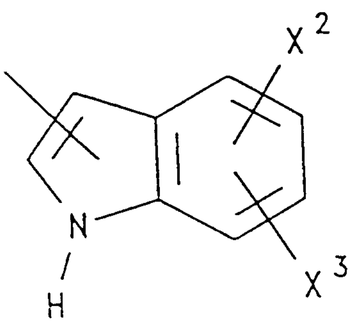 ,

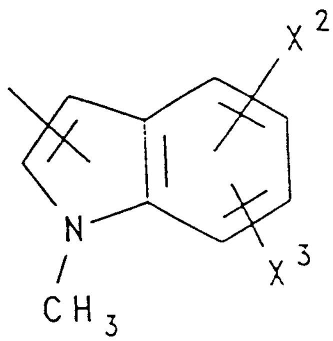

wobei
$X^2$ und $X^3$ unabhängig voneinander Wasserstoff, Hydroxy, Fluor oder Chlor sind,
$R^6$ Wasserstoff, Methyl oder Ethyl ist
$R^{14}$ tert.-Butyl ist,

$R^9$ und $R^{10}$ unabhägig voneinander Wassserstoff oder Hydroxy sind,
$R^{13}$ Wasserstoff ist,
$X^1$ Wasserstoff, 7-Chlor, 7-Fluor oder 7-Nitro ist,
$X^7$ Sauerstoff ist
--- eine Einfach- oder Doppelbindung angibt,
p 0 ist, wenn die angrenzende --- Bindung eine Doppelbindung ist und 1, wenn die angrenzende --- Bindung eine Einfachbindung ist, und
r 1 ist.

**5.** Verwendung naach Anspruch 1 dadurch gekennzeichnet, daß die Verbindung der Formel I
1,3-Dihydro-5-(2-fluorophenyl)-3(R)-(3'-indolyl)methyl-2H-1,4-benzodiazepin-2-on
1,3-Dihydro-5-(2-fluor-phenyl)-3(R)-[3'-(1'-methylindolyl)-methyl]-1-methyl-2H-1,4-benzodiazepin-2-on

1,3 – Dihydro – 5 – (2 – fluor – phenyl) – 3(R) – (3' – indolyl)methyl – 1 – methyl – 2H – 1,4 – benzodiazepin – 2 – on,

7 – Chlor – – 1,3 – dihydro – 3(R) – (3' – indolyl)methyl – 5 – phenyl – 2H – 1,4 – benzodiazepin – 2 – on,

1,3 – Dihydro – 3(R) – (3' – indolyl)methyl – 5 – phenyl – 2H – 1,4 – benzodiazepin – 2 – on,

1,3 – Dihydro – 5 – (2 – fluor – phenyl) – 3(R) – (3' – α – indolenyl)methyl – 2H – 1,4 – benzodiazepin – 2 – on,

1,3 – Dihydro – 5 – (2 – fluor – phenyl) – 3(R) – (3' – β – indolenyl)methyl – 2H – 1,4 – benzodiazepin – 2 – on,

1,3 – Dihydro – 5 – (2 – fluor – phenyl) – 3(R) – (3' – indolyl)methyl – 2H – 1,4 – benzodiazepin – 2 – thion,

1,3 – Dihydro – 5 – (2 – fluor – phenyl) – 3(R) – (3' – indolyl)methyl – 2H – 1,4 – benzodiazepin,

7 – Chlor • 1,3 – dihydro – 3(R) – benzyl – 5 – phenyl – 2H – 1,4 – benzodiazepin – 2 – on,

3(R) – Benzyloxymethyl – 7 – chlor – 1,3 – dihydro – 5 – phenyl – 2H – 1,4 – benzodiazepin – 2 – on,

7 – Chlor – – 1,3 – dihydro – 3(RS) – (1 – naphthyl)methyl – 5 – phenyl – 2H – 1,4 – benzodiazepin – 2 – on,

7 – Chlor – – 1,3 – dihydro – 3(RS) – (2 – naphthyl)methyl – 5 – phenyl – 2H – 1,4 – benzodiazepin – 2 – on,

1,3 – Dihydro – 5 – (2 – fluor – phenyl) – 3(RS) – (2 – thienyl)methyl – 2H – 1,4 – benzodiazepin – 2 – on,

1,3 – Dihydro – 5 – (2 – fluor – phenyl) – 3(RS) – (3 – thienyl) – 2H – 1,4 – benzodiazepin – 2 – on,

1,3 – Dihydro – 5 – (2 – fluor – phenyl) – 3(R) – [3' – β – (1' – t – Boc – L – leucyl) – indolenyl]methyl – 2H – 1,4 – benzodiazepin – 2 – on,

1,3 – Dihydro – 5 – (2 – fluor phenyl) – 3(R) – [3' – β – (1' – t – Boc – D – leucyl) – indolenyl]methyl – 2H – 1,4 – benzodiazepin – 2 – on,

1,3 – Dihydro – 5 – (2 – fluor – phenyl) – 3(R) – [3' – α – (1' – t – Boc – L – leucyl) – indolenyl]methyl – 2H – 1,4 – benzodiazepin – 2 – on,

1,3 – Dihydro – 5 – (2 – fluor – phenyl) – 3(R) – [3' – α – (1' – t – Boc – D – leucyl) – indolenyl]methyl – 2H – 1,4 – benzodiazepin – 2 – on,

7 – Chlor – 1,3,4,5 – tetrahydro – 3(R) – (3' – indolyl)methyl – 5 – phenyl – 2H – 1,4 – benzodiazepin – 2 – on,

7 – Chlor – 1,3,4,5 – tetrahydro – 3(S) – (3' – indolyl)methyl – 5 – phenyl – 2H – 1,4 – benzodiazepin – 2 – on,

4 – (p – Chlor – benzoyl) – 5 – (2 – fluor – phenyl) – 3(R) – [3' – (1' – methylindolyl) – methyl] – 1 – methyl – 1,3,4,5 – tetrahydro – 2H – 1,4 – benzodiazepin – 2 – on,

4 – Acetyl – 5 – (2 – fluor – phenyl) – 3(R) – [3' – (1' – methylindolyl)methyl] – 1 – methyl – 1,3,4,5 – tetrahydro – 2H – 1,4 – benzodiazepin – 2 – on,

7 – Chlor – 5 – (2 – chlor – phenyl) – 1,3 – dihydro – 3(R) – (3' – indolyl)methyl – 2H – 1,4 – benzodiazepin – 2 – on,

1,3 – Dihydro – 3(R) – (3' – indolyl)methyl – 5 – methyl – 2H – 1,4 – benzodiazepin – 2 – on,

1 – Benzyl – 7 – chlor – 1,3 – dihydro – 3(R) – (3' – indolyl)methyl – 5 – phenyl – 2H – 1,4 – benzodiazepin – 2 – on,

7 – Chlor – 1,3 – dihydro – 3(R) – (3' – indolyl)methyl – 1 – methyl – 5 – phenyl – 2H – 1,4 – benzodiazepin – 2 – on,

1,3 – Dihydro – 5 – (2 – fluor – phenyl) – 3(S) – (3' – indolyl)methyl – 2H – 1,4 – benzodiazepin – 2 – on,

1 – Benzyl – 7 – chlor – 1,3 – dihydro – 3(S) – (3' – indolyl)methyl – 5 – phenyl – 2H – 1,4 – benzodiazepin – 2 – on,

7 – Chlor – 1,3 – dihydro – 3(R) – (3' – indolyl)methyl – 5 – phenyl – 2H – 1,4 – benzodiazepin – 2 – thion,

1,3 – Dihydro – 5 – (2 – fluor – phenyl) – 3(R) – (3' – indolyl)methyl – 2H – 1,4 – benzodiazepin – 2 – [N' – (3 – thienoyl)] hydrazid,

1,3 – Dihydro – 1 – ethyl – 5 – (2 – fluor – phenyl) – 3(R) – (3' – indolyl)methyl – 2H – 1,4 – benzodiazepin – 2 – on,

1 – Cyclopropylmethyl – 1,3 – dihydro – 5 – (2 – fluor – phenyl) – 3(R) – (3' – indolyl)methyl – 2H – 1,4 – benzodiazepin – 2 – on,

1,3 – Dihydro – 5 – (2 – fluor – phenyl) – 3(R) – (3' – indolyl)methyl – 1 – pentyl – 2H – 1,4 – benzodiazepin – 2 – on,

1,3 – Dihydro – 5 – (2 – fluor – phenyl) – 3(R) – (3' – indolyl)methyl – 1 – (3 – methylbutyl) – 2H – 1,4 – benzodiazepin – 2 – on,

1,3 – Dihydro – 5 – (2 – fluor – phenyl) – 3(R) – (3' – indolyl)methyl – 1 – (2,2,2 – trifluor – ethyl) – 2H – 1,4 – benzodiazepin – 2 – on,

1,3 – Dihydro – 1 – (2 – dimethylaminoethyl) – 5 – (2 – fluor – phenyl) – 3(R) – (3' – indolyl)methyl – 2H – 1,4 – benzodiazepin – 2 – on,

1,3 – Dihydro – 1 – (ethoxycarbonylmethyl) – 5 – (2 – fluor – phenyl) – 3(R) – (3' – indolyl)methyl – 2H – 1,4 – benzodiazepin – 2 – on,

1 – Carboxymethyl – 1,3 – dihydro – 5 – (2 – fluor – phenyl) – 3(R) – 3' – indolyl)methyl – 2H – 1,4 – benzodiazepin – 2 – on,

1,3 – Dihydro – 5 – (2 – fluor – phenyl) – 3(R) – [3' – (1' – p – chlorbenzyloylindolyl)methyl] – 1 – methyl – 2H – 1,4 – benzodiazepin – 2 – on,

7 – Chlor – 1,3 – dihydro – 3(R) – [3'(1' – benzylindolyl)methyl] – 1 – methyl – 5 – phenyl – 2H – 1,4 – benzodiazepin – 2 – on,

1,3 – Dihydro – 3(RS) – [1 – hydroxy – 1 – (3' – indolyl)]methyl – 1 – methyl – 5 – phenyl – 2H – 1,4 – benzodiazepin – 2 – on,

1,3 – Dihydro – 1 – methyl – 5 – phenyl – 3 – (RS) – (3 – thienoyl) – 2H – 1,4 – benzodiazepin – 2 – on,

1,3 – Dihydro – 3 – (RS) – [1 – hydroxy – 1 – (3 – thienyl)]methyl – 1 – methyl – 5 – phenyl – 2H – 1,4 – benzodiazepin – 2 – on,

1,3 – Dihydro – 3(RS) – [1 – hydroxy – 1 – [3 – (1 – methylindolyl)]]methyl – 1 – methyl – 5 – phenyl – 2H – 1,4 – benzodiazepin – 2 – on, 2 Stereoisomere,

1,3 – Dihydro – 3(RS) – (1 – hydroxy – 1 – phenyl)methyl – 1 – methyl – 5 – phenyl – 2H – 1,4 – benzodiazepin – 2 – on,

1,3 – Dihydro – 3(RS) – [1 – hydroxy – 1 – (2 – thienyl)]methyl – 1 – methyl – 5 – phenyl – 2H – 1,4 – benzodiazepin – 2 – on,

1,3 – Dihydro – 3 – (RS) – hydroxy – 1 – methyl – 5 – phenyl – 3 – (3' – thienoyl) – 2H – 1,4 – benzodiazepin – 2 – on,

1,5 – Dihydro – 5 – (RS) – hydroxy – 1 – methyl – 5 – phenyl – 3 – (3' – thienoyl) – 2H – 1,4 – benzodiazepin – 2 – on,

7 – Chlor – 1,3 – dihydro – 3(R) – [(2',3' – dihydro – 2' – oxo – 1'H – indol – 3' – yl)methyl] – 5 – phenyl – 2H – 1,4 – benzodiazepin – 2 – on,

7 – Chlor – 1,3 – dihydro – 3(R) – [(3' – (2,4 – dinitrophenyl)imidazol – 5' – yl) – methyl] – 5 – phenyl – 2H – 1,4 – benzodiazepin – 2 – on,

7 – Chlor – 1,3 – dihydro – 3(R) – (3' – imidazol – 5' – yl)methyl – 5 – phenyl – 2H – 1,4 – benzodiazepin – 2 – on,

3(RS) – [3' – (5' – Brom – indolyl)methyl] – 1,3 – dihydro – 5 – phenyl – 2H – 1,4 – benzodiazepin – 2 – on,

5 – o – Carboxyphenyl – 1,3 – dihydro – 3(R) – (3' – indolyl)methyl – 2H – 1,4 – benzodiazepin – 2 – on,

1,3 – Dihydro – 3(RS) – [3' – (5' – fluor – indolyl)methyl] – 5 – o – fluor – phenyl – 2H – 1,4 – benzodiazepin – 2 – on,

1,3 – Dihydro – 3(RS) – [3' – (6' – fluor – indolyl)methyl] – 5 – o – fluor – phenyl – 2H – 1,4 – benzodiazepin – 2 – on,

2 – N – [2(RS),3 – bis – (Bocamino)propanoyl]amino – 2' – fluor benzophenon,

2 – N – [2(RS),3 – Diphthalylaminopropanoyl]amino – 2' – fluor – benzophenon,

1,3 – Dihydro – 5 – (2' – fluor – phenyl) – 3(RS) – aminomethyl – 2H – 1,4 – benzodiazepin – 2 – on,

1,3 – Dihydro – 5 – (2' – fluor – phenyl) – 3(R) – (4 – amino)butyl – 2H – 1,4 – benzodiazepin – 2 – on,

1,3 – Dihydro – 5 – (2' – fluor – phenyl) – 3(RS) – benzyloxycarbonylaminomethyl – 2H – 1,4 – benzodiazepin – 2 – on,

1,3 – Dihydro – 5 – (2' – fluor – phenyl) – 3(RS) – (3 – thiophencarbonyl)aminomethyl – 2H – 1,4 – benzodiazepin – 2 – on,

1,3 – Dihydro – 5 – (2' – fluor – phenyl) – 3(RS) – (2 – indol ) – carbonylaminomethyl) – 2H – 1,4 – benzodiazepin – 2 – on,

1,3 – Dihydro – 5 – (2' – fluor – phenyl) – 3(RS) – (6' – chlorpyrazinyl)aminomethyl – 2H – 1,4 – benzodiazepin – 2 – on,

1,3 – Dihydro – 5 – (2' – fluor – phenyl) – 3(RS) – aminomethyl – 1 – methyl – 2H – 1,4 – benzodiazepin – 2 – on,

3(RS) – (2 – Indolecarbonylamino) – 1,3 – dihydro – 5 – phenyl – 2H – 1,4 – benzodiazepin – 2 – on,

1,3 – Dihydro – 3(RS) – [2 – (3 – indolyl)ethyl]amino – 5 – phenyl – 2H – 1,4 – benzodiazepin – 2 – on,

3(RS) – [3 – (3 – Indol )propionylamino] – 1,3 – dihydro – 5 – phenyl – 2H – 1,4 – benzodiazepin – 2 – on,

3(RS) – (3 – Indol – acetylamino) – 1,3 – dihydro – 5 – phenyl – 2H – 1,4 – benzodiazepin – 2 – on,
3(RS) – (Boc – L – Tryptophanyl)amino – 1,3 – dihydro – 5 – phenyl – 2H – 1,4 – benzodiazepin – 2 – on,
Z – 1,3 – Dihydro – 1 – methyl – 5 – phenyl – 3 – (3 – thienylmethylen ) – 2H – 1,4 – benzodiazepin – 2 – on,
E – 1,3 – Dihydro – 1 – methyl – 5 – phenyl – 3 – (3 – thienylmethylen ) – 2H – 1,4 – benzodiazepin – 2 – on,
3(RS) – (BOC – D – Tryptophyl)amino – 1,3 – dihydro – 5 – phenyl – 2H – 1,4 – benzodiazepin – 2 – on,
3(RS) – [4 – (3 – Indol )butyrylamino] – 1,3 – dihydro – 5 – phenyl – 2H – 1,4 – benzodiazepin – 2 – on,
1,3 – Dihydro – 3(RS) – (benzyloxycarbonyl)aminomethyl – 5 – (2 – fluor – phenyl) – 2H – 1,4 – benzodiazepin,
1,3 – Dihydro – 3(RS) – [3' – (thiophen )carbonyl]aminomethyl – 5 – (2 – fluor phenyl) – 2H – 1,4 – benzodiazepin,
1,3 – Dihydro – 3(RS) – (2 – indol – carbonyl)aminomethyl – 5 – (2 – fluor – phenyl) – 2H – 1,4 – benzodiazepin,
1,3 – Dihydro – 3(RS) – (2 – L – hydroxy – 2 – phenylacetyl)aminomethyl – 5 – (2 – fluor – phenyl) – 2H – 1,4 – benzodiazepin,
1 – (2 – Cyanoethyl) – 1,3 – dihydro – 5 – (2 – fluor – phenyl) – 3(R) – (3' – indolyl)methyl – 2H – 1,4 – benzodiazepin – 2 – on,
1 – (2 – Cyanoethyl) – 1,3 – dihydro – 5 – (2 – fluor – phenyl) – 3(R) – [1' – (2 – cyanoethyl) – 3' – indolyl] – methyl – 2H – 1,4 – benzodiazpin – 2 – on,
1 – (2 – Carboxyethyl) – 1,3 – dihydro – 5 – (2 – fluor – phenyl) – 3(R) – (3' – indolyl)methyl – 2H – 1,4 – benzodiazepin – 2 – on,
1,3 – Dihydro – 3(R) – (3' – indolyl)methyl – 5 – (2 – fluor – phenyl) – 2H – 1,4 – benzodiazepin – 2 – on – 4 – oxid,
1,3 – Dihydro – 5 – (2 – fluor – phenyl) – 3 – (RS) – (2 – indol – carbonylamino) – 2H – 1,4 – benzodiazepin – 2 – on,
1,3 – Dihydro – 5 – (2 – fluor – phenyl) – 3(RS) – (2 – indol – carbonylamino) – 1 – methyl – 2H – 1,4 – benzodiazepin – 2 – on,
1,3 – Dihydro – 5 – (2 – fluor – phenyl) – 1 – methyl – 3(RS) – [2' – (1 – methylindol )carbonylamino] – 2H – 1,4 – benzodiazepin – 2 – on,
1,3 – Dihydro – 3(RS) – (4 – nitrophenylcarbonyl)amino – 5 – (2 – fluor – phenyl) – 2H – 1,4 – benzodiazepin – 2 – on,
1,3 – Dihydro – 3(RS) – (2 – indol – carbonyloxy) – 5 – phenyl – 2H – 1,4 – benzodiazepin – 2 – on,
1,3 – Dihydro – 5 – (2 – fluor – phenyl) – 3(RS) – (3 – thiophencarbonylamino) – 2H – 1,4 – benzodiazepin – 2 – on,
1,3 – Dihydro – 3(RS) – (3 – indol – carbonylamino) – 5 – phenyl – 2H – 1,4 – benzodiazepin – 2 – on,
1,3 – Dihydro – 3(RS) – (4 – thianaphthen – acetyl)amino – 5 – (2 – fluor – phenyl) – 2H – 1,4 – benzodiazepin – 2 – on,
1,3 – Dihydro – 3(RS) – (4 – chlor – phenylcarbonyl)amino – 5 – (2 – fluor – phenyl) – 2H – 1,4 – benzodiazepin – 2 – on,
1,3 – Dihydro – 3(RS) – (4 – methylphenylsulfonyl)amino – 5 – (2 – fluor – phenyl) – 2H – 1,4 – benzodiazepin – 2 – on,
1 – Carboxymethyl – 1,3 – dihydro – 5 – (2 – fluor – phenyl) – 3(RS) – (2 – indol – carbonylamino) – 2H – 1,4 – benzodiazepin – 2 – on,
1,3 – Dihydro – 3(RS) – (5 – fluor – indol – 2 – carbonylamino) – 5 – (2 – fluor – phenyl) – 2H – 1,4 – benzodiazepin – 2 – on,
1,3 – Dihydro – 3(RS) – (3' – methylindenyl – 2 – carbonyl)amino – 5 – (2 – fluor – phenyl) – 2H – 1,4 – benzodiazepin – 2 – on,
1,3 – Dihydro – 3(RS) – (2 – Chinaldyl)amino – 5 – (2 – fluor – phenyl) – 2H – 1,4 – benzodiazepin – 2 – on,
1,3 – Dihydro – 3(RS) – (2 – L – hydroxy – 2 – phenylacetyl)amino – 5 – (2 – fluor – phenyl) – 2H – 1,4 – benzodiazepin – 2 – on,
1,3 – Dihydro – 3(RS) – (5 – chlor – indol – 2 – carbonylamino) – 5 – (2 – fluor – phenyl) – 2H – 1,4 – benzodiazepin – 2 – on,
3(RS) – [N – (2 – Indol – carbonyl) – N – methylamino] – 1,3 – dihydro – 5 – phenyl – 2H – 1,4 – benzodiazepin – 2 – on,
1,3 – Dihydro – 3(RS) – (2 – indol – carbonylamino) – 1 – methyl – 5 – phenyl – 2H – 1,4 – benzodiazepin – 2 – on,

1,3 – Dihydro – 1 – methyl – 3(RS) – [2 – (1 – methylindol )carbonylamino] – 5 – phenyl – 2H – 1,4 – benzodiazepin – 2 – on,
1 – Carboxymethyl – 1,3 – dihydro – 3(RS) – (2 – indolecarbonylamino) – 5 – phenyl – 2H – 1,4 – benzodiazepin – 2 – on,
1,3 – Dihydro – 3(RS) – (5 – brom – indol – 2 – carbonylamino) – 5 – (2 – fluor – phenyl) – 2H – 1,4 – benzodiazepin – 2 – on,
3(RS) – Cinnamoylamino – 1,3 – dihydro – 5 – (2 – fluor – phenyl) – 2H – 1,4 – benzodiazepin – 2 – on,
1,3 – Dihydro – 3(RS) – (5 – hydroxy – 2 – indolylcarbonyl)amino – 5 – (2 – fluor – phenyl) – 2H – 1,4 – benzodiazepin – 2 – on,
1 – Carboxamidomethyl – 1,3 – dihydro – 3R – (3 – indolylmethyl) – 5 – (2 – fluor – phenyl) – 2H – 1,4 – benzodiazepin – 2 – on,
1,3 – Dihydro – 5 – (2 – fluor – phenyl) – 3 – (RS) – (2 – indolylmethylamino) – 2H – 1,4 – benzodiazepin – 2 – on,
1,3 – Dihydro – 3(RS) – (phenylaminomethylcarbonyl)amino – 5 – (2 – fluor – phenyl) – 2H – 1,4 – benzodiazepin – 2 – on,
1,3 – Dihydro – 3(RS) – (5 – methoxyindol – 2 – carbonylamino) – 5 – (2 – fluorophenyl) – 2H – 1,4 – benzodiazepin – 2 – one;
1,3 – Dihydro – 3(RS) – (1 – methylindol – 2 – carbonylamino) – 5 – (2 – fluor – phenyl) – 2H – 1,4 – benzodiazepin – 2 – on,
1,3 – Dihydro – 1 – methyl – 3(RS) – (4 – chlor – phenylcarbonyl)amino – 5 – (2 – fluor – phenyl) – 2H – 1,4 – benzodiazepin – 2 – on,
1,3 – Dihydro – 5 – (2 – fluor – phenyl) – 3(RS) – (2 – benzofurancarbonylamino) – 2H – 1,4 – benzodiazepin – 2 – on,
1 – Ethoxycarbonylmethyl – 1,3 – dihydro – 3(RS) – (4 – chlor – phenylcarbonyl)amino – 5 – (2 – fluor – phenyl) – 2H – 1,4 – benzodiazepin – 2 – on,
1,3 – Dihydro – 3(RS) – (4 – chlor – phenylcarbonyl)amino – 5 – phenyl – 2H – 1,4 – benzodiazepin – 2 – on,
1,3 – Dihydro – 1 – methyl – 3(RS) – (4 – chlor – phenylcarbonyl)amino – 5 – phenyl – 2H – 1,4 – benzodiazepin – 2 – on,
1 – Carboxymethyl – 1,3 – dihydro – 3(RS) – (4 – chlor – phenylcarbonyl)amino – 5 – (2 – fluor – phenyl) – 2H – 1,4 – benzodiazepin – 2 – on,
3(RS) – (2(S) – tert – Butoxycarbonylamino – 3 – phenylpropanoylamino) – 1,3 – dihydro – 5 – phenyl – 2H – 1,4 – benzodiazepin – 2 – on,
3(RS) – (2(S) – tert – Butoxycarbonylamino – 3 – phenylpropanoylamino) – 1,3 – dihydro – 1 – methyl – 5 – phenyl – 2H – 1,4 – benzodiazepin – 2 – on,
3(R) – nd 3(S) – (2(S) – Amino – 3 – phenylpropanoylamino) – 1,3 – dihydro – 1 – methyl – 5 – phenyl – 2H – 1,4 – benzodiazepin – 2 – on,
3(R) – und 3(S) – Amino – 1,3 – dihydro – 1 – methyl – 5 – phenyl – 2H – 1,4 – benzodiazepin – 2 – on,
3(R) – und 3(S) – Amino – 1,3 – dihydro – 5 – (2 – fluor – phenyl) – 1 – methyl – 2H – 1,4 – benzodiazepin – 2 – on,
3(S) – ( – ) – 1,3 – Dihydro – 3 – (2 – indol – carbonylamino) – 1 – methyl – 5 – phenyl – 2H – 1,4 – benzodiazepin – 2 – on,
3(S) – ( + ) – 1,3 – Dihydro – 5 – (2 – fluor – phenyl) – 3 – (2 – indolcarbonylamino) – 1 – methyl – 2H – 1,4 – benzodiazepin – 2 – on,
3(R) – ( – ) – 1,3 – Dihydro – 5 – (2 – fluor – phenyl) – 3 – (2 – indol – carbonylamino) – 1 – methyl – 2H – 1,4 – benzodiazepin – 2 – on,
3(R) – ( – ) – 1,3 – Dihydro – 3 – (4 – chlor – benzoylamino) – 5 – (2 – fluor – phenyl) – 1 – methyl – 2H – 1,4 – benzodiazepin – 2 – on,
3(S) – ( + ) – 1,3 – Dihydro – 3 – (4 – chlor – benzoylamino) – 5 – (2 – fluor – phenyl) – 1 – methyl – 2H – 1,4 – benzodiazepin – 2 – on,
3(S) – ( – ) – 1,3 – Dihydro – 3 – (4 – brom – benzoylamino) – 1 – methyl – 5 – phenyl – 2H – 1,4 – benzodiazepin – 2 – on,
3(R) – ( + ) – 1,3 – Dihydro – 3 – (4 – brom – benzoylamino) – 1 – methyl – 5 – phenyl – 2H – 1,4 – benzodiazepin – 2 – on,
3(R) – ( + ) – 1,3 – Dihydro – 3 – (2 – indol – carbonylamino) – 1 – methyl – 5 – phenyl – 2H – 1,4 – benzodiazepin – 2 – on,
1,3 – Dihydro – 3 – (RS) – (2 – indolin – carbonylamino) – 5 – phenyl – 2H – 1,4 – benzodiazepin – 2 – on,

1,3 – Dihydro – 5 – (2 – fluor – phenyl) – 3 – (RS) – (p – trifluormethylbenzoylamino) – 2H – 1,4 – benzodiazepin – 2 – on,

1,3 – Dihydro – 5 – (2 – fluor – phenyl) – 3 – (RS) – (p – methylbenzoylamino) – 2H – 1,4 – benzodiazepin – 2 – on,

1,3 – Dihydro – 5 – (2 – fluor – phenyl) – 3 – (RS) – (p – methoxybenzoylamino) – 2H – 1,4 – benzodiazepin – 2 – on,

3 – (RS) – (o – Chlor – benzoylamino) – 1,3 – dihydro – 5 – phenyl – 2H – 1,4 – benzodiazepin – 2 – on,

3 – (RS) – (o – Chlor – benzoylmethylamino – 1,3 – dihydro – 1 – methyl – 5 – phenyl – 2H – 1,4 – benzodiazepin – 2 – on,

3 – (RS) – (o – Chlor – benzoylamino) – 1,3 – dihydro – 1 – methyl – 5 – phenyl – 2H – 1,4 – benzodiazepin – 2 – on,

3 – (RS) – (m – Chlor – benzoylamino) – 1,3 – dihydro – 5 – phenyl – 2H – 1,4 – benzodiazepin – 2 – on,

3 – (RS) – (3,4 – Dichlor – benzoylamino) – 1,3 – dihydro – 5 – phenyl – 2H – 1,4 – benzodiazepin – 2 – on,

3 – (RS) – (p – Chlor – benzoylamino) – 1,3 – dihydro – 5 – (2' – fluor – phenyl) – 1 – methyl – 4 – oxo – 2H – 1,4 – benzodiazepin – 2 – on,

1,3 – Dihydro – 5 – phenyl – 3 – (RS) – (4' – methylthiobenzoylamino) – 2H – 1,4 – benzodiazepin – 2 – on,

1 – 3 – Dihydro – 3 – (RS) – (4' – fluor – benzoylamino) – 5 – phenyl – 2H – 1,4 – benzodiazepin – 2 – on,

1,3 – Dihydro – 5 – phenyl – 3 – (RS) – (4' – trifluoromethylbenzoylamino) – 2H – 1,4 – benzodiazepin – 2 – on,

1,3 – Dihydro – 3 – (RS) – (4' – tert – butylbenzoylamino) – 5 – phenyl – 2H – 1,4 – benzodiazepin – 2 – on,

3 – (RS) – (3,5 – Dichlor – benzoylamino)1,3 – dihydro – 5 – phenyl – 2H – 1,4 – benzodiazepin – 2 – on,

1 – 3 – Dihydro – 3 – (RS) – (p – hydroxybenzoylamino) – 5 – phenyl – 2H – 1,4 – benzodiazepin – 2 – on,

3 – (RS) – (4' – Cyanobenzoylamino)1,3 – dihydro – 5 – phenyl – 2H – 1,4 – benzodiazepin – 2 – on,

3(S) – ( – ) – 3 – (2 – Chlor – benzoylamino) – 1,3 – dihydro – 1 – methyl – 5 – phenyl – 2H – 1,4 – benzodiazepin – 2 – on,

3(R) – ( + ) – 3 – (2 – Chlor – benzoylamino) – 1,3 – dihydro – 1 – methyl – 5 – phenyl – 2H – 1,4 – benzodiazepin – 2 – on,

1,3 – Dihydro – 3(RS) – (p – dimethylaminobenzoylamino) – 5 – (2 – fluor – phenyl) – 2H – 1,4 – benzodiazepin – 2 – on,

1,3 – Dihydro – 3(RS) – (3,4 – dimethoxybenzoylamino) – 5 – (2 – fluor – phenyl) – 2H – 1,4 – benzodiazepin – 2 – on,

3(S) – ( + ) – 3 – (3 – Brom – benzoylamino) – 1,3 – dihydro – 5 – (2 – fluor – phenyl) – 1 – methyl – 2H – 1,4 – benzodiazepin – 2 – on,

1,3 – Dihydro – 5 – phenyl – 3(RS) – (3 – trifluor – methylthiobenzoylamino) – 2H – 1,4 – benzodiazepin – 2 – on,

3(S) – ( + ) – 3 – (4 – Brom – benzoylamino) – 1,3 – dihydro – 5 – (2 – fluor – phenyl) – 1 – methyl – 2H – 1,4 – benzodiazepin – 2 – on,

3(S) – ( + ) – 3 – (4 – t – Butylbenzoylamino) – 1,3 – dihydro – 5 – (2 – fluor – phenyl) – 1 – methyl – 2H – 1,4 – benzodiazepin – 2 – on,

1,3 – Dihydro – 5 – (2 – fluor – phenyl) – 3(RS) – (pyrrol – 2 – carbonylamino) – 2H – 1,4 – benzodiazepin – 2 – on,

3(S) – ( + ) – 1,3 – Dihydro – 5 – (2 – fluor – phenyl) – 3 – (4 – iodbenzoylamino) – 1 – methyl – 2H – 1,4 – benzodiazepin – 2 – on,

1,3 – Dihydro – 3(RS) – (2 – naphthoylamino) – 5 – phenyl – 2H – 1,4 – benzodiazepin – 2 – on,

3(S) – ( – ) – 3 – (2 – Brom – benzoylamino) – 1,3 – dihydro – 5 – (2 – fluorphenyl) – 1 – methyl – 2H – 1,4 – benzodiazepin – 2 – on,

3(S) – ( + ) – 3 – (4 – Cyanobenzoylamino) – 1,3 – dihydro – 5 – (2 – fluor – phenyl) – 1 – methyl – 2H – 1,4 – benzodiazepin – 2 – on,

1,3 – Dihydro – 5 – phenyl – 3(RS) – (4 – n – propylbenzoylamino) – 2H – 1,4 – benzodiazepin – 2 – on,

1,3 – Dihydro – 5 – phenyl – 3(RS) – (4 – phenylbenzoylamino) – 2H – 1,4 – benzodiazepin – 2 – on,

1,3 – Dihydro – 3(RS) – (4 – n – pentylbenzoylamino) – 5 – phenyl – 2H – 1,4 – benzodiazepin – 2 – on,

1,3 – Dihydro – 3(RS) – (1 – naphthoylamino) – 5 – phenyl – 2H – 1,4 – benzodiazepin – 2 – on,

3(S) – ( + ) – 1,3 – Dihydro – 5 – (2 – fluor – phenyl) – 3 – (3 – iodbenzoylamino) – 1 – methyl – 2H – 1,4 – benzodiozepin – 2 – on,

3(R) – ( – ) – 1,3 – Dihydro – 5 – (2 – fluor – phenyl) – 3 – (3 – iodbenzoylamino) – 1 – methyl – 2H – 1,4 – benzodiazepin – 2 – on,
3(R) – ( + ) – 1,3 – Dihydro – 5 – (2 – fluor – phenyl) – 3 – (2 – iodbenzoylamino) – 1 – methyl – 2H – 1,4 – benzodiazepin – 2 – on,
3(S) – ( – ) – 1,3 – Dihydro – 5 – (2 – fluor – phenyl) – 3 – (2 – iodbenzoylamino) – 1 – methyl – 2H – 1,4 – benzodiazepin – 2 – on,
3(R) – ( + ) – 3 – (2 – Brom – benzoylamino) – 1,3 – dihydro – 5 – (2 – fluor – phenyl) – 1 – methyl – 2H – 1,4 – benzodiazepin – 2 – on,
3(R) – ( + ) – 3 – (2 – Chlor – benzoylamino) – 1,3 – dihydro – 5 – (2 – fluor – phenyl) – 1 – methyl – 2H – 1,4 – benzodiazepin – 2 – on,
1,3 – Dihydro – 5 – (2 – fluor – phenyl) – 3(RS) – phenylcarbonylamino – 2H – 1,4 – benzodiazepin – 2 – on,
1,3 – Dihydro – 5 – (2 – fluor – phenyl) – 3(RS) – (2 – chlor – phenyl)carbonylamino – 2H – 1,4 – benzodiazepin – 2 – on,
1,3 – Dihydro – 5 – (2 – fluor – phenyl) – 3(RS) – benzyloxycarbonylamino – 2H – 1,4 – benzodiazepin – 2 – on,
1,3 – Dihydro – 5 – (2 – fluor – phenyl) – 3 – (RS) – benzyloxycarbonylamino – 2H – 1,4 – benzodiazepin – 2 – thion,
1 – (4 – Chlor – phenyl)carbonyl – 1,3 – dihydro – 5 – (2 – fluor – phenyl) – 3(RS) – (4 – chlor – phenyl) – carbonylamino – 2H – 1,4 – benzodiazepin – 2 – on,
1 – (4 – Chlor – phenyl)carbonyl – 1,3 – dihydro – 5 – (2 – fluor – phenyl) – 3(RS) – (4 – chlor – phenyl) – carbonyloxy – 2H – 1,4 – benzodiazepin – 2 – on,
1,3 – Dihydro – 5 – (2 – fluor – phenyl) – 3(RS) – (4 – chlor – phenyl)oxy – 2H – 1,4 – benzodiazepin – 2 – on,
1,3 – Dihydro – 5 – (2 – fluor – phenyl) – 3 – (RS) – (4 – chlor – phenyl)carbonylamino – 2H – 1,4 – benzodiazepin – 2 – thion,
1,3 – Dihydro – 5 – (2 – fluor – phenyl) – 3 – (RS) – (2 – indol)carbonylamino – 2H – 1,4 – benzodiazepin – 2 – thion,
1,3 – Dihydro – 3(RS) – (4 – chlor – phenyl)aminocarbonylamino – 2H – 1,4 – benzodiazepin – 2 – on,
ist.

**6.** Verwendung nach Anspruch 1, dadurch gekennzeichnet, daß die Verbindung der Formel I
(S) – ( – ) – 1,3 – Dihydro – 3 – (4 – brom – benzoylamino) – 1 – methyl – 5 – phenyl – 2H – 1,4 – benzodiazepin – 2 – on
3(RS) – (2 – Indol – carbonylamino) – 1,3 – dihydro – 5 – phenyl – 2H – 1,4 – benzodiazepin – 2 – on,
1,3 – Dihydro – 3(RS) – (2 – indol – carbonylamino) – 1 – methyl – 5 – phenyl – 2H – 1,4 – benzodiazepin – 2 – on,
1,3 – Dihydro – 1 – methyl – 3(RS) – [2 – (1 – methylindol)carbonylamino] – 5 – phenyl – 2H – 1,4 – benzodiazepin – 2 – on,
1,3 – Dihydro – 1 – methyl – 3(RS) – (4 – chlor – phenylcarbonyl)amino – 5 – (2 – fluor – phenyl) – 2H – 1,4 – benzodiazepin – 2 – on,
1,3 – Dihydro – 5 – (2 – fluor – phenyl) – 3(RS) – (2 – indol – carbonylamino) – 1 – methyl – 2H – 1,4 – benzodiazepin – 2 – on,
1,3 – Dihydro – 5 – (2 – fluor – phenyl) – 1 – methyl – 3(RS) – [2' – (1 – methylindol )carbonylamino] – 2H – 1,4 – benzodiazepin – 2 – on,
3(R) – ( – ) – 1,3 – Dihydro – 3 – (4 – chlor – benzoylamino) – 5 – (2 – fluor – phenyl) – 1 – methyl – 2H – 1,4 – benzodiazepin – 2 – on,
3(S) – ( + ) – 1,3 – Dihydro – 3 – (4 – chlor benzoylamino) – 5 – (2 – fluor – phenyl) – 1 – methyl – 2H – 1,4 – benzodiazepin – 2 – on,
3(R) – ( + ) – 1,3 – Dihydro – 3 – (4 – brom – benzoylamino) – 1 – methyl – 5 – phenyl – 2H – 1,4 – benzodiazepin – 2 – on,
3(R) – ( + ) – 1,3 – Dihydro – 3 – (2 – indol – carbonylamino) – 1 – methyl – 5 – phenyl – 2H – 1,4 – benzodiazepin – 2 – on,
1,3 – Dihydro – 5 – (2 – fluor – phenyl) – 3 – (RS) – (2 – indol – carbonylamino) – 2H – 1,4 – benzodiazepin – 2 – on,
1,3 – Dihydro – 3 – (RS) – (2 – indol – carbonyloxy) – 5 – phenyl – 2H – 1,4 – benzodiazepin – 2 – on,
1,3 – Dihydro – 3 – (RS) – (4 – chlor – phenylcarbonyl)amino – 5 – (2 – fluor – phenyl) – 2H – 1,4 – benzodiazepin – 2 – on,

1 – Carboxymethyl – 1,3 – dihydro – 5 – (2 – fluor – phenyl) – 3(RS) – (2 – indol – carbonylamino) – 2H – 1,4 – benzodiazepin – 2 – on,

1,3 – Dihydro – 3 – (RS) – (5 – fluor – indol – 2 – carbonylamino) – 5 – (2 – fluor – phenyl) – 2H – 1,4 – benzodiazepin – 2 – on,

3 – (RS) – Cinnamoylamino – 1,3 – dihydro – 5 – (2 – fluor – phenyl) – 2H – 1,4 – benzodiazepin – 2 – on,

1,3 – Dihydro – 5 – (2 – fluor – phenyl) – 3 – (RS) – (2 – benzofurancarbonylamino) – 2H – 1,4 – benzodiazepin – 2 – on,

1,3 – Dihydro – 1 – methyl – 3 – (RS) – (4 – chlor – phenylcarbonyl)amino – 5 – phenyl – 2H – 1,4 – benzodiazepin – 2 – on,

1 – Carboxymethyl – 1,3 – dihydro – 3 – (RS) – (4 – chlor – phenylcarbonyl)amino – 5 – (2 – fluor – phenyl) – 2H – 1,4 – benzodiazepin – 2 – on,

1,3 – Dihydro – 5 – (2 – fluor – phenyl) – 3 – (RS) – (p – trifluormethylbenzoylamino) – 2H – 1,4 – benzodiazepin – 2 – on,

3(S) – ( + ) – 3 – (3 – Brom – benzoylamino) – 1,3 – dihydro – 5 – (2 – fluor – phenyl) – 1 – methyl – 2H – 1,4 – benzodiazepin – 2 – on,

3(S) – ( + ) – 3 – (4 – Brom – benzoylamino) – 1,3 – dihydro – 5 – (2 – fluor – phenyl) – 1 – methyl – 2H – 1,4 – benzodiazepin – 2 – on,

3(S) – ( + ) – 1,3 – Dihydro – 5 – (2 – fluor – phenyl) – 3 – (4 – iodbenzoylamino) – 1 – methyl – 2H – 1,4 – benzodiazepin – 2 – on,

1,3 – Dihydro – 3(RS) – (2 – naphthoylamino) – 5 – phenyl – 2H – 1,4 – benzodiazepin – 2 – on,

3(S) – ( + ) – 1,3 – Dihydro – 5 – (2 – fluor – phenyl) – 3 – (3 – iodbenzoylamino) – 1 – methyl – 2H – 1,4 – benzodiazepin – 2 – on,

3(R) – ( – ) – 1,3 – Dihydro – 5 – (2 – fluor – phenyl) – 3 – (3 – iodbenzoylamino) – 1 – methyl – 2H – 1,4 – benzodiazepin – 2 – on,

3(R) – ( – ) – 1,3 – Dihydro – 5 – (2 – fluor – phenyl) – 3 – (2 – indolcarbonylamino) – 1 – methyl – 2H – 1,4 – benzodiazepin – 2 – on. ist.

**7.** Verwendung nach Anspruch 1, dadurch gekennzeichnet, daß die Verbindung der Formel I

1 – Carboxymethyl – 1,3 – dihydro – 3(RS) – (2 – indol – carbonylamino) – 5 – phenyl – 2H – 1,4 – benzodiazepin – 2 – on,

(S) – ( – ) – 1,3 – Dihydro – 3 – (2 – indol – carbonylamino) – 1 – methyl – 5 – phenyl – 2H – 1,4 – benzodiazepin – 2 – on,

(S) – ( + ) – 1,3 – Dihydro – 5 – (2 – fluor – phenyl) – 3 – (2 – indol – carbonylamino) – 1 – methyl – 2H – 1,4 – benzodiazepin – 2 – on,

(S) – ( + ) – 1,3 – Dihydro – 3 – (4 – chlor – benzoylamino) – 5 – (2 – fluor – phenyl) – 1 – methyl – 2H – 1,4 – benzodiazepin – 2 – on ist

**8.** Benzodiazepin – Derivat, entsprechend der folgenden Formel Ib

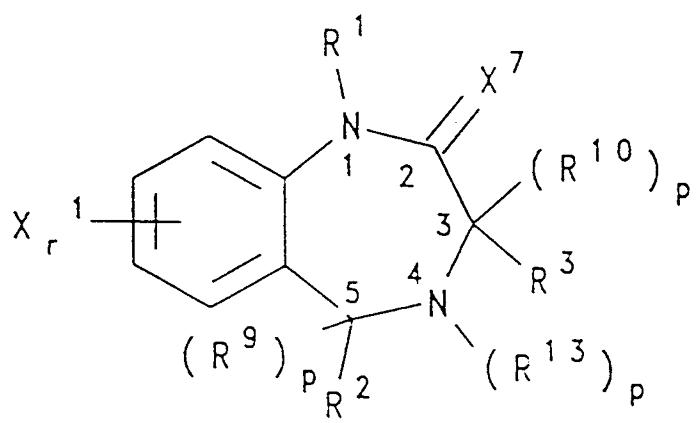

I b

wobei

$X^{7'}$ S, HH oder $NR^{15}$ ist und

die restlichen Substituenten und Symbole die gleichen sind wie bei den Verbindungen der Formel I in

Anspruch 1 definiert, mit der Maßgabe, daß $X^{7'}$ nur dann $NR^{15}$ sein kann, wenn $R^1$ nicht H ist, oder Salze oder quaternäre Ammoniumsalze davon.

**9.** Benzodiazepin – Derivat, entsprechend der folgenden Formel Ic

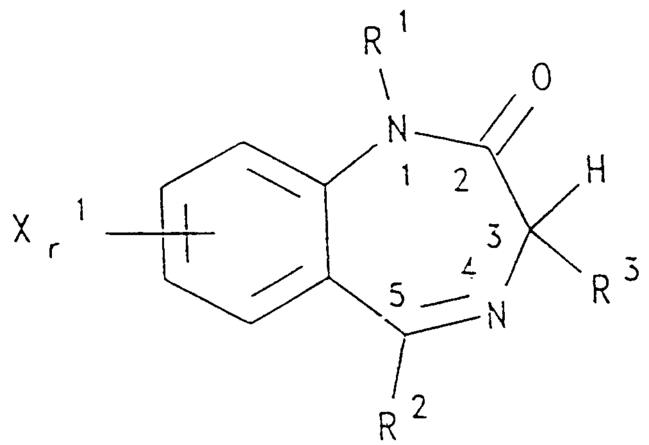

I c

oder Salze oder quaternäre Ammoniumsalze der Verbindungen der Formel Ic, wobei bei den Verbin – dungen der Formel Ic

$R^1$ Alkyl mit 1 bis 5 Kohlenstoffatomen oder Wasserstoff ist,

$R^2$ unsubstituiertes Phenyl, substituiertes Phenyl mit 1 oder 2 Substituenten, ausgewählt aus Halogen, Alkyl mit 1 bis 4 Kohlenstoffatomen, Alkoxy mit 1 bis 4 Kohlenstoffatomen, Carboxy – substituiertes Alkyl mit 1 bis 4 Kohlenstoffatomen in der Alkylgruppe, Carboxyl, Nitro, Trifluormethyl und Hydroxy ist,

$R^3$ eine Gruppe ist der Formeln

$$-(CH_2)_nNH\overset{\overset{O}{\parallel}}{C}(CH_2)_qR^7 \quad \text{oder} \quad -(CH_2)_nNH\overset{\overset{O}{\parallel}}{C}NH(CH_2)_nR^7$$

wobei

$R^7$ unsubstituiertes Phenyl oder substituiertes Phenyl mit 1 oder 2 Substituenten, ausgewählt aus Halogen, Nitro, Hydroxy, Alkyl mit 1 bis 4 Kohlenstoffatomen, Alkoxy mit 1 bis 4 Kohlen – stoffatomen, Alkylthio mit 1 bis 4 Kohlenstoffatomen, Trifluormethyl oder Gruppen der Formeln CN, – C≡CH, $SCF_3$,

$$O\overset{\overset{O}{\parallel}}{C}CH_3,$$

$OCHF_2$, SPh, oder – $NR^4R^5$ ist, wobei $R^4$ und $R^5$ unabhängig voneinander Wasserstoff oder Alkyl mit 1 bis 4 Kohlenstoffatomen sind,

oder der Rest

$R^7$ eine Gruppe ist der Formel

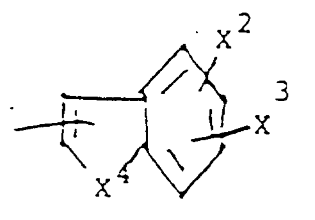

,

wobei
$X^2$ und $X^3$ unabhängig voneinander Wasserstoff, Hydroxy, Nitro, Halogen, Alkylthio mit 1 bis 4 Kohlenstoffatomen, Alkyl mit 1 bis 4 Kohlenstoffatomen oder Alkoxy mit 1 bis 4 Kohlenstoffatomen sind,
$X^4$ NH ist,
q 0 bis 4, vorzugsweise 0, ist und
n 0 bis 4, vorzugsweise 0, ist.

**10.** Benzodiazepin-Derivat nach Anspruch 9, wobei
$R^1$ Methyl ist,
$R^2$ unsubstituiertes Phenyl ist,
$R^3$ wie in Anspruch 9 definiert ist, wobei
q und n 0 sind, und
$R^7$ wie in Anspruch 9 definiert ist, wobei in der Indolyl-Gruppe $X^2$ und $X^3$ jedoch Wasserstoff sind.

**11.** Verbindung 3(S)-(-)-1,3-Dihydro-3-(2-indolcarbonylamino)-1-methyl-5-phenyl-2H-1,4-benzodiazepin-2-on.

**Patentansprüche für folgenden Vertragsstaat : AT**

**1.** Verwendung von Verbindungen der Formel I

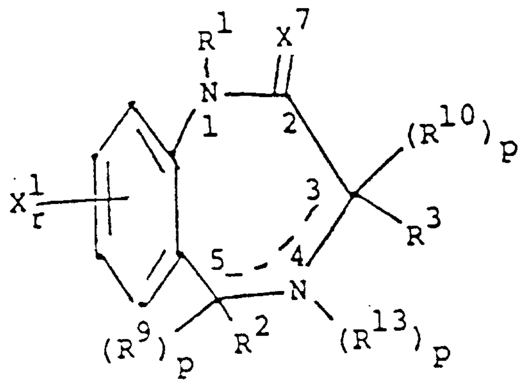

I

oder eines pharmazeutisch verträglichen Salzes oder eines pharmazeutisch verträglichen quaternären Ammoniumsalzes der Verbindung der Formel I,
wobei bei den Verbindungen der Formel I

$R^1$ Wasserstoff, Alkyl mit 1 bis 5 Kohlenstoffatomen, Alkenyl mit bis zu 5 Kohlenstoffatomen, Alkinyl mit bis zu 5 Kohlenstoffatomen, oder eine Gruppe der Formeln $-(CH_2)_mCOOR^6$, $-(CH_2)_n-C_3-C_5$-Cycloalkyl, $-(CH_2)_mNR^4R^5$, $-(CH_2)_mCONR^4R^5$, $-(CH_2)_mCN$ oder $-(CH_2)_nCX^{10}_3$ ist,
wobei $R^4$ und $R^5$ unabhängig voneinander Wasserstoff, Alkyl mit 1 bis 4 Kohlenstoffatomen oder Cycloalkyl mit 3 bis 5 Kohlenstoffatomen ist,
$R^6$ Wasserstoff, Alkyl mit 1 bis 4 Kohlenstoffatomen, Cycloalkyl mit 3 bis 5 Kohlenstoffatomen, unsubstituiertes Phenyl, substituiertes Phenyl, unsubstituiertes Phenylalkyl oder substituiertes Phenyl-alkyl ist, wobei bei den Gruppen die Substituenten des Phenylrestes 1 oder 2 Substituenten sind, ausgewählt aus Halogen, Alkyl mit 1 bis 4 Kohlenstoffatomen, Alkoxy mit 1 bis 4 Kohlenstoffatomen, Nitro oder Trifluormethyl sind und wobei die Alkylgruppe der entsprechenden substituierten oder unsubstituierten Phenyl-alkyl-Gruppe 1 bis 4 Kohlenstoffatome enthält,
$X^{10}$ Fluor, Chlor oder Brom ist,
m 1 bis 4 ist und
n 0 bis 4 ist;

$R^2$ Wasserstoff, Alkyl mit 1 bis 4 Kohlenstoffatomen, unsubstituiertes Phenyl, substituiertes Phenyl mit 1 oder 2 Substituenten, ausgewählt aus Halogen, Alkyl mit 1 bis 4

Kohlenstoffatomen, Alkoxy mit 1 bis 4 Kohlenstoffatomen, Alkylthio mit 1 bis 4 Kohlenstoffatomen, Carboxy-substituiertes Alkyl mit 1 bis 4 Kohlenstoffatomen in der Alkylgruppe, Carboxyl, Nitro, Trifluormethyl und Hydroxy, ist,
oder der Rest $R^2$ eine Gruppe ist der folgenden Formeln

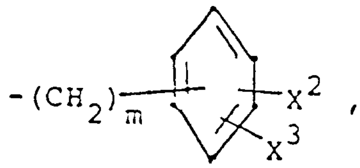

$-(CH_2)_mSCH_3$, $-(CH_2)_mSOCH_3$, $-(CH_2)_mSO_2CH_3$, oder $-(CH_2)_mCOOR^6$;
wobei
$R^6$ und m wie im Zusammenhang mit dem Rest $R^1$ definiert sind,
$X^2$ und $X^3$ unabhängig voneinander Wasserstoff, Hydroxy, Nitro, Halogen, Alkyl mit 1 bis 4 Kohlenstoffatomen, Alkylthio mit 1 bis 4 Kohlenstoffatomen oder Alkoxy mit 1 bis 4 Kohlenstoffatomen ist,

$R^3$ eine Gruppe ist der Formeln
$-(CH_2)_nR^7$,

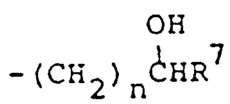 , 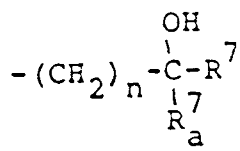

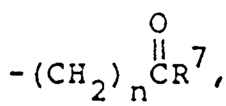

$-(CH_2)_nNR^{18}(CH_2)_qR^7$,

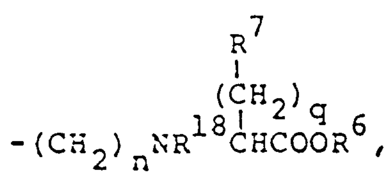

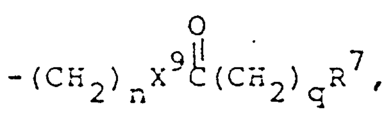

$-NH(CH_2)_{2-3}NHR^7$, $-NH(CH_2)_{2-3}NHCOR^7$,

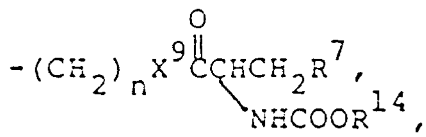

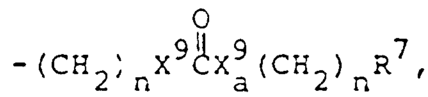 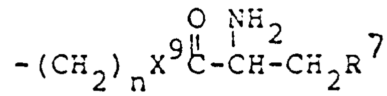

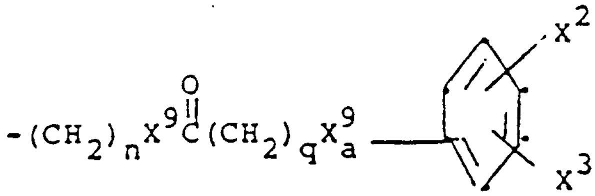

oder $-(CH_2)_nNR^{18}SO_2(CH_2)_qR^7$,
wobei
$R^7$ und $R^7{}_a$ unabhängig voneinander unsubstituiertes Phenyl oder substituiertes Phenyl mit 1 oder 2 Substituenten, ausgewählt aus Halogen, Nitro, Hydroxy, Alkyl mit 1 bis 4 Kohlenstoffatomen, oder Gruppen der Formeln $CF_3$, CN, $SCF_3$, C≡CH, $CH_2SCF_3$, $OCOCH_3$, $OCHF_2$, SH; SPh, $PO_3H$ oder $-NR^4R^5$, Alkoxy mit 1 bis 4 Kohlenstoffatomen oder Alkylthio mit 1 bis 4 Kohlenstoffatomen bedeuten, oder die Reste
$R^7$ und $R^7{}_a$ α-Naphthyl oder β-Naphthyl oder eine Gruppe der folgenden Formeln bedeuten

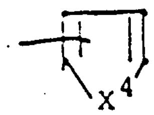, 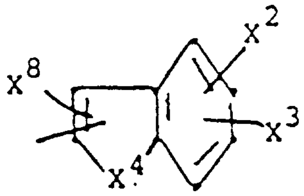, 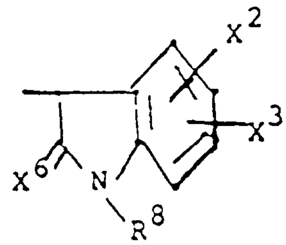

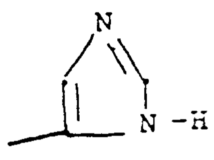, 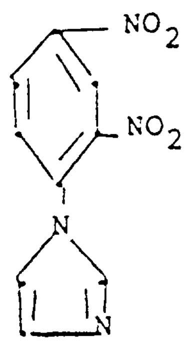,

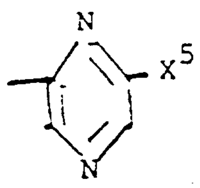, 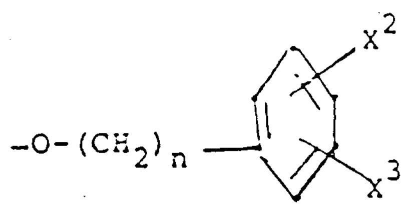,

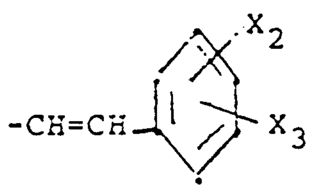, 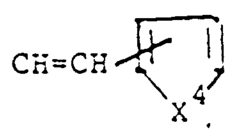

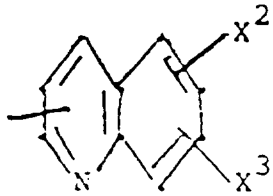, 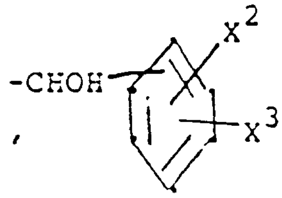,

oder

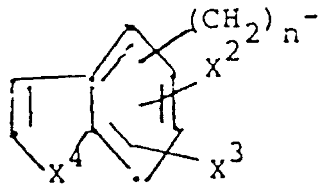

wobei
$R^8$ Wasserstoff, Alkyl mit 1 bis 4 Kohlenstoffatomen, Cycloalkyl mit 3 bis 5 Kohlenstoffatomen bedeutet, das entweder direkt oder über eine Alkylenkette, umfassend 1 bis 4 $-CH_2-$Gruppen, gebunden ist, oder $R^8$ eine Gruppe ist der Formeln $-(CH_2)_mCONH_2$, $-(CH_2)_mCOOR^6$, $-(CH_2)_mNR^4R^5$,

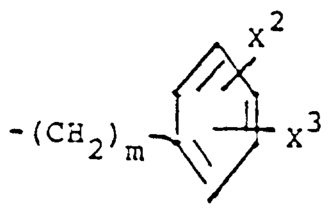
,
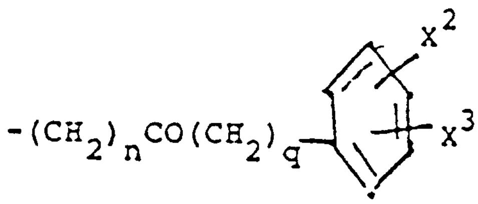

oder

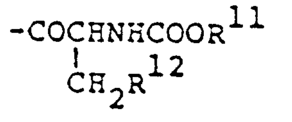
;

wobei
$X^2$ und $X^3$ wie im Zusammenhang mit dem Rest $R^2$ definiert sind,
$X^4$ S, O, $CH_2$ oder $NR^8$ ist, wobei $R^8$ wie oben definiert ist,
$X^5$ Wasserstoff, Halogen, Nitro oder eine Gruppe der Formeln $CF_3$, CN, $-COOR^6$ ist, wobei $R^6$ wie oben definiert ist,
$X^5$ O oder HH ist,
$X^5$ Wasserstoff oder Alkyl mit 1 bis 4 Kohlenstoffatomen ist,
$X^5$ und $X^5{}_a$ unabhängig voneinander Gruppen sind der Formeln $-O-$ oder $-NR^{18}-$
wobei
$R^{18}$ Wasserstoff, Alkyl mit 1 bis 4 Kohlenstoffatomen, Formyl, Acety, Propionyl oder Butyryl ist
$R^{11}$ und $R^{12}$ unabhängig voneinander Alkyl mit 1 bis 4 Kohlenstoffatomen oder Cycloalkyl mit 3 bis 5 Kohlenstoffatomen sind,
$R^{14}$ Alkyl mit 1 bis 4 Kohlenstoffatomen oder Phenylalkyl ist, wobei die Alkylgruppe 1 bis 4 Kohlenstoffatomen enthält,

$R^9$ und $R^{10}$ unabhängig voneinander Wasserstoff, Hydroxy oder Methyl bedeuten,

$R^{13}$ Wasserstoff, Alkyl mit 1 bis 4 Kohlenstoffatomen, Cycloalkyl mit 3 bis 5 Kohlenstoffatomen, $-O$, Formyl, Acetyl, Propionyl oder Butyryl bedeutet,

$X^1$ Wasserstoff, Halogen, Alkyl mit 1 bis 4 Kohlenstoffatomen, Alkylthio mit 1 bis 4 Kohlenstoffatomen, Alkoxy mit 1 bis 4 Kohlenstoffatomen oder eine Gruppe bedeutet der Formeln $-NO_2$, $CF_3$, CN, OH, $-(CH_2)_nCOCR^6$ oder $-NR^4R^5$, wobei
$R^4$, $R^5$, $R^6$ und n wie im Zusammenhang mit $R^1$ definiert sind,

r 1 oder 2 ist,

$X^7$ O, S, HH oder $NR^{15}$ ist, wobei
$R^{15}$ Wasserstoff, Alkyl mit 1 bis 4 Kohlenstoffatomen oder eine Gruppe ist der

Formeln

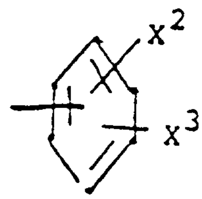

oder $-NR^{16}R^{17}$
wobei $R^{16}$ und $R^{17}$ Wasserstoff oder eine Gruppe bedeuten der Formel

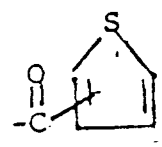

jedoch mit der Maßgabe, daß
$X^7$ nur dann $NR^{15}$ sein kann, wenn $R^1$ nicht H ist;
q 0 bis 4 ist,
– – – eine Einfach – oder Doppelbindung angibt,
p 0 ist, wenn die angrenzende – – – Bindung eine Doppelbindung ist, und 1, wenn die angrenzende – – – Bindung eine Einfachbindung ist, außer wenn $R^{13}$ Sauerstoff ist und p 1 ist und – – – eine Doppelbindung ist, und mit der Maßgabe, daß, wenn $R^3$ eine Gruppe ist der Formel $-(CH_2)_nNH(CH_2)_qR^7$, in der q 0 oder 1 ist, oder eine Gruppe der Formel

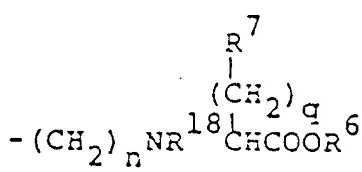

in der q 0 ist, dann $R^7$ eine andere Bedeutung haben muß als eine Gruppe der Formel

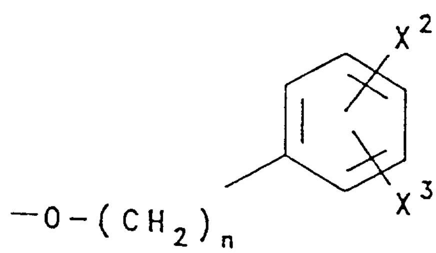

zur Herstellung eines Arzneimittels zur Verwendung als Antagonist gegenüber Cholecystokinin in gastrointestinalem (Magen – Darm – ) Gewebe und als Antagonist gegenüber Gastrin durch Bindung an den entsprechenden Cholecystokinin – Rezeptor oder Gastrin – Rezeptor.

**2.** Verwendung nach Anspruch 1, dadurch gekennzeichnet, daß zur Herstellung eines Arzneimittels als pharmazeutisch wirksamer Bestandteil eine Verbindung der Formel I oder ein pharmazeutisch an – nehmbares Salz oder ein pharmazeutisch annehmbares quaternäres Ammoniumsalz der Verbindung der Formel I verwendet wird, wobei bei der Verbindung der Formel I

$R^1$ Wasserstoff, Alkyl mit 1 bis 5 Kohlenstoffatomen oder eine Gruppe der Formel

$-(CH_2)_mCOOR^6$ ist, wobei
$R^6$ und m wie in Anspruch 1 definiert sind,

$R^2$ eine Gruppe der Formel $-(CH_2)_mCOOR^6$ ist, wobei $R^6$ und m wie oben definiert sind, oder $R^2$ unsubstituiertes Phenyl, substituiertes Phenyl mit 1 oder 2 Substituenten, ausgewählt aus Halogen, Alkyl mit 1 bis 4 Kohlenstoffatomen, Alkoxy mit 1 bis 4 Kohlenstoffatomen, Carboxy-substituiertes Alkyl mit 1 bis 4 Kohlenstoffatomen in der Alkylgruppe, Carboxyl, Nitro, Trifluormethyl und Hydroxy ist,

$R^3$ eine Gruppe ist der Formeln
$-(CH_2)_nR^7$,

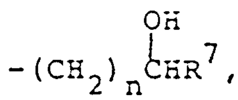

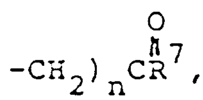

$-(CH_2)_nNH(CH_2)_qR^7$,

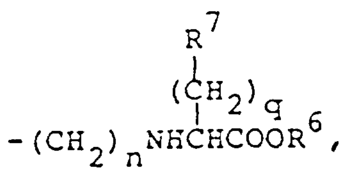 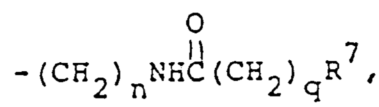

$-NH(CH_2)_{2-3}NHR^7$,

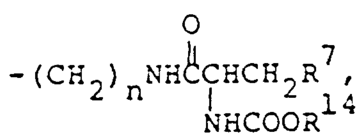

$-NH(CH_2)_{2-3}NHCOR^7$ oder

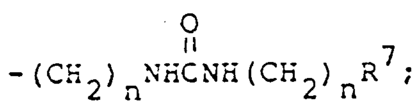

wobei

$R^7$ unsubstituiertes Phenyl oder substituiertes Phenyl mit 1 oder 2 Substituenten, ausgewählt aus Halogen, Nitro, Hydroxy, Alkyl mit 1 bis 4 Kohlenstoffatomen, Alkoxy mit 1 bis 4 Kohlenstoffatomen, Alkylthio mit 1 bis 4 Kohlenstoffatomen, Trifluormethyl oder Gruppen der Formeln CN, $-C{\equiv}CH$, $SCF_3$, $OCCH_3$, $OCHF_2$, SPh, oder $-NR^4R^5$ ist,
wobei $R^4$ und $R^5$ unabhängig voneinander Wasserstoff oder Alkyl mit 1 bis 4 Kohlenstoffatomen sind, oder der Rest

$R^7$ $\alpha$-Naphthyl oder $\beta$-Naphthyl ist oder eine Gruppe ist der Formeln

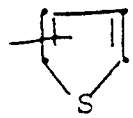 ,

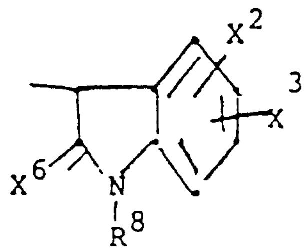 ,

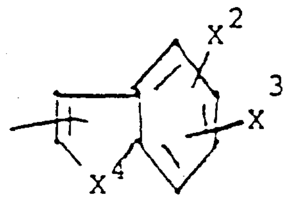 ;

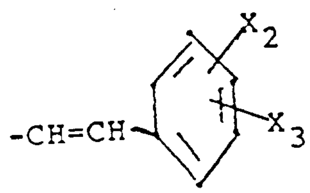 oder 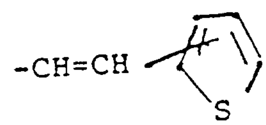

wobei $R^8$ Wasserstoff, Alkyl mit 1 bis 4 Kohlenstoffatomen ist oder eine Gruppe der Formel

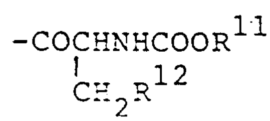

wobei
$R^{11}$ und $R^{12}$ unabhängig voneinander Alkyl mit 1 bis 4 Kohlenstoffatomen sind,
$X^2$ und $X^3$ unabhängig voneinander Wasserstoff, Hydroxy, Nitro, Halogen, Alkylthio mit 1 bis 4 Kohlenstoffatomen, Alkyl mit 1 bis 4 Kohlenstoffatomen oder Alkoxy mit 1 bis 4 Kohlenstoffatomen sind,
$X^4$ S, O, oder $NR^8$ ist, wobei $R^8$ wie oben definiert ist,
$X^6$ O oder HH ist,
$R^{14}$ Alkyl mit 1 bis 4 Kohlenstoffatomen ist und
q 0 bis 4 ist,

| | |
|---|---|
| $R^9$ und $R^{10}$ | unabhängig voneinander Wasserstoff, Hydroxy oder Methyl sind, |
| $R^{13}$ | Wasserstoff, Alkyl mit 1 bis 4 Kohlenstoffatomen −O, Formyl, Acetyl, Propionyl oder Butyryl ist, |
| $X^1$ | Wasserstoff, Halogen, Alkyl mit 1 bis 4 Kohlenstoffatomen, Alkylthio mit 1 bis 4 Kohlenstoffatomen, Alkoxy mit 1 bi 4 Kohlenstoffatomen oder eine Gruppe ist der Formeln $-NO_2$, $CF_3$, CN, OH, $-(CH_2)_nCOOR^6$ oder $-NR^4R^5$, wobei |
| $R^6$ | wie im Zusammenhang mit dem Rest $R^1$ definiert ist und $R^4$ und $R^5$ wie im Zusammenhang mit dem Rest $R^3$ definiert sind, |
| $X^7$ | O ist, |
| m | 1 bis 4 ist, |
| n | 0 bis 4 ist, |
| - - - | eine Einfachbindung oder eine Doppelbindung angibt, |
| p | 0 ist, wenn die angrenzende - - - Bindung eine Doppelbindung ist, und 1, wenn die angrenzende - - - Bindung eine Einfachbindung ist, außer wenn $R^{13}$ Sauerstoff ist und p 1 ist und - - - eine Doppelbindung ist und |
| r | 1 oder 2 ist. |

3. Verwendung nach Anspruch 2, dadurch gekennzeichnet, daß zur Herstellung eines Arzneimittels als pharmazeutisch wirksamer Bestandteil eine Verbindung der Formel I oder ein pharmazeutisch annehmbares Salz oder ein pharmazeutisch annehmbares quaternäres Ammoniumsalz der Verbindung der Formel I verwendet wird, wobei

$R^1$ Wasserstoff, Methyl, Ethyl, Carboxymethyl oder Ethylcarboxymethyl ist,

$R^2$ eine Gruppe ist der Formel $-(CH_2)_{1-2}COOR^6$, wobei $R^6$ Wasserstoff oder Alkyl mit 1 bis 4 Kohlenstoffatomen ist, oder

$R^2$ unsubstituiertes Phenyl, substituiertes Phenyl mit 1 oder 2 Substituenten, die Halogen oder Carboxyl sind, bedeutet,

$R^3$ eine Gruppe ist der Formeln

$-(CH_2)_nR^7$,

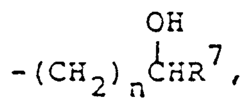

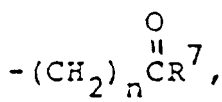

$-(CH_2)_nNH(CH_2)_qR^7$,

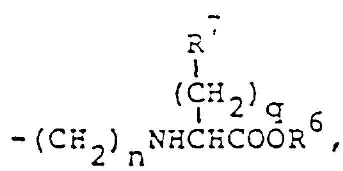

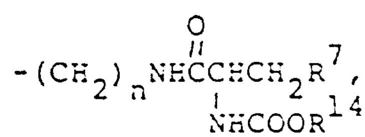

oder

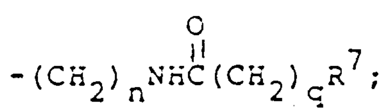

wobei

$R^7$ $\alpha$-Naphthyl, $\beta$-Naphthyl oder substituiertes Phenyl mit 1 oder 2 Substituenten ist, ausgewählt aus Halogen, Trifluormethyl und Alkyl mit 1 bi 4 Kohlenstoffatomen ist oder

$R^7$ eine Gruppe ist der Formeln

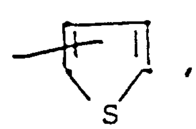

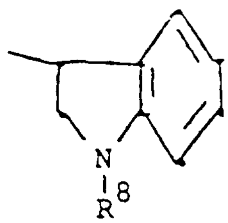,

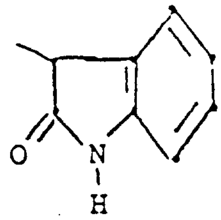,

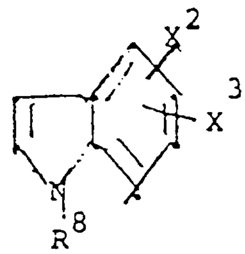,

oder

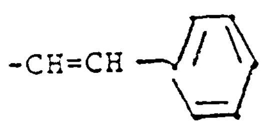

wobei
$R^8$ Wasserstoff, Methyl oder Ethyl ist
$X^2$ und $X^3$ unabhängig voneinander Wasserstoff, Hydroxy, Nitro oder Halogen ist,
$R^{14}$ tert. – Butyl ist,
n 0 bis 4 ist,
q 0 bis 4 ist, und
$R^6$ wie im Zusammenhang mit dem Rest $R^2$ definiert ist,

| | |
|---|---|
| $R^9$ und $R^{10}$ | unabhängig voneinander Wasserstoff, Hydroxy oder Methyl sind, |
| $R^{13}$ | Wasserstoff, Methyl oder Formyl ist, |
| $X^1$ | Wasserstoff, Halogen oder eine Gruppe ist der Formeln $-NO_2$, $CF_3$, CN oder OH ist, |
| $X^7$ | O ist, |
| - - - | eine Einfach – oder Doppelbindung angibt, |
| p | 0 ist, wenn die angrenzende - - - Bindung eine Doppelbindung ist und 1, wenn die angrenzende - - - Bindung eine Einfachbindung ist, und |
| r | 1 oder 2 ist. |

**4.** Verwendung nach Anspruch 3, dadurch gekennzeichnet, daß zur Herstellung eines Arzneimittels als pharmazeutisch wirksamer Bestandteil eine Verbindung der Formel I oder ein pharmazeutisch annehmbares Salz oder ein pharmazeutisch annehmbares quaternäres Ammoniumsalz der Verbindung der Formel I verwendet wird, wobei bei der Formel I

| | |
|---|---|
| $R^1$ | Wasserstoff, Methyl, oder Carboxmethyl ist |
| $R^2$ | Phenyl, o – Fluorphenyl, p – Fluorphenyl, o – Chlorphenyl, p – Chlorphenyl, o – Carboxyphenyl, 2,4 – Dichlorphenyl, 2,6 – Difluorphenyl, $-CH_2COOEt$, $-CH_2COO$ – t – Bu, $-CH_2CH_2COOEt$ oder $CH_2CH_2COO$ – t – Bu ist |
| $R^3$ | $-(CH_2)_{1-2}R^7$, |

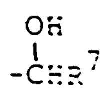,

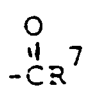,

$-(CH_2)_{0-1}NH(CH_2)_{1-2}R^7$,

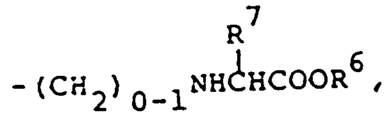, 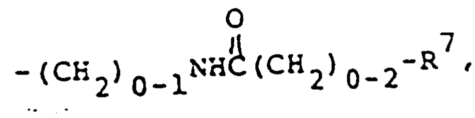

oder

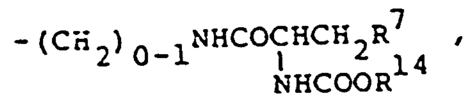

ist,
und die Stöchiometrie sich auf D – Tryptophan bezieht,

R⁷ — α – Naphthyl, β – Naphthyl, Monohalogen – phenyl oder Dihalogen – phenyl oder eine Gruppe ist der Formeln

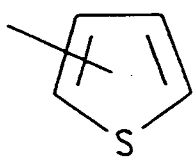,

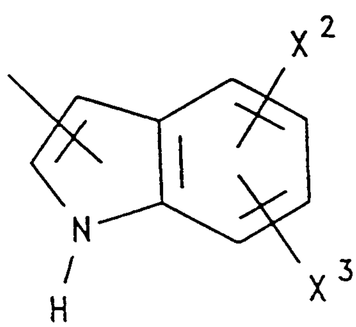,

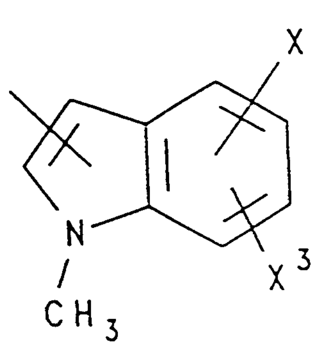

wobei
$X^2$ und $X^3$ unabhängig voneinander Wasserstoff, Hydroxy, Fluor oder Chlor sind,
$R^6$ Wasserstoff, Methyl oder Ethyl ist
$R^{14}$ tert. – Butyl ist,

| | |
|---|---|
| $R^9$ und $R^{10}$ | unabhägig voneinander Wassserstoff oder Hydroxy sind, |
| $R^{13}$ | Wasserstoff ist, |
| $X^1$ | Wasserstoff, 7 – Chlor, 7 – Fluor oder 7 – Nitro ist, |
| $X^7$ | Sauerstoff ist |
| – – – | eine Einfach – oder Doppelbindung angibt, |
| p | 0 ist, wenn die angrenzende – – – Bindung eine Doppelbindung ist und 1, wenn die angrenzende – – – Bindung eine Einfachbindung ist, und |
| r | 1 ist. |

**5.** Verwendung naach Anspruch 1 dadurch gekennzeichnet, daß die Verbindung der Formel I
1,3 – Dihydro – 5 – (2 – fluor – phenyl) – 3(R) – (3' – indolyl)methyl – 2H – 1,4 – benzodiazepin – 2 – on,
1,3 – Dihydro – 5 – (2 – fluor – phenyl) – 3(R) – [3' – (1' – methyl – indolyl) – methyl] – 1 – methyl – 2H – 1,4 – benzodiazepin – 2 – on,

1,3 – Dihydro – 5 – (2 – fluor – phenyl) – 3(R) – (3' – indolyl)methyl – 1 – methyl – 2H – 1,4 – benzodiazepin – 2 – on,

7 – Chlor – – 1,3 – dihydro – 3(R) – (3' – indolyl)methyl – 5 – phenyl – 2H – 1,4 – benzodiazepin – 2 – on,

1,3 – Dihydro – 3(R) – (3' – indolyl)methyl – 5 – phenyl – 2H – 1,4 – benzodiazepin – 2 – on,

1,3 – Dihydro – 5 – (2 – fluor – phenyl) – 3(R) – (3' – α – indolenyl)methyl – 2H – 1,4 – benzodiazepin – 2 – on,

1,3 – Dihydro – 5 – (2 – fluor – phenyl) – 3(R) – (3' – β – indolenyl)methyl – 2H – 1,4 – benzodiazepin – 2 – on,

1,3 – Dihydro – 5 – (2 – fluor – phenyl) – 3(R) – (3' – indolyl)methyl – 2H – 1,4 – benzodiazepin – 2 – thion,

1,3 – Dihydro – 5 – (2 – fluor – phenyl) – 3(R) – (3' – indolyl)methyl – 2H – 1,4 – benzodiazepin,

7 – Chlor • 1,3 – dihydro – 3(R) – benzyl – 5 – phenyl – 2H – 1,4 – benzodiazepin – 2 – on,

3(R) – Benzyloxymethyl – 7 – chlor – 1,3 – dihydro – 5 – phenyl – 2H – 1,4 – benzodiazepin – 2 – on,

7 – Chlor – 1,3 – dihydro – 3(RS) – (1 – naphthyl)methyl – 5 – phenyl – 2H – 1,4 – benzodiazepin – 2 – on,

7 – Chlor – – 1,3 – dihydro – 3(RS) – (2 – naphthyl)methyl – 5 – phenyl – 2H – 1,4 – benzodiazepin – 2 – on,

1,3 – Dihydro – 5 – (2 – fluor – phenyl) – 3(RS) – (2 – thienyl)methyl – 2H – 1,4 – benzodiazepin – 2 – on,

1,3 – Dihydro – 5 – (2 – fluor – phenyl) – 3(RS) – (3 – thienyl) – 2H – 1,4 – benzodiazepin – 2 – on,

1,3 – Dihydro – 5 – (2 – fluor – phenyl) – 3(R) – [3' – β – (1' – t – Boc – L – leucyl) – indolenyl]methyl – 2H – 1,4 – benzodiazepin – 2 – on,

1,3 – Dihydro – 5 – (2 – fluor phenyl) – 3(R) – [3' – β – (1' – t – Boc – D – leucyl) – indolenyl]methyl – 2H – 1,4 – benzodiazepin – 2 – on,

1,3 – Dihydro – 5 – (2 – fluor – phenyl) – 3(R) – [3' – α – (1' – t – Boc – L – leucyl) – indolenyl]methyl – 2H – 1,4 – benzodiazepin – 2 – on,

1,3 – Dihydro – 5 – (2 – fluor – phenyl) – 3(R) – [3' – α – (1' – t – Boc – D – leucyl) – indolenyl]methyl – 2H – 1,4 – benzodiazepin – 2 – on,

7 – Chlor – 1,3,4,5 – tetrahydro – 3(R) – (3' – indolyl)methyl – 5 – phenyl – 2H – 1,4 – benzodiazepin – 2 – on,

7 – Chlor – 1,3,4,5 – tetrahydro – 3(S) – (3' – indolyl)methyl – 5 – phenyl – 2H – 1,4 – benzodiazepin – 2 – on,

4 – (p – Chlor – benzoyl) – 5 – (2 – fluor – phenyl) – 3(R) – [3' – (1' – methylindolyl) – methyl] – 1 – methyl – 1,3,4,5 – tetrahydro – 2H – 1,4 – benzodiazepin – 2 – on,

4 – Acetyl – 5 – (2 – fluor – phenyl) – 3(R) – [3' – (1' – methylindolyl)methyl] – 1 – methyl – 1,3,4,5 – tetrahydro – 2H – 1,4 – benzodiazepin – 2 – on,

7 – Chlor – 5 – (2 – chlor – phenyl) – 1,3 – dihydro – 3(R) – (3' – indolyl)methyl – 2H – 1,4 – benzodiazepin – 2 – on,

1,3 – Dihydro – 3(R) – (3' – indolyl)methyl – 5 – methyl – 2H – 1,4 – benzodiazepin – 2 – on,

1 – Benzyl – 7 – chlor – 1,3 – dihydro – 3(R) – (3' – indolyl)methyl – 5 – phenyl – 2H – 1,4 – benzodiazepin – 2 – on,

7 – Chlor – 1,3 – dihydro – 3(R) – (3' – indolyl)methyl – 1 – methyl – 5 – phenyl – 2H – 1,4 – benzodiazepin – 2 – on,

1,3 – Dihydro – 5 – (2 – fluor – phenyl) – 3(S) – (3' – indolyl)methyl – 2H – 1,4 – benzodiazepin – 2 – on,

1 – Benzyl – 7 – chlor – 1,3 – dihydro – 3(S) – (3' – indolyl)methyl – 5 – phenyl – 2H – 1,4 – benzodiazepin – 2 – on,

7 – Chlor – 1,3 – dihydro – 3(R) – (3' – indolyl)methyl – 5 – phenyl – 2H – 1,4 – benzodiazepin – 2 – thion,

1,3 – Dihydro – 5 – (2 – fluor – phenyl) – 3(R) – (3' – indolyl)methyl – 2H – 1,4 – benzodiazepin – 2 – [N' – (3 – thienoyl)] hydrazid,

1,3 – Dihydro – 1 – ethyl – 5 – (2 – fluor – phenyl) – 3(R) – (3' – indolyl)methyl – 2H – 1,4 – benzodiazepin – 2 – on,

1 – Cyclopropylmethyl – 1,3 – dihydro – 5 – (2 – fluor – phenyl) – 3(R) – (3' – indolyl)methyl – 2H – 1,4 – benzodiazepin – 2 – on,

1,3 – Dihydro – 5 – (2 – fluor – phenyl) – 3(R) – (3' – indolyl)methyl – 1 – pentyl – 2H – 1,4 – benzodiazepin – 2 – on,

1,3 – Dihydro – 5 – (2 – fluor – phenyl) – 3(R) – (3' – indolyl)methyl – 1 – (3 – methylbutyl) – 2H – 1,4 – benzodiazepin – 2 – on,

1,3 – Dihydro – 5 – (2 – fluor – phenyl) – 3(R) – (3' – indolyl)methyl – 1 – (2,2,2 – trifluor – ethyl) – 2H – 1,4 – benzodiazepin – 2 – on,

1,3 – Dihydro – 1 – (2 – dimethylaminoethyl) – 5 – (2 – fluor – phenyl) – 3(R) – (3' – indolyl)methyl – 2H – 1,4 – benzodiazepin – 2 – on,

1,3 – Dihydro – 1 – (ethoxycarbonylmethyl) – 5 – (2 – fluor – phenyl) – 3(R) – (3' – indolyl)methyl – 2H – 1,4 – benzodiazepin – 2 – on,

1 – Carboxymethyl – 1,3 – dihydro – 5 – (2 – fluor – phenyl) – 3(R) – 3' – indolyl)methyl – 2H – 1,4 – benzodiazepin – 2 – on,

1,3 – Dihydro – 5 – (2 – fluor – phenyl) – 3(R) – [3' – (1' – p – chlor – benzyloylindolyl)methyl] – 1 – methyl – 2H – 1,4 – benzodiazepin – 2 – on,

7 – Chlor – 1,3 – dihydro – 3(R) – [3'(1' – benzylindolyl)methyl] – 1 – methyl – 5 – phenyl – 2H – 1,4 – benzodiazepin – 2 – on,

1,3 – Dihydro – 3(RS) – [1 – hydroxy – 1 – (3' – indolyl)]methyl – 1 – methyl – 5 – phenyl – 2H – 1,4 – benzodiazepin – 2 – on,

1,3 – Dihydro – 1 – methyl – 5 – phenyl – 3 – (RS) – (3 – thienoyl) – 2H – 1,4 – benzodiazepin – 2 – on,

1,3 – Dihydro – 3 – (RS) – [1 – hydroxy – 1 – (3 – thienyl)]methyl – 1 – methyl – 5 – phenyl – 2H – 1,4 – benzodiazepin – 2 – on,

1,3 – Dihydro – 3(RS) – [1 – hydroxy – 1 – [3 – (1 – methylindolyl)]] – methyl – 1 – methyl – 5 – phenyl – 2H – 1,4 – benzodiazepin – 2 – on,

2 Stereoisomere,

1,3 – Dihydro – 3(RS) – (1 – hydroxy – 1 – phenyl)methyl – 1 – methyl – 5 – phenyl – 2H – 1,4 – benzodiazepin – 2 – on,

1,3 – Dihydro – 3(RS) – [1 – hydroxy – 1 – (2 – thienyl)]methyl – 1 – methyl – 5 – phenyl – 2H – 1,4 – benzodiazepin – 2 – on,

1,3 – Dihydro – 3 – (RS) – hydroxy – 1 – methyl – 5 – phenyl – 3 – (3' – thienoyl) – 2H – 1,4 – benzodiazepin – 2 – on,

1,5 – Dihydro – 5 – (RS) – hydroxy – 1 – methyl – 5 – phenyl – 3 – (3' – thienoyl) – 2H – 1,4 – benzodiazepin – 2 – on,

7 – Chlor – 1,3 – dihydro – 3(R) – [(2',3' – dihydro – 2' – oxo – 1'H – indol – 3' – yl)methyl] – 5 – phenyl – 2H – 1,4 – benzodiazepin – 2 – on,

7 – Chlor – 1,3 – dihydro – 3(R) – [(3' – (2,4 – dinitrophenyl)imidazol – 5' – yl) – methyl] – 5 – phenyl – 2H – 1,4 – benzodiazepin – 2 – on,

7 – Chlor – 1,3 – dihydro – 3(R) – (3' – imidazol – 5' – yl)methyl – 5 – phenyl – 2H – 1,4 – benzodiazepin – 2 – on,

3(RS) – [3' – (5' – Brom – indolyl)methyl] – 1,3 – dihydro – 5 – phenyl – 2H – 1,4 – benzodiazepin – 2 – on,

5 – o – Carboxyphenyl – 1,3 – dihydro – 3(R) – (3' – indolyl)methyl – 2H – 1,4 – benzodiazepin – 2 – on,

1,3 – Dihydro – 3(RS) – [3' – (5' – fluor – indolyl)methyl] – 5 – o – fluor – phenyl – 2H – 1,4 – benzodiazepin – 2 – on,

1,3 – Dihydro – 3(RS) – [3' – (6' – fluor – indolyl)methyl] – 5 – o – fluor – phenyl – 2H – 1,4 – benzodiazepin – 2 – on,

2 – N – [2(RS),3 – bis – (Bocamino)propanoyl]amino – 2' – fluor benzophenon,

2 – N – [2(RS),3 – Diphthalylaminopropanoyl]amino – 2' – fluor – benzophenon,

1,3 – Dihydro – 5 – (2' – fluor – phenyl) – 3(RS) – aminomethyl – 2H – 1,4 – benzodiazepin – 2 – on,

1,3 – Dihydro – 5 – (2' – fluor – phenyl) – 3(R) – (4 – amino)butyl – 2H – 1,4 – benzodiazepin – 2 – on,

1,3 – Dihydro – 5 – (2' – fluor – phenyl) – 3(RS) – benzyloxycarbonylaminomethyl – 2H – 1,4 – benzodiazepin – 2 – on,

1,3 – Dihydro – 5 – (2' – fluor – phenyl) – 3(RS) – (3 – thiophencarbonyl)aminomethyl – 2H – 1,4 – benzodiazepin – 2 – on,

1,3 – Dihydro – 5 – (2' – fluor – phenyl) – 3(RS) – (2 – indol )carbonylaminomethyl) – 2H – 1,4 – benzodiazepin – 2 – on,

1,3 – Dihydro – 5 – (2' – fluor – phenyl) – 3(RS) – (6' – chlor – pyrazinyl)aminomethyl – 2H – 1,4 – benzodiazepin – 2 – on,

1,3 – Dihydro – 5 – (2' – fluor – phenyl) – 3(RS) – aminomethyl – 1 – methyl – 2H – 1,4 – benzodiazepin – 2 – on,

3(RS) – (2 – Indolecarbonylamino) – 1,3 – dihydro – 5 – phenyl – 2H – 1,4 – benzodiazepin – 2 – on,

1,3 – Dihydro – 3(RS) – [2 – (3 – indolyl)ethyl]amino – 5 – phenyl – 2H – 1,4 – benzodiazepin – 2 – on,

3(RS) – [3 – (3 – Indol )propionylamino] – 1,3 – dihydro – 5 – phenyl – 2H – 1,4 – benzodiazepin – 2 –

on,
3(RS) – (3 – Indol – acetylamino) – 1,3 – dihydro – 5 – phenyl – 2H – 1,4 – benzodiazepin – 2 – on,
3(RS) – (Boc – L – Tryptophanyl)amino – 1,3 – dihydro – 5 – phenyl – 2H – 1,4 – benzodiazepin – 2 – on,
Z – 1,3 – Dihydro – 1 – methyl – 5 – phenyl – 3 – (3 – thienylmethylen ) – 2H – 1,4 – benzodiazepin – 2 – on,
E – 1,3 – Dihydro – 1 – methyl – 5 – phenyl – 3 – (3 – thienylmethylen ) – 2H – 1,4 – benzodiazepin – 2 – on,
3(RS) – (BOC – D – Tryptophyl)amino – 1,3 – dihydro – 5 – phenyl – 2H – 1,4 – benzodiazepin – 2 – on,
3(RS) – [4 – (3 – Indol )butyrylamino] – 1,3 – dihydro – 5 – phenyl – 2H – 1,4 – benzodiazepin – 2 – on,
1,3 – Dihydro – 3(RS) – (benzyloxycarbonyl)aminomethyl – 5 – (2 – fluor – phenyl) – 2H – 1,4 – benzodiazepin,
1,3 – Dihydro – 3(RS) – [3' – (thiophen )carbonyl]aminomethyl – 5 – (2 – fluor phenyl) – 2H – 1,4 – benzodiazepin,
1,3 – Dihydro – 3(RS) – (2 – indol – carbonyl)aminomethyl – 5 – (2 – fluor – phenyl) – 2H – 1,4 – benzodiazepin,
1,3 – Dihydro – 3(RS) – (2 – L – hydroxy – 2 – phenylacetyl)aminomethyl – 5 – (2 – fluor – phenyl) – 2H – 1,4 – benzodiazepin,
1 – (2 – Cyanoethyl) – 1,3 – dihydro – 5 – (2 – fluor – phenyl) – 3(R) – (3' – indolyl)methyl – 2H – 1,4 – benzodiazepin – 2 – on,
1 – (2 – Cyanoethyl) – 1,3 – dihydro – 5 – (2 – fluor – phenyl) – 3(R) – [1' – (2 – cyanoethyl) – 3' – indolyl] – methyl – 2H – 1,4 – benzodiazpin – 2 – on,
1 – (2 – Carboxyethyl) – 1,3 – dihydro – 5 – (2 – fluor – phenyl) – 3(R) – (3' – indolyl)methyl – 2H – 1,4 – benzodiazepin – 2 – on,
1,3 – Dihydro – 3(R) – (3' – indolyl)methyl – 5 – (2 – fluor – phenyl) – 2H – 1,4 – benzodiazepin – 2 – on – 4 – oxid,
1,3 – Dihydro – 5 – (2 – fluor – phenyl) – 3 – (RS) – (2 – indol – carbonylamino) – 2H – 1,4 – benzodiazepin – 2 – on,
1,3 – Dihydro – 5 – (2 – fluor – phenyl) – 3(RS) – (2 – indol – carbonylamino) – 1 – methyl – 2H – 1,4 – benzodiazepin – 2 – on,
1,3 – Dihydro – 5 – (2 – fluor – phenyl) – 1 – methyl – 3(RS) – [2' – (1 – methylindol )carbonylamino] – 2H – 1,4 – benzodiazepin – 2 – on,
1,3 – Dihydro – 3(RS) – (4 – nitrophenylcarbonyl)amino – 5 – (2 – fluor – phenyl) – 2H – 1,4 – benzodiazepin – 2 – on,
1,3 – Dihydro – 3(RS) – (2 – indol – carbonyloxy) – 5 – phenyl – 2H – 1,4 – benzodiazepin – 2 – on,
1,3 – Dihydro – 5 – (2 – fluor – phenyl) – 3(RS) – (3 – thiophencarbonylamino) – 2H – 1,4 – benzodiazepin – 2 – on,
1,3 – Dihydro – 3(RS) – (3 – indol – carbonylamino) – 5 – phenyl – 2H – 1,4 – benzodiazepin – 2 – on,
1,3 – Dihydro – 3(RS) – (4 – thianaphthen – acetyl)amino – 5 – (2 – fluor – phenyl) – 2H – 1,4 – benzodiazepin – 2 – on,
1,3 – Dihydro – 3(RS) – (4 – chlor – phenylcarbonyl)amino – 5 – (2 – fluor – phenyl) – 2H – 1,4 – benzodiazepin – 2 – on,
1,3 – Dihydro – 3(RS) – (4 – methylphenylsulfonyl)amino – 5 – (2 – fluor – phenyl) – 2H – 1,4 – benzodiazepin – 2 – on,
1 – Carboxymethyl – 1,3 – dihydro – 5 – (2 – fluor – phenyl) – 3(RS) – (2 – indol – carbonylamino) – 2H – 1,4 – benzodiazepin – 2 – on,
1,3 – Dihydro – 3(RS) – (5 – fluor – indol – 2 – carbonylamino) – 5 – (2 – fluor – phenyl) – 2H – 1,4 – benzodiazepin – 2 – on,
1,3 – Dihydro – 3(RS) – (3' – methylindenyl – 2 – carbonyl)amino – 5 – (2 – fluor – phenyl) – 2H – 1,4 – benzodiazepin – 2 – on,
1,3 – Dihydro – 3(RS) – (2 – chinaldyl)amino – 5 – (2 – fluor – phenyl) – 2H – 1,4 – benzodiazepin – 2 – on,
1,3 – Dihydro – 3(RS) – (2 – L – hydroxy – 2 – phenylacetyl)amino – 5 – (2 – fluor – phenyl) – 2H – 1,4 – benzodiazepin – 2 – on,
1,3 – Dihydro – 3(RS) – (5 – chlor – indol – 2 – carbonylamino) – 5 – (2 – fluor – phenyl) – 2H – 1,4 – benzodiazepin – 2 – on,
3(RS) – [N – (2 – Indol – carbonyl) – N – methylamino] – 1,3 – dihydro – 5 – phenyl – 2H – 1,4 – benzodiazepin – 2 – on,
1,3 – Dihydro – 3(RS) – (2 – indol – carbonylamino) – 1 – methyl – 5 – phenyl – 2H – 1,4 –

benzodiazepin – 2 – on,

1,3 – Dihydro – 1 – methyl – 3 – (RS) – [2 – (1 – methylindol )carbonylamino] – 5 – phenyl – 2H – 1,4 – benzodiazepin – 2 – on,

1 – Carboxymethyl – 1,3 – dihydro – 3(RS) – (2 – indolecarbonylamino) – 5 – phenyl – 2H – 1,4 – benzodiazepin – 2 – on,

1,3 – Dihydro – 3(RS) – (5 – brom – indol – 2 – carbonylamino) – 5 – (2 – fluor – phenyl) – 2H – 1,4 – benzodiazepin – 2 – on,

3(RS) – Cinnamoylamino – 1,3 – dihydro – 5 – (2 – fluor – phenyl) – 2H – 1,4 – benzodiazepin – 2 – on,

1,3 – Dihydro – 3(RS) – (5 – hydroxy – 2 – indolylcarbonyl)amino – 5 – (2 – fluor – phenyl) – 2H – 1,4 – benzodiazepin – 2 – on,

1 – Carboxamidomethyl – 1,3 – dihydro – 3R – (3 – indolylmethyl) – 5 – (2 – fluor – phenyl) – 2H – 1,4 – benzodiazepin – 2 – on,

1,3 – Dihydro – 5 – (2 – fluor – phenyl) – 3 – (RS) – (2 – indolylmethylamino) – 2H – 1,4 – benzodiazepin – 2 – on,

1,3 – Dihydro – 3(RS) – (phenylaminomethylcarbonyl)amino – 5 – (2 – fluor – phenyl) – 2H – 1,4 – benzodiazepin – 2 – on,

1,3 – Dihydro – 3(RS) – (5 – methoxyindol – 2 – carbonylamino) – 5 – (2 – fluorophenyl) – 2H – 1,4 – benzodiazepin – 2 – one;

1,3 – Dihydro – 3(RS) – (1 – methylindol – 2 – carbonylamino) – 5 – (2 – fluor – phenyl) – 2H – 1,4 – benzodiazepin – 2 – on,

1,3 – Dihydro – 1 – methyl – 3(RS) – (4 – chlor – phenylcarbonyl)amino – 5 – (2 – fluor – phenyl) – 2H – 1,4 – benzodiazepin – 2 – on,

1,3 – Dihydro – 5 – (2 – fluor – phenyl) – 3(RS) – (2 – benzofurancarbonylamino) – 2H – 1,4 – benzodiazepin – 2 – on,

1 – Ethoxycarbonylmethyl – 1,3 – dihydro – 3(RS) – (4 – chlor – phenylcarbonyl)amino – 5 – (2 – fluor – phenyl) – 2H – 1,4 – benzodiazepin – 2 – on,

1,3 – Dihydro – 3(RS) – (4 – chlor – phenylcarbonyl)amino – 5 – phenyl – 2H – 1,4 – benzodiazepin – 2 – on,

1,3 – Dihydro – 1 – methyl – 3(RS) – (4 – chlor – phenylcarbonyl)amino – 5 – phenyl – 2H – 1,4 – benzodiazepin – 2 – on,

1 – Carboxymethyl – 1,3 – dihydro – 3(RS) – (4 – chlor – phenylcarbonyl)amino – 5 – (2 – fluor – phenyl) – 2H – 1,4 – benzodiazepin – 2 – on,

3(RS) – (2(S) – tert – Butoxycarbonylamino – 3 – phenylpropanoylamino) – 1,3 – dihydro – 5 – phenyl – 2H – 1,4 – benzodiazepin – 2 – on,

3(RS) – (2(S) – tert – Butoxycarbonylamino – 3 – phenylpropanoylamino) – 1,3 – dihydro – 1 – methyl – 5 – phenyl – 2H – 1,4 – benzodiazepin – 2 – on,

3(R) – nd 3(S) – (2(S) – Amino – 3 – phenylpropanoylamino) – 1,3 – dihydro – 1 – methyl – 5 – phenyl – 2H – 1,4 – benzodiazepin – 2 – on,

3(R) – und 3(S) – Amino – 1,3 – dihydro – 1 – methyl – 5 – phenyl – 2H – 1,4 – benzodiazepin – 2 – on,

3(R) – und 3(S) – Amino – 1,3 – dihydro – 5 – (2 – fluor – phenyl) – 1 – methyl – 2H – 1,4 – benzodiazepin – 2 – on,

3(S) – ( – ) – 1,3 – Dihydro – 3 – (2 – indol – carbonylamino) – 1 – methyl – 5 – phenyl – 2H – 1,4 – benzodiazepin – 2 – on,

3(S) – ( + ) – 1,3 – Dihydro – 5 – (2 – fluor – phenyl) – 3 – (2 – indolcarbonylamino) – 1 – methyl – 2H – 1,4 – benzodiazepin – 2 – on,

3(R) – ( – ) – 1,3 – Dihydro – 5 – (2 – fluor – phenyl) – 3 – (2 – indolcarbonylamino) – 1 – methyl – 2H – 1,4 – benzodiazepin – 2 – on,

3(R) – ( – ) – 1,3 – Dihydro – 3 – (4 – chlor – benzoylamino) – 5 – (2 – fluor – phenyl) – 1 – methyl – 2H – 1,4 – benzodiazepin – 2 – on,

3(S) – ( + ) – 1,3 – Dihydro – 3 – (4 – chlor – benzoylamino) – 5 – (2 – fluor – phenyl) – 1 – methyl – 2H – 1,4 – benzodiazepin – 2 – on,

3(S) – ( – ) – 1,3 – Dihydro – 3 – (4 – brom – benzoylamino) – 1 – methyl – 5 – phenyl – 2H – 1,4 – benzodiazepin – 2 – on,

3(R) – ( + ) – 1,3 – Dihydro – 3 – (4 – brom – benzoylamino) – 1 – methyl – 5 – phenyl – 2H – 1,4 – benzodiazepin – 2 – on,

3(R) – ( + ) – 1,3 – Dihydro – 3 – (2 – indol – carbonylamino) – 1 – methyl – 5 – phenyl – 2H – 1,4 – benzodiazepin – 2 – on,

1,3 – Dihydro – 3 – (RS) – (2 – indolin – carbonylamino) – 5 – phenyl – 2H – 1,4 – benzodiazepin – 2 –

on,

1,3 – Dihydro – 5 – (2 – fluor – phenyl) – 3 – (RS) – (p – trifluormethylbenzoylamino) – 2H – 1,4 – benzodiazepin – 2 – on,

1,3 – Dihydro – 5 – (2 – fluor – phenyl) – 3 – (RS) – (p – methylbenzoylamino) – 2H – 1,4 – benzodiazepin – 2 – on,

1,3 – Dihydro – 5 – (2 – fluor – phenyl) – 3 – (RS) – (p – methoxybenzoylamino) – 2H – 1,4 – benzodiazepin – 2 – on,

3 – (RS) – (o – Chlor – benzoylamino) – 1,3 – dihydro – 5 – phenyl – 2H – 1,4 – benzodiazepin – 2 – on,

3 – (RS) – (o – Chlor – benzoylmethylamino – 1,3 – dihydro – 1 – methyl – 5 – phenyl – 2H – 1,4 – benzodiazepin – 2 – on,

3 – (RS) – (o – Chlor – benzoylamino) – 1,3 – dihydro – 1 – methyl – 5 – phenyl – 2H – 1,4 – benzodiazepin – 2 – on,

3 – (RS) – (m – Chlor – benzoylamino) – 1,3 – dihydro – 5 – phenyl – 2H – 1,4 – benzodiazepin – 2 – on,

3 – (RS) – (3,4 – Dichlor – benzoylamino) – 1,3 – dihydro – 5 – phenyl – 2H – 1,4 – benzodiazepin – 2 – on

3 – (RS) – (p – Chlor – benzoylamino)1,3 – dihydro – 5 – (2' – fluor – phenyl) – 1 – methyl – 4 – oxo – 2H – 1,4 – benzodiazepin – 2 – on,

1,3 – Dihydro – 5 – phenyl – 3 – (RS) – (4' – methylthiobenzoylamino) – 2H – 1,4 – benzodiazepin – 2 – on,

1 – 3 – Dihydro – 3 – (RS) – (4' – fluor – benzoylamino) – 5 – phenyl – 2H – 1,4 – benzodiazepin – 2 – on,

1,3 – Dihydro – 5 – phenyl – 3 – (RS) – (4' – trifluoromethylbenzoylamino) – 2H – 1,4 – benzodiazepin – 2 – on,

1,3 – Dihydro – 3 – (RS) – (4' – tert – butylbenzoylamino) – 5 – phenyl – 2H – 1,4 – benzodiazepin – 2 – on,

3 – (RS) – (3,5 – Dichlor – benzoylamino)1,3 – dihydro – 5 – phenyl – 2H – 1,4 – benzodiazepin – 2 – on,

1 – 3 – Dihydro – 3 – (RS) – (p – hydroxybenzoylamino) – 5 – phenyl – 2H – 1,4 – benzodiazepin – 2 – on,

3 – (RS) – (4' – Cyanobenzoylamino)1,3 – dihydro – 5 – phenyl – 2H – 1,4 – benzodiazepin – 2 – on,

3(S) – ( – ) – 3 – (2 – Chlor – benzoylamino) – 1,3 – dihydro – 1 – methyl – 5 – phenyl – 2H – 1,4 – benzodiazepin – 2 – on,

3(R) – ( + ) – 3 – (2 – Chlor – benzoylamino) – 1,3 – dihydro – 1 – methyl – 5 – phenyl – 2H – 1,4 – benzodiazepin – 2 – on,

1,3 – Dihydro – 3 – (RS) – (p – dimethylaminobenzoylamino) – 5 – (2 – fluor – phenyl) – 2H – 1,4 – benzodiazepin – 2 – on,

1,3 – Dihydro – 3(RS) – (3,4 – dimethoxybenzoylamino) – 5 – (2 – fluor – phenyl) – 2H – 1,4 – benzodiazepin – 2 – on,

3(S) – ( + ) – 3 – (3 – Brom – benzoylamino) – 1,3 – dihydro – 5 – (2 – fluor – phenyl) – 1 – methyl – 2H – 1,4 – benzodiazepin – 2 – on,

1,3 – Dihydro – 5 – phenyl – 3(RS) – (3 – trifluor – methylthiobenzoylamino) – 2H – 1,4 – benzodiazepin – 2 – on,

3(S) – ( + ) – 3 – (4 – Brom – benzoylamino) – 1,3 – dihydro – 5 – (2 – fluor – phenyl) – 1 – methyl – 2H – 1,4 – benzodiazepin – 2 – on,

3(S) – ( + ) – 3 – (4 – t – Butylbenzoylamino) – 1,3 – dihydro – 5 – (2 – fluor – phenyl) – 1 – methyl – 2H – 1,4 – benzodiazepin – 2 – on,

1,3 – Dihydro – 5 – (2 – fluor – phenyl) – 3(RS) – (pyrrol – 2 – carbonylamino) – 2H – 1,4 – benzodiazepin – 2 – on,

3(S) – ( + ) – 1,3 – Dihydro – 5 – (2 – fluor – phenyl) – 3 – (4 – iodbenzoylamino) – 1 – methyl – 2H – 1,4 – benzodiazepin – 2 – on,

1,3 – Dihydro – 3(RS) – (2 – naphthoylamino) – 5 – phenyl – 2H – 1,4 – benzodiazepin – 2 – on,

3(S) – ( – ) – 3 – (2 – Brom – benzoylamino) – 1,3 – dihydro – 5 – (2 – fluorphenyl) – 1 – methyl – 2H – 1,4 – benzodiazepin – 2 – on,

3(S) – ( + ) – 3 – (4 – Cyanobenzoylamino) – 1,3 – dihydro – 5 – (2 – fluor – phenyl) – 1 – methyl – 2H – 1,4 – benzodiazepin – 2 – on,

1,3 – Dihydro – 5 – phenyl – 3(RS) – (4 – n – propylbenzoylamino) – 2H – 1,4 – benzodiazepin – 2 – on,

1,3 – Dihydro – 5 – phenyl – 3(RS) – (4 – phenylbenzoylamino) – 2H – 1,4 – benzodiazepin – 2 – on,

1,3 – Dihydro – 3(RS) – (4 – n – pentylbenzoylamino) – 5 – phenyl – 2H – 1,4 – benzodiazepin – 2 – on,

1,3 – Dihydro – 3(RS) – (1 – naphthoylamino) – 5 – phenyl – 2H – 1,4 – benzodiazepin – 2 – on,

3(S) – ( + ) – 1,3 – Dihydro – 5 – (2 – fluor – phenyl) – 3 – (3 – iodbenzoylamino) – 1 – methyl – 2H –

1,4 – benzodiazepin – 2 – on,
3(R) – ( – ) – 1,3 – Dihydro – 5 – (2 – fluor – phenyl) – 3 – (3 – iodbenzoylamino) – 1 – methyl – 2H – 1,4 – benzodiazepin – 2 – on,
3(R) – ( + ) – 1,3 – Dihydro – 5 – (2 – fluor – phenyl) – 3 – (2 – iodbenzoylamino) – 1 – methyl – 2H – 1,4 – benzodiazepin – 2 – on,
3(S) – ( – ) – 1,3 – Dihydro – 5 – (2 – fluor – phenyl) – 3 – (2 – iodbenzoylamino) – 1 – methyl – 2H – 1,4 – benzodiazepin – 2 – on,
3(R) – ( + ) – 3 – (2 – Brom – benzoylamino) – 1,3 – dihydro – 5 – (2 – fluor – phenyl) – 1 – methyl – 2H – 1,4 – benzodiazepin – 2 – on,
3(R) – ( + ) – 3 – (2 – Chlor – benzoylamino) – 1,3 – dihydro – 5 – (2 – fluor – phenyl) – 1 – methyl – 2H – 1,4 – benzodiazepin – 2 – on,
1,3 – Dihydro – 5 – (2 – fluor – phenyl) – 3(RS) – phenylcarbonylamino – 2H – 1,4 – benzodiazepin – 2 – on,
1,3 – Dihydro – 5 – (2 – fluor – phenyl) – 3(RS) – (2 – chlor – phenyl)carbonylamino – 2H – 1,4 – benzodiazepin – 2 – on,
1,3 – Dihydro – 5 – (2 – fluor – phenyl) – 3(RS) – benzyloxycarbonylamino – 2H – 1,4 – benzodiazepin – 2 – on,
1,3 – Dihydro – 5 – (2 – fluor – phenyl) – 3 – (RS) – benzyloxycarbonylamino – 2H – 1,4 – benzodiazepin – 2 – thion,
1 – (4 – Chlor – phenyl)carbonyl – 1,3 – dihydro – 5 – (2 – fluor – phenyl) – 3(RS) – (4 – chlor – phenyl) – carbonylamino – 2H – 1,4 – benzodiazepin – 2 – on,
1 – (4 – Chlor – phenyl)carbonyl – 1,3 – dihydro – 5 – (2 – fluor – phenyl) – 3(RS) – (4 – chlor – phenyl) – carbonyloxy – 2H – 1,4 – benzodiazepin – 2 – on,
1,3 – Dihydro – 5 – (2 – fluor – phenyl) – 3(RS) – (4 – chlor – phenyl)oxy – 2H – 1,4 – benzodiazepin – 2 – on,
1,3 – Dihydro – 5 – (2 – fluor – phenyl) – 3 – (RS) – (4 – chlor – phenyl)carbonylamino – 2H – 1,4 – benzodiazepin – 2 – thion,
1,3 – Dihydro – 5 – (2 – fluor – phenyl) – 3 – (RS) – (2 – indol)carbonylamino – 2H – 1,4 – benzodiazepin – 2 – thion
1,3 – Dihydro – 3(RS) – (4 – chlor – phenyl)aminocarbonylamino – 2H – 1,4 – benzodiazepin – 2 – on,
ist.

**6.** Verwendung nach Anspruch 1, dadurch gekennzeichnet, daß die Verbindung der Formel I
(S) – ( – ) – 1,3 – Dihydro – 3 – (4 – brom – benzoylamino) – 1 – methyl – 5 – phenyl – 2H – 1,4 – benzodiazepin – 2 – on,
3(RS) – (2 – Indol – carbonylamino) – 1,3 – dihydro – 5 – phenyl – 2H – 1,4 – benzodiazepin – 2 – on,
1,3 – Dihydro – 3(RS) – (2 – indol – carbonylamino) – 1 – methyl – 5 – phenyl – 2H – 1,4 – benzodiazepin – 2 – on,
1,3 – Dihydro – 1 – methyl – 3(RS) – [2 – (1 – methylindol)carbonylamino] – 5 – phenyl – 2H – 1,4 – benzodiazepin – 2 – on,
1,3 – Dihydro – 1 – methyl – 3(RS) – (4 – chlor – phenylcarbonyl)amino – 5 – (2 – fluor – phenyl) – 2H – 1,4 – benzodiazepin – 2 – on,
1,3 – Dihydro – 5 – (2 – fluor – phenyl) – 3(RS) – (2 – indol – carbonylamino) – 1 – methyl – 2H – 1,4 – benzodiazepin – 2 – on,
1,3 – Dihydro – 5 – (2 – fluor – phenyl) – 1 – methyl – 3(RS) – [2' – (1 – methylindol )carbonylamino] – 2H – 1,4 – benzodiazepin – 2 – on,
3(R) – ( – ) – 1,3 – Dihydro – 3 – (4 – chlor – benzoylamino) – 5 – (2 – fluor – phenyl) – 1 – methyl – 2H – 1,4 – benzodiazepin – 2 – on,
3(S) – ( + ) – 1,3 – Dihydro – 3 – (4 – chlor benzoylamino) – 5 – (2 – fluor – phenyl) – 1 – methyl – 2H – 1,4 – benzodiazepin – 2 – on,
3(R) – ( + ) – 1,3 – Dihydro – 3 – (4 – brom – benzoylamino) – 1 – methyl – 5 – phenyl – 2H – 1,4 – benzodiazepin – 2 – on,
3(R) – ( + ) – 1,3 – Dihydro – 3 – (2 – indol – carbonylamino) – 1 – methyl – 5 – phenyl – 2H – 1,4 – benzodiazepin – 2 – on,
1,3 – Dihydro – 5 – (2 – fluor – phenyl) – 3 – (RS) – (2 – indol – carbonylamino) – 2H – 1,4 – benzodiazepin – 2 – on,
1,3 – Dihydro – 3 – (RS) – (2 – indol – carbonyloxy) – 5 – phenyl – 2H – 1,4 – benzodiazepin – 2 – on,
1,3 – Dihydro – 3 – (RS) – (4 – chlor – phenylcarbonyl)amino – 5 – (2 – fluor – phenyl) – 2H – 1,4 –

benzodiazepin-2-on,
1-Carboxymethyl-1,3-dihydro-5-(2-fluor-phenyl)-3(RS)-(2-indol-carbonylamino)-2H-1,4-benzodiazepin-2-on,
1,3-Dihydro-3-(RS)-(5-fluor-indol-2-carbonylamino)-5-(2-fluor-phenyl)-2H-1,4-benzodiazepin-2-on,
3-(RS)-Cinnamoylamino-1,3-dihydro-5-(2-fluor-phenyl)-2H-1,4-benzodiazepin-2-on,
1,3-Dihydro-5-(2-fluor-phenyl)-3-(RS)-(2-benzofurancarbonylamino)-2H-1,4-benzodiazepin-2-on,
1,3-Dihydro-1-methyl-3-(RS)-(4-chlor-phenylcarbonyl)amino-5-phenyl-2H-1,4-benzodiazepin-2-on,
1-Carboxymethyl-1,3-dihydro-3-(RS)-(4-chlor-phenylcarbonyl)amino-5-(2-fluor-phenyl)-2H-1,4-benzodiazepin-2-on,
1,3-Dihydro-5-(2-fluor-phenyl)-3-(RS)-(p-trifluormethylbenzoylamino)-2H-1,4-benzodiazepin-2-on,
3(S)-(+)-3-(3-Brom-benzoylamino)-1,3-dihydro-5-(2-fluorphenyl)-1-methyl-2H-1,4-benzodiazepin-2-on,
3(S)-(+)-3-(4-Brom-benzoylamino)-1,3-dihydro-5-(2-fluorphenyl)-1-methyl-2H-1,4-benzodiazepin-2-on,
3(S)-(+)-1,3-Dihydro-5-(2-fluor-phenyl)-3-(4-iodbenzoylamino)-1-methyl-2H-1,4-benzodiazepin-2-on,
1,3-Dihydro-3(RS)-(2-naphthoylamino)-5-phenyl-2H-1,4-benzodiazepin-2-on,
3(S)-(+)-1,3-Dihydro-5-(2-fluor-phenyl)-3-(3-iodbenzoylamino)-1-methyl-2H-1,4-benzodiazepin-2-on,
3(R)-(-)-1,3-Dihydro-5-(2-fluor-phenyl)-3-(3-iodbenzoylamino)-1-methyl-2H-1,4-benzodiazepin-2-on,
3(R)-(-)-1,3-Dihydro-5-(2-fluor-phenyl)-3-(2-indol-carbonylamino)-1-methyl-2H-1,4-benzodiazepin-2-on,
ist.

7. Verwendung nach Anspruch 1, dadurch gekennzeichnet, daß die Verbindung der Formel I
1-Carboxymethyl-1,3-dihydro-3(RS)-(2-indol-carbonylamino)-5-phenyl-2H-1,4-benzodiazepin-2-on,
(S)-(-)-1,3-Dihydro-3-(2-indol-carbonylamino)-1-methyl-5-phenyl-2H-1,4-benzodiazepin-2-on,
(S)-(+)-1,3-Dihydro-5-(2-fluor-phenyl)-3-(2-indol-carbonylamino)-1-methyl-2H-1,4-benzodiazepin-2-on,
(S)-(+)-1,3-Dihydro-3-(4-chlor-benzoylamino)-5-(2-fluor-phenyl)-1-methyl-2H-1,4-benzodiazepin-2-on ist

8. Verfahren zur Herstellung von Benzodiazepin-Derivaten, entsprechend der folgenden Formel Ia

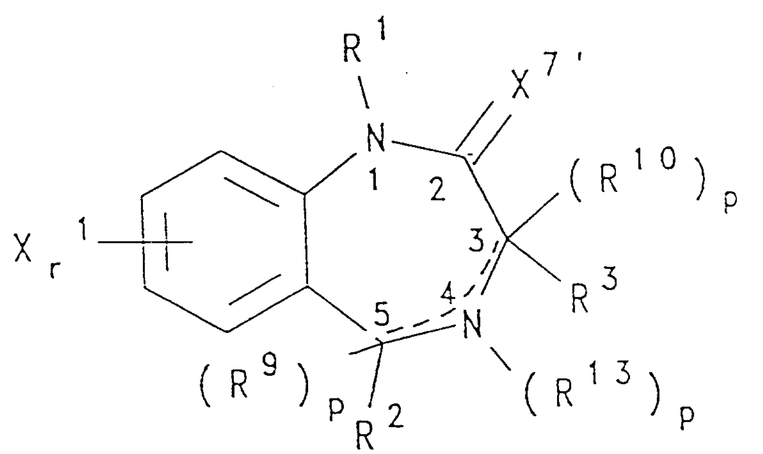

Ia

wobei $X^{7'}$ S, oder HH ist und die restlichen Substituenten und Symbole die gleichen sind wie bei den

Verbindungen der Formel I in Anspruch 1 definiert, oder von Salzen oder quaternären Ammoniumsalzen davon, umfassend:

a) Kuppeln von 2-Aminoaryl-ketonen

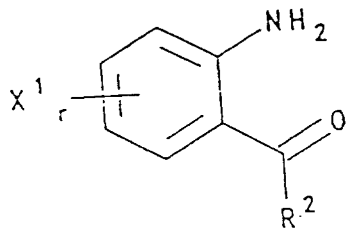

1

mit N-geschützten D-Aminosäuren

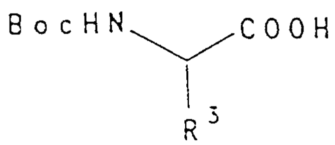

2,

in Gegenwart eines Peptid-Kupplungs-Reagens, Entfernen der N-Schutzgruppe von dem Produkt

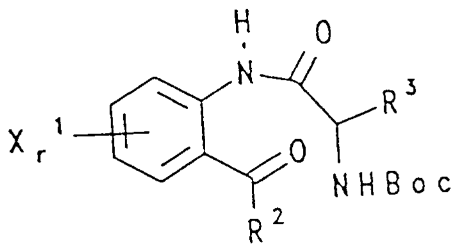

3

mit einer Säure, vorzugsweise wasserfreier Chlorwasserstoffsäure, unter Bildung des α-Aminoacyl-Derivats

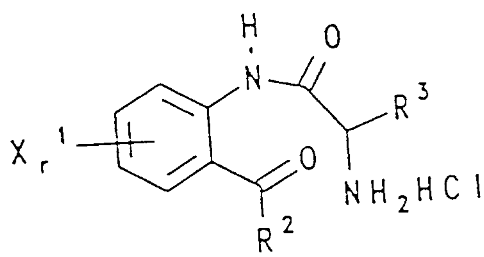

4

wobei dieses Derivat alternativ erhalten wird durch Behandlung des 2-Arylamino-ketons 1 mit einem Säurechlorid-hydrochlorid

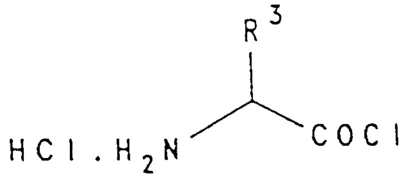

8

Behandlung des α – Aminoacyl – Derivats 4 mit Base und Cyclisieren der freien Base

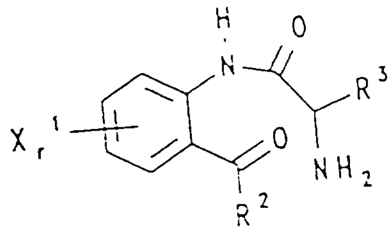

6

zu dem 3,5 – disubstituierten Benzodiazepin durch 2 bis 120 Stunden langes Rühren in der methanolischen Base

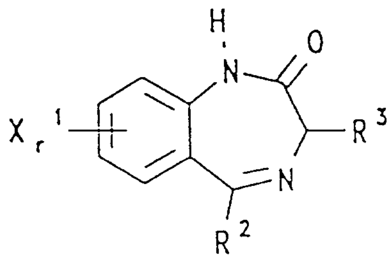

7

wobei dieses Benzodiazepin alternativ erhalten wird durch Erhitzen des Ketons 1 mit einem Ester

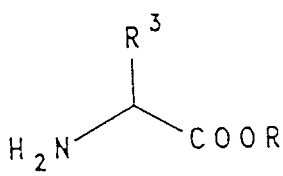

8

wobei bei dem Ester R vorzugsweise Methyl oder Ethyl ist, Behandeln der 3,5 – disubstituierten Benzodiazepine 7 mit $P_2S_5$ oder Lawessonschem Reagens unter Bildung von 2 – Thionen

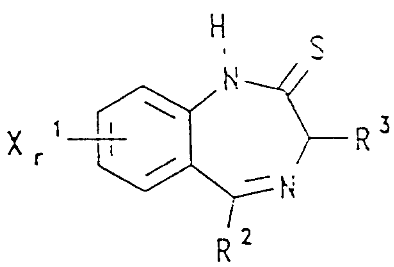

1 4

gegebenenfalls Reduzieren der 2 – Thione 14 mit Raney – Nickel zu den 2 – unsubstituierten Ben – zodiazepinen

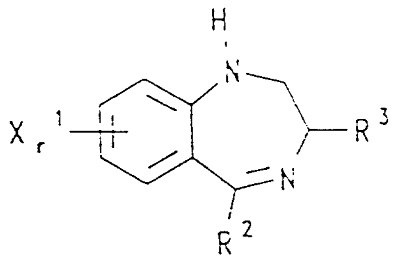

15

und gegebenenfalls Alkylieren mit Alkylhalogenid oder −sulfat, Acylieren mit Acylhalogenid oder −anhydrid, Reduzieren mit Natrium−cyanoborhydrid oder Substituieren mit Alkyl− oder Aryl−magnesiumhalogenid unter Bildung der entsprechenden Alkyl−Derivate, Acyl−Derivate 4,5−Dihydro−Derivate und substituierten Derivate oder
b) Kuppeln der Ketone 1 mit N−Phthalaminosäuren

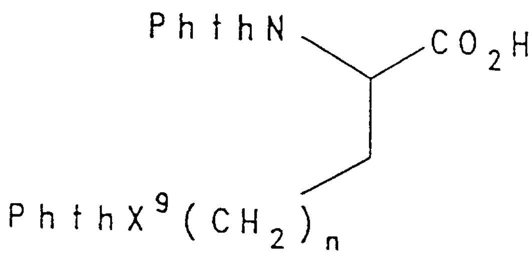

2 b

in Gegenwart eines Peptid−Kupplungs−Reagens und Cyclisieren des Zwischenprodukts durch Behandeln mit Hydrazin

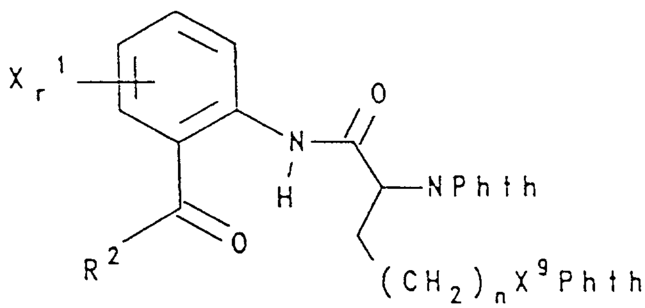

3 b

möglicherweise nach N−Alkylieren durch Behandlung mit Natriumhydrid und anschließend $R^1X$ in Dimethylformamid unter Bildung des Benzodiazepin−Derivats 9

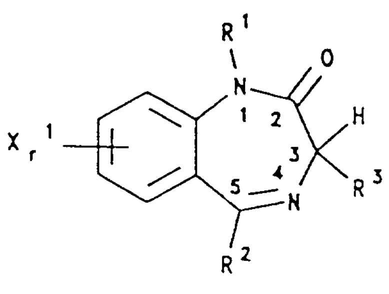

9

wobei
$R^3$ $-(CH_2)_{n+1}-X^9H$ ist; derartige Verbindungen der Formel 9 können umgewandelt werden in weitere Verbindungen der Formel 9 durch:

a) Alkylieren durch Behandlung mit einem Alkylhalogenid oder Dialkylsulfat der Formel
$R^7-(CH_2)_q-X$
unter Bildung von Verbindungen der Formel 9a, in der $R^3$
$-(CH_2)_{n+1}-X^9(CH_2)_q-R^7$ ist,
b) Acylieren durch Behandlung mit Säurehalogeniden oder Säureanhydriden der Formel

$$R^7-(CH_2)_q-\overset{O}{\overset{\vdots}{C}}-X$$

unter Bildung von acylierten Derivaten der Formel 9b, wobei

$$R^3 \qquad -(CH_2)_{n+1}-X^9\overset{O}{C}(CH_2)_q-R^7$$

ist, oder
c) Umwandeln der Verbindungen der Formel 9, wobei $R^3$ $-(CH_2)_{n+1}-NH_2$ ist, zu einem entsprechenden Derivat, bei dem die Gruppe $NH_2$ geschützt ist mit der Benzoyl-carbonyl-Gruppe, und Umwandlung dieser Verbindung zu dem entsprechenden 2-Thion durch Umsetzung mit $P_2S_5$ oder Lawessonschem Reagens und gegebenenfalls Reduzieren des 2-Thions mit Raney-Nickel in Methanol zu den 2-unsubstituierten Benzodiazepinen, anschließende Entfernung der Schutzgruppe durch Hydrogenolyse und gegebenenfalls Alkylieren oder Acylieren der erhaltenen Produkte durch Behandlung mit Alkylhalogenid oder Dialklylsulfat in Gegenwart eines Peptid-Kupplungs-Reagens, und Herstellung von Salzen oder quaternären Ammoniumsalzen davon, soweit erwünscht.

**9.** Verfahren zur Herstellung von neuen Benzodiazepin-Derivaten, entsprechend der folgenden Formel Ib

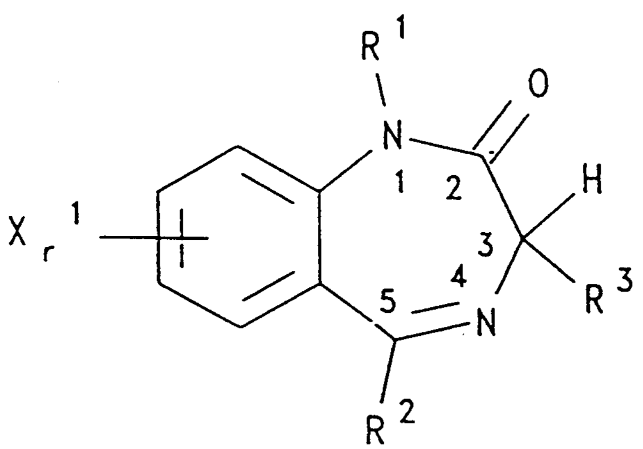

I b

oder Salzen oder quaternären Ammoniumsalzen der Verbindungen der Formel Ib, wobei bei den Verbindungen der Formel Ib

- $R^1$ Alkyl mit 1 bis 5 Kohlenstoffatomen oder Wasserstoff ist,
- $R^2$ unsubstituiertes Phenyl, substituiertes Phenyl mit 1 oder 2 Substituenten, ausgewählt aus Halogen, Alkyl mit 1 bis 4 Kohlenstoffatomen, Alkoxy mit 1 bis 4 Kohlenstoffatomen, Carboxy-substituiertes Alkyl mit 1 bis 4 Kohlenstoffatomen in der Alkylgruppe, Carboxyl, Nitro, Trifluormethyl und Hydroxy ist,
- $R^3$ eine Gruppe ist der Formeln

$$-(CH_2)_nNH\overset{O}{\overset{\|}{C}}(CH_2)_qR^7 \qquad \text{oder} \qquad -(CH_2)_nNH\overset{O}{\overset{\|}{C}}NH(CH_2)_nR^7;$$

wobei

$R^7$ unsubstituiertes Phenyl oder substituiertes Phenyl mit 1 oder 2 Substituenten, ausgewählt aus Halogen, Nitro, Hydroxy, Alkyl mit 1 bis 4 Kohlenstoffatomen, Alkoxy mit 1 bis 4 Kohlenstoffatomen, Alkylthio mit 1 bis 4 Kohlenstoffatomen, Trifluormethyl oder Gruppen der Formeln CN, $-C \equiv CH$, $SCF_3$, $OCCH_3$, $OCHF_2$, SPh, oder $-NR^4R^5$ ist,

wobei $R^4$ und $R^5$ unabhängig voneinander Wasserstoff oder Alkyl mit 1 bis 4 Kohlenstoffatomen sind,

oder der Rest

$R^7$ eine Gruppe ist der Formel

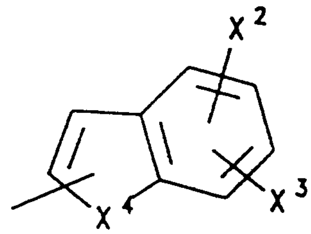

,

wobei

$X^2$ und $X^3$ unabhängig voneinander Wasserstoff, Hydroxy, Nitro, Halogen, Alkylthio mit 1 bis 4 Kohlenstoffatomen, Alkyl mit 1 bis 4 Kohlenstoffatomen oder Alkoxy mit 1 bis 4 Kohlenstoffatomen sind,

$X^4$ NH ist,

q 0 bis 4, vorzugsweise 0, ist und

n 0 bis 4, vorzugsweise 0, ist,

umfassend die Wege a) oder b), wie in Anspruch 8 angegeben in dem erforderlichen Ausmaß, oder

c) Einführen des Substituenten $R^3$ der Formel

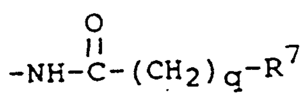

in ein 3-chlor-substituiertes-5-substituiertes-1-substituiertes Benzodiazepin 9d

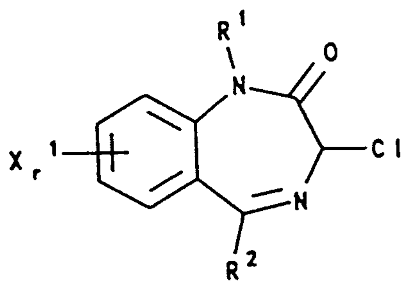

9d

oder

d) Umsetzung eines 3-amino- oder 3-aminoalkyl-5-substituierten-1-substituierten Benzodiazepins

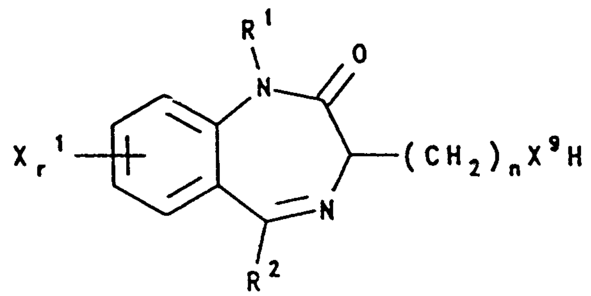 23

mit einem Acylhalogenid oder Isocyanat unter Bildung des entsprechenden Amids oder Harnstoffs

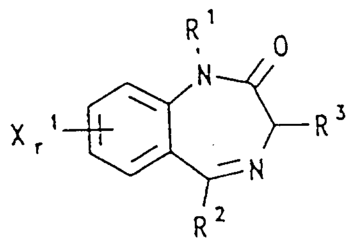 24

$(R^3 = (CH_2)_n NH\overset{O}{\overset{\|}{C}}(CH_2)_q R^7$ oder $(CH_2)_n \overset{H}{N}-\overset{O}{\overset{\|}{C}}-\overset{H}{N}(CH_2)_n R^7$

und gegebenenfalls Alkylieren der erhaltenen Benzodiazepine der Formel Ib, wobei $R^1 = H$ ist, durch Behandeln mit Natriumhydrid in einem geeigneten Lösungsmittel, wie DMF, und anschließend mit einem Alkylhalogenid und Herstellung von Salzen oder quaternären Ammoniumsalzen davon, soweit erwünscht.

**10.** Verfahren nach Anspruch 9, wobei ein Benzodiazepin – Derivat der Formel Ib hergestellt wird, wobei
$R^1$ Methyl ist,
$R^2$ unsubstituiertes Phenyl ist,
$R^3$ wie in Anspruch 9 definiert ist, wobei
q und n 0 sind, und
$R^7$ wie in Anspruch 9 definiert ist, wobei in der Indolyl – Gruppe $X^2$ und $X^3$ jedoch Wasserstoff sind.

**11.** Verfahren nach Anspruch 9 zur Herstellung der Verbindung 3(S) – ( – ) – 1,3 – Dihydro – 3 – (2 – indol – carbonylamino) – 1 – methyl – 5 – phenyl – 2H – 1,4 – benzodiazepin – 2 – on.

**Revendications**

**Revendications pour les Etats contractants suivants : BE, CH, DE, FR, GB, IT, LI, LU, NL, SE**

**1.** Utilisation de composés de formule I

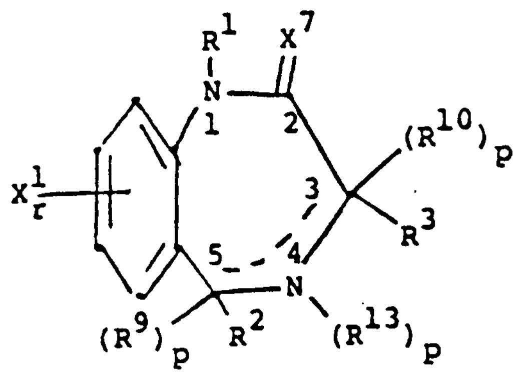

I

ou de sels pharmaceutiquement acceptables ou de sels d'ammonium quaternaire pharmaceutiquement acceptables desdits composés de formule I, composés de formule I dans lesquels

$R^1$ est un atome d'hydrogène ou un groupe alkyle ayant de 1 à 5 atomes de carbone, alcényle ayant jusqu'à 5 atomes de carbone, alcynyle ayant jusqu'à 5 atomes de carbone, ou un groupe de formules $-(CH_2)_mCOOR^6$, $-(CH_2)_n-$cycloalkyle($C_3-C_5$), $-(CH_2)_mNR^4R^5$, $-(CH_2)_mCONR^4R^5$, $-(CH_2)_mCN$ ou $-(CH_2)_nCX_3^{10}$, formules dans lesquelles

$R^4$ et $R^5$ sont indépendamment un atome d'hydrogène ou un groupe alkyle ayant de 1 à 4 atomes de carbone ou cycloalkyle ayant de 3 à 5 atomes de carbone,

$R^6$ est un atome d'hydrogène ou un groupe alkyle ayant de 1 à 4 atomes de carbone, cycloalkyle ayant de 3 à 5 atomes de carbone, le radical phényle non substitué, un radical phényle substitué, un radical phénylalkyle non substitué ou un radical phénylalkyle substitué, groupes dans lesquels les substituants du radical phényle sont un ou deux substituants choisis parmi des atomes d'halogène, des groupes alkyle ayant de 1 à 4 atomes de carbone, alcoxy ayant de 1 à 4 atomes de carbone, nitro ou trifluorométhyle et dans lesquels le fragment alkyle du groupe phénylalkyle substitué ou non substitué correspondant comporte de 1 à 4 atomes de carbone,

$X^{10}$ est un atome de fluor, de chlore ou de brome,

m est 1 − 4 et

n est 0 − 4;

$R^2$ est un atome d'hydrogène ou un groupe alkyle ayant de 1 à 4 atomes de carbone, le radical phényle non substitué, un radical phényle substitué comportant un ou deux substituants choisis parmi des atomes d'halogène et des groupes alkyle ayant de 1 à 4 atomes de carbone, alcoxy ayant de 1 à 4 atomes de carbone, alkylthio ayant de 1 à 4 atomes de carbone, un radical alkyle substitué par le groupe carboxy, ayant de 1 à 4 atomes de carbone dans le fragment alkyle, le radical carboxy, nitro, trifluorométhyle et hydroxy,

ou $R^2$ est un groupe de formules suivantes

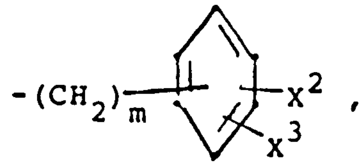

$-(CH_2)_mSCH_3$, $-(CH_2)_mSOCH_3$, $-(CH_2)_mSO_2CH_3$ ou $-(CH_2)_mCOOR^6$,

dans lesquelles

| | |
|---|---|
| $R^6$ et m | sont tels que définis à propos du radical $R^1$, |
| $X^2$ et $X^3$ | sont indépendamment un atome d'hydrogène ou d'halogène ou un groupe hydroxy, nitro, alkyle ayant de 1 à 4 atomes de carbone, alkylthio ayant de 1 à 4 atomes de carbone ou alcoxy ayant de 1 à 4 atomes de carbone, |
| $R^3$ | est un groupe de formules $-(CH_2)_nR^7$, |

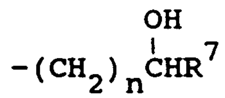

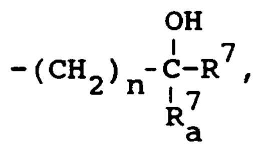 , 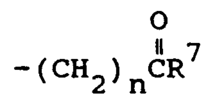 ,

$-(CH_2)_nNR^{18}(CH_2)_qR^7$,

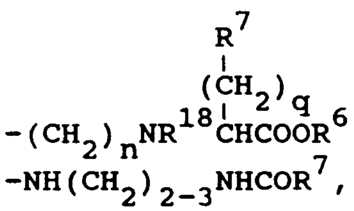 , 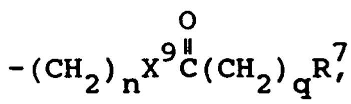 , $-NH(CH_2)_{2-3}NHR^7$,
$-NH(CH_2)_{2-3}NHCOR^7$,

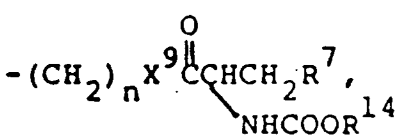 ,

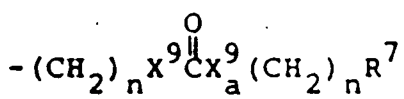 , 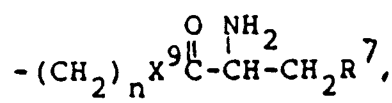 ,

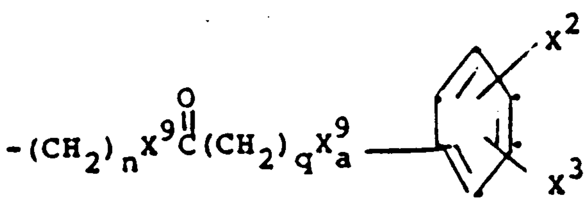

ou
$-(CH)_2)_nNR^{18}SO_2(CH_2)_qR^7$
dans lesquelles

| | |
|---|---|
| $R^7$ et $R^7_a$ | sont indépendamment un radical phényle non substitué ou un radical phényle substitué portant un ou deux substituants choisis parmi des atomes d'halogène et des groupes nitro, hydroxy, alkyle ayant de 1 à 4 atomes de carbone, des groupes de formule $CF^3$, CN, $SCF_3$, C≡CH, $CH_2SCF_3$, |

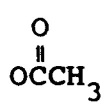 ,

$OCHF_2$, SH, SPh, $PO_3H$ ou $-NR^4R^5$, alcoxy ayant de 1 à 4 atomes de carbone ou alkylthio ayant de 1 à 4 atomes de carbone, ou $R^7$ et $R^7_a$ sont des groupes $\alpha$-naphtyle ou $\beta$-naphtyle, ou un groupe de formules

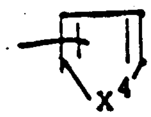 , 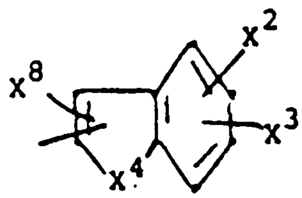 , 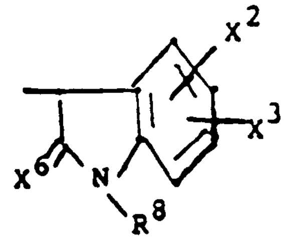

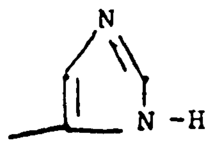, 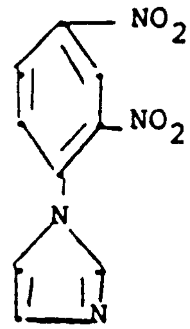 ,

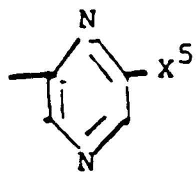 , 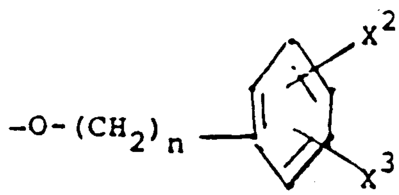 ,

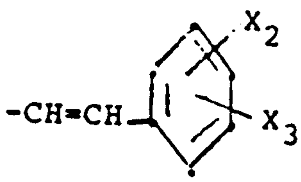 , 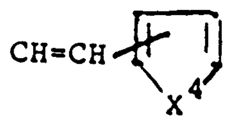

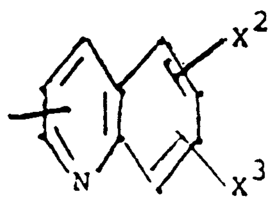 , 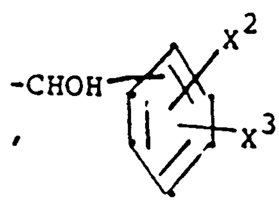 , ou

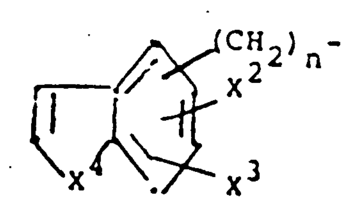

dans lesquelles

$R^8$ est un atome d'hydrogène ou un groupe alkyle ayant de 1 à 4 atomes de carbone, cycloalkyle ayant de 3 à 5 atomes de carbone, qui est lié soit directement, soit par une chaîne alkylène comprenant de 1 à 4 groupes $CH_2$, ou $R^8$ est un groupe de formules

$-(CH_2)_mCONH_2$, $-(CH_2)_mCOOR^6$, $-(CH_2)_mNR^4R^5$,

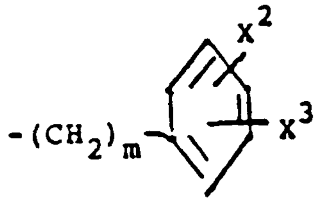,

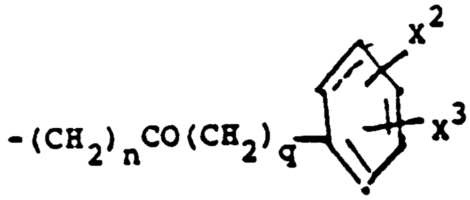,

ou

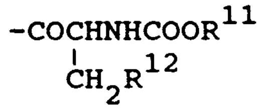,

dans lesquelles

$X^2$ et $X^3$ sont tels que définis à propos du radical $R^2$,

$X^4$ est S, O, $CH_2$ ou $NR^8$, $R^8$ étant tel que défini plus haut,

$X^5$ est un atome d'hydrogène ou d'halogène, le groupe nitro ou un groupe de formules $CF_3$, CN, $-COOR^6$, $R^6$ étant tel que défini plus haut,

$X^5$ est O ou HH,

$X^5$ est un atome d'hydrogène ou un groupe alkyle ayant de 1 à 4 atomes de carbone,

$X^5$ et $X^9_a$ sont indépendamment des groupes de formule $-O-$ ou $-NR^{18}$,
$R^{18}$ étant un atome d'hydrogène ou un groupe alkyle ayant de 1 à 4 atomes de carbone, formyle, acétyle, propionyle ou butyryle,

$R^{11}$ et $R^{12}$ sont indépendamment un groupe alkyle ayant de 1 à 4 atomes de carbone ou cycloalkyle ayant de 3 à 5 atomes de carbone,

$R^{14}$ est un groupe alkyle ayant de 1 à 4 atomes de carbone ou phénylalkyle dans lequel le groupe alkyle a de 1 à 4 atomes de carbone;

$R^9$ et $R^{10}$ sont indépendamment un atome d'hydrogène ou le groupe hydroxy ou méthyle;

$R^{13}$ est un atome d'hydrogène ou un groupe alkyle ayant de 1 à 4 atomes de carbone, cycloalkyle ayant de 3 à 5 atomes de carbone, $-O-$, le groupe formyle, acétyle, propionyle ou butyryle;

$X^1$ est un atome d'hydrogène ou d'halogène ou un groupe alkyle ayant de 1 à 4 atomes de carbone, alkylthio ayant de 1 à 4 atomes de carbone, alcoxy ayant de 1 à 4 atomes de carbone, ou un groupe de formules $-NO_2$, $CF_3$, CN, OH, $-(CH_2)-_nCOOR^6$ ou $-NR^4R^5$, dans lesquelles
$R^4$, $R^5$, $R^6$ et n sont tels que définis à propos de $R^1$;

r est 1 ou 2;

$X^7$ est O, S, HH ou $NR^{15}$;

$R^{15}$ étant un atome d'hydrogène ou un groupe alkyle ayant de 1 à 4 atomes de carbone, ou un groupe de formule

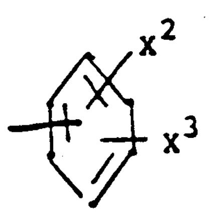,

| | |
|---|---|
| | ou $-NR^{16}R^{17}$, |
| $R^{16}$ et $R^{17}$ | étant indépendamment un atome d'hydrogène ou un groupe de formule |

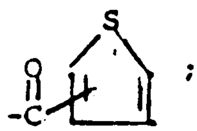

| | |
|---|---|
| | étant toutefois entendu que |
| $X^7$ | ne peut être $NR^{15}$ que lorsque $R^1$ n'est pas H; |
| q | va de 0 à 4; |
| $---$ | représente une simple liaison ou une double liaison; |
| p | est O lorsque sa liaison $---$ adjacente est une double liaison et est 1 lorsque sa liaison $---$ adjacente est une simple liaison, sauf lorsque $R^{13}$ est un atome d'oxygène et p est 1 et $---$ est une double liaison; et étant entendu que si |
| $R^3$ | est un groupe de formule $-(CH_2)_nNH(CH_2)_qR^7$, dans lequel q est 0 ou 1 ou est un groupe de formule |

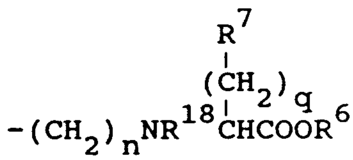

dans laquelle q est 0,

$R^7$ doit avoir une autre signification que celle d'un groupe de formule

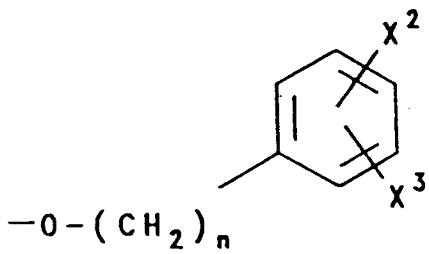

pour la fabrication d'un médicament pour utilisation en tant qu'antagoniste de la cholécystokinine dans le tissu gastrointestinal, et en tant qu'antagoniste de la gastrine, par liaison au récepteur de cholécystokinine ou au récepteur de gastrine correspondant.

**2.** Utilisation selon la revendication 1, caractérisée en ce que, pour la fabrication d'un médicament, on utilise, en tant que composant pharmaceutiquement actif, un composé de formule I ou un sel pharmaceutiquement acceptable ou un sel d'ammonium quaternaire pharmaceutiquement acceptable dudit composé de formule I, composés de formule I dans lesquels

| | |
|---|---|
| $R^1$ | est un atome d'hydrogène ou un groupe alkyle ayant de 1 à 5 atomes de carbone ou un groupe de formule $-(CH_2)_mCOOR^6$, dans laquelle $R^6$ et m sont tels que définis dans la revendication 1; |
| $R^2$ | est un groupe de formule $-(CH_2)_mCOOR^6$ dans laquelle $R^6$ et m sont tels que définis plus haut, ou $R^2$ est le radical phényle non substitué ou un radical phényle substitué comportant un ou deux substituants choisis parmi des atomes d'halogène et des groupes alkyle ayant de 1 à 4 atomes de carbone, alcoxy ayant de 1 à 4 atomes de carbone, alkyle substitué par le radical carboxy, ayant de 1 à 4 atomes de carbone dans le fragment alkyle, carboxy, nitro, trifluorométhyle et hydroxy; |
| $R^3$ | est un groupe de formules $-(CH_2)_nR^7$, |

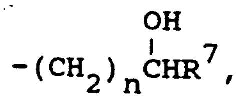

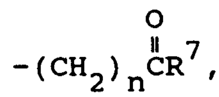

$-(CH_2)_nNH(CH_2)_qR^7$,

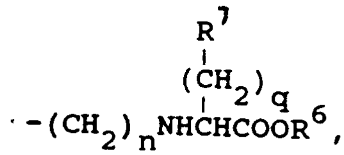

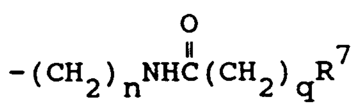

$-NH(CH_2)_{2-3}NHR^7$,

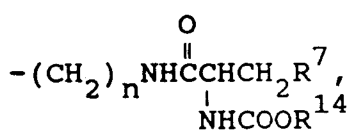

$-NH(CH_2)_{2-3}NHCOR^7$ ou

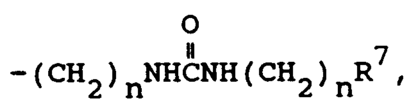

dans lesquelles

$R^7$ est le radical phényle non substitué ou un radical phényle substitué comportant un ou deux substituants choisis parmi des atomes d'halogène et des groupes nitro, hydroxy, alkyle ayant de 1 à 4 atomes de carbone, alcoxy ayant de 1 à 4 atomes de carbone, alkylthio ayant de 1 à 4 atomes de carbone, trifluorométhyle ou des groupes de formules CN, $-C{\equiv}CH$, $SCF_3$,

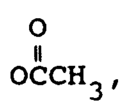

$OCHF_2$, SPh ou $-NR^4R^5$

dans lesquelles

$R^4$ et $R^5$ sont indépendamment un atome d'hydrogène ou un groupe alkyle ayant de 1 à 4 atomes de carbone, ou

$R^7$ est un groupe $\alpha$-naphtyle ou $\beta$-naphtyle ou un groupe de formules

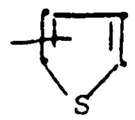 ,

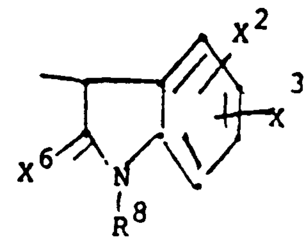 ,

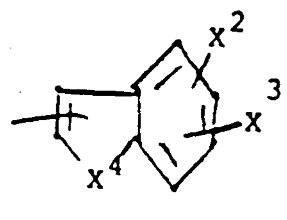 ,

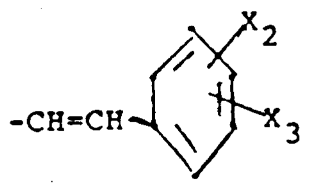 , ou 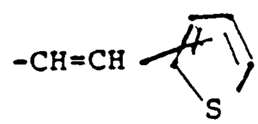

dans lesquelles

$R^8$ est un atome d'hydrogène ou un groupe alkyle ayant de 1 à 4 atomes de carbone, ou un groupe de formule

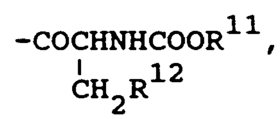

$R^{11}$ et $R^{12}$ étant indépendamment un groupe alkyle ayant de 1 à 4 atomes de carbone,

$X^2$ et $X^3$ sont indépendamment un atome d'hydrogène ou d'halogène ou un groupe hydroxy, nitro, alkylthio ayant de 1 à 4 atomes de carbone, alkyle ayant de 1 à 4 atomes de carbone ou alcoxy ayant de 1 à 4 atomes de carbone,

$X^4$ est S, O ou $NR^8$, $R^8$ étant tel que défini plus haut,

$X^6$ est O ou HH,

$R^{14}$ est un groupe alkyle ayant de 1 à 4 atomes de carbone, et

q est 0 – 4;

$R^9$ et $R^{10}$ sont indépendamment un atome d'hydrogène ou le groupe hydroxy ou méthyle;

$R^{13}$ est un atome d'hydrogène ou un groupe alkyle ayant de 1 à 4 atomes de carbone, – O, formyle, acétyle, propionyle ou butyryle;

$X^1$ est un atome d'hydrogène ou d'halogène ou un groupe alkyle ayant de 1 à 4 atomes de carbone, alkylthio ayant de 1 à 4 atomes de carbone, alcoxy ayant de 1 à 4 atomes de carbone ou un groupe de formules $-NO_2$, $CF_3$, CN, OH, $-(CH_2)_nCOOR^6$ ou $-NR^4R^5$,

$R^6$ étant tel que défini à propos de $R^1$, et $R^4$ et $R^5$ étant tels que définis à propos de $R^3$;

$X^7$ est O;

m va de 1 à 4;

n va de 0 à 4;

- - - représente une simple liaison ou une double liaison,

p est 0 lorsque la liaison - - - adjacente est une double liaison et est 1 lorsque sa liaison - - - adjacente est une simple liaison, à l'exception du cas où $R^{13}$ est un atome d'oxygène et p est 1 et - - - est une double liaison; et

r est 1 ou 2.

3. Utilisation selon la revendication 2, caractérisée en ce que, pour la fabrication d'un médicament, on utilise, en tant que composant pharmaceutiquement actif, un composé de formule I ou un sel pharmaceutiquement acceptable ou sel d'ammonium quaternaire pharmaceutiquement acceptable dudit composé de formule I, dans lequel

$R^1$ est un atome d'hydrogène ou le groupe méthyle, éthyle, carboxyméthyle ou éthylcarboxyméthyle;

$R^2$ est un groupe de formule $-(CH_2)_{1-2}COOR^6$,

$R^6$ étant un atome d'hydrogène ou un groupe alkyle ayant de 1 à 4 atomes de carbone, ou

$R^2$ est le radical phényle non substitué ou un radical phényle substitué comprenant un ou deux substituants qui sont des atomes d'halogène ou le groupe carboxy;

$R^3$ est un groupe de formules $-(CH_2)_nR^7$

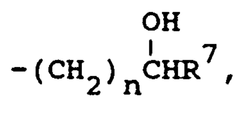

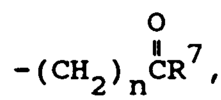

$-(CH_2)_nNH(CH_2)_qR^7$,

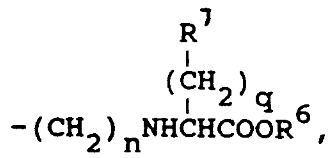

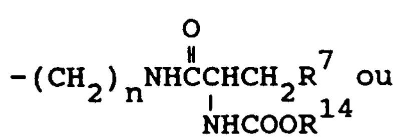 ou 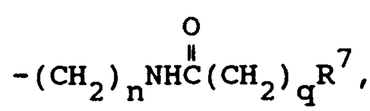

$R^7$ étant un groupe $\alpha$-naphtyle, $\beta$-naphtyle ou un groupe phényle substitué comportant un ou deux substituants choisis parmi des atomes d'halogène et des groupes trifluorométhyle et alkyle ayant de 1 à 4 atomes de carbone, ou

$R^7$ est un groupe de formules

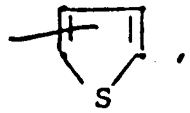

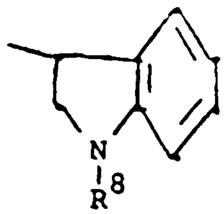,

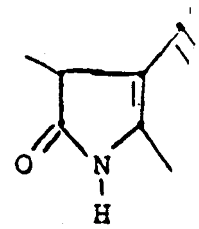

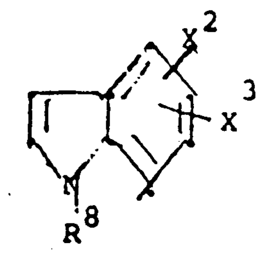,

ou

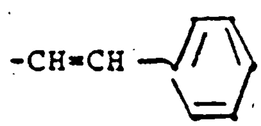

dans lesquelles

$R^8$ est un atome d'hydrogène ou le groupe méthyle ou éthyle,

$X^2$ et $X^3$ sont indépendamment des atomes d'hydrogène ou d'halogène ou le groupe hydroxy ou nitro,

$R^{14}$ est le groupe tert-butyle,

n va de 0 à 4,

q va de 0 à 4 et

$R^6$ est tel que défini à propos du radical $R^2$;

$R^9$ et $R^{10}$ sont indépendamment un atome d'hydrogène ou le groupe hydroxy ou méthyle;

$R^{13}$ est un atome d'hydrogène ou le groupe méthyle ou formyle;

$X^1$ est un atome d'hydrogène ou d'halogène ou un groupe de formule $-NO_2$, $CF_3$, CN ou OH;

$X^7$ est O;

– – – représente une simple liaison ou une double liaison;

p est O lorsque la liaison – – – adjacente est une double liaison et est 1 lorsque sa liaison – – – adjacente est une simple liaison; et

r est 1 ou 2.

**4.** Utilisation selon la revendication 3, caractérisée en ce que, pour la fabrication d'un médicament, on utilise un composé de formule I ou un sel pharmaceutiquement acceptable ou un sel d'ammonium quaternaire pharmaceutiquement acceptable dudit composé de formule I, composés de formule I dans lesquels

$R^1$ est un atome d'hydrogène ou le groupe méthyle ou carboxyméthyle;

$R^2$ est le groupe phényle, o-fluorophényle, p-fluorophényle, o-chlorophényle, p-chlorophényle, o-carboxyphényle, 2,4-dichlorophényle, 2,6-difluorophényle, $-CH_2COOEt$, $-CH_2COO-t-Bu$, $-CH_2CH_2COOEt$ ou $-CH_2CH_2COO-t-Bu$;

$R^3$ est $-(CH_2)_{1-2}R^7$,

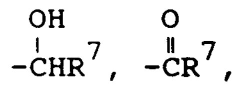

$-(CH_2)_{0-1}NH(CH_2)_{1-2}R^7$,

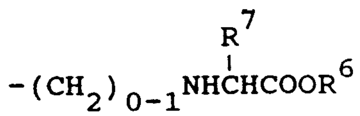, 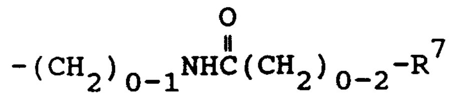

ou

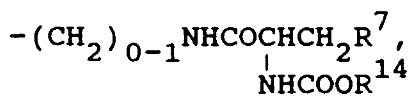,

et la configuration stéréochimique s'apparente à celle du D-tryptophane;

$R^7$ est un groupe $\alpha$-naphtyle, $\beta$-naphtyle, monohalogénophényle ou dihalogénophényle, ou un groupe de formules

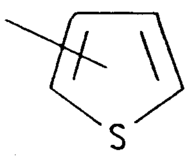,

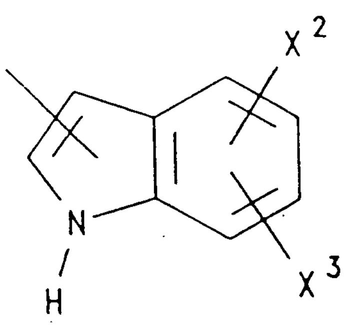,

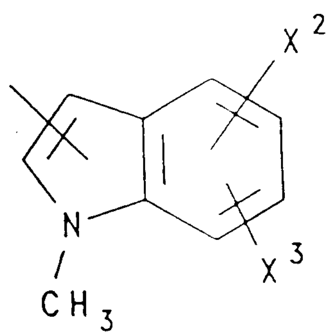

dans lesquelles

$X^2$ et $X^3$ sont indépendamment un atome d'hydrogène, de fluor ou de chlore ou le groupe hydroxy,

$R^6$ est un atome d'hydrogène ou le groupe méthyle ou éthyle,

$R^{14}$ est le groupe tert-butyle;

$R^9$ et $R^{10}$ sont indépendamment un atome d'hydrogène ou le groupe hydroxy;

$R^{13}$ est un atome d'hydrogène;

$X^1$ est un atome d'hydrogène, 7-chloro, 7-fluoro ou 7-nitro;

$X^7$ est un atome d'oxygène;

- - - représente une simple liaison ou une double liaison;

p est 0 lorsque sa liaison - - - adjacente est une double liaison et est 1 lorsque sa liaison - - - adjacente est une simple liaison; et

r est 1.

**5.** Utilisation selon la revendication 1, caractérisée en ce que le composé de formule I est l'un des suivants:

1,3 – dihydro – 5 – (2 – fluorophényl) – 3(R) – (3' – indolyl)méthyl – 2H – 1,4 – benzodiazépine – 2 – one,

1,3 – dihydro – 5 – (2 – fluorophényl) – 3(R) – [3' – (1' – méthylindolyl)méthyl] – 1 – méthyl – 2H – 1,4 – benzodiazépine – 2 – one,

1,3 – dihydro – 5 – (2 – fluorophényl) – 3(R) – (3' – indolyl)méthyl – 1 – méthyl – 2H – 1,4 – benzodiazépine – 2 – one,

7 – chloro – 1,3 – dihydro – 3(R) – (3' – indolyl)méthyl – 5 – phényl – 2H – 1,4 – benzodiazépine – 2 – one,

1,3 – dihydro – 3(R) – (3' – indolyl)méthyl – 5 – phényl – 2H – 1,4 – benzodiazépine – 2 – one,

1,3 – dihydro – 5 – (2 – fluorophényl) – 3(R) – (3' – α – indolényl)méthyl – 2H – 1,4 – benzodiazépine – 2 – one,

1,3 – dihydro – 5 – (2 – fluorophényl) – 3(R) – (3' – β – indolényl)méthyl – 2H – 1,4 – benzodiazépine – 2 – one,

1,3 – dihydro – 5 – (2 – fluorophényl) – 3(R) – (3' – indolyl)méthyl – 2H – 1,4 – benzodiazépine – 2 – thione,

1,3 – dihydro – 5 – (2 – fluorophényl) – 3(R) – (3' – indolyl)méthyl – 2H – 1,4 – benzodiazépine,

7 – chloro – 1,3 – dihydro – 3(R) – benzyl – 5 – phényl – 2H – 1,4 – benzodiazépine – 2 – one,

3(R) – benzyloxyméthyl – 7 – chloro – 1,3 – dihydro – 5 – phényl – 2H – 1,4 – benzodiazépine – 2 – one,

7 – chloro – 1,3 – dihydro – 3(RS) – (1 – naphtyl)méthyl – 5 – phényl – 2H – 1,4 – benzodiazépine – 2 – one,

7 – chloro – 1,3 – dihydro – 3(RS) – (2 – naphtyl)méthyl – 5 – phényl – 2H – 1,4 – benzodiazépine – 2 – one,

1,3 – dihydro – 5 – (2 – fluorophényl) – 3(RS) – (2 – thiényl)méthyl – 2H – 1,4 – benzodiazépine – 2 – one,

1,3 – dihydro – 5 – (2 – fluorophényl) – 3(RS) – (3 – thiényl) – 2H – 1,4 – benzodiazépine – 2 – one,

1,3 – dihydro – 5 – (2 – fluorophényl) – 3(R) – [3' – β – (1' – t – Boc – L – leucyl) – indolényl]méthyl – 2H – 1,4 – benzodiazépine – 2 – one,

1,3 – dihydro – 5 – (2 – fluorophényl) – 3(R) – [3' – β – (1' – t – Boc – D – leucyl)indolényl]méthyl – 2H – 1,4 – benzodiazépine – 2 – one,

1,3 – dihydro – 5 – (2 – fluorophényl) – 3(R) – [3' – α – (1' – t – Boc – L – leucyl)indolényl]méthyl – 2H – 1,4 – benzodiazépine – 2 – one,

1,3 – dihydro – 5 – (2 – fluorophényl) – 3(R) – [3' – α – (1' – t – Boc – D – leucyl)indolényl]méthyl – 2H – 1,4 – benzodiazépine – 2 – one,

7 – chloro – 1,3,4,5 – tétrahydro – 3(R) – (3' – indolyl)méthyl – 5 – phényl – 2H – 1,4 – benzodiazépine – 2 – one,

7 – chloro – 1,3,4,5 – tétrahydro – 3(S) – (3' – indolyl)méthyl – 5 – phényl – 2H – 1,4 – benzodiazépine – 2 – one,

4 – (p – chlorobenzoyl) – 5 – (2 – fluorophényl) – 3(R) – [3' – (1' – méthylindolyl)méthyl] – 1 – méthyl – 1,3,4,5 – tétrahydro – 2H – 1,4 – benzodiazépine – 2 – one,

4 – acétyl – 5 – (2 – fluorophényl) – 3(R) – [3' – (1' – méthylindolyl)méthyl] – 1 – méthyl – 1,3,4,5 – tétrahydro – 2H – 1,4 – benzodiazépine – 2 – one,

7 – chloro – 5 – (2 – chlorophényl) – 1,3 – dihydro – 3(R) – (3' – indolyl)méthyl – 2H – 1,4 – benzodiazépine – 2 – one,

1,3 – dihydro – 3(R) – (3' – indolyl)méthyl – 5 – méthyl – 2H – 1,4 – benzodiazépine – 2 – one,

1 – benzyl – 7 – chloro – 1,3 – dihydro – 3(R) – (3' – indolyl)méthyl – 5 – phényl – 2H – 1,4 – benzodiazépine – 2 – one,

7 – chloro – 1,3 – dihydro – 3(R) – (3' – indolyl)méthyl – 1 – méthyl – 5 – phényl – 2H – 1,4 – benzodiazépine – 2 – one,

1,3 – dihydro – 5 – (2 – fluorophényl) – 3(S) – (3' – indolyl)méthyl – 2H – 1,4 – benzodiazépine – 2 – one,

1 – benzyl – 7 – chloro – 1,3 – dihydro – 3(S) – (3' – indolyl)méthyl – 5 – phényl – 2H – 1,4 – benzodiazépine – 2 – one,

7 – chloro – 1,3 – dihydro – 3(R) – (3' – indolyl)méthyl – 5 – phényl – 2H – 1,4 – benzodiazépine – 2 – thione,

1,3 – dihydro – 5 – (2 – fluorophényl) – 3(R) – (3' – indolyl)méthyl – 2H – 1,4 – benzodiazépine – 2 – [N' – (3 – thiénoyl)]hydrazide,

1,3 – dihydro – 1 – éthyl – 5 – (2 – fluorophényl) – 3(R) – (3' – indolyl)méthyl – 2H – 1,4 – benzodiazépine – 2 – one,

1,3 – cyclopropylméthyl – 1,3 – dihydro – 5 – (2 – fluorophényl) – 3(R) – (3' – indolyl)méthyl – 2H – 1,4 – benzodiazépine – 2 – one,

1,3 – dihydro – 5 – (2 – fluorophényl) – 3(R) – (3' – indolyl)méthyl – 1 – pentyl – 2H – 1,4 – benzodiazépine – 2 – one,

1,3 – dihydro – 5 – (2 – fluorophényl) – 3(R) – (3' – indolyl)méthyl – 1 – (3 – méthylbutyl) – 2H – 1,4 – benzodiazépine – 2 – one,

1,3 – dihydro – 5 – (2 – fluorophényl) – 3(R) – (3' – indolyl)méthyl – 1 – (2,2,2 – trifluoroéthyl) – 2H – 1,4 – benzodiazépine – 2 – one,

1,3 – dihydro – 1 – (2 – diméthylaminoéthyl) – 5 – (2 – fluorophényl) – 3(R) – (3' – indolyl)méthyl – 2H – 1,4 – benzodiazépine – 2 – one,

1,3 – dihydro – 1 – (éthoxycarbonylméthyl) – 5 – (2 – fluorophényl) – 3(R) – (3' – indolyl)méthyl – 2H – 1,4 – benzodiazépine – 2 – one,

1 – carboxyméthyl – 1,3 – dihydro – 5 – (2 – fluorophényl) – 3(R) – (3' – indolyl)méthyl – 2H – 1,4 – benzodiazépine – 2 – one,

1,3 – dihydro – 5 – (2 – fluorophényl) – 3(R) – [3' – (1' – p – chlorobenzyloylindolyl)méthyl] – 1 – méthyl – 2H – 1,4 – benzodiazépine – 2 – one,

7 – chloro – 1,3 – dihydro – 3(R) – [3' – (1' – benzylindolyl)méthyl] – 1 – méthyl – 5 – phényl – 2H – 1,4 – benzodiazépine – 2 – one,

1,3 – dihydro – 3(RS) – [1 – hydroxy – 1 – (3' – indolyl)]méthyl – 1 – méthyl – 5 – phényl – 2H – 1,4 – benzodiazépine – 2 – one,

1,3 – dihydro – 1 – méthyl – 5 – phényl) – 3(RS) – (3 – thiénoyl) – 2H – 1,4 – benzodiazépine – 2 – one,

1,3 – dihydro – 3 RS) – [1 – hydroxy – 1 – (3 – thiényl)]méthyl – 1 – méthyl – 5 – phényl – 2H – 1,4 – benzodiazépine – 2 – one,

1,3 – dihydro – 3(RS) – [1 – hydroxy – 1 – [3 – (1 – méthylindolyl)]]méthyl – 1 – méthyl – 5 – phényl – 2H – 1,4 – benzodiazépine – 2 – one, deux stéréoisomères,

1,3 – dihydro – 3(RS) – (1 – hydroxy – 1 – phényl)méthyl – 1 – méthyl – 5 – phényl – 2H – 1,4 – benzodiazépine – 2 – one,

1,3 – dihydro – 3(RS) – [1 – hydroxy – 1 – (2 – thiényl)]méthyl – 1 – méthyl – 5 – phényl – 2H – 1,4 – benzodiazépine – 2 – one,

1,3 – dihydro – 3(RS) – hydroxy – 1 – méthyl – 5 – phényl – 3 – (3' – thiénoyl) – 2H – 1,4 – benzodiazépine – 2 – one,

1,5 – dihydro – 5(RS) – hydroxy – 1 – méthyl – 5 – phényl – 3 – (3' – thiénoyl) – 2H – 1,4 – benzodiazépine – 2 – one,

7 – chloro – 1,3 – dihydro – 3(R) – [(2',3' – dihydro – 2' – oxo – 1'H – indole – 3' – yl)méthyl] – 5 – phényl – 2H – 1,4 – benzodiazépine – 2 – one,

7 – chloro – 1,3 – dihydro – 3(R) – [(3' – (2,4 – dinitrophényl)imidazole – 5' – yl)méthyl] – 5 – phényl – 2H – 1,4 – benzodiazépine – 2 – one,

7 – chloro – 1,3 – dihydro – 3(R) – (3' – imidazole – 5' – yl)méthyl – 5 – phényl – 2H – 1,4 – benzodiazépine – 2 – one,

3(RS) – [3' – (5' – bromo – indolyl)méthyl] – 1,3 – dihydro – 5 – phényl – 2H – 1,4 – benzodiazépine – 2 – one,

5 – o – carboxyphényl – 1,3 – dihydro – 3(R) – (3' – indolyl)méthyl – 2H – 1,4 – benzodiazépine – 2 – one,

1,3 – dihydro – 3(RS) – [3' – (5' – fluoro – indolyl)méthyl] – 5 – o – fluorophényl – 2H – 1,4 – benzodiazépine – 2 – one,

1,3 – dihydro – 3(RS) – [3' – (6' – fluoro – indolyl)méthyl] – 5 – o – fluorophényl – 2H – 1,4 – benzodiazépine – 2 – one,

2 – N – [2(RS),3 – bis(Bocamino)propanoyl]amino – 2' – fluorobenzophénone,

2 – N – [2(RS),3 – diphtalylaminopropanoyl]amino – 2' – fluorobenzophénone,

1,3 – dihydro – 5 – (2' – fluorophényl) – 3(RS) – aminométhyl – 2H – 1,4 – benzodiazépine – 2 – one,

1,3 – dihydro – 5 – (2' – fluorophényl) – 3(R) – (4 – amino)butyl – 2H – 1,4 – benzodiazépine – 2 – one,

1,3 – dihydro – 5 – (2' – fluorophényl) – 3(RS) – benzyloxycarbonylaminométhyl – 2H – 1,4 – benzodiazépine – 2 – one,

1,3 – dihydro – 5 – (2' – fluorophényl) – 3(RS) – (3 – thiophènecarbonylaminométhyl – 2H – 1,4 – benzodiazépine – 2 – one,

1,3 – dihydro – 5 – (2' – fluorophényl) – 3(RS) – (2 – indole)carbonylaminométhyl – 2H – 1,4 – benzodiazépine – 2 – one,

1,3 – dihydro – 5 – (2' – fluorophényl) – 3(RS) – (6' – chloropyrazinyl)aminométhyl – 2H – 1,4 –

benzodiazépine – 2 – one,

1,3 – dihydro – 5 – (2' – fluorophényl) – 3(RS) – aminométhyl – 1 – méthyl – 2H – 1,4 – benzodiazépine – 2 – one,

3(RS) – (2 – indole – carbonylamino) – 1,3 – dihydro – 5 – phényl – 2H – 1,4 – benzodiazépine – 2 – one,

1,3 – dihydro – 3(RS) – [2 – (3 – indolyl)éthyl]amino – 5 – phényl – 2H – 1,4 – benzodiazépine – 2 – one,

3(RS) – [3 – (3 – indole)propionylamino] – 1,3 – dihydro – 5 – phényl – 2H – 1,4 – benzodiazépine – 2 – one,

3(RS) – (3 – indole – acétylamino) – 1,3 – dihydro – 5 – phényl – 2H – 1,4 – benzodiazépine – 2 – one,

3(RS) – (Boc – L – tryptophyl)amino – 1,3 – dihydro – 5 – phényl – 2H – 1,4 – benzodiazépine – 2 – one,

Z – 1,3 – dihydro – 1 – méthyl – 5 – phényl – 3 – (3 – thiénylméthylène) – 2H – 1,4 – benzodiazépine – 2 – one,

E – 1,3 – dihydro – 1 – méthyl – 5 – phényl – 3 – (3 – thiénylméthylène) – 2H – 1,4 – benzodiazépine – 2 – one,

3(RS) – (Boc – D – tryptophyl)amino – 1,3 – dihydro – 5 – phényl – 2H – 1,4 – benzodiazépine – 2 – one,

3(RS) – [4 – (3 – indole)butyrylamino] – 1,3 – dihydro – 5 – phényl – 2H – 1,4 – benzodiazépine – 2 – one,

1,3 – dihydro – 3(RS) – (benzyloxycarbonyl)aminométhyl – 5 – (2 – fluorophényl) – 2H – 1,4 – benzodiazépine,

1,3 – dihydro – 3(RS) – [3' – (thiophène)carbonyl]aminométhyl – 5 – (2 – fluorophényl) – 2H – 1,4 – benzodiazépine,

1,3 – dihydro – 3(RS) – (2 – indole – carbonyl)aminométhyl – 5 – (2 – fluorophényl) – 2H – 1,4 – benzodiazépine,

1,3 – dihydro – 3(RS) – (2 – L – hydroxy – 2 – phénylacétyl)aminométhyl – 5 – (2 – fluorophényl) – 2H – 1,4 – benzodiazépine,

1 – (2 – cyanoéthyl) – 1,3 – dihydro – 5 – (2 – fluorophényl) – 3(R) – (3' – indolyl)méthyl) – 2H – 1,4 – benzodiazépine – 2 – one,

1 – (2 – cyanoéthyl) – 1,3 – dihydro – 5 – (2 – fluorophényl) – 3(R) – [1' – (2 – cyanoéthyl) – 3' – indolyl] – méthyl – 2H – 1,4 – benzodiazépine – 2 – one

1 – (2 – carboxyéthyl) – 1,3 – dihydro – 5 – (2 – fluorophényl) – 3(R) – (3' – indolyl)] – méthyl – 2H – 1,4 – benzodiazépine – 2 – one,

1,3 – dihydro – 3(R) – 3' – indolyl)méthyl – 5 – (2 – fluorophényl) – 2H – 1,4 – benzodiazépine – 2 – one – 4 – oxyde,

1,3 – dihydro – 5 – (2 – fluorophényl) – 3(RS) – (2 – indole – carbonylamino) – 2H – 1,4 – benzodiazépine – 2 – one,

1,3 – dihydro – 5 – (2 – fluorophényl) – 3(RS) – (2 – indole – carbonylamino) – 1 – méthyl – 2H – 1,4 – benzodiazépine – 2 – one,

1,3 – dihydro – 5 – (2 – fluorophényl) – 1 – méthyl – 3(RS) – [2' – (1 – méthylindole)carbonylamino] – 2H – 1,4 – benzodiazépine – 2 – one,

1,3 – dihydro – 3(RS) – (4 – nitrophénylcarbonyl)amino – 5 – (2 – fluorophényl) – 2H – 1,4 – benzodiazépine – 2 – one,

1,3 – dihydro – 3(RS) – (2 – indole – carbonyloxy) – 5 – phényl – 2H – 1,4 – benzodiazépine – 2 – one,

1,3 – dihydro – 5 – (2 – fluorophényl) – 3(RS) – (3 – thiophènecarbonylamino) – 2H – 1,4 – benzodiazépine – 2 – one,

1,3 – dihydro – 3(RS) – (3 – indole – carbonylamino) – 5 – phényl – 2H – 1,4 – benzodiazépine – 2 – one,

1,3 – dihydro – 3(RS) – (4 – thianaphtène – acétyl)amino – 5 – (2 – fluorophényl) – 2H – 1,4 – benzodiazépine – 2 – one,

1,3 – dihydro – 3(RS) – (4 – chlorophénylcarbonyl)amino – 5 – (2 – fluorophényl) – 2H – 1,4 – benzodiazépine – 2 – one,

1,3 – dihydro – 3(RS) – (4 – méthylphénylsulfonyl)amino – 5 – (2 – fluorophényl) – 2H – 1,4 – benzodiazépine – 2 – one,

1 – carboxyméthyl – 1,3 – dihydro – 5 – (2 – fluorophényl) – 3(RS) – (2 – indole – carbonylamino) – 2H – 1,4 – benzodiazépine – 2 – one,

1,3 – dihydro – 3(RS) – (5 – fluoro – indole – 2 – carbonylamino) – 5 – (2 – fluorophényl) – 2H – 1,4 – benzodiazépine – 2 – one,

1,3 – dihydro – 3(RS) – (3' – méthylindényl – 2 – carbonyl)amino – 5 – (2 – fluorophényl) – 2H – 1,4 – benzodiazépine – 2 – one,

1,3 – dihydro – 3(RS) – (2 – quinaldyl)amino – 5 – (2 – fluorophényl) – 2H – 1,4 – benzodiazépine – 2 – one,

1,3 – dihydro – 3(RS) – (2 – L – hydroxy – 2 – phénylacétyl)amino – 5 – (2 – fluorophényl) – 2H – 1,4 – benzodiazépine – 2 – one,

1,3 – dihydro – 3(RS) – (5 – chloro – indole – 2 – carbonylamino) – 5 – (2 – fluorophényl) – 2H – 1,4 – benzodiazépine – 2 – one,

3(RS) – [N – (2 – indole – carbonyl) – N – méthylamino] – 1,3 – dihydro – 5 – phényl – 2H – 1,4 – benzodiazépine – 2 – one,

1,3 – dihydro – 3(RS) – (2 – indole – carbonylamino) – 1 – méthyl – 5 – phényl – 2H – 1,4 – benzodiazépine – 2 – one,

1,3 – dihydro – 1 – méthyl – 3(RS) – [2 – (1 – méthylindole)carbonylamino] – 5 – phényl – 2H – 1,4 – benzodiazépine – 2 – one,

1 – carboxyméthyl – 1,3 – dihydro – 3(RS) – (2 – indole – carbonylamino) – 5 – phényl) – 2H – 1,4 – benzodiazépine – 2 – one,

1,3 – dihydro – 3(RS) – (5 – bromo – indole – 2 – carbonylamino) – 5 – (2 – fluorophényl) – 2H – 1,4 – benzodiazépine – 2 – one,

3(RS) – cinnamoylamino – 1,3 – dihydro – 5 – (2 – fluorophényl) – 2H – 1,4 – benzodiazépine – 2 – one,

1,3 – dihydro – 3(RS) – (5 – hydroxy – 2 – indolylcarbonyl)amino – 5 – (2 – fluorophényl) – 2H – 1,4 – benzodiazépine – 2 – one,

1 – carboxamidométhyl – 1,3 – dihydro – 3R – (3 – indolylméthyl) – 5 – (2 – fluorophényl) – 2H – 1,4 – benzodiazépine – 2 – one,

1,3 – dihydro – 5 – (2 – fluorophényl) – 3(RS) – (2 – indolylméthylamino) – 2H – 1,4 – benzodiazépine – 2 – one,

1,3 – dihydro – 3(RS) – (phénylaminométhylcarbonyl)amino – 5 – (2 – fluorophényl) – 2H – 1,4 – benzodiazépine – 2 – one,

1,3 – dihydro – 3(RS) – (5 – méthoxyindole – 2 – carbonylamino) – 5 – (2 – fluorophényl) – 2H – 1,4 – benzodiazépine – 2 – one,

1,3 – dihydro – 3(RS) – (1 – méthylindole – 2 – carbonylamino) – 5 – (2 – fluorophényl) – 2H – 1,4 – benzodiazépine – 2 – one,

1,3 – dihydro – 1 – méthyl – 3(RS) – (4 – chlorophénylcarbonyl)amino – 5 – (2 – fluorophényl) – 2H – 1,4 – benzodiazépine – 2 – one,

1,3 – dihydro – 5 – (2 – fluorophényl) – 3(RS) – (2 – benzofurannecarbonylamino) – 2H – 1,4 – benzodiazépine – 2 – one,

1 – éthoxycarbonylméthyl – 1,3 – dihydro – 3(RS) – (4 – chlorophénylcarbonyl)amino – 5 – (2 – fluorophényl) – 2H – 1,4 – benzodiazépine – 2 – one

1,3 – dihydro – 3(RS) – (4 – chlorophénylcarbonyl)amino – 5 – phényl – 2H – 1,4 – benzodiazépine – 2 – one,

1,3 – dihydro – 1 – méthyl – 3(RS) – (4 – chlorophénylcarbonyl)amino – 5 – phényl) – 2H – 1,4 – benzodiazépine – 2 – one,

1 – carboxyméthyl – 1,3 – dihydro – 3(RS) – (4 – chlorophénylcarbonyl)amino – 5 – (2 – fluorophényl) – 2H – 1,4 – benzodiazépine – 2 – one,

3(RS) – (2(S) – tert – butoxycarbonylamino – 3 – phénylpropanoylamino) – 1,3 – dihydro – 5 – phényl – 2H – 1,4 – benzodiazépine – 2 – one,

3(RS) – (2(S) – tert – butoxycarbonylamino – 3 – phénylpropanoylamino) – 1,3 – dihydro – 1 – méthyl – 5 – phényl – 2H – 1,4 – benzodiazépine – 2 – one,

3(R) – et 3(S) – (2(S) – amino – 3 – phénylpropanoylamino) – 1,3 – dihydro – 1 – méthyl – 5 – phényl – 2H – 1,4 – benzodiazépine – 2 – one,

3(R) – et 3(S) – amino – 1,3 – dihydro – 1 – méthyl – 5 – phényl – 2H – 1,4 – benzodiazépine – 2 – one,

3(R) – et 3(S) – amino – 1,3 – dihydro – 5 – (2 – fluorophényl) – 1 – méthyl – 2H – 1,4 – benzodiazépine – 2 – one,

3(S) – ( – ) – 1,3 – dihydro – 3 – (2 – indole – carbonylamino) – 1 – méthyl – 5 – phényl – 2H – 1,4 – benzodiazépine – 2 – one,

3(S) – ( + ) – 1,3 – dihydro – 5 – (2 – fluorophényl) – 3 – (2 – indolecarbonylamino) – 1 – méthyl – 2H – 1,4 – benzodiazépine – 2 – one,

3(R) – ( – ) – 1,3 – dihydro – 5 – (2 – fluorophényl) – 3 – (2 – indolecarbonylamino) – 1 – méthyl – 2H – 1,4 – benzodiazépine – 2 – one,

3(R) – ( – ) – 1,3 – dihydro – 3 – (4 – chlorobenzoylamino) – 5 – (2 – fluorophényl) – 1 – méthyl – 2H – 1,4 – benzodiazépine – 2 – one,

3(S) – ( + ) – 1,3 – dihydro – 3 – (4 – chlorobenzoylamino) – 5 – (2 – fluorophényl) – 1 – méthyl – 2H – 1,4 – benzodiazépine – 2 – one,

3(S) – ( – ) – 1,3 – dihydro – 3 – (4 – bromobenzoylamino) – 1 – méthyl – 5 – phényl – 2H – 1,4 – benzodiazépine – 2 – one,

3(R) – ( + ) – 1,3 – dihydro – 3 – (4 – bromobenzoylamino) – 1 – méthyl – 5 – phényl – 2H – 1,4 – benzodiazépine – 2 – one,

3(R) – ( + ) – 1,3 – dihydro – 3 – (2 – indole – carbonylamino) – 1 – méthyl – 5 – phényl – 2H – 1,4 – benzodiazépine – 2 – one,

1,3 – dihydro – 3(RS) – (2 – indolinecarbonylamino) – 5 – phényl – 2H – 1,4 – benzodiazépine – 2 – one,

1,3 – dihydro – 5 – (2 – fluorophényl) – 3(RS) – (p – trifluorométhylbenzoylamino) – 2H – 1,4 – benzodiazépine – 2 – one,

1,3 – dihydro – 5 – (2 – fluorophényl) – 3(RS) – (p – méthylbenzoylamino) – 2H – 1,4 – benzodiazépine – 2 – one,

1,3 – dihydro – 5 – (2 – fluorophényl) – 3(RS) – (p – méthoxybenzoylamino) – 2H – 1,4 – benzodiazépine – 2 – one,

3(RS) – (o – chlorobenzoylamino) – 1,3 – dihydro – 5 – phényl – 2H – 1,4 – benzodiazépine – 2 – one,

3(RS) – (o – chlorobenzoylméthylamino) – 1,3 – dihydro – 1 – méthyl – 5 – phényl – 2H – 1,4 – benzodiazépine – 2 – one,

3(RS) – (o – chlorobenzoylamino) – 1,3 – dihydro – 1 – méthyl – 5 – phényl – 2H – 1,4 – benzodiazépine – 2 – one,

3(RS) – (m – chlorobenzoylamino) – 1,3 – dihydro – 5 – phényl – 2H – 1,4 – benzodiazépine – 2 – one,

3(RS) – (3,4 – dichlorobenzoylamino) – 1,3 – dihydro – 5 – phényl – 2H – 1,4 – benzodiazépine – 2 – one,

3(RS) – (p – chlorobenzoylméthylamino) – 1,3 – dihydro – 5 – (2' – fluorophényl) – 1 – méthyl – 4 – oxo – 2H – 1,4 – benzodiazépine – 2 – one,

1,3 – dihydro – 5 – phényl – 3(RS) – (4' – méthylthiobenzoylamino) – 2H – 1,4 – benzodiazépine – 2 – one,

1,3 – dihydro – 3(RS) – (4' – fluorobenzoylamino) – 5 – phényl – 2H – 1,4 – benzodiazépine – 2 – one,

1,3 – dihydro – 5 – phényl – 3(RS) – (4' – trifluorométhylbenzoylamino) – 2H – 1,4 – benzodiazépine – 2 – one,

1,3 – dihydro – 3(RS) – (4' – tert – butylbenzoylamino) – 5 – phényl – 2H – 1,4 – benzodiazépine – 2 – one,

3(RS) – (3,5 – dichlorobenzoylamino) – 1,3 – dihydro – 5 – phényl – 2H – 1,4 – benzodiazépine – 2 – one,

1,3 – dihydro – 3(RS) – (p – hydroxybenzoylamino) – 5 – phényl – 2H – 1,4 – benzodiazépine – 2 – one,

3(RS) – (4' – cyanobenzoylamino) – 1,3 – dihydro – 5 – phényl – 2H – 1,4 – benzodiazépine – 2 – one,

3(S) – ( – ) – 3 – (2 – chlorobenzoylamino) – 1,3 – dihydro – 1 – méthyl – 5 – phényl – 2H – 1,4 – benzodiazépine – 2 – one,

3(R) – ( + ) – 3 – (2 – chlorobenzoylamino) – 1,3 – dihydro – 1 – méthyl – 5 – phényl – 2H – 1,4 – benzodiazépine – 2 – one,

1,3 – dihydro – 3(RS) – (p – diméthylaminobenzoylamino) – 5 – (2 – fluorophényl) – 2H – 1,4 – benzodiazépine – 2 – one,

1,3 – dihydro – 3(RS) – (3,4 – diméthoxybenzoylamino) – 5 – (2 – fluorophényl) – 2H – 1,4 – benzodiazépine – 2 – one,

3(S) – ( + ) – 3 – (3 – bromobenzoylamino) – 1,3 – dihydro – 5 – (2 – fluorophényl – 1 – méthyl – 2H – 1,4 – benzodiazépine – 2 – one,

1,3 – dihydro – 5 – phényl – 3(RS) – (3 – trifluorométhylthiobenzoylamino) – 2H – 1,4 – benzodiazépine – 2 – one,

3(S) – ( + ) – 3 – (4 – bromobenzoylamino) – 1,3 – dihydro – 5 – (2 – fluorophényl) – 1 – méthyl – 2H – 1,4 – benzodiazépine – 2 – one,

3(S) – ( + ) – 3 – (4 – tert – butylbenzoylamino) – 1,3 – dihydro – 5 – (2 – fluorophényl) – 1 – méthyl – 2H – 1,4 – benzodiazépine – 2 – one,

1,3 – dihydro – 5 – (2 – fluorophényl) – 3(RS) – (pyrrole – 2 – carbonylamino) – 2H – 1,4 – benzodiazépine – 2 – one,

3(S) – ( + ) – 1,3 – dihydro – 5 – (2 – fluorophényl) – 3 – (4 – iodobenzoylamino) – 1 – méthyl – 2H – 1,4 – benzodiazépine – 2 – one,

1,3 – dihydro – 3(RS) – (2 – naphtoylamino) – 5 – phényl – 2H – 1,4 – benzodiazépine – 2 – one,

3(S) – ( – ) – 3 – (2 – bromobenzoylamino) – 1,3 – dihydro – 5 – (2 – fluorophényl) – 1 – méthyl – 2H – 1,4 – benzodiazépine – 2 – one,

3(S) – ( + ) – 3 – (4 – cyanobenzoylamino) – 1,3 – dihydro – 5 – (2 – fluorophényl) – 1 – méthyl – 2H –

1,4 – benzodiazépine – 2 – one,
1,3 – dihydro – 5 – phényl – 3(RS) – (4 – n – propylbenzoylamino) – 2H – 1,4 – benzodiazépine – 2 – one,
1,3 – dihydro – 5 – phényl – 3(RS) – (4 – phénylbenzoylamino) – 2H – 1,4 – benzodiazépine – 2 – one,
1,3 – dihydro – 3(RS) – (4 – n – pentylbenzoylamino) – 5 – phényl – 2H – 1,4 – benzodiazépine – 2 – one,
1,3 – dihydro – 3(RS) – (1 – naphtoylamino) – 5 – phényl – 2H – 1,4 – benzodiazépine – 2 – one,
3(S) – ( + ) – 1,3 – dihydro – 5 – (2 – fluorophényl) – 3 – (3 – iodobenzoylamino) – 1 – méthyl – 2H – 1,4 – benzodiazépine – 2 – one,
3(R) – ( – ) – 1,3 – dihydro – 5 – (2 – fluorophényl) – 3 – (3 – iodobenzoylamino) – 1 – méthyl – 2H – 1,4 – benzodiazépine – 2 – one,
3(R) – ( + ) – 1,3 – dihydro – 5 – (2 – fluorophényl) – 3 – (2 – iodobenzoylamino) – 1 – méthyl – 2H – 1,4 – benzodiazépine – 2 – one,
3(S) – ( – ) – 1,3 – dihydro – 5 – (2 – fluorophényl) – 3 – (2 – iodobenzoylamino) – 1 – méthyl – 2H – 1,4 – benzodiazépine – 2 – one,
3(R) – ( + ) – 3 – (2 – bromobenzoylamino) – 1,3 – dihydro – 5 – (2 – fluorophényl) – 1 – méthyl – 2H – 1,4 – benzodiazépine – 2 – one,
3(R) – ( + ) – 3 – (2 – chlorobenzoylamino) – 1,3 – dihydro – 5 – (2 – fluorophényl) – 1 – méthyl – 2H – 1,4 – benzodiazépine – 2 – one,
1,3 – dihydro – 5 – (2 – fluorophényl) – 3(RS) – phénylcarbonylamino) – 2H – 1,4 – benzodiazépine – 2 – one,
1,3 – dihydro – 5 – (2 – fluorophényl) – 3(RS) – (2 – chlorophényl)carbonylamino – 2H – 1,4 – benzodiazépine – 2 – one,
1,3 – dihydro – 5 – (2 – fluorophényl) – 3(RS) – benzyloxycarbonylamino – 2H – 1,4 – benzodiazépine – 2 – one,
1,3 – dihydro – 5 – (2 – fluorophényl) – 3(RS) – benzyloxycarbonylamino – 2H – 1,4 – benzodiazépine – 2 – thione,
1 – (4 – chlorophényl)carbonyl – 1,3 – dihydro – 5 – (2 – fluorophényl) – 3(RS) – (4 – chlorophényl) – carbonylamino – 2H – 1,4 – benzodiazépine – 2 – one,
1 – (4 – chlorophényl)carbonyl – 1,3 – dihydro – 5 – (2 – fluorophényl) – 3(RS) – (4 – chlorophényl) – carbonyloxy – 2H – 1,4 – benzodiazépine – 2 – one,
1,3 – dihydro – 5 – (2 – fluorophényl) – 3(RS) – (4 – chlorophényl)oxy – 2H – 1,4 – benzodiazépine – 2 – one,
1,3 – dihydro – 5 – (2 – fluorophényl) – 3(RS) – (4 – chlorophényl)carbonylamino – 2H – 1,4 – benzodiazépine – 2 – thione,
1,3 – dihydro – 5 – (2 – fluorophényl) – 3(RS) – (2 – indole)carbonylamino – 2H – 1,4 – benzodiazépine – 2 – thione ou
1,3 – dihydro – 3(RS) – (4 – chlorophényl)aminocarbonylamino – 2H – 1,4 – benzodiazépine – 2 – one.

**6.** Utilisation selon la revendication 1, caractérisée en ce que le composé de formule I est l'un des suivants:
(S) – ( – ) – 1,3 – dihydro – 3 – (4 – bromobenzoylamino) – 1 – méthyl – 5 – phényl – 2H – 1,4 – benzodiazépine – 2 – one,
3(RS) – (2 – indole – carbonylamino) – 1,3 – dihydro – 5 – phényl – 2H – 1,4 – benzodiazépine – 2 – one,
1,3 – dihydro – 3(RS) – (2 – indole – carbonylamino) – 1 – méthyl – 5 – phényl – 2H – 1,4 – benzodiazépine – 2 – one,
1,3 – dihydro – 1 – méthyl – 3(RS) – [2 – (1 – méthylindole)carbonyl amino] – 5 – phényl – 2H – 1,4 – benzodiazépine – 2 – one,
1,3 – dihydro – 1 – méthyl – 3(RS) – (4 – chlorophénylcarbonyl)amino – 5 – (2 – fluorophényl) – 2H – 1,4 – benzodiazépine – 2 – one,
1,3 – dihydro – 5 – (2 – fluorophényl) – 3(RS) – (2 – indole – carbonylamino) – 1 – méthyl – 2H – 1,4 – benzodiazépine – 2 – one,
1,3 – dihydro – 5 – (2 – fluorophényl) – 1 – méthyl – 3(RS) – [2' – (1 – méthylindole)carbonylamino] – 2H – 1,4 – benzodiazépine – 2 – one,
3(R) – ( – ) – 1,3 – dihydro – 3 – (4 – chlorobenzoylamino) – 5 – (2 – fluorophényl) – 1 – méthyl – 2H – 1,4 – benzodiazépine – 2 – one,
3(S) – ( + ) – 1,3 – dihydro – 3 – (4 – chlorobenzoylamino) – 5 – (2 – fluorophényl) – 1 – méthyl – 2H – 1,4 – benzodiazépine – 2 – one,

3(R) – ( + ) – 1,3 – dihydro – 3 – (4 – bromobenzoylamino) – 1 – méthyl – 5 – phényl – 2H – 1,4 – benzodiazépine – 2 – one,

3(R) – ( + ) – 1,3 – dihydro – 3 – (2 – indole – carbonylamino) – 1 – méthyl – 5 – phényl – 2H – 1,4 – benzodiazépine – 2 – one,

1,3 – dihydro – 5 – (2 – fluorophényl) – 3(RS) – (2 – indole – carbonylamino) – 2H – 1,4 – benzodiazépine – 2 – one,

1,3 – dihydro – 3(RS) – (2 – indole – carbonyloxy) – 5 – phényl – 2H – 1,4 – benzodiazépine – 2 – one,

1,3 – dihydro – 3(RS) – (4 – chlorophénylcarbonyl)amino – 5 – (2 – fluorophényl) – 2H – 1,4 – benzodiazépine – 2 – one,

1 – carboxyméthyl – 1,3 – dihydro – 5 – (2 – fluorophényl) – 3(RS) – (2 – indole – carbonylamino) – 2H – 1,4 – benzodiazépine – 2 – one,

1,3 – dihydro – 3(RS) – (5 – fluoro – indole – 2 – carbonylamino) – 5 – (2 – fluorophényl) – 2H – 1,4 – benzodiazépine – 2 – one,

3(RS) – cinnamoylamino – 1,3 – dihydro – 5 – (2 – fluorophényl) – 2H – 1,4 – benzodiazépine – 2 – one,

1,3 – dihydro – 5 – (2 – fluorophényl) – 3(RS) – (2 – benzofurannecarbonylamino) – 2H – 1,4 – benzodiazépine – 2 – one,

1,3 – dihydro – 1 – méthyl – 3(RS) – (4 – chlorophénylcarbonyl)amino – 5 – phényl – 2H – 1,4 – benzodiazépine – 2 – one,

1 – carboxyméthyl – 1,3 – dihydro – 3(RS) – (4 – chlorophénylcarbonyl)amino – 5 – (2 – fluorophényl) – 2H – 1,4 – benzodiazépine – 2 – one,

1,3 – dihydro – 5 – (2 – fluorophényl) – 3(RS) – (p – trifluorométhylbenzoylamino) – 2H – 1,4 – benzodiazépine – 2 – one,

3(S) – ( + ) – 3 – (3 – bromobenzoylamino) – 1,3 – dihydro – 5 – (2 – fluorophényl) – 1 – méthyl – 2H – 1,4 – benzodiazépine – 2 – one,

3(S) – ( + ) – 3 – (4 – bromobenzoylamino) – 1,3 – dihydro – 5 – (2 – fluorophényl) – 1 – méthyl – 2H – 1,4 – benzodiazépine – 2 – one,

3(S) – ( + ) – 1,3 – dihydro – 5 – (2 – fluorophényl) – 3 – (4 – iodobenzoylamino) – 1 – méthyl – 2H – 1,4 – benzodiazépine – 2 – one,

1,3 – dihydro – 3(RS) – 2 – (naphtoylamino) – 5 – phényl – 2H – 1,4 – benzodiazépine – 2 – one,

3(S) – ( + ) – 1,3 – dihydro – 5 – (2 – fluorophényl) – 3 – (3 – iodobenzoylamino) – 1 – méthyl – 2H – 1,4 – benzodiazépine – 2 – one,

3(R) – ( – ) – 1,3 – dihydro – 5 – (2 – fluorophényl) – 3 – (3 – iodobenzoylamino) – 1 – méthyl – 2H – 1,4 – benzodiazépine – 2 – one ou

3(R) – ( – ) – 1,3 – dihydro – 5 – (2 – fluorophényl) – 3 – (2 – indolecarbonylamino) – 1 – méthyl – 2H – 1,4 – benzodiazépine – 2 – one.

**7.** Utilisation selon la revendication 1, caractérisée en ce que le composé de formule I est l'un des suivants:

1 – carboxyméthyl – 1,3 – dihydro – 3(RS) – (2 – indole – carbonylamino) – 5 – phényl – 2H – 1,4 – benzodiazépine – 2 – one,

(S) – ( – ) – 1,3 – dihydro – 3 – (2 – indole – carbonylamino) – 1 – méthyl – 5 – phényl – 2H – 1,4 – benzodiazépine – 2 – one,

(S) – ( + ) – 1,3 – dihydro – 5 – (2 – fluorophényl) – 3 – (2 – indole – carbonylamino) – 1 – méthyl – 2H – 1,4 – benzodiazépine – 2 – one ou

(S) – ( + ) – 1,3 – dihydro – 3 – (4 – chlorobenzoylamino) – 5 – (2 – fluorophényl) – 1 – méthyl – 2H – 1,4 – benzodiazépine – 2 – one.

**8.** Dérivés de type benzodiazépine correspondant à la formule Ib suivante

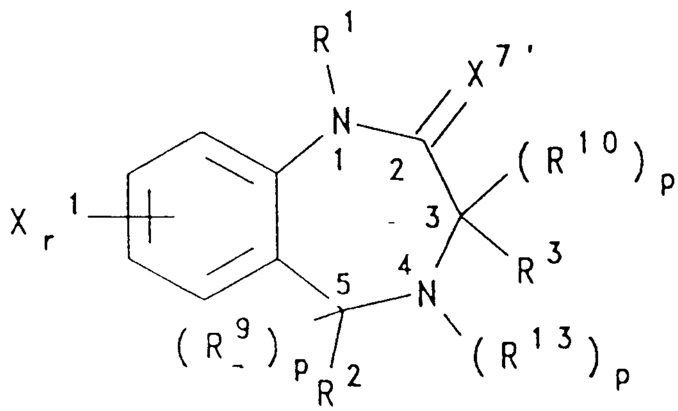

Ib

dans laquelle

$X^{7'}$ est S, HH ou $NR^{15}$ et

les autres substituants et symboles ont les mêmes significations que dans les composés de formule I définie dans la revendication 1, étant entendu que $X^{7'}$ ne peut être $NR^{15}$ que lorsque $R^1$ n'est pas H; ou sels ou sels d'ammonium quaternaire de ceux-ci.

**9.** Dérivés de type benzodiazépine correspondant à la formule Ic suivante

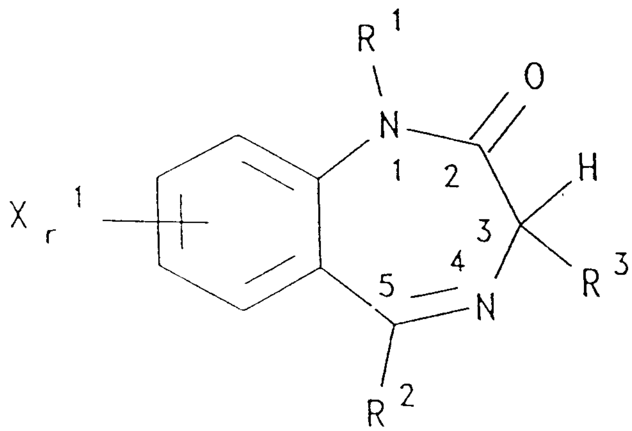

Ic

ou sels ou sels d'ammonium quaternaire des composés de formule Ic, composés de formule Ic dans lesquels

$R^1$ est un groupe alkyle ayant de 1 à 5 atomes de carbone ou un atome d'hydrogène;

$R^2$ est le radical phényle non substitué ou un radical phényle non substitué comportant 1 ou 2 substituants choisi(s) parmi des atomes d'halogène et des groupes alkyle ayant de 1 à 4 atomes de carbone, alcoxy ayant de 1 à 4 atomes de carbone, alkyle substitué par le groupe carboxy, ayant de 1 à 4 atomes de carbone dans le fragment alkyle, carboxy, nitro, trifluorométhyle et hydroxy;

$R^3$ est un groupe de formule

$$-(CH_2)_nNH\overset{O}{\overset{\|}{C}}(CH_2)_qR^7$$

ou

$$-(CH_2)_nNH\overset{\overset{\displaystyle O}{\|}}{C}NH(CH_2)_nR^7,$$

formules dans lesquelles

$R^7$ est le radical phényle non substitué ou un radical phényle substitué comportant un ou deux substituants choisi(s) parmi des atomes d'halogène et des groupes nitro, hydroxy, alkyle ayant de 1 à 4 atomes de carbone, alcoxy ayant de 1 à 4 atomes de carbone, alkylthio ayant de 1 à 4 atomes de carbone, trifluorométhyle ou des groupes de formules CN, $-C\equiv CH$, $SCF_3$,

$$O\overset{\overset{\displaystyle O}{\|}}{C}CH_3,$$

$OCHF_2$, SPh ou $-NR^4R^5$

dans lesquelles

$R^4$ et $R^5$ sont indépendamment un atome d'hydrogène ou un groupe alkyle ayant de 1 à 4 atomes de carbone, ou

$R^7$ est un groupe de formule

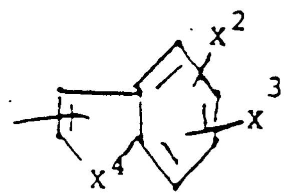

,

dans laquelle

$X^2$ et $X^3$ sont indépendamment un atome d'hydrogène ou d'halogène ou un groupe hydroxy, nitro, alkylthio ayant de 1 à 4 atomes de carbone, alkyle ayant de 1 à 4 atomes de carbone ou alcoxy ayant de 1 à 4 atomes de carbone,

$X^4$ est NH,

q est 0 – 4, de préférence 0 et

n est 0 – 4, de préférence 0.

**10.** Dérivé de type benzodiazépine selon la revendication 9, dans lequel

$R^1$ est le groupe méthyle,

$R^2$ est le groupe phényle non substitué,

$R^3$ est tel que défini dans la revendication 9, q et n étant 0 et

$R^7$ est tel que défini dans la revendication 9, $X^2$ et $X^3$ dans le groupe indolyle étant toutefois des atomes d'hydrogène.

**11.** Composé qui est la 3(S) – ( – ) – 1,3 – dihydro – 3 – (2 – indole – carbonylamino) – 1 – méthyl – 5 – phényl – 2H – 1,4 – benzodiazépine – 2 – one.

**Revendications pour l'Etat contractant suivant : AT**

**1.** Utilisation de composés de formule I

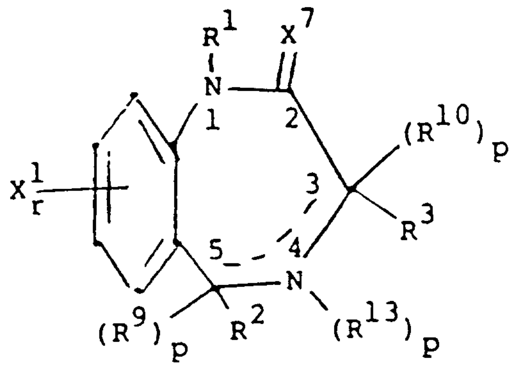

I

ou de sels pharmaceutiquement acceptables ou de sels d'ammonium quaternaire pharmaceutiquement acceptables desdits composés de formule I, composés de formule I dans lesquels

$R^1$ est un atome d'hydrogène ou un groupe alkyle ayant de 1 à 5 atomes de carbone, alcényle ayant jusqu'à 5 atomes de carbone, alcynyle ayant jusqu'à 5 atomes de carbone, ou un groupe de formules $-(CH_2)_mCOOR^6$, $-(CH_2)_n-$cycloalkyle($C_3-C_5$), $-(CH_2)_mNR^4R^5$, $-(CH_2)_mCONR^4R^5$, $-(CH_2)_mCN$ ou $-(CH_2)_nCX_3^{10}$, formules dans lesquelles

$R^4$ et $R^5$ sont indépendamment un atome d'hydrogène ou un groupe alkyle ayant de 1 à 4 atomes de carbone ou cycloalkyle ayant de 3 à 5 atomes de carbone,

$R^6$ est un atome d'hydrogène ou un groupe alkyle ayant de 1 à 4 atomes de carbone, cycloalkyle ayant de 3 à 5 atomes de carbone, le radical phényle non substitué, un radical phényle substitué, un radical phénylalkyle non substitué ou un radical phénylalkyle substitué, groupes dans lesquels les substituants du radical phényle sont un ou deux substituants choisis parmi des atomes d'halogène, des groupes alkyle ayant de 1 à 4 atomes de carbone, alcoxy ayant de 1 à 4 atomes de carbone, nitro ou trifluorométhyle et dans lesquels le fragment alkyle du groupe phénylalkyle substitué ou non substitué correspondant comporte de 1 à 4 atomes de carbone,

$X^{10}$ est un atome de fluor, de chlore ou de brome,

m est 1 – 4 et

n est 0 – 4;

$R^2$ est un atome d'hydrogène ou un groupe alkyle ayant de 1 à 4 atomes de carbone, le radical phényle non substitué, un radical phényle substitué comportant un ou deux substituants choisis parmi des atomes d'halogène et des groupes alkyle ayant de 1 à 4 atomes de carbone, alcoxy ayant de 1 à 4 atomes de carbone, alkylthio ayant de 1 à 4 atomes de carbone, un radical alkyle substitué par le groupe carboxy, ayant de 1 à 4 atomes de carbone dans le fragment alkyle, le radical carboxy, nitro, trifluorométhyle et hydroxy,

ou $R^2$ est un groupe de formules suivantes

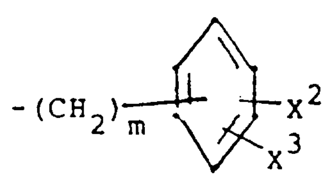

,

$-(CH_2)_mSCH_3$, $-(CH_2)_mSOCH_3$, $-(CH_2)_mSO_2CH_3$ ou $-(CH_2)_mCOOR^6$, dans lesquelles

$R^6$ et m sont tels que définis à propos du radical $R^1$,

$X^2$ et $X^3$ sont indépendamment un atome d'hydrogène ou d'halogène ou un groupe hydroxy,

nitro, alkyle ayant de 1 à 4 atomes de carbone, alkylthio ayant de 1 à 4 atomes de carbone ou alcoxy ayant de 1 à 4 atomes de carbone,

R3 est un groupe de formules $-(CH_2)_nR^7$,

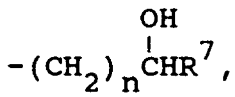

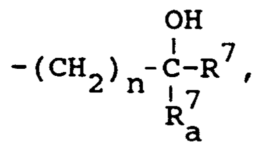

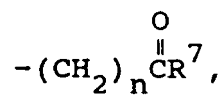

$-(CH_2)_nNR^{18}(CH_2)_qR^7$,

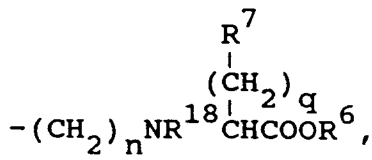

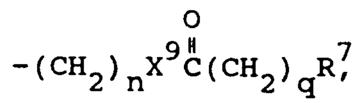

$-NH(CH_2)_{2-3}NHR^7$, $-NH(CH_2)_{2-3}NHCOR^7$,

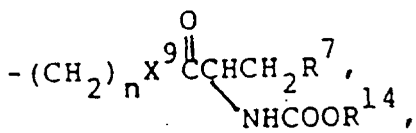

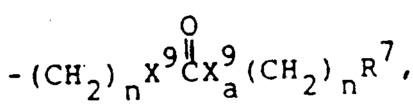

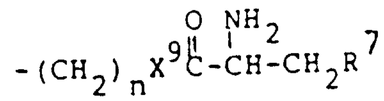

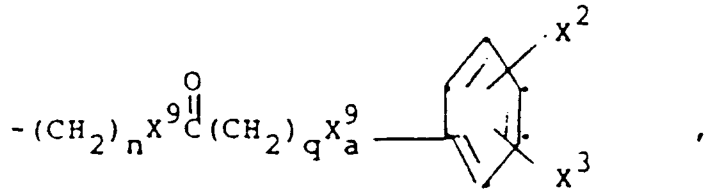

ou

$-(CH)_2)_nNR^{18}SO_2(CH_2)_qR^7$

dans lesquelles

$R^7$ et $R^7_a$ sont indépendamment un radical phényle non substitué ou un radical phényle substitué portant un ou deux substituants choisis parmi des atomes d'halogène et des groupes nitro, hydroxy, alkyle ayant de 1 à 4 atomes de carbone, des groupes de formule $CF^3$, CN, $SCF_3$, C≡CH, $CH_2SCF_3$,

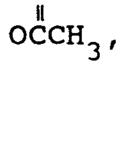

$OCHF_2$, SH, SPh, $PO_3H$ ou $-NR^4R^5$, alcoxy ayant de 1 à 4 atomes de carbone ou alkylthio ayant de 1 à 4 atomes de carbone, ou $R^7$ et $R^7_a$ sont des groupes $\alpha$-naphtyle ou $\beta$-naphtyle,
ou un groupe de formules

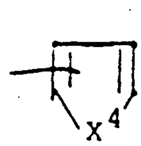 , 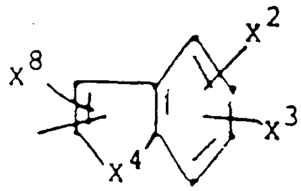 , 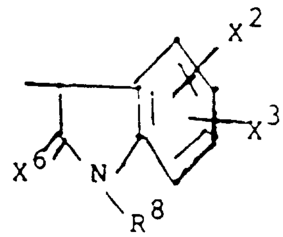

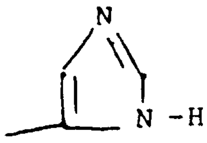 , 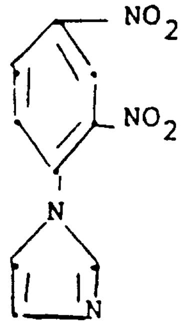 ,

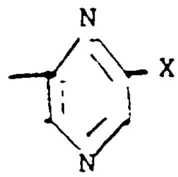 , 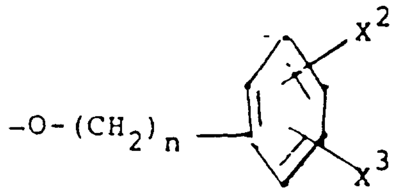 ,

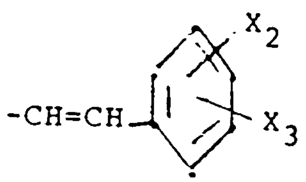 , 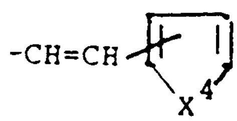

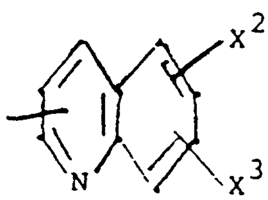 , 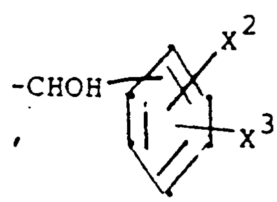 , ou

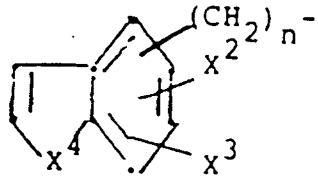

dans lesquelles

$R^8$ est un atome d'hydrogène ou un groupe alkyle ayant de 1 à 4 atomes de carbone, cycloalkyle ayant de 3 à 5 atomes de carbone, qui est lié soit directement, soit par une chaîne alkylène comprenant de 1 à 4 groupes $CH_2$, ou $R^8$ est un groupe de formules

$-(CH_2)_mCONH_2$, $-(CH_2)_mCOOR^6$, $-(CH_2)_mNR^4R^5$,

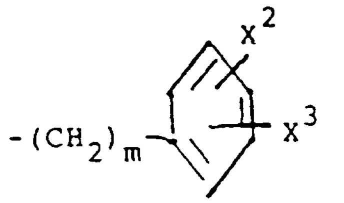,

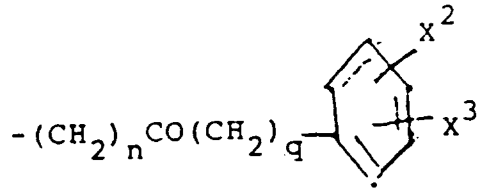,

ou

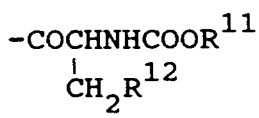,

dans lesquelles

| | |
|---|---|
| $X^2$ et $X^3$ | sont tels que définis à propos du radical $R^2$, |
| $X^4$ | est S, O, $CH_2$ ou $NR^8$, $R^8$ étant tel que défini plus haut, |
| $X^5$ | est un atome d'hydrogène ou d'halogène, le groupe nitro ou un groupe de formules $CF_3$, CN, $-COOR^6$, $R^6$ étant tel que défini plus haut, |
| $X^5$ | est O ou HH, |
| $X^5$ | est un atome d'hydrogène ou un groupe alkyle ayant de 1 à 4 atomes de carbone, |
| $X^5$ et $X_a^9$ | sont indépendamment des groupes de formule $-O-$ ou $-NR^{18}$, $R^{18}$ étant un atome d'hydrogène ou un groupe alkyle ayant de 1 à 4 atomes de carbone, formyle, acétyle, propionyle ou butyryle, |
| $R^{11}$ et $R^{12}$ | sont indépendamment un groupe alkyle ayant de 1 à 4 atomes de carbone ou cycloalkyle ayant de 3 à 5 atomes de carbone, |
| $R^{14}$ | est un groupe alkyle ayant de 1 à 4 atomes de carbone ou phénylalkyle dans lequel le groupe alkyle a de 1 à 4 atomes de carbone; |
| $R^9$ et $R^{10}$ | sont indépendamment un atome d'hydrogène ou le groupe hydroxy ou méthyle; |
| $R^{13}$ | est un atome d'hydrogène ou un groupe alkyle ayant de 1 à 4 atomes de carbone, cycloalkyle ayant de 3 à 5 atomes de carbone, $-O-$, le groupe formyle, acétyle, propionyle ou butyryle; |
| $X^1$ | est un atome d'hydrogène ou d'halogène ou un groupe alkyle ayant de 1 à 4 atomes de carbone, alkylthio ayant de 1 à 4 atomes de carbone, alcoxy ayant de 1 à 4 atomes de carbone, ou un groupe de formules $-NO_2$, $CF_3$, CN, OH, $-(CH_2)_nCOOR^6$ ou $-NR^4R^5$, dans lesquelles $R^4$, $R^5$, $R^6$ et n sont tels que définis à propos de $R^1$; |
| r | est 1 ou 2; |
| $X^7$ | est O, S, HH ou $NR^{15}$; |
| $R^{15}$ | étant un atome d'hydrogène ou un groupe alkyle ayant de 1 à 4 atomes de carbone, ou un groupe de formule |

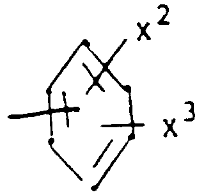

ou $-NR^{16}R^{17}$,

$R^{16}$ et $R^{17}$ étant indépendamment un atome d'hydrogène ou un groupe de formule

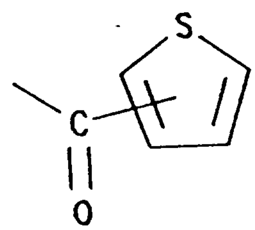

étant toutefois entendu que

$X^7$ ne peut être $NR^{15}$ que lorsque $R^1$ n'est pas H;

q va de 0 à 4;

$\overline{---}$ représente une simple liaison ou une double liaison;

p est O lorsque sa liaison $\overline{---}$ adjacente est une double liaison et est 1 lorsque sa liaison $\overline{---}$ adjacente est une simple liaison, sauf lorsque $R^{13}$ est un atome d'oxygène et p est 1 et $\overline{---}$ est une double liaison; et étant entendu que si

$R^3$ est un groupe de formule $-(CH_2)_nNH(CH_2)_qR^7$, dans lequel q est 0 ou 1 ou est un groupe de formule

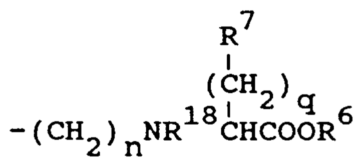

dans laquelle q est 0,

$R^7$ doit avoir une autre signification que celle d'un groupe de formule

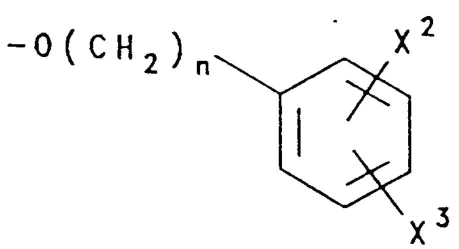

pour la fabrication d'un médicament pour utilisation en tant qu'antagoniste de la cholécystokinine dans le tissu gastrointestinal, et en tant qu'antagoniste de la gastrine, par liaison au récepteur de cholécystokinine ou au récepteur de gastrine correspondant.

**2.** Utilisation selon la revendication 1, caractérisée en ce que, pour la fabrication d'un médicament, on utilise, en tant que composant pharmaceutiquement actif, un composé de formule I ou un sel pharmaceutiquement acceptable ou un sel d'ammonium quaternaire pharmaceutiquement acceptable dudit composé de formule I, composés de formule I dans lesquels

$R^1$ est un atome d'hydrogène ou un groupe alkyle ayant de 1 à 5 atomes de carbone ou un groupe de formule $-(CH_2)_mCOOR^6$, dans laquelle $R^6$ et m sont tels que définis dans la revendication 1;

$R^2$ est un groupe de formule $-(CH_2)_mCOOR^6$ dans laquelle $R^6$ et m sont tels que définis plus haut, ou $R^2$ est le radical phényle non substitué ou un radical phényle substitué comportant un ou deux substituants choisis parmi des atomes d'halogène et des groupes alkyle ayant de 1 à 4 atomes de carbone, alcoxy ayant de 1 à 4 atomes de carbone, alkyle substitué par le radical carboxy, ayant de 1 à 4 atomes de carbone dans le fragment alkyle, carboxy, nitro, trifluorométhyle et

hydroxy;

$R^3$ est un groupe de formules $-(CH_2)_nR^7$,

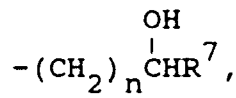

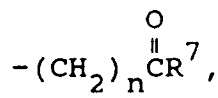

$-(CH_2)_nNH(CH_2)_qR^7$,

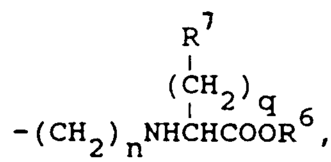

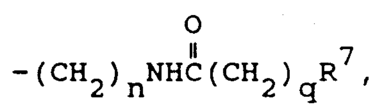

$-NH(CH_2)_{2-3}NHR^7$,

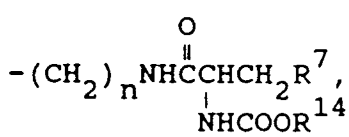

$-NH(CH_2)_{2-3}NHCOR^7$ ou

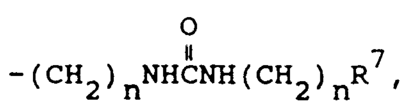

dans lesquelles

$R^7$ est le radical phényle non substitué ou un radical phényle substitué comportant un ou deux substituants choisis parmi des atomes d'halogène et des groupes nitro, hydroxy, alkyle ayant de 1 à 4 atomes de carbone, alcoxy ayant de 1 à 4 atomes de carbone, alkylthio ayant de 1 à 4 atomes de carbone, trifluorométhyle ou des groupes de formules CN, $-C{\equiv}CH$, $SCF_3$,

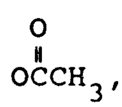

$OCHF_2$, SPh ou $-NR^4R^5$

dans lesquelles

$R^4$ et $R^5$ sont indépendamment un atome d'hydrogène ou un groupe alkyle ayant de 1 à 4

atomes de carbone, ou

R⁷ est un groupe α−naphtyle ou β−naphtyle ou un groupe de formules

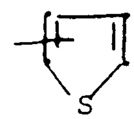

,

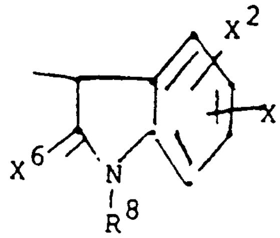

,

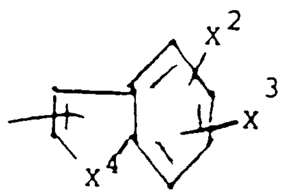

,

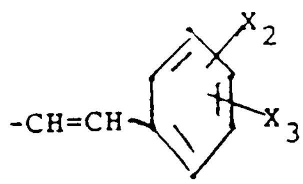

, ou

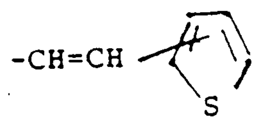

dans lesquelles

$R^8$ est un atome d'hydrogène ou un groupe alkyle ayant de 1 à 4 atomes de carbone, ou un groupe de formule

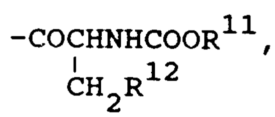

$R^{11}$ et $R^{12}$ étant indépendamment un groupe alkyle ayant de 1 à 4 atomes de carbone,

$X^2$ et $X^3$ sont indépendamment un atome d'hydrogène ou d'halogène ou un groupe hydroxy, nitro, alkylthio ayant de 1 à 4 atomes de carbone, alkyle ayant de 1 à 4 atomes de carbone ou alcoxy ayant de 1 à 4 atomes de carbone,

$X^4$ est S, O ou $NR^8$, $R^8$ étant tel que défini plus haut,

$X^6$ est O ou HH,

$R^{14}$ est un groupe alkyle ayant de 1 à 4 atomes de carbone, et

q est 0−4;

$R^9$ et $R^{10}$ sont indépendamment un atome d'hydrogène ou le groupe hydroxy ou méthyle;

$R^{13}$ est un atome d'hydrogène ou un groupe alkyle ayant de 1 à 4 atomes de carbone, −O, formyle, acétyle, propionyle ou butyryle;

$X^1$ est un atome d'hydrogène ou d'halogène ou un groupe alkyle ayant de 1 à 4 atomes de carbone, alkylthio ayant de 1 à 4 atomes de carbone, alcoxy ayant de 1 à 4 atomes de carbone ou un groupe de formules $-NO_2$, $CF_3$, CN, OH, $-(CH_2)_nCOOR^6$ ou $-NR^4R^5$,

$R^6$ étant tel que défini à propos de $R^1$, et $R^4$ et $R^5$ étant tels que définis à propos de $R^3$;

$X^7$ est O;

m va de 1 à 4;

n va de 0 à 4;

--- représente une simple liaison ou une double liaison,

p est 0 lorsque la liaison --- adjacente est une double liaison et est 1 lorsque sa liaison --- adjacente est une simple liaison, à l'exception du cas où $R^{13}$ est un

atome d'oxygène et p est 1 et $\underline{- - -}$ est une double liaison; et

r est 1 ou 2.

**3.** Utilisation selon la revendication 2, caractérisée en ce que, pour la fabrication d'un médicament, on utilise, en tant que composant pharmaceutiquement actif, un composé de formule I ou un sel pharmaceutiquement acceptable ou sel d'ammonium quaternaire pharmaceutiquement acceptable dudit composé de formule I, dans lequel

$R^1$ est un atome d'hydrogène ou le groupe méthyle, éthyle, carboxyméthyle ou éthylcarboxyméthyle;

$R^2$ est un groupe de formule $-(CH_2)_{1-2}COOR^6$,

$R^6$ étant un atome d'hydrogène ou un groupe alkyle ayant de 1 à 4 atomes de carbone, ou

$R^2$ est le radical phényle non substitué ou un radical phényle substitué comprenant un ou deux substituants qui sont des atomes d'halogène ou le groupe carboxy;

$R^3$ est un groupe de formules $-(CH_2)_nR^7$,

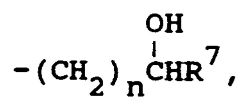

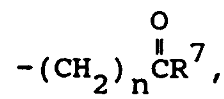

$-(CH_2)_nNH(CH_2)_qR^7$,

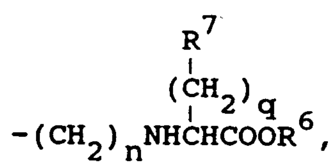

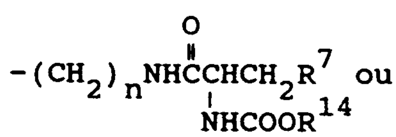 ou 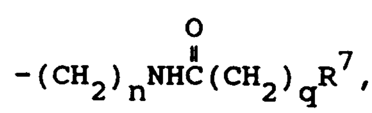

$R^7$ étant un groupe $\alpha$-naphtyle, $\beta$-naphtyle ou un groupe phényle substitué comportant un ou deux substituants choisis parmi des atomes d'halogène et des groupes trifluorométhyle et alkyle ayant de 1 à 4 atomes de carbone, ou

$R^7$ est un groupe de formules

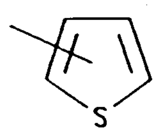,

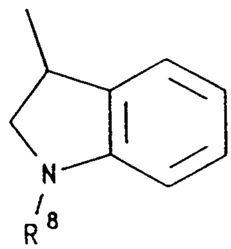,

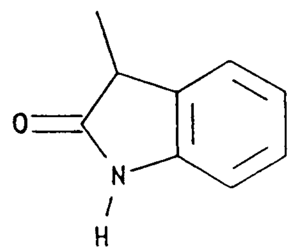

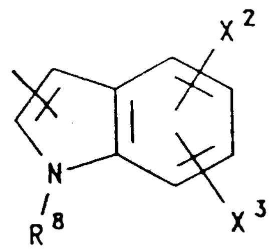,

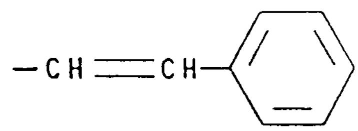

dans lesquelles

| | |
|---|---|
| $R^8$ | est un atome d'hydrogène ou le groupe méthyle ou éthyle, |
| $X^2$ et $X^3$ | sont indépendamment des atomes d'hydrogène ou d'halogène ou le groupe hydroxy ou nitro, |
| $R^{14}$ | est le groupe tert-butyle, |
| n | va de 0 à 4, |
| q | va de 0 à 4 et |
| $R^6$ | est tel que défini à propos du radical $R^2$; |
| $R^9$ et $R^{10}$ | sont indépendamment un atome d'hydrogène ou le groupe hydroxy ou méthyle; |
| $R^{13}$ | est un atome d'hydrogène ou le groupe méthyle ou formyle; |
| $X^1$ | est un atome d'hydrogène ou d'halogène ou un groupe de formule $-NO_2$, $CF_3$, CN ou OH; |
| $X^7$ | est O; |
| - - - | représente une simple liaison ou une double liaison; |
| p | est O lorsque la liaison - - - adjacente est une double liaison et est 1 lorsque sa liaison - - - adjacente est une simple liaison; et |
| r | est 1 ou 2. |

**4.** Utilisation selon la revendication 3, caractérisée en ce que, pour la fabrication d'un médicament, on utilise un composé de formule I ou un sel pharmaceutiquement acceptable ou un sel d'ammonium quaternaire pharmaceutiquement acceptable dudit composé de formule I, composés de formule I dans lesquels

| | |
|---|---|
| $R^1$ | est un atome d'hydrogène ou le groupe méthyle ou carboxyméthyle; |
| $R^2$ | est le groupe phényle, o-fluorophényle, p-fluorophényle, o-chlorophényle, p-chlorophényle, o-carboxyphényle, 2,4-dichlorophényle, 2,6-difluorophényle, $-CH_2COOEt$, $-CH_2COO-t-Bu$, $-CH_2CH_2COOEt$ ou $-CH_2CH_2COO-t-Bu$; |
| $R^3$ | est $-(CH_2)_{1-2}R^7$, |

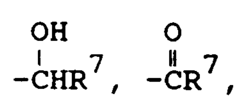

$-(CH_2)_{0-1}NH(CH_2)_{1-2}R^7$,

$-(CH_2)_{0-1}NHCH(R^7)COOR^6$, $-(CH_2)_{0-1}NHC(=O)(CH_2)_{0-2}-R^7$

ou

$-(CH_2)_{0-1}NHCOCH(NHCOOR^{14})CH_2R^7$,

et la configuration stéréochimique s'apparente à celle du D-tryptophane;

$R^7$ est un groupe α-naphtyle, β-naphtyle, monohalogénophényle ou dihalogénophényle, ou un groupe de formules

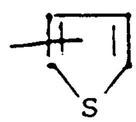
,

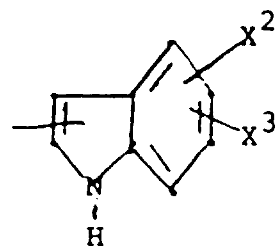
,

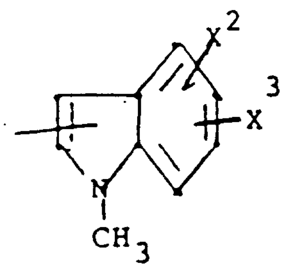
,

dans lesquelles

$X^2$ et $X^3$ sont indépendamment un atome d'hydrogène, de fluor ou de chlore ou le groupe hydroxy,

$R^6$ est un atome d'hydrogène ou le groupe méthyle ou éthyle,

$R^{14}$ est le groupe tert-butyle;

$R^9$ et $R^{10}$ sont indépendamment un atome d'hydrogène ou le groupe hydroxy;

$R^{13}$ est un atome d'hydrogène;

$X^1$ est un atome d'hydrogène, 7-chloro, 7-fluoro ou 7-nitro;

$X^7$ est un atome d'oxygène;

--- représente une simple liaison ou une double liaison;

p est 0 lorsque sa liaison --- adjacente est une double liaison et est 1 lorsque sa liaison --- adjacente est une simple liaison; et

r est 1.

**5.** Utilisation selon la revendication 1, caractérisée en ce que le composé de formule I est l'un des suivants:

1,3 – dihydro – 5 – (2 – fluorophényl) – 3(R) – (3' – indolyl)méthyl – 2H – 1,4 – benzodiazépine – 2 – one,

1,3 – dihydro – 5 – (2 – fluorophényl) – 3(R) – [3' – (1' – méthylindolyl)méthyl] – 1 – méthyl – 2H – 1,4 – benzodiazépine – 2 – one,

1,3 – dihydro – 5 – (2 – fluorophényl) – 3(R) – (3' – indolyl)méthyl – 1 – méthyl – 2H – 1,4 – benzodiazépine – 2 – one,

7 – chloro – 1,3 – dihydro – 3(R) – (3' – indolyl)méthyl – 5 – phényl – 2H – 1,4 – benzodiazépine – 2 – one,

1,3 – dihydro – 3(R) – (3' – indolyl)méthyl – 5 – phényl – 2H – 1,4 – benzodiazépine – 2 – one,

1,3 – dihydro – 5 – (2 – fluorophényl) – 3(R) – (3' – α – indolényl)méthyl – 2H – 1,4 – benzodiazépine – 2 – one,

1,3 – dihydro – 5 – (2 – fluorophényl) – 3(R) – (3' – β – indolényl)méthyl – 2H – 1,4 – benzodiazépine – 2 – one,

1,3 – dihydro – 5 – (2 – fluorophényl) – 3(R) – (3' – indolyl)méthyl – 2H – 1,4 – benzodiazépine – 2 – thione,

1,3 – dihydro – 5 – (2 – fluorophényl) – 3(R) – (3' – indolyl)méthyl – 2H – 1,4 – benzodiazépine,

7 – chloro – 1,3 – dihydro – 3(R) – benzyl – 5 – phényl – 2H – 1,4 – benzodiazépine – 2 – one,

3(R) – benzyloxyméthyl – 7 – chloro – 1,3 – dihydro – 5 – phényl – 2H – 1,4 – benzodiazépine – 2 – one,

7 – chloro – 1,3 – dihydro – 3(RS) – (1 – naphtyl)méthyl – 5 – phényl – 2H – 1,4 – benzodiazépine – 2 – one,

7 – chloro – 1,3 – dihydro – 3(RS) – (2 – naphtyl)méthyl – 5 – phényl – 2H – 1,4 – benzodiazépine – 2 – one,

1,3 – dihydro – 5 – (2 – fluorophényl) – 3(RS) – (2 – thiényl)méthyl – 2H – 1,4 – benzodiazépine – 2 – one,

1,3 – dihydro – 5 – (2 – fluorophényl) – 3(RS) – (3 – thiényl) – 2H – 1,4 – benzodiazépine – 2 – one,

1,3 – dihydro – 5 – (2 – fluorophényl) – 3(R) – [3' – β – (1' – t – Boc – L – leucyl) – indolényl]méthyl – 2H – 1,4 – benzodiazépine – 2 – one,

1,3 – dihydro – 5 – (2 – fluorophényl) – 3(R) – [3' – β – (1' – t – Boc – D – leucyl)indolényl]méthyl – 2H – 1,4 – benzodiazépine – 2 – one,

1,3 – dihydro – 5 – (2 – fluorophényl) – 3(R) – [3' – α – (1' – t – Boc – L – leucyl)indolényl]méthyl – 2H – 1,4 – benzodiazépine – 2 – one,

1,3 – dihydro – 5 – (2 – fluorophényl) – 3(R) – [3' – α – (1' – t – Boc – D – leucyl)indolényl]méthyl – 2H – 1,4 – benzodiazépine – 2 – one,

7 – chloro – 1,3,4,5 – tétrahydro – 3(R) – (3' – indolyl)méthyl – 5 – phényl – 2H – 1,4 – benzodiazépine – 2 – one,

7 – chloro – 1,3,4,5 – tétrahydro – 3(S) – (3' – indolyl)méthyl – 5 – phényl – 2H – 1,4 – benzodiazépine – 2 – one,

4 – (p – chlorobenzoyl) – 5 – (2 – fluorophényl) – 3(R) – [3' – (1' – méthylindolyl)méthyl] – 1 – méthyl – 1,3,4,5 – tétrahydro – 2H – 1,4 – benzodiazépine – 2 – one,

4 – acétyl – 5 – (2 – fluorophényl) – 3(R) – [3' – (1' – méthylindolyl)méthyl] – 1 – méthyl – 1,3,4,5 – tétrahydro – 2H – 1,4 – benzodiazépine – 2 – one,

7 – chloro – 5 – (2 – chlorophényl) – 1,3 – dihydro – 3(R) – (3' – indolyl)méthyl – 2H – 1,4 – benzodiazépine – 2 – one,

1,3 – dihydro – 3(R) – (3' – indolyl)méthyl – 5 – méthyl – 2H – 1,4 – benzodiazépine – 2 – one,

1 – benzyl – 7 – chloro – 1,3 – dihydro – 3(R) – (3' – indolyl)méthyl – 5 – phényl – 2H – 1,4 – benzodiazépine – 2 – one,

7 – chloro – 1,3 – dihydro – 3(R) – (3' – indolyl)méthyl – 1 – méthyl – 5 – phényl – 2H – 1,4 – benzodiazépine – 2 – one,

1,3 – dihydro – 5 – (2 – fluorophényl) – 3(S) – (3' – indolyl)méthyl – 2H – 1,4 – benzodiazépine – 2 – one,

1 – benzyl – 7 – chloro – 1,3 – dihydro – 3(S) – (3' – indolyl)méthyl – 5 – phényl – 2H – 1,4 – benzodiazépine – 2 – one,

7 – chloro – 1,3 – dihydro – 3(R) – (3' – indolyl)méthyl – 5 – phényl – 2H – 1,4 – benzodiazépine – 2 – thione,

1,3 – dihydro – 5 – (2 – fluorophényl) – 3(R) – (3' – indolyl)méthyl – 2H – 1,4 – benzodiazépine – 2 – [N' – (3 – thiénoyl)]hydrazide,

1,3 – dihydro – 1 – éthyl – 5 – (2 – fluorophényl) – 3(R) – (3' – indolyl)méthyl – 2H – 1,4 – benzodiazépine – 2 – one,

1,3 – cyclopropylméthyl – 1,3 – dihydro – 5 – (2 – fluorophényl) – 3(R) – (3' – indolyl)méthyl – 2H – 1,4 – benzodiazépine – 2 – one,

1,3 – dihydro – 5 – (2 – fluorophényl) – 3(R) – (3' – indolyl)méthyl – 1 – pentyl – 2H – 1,4 – benzodiazépine – 2 – one,

1,3 – dihydro – 5 – (2 – fluorophényl) – 3(R) – (3' – indolyl)méthyl – 1 – (3 – méthylbutyl) – 2H – 1,4 – benzodiazépine – 2 – one,

1,3 – dihydro – 5 – (2 – fluorophényl) – 3(R) – (3' – indolyl)méthyl – 1 – (2,2,2 – trifluoroéthyl) – 2H – 1,4 – benzodiazépine – 2 – one,

1,3 – dihydro – 1 – (2 – diméthylaminoéthyl) – 5 – (2 – fluorophényl) – 3(R) – (3' – indolyl)méthyl – 2H – 1,4 – benzodiazépine – 2 – one,

1,3 – dihydro – 1 – (éthoxycarbonylméthyl) – 5 – (2 – fluorophényl) – 3(R) – (3' – indolyl)méthyl – 2H – 1,4 – benzodiazépine – 2 – one,

1 – carboxyméthyl – 1,3 – dihydro – 5 – (2 – fluorophényl) – 3(R) – (3' – indolyl)méthyl – 2H – 1,4 – benzodiazépine – 2 – one,

1,3 – dihydro – 5 – (2 – fluorophényl) – 3(R) – [3' – (1' – p – chlorobenzyloylindolyl)méthyl] – 1 – méthyl – 2H – 1,4 – benzodiazépine – 2 – one,

7 – chloro – 1,3 – dihydro – 3(R) – [3' – (1' – benzylindolyl)méthyl] – 1 – méthyl – 5 – phényl – 2H – 1,4 – benzodiazépine – 2 – one,

1,3 – dihydro – 3(RS) – [1 – hydroxy – 1 – (3' – indolyl)]méthyl – 1 – méthyl – 5 – phényl – 2H – 1,4 – benzodiazépine – 2 – one,

1,3 – dihydro – 1 – méthyl – 5 – phényl) – 3(RS) – (3 – thiénoyl) – 2H – 1,4 – benzodiazépine – 2 – one,

1,3 – dihydro – 3(RS) – [1 – hydroxy – 1 – (3 – thiényl)]méthyl – 1 – méthyl – 5 – phényl – 2H – 1,4 – benzodiazépine – 2 – one,

1,3 – dihydro – 3(RS) – [1 – hydroxy – 1 – [3 – (1 – méthylindolyl)]]méthyl – 1 – méthyl – 5 – phényl – 2H – 1,4 – benzodiazépine – 2 – one, deux stéréoisomères,

1,3 – dihydro – 3(RS) – (1 – hydroxy – 1 – phényl)méthyl – 1 – méthyl – 5 – phényl – 2H – 1,4 – benzodiazépine – 2 – one,

1,3 – dihydro – 3(RS) – [1 – hydroxy – 1 – (2 – thiényl)]méthyl – 1 – méthyl – 5 – phényl – 2H – 1,4 – benzodiazépine – 2 – one,

1,3 – dihydro – 3(RS) – hydroxy – 1 – méthyl – 5 – phényl – 3 – (3' – thiénoyl) – 2H – 1,4 – benzodiazépine – 2 – one,

1,5 – dihydro – 5(RS) – hydroxy – 1 – méthyl – 5 – phényl – 3 – (3' – thiénoyl) – 2H – 1,4 – benzodiazépine – 2 – one,

7 – chloro – 1,3 – dihydro – 3(R) – [(2',3' – dihydro – 2' – oxo – 1'H – indole – 3' – yl)méthyl] – 5 – phényl – 2H – 1,4 – benzodiazépine – 2 – one,

7 – chloro – 1,3 – dihydro – 3(R) – [(3' – (2,4 – dinitrophényl)imidazole – 5' – yl)méthyl] – 5 – phényl – 2H – 1,4 – benzodiazépine – 2 – one,

7 – chloro – 1,3 – dihydro – 3(R) – (3' – imidazole – 5' – yl)méthyl – 5 – phényl – 2H – 1,4 – benzodiazépine – 2 – one,

3(RS) – [3' – (5' – bromo – indolyl)méthyl] – 1,3 – dihydro – 5 – phényl – 2H – 1,4 – benzodiazépine – 2 – one,

5 – o – carboxyphényl – 1,3 – dihydro – 3(R) – (3' – indolyl)méthyl – 2H – 1,4 – benzodiazépine – 2 – one,

1,3 – dihydro – 3(RS) – [3' – (5' – fluoro – indolyl)méthyl] – 5 – o – fluorophényl – 2H – 1,4 – benzodiazépine – 2 – one,

1,3 – dihydro – 3(RS) – [3' – (6' – fluoro – indolyl)méthyl] – 5 – o – fluorophényl – 2H – 1,4 – benzodiazépine – 2 – one,

2 – N – [2(RS),3 – bis(Bocamino)propanoyl]amino – 2' – fluorobenzophénone,

2 – N – [2(RS),3 – diphtalylaminopropanoyl]amino – 2' – fluorobenzophénone,

1,3 – dihydro – 5 – (2' – fluorophényl) – 3(RS) – aminométhyl – 2H – 1,4 – benzodiazépine – 2 – one,

1,3 – dihydro – 5 – (2' – fluorophényl) – 3(R) – (4 – amino)butyl – 2H – 1,4 – benzodiazépine – 2 – one,

1,3 – dihydro – 5 – (2' – fluorophényl) – 3(RS) – benzyloxycarbonylaminométhyl – 2H – 1,4 – benzodiazépine – 2 – one,

1,3 – dihydro – 5 – (2' – fluorophényl) – 3(RS) – (3 – thiophènecarbonylaminométhyl – 2H – 1,4 – benzodiazépine – 2 – one,

1,3 – dihydro – 5 – (2' – fluorophényl) – 3(RS) – (2 – indole)carbonylaminométhyl – 2H – 1,4 – benzodiazépine – 2 – one,

1,3 – dihydro – 5 – (2' – fluorophényl) – 3(RS) – (6' – chloropyrazinyl)aminométhyl – 2H – 1,4 –

benzodiazépine – 2 – one,

1,3 – dihydro – 5 – (2' – fluorophényl) – 3(RS) – aminométhyl – 1 – méthyl – 2H – 1,4 – benzodiazépine – 2 – one,

3(RS) – (2 – indole – carbonylamino) – 1,3 – dihydro – 5 – phényl – 2H – 1,4 – benzodiazépine – 2 – one,

1,3 – dihydro – 3(RS) – [2 – (3 – indolyl)éthyl]amino – 5 – phényl – 2H – 1,4 – benzodiazépine – 2 – one,

3(RS) – [3 – (3 – indole)propionylamino] – 1,3 – dihydro – 5 – phényl – 2H – 1,4 – benzodiazépine – 2 – one,

3(RS) – (3 – indole – acétylamino) – 1,3 – dihydro – 5 – phényl – 2H – 1,4 – benzodiazépine – 2 – one,

3(RS) – (Boc – L – tryptophyl)amino – 1,3 – dihydro – 5 – phényl – 2H – 1,4 – benzodiazépine – 2 – one,

Z – 1,3 – dihydro – 1 – méthyl – 5 – phényl – 3 – (3 – thiénylméthylène) – 2H – 1,4 – benzodiazépine – 2 – one,

E – 1,3 – dihydro – 1 – méthyl – 5 – phényl – 3 – (3 – thiénylméthylène) – 2H – 1,4 – benzodiazépine – 2 – one,

3(RS) – (Boc – D – tryptophyl)amino – 1,3 – dihydro – 5 – phényl – 2H – 1,4 – benzodiazépine – 2 – one,

3(RS) – [4 – (3 – indole)butyrylamino] – 1,3 – dihydro – 5 – phényl – 2H – 1,4 – benzodiazépine – 2 – one,

1,3 – dihydro – 3(RS) – (benzyloxycarbonyl)aminométhyl – 5 – (2 – fluorophényl) – 2H – 1,4 – benzodiazépine,

1,3 – dihydro – 3(RS) – [3' – (thiophène)carbonyl]aminométhyl – 5 – (2 – fluorophényl) – 2H – 1,4 – benzodiazépine,

1,3 – dihydro – 3(RS) – (2 – indole – carbonyl)aminométhyl – 5 – (2 – fluorophényl) – 2H – 1,4 – benzodiazépine,

1,3 – dihydro – 3(RS) – (2 – L – hydroxy – 2 – phénylacétyl)aminométhyl – 5 – (2 – fluorophényl) – 2H – 1,4 – benzodiazépine,

1 – (2 – cyanoéthyl) – 1,3 – dihydro – 5 – (2 – fluorophényl) – 3(R) – (3' – indolyl)méthyl) – 2H – 1,4 – benzodiazépine – 2 – one,

1 – (2 – cyanoéthyl) – 1,3 – dihydro – 5 – (2 – fluorophényl) – 3(R) – [1' – (2 – cyanoéthyl) – 3' – indolyl] – méthyl – 2H – 1,4 – benzodiazépine – 2 – one

1 – (2 – carboxyéthyl) – 1,3 – dihydro – 5 – (2 – fluorophényl) – 3(R) – (3' – indolyl)] – méthyl – 2H – 1,4 – benzodiazépine – 2 – one,

1,3 – dihydro – 3(R) – 3' – indolyl)méthyl – 5 – (2 – fluorophényl) – 2H – 1,4 – benzodiazépine – 2 – one – 4 – oxyde,

1,3 – dihydro – 5 – (2 – fluorophényl) – 3(RS) – (2 – indole – carbonylamino) – 2H – 1,4 – benzodiazépine – 2 – one,

1,3 – dihydro – 5 – (2 – fluorophényl) – 3(RS) – (2 – indole – carbonylamino) – 1 – méthyl – 2H – 1,4 – benzodiazépine – 2 – one,

1,3 – dihydro – 5 – (2 – fluorophényl) – 1 – méthyl – 3(RS) – [2' – (1 – méthylindole)carbonylamino] – 2H – 1,4 – benzodiazépine – 2 – one,

1,3 – dihydro – 3(RS) – (4 – nitrophénylcarbonyl)amino – 5 – (2 – fluorophényl) – 2H – 1,4 – benzodiazépine – 2 – one,

1,3 – dihydro – 3(RS) – (2 – indole – carbonyloxy) – 5 – phényl – 2H – 1,4 – benzodiazépine – 2 – one,

1,3 – dihydro – 5 – (2 – fluorophényl) – 3(RS) – (3 – thiophènecarbonylamino) – 2H – 1,4 – benzodiazépine – 2 – one,

1,3 – dihydro – 3(RS) – (3 – indole – carbonylamino) – 5 – phényl – 2H – 1,4 – benzodiazépine – 2 – one,

1,3 – dihydro – 3(RS) – (4 – thianaphtène – acétyl)amino – 5 – (2 – fluorophényl) – 2H – 1,4 – benzodiazépine – 2 – one,

1,3 – dihydro – 3(RS) – (4 – chlorophénylcarbonyl)amino – 5 – (2 – fluorophényl) – 2H – 1,4 – benzodiazépine – 2 – one,

1,3 – dihydro – 3(RS) – (4 – méthylphénylsulfonyl)amino – 5 – (2 – fluorophényl) – 2H – 1,4 – benzodiazépine – 2 – one,

1 – carboxyméthyl – 1,3 – dihydro – 5 – (2 – fluorophényl) – 3(RS) – (2 – indole – carbonylamino) – 2H – 1,4 – benzodiazépine – 2 – one,

1,3 – dihydro – 3(RS) – (5 – fluoro – indole – 2 – carbonylamino) – 5 – (2 – fluorophényl) – 2H – 1,4 – benzodiazépine – 2 – one,

1,3 – dihydro – 3(RS) – (3' – méthylindényl – 2 – carbonyl)amino – 5 – (2 – fluorophényl) – 2H – 1,4 – benzodiazépine – 2 – one,

1,3 – dihydro – 3(RS) – (2 – quinaldyl)amino – 5 – (2 – fluorophényl) – 2H – 1,4 – benzodiazépine – 2 – one,

1,3 – dihydro – 3(RS) – (2 – L – hydroxy – 2 – phénylacétyl)amino – 5 – (2 – fluorophényl) – 2H – 1,4 – benzodiazépine – 2 – one,

1,3 – dihydro – 3(RS) – (5 – chloro – indole – 2 – carbonylamino) – 5 – (2 – fluorophényl) – 2H – 1,4 – benzodiazépine – 2 – one,

3(RS) – [N – (2 – indole – carbonyl) – N – méthylamino] – 1,3 – dihydro – 5 – phényl – 2H – 1,4 – benzodiazépine – 2 – one,

1,3 – dihydro – 3(RS) – (2 – indole – carbonylamino) – 1 – méthyl – 5 – phényl – 2H – 1,4 – benzodiazépine – 2 – one,

1,3 – dihydro – 1 – méthyl – 3(RS) – [2 – (1 – méthylindole)carbonylamino] – 5 – phényl – 2H – 1,4 – benzodiazépine – 2 – one,

1 – carboxyméthyl – 1,3 – dihydro – 3(RS) – (2 – indole – carbonylamino) – 5 – phényl – 2H – 1,4 – benzodiazépine – 2 – one,

1,3 – dihydro – 3(RS) – (5 – bromo – indole – 2 – carbonylamino) – 5 – (2 – fluorophényl) – 2H – 1,4 – benzodiazépine – 2 – one,

3(RS) – cinnamoylamino – 1,3 – dihydro – 5 – (2 – fluorophényl) – 2H – 1,4 – benzodiazépine – 2 – one,

1,3 – dihydro – 3(RS) – (5 – hydroxy – 2 – indolylcarbonyl)amino – 5 – (2 – fluorophényl) – 2H – 1,4 – benzodiazépine – 2 – one,

1 – carboxamidométhyl – 1,3 – dihydro – 3R – (3 – indolylméthyl) – 5 – (2 – fluorophényl) – 2H – 1,4 – benzodiazépine – 2 – one,

1,3 – dihydro – 5 – (2 – fluorophényl) – 3(RS) – (2 – indolylméthylamino) – 2H – 1,4 – benzodiazépine – 2 – one,

1,3 – dihydro – 3(RS) – (phénylaminométhylcarbonyl)amino – 5 – (2 – fluorophényl) – 2H – 1,4 – benzodiazépine – 2 – one,

1,3 – dihydro – 3(RS) – (5 – méthoxyindole – 2 – carbonylamino) – 5 – (2 – fluorophényl) – 2H – 1,4 – benzodiazépine – 2 – one,

1,3 – dihydro – 3(RS) – (1 – méthylindole – 2 – carbonylamino) – 5 – (2 – fluorophényl) – 2H – 1,4 – benzodiazépine – 2 – one,

1,3 – dihydro – 1 – méthyl – 3(RS) – (4 – chlorophénylcarbonyl)amino – 5 – (2 – fluorophényl) – 2H – 1,4 – benzodiazépine – 2 – one,

1,3 – dihydro – 5 – (2 – fluorophényl) – 3(RS) – (2 – benzofurannecarbonylamino) – 2H – 1,4 – benzodiazépine – 2 – one,

1 – éthoxycarbonylméthyl – 1,3 – dihydro – 3(RS) – (4 – chlorophénylcarbonyl)amino – 5 – (2 – fluorophényl) – 2H – 1,4 – benzodiazépine – 2 – one

1,3 – dihydro – 3(RS) – (4 – chlorophénylcarbonyl)amino – 5 – phényl – 2H – 1,4 – benzodiazépine – 2 – one,

1,3 – dihydro – 1 – méthyl – 3(RS) – (4 – chlorophénylcarbonyl)amino – 5 – phényl) – 2H – 1,4 – benzodiazépine – 2 – one,

1 – carboxyméthyl – 1,3 – dihydro – 3(RS) – (4 – chlorophénylcarbonyl)amino – 5 – (2 – fluorophényl) – 2H – 1,4 – benzodiazépine – 2 – one,

3(RS) – (2(S) – tert – butoxycarbonylamino – 3 – phénylpropanoylamino) – 1,3 – dihydro – 5 – phényl – 2H – 1,4 – benzodiazépine – 2 – one,

3(RS) – (2(S) – tert – butoxycarbonylamino – 3 – phénylpropanoylamino) – 1,3 – dihydro – 1 – méthyl – 5 – phényl – 2H – 1,4 – benzodiazépine – 2 – one,

3(R) – et 3(S) – (2(S) – amino – 3 – phénylpropanoylamino) – 1,3 – dihydro – 1 – méthyl – 5 – phényl – 2H – 1,4 – benzodiazépine – 2 – one,

3(R) – et 3(S) – amino – 1,3 – dihydro – 1 – méthyl – 5 – phényl – 2H – 1,4 – benzodiazépine – 2 – one,

3(R) – et 3(S) – amino – 1,3 – dihydro – 5 – (2 – fluorophényl) – 1 – méthyl – 2H – 1,4 – benzodiazépine – 2 – one,

3(S) – ( – ) – 1,3 – dihydro – 3 – (2 – indole – carbonylamino) – 1 – méthyl – 5 – phényl – 2H – 1,4 – benzodiazépine – 2 – one,

3(S) – ( + ) – 1,3 – dihydro – 5 – (2 – fluorophényl) – 3 – (2 – indolecarbonylamino) – 1 – méthyl – 2H – 1,4 – benzodiazépine – 2 – one,

3(R) – ( – ) – 1,3 – dihydro – 5 – (2 – fluorophényl) – 3 – (2 – indolecarbonylamino) – 1 – méthyl – 2H – 1,4 – benzodiazépine – 2 – one,

3(R) – ( – ) – 1,3 – dihydro – 3 – (4 – chlorobenzoylamino) – 5 – (2 – fluorophényl) – 1 – méthyl – 2H – 1,4 – benzodiazépine – 2 – one,

3(S) – ( + ) – 1,3 – dihydro – 3 – (4 – chlorobenzoylamino) – 5 – (2 – fluorophényl) – 1 – méthyl – 2H – 1,4 – benzodiazépine – 2 – one,

3(S) – ( – ) – 1,3 – dihydro – 3 – (4 – bromobenzoylamino) – 1 – méthyl – 5 – phényl – 2H – 1,4 – benzodiazépine – 2 – one,

3(R) – ( + ) – 1,3 – dihydro – 3 – (4 – bromobenzoylamino) – 1 – méthyl – 5 – phényl – 2H – 1,4 – benzodiazépine – 2 – one,

3(R) – ( + ) – 1,3 – dihydro – 3 – (2 – indole – carbonylamino) – 1 – méthyl – 5 – phényl – 2H – 1,4 – benzodiazépine – 2 – one,

1,3 – dihydro – 3(RS) – (2 – indolinecarbonylamino) – 5 – phényl – 2H – 1,4 – benzodiazépine – 2 – one,

1,3 – dihydro – 5 – (2 – fluorophényl) – 3(RS) – (p – trifluorométhylbenzoylamino) – 2H – 1,4 – benzodiazépine – 2 – one,

1,3 – dihydro – 5 – (2 – fluorophényl) – 3(RS) – (p – méthylbenzoylamino) – 2H – 1,4 – benzodiazépine – 2 – one,

1,3 – dihydro – 5 – (2 – fluorophényl) – 3(RS) – (p – méthoxybenzoylamino) – 2H – 1,4 – benzodiazépine – 2 – one,

3(RS) – (o – chlorobenzoylamino) – 1,3 – dihydro – 5 – phényl – 2H – 1,4 – benzodiazépine – 2 – one,

3(RS) – (o – chlorobenzoylméthylamino) – 1,3 – dihydro – 1 – méthyl – 5 – phényl – 2H – 1,4 – benzodiazépine – 2 – one,

3(RS) – (o – chlorobenzoylamino) – 1,3 – dihydro – 1 – méthyl – 5 – phényl – 2H – 1,4 – benzodiazépine – 2 – one,

3(RS) – (m – chlorobenzoylamino) – 1,3 – dihydro – 5 – phényl – 2H – 1,4 – benzodiazépine – 2 – one,

3(RS) – (3,4 – dichlorobenzoylamino) – 1,3 – dihydro – 5 – phényl – 2H – 1,4 – benzodiazépine – 2 – one,

3(RS) – (p – chlorobenzoylméthylamino) – 1,3 – dihydro – 5 – (2' – fluorophényl) – 1 – méthyl – 4 – oxo – 2H – 1,4 – benzodiazépine – 2 – one,

1,3 – dihydro – 5 – phényl – 3(RS) – (4' – méthylthiobenzoylamino) – 2H – 1,4 – benzodiazépine – 2 – one,

1,3 – dihydro – 3(RS) – (4' – fluorobenzoylamino) – 5 – phényl – 2H – 1,4 – benzodiazépine – 2 – one,

1,3 – dihydro – 5 – phényl – 3(RS) – (4' – trifluorométhylbenzoylamino) – 2H – 1,4 – benzodiazépine – 2 – one,

1,3 – dihydro – 3(RS) – (4' – tert – butylbenzoylamino) – 5 – phényl – 2H – 1,4 – benzodiazépine – 2 – one,

3(RS) – (3,5 – dichlorobenzoylamino) – 1,3 – dihydro – 5 – phényl – 2H – 1,4 – benzodiazépine – 2 – one,

1,3 – dihydro – 3(RS) – (p – hydroxybenzoylamino) – 5 – phényl – 2H – 1,4 – benzodiazépine – 2 – one,

3(RS) – (4' – cyanobenzoylamino) – 1,3 – dihydro – 5 – phényl – 2H – 1,4 – benzodiazépine – 2 – one,

3(S) – ( – ) – 3 – (2 – chlorobenzoylamino) – 1,3 – dihydro – 1 – méthyl – 5 – phényl – 2H – 1,4 – benzodiazépine – 2 – one,

3(R) – ( + ) – 3 – (2 – chlorobenzoylamino) – 1,3 – dihydro – 1 – méthyl – 5 – phényl – 2H – 1,4 – benzodiazépine – 2 – one,

1,3 – dihydro – 3(RS) – (p – diméthylaminobenzoylamino) – 5 – (2 – fluorophényl) – 2H – 1,4 – benzodiazépine – 2 – one,

1,3 – dihydro – 3(RS) – (3,4 – diméthoxybenzoylamino) – 5 – (2 – fluorophényl) – 2H – 1,4 – benzodiazépine – 2 – one,

3(S) – ( + ) – 3 – (3 – bromobenzoylamino) – 1,3 – dihydro – 5 – (2 – fluorophényl – 1 – méthyl – 2H – 1,4 – benzodiazépine – 2 – one,

1,3 – dihydro – 5 – phényl – 3(RS) – (3 – trifluorométhylthiobenzoylamino) – 2H – 1,4 – benzodiazépine – 2 – one,

3(S) – ( + ) – 3 – (4 – bromobenzoylamino) – 1,3 – dihydro – 5 – (2 – fluorophényl) – 1 – méthyl – 2H – 1,4 – benzodiazépine – 2 – one,

3(S) – ( + ) – 3 – (4 – tert – butylbenzoylamino) – 1,3 – dihydro – 5 – (2 – fluorophényl) – 1 – méthyl – 2H – 1,4 – benzodiazépine – 2 – one,

1,3 – dihydro – 5 – (2 – fluorophényl) – 3(RS) – (pyrrole – 2 – carbonylamino) – 2H – 1,4 – benzodiazépine – 2 – one,

3(S) – ( + ) – 1,3 – dihydro – 5 – (2 – fluorophényl) – 3 – (4 – iodobenzoylamino) – 1 – méthyl – 2H – 1,4 – benzodiazépine – 2 – one,

1,3 – dihydro – 3(RS) – (2 – naphtoylamino) – 5 – phényl – 2H – 1,4 – benzodiazépine – 2 – one,

3(S) – ( – ) – 3 – (2 – bromobenzoylamino) – 1,3 – dihydro – 5 – (2 – fluorophényl) – 1 – méthyl – 2H – 1,4 – benzodiazépine – 2 – one,

3(S) – ( + ) – 3 – (4 – cyanobenzoylamino) – 1,3 – dihydro – 5 – (2 – fluorophényl) – 1 – méthyl – 2H –

1,4 – benzodiazépine – 2 – one,

1,3 – dihydro – 5 – phényl – 3(RS) – (4 – n – propylbenzoylamino) – 2H – 1,4 – benzodiazépine – 2 – one,

1,3 – dihydro – 5 – phényl – 3(RS) – (4 – phénylbenzoylamino) – 2H – 1,4 – benzodiazépine – 2 – one,

1,3 – dihydro – 3(RS) – (4 – n – pentylbenzoylamino) – 5 – phényl – 2H – 1,4 – benzodiazépine – 2 – one,

1,3 – dihydro – 3(RS) – (1 – naphtoylamino) – 5 – phényl – 2H – 1,4 – benzodiazépine – 2 – one,

3(S) – ( + ) – 1,3 – dihydro – 5 – (2 – fluorophényl) – 3 – (3 – iodobenzoylamino) – 1 – méthyl – 2H – 1,4 – benzodiazépine – 2 – one,

3(R) – ( – ) – 1,3 – dihydro – 5 – (2 – fluorophényl) – 3 – (3 – iodobenzoylamino) – 1 – méthyl – 2H – 1,4 – benzodiazépine – 2 – one,

3(R) – ( + ) – 1,3 – dihydro – 5 – (2 – fluorophényl) – 3 – (2 – iodobenzoylamino) – 1 – méthyl – 2H – 1,4 – benzodiazépine – 2 – one,

3(S) – ( – ) – 1,3 – dihydro – 5 – (2 – fluorophényl) – 3 – (2 – iodobenzoylamino) – 1 – méthyl – 2H – 1,4 – benzodiazépine – 2 – one,

3(R) – ( + ) – 3 – (2 – bromobenzoylamino) – 1,3 – dihydro – 5 – (2 – fluorophényl) – 1 – méthyl – 2H – 1,4 – benzodiazépine – 2 – one,

3(R) – ( + ) – 3 – (2 – chlorobenzoylamino) – 1,3 – dihydro – 5 – (2 – fluorophényl) – 1 – méthyl – 2H – 1,4 – benzodiazépine – 2 – one,

1,3 – dihydro – 5 – (2 – fluorophényl) – 3(RS) – phénylcarbonylamino) – 2H – 1,4 – benzodiazépine – 2 – one,

1,3 – dihydro – 5 – (2 – fluorophényl) – 3(RS) – (2 – chlorophényl)carbonylamino – 2H – 1,4 – benzodiazépine – 2 – one,

1,3 – dihydro – 5 – (2 – fluorophényl) – 3(RS) – benzyloxycarbonylamino – 2H – 1,4 – benzodiazépine – 2 – one,

1,3 – dihydro – 5 – (2 – fluorophényl) – 3(RS) – benzyloxycarbonylamino – 2H – 1,4 – benzodiazépine – 2 – thione,

1 – (4 – chlorophényl)carbonyl – 1,3 – dihydro – 5 – (2 – fluorophényl) – 3(RS) – (4 – chlorophényl) – carbonylamino – 2H – 1,4 – benzodiazépine – 2 – one,

1 – (4 – chlorophényl)carbonyl – 1,3 – dihydro – 5 – (2 – fluorophényl) – 3(RS) – (4 – chlorophényl) – carbonyloxy – 2H – 1,4 – benzodiazépine – 2 – one,

1,3 – dihydro – 5 – (2 – fluorophényl) – 3(RS) – (4 – chlorophényl)oxy – 2H – 1,4 – benzodiazépine – 2 – one,

1,3 – dihydro – 5 – (2 – fluorophényl) – 3(RS) – (4 – chlorophényl)carbonylamino – 2H – 1,4 – benzodiazépine – 2 – one,

1,3 – dihydro – 5 – (2 – fluorophényl) – 3(RS) – (2 – indole)carbonylamino – 2H – 1,4 – benzodiazépine – 2 – thione ou

1,3 – dihydro – 3(RS) – (4 – chlorophényl)aminocarbonylamino – 2H – 1,4 – benzodiazépine – 2 – one.

**6.** Utilisation selon la revendication 1, caractérisée en ce que le composé de formule I est l'un des suivants:

(S) – ( – ) – 1,3 – dihydro – 3 – (4 – bromobenzoylamino) – 1 – méthyl – 5 – phényl – 2H – 1,4 – benzodiazépine – 2 – one,

3(RS) – (2 – indole – carbonylamino) – 1,3 – dihydro – 5 – phényl – 2H – 1,4 – benzodiazépine – 2 – one,

1,3 – dihydro – 3(RS) – (2 – indole – carbonylamino) – 1 – méthyl – 5 – phényl – 2H – 1,4 – benzodiazépine – 2 – one,

1,3 – dihydro – 1 – méthyl – 3(RS) – [2 – (1 – méthylindole)carbonyl amino] – 5 – phényl – 2H – 1,4 – benzodiazépine – 2 – one,

1,3 – dihydro – 1 – méthyl – 3(RS) – (4 – chlorophénylcarbonyl)amino – 5 – (2 – fluorophényl) – 2H – 1,4 – benzodiazépine – 2 – one,

1,3 – dihydro – 5 – (2 – fluorophényl) – 3(RS) – (2 – indole – carbonylamino) – 1 – méthyl – 2H – 1,4 – benzodiazépine – 2 – one,

1,3 – dihydro – 5 – (2 – fluorophényl) – 1 – méthyl – 3(RS) – [2' – (1 – méthylindole)carbonylamino] – 2H – 1,4 – benzodiazépine – 2 – one,

3(R) – ( – ) – 1,3 – dihydro – 3 – (4 – chlorobenzoylamino) – 5 – (2 – fluorophényl) – 1 – méthyl – 2H – 1,4 – benzodiazépine – 2 – one,

3(S) – ( + ) – 1,3 – dihydro – 3 – (4 – chlorobenzoylamino) – 5 – (2 – fluorophényl) – 1 – méthyl – 2H – 1,4 – benzodiazépine – 2 – one,

3(R) − ( + ) − 1,3 − dihydro − 3 − (4 − bromobenzoylamino) − 1 − méthyl − 5 − phényl − 2H − 1,4 − benzodiazépine − 2 − one,
3(R) − ( + ) − 1,3 − dihydro − 3 − (2 − indole − carbonylamino) − 1 − méthyl − 5 − phényl − 2H − 1,4 − benzodiazépine − 2 − one,
1,3 − dihydro − 5 − (2 − fluorophényl) − 3(RS) − (2 − indole − carbonylamino) − 2H − 1,4 − benzodiazépine − 2 − one,
1,3 − dihydro − 3(RS) − (2 − indole − carbonyloxy) − 5 − phényl − 2H − 1,4 − benzodiazépine − 2 − one,
1,3 − dihydro − 3(RS) − (4 − chlorophénylcarbonyl)amino − 5 − (2 − fluorophényl) − 2H − 1,4 − benzodiazépine − 2 − one,
1 − carboxyméthyl − 1,3 − dihydro − 5 − (2 − fluorophényl) − 3(RS) − (2 − indole − carbonylamino) − 2H − 1,4 − benzodiazépine − 2 − one,
1,3 − dihydro − 3(RS) − (5 − fluoro − indole − 2 − carbonylamino) − 5 − (2 − fluorophényl) − 2H − 1,4 − benzodiazépine − 2 − one,
3(RS) − cinnamoylamino − 1,3 − dihydro − 5 − (2 − fluorophényl) − 2H − 1,4 − benzodiazépine − 2 − one,
1,3 − dihydro − 5 − (2 − fluorophényl) − 3(RS) − (2 − benzofurannecarbonylamino) − 2H − 1,4 − benzodiazépine − 2 − one,
1,3 − dihydro − 1 − méthyl − 3(RS) − (4 − chlorophénylcarbonyl)amino − 5 − phényl − 2H − 1,4 − benzodiazépine − 2 − one,
1 − carboxyméthyl − 1,3 − dihydro − 3(RS) − (4 − chlorophénylcarbonyl)amino − 5 − (2 − fluorophényl) − 2H − 1,4 − benzodiazépine − 2 − one,
1,3 − dihydro − 5 − (2 − fluorophényl) − 3(RS) − (p − trifluorométhylbenzoylamino) − 2H − 1,4 − benzodiazépine − 2 − one,
3(S) − ( + ) − 3 − (3 − bromobenzoylamino) − 1,3 − dihydro − 5 − (2 − fluorophényl) − 1 − méthyl − 2H − 1,4 − benzodiazépine − 2 − one,
3(S) − ( + ) − 3 − (4 − bromobenzoylamino) − 1,3 − dihydro − 5 − (2 − fluorophényl) − 1 − méthyl − 2H − 1,4 − benzodiazépine − 2 − one,
3(S) − ( + ) − 1,3 − dihydro − 5 − (2 − fluorophényl) − 3 − (4 − iodobenzoylamino) − 1 − méthyl − 2H − 1,4 − benzodiazépine − 2 − one,
1,3 − dihydro − 3(RS) − 2 − naphtoylamino) − 5 − phényl − 2H − 1,4 − benzodiazépine − 2 − one,
3(S) − ( + ) − 1,3 − dihydro − 5 − (2 − fluorophényl) − 3 − (3 − iodobenzoylamino) − 1 − méthyl − 2H − 1,4 − benzodiazépine − 2 − one,
3(R) − ( − ) − 1,3 − dihydro − 5 − (2 − fluorophényl) − 3 − (3 − iodobenzoylamino) − 1 − méthyl − 2H − 1,4 − benzodiazépine − 2 − one ou
3(R) − ( − ) − 1,3 − dihydro − 5 − (2 − fluorophényl) − 3 − (2 − indolecarbonylamino) − 1 − méthyl − 2H − 1,4 − benzodiazépine − 2 − one.

**7.** Utilisation selon la revendication 1, caractérisée en ce que le composé de formule I est l'un des suivants:
1 − carboxyméthyl − 1,3 − dihydro − 3(RS) − (2 − indole − carbonylamino) − 5 − phényl − 2H − 1,4 − benzodiazépine − 2 − one,
(S) − ( − ) − 1,3 − dihydro − 3 − (2 − indole − carbonylamino) − 1 − méthyl − 5 − phényl − 2H − 1,4 − benzodiazépine − 2 − one,
(S) − ( + ) − 1,3 − dihydro − 5 − (2 − fluorophényl) − 3 − (2 − indole − carbonylamino) − 1 − méthyl − 2H − 1,4 − benzodiazépine − 2 − one ou
(S) − ( + ) − 1,3 − dihydro − 3 − (4 − chlorobenzoylamino) − 5 − (2 − fluorophényl) − 1 − méthyl − 2H − 1,4 − benzodiazépine − 2 − one.

**8.** Procédé pour la préparation de nouveaux dérivés de type benzodiazépine correspondant à la formule la suivante

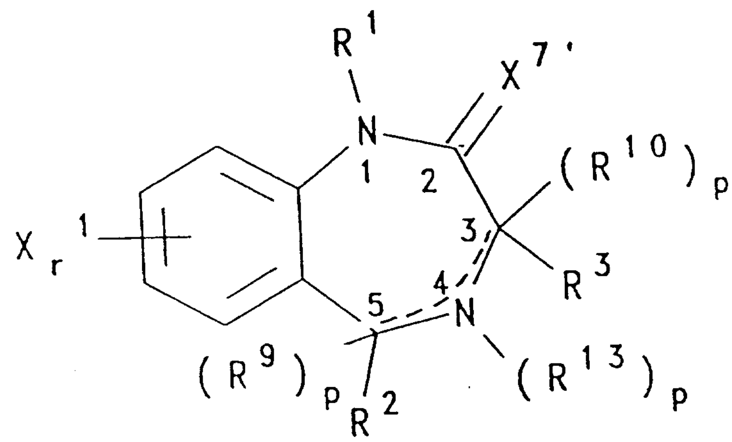

Ia

dans laquelle

$X^{7'}$ est S ou HH et

les autres substituants et symboles ont les mêmes significations que dans les composés de formule I définie dans la revendication 1,

ou de sels ou de sels d'ammonium quaternaire des composés de formule Ia, comprenant

a) le couplage de 2-aminoarylcétones

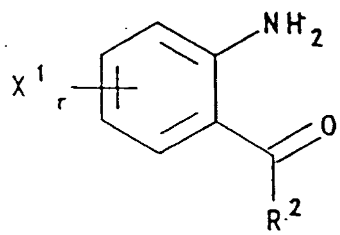

1

à des D-aminoacides protégés à l'azote

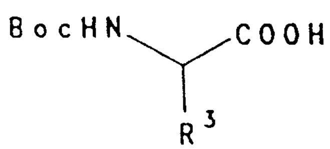

2

en présence d'un réactif de couplage de peptides, l'élimination du groupe protecteur à l'azote du produit

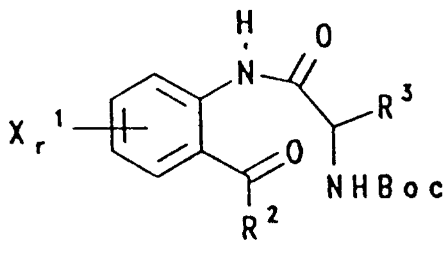

3

à l'aide d'un acide, de préférence l'acide chlorhydrique anhydre, pour obtenir le dérivé $\alpha$-aminoacylé

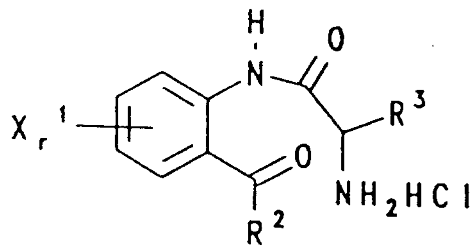

4

ce dérivé pouvant également être obtenu par traitement de la 2 – arylaminocétone 1 par un chlorure d'acide (chlorhydrate)

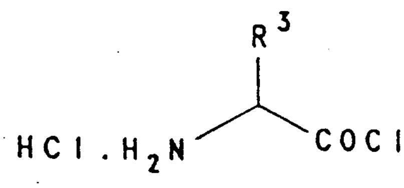

8

le traitement du dérivé α – aminoacylé 4 par une base et la cyclisation de la base libre

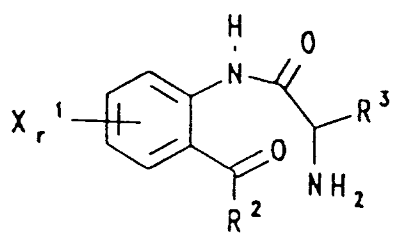

6

pour aboutir à la benzodiazépine 3,5 – disubstituée, par agitation dans la base méthanolique pendant 2 – 120 heures,

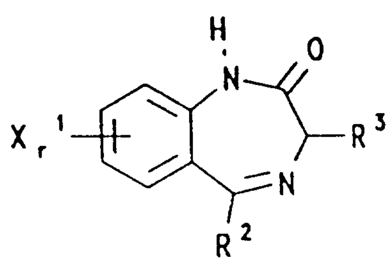

7

cette benzodiazépine pouvant également être obtenue par chauffage de la cétone 1 avec un ester

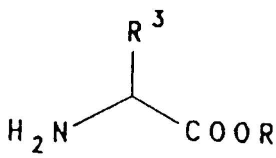

8,

ester dans lequel R est de préférence le groupe méthyle ou éthyle, le traitement des benzodiazépines 3,5 – disubstituées 7 par $P_2S_5$ ou le réactif de Lawesson, pour obtenir des 2 – thiones

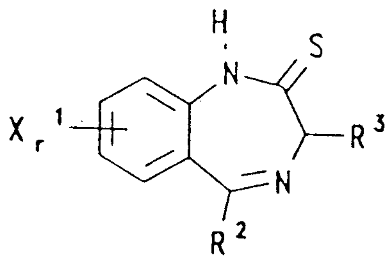

14

éventuellement la réduction des 2 – thiones 14 à l'aide de nickel de Raney, pour aboutir aux benzodiazépines non substituées en position 2

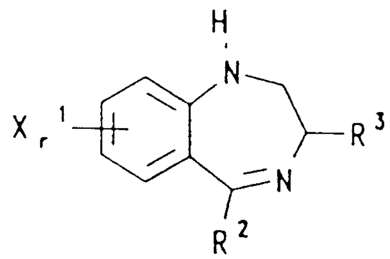

15

et éventuellement l'alkylation à l'aide d'un halogénure ou sulfate d'alkyle, l'acylation à l'aide d'un halogénure d'acyle ou anhydride d'acide, la réduction à l'aide du cyanoborohydrure de sodium ou une substitution par un halogénure d'alkyl – ou arylmagnésium, pour obtenir les dérivés alkylés, dérivés acylés, dérivés 4,5 – dihydroxylés et dérivés substitués correspondants, ou

b) le couplage des cétones 1 avec des N – phtalyaminoacides

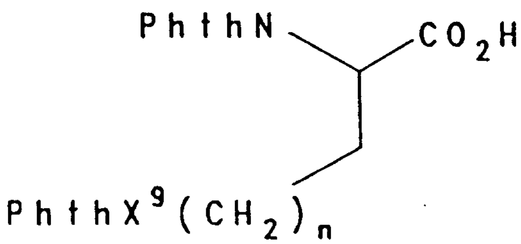

2b

en présence d'un réactif de couplage de peptides, et la cyclisation du produit intermédiaire par traitement par l'hydrazine

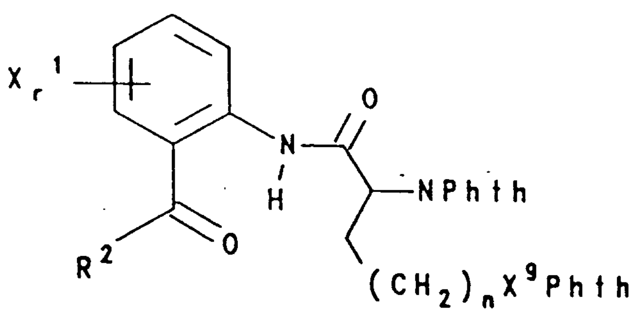

3b

éventuellement après N – alkylation par traitement par l'hydrure de sodium suivi de $R^1X$ dans du diméthylformamide, pour obtenir le dérivé de type benzodiazépine 9

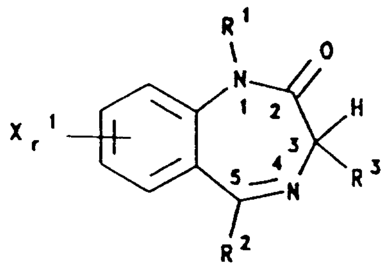

9

dans lequel

$R^3$ est $-(CH_2)_{n+1}-X^9H$, de tels composés de formule 9 pouvant être convertis en autres composés de formule 9 par:

a) alkylation par traitement par un halogénure d'alkyle ou sulfate de dialkyle de formule
$R^7-(CH_2)_q-X$
conduisant aux composés de formule 9a, dans lesquels $R^3$ est
$-(CH_2)_{n+1}X^9(CH_2)_qR^7$

b) acylation par traitement par des halogénures d'acides ou des anhydrides d'acides de formule

$$R^7-(CH_2)_q-\overset{O}{\overset{\|}{C}}-X$$

pour l'obtention des dérivés acylés de formule 9b correspondants, dans lesquels $R^3$ est

$$-(CH_2)_{n+1}X^9\overset{O}{\overset{\|}{C}}-(CH_2)_qR^7 \text{ ou}$$

c) conversion des composés de formule 9 dans lesquels $R^3$ est $-(CH_2)_{n+1}-NH_2$ en un dérivé correspondant dans lequel le groupe $NH_2$ est protégé par le groupe benzyloxycarbonyle, et conversion de ce composé en une 2-thione correspondante, par réaction avec $P_2S_5$ ou le réactif de Lawesson, et éventuellement réduction de la 2-thione à l'aide de nickel de Raney dans du méthanol, en les benzodiazépines non substituées en position 2, suivie de l'élimination du groupe protecteur par hydrogénolyse et éventuellement alkylation ou alcylation des produits résultants, par traitement par un halogénure d'alkyle ou un sulfate de dialkyle, en présence d'un agent de couplage de peptides, et, si on le désire, la préparation de sels ou de sels d'ammonium quaternaire de ces composés.

**9.** Procédé pour la préparation de nouveaux dérivés de type benzodiazépine correspondant à la formule Ib suivante

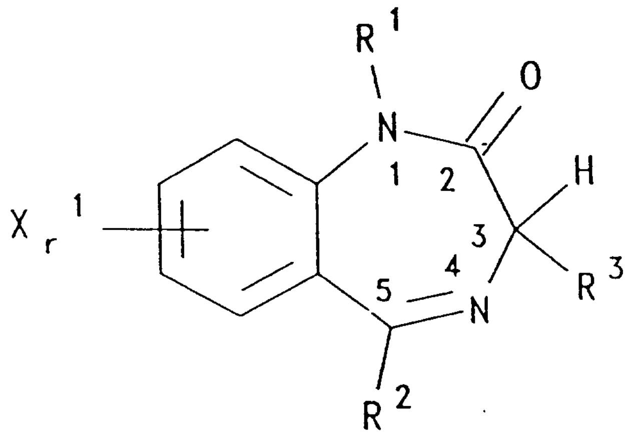

Ib

ou de sels ou de sels d'ammonium quaternaire des composés de formule Ib, composés de formule Ib dans lesquels

$R^1$ est un groupe alkyle ayant de 1 à 5 atomes de carbone ou un atome d'hydrogène;

$R^2$ est le radical phényle non substitué ou un radical phényle non substitué comportant 1 ou 2 substituants choisi(s) parmi des atomes d'halogène et des groupes alkyle ayant de 1 à 4 atomes de carbone, alcoxy ayant de 1 à 4 atomes de carbone, alkyle substitué par le groupe carboxy, ayant de 1 à 4 atomes de carbone dans le fragment alkyle, carboxy, nitro, trifluorométhyle et hydroxy;

$R^3$ est un groupe de formule

$$-(CH_2)_nNH\overset{O}{\overset{\|}{C}}(CH_2)_qR^7$$

ou

$$-(CH_2)_nNH\overset{O}{\overset{\|}{C}}NH(CH_2)_nR^7,$$

formules dans lesquelles

$R^7$ est le radical phényle non substitué ou un radical phényle substitué comportant un ou deux substituants choisi(s) parmi des atomes d'halogène et des groupes nitro, hydroxy, alkyle ayant de 1 à 4 atomes de carbone, alcoxy ayant de 1 à 4 atomes de carbone, alkylthio ayant de 1 à 4 atomes de carbone, trifluorométhyle ou des groupes de formules CN, $-C{\equiv}CH$, $SCF_3$,

$$O\overset{O}{\overset{\|}{C}}CH_3,$$

$OCHF_2$, SPh ou $-NR^4R^5$ dans lesquelles

$R^4$ et $R^5$ sont indépendamment un atome d'hydrogène ou un groupe alkyle ayant de 1 à 4 atomes de carbone, ou

$R^7$ est un groupe de formule

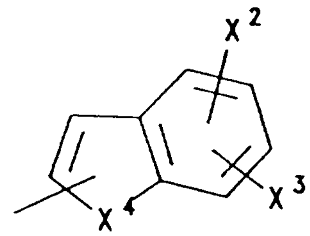,

dans laquelle

$X^2$ et $X^3$ sont indépendamment un atome d'hydrogène ou d'halogène ou un groupe hydroxy, nitro, alkylthio ayant de 1 à 4 atomes de carbone, alkyle ayant de 1 à 4 atomes de carbone ou alcoxy ayant de 1 à 4 atomes de carbone,

$X^4$ est NH,

q est 0 – 4, de préférence 0 et

n est 0 – 4, de préférence 0,

comprenant la poursuite des voies a) ou b) telles que définies dans la revendication 8, jusqu'au degré nécessaire, ou

c) l'introduction du substituant $R^3$, de formule

$$-NH-\overset{O}{\overset{\|}{C}}-(CH_2)_q-R^7$$

dans une benzodiazépine 9d substituée par un atome de chlore en position 3, substituée en position 5, substituée ou non substituée en position 1

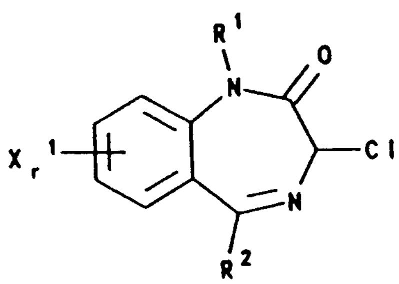

9d

ou

d) la réaction d'une benzodiazépine substituée en position 3 par un groupe amino ou aminoalkyle, substituée en position 5, substituée ou non substituée en position 1

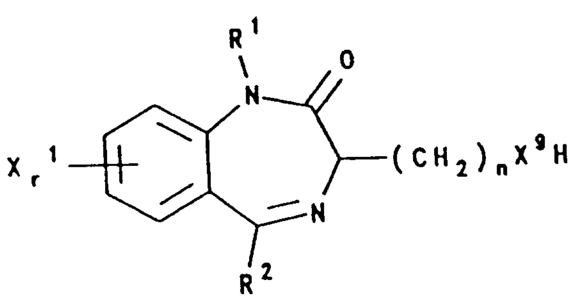

23

avec un halogénure d'acyle ou un isocyanate, pour obtenir l'amide correspondant ou l'urée correspondante

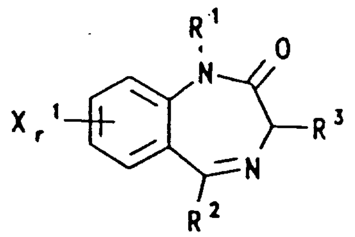

24

$$(R^3 = (CH_2)_n NH\overset{O}{\overset{\|}{C}}(CH_2)_q R^7$$

ou

$$(CH_2)_n\overset{H}{\overset{|}{N}}-\overset{O}{\overset{\|}{C}}-\overset{H}{\overset{|}{N}}(CH_2)_n R^7$$

et éventuellement l'alkylation des benzodiazépines de formule Ib obtenues, dans lesquelles $R^1$ = H, par traitement par l'hydrure de sodium dans un solvant convenable, tel que le DMF, suivi d'un halogénure d'alkyle, et la préparation, si on le désire, de sels ou de sels d'ammonium quaternaire de ces composés.

**10.** Procédé selon la revendication 9, dans lequel on prépare un dérivé de type benzodiazépine de formule Ib, dans lequel

- $R^1$ est le groupe méthyle,
- $R^2$ est le groupe phényle non substitué,
- $R^3$ est tel que défini dans la revendication 9,
- q et n étant 0 et
- $R^7$ est tel que défini dans la revendication 9, $X^2$ et $X^3$ dans le groupe indolyle étant toutefois des atomes d'hydrogène.

**11.** Procédé selon la revendication 9 pour la préparation du composé qui est la 3(S) – ( – ) – 1,3 – dihydro – 3 – (2 – indole – carbonylamino) – 1 – méthyl – 5 – phényl – 2H – 1,4 – benzodiazépine – 2 – one.